# EUROPEAN PATENT APPLICATION

(11) **EP 2 130 820 A1**
(43) Date of publication of application: **09.12.2009**
(21) Application number: 09167247.7
(22) Date of filing: 18.02.2003
(51) Int. Cl.: C07D 221/12, C07D 263/46, C07D 277/18, C07D 401/06, C07D 405/12, C07D 413/12, C07D 417/04, C07D 417/06, C07D 417/12, C07D 455/06, C07D 471/04, C07D 513/04, A61P 29/00, A61P 37/00

(54) **Antipruritics**

(30) Priority: 19.02.2002 JP 2002041408
(62) Divisional of application: 03705285.9
(71) Applicant: SHIONOGI & CO., LTD., Osaka-shi, Osaka 541-0045 (JP)
(72) Inventor: The designation of the inventor has not yet been filed
(74) Representative: Vossius & Partner

(57) **Abstract**

The present invention relates to the use of a compound having an agonistic activity to the cannabinoid receptor, a prodrug, a pharmaceutically acceptable salt or solvate thereof for the preparation of an antipruritic, wherein the compound having an agonistic activity to the cannabinoid receptor is a compound selected from 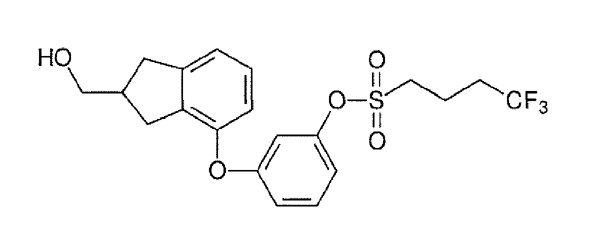 (R,S)-4,4,4-trifluoro-1-butane sulfonic acid-3-(2-hydroxymethyl-indanyl-4-oxy)-phenylester; 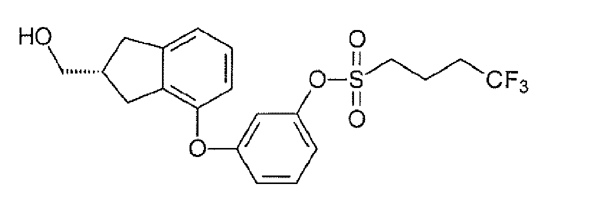 (S)-1-(4,4,4-trifluoro-1-butane sulfonic acid-3-(2-hydroxymethyl-indanyl-4-oxy)phenyl ester; and 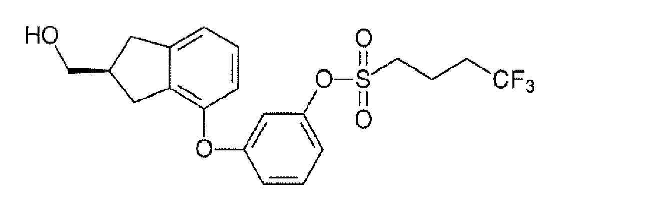 (R)-1-(4,4,4-trifluoro-1-butane sulfonic acid-3-(2-hydroxymethyl-indanyl-4-oxy)phenyl ester.

## Description

### Technical Field

The present invention relates to antipruritics, in detail, antipruritics which contains as an active ingredient a compound having an agonistic activity to the cannabinoid receptor.

### Background Art

It is well known that pruritus is produced in diseases such as atopic dermatitis, urticaria, allergic rhinitis and allergic conjunctivitis as well as inflammatory response like edema. Pruritus is the major symptom in allergic skin disorders, for example, atopic dermatitis and urticaria, and is also observed in systemic diseases such as chronic renal failure that requires hemodialysis and cholestatic chronic hepatitis.

At present, antihistamines are used as antipruritics. They are effective against pruritus accompanying with edema and urticaria, but are not enough to suppress pruritus caused by other diseases. Therefore, the development of potent antipruritics (inhibitors for pruritus, stoppers for pruritus, suppressors for pruritus) is expected.

In addition, the behaviors accompanying with pruritus such as scratching and beating possibly aggravate the symptoms of the diseases described above. Accordingly, antipruritics can also be expected as prophylactic and therapeutic agents for the disorders that are caused collaterally by the behaviors accompanying with pruritus, for example, cataract, detachment of the retina, inflammation, infection and sleeplessness.

Cannabinoid was discovered as the main active substance contained in marijuana in 1960 and found to exhibit an activity in the central nervous system (illusion, euphoria, sensory confusion of time and space) and in the peripheral cell system (immunosuppressive activity, anti-inflammatory activity, analgesic activity).

After that, anandamide and 2-arachidonoylglycerol produced from arachidonic acid-containing phospholipids were discovered as endogenous agonists to the cannabinoid receptor. These endogenous agonists are known to exhibit an activity to the central nervous system and an activity to the peripheral cell system.

A cannabinoid type 1 receptor discovered in 1990 was found to distribute in the central nervous system such as the brain. Agonists to this receptor were found to suppress the release of neurotransmitters to cause central actions such as illusion. A cannabinoid type 2 receptor discovered in 1993 was found to distribute in immune tissues such as the spleen. Agonists to this receptor were found to suppress an activation of immunocyte or inflammatory cells to exhibit an immunosuppressive activity, an anti-inflammatory activity and an analgesic activity (Nature, 1993, 365, 61-65).

However, these references do not disclose that agonists to the cannabinoid receptor have an antipruritic effect.

### Disclosure of Invention

It is intended to provide potent antipruritics (inhibitors for pruritus, stoppers for pruritus, suppressors for pruritus) having a sufficient effect against itching caused from various disease.

The inventors of the present invention have found out that the compounds having an agonistic activity to the cannabinoid receptor exhibit an antipruritic effect. Furthermore, the inventors of the present invention have recognized that the antipruritic effect caused by the compounds having an agonistic activity to the cannabinoid receptor is blocked by the compounds having an antagonistic activity to the cannabinoid receptor, whereby to achive the following inventions.

That is, the present invention relates to:
1) an antipruritics which contains as an active ingredient a compound having an agonistic activity to the cannabinoid receptor, a prodrug, a pharmaceutically acceptable salt or solvate thereof.
   In more detail, the present invention relates to the following 2) to 14).
2) an antipruritics according to 1) wherein a compound having an agonistic activity to the cannabinoid receptor is a compound having an agonistic activity to the cannabinoid type 1 receptor,
3) an antipruritics according to 1) wherein a compound having an agonistic activity to the cannabinoid receptor is a compound having an agonistic activity to the cannabinoid type 2 receptor,
4) an antipruritics according to 1) wherein a compound having an agonistic activity to the cannabinoid receptor is a compound having an agonistic activity to the cannabinoid type 1 receptor and the cannabinoid type 2 receptor,
5) an antipruritics according to 1) wherein a compound having an agonistic activity to the cannabinoid receptor is a compound selected from compounds represented by the formula (I): 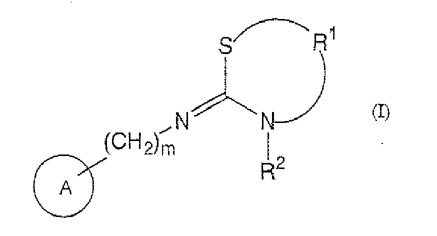 wherein R¹ is optionally substituted alkylene;
   R² is alkyl; a group of the formula: -C(=R³)-R⁴ wherein R³ is O or S, R⁴ is alkyl, alkoxy, alkylthio, alkenylthio, optionally substituted amino, optionally substituted aralkyloxy, optionally substituted aralkylthio, optionally substituted aralkylamino, alkoxyalkyl, alkylthioalkyl or optionally substituted aminoalkyl; or a group of the formula: -SO₂R⁵ wherein R⁵ is alkyl, optionally substituted amino, optionally substituted aryl or optionally substituted heteroaryl;
   m is an integer of 0 to 2;
   A is optionally substituted aryl or optionally substituted heteroaryl,
6) an antipruritics according to 5) wherein R¹ is C2-C9 straight or branched alkylene optionally substituted with alkylene; R² is a group of the formula: -C(=R³)-R⁴ wherein R³ is O or S, R⁴ is alkoxy, alkylthio, or alkenylthio; m is 0; A is aryl optionally substituted with 1 or 2 substitutent(s) selected from the group consisting of alkyl, alkoxy, haloalkoxy, and alkylthio,
7) an antipruritics according to 1) wherein a compound having an agonistic activity to the cannabinoid receptor is a compound selected from compounds represented by the formula (II): 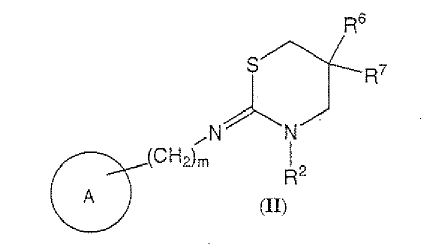 wherein R² is optionally substituted heterocyclic group or a group of the formula: - C(=Z)W-R⁸ wherein Z is O or S; W is O or S; R⁸ is optionally substituted alkyl, optionally substituted alkenyl or optionally substituted alkynyl;
   R⁶ and R⁷ each is independently hydrogen, optionally substituted alkyl, optionally substituted alkoxyalkyl, optionally substituted aminoalkyl, or optionally substituted cycloalkyl; or
   R⁶ and R⁷ taken together form optionally substituted alkylene which may contain a heteroatom(s);
   m is an integer of 0 to 2;
   A is optionally substituted aryl or optionally substituted heteroaryl,
8) an antipruritics according to 7) wherein m is 0; A is aryl optionally substituted with 1 or 2 substitutent(s) selected from group consisting of alkyl, alkoxy, haloalkoxy, or alkylthio,
9) an antipruritics according to 7) or 8) wherein R² is a group of the formula: -C(=Z)W-R⁸ wherein Z is O or S; W is O or S, R⁸ is optionally substituted alkyl or optionally substituted alkenyl; R⁶ and R⁷ each is independently optionally substituted alkyl; or R⁶ and R⁷ taken together form optionally substituted alkylene which may contain a heteroatom(s).,
10) an antipruritics according to 1) wherein a compound having an agonistic activity to the cannabinoid receptor is a compound selected from compounds represented by the formula (III): 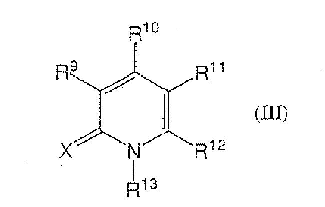 wherein R⁹ is hydrogen, halogen, cyano, formyl, acyl, carboxy, alkoxycarbonyl, optionally substituted carbamoyl, isothiocyanato, optionally substituted amino, hydroxy, alkoxy, alkylthio, alkenyloxy, alkynyloxy, alkylsulfinyl, alkylsulfonyl, nitro, or a group of the formula: -Y¹-Y²-Y³-R^{a} wherein Y¹ and Y³ each is independently single bond or optionally substituted alkylene; Y² is single bond, -O-, -O-C(=O)-, -O-C(=O)-O-, -O-C(=O)-NR^{b}-, -O-SO₂-, -NR^{b}-, -NR^{b}-C(=O)-, -NR^{b}-SO₂-, -NR^{b}-C(=NH)-, -NR^{b}-C(=O)-O-, -NR^{b}-C(=O)-NR^{b}-, -NR^{b}-C(=O)-NR^{b}-SO₂-, -NR^{b}-C(=S)-, -NR^{b}-C(=S)-NR^{b}-, -NR^{b}-SO₂-NR^{b}-, -NR^{b}-C(=NH)-NR^{b}-, -S-, -SO₂-O-, -SO₂-NR^{b}-, -SO₂-NR^{b}-C(=O)-NR^{b}-, -C(=O)-O-, -C(=O)-NR^{b}-, -C(=O)-NR^{b}-C(=O)-, -C(=O)-NR^{b}-C(=S)-, -C(=S)-NR^{b}-, -C(=S)-NR^{b}-C(=O)-, C(=NH)-NR^{b}-, -C(=O)-, -C(=O)-NR^{b}-C(=NR^{b})-, or -C(=O)-NR^{b}-NR^{b}-; R^{a} is optionally substituted alkyl, optionally substituted alkenyl, optionally substituted alkynyl, optionally substituted carbocyclic group, optionally substituted heterocyclic group, or acyl; R^{b} each is independently hydrogen, optionally substituted alkyl, optionally substituted alkenyl, optionally substituted alkynyl, optionally substituted carbocyclic group, optionally substituted heterocyclic group, acyl, hydroxy, or alkoxy;
   R¹⁰ is hydrogen, optionally substituted alkyl, optionally substituted alkenyl, optionally substituted alkynyl, halogen, cyano, formyl, acyl, carboxy, alkoxycarbonyl, optionally substituted carbamoyl, isothiocyanato, optionally substituted amino, hydroxy, alkoxy, alkylthio, alkenyloxy, alkynyloxy, alkylsulfinyl, alkylsulfonyl, nitro, or a group of the formula: -Y⁴-R^{c} wherein Y⁴ is single bond, -O-, -S-, -SO-, -SO₂-, -NH-, - C(=O)-, -CH₂-, -C(=O)-NH-, or -NH-C(=O)-; R^{c} is optionally substituted carbocyclic group or optionally substituted heterocyclic group;
   R¹¹ and R¹² each is independently hydrogen, optionally substituted alkyl, optionally substituted alkenyl, optionally substituted alkynyl, halogen, cyano, formyl, acyl, carboxy, alkoxycarbonyl, optionally substituted carbamoyl, isothiocyanato, optionally substituted amino, hydroxy, alkoxy, alkylthio, alkenyloxy, alkynyloxy, alkylsulfinyl, alkylsulfonyl, nitro or a group of the formula: -Y⁵-R^{d} wherein Y⁵ is single bond, optionally substituted alkylene, alkenylene, alkynylene, -O-, -S-, -SO-, -SO₂-,-NH-, -C(=O)-, -CH₂-, -C(=O)-NH-E-, or -NH-C(=O)-; E is single bond or optionally substituted alkylene; R^{d} is optionally substituted carbocyclic group or optionally substituted heterocyclic group;
   R¹³ is hydrogen, optionally substituted alkyl which may have heteroatom and/or unsaturated bond or a group of the formula: -Y⁶-R^{e} wherein Y⁶ is single bond, optionally substituted alkylene, alkenylene, alkynylene, -O-, -S-, -SO-, -SO₂-, -NH-, - C(=O)-, -CH₂-, -C(=O)-NH-E-, or -NH-C(=O)-; E is single bond or optionally substituted alkylene; R^{e} is optionally substituted carbocyclic group or optionally substituted heterocyclic group; or
   any one of combinations of R¹⁰ and R¹¹, R¹¹ and R¹², and R¹² and R¹³, taken together with the adjacent atoms form optionally substituted cyclic group which may have heteroatom(s) and/or unsaturated bond(s);
   X is S or O,
11) an antipruritics according to 10) wherein R⁹ is a group of the formula: -Y¹-Y²-Y³-R^{a} wherein Y¹, Y², Y³ and R^{b} are as defined in 10); R^{a} is optionally substituted carbocyclic group, optionally substituted heterocyclic group, or acyl; R¹⁰ is hydrogen atom or optionally substituted alkyl; R¹¹ is optionally substituted alkyl, halogen atom, or a group of the formula: -Y⁵-R^{d} wherein Y⁵ is a single bond or alkynylene; R^{d} are as defined in 10); R¹² is hydrogen atom or optionally substituted alkyl; R¹³ is optionally substituted C3 or more alkyl which may have heteroatom(s) and/or unsaturated bond(s) or a group of the formula: -Y⁶-R^{e} wherein Y⁶ and R^{e} are as defined in 10); or R¹¹ and R¹² taken together with the adjacent atoms form optionally substituted cyclic group which may have heteroatom(s) and/or unsaturated bond(s),
12) an antipruritics according to 10) wherein R⁹ is a group of the formula: -Y¹-Y²-Y³-R^{a} wherein Y¹ is a single bond; Y² is -C(=O)-NH-; Y³ is single bond or optionally substituted alkyl; R^{a} is optionally substituted carbocyclic group, or acyl; R¹⁰ is hydrogen atom; R¹¹ and R¹² taken together with the adjacent atoms form optionally substituted cyclic group which may have heteroatom(s) and/or unsaturated bond(s); R¹³ is optionally substituted alkyl which may have heteroatom(s) and/or unsaturated bond(s); X is S or O,
13) method for treating prurituswhich comprises the administration of a compound having an agonistic activity to the cannabinoid receptor, a prodrug, a pharmaceutically acceptable salt or solvate thereof,
14) use of a compound having an agonistic activity to the cannabinoid receptor, a prodrug, a pharmaceutically acceptable salt or solvate thereof for the preparation of an antipruritics.

### Brief Description of Drawings

Figure 1 shows that a compound acting as an agonist to the cannabinoid type 1 receptor exhibits an antipruritic effect. X-axis represents test samples, and Y-axis represents percentage of relative number of scratching to control.

### Best Mode for Carrying Out the Invention

Evaluation of pruritus can be performed according to the method described in Eur. J. Pharmacol. 1995; 275: 229-233. Briefly, mice are injected with pruritogenic agents in the back, and the number of scratching behavior by hind paws is counted. Among various pruritogenic agents, compound 48/80, a mast cell activator, is one of those frequently used.

Using the compounds acting as agonists to cannabinoid receptors, the inventor of the present invention examined the antipuritic effect of these compounds in the test described above. As a result, it was demonstrated that the compounds acting as agonists to cannaninoid both type 1 and type 2 receptors had antipruritic activities. In addition, the compounds acting as agonists to cannabinoid receptors inhibited pruritus induced by not only compound 48/80 but also substance P.

Furthermore, the inventor of the present inventions recognize that an antipruritic effect caused by the compounds having an agonistic activity to the cannabinoid type 1 receptor is blocked by the compounds having an antagonistic activity to the cannabinoid type 1 receptor, and an antipruritic effect caused by the compounds having an agonistic activity to the cannabinoid type 2 receptor is blocked by the compounds having an antagonistic activity to the cannabinoid type 2 receptor.

As a compound having an antagonistic activity to the cannabinoid type 1 receptor was used SR141716A described in FEBS Lett. 1994, Aug, 22, 350(2-3), p240-244, and as the compound having an antagonistic activity to the cannabinoid type 2 receptor was used SR144528 described in J. Pharmacol. Exp. Ther. 1998, Feb, 284(2), p644-650.

As a compound having an agonistic activity to the cannabinoid receptor can be used any kind of compounds having an agonistic activity to the cannabinoid receptor. Therefore, the chemical structures of the compounds are not to limited.

Especially, preferred are the compounds having an affinity to the cannabinoid receptor (Ki value), an inhibitory activity to the production of cAMP (LC₅₀ value), each 1000 nmol/L or less, more preferably 300 nmol/L or less, and most preferably 100 nmol/L or less.

These Ki value and LC₅₀ value can be measured by known methods such as Experimental Example 1 and 2 described in the present specification.

Examples of agonists to the cannabinoid receptor include the following compounds.
(1) The compounds described in 5),
(2) the compounds described in 6),
(3) the compounds described in 7),
(4) the compounds described in 8),
(5) the compounds described in 9),
(6) the compounds described in 10),
(7) the compounds described in 11),
(8) the compounds described in 12),
(9) the compounds described in WO 97/29079,
(10) the compounds described in WO 99/02499,
(11) the compounds described in WO 00/40562,
(12) anandamide,
(13) 2-arachidonylglycerol,
(14) 1(3)-arachidonylglycerol,
(15) palmitoylethanolamide,
(16) dronabinol (delta-9-tetrahydrocannabinol)
(17) nabilone,
(18) the compound described in Exp. Opin. Ther. Patents (1998), 8(3), p301-313,
(19) the compound described in WO 98/41519,
(20) the compound described in any one of US 3968125, EP 570920, WO 94/01429, US 4260764, US 4371720, US 5605906, WO 96/18391, WO 96/18600, US 5081122, US 5292736, WO 97/00860, US 5532237,
(21) the compound described in WO 2000-10968,
(22) the compound described in DE 11115886 A1,
(23) the compound described in DE 19837627,
(24) the compound described in Exp. Opin. Ther. Patents (2002), 12(10), p1475-1489,
(25) the compound described in WO 02/42248.

The compounds described in (1) or (2) include the compounds described in WO 01/19807. The compounds described in any one of above (3) to (5) include the compounds described in Japanese patent application number 2001-65386. The compounds described in any one of above (6) to (8) include the compounds described in PCT/JP01/11427.

As the compounds described in above (9) are preferred the following compounds.

(E)-N-[2-(4-hydroxyphenyl)ethyl]-3-(4-methoxy-3-pentyloxyphenyl)acrylamide, 3-(4-ethoxy-3-pentyloxyphenyl)-(E)-N-[2-(4-hydroxyphenyl)ethyl]acrylamide, 3-(3,4-dipentyloxyphenyl)-(E)-N-[2-(4-hydroxyphenyl)ethyl]acrylamide, (E)-N-[2-(4-hydroxyphenyl)ethyl]-3-(4-methoxy-3-butyloxyphenyl)acrylamide, (E)-N-[2-(4-hydroxyphenyl)ethyl]-3-(4-methoxy-3-hexyloxyphenyl)acrylamide, (E)-N-[2-(4-hydroxyphenyl)ethyl]-3-(4-methoxy-3-heptyloxyphenyl)acrylamide, (E)-N-[2-(3-hydroxyphenyl)ethyl]-3-(4-methoxy-3-pentyloxyphenyl)acrylamide, (E)-N-[2-(2-hydroxyphenyl)ethyl]-3-(4-methoxy-3-pentyloxyphenyl)acrylamide, (E)-N-[2-(4-hydroxycyclohexyl)ethyl]-3-(4-methoxy-3-pentyloxyphenyl)acrylamide, (E)-N-[2-(4-hydroxyphenyl)ethyl]-N-methyl-3-(4-methoxy-3-pentyloxyphenyl)-acrylamide, (E)-N-[2-(4-hydroxyphenyl)ethyl]-3-(3-isopentyloxy-4-methoxyphenyl)acrylamide, 3-[3-(2-ethylbutyloxy)-4-methoxyphenyl]-(E)-N-[2-(4-hydroxyphenyl)ethyl]acrylamide, (E)-N-[2-(4-hydroxy-3-methoxyphenyl)ethyl]-3-(4-methoxy-3-pentyloxyphenyl)-acrylamide, 3-[3-(1,1-dimethylheptyl)-4-methoxyphenyl]-(E)-N-[2-(4-hydroxyphenyl)ethyl]-acrylamide, (E)-N-[2-(3,4-dihydroxyphenyl)ethyl]-3-[3-(1,1-dimethylheptyl)-4-methoxyphenyl]-acrylamide, 3-(3-hexyl-4-methoxyphenyl)-(E)-N-[2-(4-hydroxyphenyl)ethyl]acrylamide, (E)-N-(4-amino-3-pentyloxyphenyl)-N-[2-(4-hydroxyphenyl)ethyl]acrylamide, (E)-N-(4-amino-3-pentyloxyphenyl)-N-[2-(4-nitrophenyl)ethyl]acrylamide, 3-(4-methoxy-3-pentyloxyphenyl)-(E)-N-[2-(4-pentyloxyphenyl)ethyl]acrylamide, (E)-N-[2-(4-methoxyphenyl)ethyl]-3-(4-methoxy-3-pentyloxyphenyl)acrylamide, 3-(4-methoxy-3-pentyloxyphenyl)-(E)-N-(2-morpholinoethyl)acrylamide, (E)-N-[2-(3,4-dihydroxyphenyl)ethyl]-3-(4-methoxy-3-pentyloxyphenyl)acrylamide, 2-[2-{3-(3-pentyloxy-4-methoxyphenyl)acryloylamino}ethyl]pyridine-N-oxide, (E)-N-[2-(4-hydroxyphenyl)ethyl]-3-(4-methoxy-3-pentylaminophenyl)acrylamide, 3-[3-(N',N'-dipentylamino)-4-methoxyphenyl]-(E)-N-[2-(4-hydroxyphenyl)ethyl]-acrylamide, (E)-N-[2-(4-hydroxyphenyl)ethyl]-3-(3-pentylamino-4-pentyloxyphenyl)acrylamide, (E)-N-[2-(4-hydraxyphenyl)ethyl]-3-[3-(N'-methyl-N'-pentylamino)-4-methoxyphenyl]-acrylamide, (E)-N-[2-(4-hydroxyphenyl)ethyl]-3-(4-methoxy-3-pentylthiophenyl)acrylamide, (E)-N-[2-(4-hydroxyphenyl)ethyl]-3-(4-pentyloxy-3-pentylthiophenyl)acrylamide, (E)-N-[2-(4-aminophenyl)ethyl]-3-(4-methoxy-3-pentyloxyphenyl)acrylamide, (E)-N-[2-(4-hydroxyphenyl)ethyl]-3-(3-pentyloxy-4-pentylthiophenyl)acrylamide, (E)-N-[2-(4-hydroxyphenyl)ethyl]-3-(3-pentyloxy-4-methylthiophenyl)acrylamide, (E)-N-[2-(4-aminophenyl)ethyl]-3-(4-methoxy-3-pentylthiophenyl)acrylamide, (E)-N-[2-(4-nitrophenyl)ethyl]-3-(4-methoxy-3-pentylthiophenyl)acrylamide, (E)-N-[2-(imidazol-4-yl)ethyl]-3-(4-methoxy-3-pentylthiophenyl)acrylamide, (E)-N-[2-(4-nitrophenyl)ethyl]-3-(4-methoxy-3-pentylaminophonyl)acrylamide, (E)-N-[2-(imidazol-4-yl)ethyl]-3-(4-methoxy-3-pentylaminophenyl)acrylamide, (E)-N-[2-(4-hydroxyphenyl)ethyl]-3-(4-methylamino-3-pentyloxyphenyl)acrylamide, (E)-N-[2-(4-aminophenyl)ethyl]-3-(4-methoxy-3-pentylaminophenyl)acrylamide, (E)-N-[2-(4-nitrophenyl)ethyl]-3-(4-methylamino-3-pentyloxyphenyl)acrylamide, 3-(4-methoxy-3-pentyloxyphenyl)-(E)-N-[2-(4-thiophen-2-yl)ethyl]acrylamide, (E)-N-[2-(4-hydroxyphenyl)ethyl]-3-[(N'-methyl-N'-pentylamino)-4-pentyloxyphenyl]-acrylamide, (E)-N-[2-(4-hydroxyphenyl)ethyl]-3-(4-pentylamino-3-pentyloxyphenyl)acrylamide, (E)-N-[2-(4-cyanophenyl)ethyl]-3-(4-methoxy-3-pentyloxyphonyl)acrylamide, (E)-N-[2-(4-carbamoylphenyl)ethyl]-3-(4-methoxy-3-pentyloxyphenyl)acrylamide, N-[2-(4-hydroxyphenyl)ethyl]-4-methoxy-3-pentyloxybenzamide, 4-ethoxy-N-[2-(4-hydroxyphenyl)ethyl]-3-pentylaxybenzamide, 3,4-dipentyloxy-N-[2-(4-hydroxyphenyl)ethyl]benzamide, 4-dimethylamino-N-[2-(4-hydroxyphenyl)ethyl]-3-pentyloxybenzamide, N-[2-(4-hydroxyphenyl)ethyl]-3-pentylamino-4-methoxybenzamide, 3-butyloxy-N-[2-(4-hydroxyphenyl)ethyl]-4-methoxybenzamide, 3-hexyloxy-N-[2-(4-hydroxyphenyl)ethyl]-4-methoxybenzamide, 3-heptyloxy-N-[2-(4-hydroxyphenyl)ethyl]-4-methoxybenzamide, N-[2-(3-hydroxyphenyl)ethyl]-4-methoxy-3-pentyloxybenzamide, N-[2-(2-hydroxyphenyl)ethyl]-4-methoxy-3-pentyloxybenzamide, N-[2-(4-hydroxycyclohexyl)ethyl]-4-methoxy-3-pentyloxybenzamide, N-[2-(4-hydroxyphenyl)ethyl]-N-methyl-4-methoxy-3-pentyloxybenzamide, 3-isopentyloxy-N-[2-(4-hydroxyphenyl)ethyl]-4-methaxybenzamide, 3-(2-ethylbutyloxy)-N-[2-(4-hydroxyphenyl)ethyl]-4-methoxybenzamide, N-[2-(4-hydroxy-3-methoxyphenyl)ethyl]-4-hydroxy-3-pentyloxybenzamide, N-[2-(4-hydroxyphenyl)ethyl]-4-hydroxy-3-pentyloxybenzamide, N-[2-(4-hydroxyphenyl)ethyl]-4-hydroxy-N-methyl-3-pentyloxybenzamide, 3-(1,1-dimethylheptane)-N-[2-(4-hydroxyphenyl)ethyl]-4-methoxybenzamide, N-[2-(3,4-dihydroxyphenyl)ethyl]-3-(1,1-dimethylheptane)-4- methoxybenzamide, 3-(1,1-dimethylheptane)-N-[2-(4-hydroxy-3-methoxyphenyl)ethyl]-4-methoxybenzamide, 3-(1,1-dimethylheptane)-N-[2-(4-hydroxyphenyl)ethyl]-4-hydroxybenzamide, N-[2-(3,4-dihydroxyphenyl)ethyl]-3-(1,1-dimethylheptane)-4-hydroxybenzamide, 3-hexyl-N-[2-(4-hydroxyphenyl)ethyl]-4-methoxybenzamide, N-[2-(4-aminophenyl)ethyl]-3,4-dipentyloxybenzamide, 3,4-dihexyloxy-N-[2-(4-hydroxyphenyl)ethyl]benzamide, 4-methoxy-N-[2-(4-pentyloxyphenyl)ethyl]-3-pentyloxybenzamide, 4-methoxy-N-(2-morpholinoethyl)-3-pentyloxybenzamide, 4-methoxy-N-[2-(4-propen-2-yloxyphenyl)ethyl]-3-pentyloxybenzamide, N-[2-(4-hydroxyphenyl)ethyl]-4-methoxy-N-[2-(phenylsulfinyl)ethyl]-3-pentyloxybenzamide, N-[2-(3,4-dihydroxyphenyl)ethyl]-4-methoxy-3-pentyloxybenzamide, N-[2-(4-acetoxyphenyl)ethyl]-4-methoxy-3-pentyloxy-N-(E)-phenylthiovinylbenzamide, N-[2-(4-acetoxyphenyl)ethyl]-N-ethyl-4-methoxy-3-pentyloxybenzamide, 4-[2-{N-(4-methoxy-3-pentyloxyphenyl)amino}ethyl]pyridine-N-oxide, 3-[2-{N-(4-methoxy-3-pentyloxybenzoyl)amino}ethyl]pyridine-N-oxide, 3-dipentylamino-N-[2-(4-hydroxyphenyl)ethyl]-4-methoxybenzamide, N-[2-(4-hydroxyphenyl)ethyl]-3-isohexyl-4-methoxybenzamide, N-[2-(4-hydroxyphenyl)ethyl]-4-methoxy-3-(N'-methyl-N'-pentylamino)benzamide, N-[2-(4-hydroxyphenyl)ethyl]-3-pentylamino-4-pentyloxybenzamide, N-[2-(4-hydroxyphenyl)ethyl]-4-pentylamino-3-pentyloxybenzamide, 3,4-dipentyloxy-N-[2-(4-sulfamoylphenyl)ethyl]benzamide, 3,4-dipentyloxy-N-[2-(imidazol-4-yl)ethyl]benzamide, 3,4-dipentyloxy-N-[2-(4-nitrophenyl)ethyl]benzamide, 3,4-dipentyloxy-N-[2-(4-fluorophenyl)ethyl]benzamide, N-[2-(4-hydroxyphenyl)ethyl]-3-pentyloxy-4-propen-2-ylbenzamide, N-[2-(4-hydroxyphenyl)ethyl]-4-propyloxy-3-pentyloxybenzamide, 3,4-dibutyloxy-N-[2-(4-hydroxyphenyl)ethyl]benzamide, 3, 4-diheptyloxy-N- [2-(4-hydroxyphenyl)ethyl]benzamide, N-[2-(4-hydroxyphenyl)ethyl]-4-methylamino-3-pentyloxybenzamide, N-[2-(4-hydroxyphenyl)ethyl]-3,4-dipentylaminobenzamide, N-[2-(4-hydroxyphenyl)ethyl]-3-(N'-methyl-N'-pentylamino)-4-pentyloxybenzamide, 4-amino-N-[2-(4-hydroxyphenyl)ethyl]-3-pentyloxybenzamide, N-[2-(4-hydroxyphenyl)ethyl]-4-methoxy-3-pentylthiobenzamide, N-[2-(4-hydroxyphenyl)ethyl]-4-pentyloxy-3-pentylthiobenzamide, 3,4-dipentyloxy-N-[2-(2-thienyl)ethyl]benzamide, 3,4-dipentyloxy-N-[2-(5-hydroxyindol-3-yl)ethyl]benzamide, 3,4-dipentyloxy-N-[2-(4-methylaminophenyl)ethyl]benzamide, N-[2-(4-dimethylaminophenyl)ethyl]-3,4-dipentyloxybenzamide, 4-butyrylamino-N-[2-(4-hydroxyphenyl)ethyl]-3-pentyloxybenzamide, N-[2-(4-hydroxyphenyl)ethyl]-4-formylamino-3-pentylthiobenzamide, N-[2-(4-hydroxyphenyl)ethyl]-4-methylthio-3-pentyloxybenzamide, N-[2-(4-hydroxyphenyl)ethyl]-3-pentyloxy-4-pentylthiobenzamide, N-[2-(4-hydroxyphenyl)ethyl]-3-(4-hydroxybutyloxy)-4-methoxybenzamide, N-[2-(4-aminophenyl)ethyl]-4-methoxy-3-pentylthiobenzamide, 4-methoxy-N-[2-(4-nitrophenyl)ethyl]-3-pentylthiobenzamide, N-[2-(imidazol-4-yl)ethyl]-4-methoxy-3-pentylthiobenzamide, N-[2-(4-aminophenyl)ethyl]-4-pentyloxy-3-pentylthiobenzamide, N-[2-(4-nitrophenyl)ethyl]-4-pentyloxy-3-pentylthiobenzamide, N-[2-(imidazol-4-yl)ethyl]-4-pentyloxy-3-pentylthiobenzamide, 2-[2-(4-hydroxyphenyl)ethyl]-5-methoxy-4-pentyloxy-2,3-dihydroisoindol-1-one, 2-[2-(4-benzyloxyphenyl)ethyl]-5-methoxy-4-pentyloxy-2,3-dihydroisoindol-1-one, 5-methoxy-2-[2-(4-nitrophenyl)ethyl]-4-pentyloxy-2,3-dihydroisoindol-1-one, 2-[2-(4-methylphenyl)ethyl]-5-methoxy-4-pentyloxy-2,3-dihydroisoindol-1-one, 4,5-dipentyloxy-2-[2-(imidazol-4-yl)ethyl]-2,3-dihydroisoindol-1-one, 2-[2-(4-benzyloxyphenyl)ethyl]-4,5-dipentyloxy-2,3-dihydroisoindol-1-one, 4,5-dipentyloxy-2-[2-(4-nitrophenyl)ethyl]-2,3-dihydroisoindol-1-one, 2-[2-(4-aminophenyl)ethyl]-4,5-dipentyloxy-2,3-dihydroisoindol-1-one, 4,5-dipentyloxy-2-[2-(4-hydroxyphenyl)ethyl]-2,3-dihydrolsoindol-1-one, 4,5-dipentyloxy-2-[2-(4-methylaminophenyl)ethyl]-2,3-dihydroisoindol-1-one, 2-[2-(4-dimethylaminophenyl)ethyl]-4,5-dipentyloxy-2,3-dihydroisoindol-1-one, 2-[2-(4-aminophenyl)ethyl]-5-methoxy-4-pentyloxy-2,3-dihydroisoindol-1-one, 2-[2-(4-hydroxyphenyl)ethyl]-5-methoxy-4-pentylamino-2,3-dihydroisoindol-1-one, 5-methoxy-4-pentyloxy-2-[2-(4-pyridine)ethyl]-2,3-dihydrolsoindol-1-one, 2-[2-(4-dimethylaminophenyl)ethyl]-5-methoxy-4-pentylaxy-2,3-dihydroisoindol-1-one, 5-methoxy-2-[2-(4-methylaminophenyl)ethyl]-4-pentyloxy-2,3-dihydroisoindol-1-one, 2-[2-(4-benzyloxyphenyl)ethyl]-6-methoxy-5-pentyloxy-2H-isoquinolin-1-one, 2-[2-(4-hydroxyphenyl)ethyl]-6-methoxy-5-pentyloxy-2H-isoquinolin-1-one, 2-[2-(4-pyridyl)ethyl]-6-methoxy-5-pentyloxy-2H-isoquinolin-1-one, 4-[2-(6-methoxy-1-oxo-5-pentyloxy-1H-isoquinolin-2-yl)ethyl]phenyl acetate, 6-methoxy-2-[2-(4-nitrophenyl)ethyl]-5-pentyloxy-2H-isoquinolin-1- one, 2-[2-(4-methylphenyl)ethyl]-6-methoxy-5-pentyloxy-2H-isoquinolin-1-one, 6-methoxy-5-pentyloxy-2-(2-phenylethyl)-2H-isoquinolin-1-one, 2-[2-(4-acetylaminophenyl)ethyl]-6-methoxy-5-pentyloxy-2H-isoquinolin-1-one, 5,6-dipentyloxy-2-[2-(4-hydroxyphenyl)ethyl]-2H-isoquinolin-1-one, 2-[2-(4-aminophenyl)ethyl]-6-methoxy-5-pentyloxy-2H-isoquinolin-1-one, 2-[2-(4-aminophenyl)ethyl]-6-methoxy-5-pentyloxy-2H-isoquinolin-1-one hydrochloride, 2-[2-(4-dimethylaminophenyl)ethyl]-6-methoxy-5-pentyloxy-2H-isoquinolin-1-one, 2-[2-(4-methylaminophenyl)ethyl]-6-methoxy-5-pentyloxy-2H-isoquinolin-1-one, 6-methoxy-2-[2-(4-piperidinophenyl)ethyl]-5-pentyloxy-2H-isoquinolin-1-one and 6-methoxy-2-[2-(4-pyridyl)ethyl]-5-pentyloxy-2H-isoquinolin-1-one hydrochloride, 6-methoxy-2-[2-(4-oxocyclohexyl)ethyl]-5-pentyloxy-3,4-dihydro-2H-isoquinolin-1-one, 4-[2-(6-methoxy-1-oxo-5-pentyloxy-3,4-dihydro-1H-isoquinolin-2-yl)ethyl]phenyl aceate, 2-[2-(4-hydroxyphenyl)ethyl]-6-methoxy-5-pentyloxy-3,4-dihydro-2H-isoquinolin-1-one, 2-(2-phenylethyl)-6-methoxy-5-pentyloxy-3,4-dihydro-2H-isoquinolin-1-one, 2-[2-(4-acetylaminophenyl)ethyl]-6-methoxy-5-pentyloxy-3,4-dihydro-2H-isoquinolin-1-one, 6-hydroxy-2-[2-(4-hydroxyphenyl)ethyl]-5-pentyloxy-3,4-dihydro-2H-isoquinolin-1-one, 2-[2-(4-methylphenyl)ethyl]-6-methoxy-5-pentyloxy-3,4-dihydro-2H-isoquinolin-1-one, 2-[2-(4-aminophenyl)ethyl]-6-methoxy-5-pentyloxy-3,4-dihydro-2H-isoquinolin-1-one, 6-methoxy-5-pentyloxy-2-[2-(4-pyridyl)ethyl]-3,4-dihydro-2H-isoquinolin-1-one, 6-methoxy-1-oxo-5-pentyloxy-3,4-dihydro-1H-isoquinolin-2-carboxylic acid N-(4-aminophenyl)amide, 6-methoxy-1-oxo-5-pentyloxy-3,4-dihydro-1H-isoquinolin-2-carboxylic acid N-[(4-aminophenyl)methyl] amide, 6-methoxy-1-oxo-5-pentyloxy-3,4-dihydro-1H-isoquinolin-2-carboxylic acid N- (4-nitrophenyl)amide, 7-methoxy-3-[2-(4-nitrophenyl)ethyl]-8-pentyloxy-(1H,3H)-quinazoline-2,4-dione, 7-methoxy-3-[2-(4-pyridyl)ethyl]-8-pentyloxy-(1H,3H)-quinazoline-2,4-dione, 3-[2-(4-aminophenyl)ethyl]-7-methoxy-8-pentyloxy-(1H,3H)-quinazoline-2,4-dione, 3-[2-(4-hydroxyphenyl)ethyl]-7-methoxy-8-pentyloxy-(1H,3H)-quinazoline-2,4-dione, 3-[2-(4-methylaminophenyl)ethyl]-7-methoxy-8-pentyloxy-(1H,3H)-quinazoline-2,4-dione, 3-[2-(4-dimethylaminophenyl)ethyl]-7-methoxy-8-pentyloxy-(1H,3H)-quinazoline-2,4-dione, 7-methoxy-8-pentyloxyquinoline-3-carboxylic acid N-[2-(4-pyridyl)ethyl]amide, 7-methoxy-8-pentyloxyquinoline-3-carboxylic acid N-[2-(4-hydroxyphenyl)ethyl]amide, 7-methoxy-8-pentyloxyquinoline-3-carboxylic acid N-[2-(4-aminophenyl)ethyl]amide, 7-methoxy-8-pentyloxyquinoline-3-carboxylic acid N-[2-(4-nitrophenyl)ethyl]amide, 7-methoxy-8-pentyloxyquinoline-3-carboxylic acid N-[2-(imidazol-4-yl)ethyl]amide, 2-(4-methoxy-3-pentyloxyphenyl)-4,4-dimethyl-4,5-dihydrooxazole, 2-(4-methoxy-3-pentylthiophenyl)-4,4-dimethyl-4,5-dihydrooxazole, 2-(3,4-dipentyloxyphenyl)-4,4-dimethyl-4,5-dihydrooxazole, 2-(4-methylthio-3-pentyloxyphenyl)-4,4-dimethyl-4,5-dihydrooxazole, 2-(3-pentyloxy-4-pentylthiophenyl)-4,4-dimethyl-4,5-dihydrooxazole, 2-(4-pentyloxy-3-pentylthiophenyl)-4,4-dimethyl-4,5-dihydrooxazole and 2-(4-methoxy-3-pentyloxyphenyl)-5-(2-pyridyl)-4,5-dihydrooxazole.

As the compounds described in above (10) are preferred the following compounds.
7-methoxy-2-oxo-8-pentyloxy-1,2-dihydroquinoline-3-carboxylic acid (2-pyridin-4-ylethyl)amide,
7-methoxy-2-oxo-8-pentyloxy-1,2-dihydroquinoline-3-carboxylic acid (4-aminobenzyl)amide,
7-methoxy-2-oxo-8-pentyloxy-1,2-dihydroquinoline-3-carboxylic acid (2-(4-aminophenyl)ethyl)amide,
7-methoxy-2-oxo-8-pentyloxy-1,2-dihydroquinoline-3-carboxylic acid (4-aminophenyl)-amide.

As the compounds described in above (11) are preferred the following compounds.
7-methoxy-2-oxo-8-pentyloxy-1,2-dihydroquinoline-3-carboxylic acid (2-pyridine-4-ylethyl)amide,
7-methoxy-2-oxo-8-pentyloxy-1,2-dihydroquinoline-3-carboxylic acid (4-aminobenzyl)amide,
7-methoxy-2-oxo-8-pentyloxy-1,2-dihydroquinoline-3-carboxylic acid [2-(4-aminophenyl)ethyl]amide,
7-methoxy-2-oxo-8-pentyloxy-1,2-dihydroquinoline-3-carboxylic acid (4-aminophenyl)amide hydrochloride,
7-methoxy-2-oxo-8-pentyloxy-1,2-dihydroquinoline-3-carboxylic acid (3,4-methylenedioxybenzyl)amide,
8-ethoxy-7-methoxy-2-oxo-1,2-dihydroquinoline-3-carboxylic acid (2-pyridine-4-ylethyl)amide,
7-methoxy-2-oxo-8-pentyloxy-1,2-dihydroquinoline-3-carboxylic acid [2-(4-hydroxyphenyl)ethyl]amide,
7-methoxy-2-oxo-8-pentyloxy-1,2-dihydroquinoline-3-carboxylic acid [2-(4-fluorophenyl)ethyl]amide,
7-methoxy-2-oxo-8-pentyloxy-1,2-dihydroquinoline-3-carboxylic acid (4-pyridyl methyl)amide,
7-methoxy-2-oxo-8-pentyloxy-1,2-dihydroquinoline-3-carbaxylic acid (2-piperidinoethyl)amide,
7-methoxy-2-oxo-8-pentyloxy-1,2-dihydroquinoline-8-carboxylic acid (2-morpholinoethyl) amide,
7-methoxy-2-oxo-8-pentyloxy-1,2-dihydroquinoline-3-carboxylic acid (3-pyridylmethyl)amide,
7-methoxy-2-oxo-8-pentyloxy-1,2-dihydroquinoline-3-carboxylic acid (2-pyridylmethyl)amide,
8-butoxy-7-methoxy-2-oxo-1,2-dihydroquinoline-3-carboxylic acid (2-phenylethyl)amide,
8-butoxy-7-methoxy-2-oxo-1,2-dihydroquinoline-3-carboxylic acid [2-(4-fluorophenyl)ethyl]amide,
8-butoxy-7-methoxy-2-oxo-1,2-dihydroquinoline-3-carboxylic acid (2-pyridine-4-ylethyl)amide,
8-butoxy-7-methoxy-2-oxo-1,2-dihydroquinoline-3-carboxylic acid (2-pyridine-4-ylethyl)amide hydrochloride,
8-ethoxy-7-methoxy-2-oxo-1,2-dihydroquinoline-3-carboxylic acid [2-(4-fluorophenyl)ethyl]amide,
7-methoxy-2-oxo-8-pentyloxy-1,2-dihydroquinoline-3-carboxylic acid [2-(2-fluorophenyl)ethyl]amide,
7-methoxy-2-oxo-8-pentyloxy-1,2-dihydroquinoline-3-carboxylic acid [2-(3-fluorophenyl)ethyl]amide,
7-methoxy-2-oxo-8-pentyloxy-1,2-dihydroquinoline-3-carboxylic acid [2-(4-hydroxy-3-methoxyphenyl)ethyl] amide,
7-methoxy-2-oxo-8-pentyloxy-1,2-dihydroquinoline-3-carboxylic acid [2-(4-chlorophenyl)ethyl]amide,
7-methoxy-2-oxo-8-pentyloxy-1,2-dihydroquinoline-3-carboxylic acid (2-phenylethyl)amide,
7-methoxy-2-oxo-8-pentyloxy-1,2-dihydroquinoline-3-carboxylic acid (4-methylbenzyl)amide,
7-methoxy-2-oxo-8-pentyloxy-1,2-dihydroquinoline-3-carboxylic acid (4-fluorobenzyl)amide,
7-methoxy-2-oxo-8-propoxy-1,2-dihydroquinoline-3-carboxylic acid (2-pyridine-4-ylethyl)amide,
7-methoxy-2-oxo-8-propoxy-1,2-dihydroquinoline-3-carboxylic acid [2-(4-fluorophenyl)ethyl]amide,
7-methoxy-2-oxo-8-propoxy-1,2-dihydroquinoline-3-carboxylic acid [2-(4-hydroxyphenyl)ethyl]amide,
7-methoxy-2-oxo-8-propoxy-1,2-dihydroquinoline-3-carboxylic acid (3,4-methylenedioxybenzyl)amide,
7-methoxy-2-oxo-8-propoxy-1,2-dihydroquinoline-3-carboxylic acid (2-phenylethyl)amide,
7,8-dimetboxy-2-oxo-1,2-dihydroquinoline-3-carboxylic acid [2-(4-fluorophenyl)ethyl]amide,
7-methoxy-2-oxo-6-pentyloxy-1,2-dihydroquinoline-3-carboxylic acid [2-(4-fluorophenyl)ethyl]amide,
7-methoxy-2-oxo-6-pentyloxy-1,2-dihydroquinoline-3-carboxylic acid (3,4-methylenedioxybenzyl)amide,
7-methoxy-2-oxo-6-pentyloxy-1,2-dihydroquinoline-3-carboxylic acid (2-morpholinoethyl)amide,
8-ethoxy-7-methoxy-2-oxo-1,2-dihydroquinoline-3-carboxylic acid (3,4-methylenedioxybenzyl)amide,
1-methyl-7-methoxy-2-oxo-3-pentyloxy-1,2-dihydroquinoline-3-carboxylic acid [2-(4-fluorophenyl)ethyl]amide,
1-methyl-7-methoxy-2-oxo-8-pentyloxy-1,2-dihydroquinoline-3-carboxylic acid (2-pyridine-4-ylethyl)amide,
1-methyl-7-methoxy-2-oxo-8-pentyloxy-1,2-dihydroquinoline-3-carboxylic acid (2-morpholinoethyl)amide,
1-methyl-7-methoxy-2-oxo-8-pentyloxy-1,2-dihydroquinoline-3-carboxylic acid (4-pyridylmethyl)amide,
1-mothyl-7-methoxy-2-oxo-8-pentyloxy-1,2-dihydroquinoline-3-carboxylic acid (4-fluorobenzyl)amide,
1-methyl-7-methoxy-2-oxo-8-pentyloxy-1,2-dihydroquinoline-3-carboxylic acid [2-(4-hydroxyphenyl)ethyl]amide,
1-methyl-7-methoxy-2-oxo-8-pentyloxy-1,2-dihydroquinoline-3-carboxylic acid (3,4-methylenedioxybenzyl)amide,
1-methyl-7-methoxy-2-oxo-6-pentyloxy-1,2-dihydroquinoline-3-carboxylic acid [2-(4-fluorophenyl)ethyl]amide,
1-mathyl-7-methoxy-2-oxo-6-pentyloxy-1,2-dihydroquinoline-3-carboxylic acid (2-morpholinoethyl)amide,
1-methyl-7-methoxy-2-oxo-6-pentyloxy-1,2-dihydroquinoline-3-carboxylic acid (3,4-methylenedioxybenzyl)amide,
7,8-dipentyloxy-2-oxo-1,2-dihydroquinoline-3-carboxylic acid [2-(4-fluorophenyl)ethyl]amide,
8-hydroxy-7-methoxy-2-oxo-1,2-dihydroquinoline-3-carboxylic acid (3,4-methylenedioxybenzyl)amide,
7-methoxy-2-oxo-8-pentyloxy-1,2-dihydroquinoline-3-carboxylic acid (3,4-dihydroxybenzyl)amide,
7-methoxy-2-oxo-8-pentyloxy-1,2-dihydroquinoline-3-carboxylic acid (4-hydroxy-3-methoxybenzyl)amide,
1-O-{2-hydroxy-5-[(7-methoxy-2-oxo-8-pentyloxy-1,2-dihydro-3-quinolyl)carbonylamino-mathyl]phenyl}glucasidouronic acid,
1-O-{2-hydroxy-4-[(7-methoxy-2-oxo-8-pentyloxy-1,2-dihydro-3-quinolyl)carbonylaminomethyl]phenyl}glucosidouronic acid,
5-[7-methoxy-3-{(3,4-methylenedioxybenzyl)carbamoyl}-2-oxo-1,2-dihydro-8-quinolyloxy]pentanoic,
5-[7-methoxy-3-{(3-hydroxy-4-methoxybenzyl)carbamoyl}-2-oxo-1,2-dihydro-8-quinolyloxy]pentanoic acid,
8-(5-hydroxypentyloxy)-7-methoxy-2-oxo-1,2-dihydroquinoline-3-carboxylic acid (3,4-methylenedioxybenzyl)amide,
8-(5-hydroxypentyloxy)-7-methoxy-2-oxo-1,2-dihydroquinoline-3-carboxylic acid (4-hydroxy-3-methoxybenzyl)amide,
8-(4-hydroxypentyloxy)-7-methoxy-2-oxo-1,2-dihydroquinoline-3-carboxylic acid (3,4-methylenedioxybenzyl)amide,
7-methoxy-2-oxo-8-(4-oxopentyloxy)-1,2-dihydroquinoline-3-carboxylic acid (3,4-methylenedioxybenzyl)amide,
8-(3-hydroxypentyloxy)-7-methoxy-2-oxo-1,2-dihydroquinoline-3-carboxylic acid (3,4-methylenedioxybenzyl)amide,
7-methoxy-2-oxo-8-(3-oxopentyloxy)-1,2-dihydroquinoline-3-carboxylic acid (3,4-methylenedioxybenzyl)amide,
8-(2-hydroxypentyloxy)-7-methoxy-2-oxo-1,2-dihydroquinoline-3-carboxylic acid (3,4-methylenedioxybenzyl)amide,
7,8-dihydroxy-2-oxo-1,2-dihydroquinaline-3-carhoxylic acid [2-(4-fluorophenyl)ethyl]amide,
8-butoxy-3-hydroxymethyl-7-methoxy-2-oxo-1,2-dihydroquinoline,
8-ethoxy-3-hydroxymethyl-7-methoxy-2-oxo-1,2-dihydroquinoline,
N-(4-fluorophenyl)carbamic acid (8-butoxy-7-methoxy-2-oxo-1,2-dihydroquinoline-3-yl)methyl ester,
N-pyridine-4-ylcarbamic acid (8-ethoxy-7-methoxy-2-oxo-1,2-dihydroquinoline-3-yl)methyl ester,
3-dimethylaminomethyl-8-ethoxy-7-methoxy-2-oxo-1,2-dihydroquinoline,
8-butoxy-3-aminomethyl-7-methoxy-2-oxo-1,2-dihydroquinoline,
8-ethoxy-7-methoxy-3-morpholinomethyl-2-oxo-1,2-dihydroquinoline,
N-[(8-butoxy-7-methoxy-2-oxo-1,2-dihydroquinoline-3-yl)methyl]-N'-(4-fluorophenyl)urea,
N-[(8-butoxy-7-methoxy-2-oxo-1,2-dihpdraquinoline-3-yl)methyl]-(4-hydroxyphenyl)acetamide,
7-methoxy-2-oxo-8-pentyloxy-1,2-dihydroquinoline-3-carboxylic acid methyl ester,
7-methoxy-2-oxo-6-pentyloxy-1,2-dihydroquinoline-3-carboxylic acid methyl ester,
1-methyl-7-methoxy-2-oxo-8-pentyloxy-1,2-dihydroquinoline-3-carboxylic acid methyl ester,
1-methyl-7-methoxy-2-oxo-6-pentyloxy-1,2-dibydroquinoline-3-carboxylic acid methyl ester,
7-methoxy-2-oxo-8-pentyloxy-1,2-dihydroquinoline-3-carboxylic acid,
8-butoxy-7-methoxy-2-oxo-1,2-dihydroquinoline-3-carboxylic acid,
8-ethoxy-7-methoxy-2-oxo-1,2-dihydroquinoline-3-carboxylic acid,
7-methoxy-2-oxo-8-propoxy-1,2-dihydroquinoline-3-carboxylic acid,
7-methoxy-2-oxo-6-pentyloxy-1,2-dihydroquinoline-3-carbaxylic acid,
1-methyl-7-methoxy-2-oxo-8-pentyloxy-1,2-dihydroquinoline-3-carboxylic acid,
1-methyl-7-methoxy-2-oxo-6-pentyloxy-1,2-dihydroquinoline-3-carboxylic acid,
7-methoxy-2-oxo-8-pentyloxy-1,2-dihydroquinoline-3-carboxamide.

The compounds described in above (1) to (11) are the compounds having an affinity to the cannabinoid receptor, many of them have an agonistic activity to the cannabinoid receptor, especially a potent agonistic activity to the cannabinoid type 2 receptor.

Furthermore, some of them have an agonistic activity to the cannabinoid type 1 receptor. Such compounds having an agonistic activity to the cannabinoid both of type 1 and 2 receptors can exhibit a potent antipruritic activity.

The compounds having an agonistic activity to the cannabinoid receptor can be selected by the method described in Experimental Example 2, that is, can be selected the compounds inhibiting production reaction of cAMP caused by forskolin stimulation through the cannabinoid receptor.

The compounds described in above (12) to (17) are the compounds having an agonistic activity to the cannabinoid type 1 receptor and their structures are disclosed in Current Medical Chemistry, 1999, V0l.6, No.8, p636.

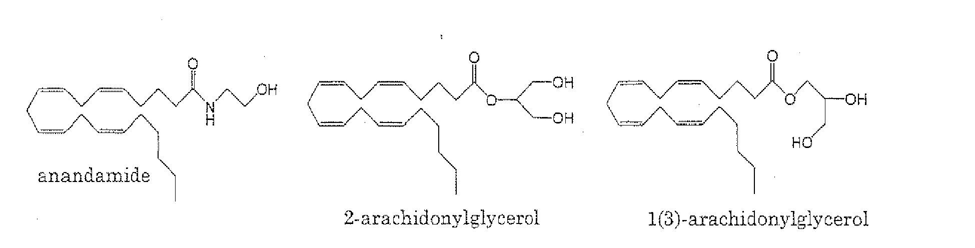

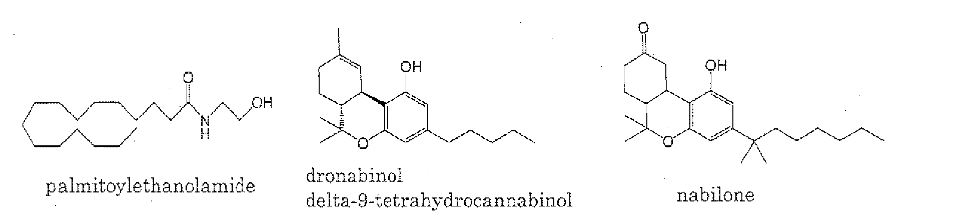

The compounds described in any one of above (18) to (20) are the compounds having an agonistic activity to the cannabinoid receptor, which can be used as antipruritics of the present invention. Examples include the following compounds.

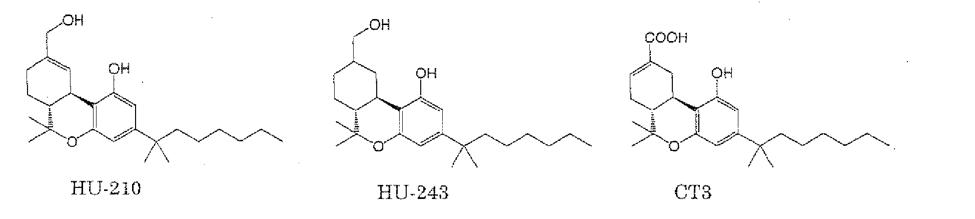

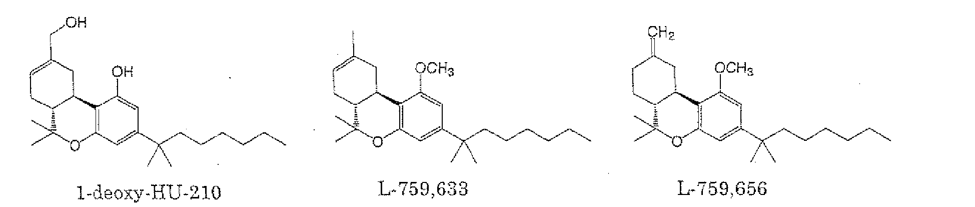

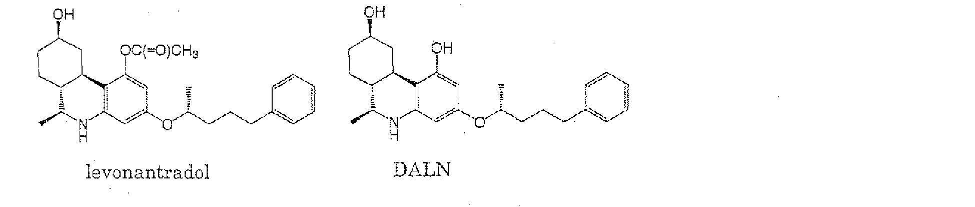

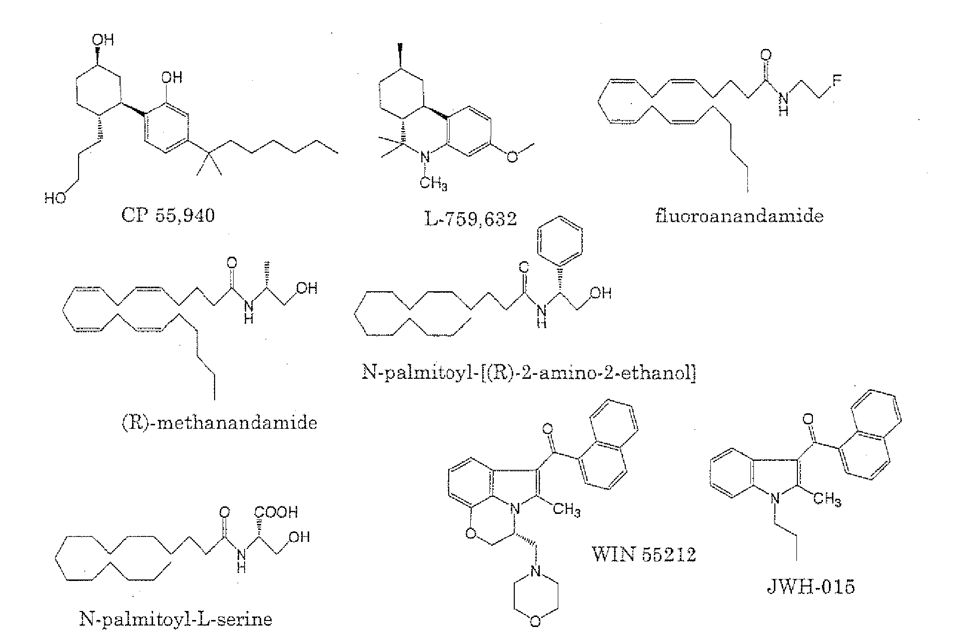

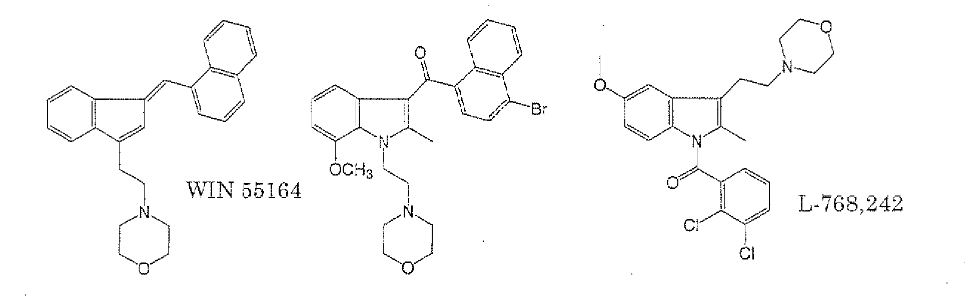

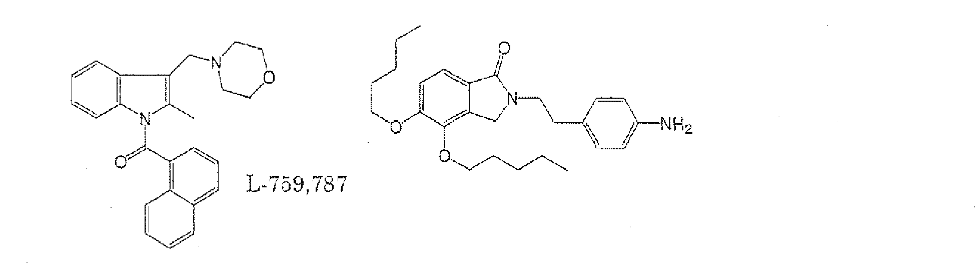

The compounds described in any one of (21) to (25) are the compounds having an agonistic activity to the cannabinoid receptor, which can be used as antipruritics of the present invention.

The meanings of each term used in the present specification are explained below. Each term employed alone or in combination with other terms expresses the same meaning.

The term "alkylene" includes a C1-C10 straight or branched alkylene, for example, methylene, ethylene, 1-methylethylene, 1-ethylethylene, 1,1-dimethylethylene, 1,2-dimethylethylene, 1,1-diethylethylene, 1,2-diethylethylene, 1-ethyl-2-methylethylene, trimethylene, 1-methyltrimethylene, 2-methyltrimethylene, 1,1-dimethyltrimethylene, 1,2-dimethyltrimethylene, 2,2-dimethyltrimethylene, 1-ethyltrimethylene, 2-ethyltrimethylene, 1,1-diethyltrimethylene, 1,2-diethyltrimethylene, 2,2-diethyltrimethylene, 2-ethyl-2-methyltrimethylene, tetramethylene, 1-methyltetramethylene, 2-methyltetramethylene, 1,1-dimethyltetramethylene, 1,2-dimethyltetramethylene, 2,2-dimethyltetramethylene, 2,2-di-n-propyltrimethylene or the like.

Preferred as alkylene of R¹ is a C2-C9 straight or branched alkylene, more preferred is a C2-C9 branched alkylene. For example, 2,2-dimethyltrimethylene, 2,2-diethyltrimethylene, 1-methyltrimethylene, 2-methyltrimethylene, trimethylene, 2,2-di-n-propyltrimethylene, 2,2-di-isopropyltrimethylene, 1,1-dimethylethylene or 1-methylethylene is preferred. The position number of these substituents is based on either the order of N-R¹-S or that of S-R¹-N.

Preferred as alkylene of Y¹, Y³, Y⁵, Y⁶ and E is C1-C4 alkylene.

Examples of substituents of "optionally substituted alkylene" include alkylene (e.g., methylene, ethylene, trimethylene, tetramethylene, pentamethylene or the like), cycloalkyl (e.g., cyclopropyl, cyclobutyl, cyclopentyl, cyclohexyl or the like), alkoxy (e.g., methoxy, ethoxy or the like), alkylthio (e.g., methylthio, ethylthio or the like), alkylamino (e.g., methylamino, ethylamino, dimethylamino or the like), acylamino (e.g., acetylamino or the like), aryl (e.g., phenyl or the like), aryloxy(e.g., phenoxy or the like), halogen (fluoro, chloro, bromo, iodo), hydroxy, amino, nitro, alkylsulfonyl (e.g., methanesulfonyl, ethanesulfonyl or the like), arylsulfonyl (e.g., benzenesulfonyl or the like), cyano, hydroxyamino, carboxy, alkoxycarbonyl (e.g., methoxycarbonyl, ethoxycarbonyl or the like), acyl (e.g., acetyl, benzoyl or the like), aralkyl (e.g., benzyl or the like), mercapto, hydorazino, amidino, guanidino or the like. One to four of these substituents may substitute at any position. Preferred as the substituent of "optionally substituted alkylene" of R¹ is alkylene.

Alkylene substituted with alkylene include alkylene substituted via a spiro atom with alkylene (e.g., 2,2-ethylenetrimethylene, 2,2-trimethylenetrimethylene, 2,2-tetramethylenetrimethylene, 2,2-pentamethylenetrimethylene or the like) and alkylene substituted at different positions with alkylene (e.g., 1,2-tetramethyleneethylene, 1,2-ethylenetrimethylene or the like). Preferred examples include 2,2-ethylenetrimethylene, 2,2-trimethylenetrimethylene, 2,2-tetramethylenetrimethylene, 2,2-pentamethylenetrimethylene, especially, 2,2-ethylenetrimethylene, 2,2-tetramethylenetrimethylene and 2,2-pentamethylenetrimethylene.

The term "alkyl" includes a C1-C10 straight or branched alkyl, for example, methyl, ethyl, n-propyl, i-propyl, n-butyl, i-butyl, sec-butyl, tert-butyl, n-pentyl, i-pentyl, neo-pentyl, n-hexyl, n-heptyl, n-octyl, n-nonyl, n-decyl or the like. Preferred is a C1-C4 straight or branched alkyl, for example, methyl, ethyl, n-propyl, i-propyl, n-butyl, i-butyl, sec-butyl and tert-butyl.

The term "alkoxy" includes an oxygen atom substituted with the above "alkyl", for example, methoxy, ethoxy, n-propoxy, i-propoxy, n-butoxy, i-butoxy, sec-butoxy, tert-butoxy, n-pentyloxy, n-hexyloxy, n-heptyloxy, n-octyloxy or the like. Preferred is a C1-C4 straight or branched alkoxy, for example, methoxy, ethoxy, n-propoxy, i-propoxy, n-butoxy, i-butoxy, sec-butoxy and tert-butoxy.

The term "alkylthio" includes a sulfur atom substituted with the above "alkyl", for example, methylthio, ethylthio, n-propylthio, i-propylthio, n-butylthio, i-butylthio, sec-butylthio, tert-butylthio, n-pentylthio, n-hexylthio or the like. Preferred is a C1-C4 straight or branched alkylthio, for example, methylthio, ethylthio, n-propylthio, i-propylthio, n-butylthio, i-butylthio, sec-butylthio and tert-butylthio.

Examples of "optionally substituted amino" include non-substituted amino, alkyl amino (e.g., methylamino, ethylamino, n-propylamino, i-propylamino, dimethylamino, diethylamino, ethylmethylamino, propylmethylamino or the like), acylamino (e.g., acetylamino, formylamino, propionylamino, benzoylamino or the like), acylalkylamino (e.g., benzylamino, 1-phenylethylamino, 2-phenylethylamino, 1-phenylpropylamino, 2-phenylpropylamino, 3-phenylpropylamino, 1-naphtylmethylamino, 2-naphtylmethylamino, dibenzylamino or the like), alkylsulfonylamino (e.g., methanesulfonylamino, ethanesulfonylamino or the like), alkenyloxycarbonylamino (e.g., vinyloxycarbonylamino, allyloxycarbonylamino or the like), alkoxycarbonylamino (e.g., methoxycarbonylamino, ethoxycarbonylamino, tert-butoxycarbonylamino benzoylamino or the like), alkenylamino (e.g., vinylamino, allulamino or the like), arylcarbonylamino (e.g., benzoylamino or the like), heteroarylcarbonylamino (e.g., pyridinecarbonylamino or the like) or the like.

The term "aryl" includes a C6-C14 aromatic carbocyclic group, for example, phenyl, naphthyl, anthryl, phenanthryl or the like.

The term "aralkyl" includes the above "alkyl" substituted with the above "aryl", for example, benzyl, phenylethyl (e.g., 1-phenylethyl, 2-phenylethyl), phenylpropyl (e.g., 1-phenylpropyl, 2-phenylpropyl, 3-phenylpropyl or the like), naphthylmethyl (e.g., 1-naphthylmethyl, 2-naphthylmethyl or the like) or the like.

The term "aralkyloxy" includes an oxygen atom substituted with the above "aralkyl", for example, benzyloxy, phenylethyloxy (e.g., 1-phenylethyloxy, 2-phenylethyloxy), phenylpropoxy (e.g., 1-phenylpropyloxy, 2-phenylpropyloxy, 3-phenylpropyloxy or the like), naphthylmethoxy (e.g., 1-naphthylmethoxy, 2-naphthylmethoxy or the like) or the like.

The term "aralkylthio" includes a sulfur atom substituted with the above "aralkyl", for example, benzylthio, phenylethylthio (e.g., 1-phenylethylthio, 2-phenylethylthio), phenylpropylthio (e.g., 1-phenylpropylthio, 2-phenylpropylthio, 3-phenylpropylthio or the like), naphthylmethylthio (e.g., 1-naphthylmethylthio, 2-naphthylmethylthio or the like) or the like.

The term "aralkylamino" includes a nitrogen atom substituted with one or two of the above "aralkyl", for example, benzylamino, phenylethylamino (e.g., 1-phenylethylamino, 2-phenylethylamino), phenylpropylamino (e.g., 1-phenylpropylamino, 2-phenylpropylamino, 3-phenylpropylamino), naphthylmethylamino (e.g., 1-naphthylmethylamino, 2-naphthylmethylamino or the like), dibenzylamino or the like.

The term "alkoxyalkyl" includes the above "alkyl" substituted with the above "alkoxy", for example, methoxymethyl, ethoxymethyl, n-propoxymethyl, 1-methoxyethyl, 2-methoxyethyl, 1-ethoxyethyl, 2-ethoxyethyl, 1-n-propoxyethyl, 2-n-propoxyethyl, 1-methoxy-n-propyl, 2-methoxy-n-propyl, 3-methoxy-n-propyl, 1-ethoxy-n-propyl, 2-ethoxy-n-propyl, 3-ethoxy-n-propyl, 1-n-propoxy-n-propyl, 2-n-propoxy-n-propyl, 3-n-propoxy-n-propyl or the like.

The term "alkylthioalkyl" includes the above "alkyl" substituted with the above "alkylthio", for example, methylthiomethyl, ethylthiomethyl, n-propylthiomethyl, 1-methylthioethyl, 2-methylthioethyl, 1-ethylthioethyl, 2-ethylthioethyl, 1-n-propylthioethyl, 2-n-propylthioethyl, 3-n-propylthioethyl, 1-methylthio-n-propyl, 2-methylthio-n-propyl, 3-methylthio-n-propyl, 1-ethylthio-n-propyl, 2-ethylthio-n-propyl, 3-ethylthio-n-propyl, 1-n-propylthio-n-propyl, 2-n-propylthio-n-propyl, 3-n-propylthio-n-propyl or the like.

The term "optionally substituted aminoalkyl" includes the above "alkyl" substituted with the above "optionally substituted amino", for example, N-methylaminomethyl, N-acetylaminomethyl, N,N-dimethylaminomethyl or the like.

The term "heteroaryl" includes a C1-C9 heteroaryl having one to four nitrogen atom(s), oxygen atom(s) and/or sulfur atom(s), for example, furyl (e.g., 2-furyl, 3-furyl), thienyl (e.g., 2-thienyl, 3-thienyl), pyrrolyl (e.g., 1-pyrrolyl, 2-pyrrolyl, 3-pyrrolyl), imidazolyl (e.g., 1-imidazolyl, 2-imidazolyl, 4-imidazolyl), pyrazolyl (e.g., 1- pyrazolyl, 3-pyrazolyl, 4-pyrazolyl), triazolyl (e.g., 1,2,4-triazol-1-yl, 1,2,4-triazol-3-yl, 1,2,4-triazol-4-yl) tetrazolyl (e.g., 1-tetrazolyl, 2-tetrazolyl, 5-tetrazolyl), oxazolyl (e.g., 2-oxazolyl, 4-oxazolyl, 5-oxazolyl), isoxazolyl (e.g., 3-isoxazolyl, 4-isoxazolyl, 5-isoxazolyl)" thiazolyl (e.g., 2-thiazolyl, 4-thiazolyl, 5-thiazolyl), thiadiazolyl, isothiazolyl (e.g., 3-isothiazolyl, 4-isothiazolyl, 5-isothiazolyl), pyridyl (e.g., 2-pyridyl, 3-pyridyl, 4-pyridyl), pyridazinyl (e.g., 3-pyridazinyl, 4-pyridazinyl) pyrimidinyl (e.g., 2-pyrimidinyl, 4-pyrimidinyl, 5-pyrimidinyl), furazanyl (e.g., 3-furazanyl), pyrazinyl (e.g., 2-pyrazinyl), oxadiazolyl (e.g., 1,3,4-oxadiazol-2-yl), benzofuryl (e.g., 2-benzo[b]furyl, 3-benzo[b]furyl, 4-benzo[b]furyl, 5-benzo[b]furyl, 6-benzo[b]furyl, 7-benzo[b]furyl), benzothienyl (e.g., 2-benzo[b]thienyl, 3-henzo[b]thienyl, 4-benzo[b]thienyl, 5-benzo[b]thienyl, 6-benzo[b]thienyl, 7-benzo[b]thienyl), benzimidazolyl (e.g., 1-benzimidazolyl, 2-benzimidazolyl, 4-benzimidazolyl, 5-benzimidazolyl), dibenzofuryl, benzoxazolyl, quinoxalyl (e.g., 2-quinoxalyl, 5-quinoxalyl, 6-quinoxalyl), cinnolyl (e.g., 3-cinnolyl, 4-cinnolyl, 5-cinnolyl, 6-cinnolyl, 7-cinnolyl, 8-cinnolyl), quinazolyl (e.g., 2-quinazolyl, 4-quinazolyl, 5-quinazolyl, 6-quinazolyl, 7-quinazolyl, 8-quinazolyl), quinolyl (e.g., 2-quinolyl, 3-quinolyl, 4-quinolyl, 5-quinolyl, 6-quinolyl, 7-quinolyl, 8-quinolyl), phthalazinyl (e.g., 1-phthalazinyl, 5-phthalazinyl, 6-phthalazinyl), isoquinolyl (e.g., 1-isoquinolyl, 3-isoquinolyl, 4-isoquinolyl, 5-isoquinolyl, 6-isoquinolyl, 7-isoquinolyl, 8-isoquinolyl), puryl, pteridinyl (e.g., 2-pteridinyl, 4-pteridinyl, 6-pteridinyl, 7-pteridinyl), carbazolyl, phenanthridinyl, acridinyl (e.g., 1-acridinyl, 2-acridinyl, 3-acridinyl, 4-acridinyl, 9-acridinyl), indolyl (e.g., 1-indolyl, 2-indolyl, 3-indolyl, 4-indolyl, 5-indolyl, 6-indolyl, 7-indolyl), isoindolyl, phenazinyl (e.g., 1-phenazinyl, 2-phenazinyl), phenothiadinyl (e.g., 1-phenothiadinyl, 2-phenothiadinyl, 3-phenothiadinyl, 4-phenothiadinyl) or the like.

Preferred as heteroaryl of R⁵ is 2-thionyl.

Preferred as heteroaryl of A is pyridyl, quinolyl, isoquinolyl.

The term "heterocyclic group" includes a C1-C14 monocyclic group or polycyclic group of fused 2 or 3 rings having one to four nitrogen atom(s), oxygen atom(s) and/or sulfur atom(s), for example, above "heterocyclic group" and below "non-aromatic heterocyclic group".

The term "non-aromatic heterocyclic group" includes a C1-C9 non-aromatic ring having one to four nitrogen atom(s), oxygen atom(s) and/or sulfur atom(s), for example, 1-pyrrolinyl, 2-pyrrolinyl, 3-pyrrolinyl, pyrrolidino, 2-pyrrolidinyl, 3-pyrrolidinyl, 1-imidazolinyl, 2-imidazolinyl, 4-imidazolinyl, 1-imidazolidinyl, 2-imidazolidinyl, 4-imidazolidinyl, 1-pyrazolinyl, 3-pyrazolinyl, 4-pyrazolinyl, 1-pyrazolidinyl, 3-pyrazolidinyl, 4-pyrazolidinyl, piperidino, 2-piperidyl, 3-piperidyl, 4-piperidyl, piperazino, 2-piperazinyl, 2-morpholinyl, 3-morpholinyl, morpholino, tetrahydropyranyl or the like. Preferred is morpholino, pyrrolidino, piperidino or piperazino.

The term "alkenyl" includes a C2-C8 straight or branched alkenyl which is the above "alkyl" having one or more double bond, for example, vinyl, 1-propenyl, allyl, isopropenyl, 1-butenyl, 2-butenyl, 3-butenyl, 2-pentenyl, 1,3-butadienyl, 3-methyl-2-butenyl or the like.

The term "alkynyl" includes a C2-C8 straight or branched alkynyl which is the above "alkyl" having one or more triple bond, for example, etynyl or the like.

The term "cycloalkyl" includes C3-C10 saturated carbocyclic group, for example, cyclopropyl, cyclobutyl, cyclopentyl, cyclohexyl, cycloheptyl, cyclooctyl or the like. Preferred is C3-C6 cycloalkyl, for example, cyclopropyl, cyclobutyl, cyclopentyl, cyclohexyl.

The term "alkylene which may contain heteroatom" includes a C2-C10 straight or branched alkylene which may contain one to three heteroatom(s), for example, ethylene, trimethylene, tetramethylene, pentamethylene, methylenedioxy, ethylenedioxy, ethyleneoxyethylene or the like. Especially preferred is C3-C5 straight alkylene which may contain one heteroatom, for example, tetramethylene, pentamethylene, ethyleneoxyethylene, ethyleneaminoethylene, ethylenethioethylene.

The term "halogen" includes fluoro, chloro, bromo and iodo. Preferred is fluoro, chloro or bromo.

The term "acyl" includes a carbonyl group substituted with a group except hydrogen, for example, alkylcarbonyl (e.g., acetyl, propionyl, butyryl, isobtyryl, valeryl, isovaleryl, pivaloyl, hexanoyl, octanoyl, lauroyl or the like), alkenylcarbonyl (e.g., acryloyl, methacryloyl), cycloalkylcarbonyl (e.g., cyclopropanecarbonyl, cyclobutanecarbonyl, cyclopentanecarbonyl, cyclohexanecarbonyl or the like), arylcarbonyl (e.g., benzoyl, naphthoyl or the like), heteroarylcarbonyl (e.g., pyridinecarbonyl or the like). These group may be optionally substituted with alkyl, halogen or and like. For example, as arylcarbonyl substituted with alkyl is toluoyl, and as alkylcarbonyl substituted with halogen is trifluoroacetyl or the like.

The term "alkoxycarbonyl" includes carbonyl substituted with the above "alkoxy", for example, methoxycarbonyl, ethoxycarbonyl, n-propoxycarbonyl, i-propoxycarbonyl, n-butoxycarbonyl, i-butoxycarbonyl, sec-butoxycarbonyl, tert-butoxycarbonyl, n-pentyloxycarbonyl, n-hexyloxycarbonyl, n-heptyloxycarbonyl, n-octyloxycarbonyl or the like. Especially, preferred is methoxycarbonyl, ethoxycarbonyl or the like.

Examples of the substituents of "optionally substituted carbamoyl" include alkyl (e.g., methyl, ethyl, n-propyl, i-propyl or the like), acyl (e.g., formyl, acetyl, propionyl, benzoyl or the like) or the like. The nitrogen atom of carbamoyl group may be mono- or di-substituted with these substituents. As "optionally substituted carbamoyl" preferred are carbamoyl, N-methylcarbamoyl, N-ethylcarbamoyl or the like.

The term "alkenyloxy" includes an oxygen atom substituted with the above "alkenyl", for example, vinyloxy, 1-propenyloxy, 2-propenyloxy, 1-butenyloxy, 2-butenyloxy, 3-butenyloxy, 1,3-butadienyloxy, 3-methyl-2-butenyloxy or the like.

The term "alkynyloxy" includes an oxygen atom substituted with the above "alkynyl", for example, ethynyloxy, 1-propynyloxy, 2-propynyloxy, 1-butynyloxy, 2-butynyloxy, 3-butynyloxy or the like.

The term "alkylsulfinyl" includes sulfinyl substituted with the above "alkyl". Especially, preferred is methanesulfinyl, ethanesulfinyl or the like.

The term "alkylsulfonyl" includes sulfonyl substituted with the above "alkyl". Especially, preferred is methanesulfonyl, ethanesulfonyl or the like.

The term "carbocyclic group" includes a cyclic substituent consisting of carbon atom and hydrogen atom, and the cyclic part may be saturated cycle or unsaturated cycle,-for example, the above "aryl", the above "cycloalkyl", the below "cycloalkenyl" or the like. Preferred is C3-C14 cyclic group.

The term "cycloalkenyl" includes C3-C12 alkenyl which is the above "cycloalkyl" having one or more double bond, for example, cyclopropenyl (e.g., 1-cyclopropenyl), cyclobutenyl (e.g., 1-cyclobutenyl), cyclopentenyl (e.g., 1-cyclopenten-1-yl, 2-cyclopenten-1-yl, and 3-cyclopenten-1-yl), cyclohexenyl (e.g., 1-cyclohexen-1-yl, 2-cyclohexen-1-yl, and 3-cyclohexen-1-yl), cycloheptenyl (e.g., 1-cycloheptenyl), cyclooctenyl (e.g., 1-cyclooetenyl) or the like. Especially, preferred is 1-cyclohexen-1-yl, 2-cyclahexen-1-yl, or 3-cyelohexen-1-yl

The term "alkenylene" includes C2-C12 straight or branched alkenylene which is the above "alkylene" having one or more double bond(s), for example, vinylene, propenylene, or butenylene. Preferred is C2-C6 straight alkenylene, for example, vinylene, propenylene, butenylene, pentenylene, hexenylene, butadienylene or the like.

The term "alkynylene" includes C2-C12 straight or branched alkynylene which is the above "alkylene" having one or more triple bond(s).

When "optionally substituted alkylene", "optionally substituted alkyl,", "optionally substituted alkenyl", "optionally substituted alkynyl", optionally substituted carbocyclic group", "optionally substituted heterocyclic group", "optionally substituted alkyl which may have heteroatom and/or unsaturated bond", or "optionally substituted cyclic group which may have heteroatom and/or unsaturated bond" have substituent, these may be substituted with same or different one to four substituent(s) at any position.

Examples of these substituents include hydroxy, carboxy, halogen (fluoro, chloro, bromo, iodo), halogenated alkyl (e.g., CF₃, CH₂CF₃, CH₂CCl₃ or the like), alkyl (e.g., methyl, ethyl, isopropyl, tert-butyl or the like), alkenyl (e.g., vinyl), formyl, acyl (e.g., asectyl, propionyl, butyryl, pivaloyl, benzoyl, piridinecarbonyl, cyclopentanecarbonyl, cyclohexanecarbonyl or the like), alkynyl (e.g., ethynyl), cycloalkyl (e.g., cyclopropyl, cyclobutyl, cyclopentyl, cyclohexyl or the like), cycloalkenyl (e.g., cyclopropenyl or the like), alkoxy (e.g., methoxy, ethoxy, propoxy, butoxy or the like), alkoxycarbonyl (e.g., methoxycarbonyl, ethoxycarbonyl, tert-butoxycarbonyl or the like), nitro, nitroso, oxo, optionally substituted amino (e.g., amino, alkylamino (e.g., methylamino, ethylamino, dimethylamino or the like), formylamino, acylamino (e.g., acetylamino, benzoylamino or the like), aralkylamino (e.g., benzylamino, tritylamino), hydroxyamino, alkylsulfonylamino, alkenyloxycarbonylamino, alkoxycarbonylamino, alkenylamino, arylcarbonylamino, heteroarylcarbonylamino or the like), azide, aryl (e.g., phenyl or the like), aryloxy (e.g., phenoxy), aralkyl (e.g., benzyl, phenethyl, phenylpropyl or the like), alkylenedioxy (e.g., methylenedioxy), alkylene (e.g., methylene, ethylene, trimethylene, tetramethylene, pentamethylene or the like), alkenylene (e.g., propenylene, butenylene, butadienylen or the like), cyano, isocyano, isocyanato, thiocyanato, isothiocyanato, mercapto, alkylthio (e.g., methylthio, ethylthio or the like), alkylsulfonyl (e.g., methanesulfonyl, ethanesulfonyl), arylsulfonyl (e.g., benzenesulfonyl or the like), optionally substituted carbamoyl, sulfamoyl, formyloxy, haloformyl, oxalo, mercapto, thioformyl, thiocarboxy, dithiocarboxy, thiocarbamoyl, sulfino, sulfo, sulfoamino, hydrazido, ureido, amidino, guanidino, alkylsulfonyloxy, trialkylsilyloxy, halogenated alkoxycarbonyloxy, formyloxy, acylthio, thioxo, alkoxyalkoxy, alkylthioalkoxy or the like.

The term "haloalkyl" includes the above "alkyl" substituted with one or more halogen, for example, chloromethyl, dichloromethyl, difluoromethyl, trifluoromethyl, chloroethyl (e.g., 1-chloroethyl, 2-chloroethyl or the like), dichloroethyl (e.g., 1,1-dichloroethyl, 1,2-dichloroethyl, 2,2-dichloroethyl or the like) or the like.

The term "haloalkoxy," includes the above "alkoxy" substituted with one or more halogen, for example, dichloromethoxy, difluoromethoxy, trifluoroznethoxy, trifluoroethoxy (2,2,2-trifluoroethoxy or the like) or the like.

The term "aryloxy" includes an oxygen atom substituted with the above "aryl", for example, phenoxy, naphthoxy (e.g., 1-naphthoxy, 2-naphthoxy or the like), anthryloxy (e.g., 1-anthryloxy, 2-anthryloxy or the like), phenanthryl (e.g., 1-phenanthryl, 2-phenanthryl or the like) or the like.

The term "alkoxyalkoxy" includes the above "alkoxy" substituted with the above "alkoxy", for example, methoxymethoxy, ethoxymethoxy, n-propoxymethoxy, isopropoxymethoxy, 1-methoxyethoxy, 2-methoxyethoxy or the like.

The term "alkylthioalkoxy" includes the above "alkoxy" substituted with the above "alkylthio", for example, methylthiomethoxy, ethylthiomethoxy, n-propylthiomethoxy, isopropylthiomethoxy, 1-methylthioethoxy, 2-methoxyethoxy or the like.

As "optionally substituted carbocyclic group" of R^{a}, preferred is optionally substituted aryl (the substituent is carboxy, optionally substituted amino, alkoxy, alkylthio, alkylenedioxy, halogen, alkyl, hydroxy, halogenated alkyl and/or halogenated alkoxy), optionally substituted cycloalkyl (the substituent is aryl and/or hydroxy), or optionally substituted cycloalkyl (the substituent is alkenylene, hydroxy, alkylsulfonyloxy, azide, amino and/or acylamino).

As "optionally substituted heterocyclic group" of R^{a}, preferred is optionally substituted heteroaryl (the substituent is oxo, heteroaryl, halogen, aryl and/or alkyl) or optionally substituted heterocycle (the substituent is aryl optionally substituted with halogen; aralkyl, acyl, arylcarbonyl, cycloalkylcarbonyl, alkylsulfonyl, arylsulfonyl, alkyl and/or halogenated alkylcarbonyl).

Examples of preferable substituent of "cyclic group optionally containing heteroatom and/or unsaturated bond" include oxo, hydroxy, alkenylene (e.g., propenylene, butenylene, butadienylene), acyl (e.g., acetyl, propionyl, butyryl, pivaloyl, benzoyl, pyridinecarbonyl, cyclopentanecarbonyl, cyclohexanecarbonyl or the like), aralkyl (e.g., benzyl or the like), alkylene (e.g., methylene, ethylene, trimethylene, tetramethylene, pentamethylene or the like).

As a substituent of "alkyl which may have heteroatom and/or unsaturated bond" of R¹³, preferred is halogen, hydroxy, azide, amino, alkoxy, alkenyloxy, alkylsulfonyloxy, acylthio, acylamino, arylcarbonylamino, cycloalkylcarbonylamino, halogenated alkylcarbonylamino, alkylsulfonylamino, arylsulfonylamino, formyl, oxo or cyano.

In a compound of the formula (I) or (II), m is an integer of 0 to 2, preferred is 0.

As a compound of the formula (III), examples of "any one of combinations of R² and R³, R³ and R⁴, and R⁹ and R⁵, taken together with the adjacent atoms form optionally substituted cyclic group which may have heteroatom and/or unsaturated bond" include the following structure. 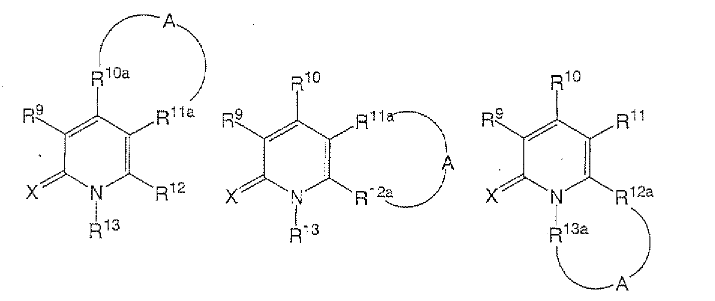 wherein each symbol is as defined above; R^{10a}-A-R^{11a}-, R^{11a}-A-R^{12a}, and R^{12a}-A-R^{13a}-each is independently optionally substituted alkylene which may have heteroatom(s) and/or unsaturated bond(s).

Especially, preferred is when an atom bonding to pyridone ring is carbon atom, that is, R^{10a}, R^{11a}, R^{12a}, or R^{13a} is carbon atom. Furthermore, this carbon atom may bond to the above substituent (e.g., alkyl, alkoxy, hydroxy, oxo, halogen, amino or the like).

The term "cyclic group" includes 4-12 membered ring, especially preferred is 5-10 membered ring, more preferred is 5-8 membered ring. The atoms structuring the ring include carbon atom, heteroatom (nitrogen atom, sulfur atom, oxygen atom), hydrogen atom or the like.

When R¹⁰ and R¹¹ taken together with the adjacent atoms form optionally substituted cyclic group which may have heteroatom and/or unsaturated bond, examples include the following compound.

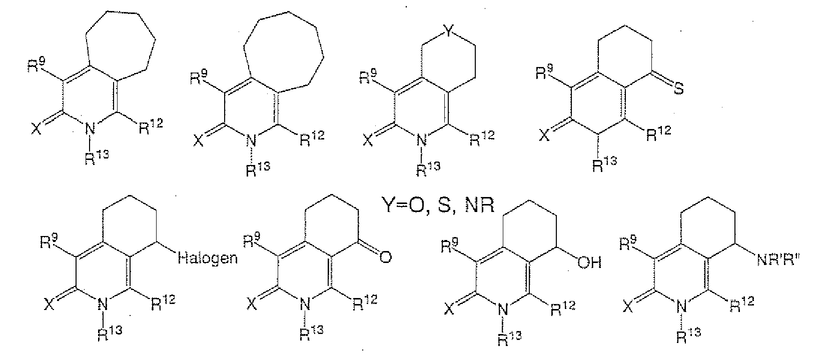

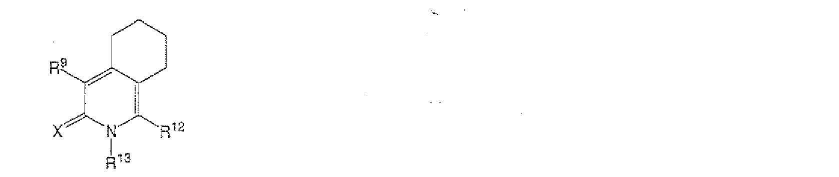

Especially, preferred are the following compounds. 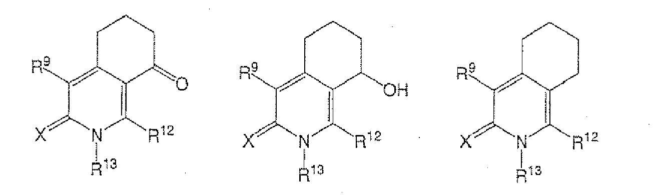 wherein each symbol is as defined above; Y is oxygen atom, sulfur atom or -NR-; R, R' and R" are hydrogen, alkyl., aralkyl, or the like.

When R¹¹ and R¹² taken together with the adjacent atoms form optionally substituted cyclic group which may have heteroatom (especially oxygen atom, nitrogen atom) and/or unsaturated bond (especially double bond), especially, preferred are the following compounds.
(1) the cyclic group is unsubstituted carbocyclic group,
(2) different positions of the cyclic group are substituted with alkenylene,
(3) the cyclic group contains oxygen atom or nitrogen atom,
(4) the cyclic group contains nitrogen atom, and the nitrogen atom is substituted with substituent (especially, alkyl, acyl, aralkyl or the like),
(5) the cyclic group is unsubstituted carbocyclic group, provided that the bond between carbon atom substituted with R¹¹ and carbon atom substituted with R¹² is a double bond, and the other bonds between carbon atoms are single bonds,
(6) the cyclic group is unsubstituted ring containing heteroatom, provided that the bond between carbon atom substituted with R¹¹ and carbon atom substituted with R¹² is the double bond, and the other bonds between carbon atoms are single bonds.

Examples include the following compounds.

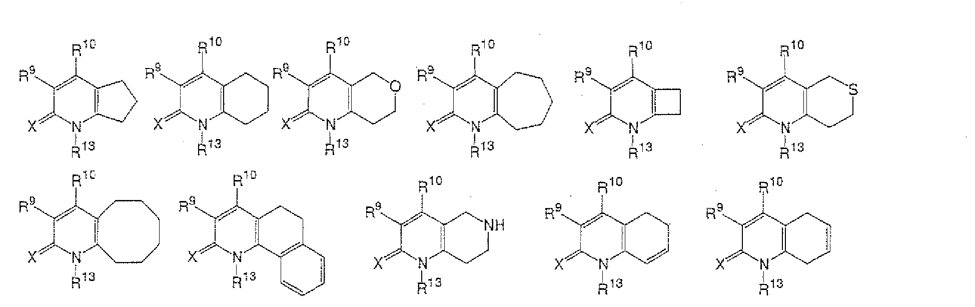

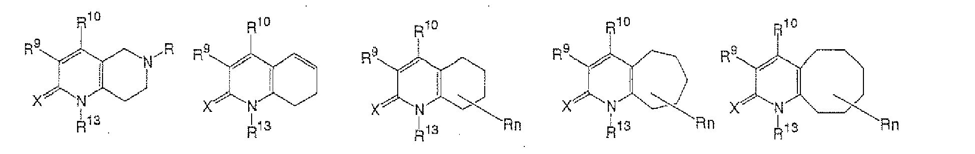

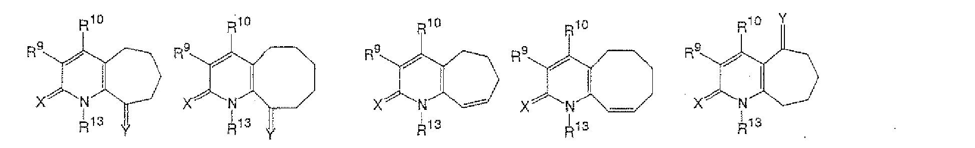

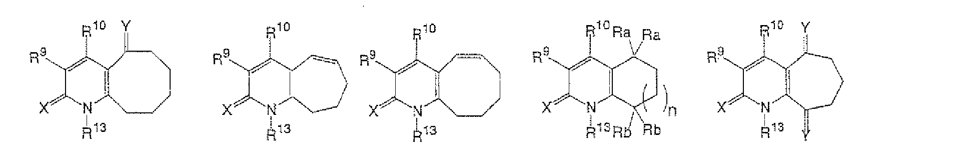

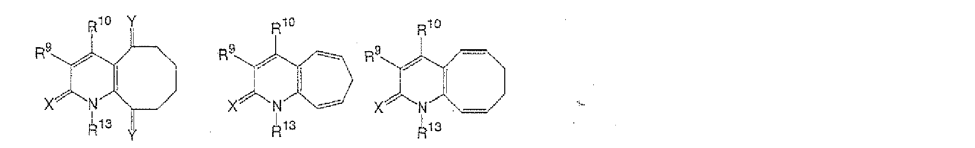

Especially, preferred is the following structures. 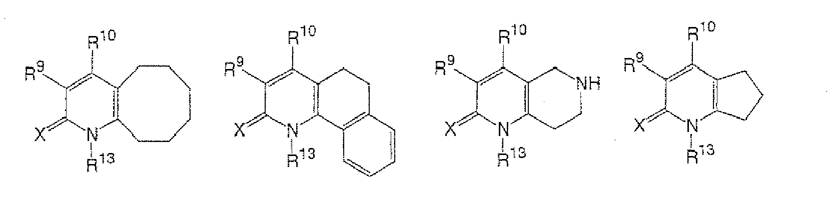 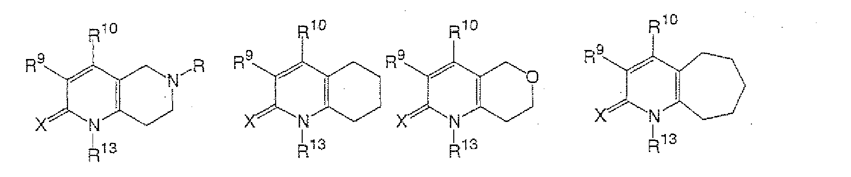 wherein each symbol is as defined above; Y is O or S; R, Ra and Rb are acyl, aralkyl, alkyl, alkoxy, oxo or the like; n is an integer of 0 to 5.

Furthermore, the present invention includes the case that R¹¹ and R¹² taken together with the adjacent atoms form cyclic group which has unsaturated bond. In this case, preferred is double bond as unsaturated bond, and preferred is cyclic group having a double bond between carbon atom substituted with R¹¹ and carbon atom substituted with R¹² and another double bond.

The case that R¹¹ and R¹² taken together with the adjacent atoms form benzene ring is included in the present invention, for example compounds described in WO 97/29079 and WO 99/02499.

When R¹² and R¹³ taken together with the adjacent atoms form optionally substituted cyclic group which may have heteroatom (especially oxygen atom, nitrogen atom) and/or unsaturated bond (especially double bond), especially, preferred is the following compound.
(1) The cyclic group is optionally substituted carbocyclic group which may have unsaturated bond (especially, double bond),
(2) the cyclic group is unsubstituted,
(3) different positions of the cyclic group are substituted with substituent (especially, alkenylene or the like). Examples include the following compound.

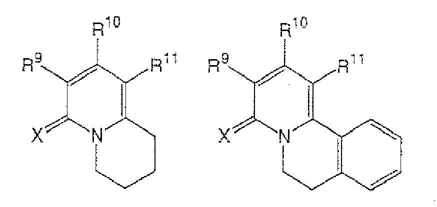

Furthermore, in combinations of R¹⁰ and R¹¹, R¹¹ and R¹², and R¹² and R¹³, preferred is combination of R¹¹ and R¹² forming optionally substituted cyclic group which may have heteroatom(s) and/or unsaturated bond(s)

In the present invention, not only the compound having the cannabinoid receptor agonist but also a prodrug, a pharmaceutically acceptable salt or solvate thereof can be used.

A prodrug is a derivative which is converted to a pharmaceutically active compound of the present invention under a physiological condition. Method for the selection and process of an appropriate prodrug derivative are described in the literature such as Design of Prodrugs, Elsevier, Amsterdam 1985.

A prodrug of the present invention can be prepared by introducing a leaving group to substituents on ring A which are substitutable (e.g., amino, hydroxy or the like). Examples of a prodrug derived form a compound having an amino group includes carbamate derivatives (e.g., methylcarbamate, cyclopropylmethylcarbamate, t-butylcarbamate, benzylcarbamate or the like), amide derivatives (e.g., formamide, acetamide or the like), N-alhyl derivative (e.g., N-allylamine, N-methoxymethylamine or the like) or the like. Examples of a prodrug derived form a compound having hydroxy group include ether derivatives (methoxymethylether, methoxyethoxymethylether or the like), ester derivatives (e.g., acetate, pivaloate, benzoate or the like) or the like.

Examples of a pharmaceutically acceptable salt include basic salts (e.g., alkali metal salts such as sodium or potassium salts; alkaline-earth metal salts such as calcium or magnesium salts; ammonium salts; aliphatic amine salts such as trimethylamine, triethylamine, dicyclohexylamine, ethanolamine, diethanolamine, triethanolamine or procaine salts; aralkyl amine salts such as N,N-dibenzylethylenediamine salts; heterocyclic aromatic amine salts such as pyridine salts, picoline salts, quinoline salts or isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts or tetrabutylammonium salts; and basic amino acid salts such as arginine salts or lysine salts). Acid addition salts include, for example, mineral acid salts such as hydrochlorides salts, sulfates salts, nitrate salts, phosphates salts, carbonates salts, hydrogen carbonates salts or perchlorates salts; organic acid salts such as acetates, propionates, lactates, maleates, fumarates, tartrates, malates, succinates, or ascorbates; sulfonates such as methanesulfonates, isethionates, benzenesulfonates, or p-toluenesulfonates; and acidic amino acid salts such as aspartates or glutamates.

A solvate includes a solvate of the compound of the present invention, a prodrug or a pharmaceutically acceptable salt thereof, for example, monosolvate, disolvate, monohydrate, dihydrate or the like.

Antipruritics of the present invention are used for prevention or suppression of pruritus. The antipruritics can inhibit pruritus caused by allergic or non-allergic reaction. Particularly, the antipruritics are useful as therapeutic agents for pruritus induced by antigen. Concretely, they can be used as prophylactic or therapeutic agents against pruritus derived from atopic dermatitis, urticaria, allergic conjunctivitis, allergic rhinitis and/or contact dermatitis.

In addition, antipruritics of the present invention are also effective as prophylactic or therapeutic agents against disorders that are caused collaterally by the behaviors accompanying with pruritus such as scratching and beating, for example, cataract, detachment of the retina, inflammation, infection and sleeplessness.

Allergic reaction means the response induced by activated mast cells and basophils and so on following the interaction of antigen specific IgE and antigen, and the responed induced by the delayed type allergic reaction such as contact dermatitis. Whereas non-allergic reaction means the IgE independent response induced by activated mast cells and basophils after chemical substance stimulation.

When using a compound of the present invention in treatment, it can be formulated into ordinary formulations for oral and parenteral administration. A pharmaceutical composition containing a compound of the present invention can be in the form for oral and parenteral administration. Specifically, it can be formulated into formulations for oral administration such as tablets, capsules, granules, powders, syrup, and the like; those for parenteral administration such as injectable solution or suspension for intravenous, intramuscular or subcutaneous injection, inhalant, eye drops, nasal drops, suppositories, or percutaneous formulations such as ointment.

When the compounds used as an active ingredient have a week agonistic activity to the cannabinoid type 1 receptor and a potent agonistic activity to the cannabinoid type 2 receptor, can be used as an active ingredient of all kinds of formulation. Especially, they can be used as formulations for oral administration such as tablets, capsules, granules, powders, syrup, and the like. When the compounds used as an active ingredient have a potent agonistic activity to the cannabinoid type 1 receptor, preferred is topical application, more preferred are formulations such as ointment, cream, lotion or the like.

In preparing the formulations, carriers, excipients, solvents and bases known to one ordinary skilled in the art may be used. Tablets are prepared by compressing or formulating an active ingredient together with auxiliary components. Examples of usable auxiliary components include pharmaceutically acceptable excipients such as binders (e.g., cornstarch), fillers (e.g., lactose, microcrystalline cellulose), disintegrates (e.g., starch sodium glycolate) or lubricants (e.g., magnesium stearate). Tablets may be coated appropriately. In the case of liquid formulations such as syrups, solutions or suspensions, they may contain suspending agents (e.g., methyl cellulose), emulsifiers (e.g., lecithin), preservatives and the like. In the case of injectable formulations, it may be in the form of solution or suspension, or oily or aqueous emulsion, which may contain suspension-stabilizing agent or dispensing agent, and the like. In the case of an inhalant, it is formulated into a liquid formulation applicable to an inhaler. In the case of eye drops, it is formulated into a solution or a suspension.

Although an appropriate dosage of the present compound varies depending on the administration route, age, body weight, sex, or conditions of the patient, and the kind of drug(s) used together, if any, and should be determined by the physician in the end, in the case of oral administration, the daily dosage can generally be between about 0.01 - 100 mg, preferably about 0.01 - 10 mg, more preferably about 0.01 - 1 mg, per kg . body weight. In the case of parenteral administration, the daily dosage can generally be betwee\n about 0.001 - 100 mg, preferably about 0.001 - 1 mg, more preferably about 0.001 - 0. 1 mg, per kg body weight. The daily dosage can be administered in 1 - 4 divisions.

The compounds described in above (1) or (2) can be prepared in accordance with the processes described in WO 01/119807. The compounds described in any one of above (3) to (5) can be prepared in accordance with the following processes. 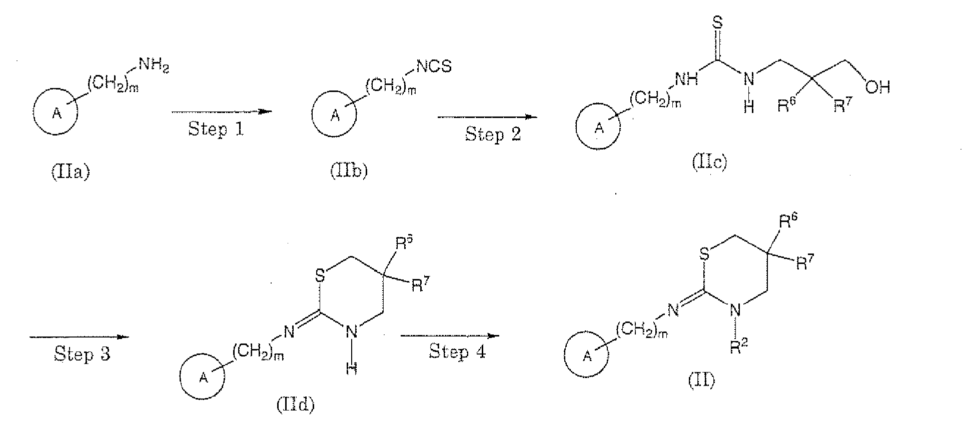 wherein each symbol is as defined above.

### Process 1

This is a process for producing a compound of the formula (IIb) which comprises converting amino group of a compound of the formula (IIa) to isothiocyanic acid ester (isothiocyanate).

A method for converting amino group to isothio cyanic acid ester (isothiocyanate) includes the following methods; (1) a method which comprises reacting the starting compound with carbon disulfide in the presence of a base such as ammonia (NH₃, NH₃OH), triethylamine (Et₃N) and reacting the obtained dithiocarbamate with ethyl chlorocarboxylate (ClCO₂Et) and triethylamine (Et₃N), (2) a method which comprises reacting the above dithiocarbamate with acid metalate such as lead nitrate or the like, (3) a method of reacting thiophosgene (CSCl₂) and (4) a method of reacting thiocarbonyldiimidazole or the like.

In the above (1), a base (1.0 to 1.5 mole equivalent) and carbon disulfide (1.0 to 1.5 mole equivalent) are added to a solution of a compound of the formula (IIa) in an aprotic solvent (e.g., diethylether, tetrahydrofuran, dimethylformamide, benzene, toluene, dichloromethane, chloroform or the like) and the mixture is stirred for 0.5 to 10 hours. After that, ethyl chlorocarboxylate (1.0 to 1.5 mole equivalent) and triethylamine (1.0 to 1.5 mole equivalent) are added thereto and the mixture is stirred in the same solvent for 0.5 to 10 hours. The reaction temperature is preferably 0 to 100 °C, especially 0 °C to room temperature.

In the above (3), thiophosgene (1.0 to 1.5 mole equivalent) is added to a solution of the compound of the formula (IIa) in an aprotic solvent (e.g., diethylether, tetrahydrofuran, dimethylformamide, benzene, toluene, dichloromethane, chloroform or the like) and stirred for 0.5 to 10 hours. The reaction temperature is preferably 0 to 100 °C, especially 0 °C to room temperature.

In the above (4), thiocarbonyldiimidazole (1.0 to 1.5 mole equivalent) is added to a solution of the compound of the formula (IIa) in an aprotic solvent (e.g., diethylether, tetrahydrofuran, dimethylformamide, benzene, toluene, dichloromethane, chloroform or the like) and stirred for 0.5 to 1.0 hours. The reaction temperature is preferably 0 to 100°C, especially 0 °C to room temperature.

Examples of the compound of the formula (IIa) wherein m is 0 include aniline, 2-methylaniline, 2-ethylaniline, 2-n-propylaniline, 2-1-propylaniline, 2-n-butylaniline, 2-sec-butylaniline, 2-t-butylaniline, 3-methylaniline, 3-i-propylaniline, 3-i-propyl-4-methylaniline, 3-t-butylaniline, 4-methylaniline, 4-i-propylaniline, 2,6-dimetbylaniline, 2,3-dimethylaniline, 2,4-dimethylaniline, 3,4-diethylaniline, 2,5-dimethylaniline, 3,4-dimethylaniline, 3,5-dimethylaniline, 2,6-diethylaniline, 2,6-di-i-propylaniline, 2-methoxyaniline, 2-ethoxyaniline, 2-i-propoxyaniline, 3-methoxyaniline, 3,5-dimethoxyaniline, 3-n-butoxyaniline, 4-n-butoxyaniline, 4-ethoxyaniline, 3,4-dimethoxyaniline, 2-methylthloaniline, 2-ethylthioaniline, 2-i-propylthioaniline, 2-N,N-dimethylaminoaniline, 2-phenylaniline, 3-phenylaniline, 4-phenoxyaniline, 2-cyclohexylaniline, 2-cyclopentylaniline, 2-nitroaniline, 2,4-dinitroaniline, 2-fluoroaniline, 2-chloroaniline, 4-chloroaniline, 2,3-dichloroaniline, 3,4-dichloroaniline, 2-i-propyl-4-nitroaniline, 2-i-propyl-6-nitroaniline, 2-hydroxyaniline, 2-N,N-dimethylaminocarbonylaniline, 2-N-acetylaniline, 2-(1-ethylpropyl)aniline, 2-i-propyl4-methylaniline, 2-i-propyl-4-hydroxyaniline, 2-i-propyl-4-chloroaniline, 2-i-propyl-4-aminoaniline, 2-1-propyl-5-methylaniline, 2-i-propyl-5-hydroxy aniline, 2-1-propyl-5-chloroaniline, 4-chloro-3-methylaniline, 3,4-methylenedioxyaniline or the like.

Examples of the compound of the formula (IIa) wherein m is 1 include benzylamine, 2-methylbenzylamine, 2-ethylbenzylamine, 2-n-propylbenzylamine, 2-i-propylbenzylamine, 2-n-butylbenzylamine, 2-sec-butylbenzylamine, 2-t-butylbenzylamine, 3-methylbenzylamine, 3-i-propylbenzylarnine, 3-1-propyl-4-methylbenzylamine, 3-t-butylbenzylamine, 4-methylbenzylamine, 4-1-propylbenzylamine, 2,6-dimethylbenzylamine, 2,3-dimethylbenzylamine, 2,4-dimethylbenzylamine, 3,4-dietbylbenzylamine, 2,5-dimethylbenzylamine, 3,4-dimethylbenzylamine, 3,5-dimethylbenzylamine, 2,6-diethylbenzylamine, 2,6-di-i-propylbenzylamine, 2-methoxybenzylamine, 2-ethoxybenzylamine, 2-i-propoxybenzylamine, 3-methoxybenzylarnine, 3,5-dimethoxybenzylamine, 3-n-butoxybenzylamine, 4-n-butoxybenzylamine, 4-ethoxybenzylamine, 3,4-dimethoxybenzylamine, 2-methylthiobenzylamine, 2-ethylthiobenzylamine, 2-i-propylthiobenzylamine, 2-N,N-diznethylaminobenzylamine, 2-phenylbenzylamine, 3-phenylbenzylamine, 4-phenoxybenzylamine, 2-cyclohexylbenzylamine, 2-cyclopentylbenzylamine, 2-nitrobenzylamine, 2,4-dinitrobenzylamine, 2-fluorobenzylamine, 2-chlorobenzylamine, 4-chlorobenzylarnine, 2,3-dichlorobenzylamine, 3,4-dichlorobenzylamine, 2-i-propyl-4-nitrobenzylamine, 2-i-propyl-6-nitrobenzylamine, 2-hydroxybenzylamine, 2-N,N-dimethylaminocarbonylbenzylamine, 2-N-acetylbenzylamine, 2-(1-ethylpropyl)benzylamine, 2-i-propyl4-methylbenzylamine, 2-i-propyl-4-hydroxybenzylamine, 2-i-propyl-4-chlorobenzylamine, 2-1-propyl-4-aminabenzylamine, 2-i-propyl-5-methylbenzylamine, 2-i-propyl-5-hydroxybenzylamine, 2-i-propyl-5-chlorobenzylamine, 4-chloro-3-methylbenzylamine, 3,4-methylenedioxybenzylamine or the like.

Examples of the compound of the formula (IIa) wherein m is 2 include phenethylamine, 2-methylphenethylamine, 2-ethylphenethylarmine, 2-n-propylphenethylamine, 2-i-propylphenethylamine, 2-n-butylphenethylamine, 2-sec-butylphenethylamine, 2-t-butylphenethylamine, 3-methylphenethylamine, 3-i-propylphenethylamine, 3-i-propyl-4-znethylphenethylamine, 3-t-butylphenethylamine, 4-methylphenethylamine, 4-i-propylphenethylamine, 2,6-dimethylphenethylamine, 2,3-dimethylphenethylamine, 2,4-dimethylphenethylamine, 3,4-dietbylphenethylamine, 2,5-dimethylphenethylamine, 3,4-dimethylphenethylamine, 3,5-dimethylphenethylamine, 2,6-diethylphenethylamine, 2,6-di-i-propylphenethylamine, 2-methoxyphenethylamine, 2-ethoxyphenethylamine, 2-i-propoxyphenethylamine, 3-methoxyphenethylamine, 3,5-dimethoxyphenethylamine, 3-n-butoxyphenethylamine, 4-n-butoxyphenethylamine, 4-ethoxyphenethylamine, 3,4-dimethoxyphenethylamine, 2-methylthiopbenethylamine, 2-ethylthiophenethylamine, 2-i-propylthiophenethylamine, 2-N,N-dimethylaminophenethylamine, 2-phenylphenethylamine, 3-phenylphenethylamine, 4-phenoxyphenethylamine, 2-cyclohexylphenethylamine, 2-cyclopentylphenethylamine, 2-nitrophenethylamine, 2,4-dinitrophenethylamine, 2-fluorophenethylamine, 2-chlorophenethylamine, 4-chlorophenethylamine, 2,3-dichlorophenethylamine, 3,4-dichlorophenethylamine, 2-i-propyl-4-nitrophenethylamine, 2-1-propyl-6-nitrophenethylamine, 2-hydroxyphenethylamine, 2-N,N-dimethylaminocarbonylphenethylamine, 2-N-acetylphenethylamine, 2-(1-ethylpropyl)phenethylamine, 2-i-propyl4-methylphenethylamine, 2-i-propyl-4-hydroxyphenethylamine, 2-i-propyl-4-chlorophenethylamine, 2-i-propyl-4-aminophenethylamine, 2-i-propyl-5-methylphenethylamine, 2-i-propyl-5-hydroxyphenethylamine, 2-i-propyl-5-chlorophenethylamine, 4-chloro-3-methylphenethylamine, 3,4-methylenedioxyphenethylamine or the like.

### Process 2

This is a process for producing a compound of the formula (IIc) which comprises reacting an isothiocyanate of the compound of the formula (IIb) with NH₂-CH₂C(R⁶)R⁷CH₂-OH.

This process can be carried out in an aprotic solvent (e.g., diethylether, tetrahydrofuran, dimethylformamide, benzene, toluene, dichloromethane, chloroform or the like).

The reaction temperature is preferably 0 to 100 °C, especially 0 °C to room temperature. The reaction time is 0.5 to 10 hours.

The amount of NH₂-CH₂C(R⁶)R⁷CH₂-OH is 1.0 to 1-5 mole equivalent to that of the compound of the formula (IIb).

Examples of NH₂-CH₂C(R⁶)R⁷CH₂-OH include 3-aminopropanol, 3-amino-2,2-dimethylpropanol, 3-amino-l-methylpropanol, 3-amino-2-methylpropanol, 3-amino-3-methylpropanol, 3-amino-2,2-diethylpropanol, 1-aminomethyl-1-hydroxymethylcyclopropane, 1-aminomethyl-1-(hydroxymethyl)cyclobutane, 1-aminomethyl-1-(hydroxymethyl)cyclohexane, 1-aminomethyl-1-hydroxymethyl-4-oxacyclobutane or the like.

### Process 3

This is a process for producing a compound of the formula (IId) which comprises the cyclization of the compound of the formula (IIc).

A method of the cyclization includes 1) a method which comprises reacting with diethylazodicarboxylate (DEAD) and triphenylphosphine (Ph₃P), 2) a method which comprises reacting with hydrochloric acid or the like.

In the above (1), the reaction can be carried out in an aprotic solvent (e.g., diethylether, tetrahydrofuran, dimethylformamide, benzene, toluene, dichloromethane, chloroform or the like) with stirring for 0.5 to 5 hours at 0 °C to room temperature. The amount of diethylazodicarboxylate (DEAD) and triphenylphosphine (Ph₃P) are 1.0 to 1.5 mole equivalent to that of the compound (IIc).

In the above (2), the reaction can be carried out in concentrated hydrochloric acid with refluxing for 0.5 to 10 hours.

### Process 4

This is a process for producing a compound of the formula (II) which comprises introducing R² to the compound of the formula (IId).

This process can be carried out by reacting with a compound of the formula: X-C(=Z)W-R⁸ wherein Z is O or S; W is O or S; R⁸ is optionally substituted alkyl, optionally substituted alkenyl, or optionally substituted alkynyl; X is halogen in the presence of a base (e.g., triethylamine, pyridine, N,N-dimethylaminopyridine or the like). This process can be carried out under generally known conditions of N-acylation. For example, the reaction can be carried out in an aprotic solvent (e.g., diethylether, tetrahydrofuran, dimethylformamide, benzene, toluene, dichloromethane, chloroform or the like) with stirring at 0 to 100 °C for 0.5 to 10 hours.

The compounds described in any one of above (6) to (8) can be prepared in accordance with the following processes. 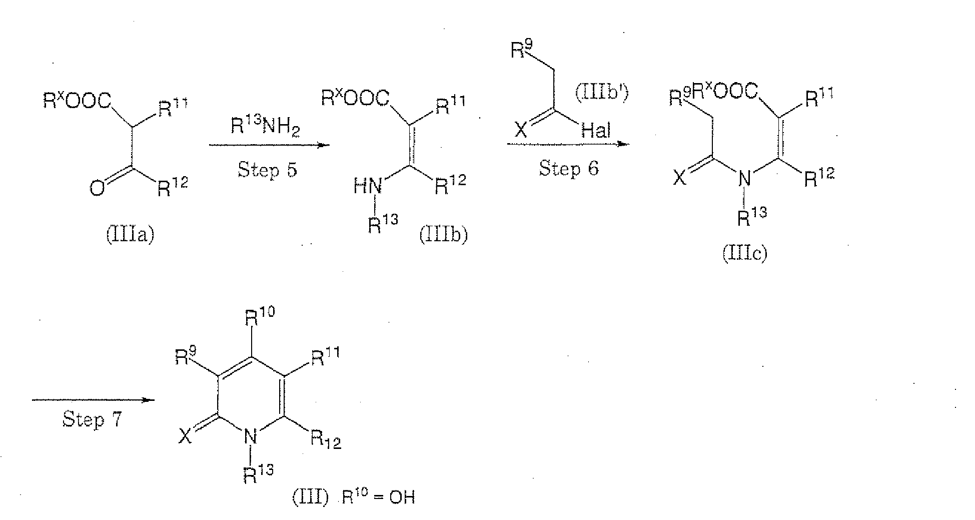 wherein each symbol is as defined above; R^{x} is alkyl or the like; Hal is halogen.

### Step 5

This is a step for preparing a compound represented by the formula (IIIb) which comprises reacting a compound represented by the formula (IIIa) and a compound represented by the formula: R¹³NH₂ wherein R¹³ is as defined above.

Examples of a compound represented by the formula (IIIa) include ethyl acetate, ethyl 2-methylactate, ethyl 2-ethylactate or the like. Examples of a compound represented by the formula: R¹³NH₂ include alkylamine (e.g., methylamine, ethylamine, n-propylamine, n-butylamine or the like), aralkylamine (e.g., benzylamine, phenethylamine or the like) or the like. Examples of a reaction solvent include benzene, toluene, xylene or the like, especially, preferred is toluene or xylene. Example of the reaction temperature includes room temperature to 200 °C, especially preferred is 80 to 180 °C. This step can be carried out by azeotropical dehydration and the obtained product represented by the formula (A-2) can be purified by distillation under reduced or atmosphere pressure or the like.

### Step 6

This is a step for preparing a compound represented by the formula (IIIc) which comprises reacting a compound represented by the formula (IIIb) and a compound represented by the formula (IIIb') in the presence of a base.

Examples of a base include pyridine, dimethylaminopyridine, triethylamine or the like, especially preferred is pyridine. Examples of a reaction solvent include diethyl ether, tetrahydrofuran, ethylene chloride, tluene or the like, especially, preferred is diethyl ether. Example of the reaction temperature includes 0 to 200 °C, especially preferred is room temperature to 100 °C.

### Step 7

This is a step for preparing a compound represented by the formula (III) which comprises cyclizing a compound represented by the formula (IIIc) in the presence of a base.

Examples of a base used include sodium metal, metal alkoxide (e.g., sodium methoxide or the like). As a reaction solvent, preferred is a mixture of alcohol (e.g., methanol or ethanol) and benzene, toluene or the like. Example of the reaction temperature includes 0 to 200 °C, especially preferred is room temperature to 100 °C. 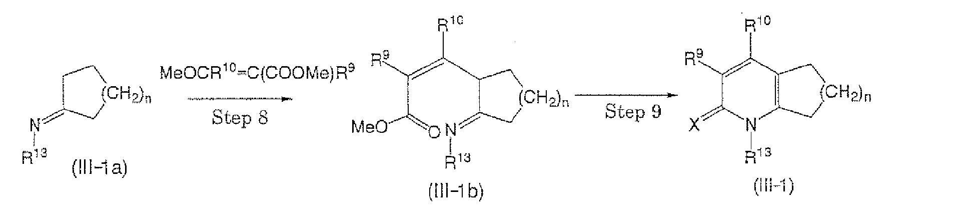 wherein each symbol is as defined above; n is 1. or more.

### Step 8

This is a step for preparing a compound represented by the formula (III-1b) which comprises reacting a compound represented by the formula (III-1a) and a compound represented by the formula MeOCR¹⁰=C(COOMe)R⁹.

Examples of a compound represented by the formula: MeOCR¹⁰=C(COOMe)R⁹ include dimethyl methoxymethylenemalonate, diethyl Methoxymethylenemalonate or the like. Examples of a reaction solvent include diglyme, toluene or the like. Example of the reaction temperature includes room temperature to 200 °C, preferred is 100 to 150 °C.

### Step 9

This is a step for preparing a compound represented by the formula (III-1) which comprises heating a compound represented by the formula (111-1b).

This step using as a reaction solvent diglyme or toluene, is carried out at room temperature to 200 °C, preferred is 100 to 150 °C. Step 8 and Step 9 may be carried out continuously, without isolation of a compound represented by the formula (III-16).

The compounds described in above (9) can be prepared in accordance with the preparation in WO 97/29079. The compounds described in above (10) can be prepared in accordance with the preparation in WO 99/02499. The compounds described in above (11) can be prepared in accordance with the preparation in WO 00/40562. The compounds described in any one of above (12) to (23) can be prepared in accordance with the preparation in these references.

### Example

For example, a compound having an agonistic activity to the cannabinoid receptor used in the present invention can be prepared as follows.

### Example A Preparation of 2-(2-isopropylphenyl)imino-3-(allylthio)thiocarbonyl-5,5-dixnethyl-1,3-thiazine (Compound II-1).

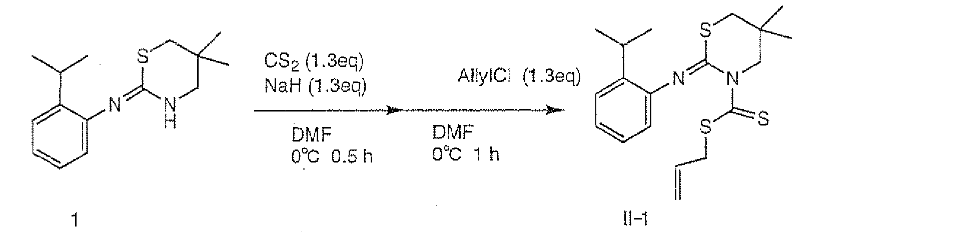

To a solution of 2-(2-isopropylphenyl)imino-5,5-dimethyl-1,3-thiazine (0.26 g) obtained by the preparation described in WO 01/19087, and carbondisulfide (0.10 g) in N,N-dimethylformamide (3 mL) was added 60 % sodium hydride (0.05 g) under ice-cooling. The reaction mixture was stirred for 30 minutes. Allylchloride (0-10 g) was added thereto. The reaction mixture was stirred at 0 °C for 1 h. To the reaction mixture was added water (80 mL), which was extracted with diethylether (100 ml), dried over anhydrous Magnesium sulfate, and concentrated under reduced pressure. The obtained residue was purified by silica gel column chromatography (n-hexane/ethyl acetate) to give 2-(2-isopropylphenyl)imino-3-(allylthio)thiocarbonyl-5,5-dimethyl-1,3-thiazine (0.26 g, yield: 69%) as a pale yellow oil.

### Example B Preparation of 2-(2-isopropylphenyl)imino-3-(5-trinuoromethyl-2-pyridyl)-5,5-dimethyl-1,3-thiazine (Compound II-106).

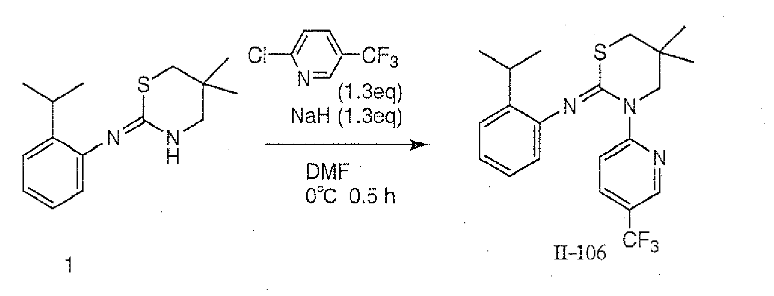

To a solution of 2-(2-isopropylpbenyl)imino-5,5-dimethyl-1,3-thiazine (0.26 g) obtained by the preparation described in WO 01/19087, and 5-trifluoromethyl-2-chloropyridine (0.24 g) in N,N-dimethylformamide (3 mL) was added 60 % sodium hydride (0.05 g) under ice-cooling. The mixture was stirred at room temperature for 2 h. To the reaction mixture was added water (80 mL), which was extracted with diethylether (100 mL), dried over anhydrous magnesium sulfate, and concentrated under reduced pressure. The obtained residue was purified by silica gel column chromatography (n-hexane/ethyl acetate) to give 2-(2-isopropylphenyl)imino-3-(5-trifluoromethyl-2-pyridyl)-5,5-dimethyl-1,3-thiazine (0.13g, yield: 32%) as colorless oil.

### Example C

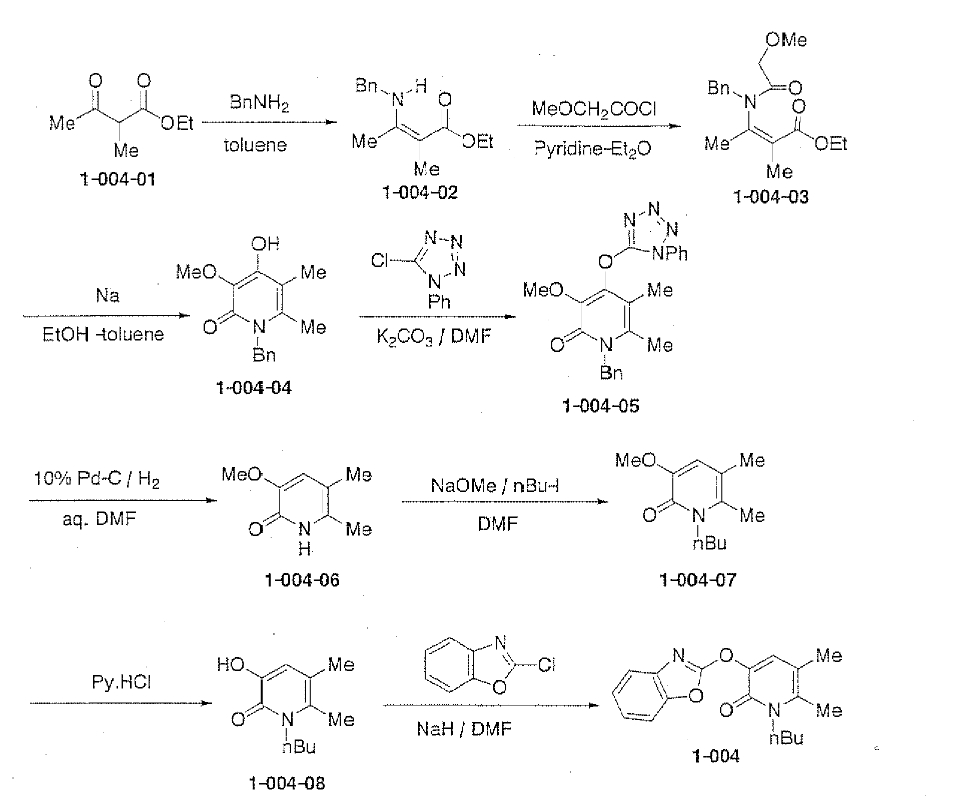

### a) Preparation of ethyl 3-benzylamino-2-methylcrotonate (1-004-02)

A solution of ethyl 2-methylacetoaceate (1-004-01) (115.34 g) and benzylamine (85.73 g) in toluene (1.6 L) was azeotropically dehydrated in an oil bath at 145 °C for 8 h under nitrogen atmosphere. Benzylamine (12.86 g) was added to the reaction mixture and the reaction mixture was dehydrated again. After 6 h, the reaction mixture was distilled under atmospheric pressure to remove about 600 mL of the solvent, and evaporated under reduced pressure to give ethyl 3-benzylamino-2-methylcrotonate (1-004-02) (195.66 g).
¹H NMR (300 MHz, CDCl₃): δ 1.28 (t, *J* = 7.2 Hz, 3H), 1.80 (s, 3H), 1.93 (s, 3H), 4.13 (q, *J* = 7.2 Hz, 2H), 4.43 (d, *J* = 6.3 Hz, 2H), 7.20-7.40 (m, 5H), 9.65 (br s, 1H).

### b) Preparation of ethyl 3-(N-benzylmethoxyacetamide)-2-methylcrotonate (1-004-03)

Ethyl 3-benzylamino-2-methylcrotonate (1-004-02) (97.83 g) was dissolved in diethyl ether (2 L). The reaction mixture was stirred under ice-cooling and nitrogen atmosphere. After pyridine (35.6 mL) was added to the reaction mixture, a solution of methoxyacetyl chloride (90.2 mL) in diethyl ether was added dropwise to the reaction mixture at 5 to 6 °C as internal temperature for 45 min. After atirring 30 min, the reaction mixture was for the stirred at room temperature for 2 h, the reaction mixture was poured into ice-water (1.5 L), extracted twice with diethyl ether, and washed with water (1 L) and an aqueous solution of sodium bicarbonate. The extract was dried over magnesium sulfate, and evaporated under reduced pressure to give ethyl 1-benzylmethoxyacetamide-2-methylcrotonate (1-004-03) (111.47 g, 91.3%) as an oil. ¹H NMR (300 MHz, CDCl₃): δ 1.21 (t, *J* = 7.2 Hz, 3H), 1.74 (s, 3H), 1.89 (s, 3H), 3.44 (s, 3H), 3.97 (d, *J* = 14.7 Hz, 1H), 3.98 (q, *J =* 7.2 Hz, 2H), 4.12 (d, *J =* 14.7 Hz, 1H), 4.31 (d, *J* = 14.4 Hz, 1H), 4.95 (d, *J* = 14.4 Hz, 1H), 7.20-7.40 (m, 5H).

### c)Preparation of 1-benzyl-5,6-dinzethyl-4-hydroxy-3-methoxy-2-pyridone (1-004-04)

To a solution of toluene (1.39 L) and ethanol (2.08 mL) was added sodium metal (7.98 g) under nitrogen atmosphere, and the reaction mixture was stirred in an oil bath at 140 °C. A solution of ethyl 1-benzylmethoxyacetamide-2-methylcrotonate (1-004-03) (105.93 g) in toluene (340 mL) was added dropwise to the reaction mixture over 1 h and 20 min, and the reaction mixture was stirred under reflux. After 2 h, the reaction mixture was stirred under ice-cooling and 4 mole/L hydrochloric acid/dioxane (86.8 mL) was added dropwise over 10 min to the reaction mixture, and the reaction mixture was stirred at room temperature. After 2 h, the resulting precipitate was filtered, and washed with toluene to give the precipitate (73.16 g). To the obtained precipitate (73.16 g) were added chloroform (500 mL) and water (500 mL), and the reaction mixture was dissolved in a water bath at 65 °C, and shaken to separate. Further the reaction mixture was extracted with chloroform (250 mL), washed with water (250 mL), dried over magnesium sulfate, and evaporated under reduced pressure to give the residue (54.95 g). The obtained residue (54.95 g) was washed with ethyl acetate (50 mL) and diethyl ether (50 mL) to give 1-benzyl-5,6-dimethyl-4-bydroxy-3-methoxy-2-pyridone (1-004-04) (53.95 g, 60.0%) as a skin-colored crystal.
m.p.: 212 °C.
¹H NMR (300 MHz, CDCl₃): δ 2.03 (s, 3H), 2.19 (s, 3H), 3.99 (s, 3H), 5.38 (br s, 2H), 6.41 (br s, 1H), 7.11-7.33 (m, 5H).

### d) Preparation of 1-benzyl-5,6-dimethyl-3-methoxy-4-O-(1-phenyl-1H-tetrazolyl)-2-pyridone (1-004-05)

DMF (300 mL) was added to a mixture of 1-benzyl-5,6-dimethyl-4-hydroxy-3-methoxy-2-pyridone (1-004-04) (25.93 g), 5-chloro-1-phenyl-1*H-*tetrazole (21.67 g) and potassium carbonate (27.64 g) under nitrogen atmosphere, and the suspension was stirred at room temperature. After 4 and half hour, the reaction mixture was poured into ice-water (1 L), extracted three times with ethyl acetate (500 mL), washed twice with water (500 mL), dried over magnesium sulfate, and evaporated under reduced pressure. The obtained residue (42.4 g) was dissolved in acetone (300 mL). After concentration under reduced pressure, diethyl ether (300 mL) was added to the residue. The resulting crystal was filtered to give 1-benzyl-5,6-dimethyl-3-methoxy-4-O-(1-phenyl-1*H-*tetrazolyl)-2-pyridone (1-004-05) (29.87 g, 74.0%, m.p.: 178 °C). Further the filtrate was purified by silica gel column chromatography (150 g, CHCl₃) to give an additional 1-004-05 (4.3 g, 10.7%).
¹H NMR (300 MHz, CDCl₃): δ 2.07 (s, 3H), 2.28 (s, 3H), 3.79 (s, 3H), 5.41 (br s, 2H), 7.15-7.84 (m, 10H).

### e) Preparation of 5,6-dimethyl-3-methoxy-2-pyridone (1-004-06)

A solution of 1-benzyl-5,6-dimethyl-3-methoxy-4-0-(1-phenyl-1*H*-tetrazolyl)-2-pyridone (1-004-05) (27.15 g) in DMF (272 mL) was added a suspension of 10% palladium on carbon (5.43 g) in water (27 mL). The reaction mixture was reduced under medium hydrogen pressure (5 kg/cm²) at room temperature. In the course of the reaction, 10% palladium on carbon (2.72 g) was added to the reaction mixture. After 48 h, catalyst was filtered off on Celite and washed with methanol, and the filtrate was evaporated under reduced pressure. After water (160 mL) was added to the obtained residue and the reaction mixture was heated at 85 °C in a water bath, the insoluble substance was filtered off. The insoluble substance was washed with hot water to give an insoluble substance (8.77 g). The filtrate was evaporated under reduced pressure, and acetone (110 mL) was added to the obtained residue (11.55 g). After the mixture was stirred at room temperature, a colorless powder was filtered to give 1-004-06 (8.23 g, 79.8%, m.p.: 215-219 °C). 1-004-06 (0.31 g, 3.0%) was obtained from the filtrate in a similar manner to the treatment described above.
¹H NMR (300 MHz, CDCl₃): δ 2.07 (s, 3H), 2.28 (s, 3H), 3.79 (s, 3H), 5.41 (br s, 2H), 7.15-7.84 (m, 10H).

### f) Preparation of 1-butyl-5,6-dimethyl-3-methoxy-2-pyridone (1-004-07)

n-Butanol (13 mL) was added to a mixture of 5,6-dimethyl-3-methoxy-2-pyridone (1-004-06) (306 mg) and potassium hydroxide (157 mg). 1-Iodobutane (0.44 mL) was added to the suspension, and the reaction mixture was heated with stirring in an oil bath at 85 °C under nitrogen atmosphere. After 24 h, the reaction mixture was evaporated under reduced pressure, and the residue was dissolved in acetyl acetate and water. The reaction mixture was extracted twice with ethyl acetate, and once with water, dried over magnesium sulfate, and evaporated under reduced pressure. The obtained residue (330 mg) was purified by silica gel column chromatography (Lobar column B, toluene/acetone (3/1)) to give 1-butyl-5,6-dimethyl-3-methoxy-2-pyridone (1-004-07) (124 mg, 29.6 %).
¹H NMR (300 MHz, CDCl₃): δ 0.96 (t, *J* = 7.2 hz, 3H), 1.36-1.48 (m, 2H), 1.60-1.70 (m, 2H), 2.09 (s, 3H), 2.26 (s, 3H), 3.78 (s, 3H), 4.08 (t, *J* = 7.8 Hz, 2H), 6.44 (s, 2H).

### g) Preparation of 1-butyl-5,6-dimethyl-3-hydroxy-2-pyridone (1-004-08)

Pyridinium chloride (293 mg) was added to 1-butyl-5,6-dimethyl-3-methoxy-2-pyridone (1-004-07) (124 mg), and the reaction mixture was heated with stirring in an oil bath at 200 °C under nitrogen atmosphere. After 30 min, the reaction mixture was dissolved in diethyl ether and water, extracted twice with diethyl ether, washed once with water, and dried over magnesium sulfate. The solvent was evaporated under reduced pressure to give 1-butyl-5,6-dimethyl-3-hydroxy-2-pyridone (1-004-08) (94 mg, 81%, m.p.: 112-116 °C)
¹H NMR (300 MHz, CDCl₃): δ 0.98 (t, *J =* 7.2 hz, 3H), 1.37-1.50 (m, 2H), 1.61-1.72 (m, 2H), 2.08 (s, 3H), 2.26 (s, 3H), 4.10 (t, *J =* 7.8 Hz, 2H), 6.66 (br s, 2H).

### h) Preparation of 3-(benzoxazole-2-yloxy)-1-butyl-5,6-dimethyl-1H-pyridine-2-one (1-004)

3-(Benzoxazole-2-yloxy)-1-butyl-5,6-dimethyl-1*H*-pyridine-2-one (1-004) (66.7%, m.p. 106-108 °C) was synthesized in a similar manner to the preparation of 1-015.

### Example D

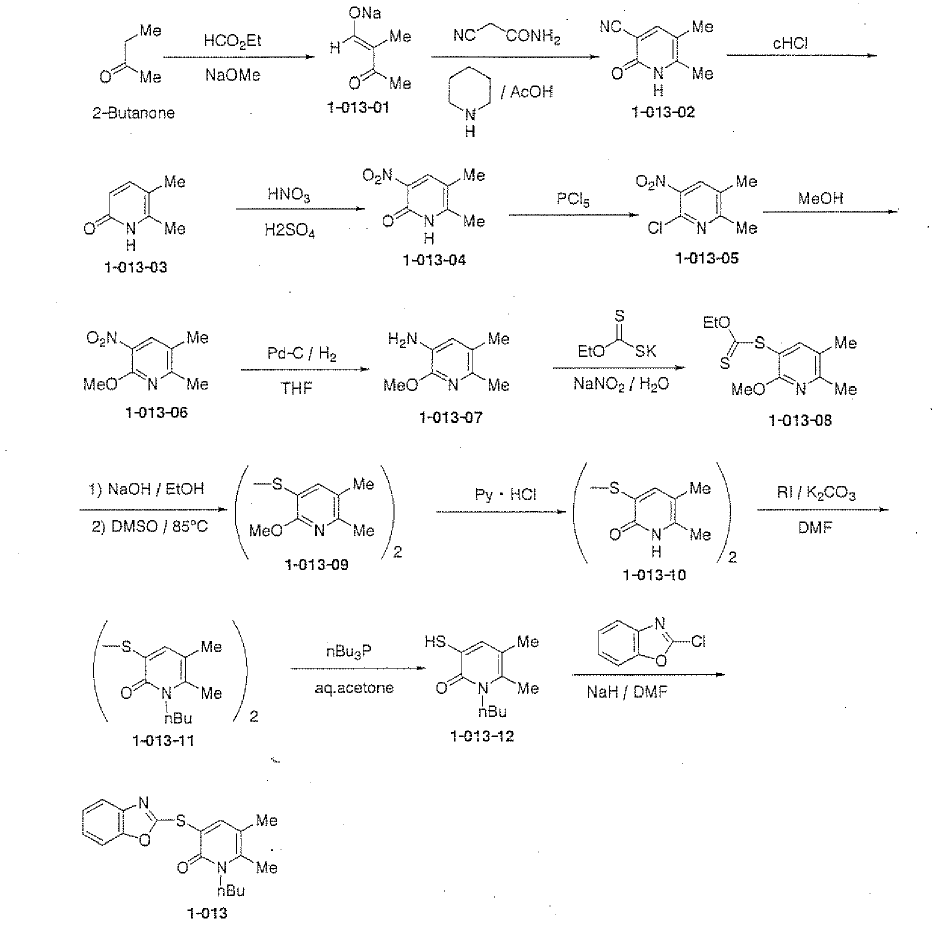

### a) Preparation of 2-methyl-3-oxobutanal sodium salt (1-013-01)

A solution of 28% sodium methoxide/methanol (138 mL) was diluted with diethyl ether (920 mL) and the reaction mixture was stirred under ice-cooling, and to the reaction mixture was added dropwise a mixture of 2-butanone (51.2 g) and ethyl formate (57.2 g) at 4 to 6 °C as internal temperature for 45 min. After the reaction mixture was stirred at the same temperature for an additional 30 min, and at room temperature overnight. The resulting colorless powder was filtered to give 2-methyl-3-oxobutanal sodium salt (1-013-01) (60.66 g, 70%).
¹H NMR (300 MHz, CDCl₃): δ 1.62 (s, 3H), 2.13 (s, 3H), 8.99 (s, 1H).

### b) Preparation of 3-eyano-5,6-dimethyl-2-pyridone (1-013-02)

Water (546 mL) was added to 2-methyl-3-oxobutanal sodium salt (1-013-01) (34.73 g), and to the reaction mixture was added 2-cyanoacetamide (23.91 g) and 1.76 mol/L piperidinium acetate (119.4 mL), and the reaction mixture was stirred under reflux in an oil bath at 127 °C. After 21 h, to the reaction mixture was added gradually dropwise acetic acid (42.7 mL) at 65 °C as internal temperature for 15 min. After the stirring was continued until internal temperature became to 24 °C, the resulting crystal was filtered, and washed with water to give 3-cyano-5,6-dimethyl-2-pyridone (1-013-02) (27.76 g, 65.9%, m.p. 258-263 °C).
¹H NMR (300 MHz, DMSO): δ 1.98 (s, 3H), 2.23 (s, 3H), 7.95 (s, 1H), 12.45 (br s, 1H).

### c) Preparation of 5,6-dimethyl-2-pyridone (1-013-03)

To a suspension of 3-cyano-5,6-dimethyl-2-pyridone (1-013-02) (12.0 g) in water (293 mL) was added cone. hydrochloric acid (293 mL), and the reaction mixture was reflux with stirring in oil-bath at 135 °C. After 3 days, the reaction mixture was cooled, and evaporated under reduced pressure. To the residue (24.75 g) were added chloroform (300 mL) and methanol (15 mL), and the reaction mixture was heated in a water bath at 65 °C, and the dissolble material was filtered off. Furthermore, the dissolble material was treated by chloroform (200 mL) and methanol (10 mL) in a similar manner to described above. The combined filtrates were evaporated under reduced pressure, To the obtained residue (13.26 g) were added methanol (150 mL) and potassium carbonate (10 g). After stirred at room temperature for 30 min, the dissolble material was filtered off. The filtrate was evaporated under reduced pressure. To the obtained residue (14.7 g) was added chloroform (200 mL), and the dissolble material was filtered off again. The filtrate was evaporated under reduced pressure to give 5,6-dimethyl-2-pyridone (1-013-03) (9.41 g, 94.3%, m.p.: 202-207 °C)
¹H NMR (300 MHz, CDCl₃): δ 2.05 (s, 3H), 2.31 (s, 3H), 6.38 (d, *J* = 9.0 Hz, 1H), 7.26 (d, *J* = 9.0 Hz, 1H), 13.17 (br s, 1H).

### d) Preparation of 5,6-dimethyl-3-nitro-2-pyridone (1-013-04)

5,6-Dimethyl-2-pyridone (1-013-03) (3.695 g) was dissolved in conc. sulfuric acid (38 mL) under ice-cooling, and the reaction mixture was stirred under ice-cooling, and then to the reaction mixture was added dropwise 70% nitric acid (3.53 mL) at 3 to 5 °C as internal temperature for 50 min, and then the reaction mixture was stirred. After 2 h, the reaction mixture was poured gradually into ice and the resulting crystal was filtered. The crystal was washed with water to give 5,6-dimethyl-3-nitro-2-pyridone (1-013-04) (3.102 g, 61.5%, m.p.;251-257 (dec) ). Furthermore the aqueous layer was extracted five times with chloroform, the organic layer was dried over magnesium sulfate, and evaporated under reduced pressure. The resulting crystal was filtered to give an additional 5,6-dimethyl-3-nitro-2-pyridone (1-013-04)(271 mg, 5.4%).
¹H-NMR (300MHz, DMSO):2.06 (s, 3H), 2.29 (s, 3H), 8.35 (s, 1H), 12.79 (br s, 1H).

### e) Preparation of 2-chloro-5,6-dimethyl-3-nitropyridine (1-013-05)

5,6-Dimethyl-3-nitro-2-pyridone (1-013-04)(841 mg) and phosphorus pentachloride (1.25 g) was heated with stirring in an oil bath at 140 °C under nitrogen atmosphere. After 35 min, the reaction mixture was cooled under ice-cooling, poured into ice-water, extracted with twice with chloroform, and washed once with water and a saturated aqueous solution of sodium bicarbonate. To the extract was added a decolorzing charcoal, and the extract was dried over magnesium sulfate, and evaporated under reduced pressure to give 2-chloro-5,6-dimethyl-3-nitropyridine (1-013-05) (842 mg, 90.2%) as a crystalline residue.
¹H NMR (300 MHz, CDCl₃): δ 2.38 (s, 3H), 2.58 (s, 3H), 8.01 (s, 1H).

### f) Preparation of 5,6-dimetbyl-2-methoxy-3-nitropyridine (1-013-06)

To a solution of 28% sodium methoxide (1.11 mL) and methanol (5.5 mL) was added dropwise a solution of 2-chloro-5.6-dimethyl-3-nitropyridine (1-013-05) (837 mg) in methanol (6.6 mL) for 5 min at room temperature under nitrogen atmosphere, and the reaction mixture was heated with stirring in oil-bath at 50 °C for 7 h. To the reaction mixture was added diethyl ether and the reaction mixture was poured into water, extracted with twice with diethyl ether, washed once with water, and dried over magnesium sulfate. The solvent was evaporated under reduced pressure to give 5,6-dimethyl-2-methoxy-3-nitropyridine (1-013-06) (675 mg, 82.6%, m.p.: 71-73 °C) as an orange crystal.
¹H NMR (300 MHz, CDCl₃): δ 2.28 (s, 3H), 2.48 (s, 3H), 4.08 (s, 3H), 8.07 (s, 1H).

### g) Preparation of 3-amino-5,6-dimethyl-2-methoxypyridine (1-013-07)

5,6-Dimethyl-2-methoxy-3-nitropyridine (1-013-06) (2.56 g) was dissolved in tetrahydrofuran (41 mL), and to the reaction mixture was added a suspension of 5% palladium on carbon (450 mg) in methanol (41 mL), and then the catalytic reduction was carried out. After 3 h, catalyst was filtered off, and the filtrate was evaporated under reduced pressure to give 3-amino-5,6-dimethyl-2-methoxypyridine (1-013-07) (2.096 g, 97.9%, m.p.: 56-58°C) as a blackish brown crystal.
¹H NMR (300 MHz, CDCl₃): δ 2.12 (s, 3H), 2.30 (s, 3H), 2.48-3.49 (br s, 2H), 3.95 (s, 3H), 6.70 (s, 1H).

### h) Preparation of 5,6-dimethyl-3-[[ethoxy(thiocarbonyl)]thio]-2-methoxypyridine (1-013-08)

3-Amino-5,6-dimethyl-2-methoxypyridine (1-013-07) (1.787 g) was dissolved in water (3 mL) and conc. hydrochloric acid (3 mL), and the reaction mixture was cooled under ice-acetone bath, and stirred under cooling. To the reaction mixture was added dropwise a solution of sodium nitrite (4.81 g) in water (27.1 mL) at 4 to 5 °C as internal temperature for 45 min, and the reaction mixture was stirred at the same temperature. On the other hand, potassium ethylxanthrate (12.64 g) was dissolved in water (17.3 mL) , and the reaction mixture was heated with stirring in an oil bath at 40 °C. To the reaction mixture was added dropwise a cooling solution of the above prepared diazonium salt for 35 min, and the reaction mixture was heated with stirring for 40 min. The reaction mixture was cooled, extracted three times with chloroform, washed once with a saturated aqueous solution of sodium bicarbonate and brine, and dried over magnesium sulfate. The extract was evaporated under reduced pressure. The red oily residue (12.49 g) was purified by silica gel column chromatography (300 g, toluene/hexane = 2/3) to give 5,6-dimethyl-3-[[ethoxy(thiocarbonyl)]thio]-2-methoxypyridine (1-013-08) (6.281 g, 36%) as an red oil. The obtained compound contained 2 isomers caused by rotatory hindrance.
¹H NMR (300 MHz, CDCl₃): δ 1.45 (t, *J* = 7.2 Hz , 3H), 2.18 (s, 3H), 2.39 (s, 3H), 3.98 (s, 3H), 4.70 (q, *J =* 7.2 Hz, 2H), 7.47 (s, 1H).
¹H NMR (300 MHz, CDCl₃): δ 1.33 (t, *J =* 7.2 Hz , 3H), 2.21 (s, 3H), 2.44 (s, 3H), 3.94 (s, 3H), 4.60 (q, *J* = 7.2 Hz, 2H), 7.43 (s, 1H).

### i) Preparation of (5,6-dimethyl-2-methoxypyridin-3-yl)disulfide (1-013-09)

5,6-Dimethyl-3-[[ethoxy(thiocarbonyl)]thio]2-methoxypyridine (1-013-08) (6.275 g) was dissolved in ethanol (200 mL) and the reaction mixture was stirred at room temperature. To the reaction mixture was added 1 mole/L sodium hydroxide solution (67 mL) at a time under nitrogen atmosphere, and the reaction mixture was stirred overnight. After 15 h, the resulting precipitate was filtered, and washed with water to give the precipitate (543 mg). Furthermore, the filtrate was adjusted with 5 mole/L hydrochloric acid to pH 3, and evaporated under reduced pressure. To the residue was added methylene chloride (100 mL), and the mixture was stirred at room temperature. The insoluble material was filtered off and the filtrate was evaporated under reduced pressure to give the residue (2.00 g).

The combined the residue (2.00 g + 543 mg) was suspended with dimethylsulfoxide (20 mL), and the mixture was heated with stirring in an oil bath at 85 °C under nitrogen atmosphere. After 7 h, the reaction mixture was stirred at room temperature, and to the reaction mixture was added water (100 mL). The reaction mixture was stirred under ice-cooling for 30 min to give (5,6-dimethyl-2-methoxypyridin-3-yl)disulfide (1-013-09) (2.23 g, 54.4%) as a yellow powder.
¹H NMR (300 MHz, CDCl₃): δ 2.16 (s, 3H), 2.36 (s, 3H), 3.96 (s, 3H), 7.53 (s, 1H).

### j) Preparation of (5,6-dimethyl-2-pyridon-3-yl)disulfide (1-013-10)

Pyridium chloride (7.69 g) was added to (5,6-dimethyl-2-methoxypyridin-3-yl)disulfide (1-013-09) (2.225 g) and the reaction mixture was heated with stirring in an oil bath at 160 °C under nitrogen atmosphere. After 40 min, the reaction mixture was cooled and water (100 mL) was added to the reaction mixture. The reaction mixture was stirred at room temperature, and the resulting precipitate was filtered, and washed with water to give (5,6-dimethyl-2-pyridon-3-yl)disulfide (1-013-10) (1.736 g, 85.1%) as a blackish brown powder. The aqueous layer was extracted two times with chloroform, washed with two times with water, dried over magnesium sulfate, and evaporated. The obtained residue (81 mg) was washed with ethanol to give the desired compound (41 mg, 2.0%) as yellow powder.
¹H NMR (300 MHz, CDCl₃): δ 1.95 (s, 3H), 2.13 (s, 3H), 7.42 (s, 1H), 11.89 (br s, 1H),

### k) Preparation of (1-butyl-5,6-dimethyl-2-pyridon-3-yl)disulfide (1-013-11)

(5,6-Dimethyl-2-pyridon-3-yl)disulfide (1-013-10) (31 mg) was suspended with DMF (1 mL), and to the reaction mixture were added 1-iodobutane (78 mg) and potassium carbonate (42 mg). The reaction mixture was stirred at room temperature for 3 days under nitrogen atmosphere. Ethyl acetate was added to the reaction mixture and the reaction mixture was poured into water, extracted twice with ethyl acetate, washed once with water, dried over magnesium sulfate, and evaporated to give the residue (39 mg, 92.9%). The obtained compound contained a 1-butyl derivative (1-013-11) at the rate of 20% judging from NMR data.

### 1) Preparation of (1-butyl-5,6-dimethyl-3-mercapto-2-pyridone (1-013-12)

(1-Butyl-5,6-dimethyl-2-pyridon-3-yl)disulfide (1-013-11) (123 mg) was dissolved in acetone (8 mL) and the reaction mixture was stirred at room temperature, to the reaction mixture was added tri-n-butylphosphine (0.16 mL), and added gradually water (4 ml). The reaction mixture was stirred at the same temperature for 2 h, and then was stirred at room temperature overnight. The reaction mixture was diluted with methylene chloride, and water, extracted twice with mathylene chloride, washed once with water, dried over magnesium sulfate, and evaporated under reduced pressure. The obtained residue (277 mg) was subjected to preparative thin-layer chromatography (toluene/acetone = 39/1) to give (1-butyl-5,6-dimethyl-3-mercapto-2-pyridone (1-013-12) (11 mg, 8.9%) as a crystal.
¹H NMR (300 MHz, CDCl₃): δ 0.93 (t, *J* = 7.2 Hz, 3H), 1.32-1.45 (m, 2H), 1.62-1.72 (m, 2H), 2.11 (s, 3H), 2.32 (s, 3H), 4.20 (t, *J* = 7.8 Hz, 2H), 7.68 (s, 1H).

### m) Preparation of N-1-butyl-5,6-dimethyl-3-(benzoxazol-2-yl)thio-2-pyridone (1-013)

1-013 (4.5 mg, 26.5%) as a crystal was obtained from (1-butyl-5,6-dimethyl-3-mercapto-2-pyridone (11 mg) in a similar manner to the preparation of Example 1-015.

### Example E

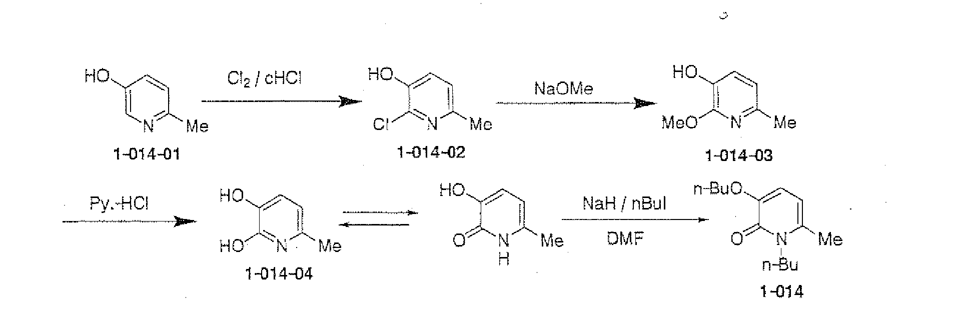

### a) Preparation of 2-chloro-3-hyroxy-6-methylpyridine (1-014-02)

5-Hydroxy-2-methylpyridine (1-014-01) (27.01 g) was dissolved in conc. hydrochloric acid (200 mL) and to the reaction mixture was bubbled chlorine gass at 68 to 74 °C. After the reaction mixture was stood overnight, the volatile was removed by bubbling nitrogen gas. The reaction mixture was evaporated under reduced pressure to give the crystal residue. The crystal residue was dissolved in methanol, and treated with active charcoal. After recrystallization, the desired 2-chloro-3-hyroxy-6-methylpyridine (1-014-02) (23.96 g, 67.3%) was obtained.

### b) Preparation of 2-methoxy-3-hyroxy-6-methylpyridine (1-014-03)

In metal sealed tube were added 2-chloro-3-hyroxy-6-methylpyridine (1-014-02) (22.91 g) and 28% sodium methoxide/methanol solution (120 mL) and the reaction mixture was reacted at 150°C for 3 days. To the reaction mixture was added ice and water (100 mL) and the reaction mixture was neutralized with acetic acid, and evaporated completely. The residue was purified by silica gel column chromatography (chloroform) to give the desired 2-methoxy-3-hyroxy-6-methylpyridine (1-014-03) (10.44 g, 48.1%). Furthermore, the fraction contained the starting material was reacted in sealed tube again.
¹H-NMR (300MHz, CDCl₃): δ 2.35 (s,3H), 3.97 (s,3H), 6.60 (d, *J* = 7.8Hz, 1H), 6.98 (d, *J* = 7.8Hz, 1H).

### c) Preparation of 2,3-dihyroxy-6-methylpyridine (1-014-04)

Pyridine hydrochloride salt (43.3 g) was added to 2-methoxy-3-hyroxy-6-methylpyridine (1-014-03) (10.43 g) obtained above, the reaction mixture was heated at 160°C for 1 h and at 170°C for 20 min. To the reaction mixture was added water (50 mL) and the reaction mixture was extracted repeatingly with 5% methanol/ethyl acetate and ethyl acetate. The combined extracts were evaporated completely to give the light gray residue. This 2,3-dihyroxy-6-methylpyridine (1-014-04) was used in the next reaction without purification.

### d) Preparation of 1-butyl-3-butyl-6-methyl-2-pyridone (1-014-05)

The crude 2,3-dihyroxy-6-methylpyridine (1-014-04) (16.04 g) was dissolved in dry DMF (70 mL) and 60% solid sodium hydride (10.25 g) was added gradually to the reaction mixture. The reaction mixture was stirred at room temperature for 30 min under nitrogen atmosphere. A solution of 1-iodobutane (29.1 mL) in DMF (30 mL) was added dropwise to the reaction mixture for 20 min and the reaction mixture was stirred at room temperature for 3 h. To the reaction mixture was added a saturated aqueous solution of ammonium chloride, and the reaction mixture was extracted three times with 150 mL of ethyl acetate. The aqueous layer was extracted repeatingly with chloroform and the combined organic layers were treated with active charcoal, and evaporated under reduced pressure. The obtained residue was purified by silica gel column chromatography (n-hexane/ethyl acetate) to give 1-butyl-3-0-butyl-6-methyl-2-pyridone (1-014) (8.915 g). Total yield of two steps was 50.1%.
¹H-NMR (300MHz, CDCl₃): δ 0.96 (t, *J* = 7.5 Hz, 6H), 1.43 (m,4H), 1.67 (m,2H), 1.82 (m,2H), 2.31 (s,3H), 3.88 (t, *J* = 6.6 Hz, 2H), 4.01 (t, *J* = 7.8 Hz, 2H), 5.87 (d, *J* = 7.8 Hz, 1H),6.52(d, *J* = 7.8 Hz, 1H).

### Example F

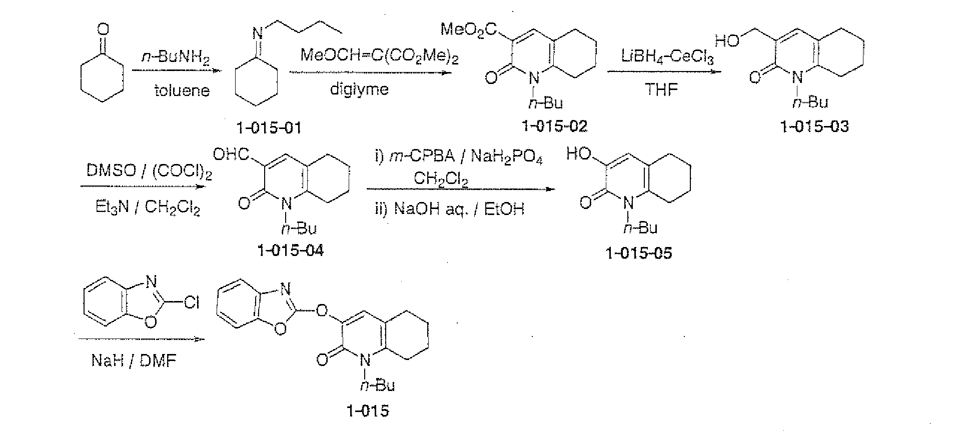

### a) Preparation of butyl-cyclohexylideneamine (1-015-01)

1-Butylamine (9.88 mL, 0.1 mol) and toluene (15 mL) was added to cyclohexanone (10.36 mL, 0.1 mol) and the reaction mixture was heated under reflux for 24 h under dehydration condition by using Dienstark reflux condenser packing molecular sieves 4A. After the reaction mixture was cooled to room temperature, evaporated under reduced pressure. The residue was distilled under reduced pressure (2 mmHg) at 64 °C to give butyl-cyclohexylideneamine (1-015-01) (12.8 g, 84%) as a colorless oil.
¹H NMR (300 MHz, CDCl₃): δ 0.93 (t, *J* = 7.5 Hz, 3H), 1.35 (sextet, *J* = 7.5 Hz, 2H), 1.58 (quint, *J* = 7.5 Hz, 2H), 1.61-1.70 (m, 4H), 1.71-1.17 (m, 2H), 2.30 (t, *J* = 6.0 Hz, 2H), 2.34 (t, *J* = 6.0 Hz, 2H), 3.30 (t, *J* = 7.5 Hz, 2H).

### b) Preparation of 1-butyl-2-oxo-1,2,5,6,7,8-hexahydroisoquinoline-3-carboxylic acid methyl ester (1-015-02)

Butyl-cyclohexylideneamine (1-015-01) (12.8 g, 83.6 mmol) was dissolved in diglyme (75 mL) and the reaction mixture was heated at 120 °C. To the reaction mixture was added dropwise a solution of dimethyl methoxymethylenemalonate (14 g, 80.4 mmol) in diglyme (75 mL) for 1 h and the reaction mixture was reacted at 120 °C for 3 h. After cooling, the diglyme was evaporated under reduced pressure. The obtained residue was purified by silica gel column chromatography (toluene/ethyl acetate) to give 1-buthyl-2-oxo-1,2,5,6,7,8-hexahydroisoquinoline-3-carboxylic acid methyl ester (1-015-02) (15 g, 71%) as an yellow oil.
¹H NMR (300 MHz, CDCl₃): δ 0.97 (t, *J* = 7.5 Hz, 3H), 1.43 (sextet, *J* = 7.5 Hz, 2H), 1.63-1.78 (m, 4H), 1.87 (quint, *J* = 6.0 Hz, 2H), 2.57 (t, *J* = 6.0 Hz, 2H), 2.73 (t, *J* = 6.0 Hz, 2H), 3.90 (s, 3H), 4.02 (t, *J* = 7.8 Hz, 2H), 7.92 (s, 1H).

### c) Preparation of 1-butyl-3-hydroxymethyl-5,6,7,8-tetrahydro-1H-quinoline-2-one (1-015-03)

1-Buthyl-2-oxo-1,2,5,6,7,8-hexahydroisoquinoline-3-carboxylic acid methyl ester (1-015-02) (130 mg, 0.5 mmol) was dissolved in THF (12 mL), and to the reaction mixture were added CeCl₃· 7H₂O (372.6 mg, 1 mmol) and lithium borohydride (21.8 mg, 1 mmol). The reaction mixture was stirred at room temperature for 20 min and to the reaction mixture was added 1 mol/L diluted hydrochloric acid (20 mL). The reaction mixture was extracted with ethyl acetate (40 mL), washed with brine (30 mL), dried over anhydrous magnesium sulfate, and evaporated under reduced pressure. The residue was reacted once more under the same conditions described above and the similar post treatment was performed. The obtained residue was purified by silica gel column chromatography (toluene/ethyl acetate) to give 1-butyl-3-hydroxymethyl-5,6,7,8-tetrahydro-1*H*-quinoline-2-one (1-015-03) (80 mg, 68%) as a colorless oil.
¹H NMR (300 MHz, CDCl₃): δ 0.98 (t, *J* = 7.5 Hz, 3H), 1.43 (sextet, *J* = 7.5 Hz, 2H), 1.65 (quint, *J* = 7.5 Hz, 2H), 1.71 (quint, *J* = 6.0 Hz, 2H), 1.85 (quint, *J* = 6.0 Hz, 2H), 2.52 (t, *J* = 6.0 Hz, 2H), 2.68 (t, *J* = 6.0 Hz, 2H), 4.00 (t, *J* = 7.8 Hz, 2H), 4.53 (s, 2H), 7.02 (s, 1H).

### d) Preparation of 1-butyl-2-oxo-1,2,5,6,7,8-hexahydroquinoline-3-carboaldehyde (1-015-04)

DMSO (0.54 mL, 7.64 mmol) was dissolved in methylene chloride (27 mL) and the solvent was cooled at - 78 °C. To the solvent were added dropwise oxalyl chloride (0.4 mL, 4.58 mL), a solution of 1-butyl-3-hydroxymethyl-5,6,7,8-tetrahydro-1*H*-quinaline-2-one (1-015-03) (0.9 g, 3.82 mmol) in methylene chloride (20 mL) and triethylamine (1.33 mL, 9.55 mmol), and then the reaction mixture was stirred at - 78 °C for 5 min. The reaction mixture was gradually warmed to room temperature, and stirred at room temperature for 20 min. To the reaction mixture was added 1 mol/L diluted hydrochloric acid (50 mL), and the reaction mixture was extracted with ethyl acetate (200 mL), washed with aqueous saturated sodium hydrogen carbonate solution (50 mL) and brine (50 mL), dried over anhydrous magnesium sulfate, and evaporated under reduced pressure. The obtained residue was purified by silica gel column chromatography (toluene/ethyl acetate) to give 1-butyl-2-oxo-1,2,5,6,7,8-hexahydroquinoline-3-carboaldehyde (1-015-04) (0.5 g, 56%) as a pale yellow bubbly substance.
¹H NMR (300 MHz, CDCl₃): δ 0.99 (t, *J* = 7.5 Hz, 3H), 1.46 (sextet, *J* = 7.5 Hz, 2H), 1.68 (quint, *J* = 7.5 Hz, 2H), 1.74 (quint, *J* = 6.0 Hz, 2H), 1.88 (quint, *J* = 6.0 Hz, 2H), 2.59 (t, *J* = 6.0 Hz, 2H), 2.76 (t, *J* = 6.0 Hz, 2H), 4.05 (t, *J* = 7.8 Hz, 2H), 7.76 (s, 1H), 10.34 (s, 1H).

### e) Preparation of 1-butyl-3-hydroxy-5,6,7,8-tetrahydro-1H-quinoline-2-one (1-015-05)

1-Butyl-2-oxo-1,2,5,6,7,8-hexahydroquinoline-3-carboaldehyde (1-015-04) (160 mg, 0.69 mmol) was dissolved in methylene chloride (10 mL), and to the reaction mixture were added NaH₂PO₄·H₂O (190 mg, 1.38 mmol) and meta-chloroperbenzoic acid (237 mg, 1.38 mmol). The reaction mixture was stirred at room temperature for 30 min, and to the reaction mixture was added 5% aqueous sodium thiosulfate solution (20 mL). The reaction mixture was extracted with ethyl acetate (50 mL), washed with aqueous saturated sodium hydrogen carbonate solution (20 mL) and brine (20 mL), dried over anhydrous magnesium sulfate, and evaporated under reduced pressure. The residue was dissolved in ethanol (5 mL), 2 mol/L aqueous sodium hydroxide solution (0.35 mL, 0.7 mmol) was added to the reaction mixture, and the reaction mixture was stirred at room temperature for 30 min. To the reaction mixture was added 0.2 mol/L diluted hydrochloric acid (7 mL), and the reaction mixture was extracted with ethyl acetate (25 ml), washed brine (10 mL), dried over anhydrous magnesium sulfate, and evaporated under reduced pressure. The obtained residue was purified by silica gel column chromatography (toluene/ethyl acetate) to give 1-butyl-3-hydroxy-5,6,7,8-tetrahydro-1*H*-quinoline-2-one (1-015-05) (82 mg, 54%) as a pale brown color crystal.
¹H NMR (300 MHz, CDCl₃): δ 0.97 (t, *J* = 7.5 Hz, 3H), 1.42 (sextet, *J* = 7.5 Hz, 2H), 1.60-1.74 (m, 4H), 1.83 (quint, *J* = 6.0 Hz, 2H), 2.50 (t, *J* = 6.0 Hz, 2H), 2.62 (t, *J* = 6.0 Hz, 2H), 4.02 (t, *J* = 7.8 Hz, 2H), 6.57 (s, 1H).

### f) Preparation of 3-(benzoxazole-2-yloxy)-1-butyl-5,6,7,8-tetrahydro-1H-quinoline-2-one (1-015)

1-Butyl-3-hydroxy-5,6,7,8-tetrahydro-1*H*-quinoline-2-one (1-015-05) (10 mg, 0.045 mmol) was dissolved in DMF (1 mL), and to the reaction mixture was added sodium hydride (60% oil suspension, 2.7 mg, 0.068 mmol). The reaction mixture was stirred at room temperature for 5 min, and to the reaction mixture was added 2-chlorobenzoxazole (7.7 µL, 0.068 mmol). The reaction mixture was stirred at room temperature for 20 min and 1 mol/L hydrochloric acid (3 mL) was added to the reaction mixture. The reaction mixture was extracted with ethyl acetate (50 mL), washed with aqueous saturated sodium hydrogen carbonate solution (8 mL) and brine (4 mL), dried over anhydrous magnesium sulfate, and evaporated under reduced pressure. The obtained residue was purified by silica gel column chromatography (toluene/ethyl acetate) to give 3-(benzoxazole-2-yloxy)-1-butyl-,5,6,7,8-tetrahydro-1*H*-quinoline-2-one (1-015) (12 mg, 74%) as a pale yellow powder.
¹H NMR (300 MHz, CDCl₃): δ 0.94 (t, *J* = 7.5 Hz, 3H), 1.40 (sextet, *J* = 7.5 Hz, 2H), 1.66 (quint, *J* = 7.5 Hz, 2H), 1.74 (quint, *J* = 6.0 Hz, 2H), 1.87 (quint, *J* = 6.0 Hz, 2H), 2.58 (t, *J* = 6.0 Hz, 2H), 2.69 (t, *J* = 6.0 Hz, 2H), 4.02 (t, *J* = 7.8 Hz, 2H), 7.16-7.26 (m, 2H), 7.24 (s, 1H), 7.40 (dd, *J* = 6.9 Hz, 2.4 Hz, 1H), 7.48 (dd, *J* = 6.9 Hz, 2.4 Hz, 1H).

### Example G

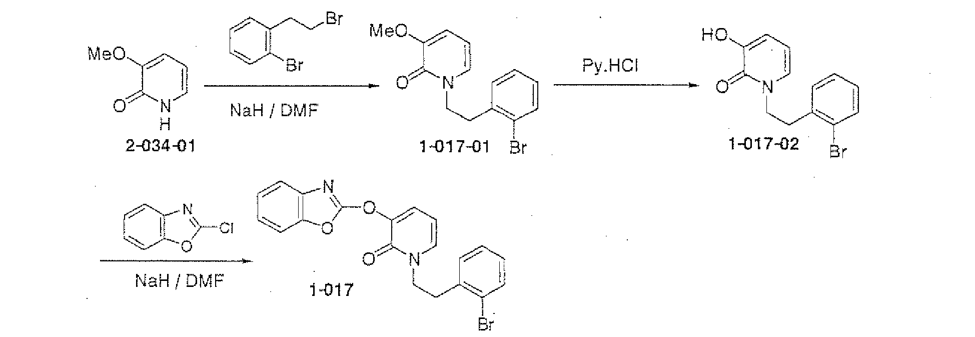

### a) Preparation of 1-(2-bromophenethyl)-3-methoxy-2-pyridone (1-017-01)

1-(2-Bromophenethyl)-3-methoxy-2-pyridone (1-017-01) (44%) was synthesized in a similar manner to the preparation of 2-034-02.
¹H NMR (300 MHz, CDCl₃): δ 3.22 (t, *J* = 7.3 Hz, 2H), 3.83 (s, 3H), 4.21 (t, *J* = 7.6 Hz, 2H), 5.96 (t, *J* = 7.3 Hz, 1H), 6.60 (m, 2H), 7.07-7.22 (m, 3H), 7.55 (d, *J* = 7.6 Hz, 1H).

### b) Preparation of 1-(2-bromophenethyl)-3-hydroxy-2-pyridone (1-017-02)

1-(2-Bromophenethyl)-3-hydroxy-2-pyridone (1-017-02) (100%) was synthesized in a similar manner to the preparation of 1-004-08.
¹H NMR (300 MHz, CDCl₃): δ 3.23 (t, *J* = 7.3 Hz, 2H), 4.23 (t, *J* = 7.3 Hz, 2H), 6.02 (t, *J* = 7.0 Hz, 1H), 6.57 (dd, *J* = 7.0, 1.2 Hz, 1H), 6.78 (dd, *J* = 7.3, 1.8 Hz, 1H), 7.08-7.14 (m, 2H), 7.18-7.23 (m, 1H), 7.56 (dd, *J* = 7.0, 1.2 Hz, 1H).

### c) Preparation of 3-(benzoxazol-2-yloxy)-1-(2-bromophenethyl)-3-hydroxy-2-pyridone (1-017)

3-(Benzoxazol-2-yloxy)-1-(2-Bromophenethyl)-3-hydroxy-2-pyridone (1-017-02) (70%) was synthesized in a similar manner to the preparation of 1-015.

### Example H

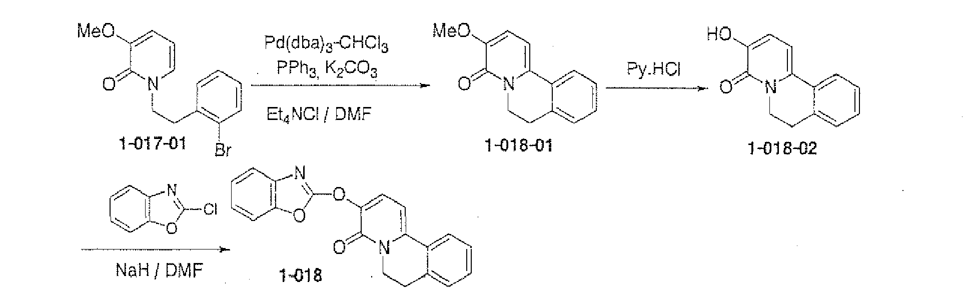

### a) Preparation of 3-methoxy-6,7-dihydroxypyrido[2,1,a]isoquinolin-4-one (1-018-01)

To a solution of 1-(2-bromophenethyl)-3-methoxy-2-pyridone (1-017-01) (100 mg) in DMF (4 mL) were added Pd(dba) · CHCl₃ (30 mg), Et₃NCl (54 mg), and potassium carbonate (67 mg) at room temperature. After the reaction mixture was stirred at 120 °C for 3 h, the solvent was evaporated. To the residue were added aqueous saturated ammonium chloride solution and ethyl acetate, and organic layer was separated. The aqueous layer was extracted three times with ethyl acetate, and combined organic layers were washed successively with successive water and brain, dried over anhydrous magnesium sulfate, and evaporated under reduced pressure. The obtained crude product was purified by preparative thin-layer chromatography (toluene/acetone=1/1) to give 3-methoxy-6,7-dihydroxypyrido[2,1,a]isoquinolin-4-one (1-018-01) (50.6 mg, 69%) as an oil.
¹H NMR (300 MHz, CDCl₃): δ 2.97 (t, *J* = 6.4 Hz, 2H), 3.88 (s, 3H), 4.34 (t, *J* = 6.3 Hz, 2H), 6.64 (d, *J* = 7.9 Hz, 1H), 6.74 (d, *J* = 7.9 Hz, 1H), 7.23-7.34 (m, 3H), 7.64-7.67 (m, 1H).

### b) Preparation of 3-hydroxy-6,7-dihydroxypyrido[2,1,a]isoquinolin-4-one (1-018-02)

3-Hydroxy-6,7-dihydroxypyrido[2,1,a]isoquinolin-4-one (1-018-02) (98%) was synthesized in a similar manner to the preparation of 1-004-08.
¹H NMR (300 MHz, CDCl₃): δ 3.00 (t, *J* = 6.6 Hz, 2H), 4.35 (t, *J* = 6.6 Hz, 2H), 6.71 (d, *J* = 7.8 Hz, 1H), 6.93 (d, *J* = 7.8 Hz, 1H), 7.24-7.34 (m, 3H), 7.64-7.68 (m, 1H).

### d) Preparation of 3-(benzoxazole-2-yloxy)-6,7-dihydroxypyrido[2,1,a]isoquinolin-4-one (1-018)

3-(Benzoxazole-2-yloxy)-6,7-dihydroxypyrido[2,1,a]isoquinolin-4-one (1-018) (47%) was synthesized in a similar manner to the preparation of 1-004.

### Example I

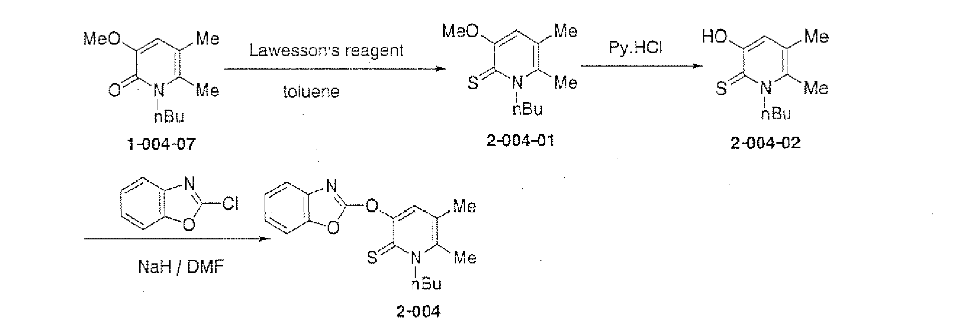

### a) Preparation of 1-butyl-5,6-dimethyl-3-methoxy-2-thiopyridone (2-004-01)

To a mixture of 1-butyl-5,6-dimethyl-3-methoxy-2-pyridone (1-004-07) (222 mg) and Lawesson's reagent (502 mg) was added toluene (8 mL), and the suspension was refluxed with stirring for 7 h under nitrogen atmosphere. To a reaction mixture was added methanol (25 mL) and the reaction mixture was stirred at room temperature for 1 h, and evaporated under reduced pressure. The residue (0.80 g) was purified by silica gel column chromatography (toluene/acetone-4/1) using by Lobar column B to give 1-butyl-5,6-dimethyl-3-methoxy-2-thiopyridone (2-004-01) (177 mg, 74.1%, m.p. 111-112 °C)
¹H NMR, (300 MHz, CDCl₃): δ 1.00 (t, *J* = 7.2 Hz, 3H), 1.43-1.55 (m, 2H), 1.70-1.95 (br s, 2H), 2.22 (s, 3H), 2.46 (s, 3H), 3.89 (s, 3H), 4.90 (br s, 2H), 6.54 (s, 1H).

### b) Preparation of N-1-butyl-5,6-dimethyl-3-hydroxy-2-thiopyridone (2-004-02)

*N*-1-Butyl-5,6-dimethyl-3-hydroxy-2-thiopyridone (2-004-02) (118 mg, 74.2%, m.p. 81-88°C) was synthesized from 2-004-01 (170 mg) in a similar manner to the preparation of 1-013-10.
¹H NMR (300 MHz, CDCl₃): δ 1.02 (t, *J* = 7.2 Hz, 3H), 1.45-1.57 (m, 2H), 1.70-1.90 (m, 2H), 2.21 (s, 3H), 2.45 (s, 3H), 4.72 (br s, 2H), 6.87 (s, 1H), 8.44 (br s, 1H).

### c) Preparation of 1-butyl-5,6-dimethyl-3-O-(benzoxazol-2-yl)-2-thiopyridone (2-004)

1-Butyl-5,6-dimethyl-3-O-(benzoxazol-2-yl)-2-thiopyridone (2-004) (84 mg, 45.9%, m.p. 185-187 °C) was synthesized from 2-004-02 (118 mg) in a similar manner to the preparation of Example 1-004.

### Example J

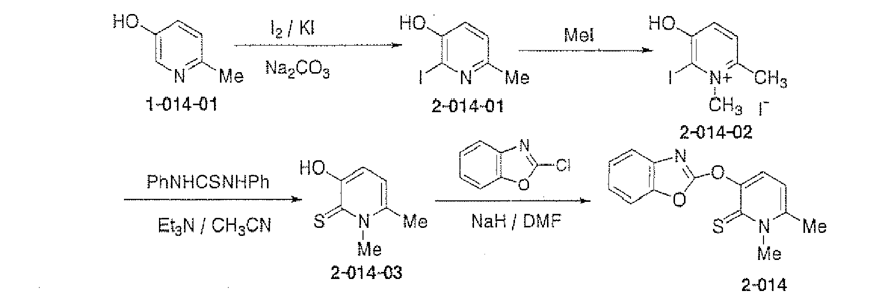

### a) Preparation of 2-iodo-3-hydroxy-6-methylpyridine (2-014-01)

Sodium carbonate (68.0 g) and water (810 mL) were added to 5-hydroxy-2-methylpyridine (1-014-01) (36.11 g), and the reaction mixture was stirred at room temperature, dissolved. To the reaction mixture was added dropwise a solution of iodine (117 g) and potassium iodide (117 g) in water (810 mL) for 35 min. The resulting pink-yellow crystal was filtered and dried under reduced pressure to give 2-iodo-3-hydroxy-6-methylpyridine (2-014-01) (34.1 g, 43.9%, m.p. 187-190 °C)
¹H-NMR (300MHz, CDCl₃+CD₃OD): δ 2.45 (s,3H), 6.45 (d, *J* = 6.9 Hz, 1H), 7.02 (dd, *J* = 6.6, 1.5 Hz, 1H)

### b) Preparation of 3-hydroxy-2-iodo-1,6-dimethylpyridinium iodide (2-014-02)

2-Iodo-3-hydroxy-6-methylpyridine (835 mg) and iodomethane (3 mL) were added in glass sealed cube, and the reaction mixture was reacted at 130°C for 4 h and at 180°C for 1 h. The reaction mixture was completely evaporated to give 3-hydroxy-2-iodo-1,6-dimethylpyridinium iodide (2-014-02) (1.42 g).

### c) Preparation of 1,6-diruethyl-3-hydroxy-2-thiopyridone (2-014-03)

To a solution of 3-hydroxy-2-iodo-1,6-dimethylpyridinium iodide (2-014-02) (852 mg) and triethylamine (457 mg) in acetonitrile (10 mL) was added 1,3-diphenylthiourea (517 mg), and the reaction mixture was refluxed for 2 h, and evaporated. The residue was purified by silica gel column chromatography (n-hexane/ethyl acetate) to give 1,6-dimethyl-3-hydroxy-2-thiopyridone (2-014-03) (279 mg).
¹H NMR (300 MHz, CDCl₃): δ 2.47 (s,3H),4.12 (s,3H), 6.53 (d, *J =* 8.1Hz, 1H), 6.95 (d, *J* = 8.1Hz, 1H), 8.35 (br s, 1H).

### d) Preparation of 3-(benzoxazol-2-yloxy)-1,6-dimethyl-1H-pyridine-2-thione (2-014)

1,6-Dimethyl-3-hydroxy-2-thiopyridone (2-014-03) (157 mg) was dissolved in DMF (3 mL), and to the reaction mixture was added 60% sodium hydride (52 mg), and the reaction mixture was reacted at room temperature for 7 min. 2-Chlorobenzoxazole (184 mg) was added to the reaction mixture by washing with DMF (0.5 mL), and the reaction mixture was reacted at room temperature for 2 h. The reaction mixture was repeatedly extracted with aqueous saturated ammonium chloride solution and ethyl acetate, and evaporated under reduced pressure. The residue was purified by silica gel column chromatography and recrystallized from chloroform to give 3-(benzoxazol-2-yloxy)-1,6-dimethyl-1*H*-pyridine-2-thione (2-014) (182 mg, 66.8%, m.p. 245-247 °C)

### Example K

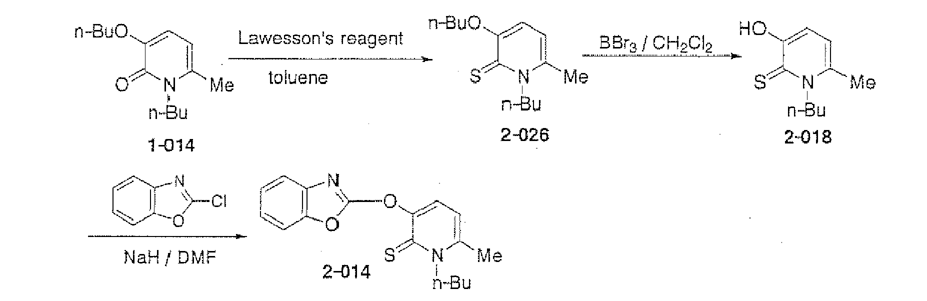

### a) Preparation of 1-butyl-3-butyl-6-methyl-2-thlopyridone (2-026)

1-Butyl-3-butyl-6-methyl-2-pyridone (1-014) (8.91 g) was dissloved in dry toluene (200 mL), and to the reaction mixture was added Lawesson's reagent (19.41 g), and the reaction mixture was reacted under reflux for 3.5 h under nitrogen atmosphere. To a reaction mixture was added methanol (80 mL) and the reaction mixture was stirred at room temperature for 1.5 h, and evaporated. The residual solution was purified by silica gel column chromatography (n-hexane/ethyl acetate) to give 1-butyl-3-butyl-6-methyl-2-thiopyridone (2-026) (12.97 g). The product was used at next reaction without further purification.
¹H-NMR. (300MHz, CDCl₃): δ 0.94 (t, *J* = 7.5Hz, 3H), 0.99 (t, *J* = 7.5Hz, 3H), 1.46 (m, 4H), 1.87 (m, 4H), 2.50 (s,3H), 3.98 (t, *J* = 6.9Hz, 2H), 4.75 (brs, 2H), 6.40 (d, *J* = 7.8Hz, 1H), 7.80 (d, *J* = 7.8Hz,1H)

### b) Preparation of 1-butyl-3-hydroxy-6-methyl-2-thiopyridone (2-018)

1-Butyl-3-butyl-6-methyl-2-thiopyridone (2-026) (12.97 g) was dissolved in dry methylene chloride (200 mL), and to the reaction mixture was slowly added a solution of 1 mmol/mL boron tribromide in methylene chloride (5.6 mL), and the reaction mixture was stirred at room temperature for 5 h. The reaction mixture was poured into ice and water, adjusted at pH 8 to 9 with conc. aqueous ammonia, extracted with chloroform, washed with brine, purified by alumina (150 g) column chromatography, eluted with chloroform to give 1-butyl-3-hydroxy-6-methyl-2-thiopyridone (2-018) (5.439 g, 73.4%) as an orange oil.
¹H-NMR (300MHz, CDCl₃): δ 1.02 (t, *J* = 7.8 Hz), 1.50 (m,2H), 1.85 (m,2H), 2.51 (s,3H), 4.66 (br s,2H), 6.49 (d, *J* = 7.8 Hz, 1H), 6.91 (d, *J* = 7.8 Hz, 1H),8.44(brs. 1H).

### c) Preparation of 1-butyl-3-(benzoxazol-2-yl)-6-methyl-2-thiopyridone (2-014)

1-Butyl-3-hydroxy-6-methyl-2-thiopyridone (2-018) (113 mg) was dissolved in dry DMF
(1.1 mL), and to the reaction mixture was added 60% sodium hydride (36 mg), and the reaction mixture was stirred at room temperature for 30 min. To the reaction mixture was added 2- chlorobenzoxazole (112 mg), and the reaction mixture was reacted for 2 h and 40 min. The reaction mixture was poured into ice-water (20 mL), extracted twice with ethyl acetate (30 mL), washed with brine, and evaporated. The residue was purified by preparative thin-layer chromatography using chloroform as a developing solvent to give 1-butyl-3-(benzoxazol-2-yl)-6-methyl-2-thiopyridone (2-014) (117 mg, m.p. 125-127.5 °C)

### Example L

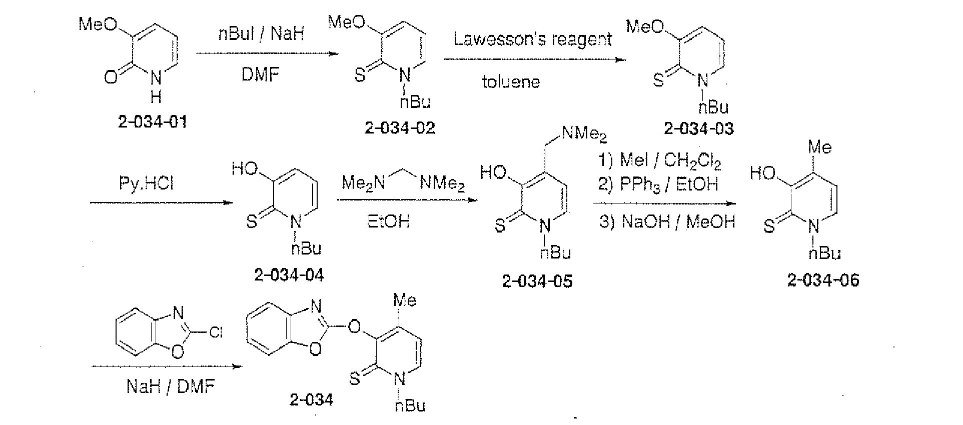

### a) Preparation of 1-butyl-3-methoxy-2-pyridone (2-034-02)

To a solution of 3-methoxy-2(1*H*)-pyridone (2-034-01) (5.0 g) in DMF (40 mL) was added sodium hydride (60%wt, 2.2 g) at room temperature. After the reaction mixture was stirred for 20 min, 1-iodobutane (15.5 g) was added to the reaction mixture, and the reaction mixture was stirred for 40 min. After the reaction was quenched with water, the solvent was removed. To the residue were added a saturated aqueous solution of ammonium chloride and ethyl acetate, and the organic layer was separated, and the aqueous layer was extracted three times with ethyl acetate. The combined organic layers were washed water and brine, dried over anhydrous magnesium sulfate, and evaporated under reduced pressure. The crude product was purified by silica gel column chromatography (toluene/acetone=4/1) to give 1-butyl-3-methony-2-pyridone (2-034-02) (6.7 g, 93%) as an oil.
¹H NMR (300 MHz, CDCl₃): 5 0.93 (t, *J* = 7.2 Hz, 2H), 1.30-1.42 (m, 2H), 1.68-1.78 (m, 2H), 3.81 (s, 3H), 3.97 (t, *J* = 7.2 Hz, 2H), 6.09 (t, *J* = 7.2 Hz, 1H), 6.59 (dd, *J* = 7.2, 1.5 Hz, 1H), 6.88 (dd, *J* = 7.2, 1.5 Hz, 1H).

### b) Preparation of 1-butyl-3-methoxypyridine-2-thione (2-034-03)

To a solution of 1-butyl-3-methoxy-2-pyridone (2-034-02) (6.4 g) in toluene (150 mL) was added Lawesson's reagent (16.8 g), and the reaction mixture was heated under reflux. After the reaction mixture was stirred for 3 h, to the reaction mixture was added methanol (100 mL), and the reaction mixture was stirred for 30 min. After the solvent was evaporated under reduced pressure, to the reaction mixture were added water and ethyl acetate, and the organic layer was separated. The aqueous layer was extracted three times with ethyl acetate, and the combined organic layers were washed with water and brine, dried over anhydrous magnesium sulfate, and evaporated under reduced pressure. The crude product was purified by silica gel column chromatography to give 1-butyl-3-methoxypyridine-2-thione (2-034-03) (5.6 g, 80%) as an oil.
¹H NMR (300 MHz, CDCl₃): δ 0.98 (t, *J* = 7.3 Hz, 3H), 1.36-1.48 (m, 2H), 1.84-1.94 (m, 2H), 3.92 (s, 3H), 4.62 (t, *J* = 7.6 Hz, 2H), 6.61 (dd, *J* = 7.9, 6.2 Hz, 1H), 6.69 (dd, *J* = 7.9, 1.2 Hz, 1H), 7.38 (dd, *J* = 6.2, 1.2 Hz, 1H).

### c) Preparation of 1-butyl-3-hydroxypyridine-2-thione (2-034-04)

Pyridine hydrochloride salt (3.6 g) was added to 1-butyl-3-methoxypyridine-2-thione (2-034-03) (1.4 g), and the reaction mixture was stirred at 190 °C for 40 min. To the reaction mixture were added water and ethyl acetate, and the organic layer was separated, and the organic layer was extracted three times with ethyl acetate. The combined organic layers were washed with water and brine, dried over anhydrous magnesium sulfate, and evaporated under reduced pressure to give 1-butyl-3-hydroxypyridine-2-thione (2-034-04) (1.02 g, 78%) as an oil.
¹H NMR (300 MHz, CDCl₃): δ 0.99 (t, *J* = 7.3 Hz, 3H), 1.43 (m, 2H), 1.91 (m, 2H), 4.53 (t, *J* = 7.6 Hz, 2H), 6.66 (dd, *J* = 7.6, 6.7 Hz, 1H), 6.97 (dd, *J* = 7.6, 1.2 Hz, 1H), 7.34 (dd, *J* = 6.7, 1.2 Hz, 1H), 8.61 (br s, 1H).

### d) Preparation of 1-butyl-3-hydroxy-4(N,Ndimethylaminomethyl)pyridine-2-thione (2-034-05)

To a solution of 1-butyl-3-hydroxypyridine-2-thione (2-034-04) (1.0 g) in ethanol containing 10% water (20 mL) was added *N*, *N*, *N*', *N*'-teteramethyldiaminomethane (1.70 g) at room temperature. After the reaction mixture was stirred at 75 °C for 24 h, the solvent was evaporated under reduced pressure to give 1-butyl-3-hydroxy-4(*N*,*N-*dimethylaminomethyl)pyridine-2-thione (2-034-05) (1.3 g, 95%) as an oil.
¹H NMR (300 MHz, CDCl₃): δ 0.99 (t, *J* = 7.3 Hz, 3H), 1.39-1.47 (m, 2H), 1.86-1.93 (m. 2H), 2.29 (s, 6H), 3.48 (s, 2H), 4.51 (t, *J* = *7.3* Hz, 2H), 6.87 (d, *J* = 6.7 Hz, 1H), 7.32 (d, *J* = 6.7 Hz, 1H).

### d) Preparation of 1-butyl-3-hydroxy-4-methylpyridine-2-thione (2-034-06)

To a solution of 1-butyl-3-hydroxy-4(*N*,*N*-dimethylaminomethy)pyridine-2-thione (2-034-05) (1.0 g) in methylene chloride (20 mL) was added iodomethane (2.1 g) at room temperature. After the reaction mixture was stirred for 1 h, the solvent was evaporated under reduced pressure. To the residue were added ethanol (20 mL) and triphenylphosphine (1.6 g), and the reaction mixture was stirred at 75°C for 20 h, and evaporated under reduced pressure. To the residue were added methanol (10 mL) and 1 mol/L aqueous sodium hydroxide solution (8 mL), and the reaction mixture was stirred at 60 °C for 2h, and evaporated under reduced pressure. The crude product was purified by silica gel column chromatography (toluene/acetone=4/1) to give 1-butyl-3-hydroxy-4-methylpyridine-2-thione (2-034-06) (0.57 g, 70%) as an oil.
¹H NMR (300 MHz, CDCl₃): δ 0.98 (t, *J* = 7.3 Hz, 3H), 1.36-1.48 (m, 2H), 1.84-1.94 (m, 2H), 2.25 (s, 3H), 4.50 (t, *J* = 7.6 Hz, 2H), 6.55 (d, *J* = 6.7 Hz, 1H), 7.25 (d, *J* = 6.7 Hz, 1H), 8.67 (s, 1H).

### f) Preparation of 3-(benzoxazol-2-yloxy)-1-butyl-4-methoxypyridine-2-thione (2-034)

To a solution of 1-butyl-3-hydroxy-4-methylpyridine-2-thione (2-034-06) (50 mg) in DMF (1.0 mL) was added sodium hydride (60%wt, 15 mg) at room temperature. After the reaction mixture was stirred for 20 min, to the reaction mixture was added 2-chlorobenzoxazole (85 mg), and the reaction mixture was stirred at 75°C for 17 h. After the reaction was quenched with water, the solvent was evaporated. To the residue were added aqueous saturated ammonium chloride solution and ethyl acetate, and the organic layer was separated, and the aqueous layer was extracted three times with ethyl acetate. The combined organic layers were washed water and brine, dried over anhydrous magnesium sulfate, and evaporated under reduced pressure. The crude product was purified by column chromatography (toluene/acetone=4/1) to give 3-(benzoxazol-2-yloxy)-1-butyl-4-methoxypyridine-2-thione (2-034) (73 mg, 92%) as a yellow crystal. The obtained crystal was purified by recrystallization from methylene chloride and diethyl ether.

### Example M

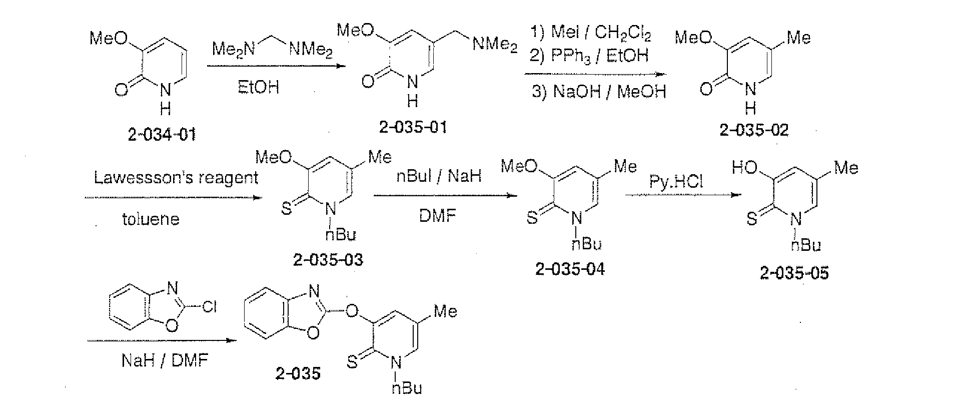

### a) Preparation of 5-(N,N-dimethylaminomethyl)-3-methoxy-2(1H)-pyridone (2-035-01)

To a solution of 3-methoxy-2(1*H*)-pyridone (2-034-01) (5.0 g) in ethanol containing 10% water (150 mL) was added *N, N, N', N*'-teteramethyldiaminomethane (54 mL) at room temperature, and the reaction mixture was heated under reflux. After the reaction mixture was stirred for 48 h, the solvent was removed under reduced pressure, and the obtained crude product was purified by silica gel column chromatography (chloroform/methanol/water=6/4/1) to give 5-(*N*,*N*-dimethylaminomethyl)-3-methoxy-2(1*H*)-pyridone (2-035-01) (4.5 g, 53%) as an oil.
¹H NMR (300 MHz, CDCl₃): δ 2.21 (s, 6H), 3.17 (s, 2H), 3.87 (s, 3H), 6.86 (d, *J* = 1.8 Hz, 1H), 6.90 (d, *J* = 1.8 Hz, 1H).

### b) Preparation of 3-methoxy-5-methyl-2(1H)-pyridone (2-035-02)

3-Methoxy-5-methyl-2(1*H*)-pyridone (2-035-02) (71%) was synthesized in a similar manner to the preparation of 2-034-06.
¹H NMR (300 MHz, CDCl₃): δ 2.11 (d, *J* = 1.2 Hz, 3H), 3.84 (s, 3H), 6.62 (d, *J* = 2.1 Hz, 1H), 6.80 (dd, *J* = 2.1, 1.2 Hz, 1H).

### c) Preparation of 1-butyl-3-methoxy-5-methyl-2-pyridone (2-035-03)

1-Butyl-3-methoxy-5-methyl-2-pyridone (2-035-03) (63%) was synthesized in a similar manner to the preparation of 2-034-02.
¹H NMR (300 MHz, CDCl₃): δ 0.94 (t, *J* = 7.3 Hz, 3H), 1.20-1.42 (m, 2H), 1.66-1.76 (m, 2H), 2.08 (d, *J* = 1.2 Hz, 3H), 3.80 (s, 3H), 3.92 (t, *J* = 7.3 Hz, 2H), 6.45 (d, *J* = 1.2 Hz, 1H), 6.65 (dd, *J* = 2.1,1.2 Hz, 1H).

### d) Preparation of 1-butyl-3-methoxy-5-methylpyridine-2-thione (2-035-04)

1-Butyl-3-methoxy-5-methylpyridine-2-thione (2-035-04) (100%) was synthesized in a similar manner to the preparation of 2-034-03.
¹H NMR (300 MHz, CDCl₃): δ 0.97 (t, *J* = 7.4 Hz, 3H), 1.35-1.48 (m, 2H), 1.53-1.93 (m, 2H), 2.21 (s, 3H), 3.91 (s, 3H), 4.59 (t, *J* = 7.7 Hz, 2H), 6.55 (s, 1H), 7.21 (s, 1H).

### e) Preparation of 1-butyl-3-hydroxy-5-methylpyridine-2-thione (2-035-05)

1-Butyl-3-hydroxy-5-methylpyridine-2-thione (2-035-05) (76%) was synthesized in a similar manner to the preparation of 2-034-04.
¹H-NMR (CDCl₃, 300 MHz): δ 0.99 (t, *J* = 7.3 Hz, 3H), 1.37-1.50 (m, 2H), 1.85-1.95 (m, 2H), 2.19 (d, *J* = 0.9 Hz, 3H), 4.49 (t, *J* = 7.6 Hz, 2H), 6.86 (d, *J* = 1.2 Hz, 1H), 7.16 (dd, *J* = 1.9, 0.9 Hz, 1H), 8.55 (s, 1H).

### f) Preparation of 3-(benzoxazol-2-yloxy)-1-butyl-5-methylpyridine-2-thione (2-035)

To a solution of 1-butyl-3-hydroxy-5-methylpyridine-2-thione (2-035-05) (300 mg) in DMF (6.0 mL) was added sodium hydride (60%wt, 79 mg) at room temperature. After the reaction mixture was stirred for 20 min, to the reaction mixture was added 2-chlorobanzoxazole (432 mg), and the reaction mixture was stirred at room temperature for 2 h. After the reaction was quenched with water, the solvent was removed. To the residue were added aqueous saturated ammonium chloride solution and ethyl acetate, and the organic layer was separated, and the aqueous layer was extracted three times with ethyl acetate. The combined organic layers were washed with water and brine, dried over anhydrous magnesium sulfate, and evaporated under reduced pressure. The crude product was purified by silica gel column chromatography (toruene/acetone=4/1) to give 3-(benzoxazol-2-yloxy)-1-butyl-4-methylpyridine-2-thione (2-035) (372 mg, 73%) as a yellow crystal. The obtained crystal was purified by recrystallization from methylene chloride and diethyl ether.

### Example N

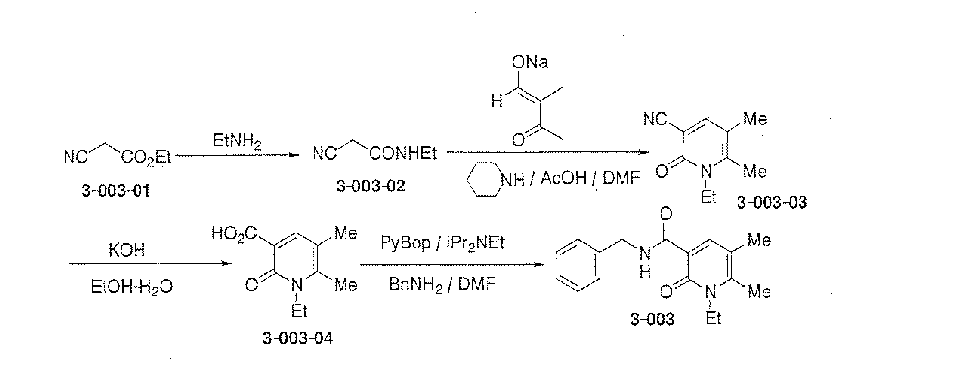

### a) Preparation of N-ethyl cyanoacetamide (3-003-02)

Aqueous 70% ethylamine solution (15.5 mL) was added dropwise to ethylcyanoacetete (3-003-01) (11.31 g) at room temperature. Since the inner temperature rose up to 44 °C, the reaction mixture was stirred at 32 to 37 °C for 15 min under ice-cooling. After the reaction mixture was stirred for 9 h at the same temperature, stood overnight. The reaction mixture was evaporated under reduced pressure, and to the obtained brown crystalloid residue (11.93 g) were diethyl ether (20 mL) and n-hexane (10 mL). The orange crystal was filtered to give *N*-ethyl cyanoacetamide (3-003-02) (9.05 g, 80.7%, m.p. 54-59 °C)
¹H NMR (300 MHz, CDCl₃): δ 1.20 (t, *J =* 7.2 Hz, 3H), 3.31-3.40 (m, 4H), 6.22 (br s, 1H).

### b) Preparation of 1-ethyl-3-cyano-5,6-dimethyl-2-pyridone (3-003-03)

To a suspension of 2-methyl-3-oxobutanal sodium salt (3.18 g) and *N-*ethylcyanoacetamide (3-003-02) (2.243 g) in DMF (20 mL) were added acetic acid (1.49 mL) and piperidine (0.40 mL) at room temperature, and the reaction mixture was refluxed with stirring in an oil bath at 135 °C for 5 h. The reaction mixture was dissolved in chloroform and water, extracted three times with chloroform, dried over magnesium sulfate, and evaporated under reduced pressure. The obtained crystal residue (4.07 g) was washed three times with n-hexane (15 mL) to give I-ethyl-3-cyano-5,6-dimethyl-2-pyridone (3-003-03) (3.38 g, 96%) as a brown crystal.
¹H NMR (300 MHz, CDCl₃): δ 1.33 (t, *J* = 7.2 Hz, 3H), 2.73 (s, 3H), 2.43 (s, 3H), 4.19 (q, *J* = 7.2 Hz, 2H), 7.59 (s, 1H).

### c) Preparation of 1-ethyl-3-carboxy-5,6-dimethyl-2-pyridone (3-003-04)

1-Ethyl-3-cyano-5,6-dimethyl-2-pyridone (3-003-03) (3.37 g) was dissolved in 80% ethanol (65 mL), and to the reaction mixture was added potassium hydroxide (7.96 g), and the reaction mixture was refluxed with stirring in oil bath at 102 °C for 24 h. The reaction mixture was evaporated under reduced pressure, and to the residue were added water (50 mL) and ethyl acetate (50 mL). After the reaction mixture was stirred under ice-cooling, and was separated by shaking, and to the aqueous layer was conc. hydrochloric acid (13 mL). The resulting crystal was filtered, and washed with cooled water to give 1-ethyl-3-carboxy-5,6-dimethyl-2-pyridone (3-003-04) (2.734 g, 73.3%, m.p. 164-165 °C) as a yellow ocher crystal.
¹H NMR (300 MHz, CDCl₃): δ 1.38 (t, *J* = 7.2 Hz, 3H), 2.20 (s, 3H), 2.49 (s, 3H), 4.28 (q, *J* = 7.2 Hz, 2H), 8.28 (s, 1H), 14.73 (br s, 1H).

### d) Preparation of 1-ethyl-2-oxo-5,6-dimethyl-1,2-dihydropyridine-3-carboxylic acid benzylamide (3-003)

1-Ethyl-3-carboxy-5,6-dimethyl-2-pyridone (3-003-04) (195 mg) was dissolved in DMF (3 mL), and to the reaction mixture were added benzylamine (0.17 mL), diisopropylethylamine (0.35 mL), and benzotriazol-1-yloxytripyrrolidinophosphonium hexafluorophosphate (PyBOP, 624 mg), and the reaction mixture was stirred at room temperature for 1h. The reaction mixture was diluted with ethyl acetate, washed twice with hydrochloric acid and aqueous odium bicarbonate solution, respectively, and water, dried over magnesium sulfate, and evaporated under reduced pressure. The residue (0.40 g) was subjected to silica gel (30 g) column chromatography (chloroform) to give 1-ethyl-2-oxo-5,6-dimethyl-1,2-dihydropyridine-3-carboxylic acid benzylamide (3-003) (259 mg, 91.1%) as a crystal, followed by recrystallization from methylene/n-hexane to give colorless needle crystal (207 mg, 72.9%, m.p. 117°C)

### Example O

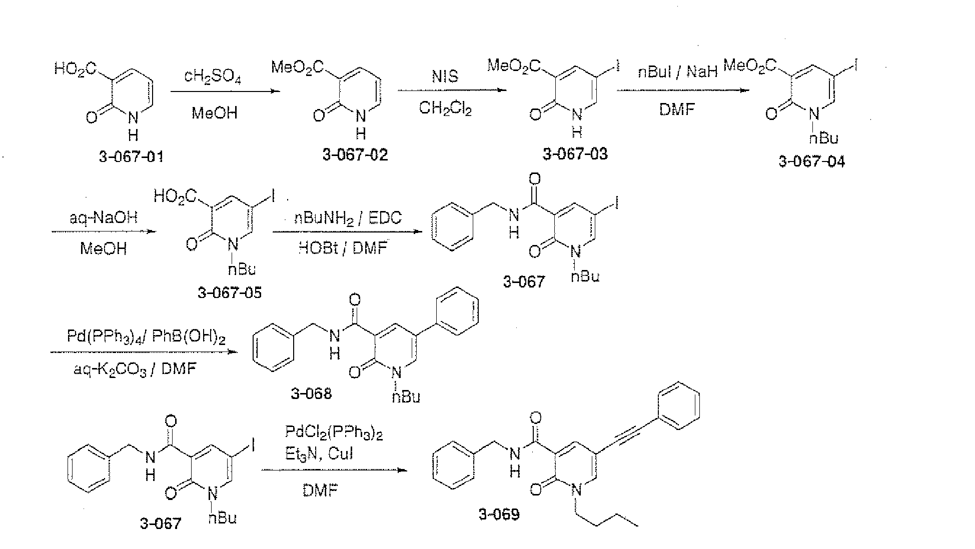

### a) Preparation of 2-hydroxynicotinic acid methyl ester (3-067-02)

To a solution of 2-hydroxynicotinic acid (3-067-01) (50 g) in methanol (500 ml) were added conc. sulfuric acid (15 mL) and toluene (100 mL) at room temperature. After the reaction mixture was stirred for 28 h attached Dienstark reflux tube and neutralized with an aqueous potassium carbonate solution, the solvent was evaporated. To the residue were added aqueous saturated ammonium chloride solution and chloroform, and the organic layer was separated, and the aqueous layer was extracted with chloroform. The combined organic layers were dried over anhydrous magnesium sulfate, and evaporated under reduced pressure to give 2-hydroxynicotinic acid methyl ester (3-067-02) (46 g, 84%) as a white solid.
¹H NMR (300 MHz, CDCl₃): δ 3.92 (s, 3H), 6.45 (dd, *J* = 7.3, 6.4 Hz, 1H), 7.78 (dd, *J* = 6.4, 2.4 Hz, 1H), 8.29 (dd, *J* = 7.3, 2.4 Hz, 1H).

### b) Preparation of 2-hydroxy-5-iodonicotinic acid methyl ester (3-067-03)

To a solution of 2-hydroxynicotinic acid methyl ester (3-067-02) (20 g) in methylene chloride (500 mL) was added *N*-iodosuccinimide (NIS, 38 g) at room temperature, and the reaction mixture was heated under reflux for 16 h, and evaporated. To the residue was added ethyl acetate (200 mL) and the reaction mixture was heated under reflux for 2 h. The insoluble solid was filtered to give 2-hydroxy-5-iodonicotinic acid methyl ester (3-067-03) (30 g, 81%) as a white solid.
¹H NMR (300 MHz, CDCl₃): δ 3.97 (s, 3H), 8.33 (brs, 1H), 8.43 (d, *J* = 2.4 Hz, 1H).

### c) Preparation of 1-butyl-5-iodo-2-oxo-1,2-dihydropridine-3-carboxylic acid methyl ester (3-067-04)

1-Butyl-5-iodo-2-oxo-1,2-dihydropridine-3-carboxylic acid methyl ester (3-067-04) (89%) was synthesized in a similar manner to the preparation of 2-034-02.
¹H NMR (300 MHz, CDCl₃): δ 0.96 (t, *J* = 7.4 Hz, 3H), 1.31-1.44 (m, 2H), 1.69-1.79 (m, 2H), 3.90 (s, 3H), 3.94 (t, *J* = 7.4 Hz, 2H), 7.71 (d, *J* = 2.8 Hz, 1H), 8.24 (d, *J* = 2.8 Hz, 1H).

### c) Preparation of 1-butyl-5-iodo-2-oxo-1,2-dihydropridine-3-carboxylic acid (3-067-05)

1-Butyl-5-iodo-2-oxo-1,2-dihydropridine-3-carboxylic acid (3-067-05) was synthesized in a similar manner to the preparation of 3-003-04.
¹H NMR (300 MHz, CDCl₃): δ 0.99 (t, *J* = 7.4 Hz, 3H), 1.35-1.47 (m, 2H), 1.74-1.84 (m, 2H), 4.05 (t, *J* = 7.5 Hz, 2H), 7.83 (d, *J* = 2.7 Hz, 1H), 8.63 (d, *J* = 2.7 Hz, 1H), 14.13 (s, 1H).

### d) Preparation of 1-butyl-5-iodo-2-oxo-1,2-dihydropridine-3-carboxylic acid benzylamide (3-067)

1-Butyl-5-iodo-2-oxo-1,2-dihydropridine-3-carboxylic acid benzylamide (3-067) (82%) was synthesized in a similar manner to the preparation of 3-003.

### e) Preparation of 1-butyl-2-oxo-5-phenyl-1,2-dihydropridine-3-carboxylic acid benzylamide (3-068)

To a solution of 1-butyl-5-iodo-2-oxo-1,2-dihydropridine-3-carboxylic acid benzylamide (3-067) (100 mg) in DMF (2.0 mL) were added Pd(PPh₃)₄ (20 mg), phenyl boric acid (89 mg), and an aqueous solution of potassium carbonate (2 mole/L, 0.24 mL) at room temperature. After stirred at 90 °C for 18 h, to the reaction mixture were added a saturated aqueous solution of ammonium chloride and ethyl acetate. The organic layer was separated and the aqueous layer was extracted three times with ethyl acetate, and the combined organic layers were washed with water and brine, dried over anhydrous magnesium sulfate, and evaporated under reduced pressure. The obtained crude product was purified by the preparative thin-layer chromatography (toluene/acetone=7/1) to give 1-butyl-2-oxo-5-phenyl-1,2-dihydropridine-3-carboxylic acid benzylamide (3-068) (77 mg, 88%) as an oil.

### f) Preparation of 1-butyl-2-oxo-5-phenylethynyl-1,2-dihydropridine-3-carboxylic acid benzylamide (3-069)

To a solution of 1-butyl-5-iodo-2-oxo-1,2-dihydropridine-3-carboxylic acid benzylamide (3-067) (78 mg) in DMF (2.0 mL) were added PdCl₂(PPh₃)₂ (15 mg), phenylacetylene (89 mg), CuI (11 mg), and triethylamine (48 mg) at room temperature. After stirred at 90 °C for 18 h, to the reaction mixture were added a saturated aqueous solution of ammonium chloride and ethyl acetate. The organic layer was separated and the aqueous layer was extracted three times with ethyl acetate, and the combined organic layers were washed with water and brine, dried over anhydrous magnesium sulfate, and evaporated under reduced pressure. The obtained crude product was purified by the preparative thin-layer chromatography (toluene/acetone=7/1) to give 1-butyl-2-oxo-5-phenylethynyl-1,2-dihydropridine-3-carboxylic acid benzylamide (3-069) (65 mg, 89%) as an oil.

### Example P

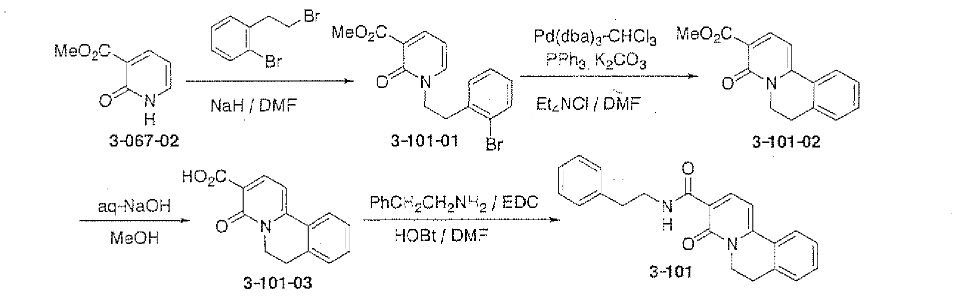

### a) Preparation of 2-hydroxy-1-(2-bromophenethyl)nicothinic acid methyl ester (3-101-01)

2-Hydroxy-1-(2-bromophenethyl)nicotinic acid methyl ester (3-101-01) (59%) was synthesized in a similar manner to the preparation of 2-034-02.
¹H NMR (300 MHz, CDCl₃): δ 3.25 (t, *J* = 7.2 Hz, 2H), 3.93 (s, 3H), 4.23 (t, *J* = 7.2 Hz, 2H), 6.09 (t, *J* = 7.5 Hz, 1H), 7.08-7.23 (m, 4H), 7.56 (dd, *J* = 8.1, 2.1 Hz, 1H), 8.15 (dd, *J* = 7.5, 2.4 Hz, 1H).

### b) Preparation of 4-oxo-6,7-dihydropyrido[2,1,a]isoquinoline-3-carboxylic acid methyl ester (3-101-02)

4-Oxo-6,7-dihydropyrido[2,1,a]isoquinoline-3-carboxylic acid methyl ester (3-101-02) (42 %) was synthesized in a similar manner to the preparation of 1-018-01.
¹H NMR (300 MHz, CDCl₃): δ 3.01 (t, *J* = 6.7 Hz, 2H), 3.93 (s, 3H), 4.35 (t, *J*=6.7 Hz, 2H), 6.76 (d, *J* = 7.6 Hz, 1H), 7.32 (dd, *J* = 7.3, 1.2 Hz, 1H), 7.39 (ddd, *J* = 7.6, 7.3, 1.5 Hz, 1H), 7.46 (ddd, *J* = 7.9, 7.6, 1.2 Hz, 1H), 7.78 (dd, *J* = 7.9, 1.5 Hz, 1H), 8.25 (d, *J* = 7.6 Hz, 1H).

### c) Preparation of 4-oxo-6,7-dihydropyrido[2,1,a]isoquinoline-3-carboxylic acid (3-101-03)

To a solution of 4-oxo-6,7-dihydropyrido[2,1,a]isoquinoline-3-carboxylic acid methyl ester (3-101-02) (252 mg) in dioxane (2.0 mL) was 2 mol/L aqueous sodium hydroxide solution (2.0 mL) at room temperature. After the reaction mixture was stirred for 1 h, and washed with diethyl ether, adjusted to be acidic with conc. hydrochloric acid. To the reaction mixture were added water and ethyl acetate, and the organic layer was separated. The aqueous layer was extracted three times with ethyl acetate and the combined organic layers were washed with water and brine, dried over anhydrous magnesium sulfate, and evaporated under reduced pressure to give 4-oxo-6,7-dihydropyrido[2,1,a]isoquinoline-3-carboxylic acid (3-101-03) (209 mg, 88%) as a white solid.
¹H NMR (300 MHz, CDCl₃): δ 3.10 (t, *J* = 6.7 Hz, 2H), 4.42 (t, *J* = 6.7 Hz, 2H), 7.05 (d, *J* = 7.6 Hz, 1H), 7.36 (d, *J* = 7.3 Hz, 1H), 7.41-7.56 (m, 2H), 7.84 (d, *J* = 7.3 Hz, 1H), 8.56 (d, *J* = 7.6 Hz, 1H), 14.40 (s, 1H).

### d) Preparation of 4-oxo-6,7-dihydropyrido[2,1,a]isoquinoline-3-carboxylic acid phenethylamide (3-101)

To a solution of 4-oxo-6,7-dihydropyrido[2,1,a]isoquinaline-3-carboxylic acid (3-101-03) (76 mg) in DMF (2.0 mL) were added 1-ethyl-3-(3-dimethylaminopropyl)carbodiimide hydrochloric acid salt (EDC, 83 mg), 1-hydroxybenzotriazole (HOBt, 58 mg), and phenethylamine (80 mg) at room temperature. After the reaction mixture was stirred for 18 h, the reaction was quenched with 0.5 mol/L hydrochloric acid. To the reaction mixture was added ethyl acetate and the organic layer was separated. The aqueous layer was extracted three times with ethyl acetate, and the combined organic layers were washed with water and brine, dried over anhydrous magnesium sulfate, and evaporated under reduced pressure. The obtained crude crystal was recrystallized to give 4-oxo-6,7-dihydropyrido[2,1,a]isoquinoline-3-carboxylic acid phenethylamide (3-101) (84 mg, 74%) as an yellow crystal.

### Example Q

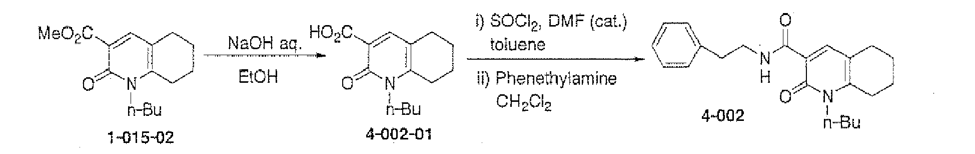

### a) Preparation of 1-butyl-2-oxo-1,2,5,6,7,8-hexahydroquinoline-3-carboxylic acid (4-002-01)

1-Butyl-2-oxo-1,2,5,6,7,8-hexahydroquinoline-3-carboxylic acid ethyl ester (1-015-02) (263 mg, 1 mmol) was dissolved in ethanol (6 mL), and to the reaction mixture was added an aqueous solution of sodium hydroxide (2 mol/L, 0.6 mL, 1.2 mmol), and the reaction mixture was stirred at room temperature for 30 min. To the reaction mixture was added diluted hydrochloric acid (0.4 mol/L, 6 mL) and the reaction mixture was extracted with ethyl acetate (25 mL). To the aqueous layer was added sodium chloride, followed by extraction with ethyl acetate (25 mL), and the combined organic layers were dried over anhydrous magnesium sulfate, and evaporated under reduced pressure. The obtained crystal residue was recrystallized from hexane to give 1-butyl-2-oxo-1,2,5,6,7,8-hexahydroquinoline-3-carboxylic acid (4-002-01) (220 mg, 88%) as a white crystal.
¹H NMR (300 MHz, CDCl₃): δ 1.00 (t, *J* = 7.5 Hz, 3H), 1.46 (sextet, *J* = 7.5 Hz, 2H), 1.68-1.73 (m, 2H), 1.77 (quint, *J* = 6.0 Hz, 2H), 1.92 (quint, *J* = 6.0 Hz, 2H), 2.65 (t, *J* = 6.0 Hz, 2H), 2.80 (t, *J* = 6.0 Hz, 2H), 4.10 (t, *J* = 7.8 Hz, 2H), 8.22 (s, 1H), 14.82 (s, 1H).

### b) Preparation of 1-butyl-2-oxo-1,2,5,6,7,8-hexahydroquinoline-3-carboxylic acid phenethylamide (4-002)

1-Butyl-2-oxo-1,2,5,6,7,8-hexahydroquinoline-3-carboxylic acid (4-002-01) (100 mg, 0.38 mmol) was dissloved in toluene (10 mL), and to the reaction mixture were added thionyl chloride (83 µL, 1.14 mmol) and catalytic amount of DMF, and the reaction mixture was reacted at 75 °C for 30 min. The reaction mixture was evaporated under reduced pressure and the residue was dissolved in methylene chloride (5 mL). To the reaction mixture was added phenethylamine (143 µL, 1.14 mmol) and the reaction mixture was stirred at room temperature for 10 min. To the reaction mixture was added diluted hydrochloric acid (1 mol/L, 10 mL) and the reaction mixture was extracted with ethyl acetate (30 mL), washed with brine (10 mL), dried over anhydrous magnesium sulfate, and evaporated under reduced pressure. The residue was purified by silica gel column chromatography (toluene/ethyl acetate), followed by recrystallization from diethyl ether to give 1-butyl-2-oxo-1,2,5,6,7,8-hexahydroquinoline-3-carboxylic acid phenethylamide (4-002) (100 mg, 74%) as white crystal.
¹H NMR (300 MHz, CDCl₃): δ 0.99 (t, *J* = 7.5 Hz, 3H), 1.45 (sextet, *J* = 7.5 Hz, 2H), 1.63 (quint, *J* = 7.5 Hz, 2H), 1.74 (quint, *J* = 6.0 Hz, 2H), 1.88 (quint, *J* = 6.0 Hz, 2H), 2.62 (t, *J* = 6.0 Hz, 2H), 2.74 (t, *J* = 6.0 Hz, 2H), 2.93 (t, *J* = 7.8 Hz, 2H), 3.66 (dt, *J* = 9.0 Hz, 6.0 Hz, 2H), 4.03 (t, *J* = 7.8 Hz, 2H), 7.20-7.33 (m, 5H), 8.25 (s, 1H), 10.05 (br t, *J* = 6.0 Hz, 1H).

### Example R

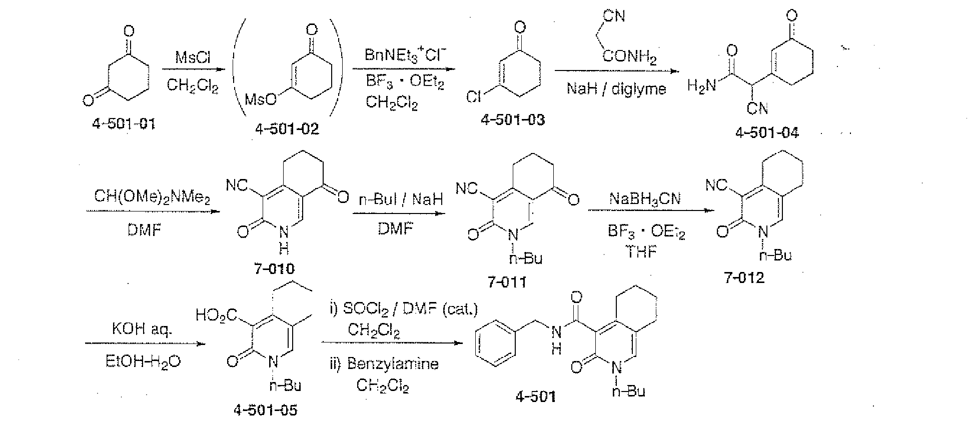

a) Preparation of 3-chloro-2-cyclohexene-1-one (4-501-03)
   1,3-cyclohexanedione (4-501-01) (8.72 g, 77.6 mmol) was dissolved in methylene chloride (400 mL) and to the reaction mixture were added methanesulfonyl chloride (6 mL, 77.6 mmol) and potassium carbonate (32 g, 232 mmol), and the reaction mixture was stirred at room temperature for 2 h. The reaction mixture was poured into a mixture of methylene chloride (1.4 L) and water (400 mL), separated, and the organic layer was washed with brine (400 mL), dried over anhydrous magnesium sulfate, and evaporated until the total amount became 300 mL under reduced pressure. To a solution of the mesylate derivative (4-501-02) were benzyltriethylammonium chloride (25 g, 110 mmol) and boron trifluoride diethyl ether complex (1.9 mL, 15.4 mmol), and the reaction mixture was stirred at room temperature for 1.5 h, poured into a mixture of methylene chloride (0.8 L) and water (400 mL), and separated. The organic layer was washed with brine (400 mL), dried over anhydrous magnesium sulfate, and evaporated under reduced pressure. The obtained residue was subjected to silica gel column chromatography (toluene/ethyl acetate) to give 3-chloro-2-cyclohexene-1-one (4-501-03) (7.24 g, 72%) as an yellow oil.
   ¹H NMR (300 MHz, CDCl₃): δ 2.09 (quint, *J* = 6.0 Hz, 2H), 2.40 (t, *J* = 6.6 Hz, 2H), 2.69 (td, *J* = 6.0 Hz, 1.5 Hz, 2H), 6.23 (t, *J* = 1.5 Hz, 1H).
b) Preparation of 3-cyanoacetamide-2-cyclohexene-1-one (4-501-04)
   2-Cyanoacetamide (4.42 g, 52.8 mmol) was dissolved in diglyme (50 mL) and to the reaction mixture was added sodium hydride (60% oil suspension, 2.1 g, 52.8 mmol), and the reaction mixture was vigorously stirred at room temperature for 5 min. To the reaction mixture was gradually added a solution of 3-chloro-2-cyclohexen-1-one (4-501-03) (6.24 g, 48 mmol) in diglyme (60 mL), and the reaction mixture was stirred at room temperature for 2.5 h. Then to the reaction mixture were 2-cyanoacetamide (1.6 g, 19.2 mmol) and sodium hydride (60% oil suspension, 0.76 g, 19.2 mmol), and the reaction mixture was stirred at room temperature for 1.5 h. To the reaction mixture was added diluted hydrochloric acid (1 mol/L, 100 mL) and the reaction mixture was extracted with ethyl acetate (300 mL). To the aqueous layer was added sodium chloride, followed by extraction with ethyl acetate (300 mL). The combined organic layers were dried over anhydrous magnesium sulfate, and evaporated under reduced pressure. The obtained crystal residue purified by silica gel column chromatography (toluene/ethyl acetate), followed by recrystallization from hexane to give 3-cyanoacetamide-2-cyclohexene-1-one (4-501-04) (6.5 g, 76%) as a white crystal.
   ¹H NMR (300 MHz, *d₆*-DMSO) : δ 1.71 (quint, *J* = 6.0 Hz, 2H), 1.79 (quint, *J* = 6.0 Hz, 2H), 2.78 (t, *J* = 6.0 Hz, 2H), 5.90 (s, 1H), 6.90 (s, 1H), 11.16 (br d, *J* = 1.5 Hz, 2H).
c) Preparation of 3,8-dioxo-2,3,5,6,7,8-hexahydroisoquinoline-4-carbonitrile (7-010)
   3-Cyanoacetamide-2-cyclohexene-1-one (4-501-04) (1.25 g, 7 mmol) was dissolved in DMF (25 mL) and to the reaction mixture was added *N*, *N-*dimethylformamidedimethylacetal (1.1 mL, 8.4 mmol), and the reaction mixture was stirred at room temperature for 70 h. To the reaction mixture was added diluted hydrochloric acid (1 mol/L, 100 mL), the reaction mixture was extracted with ethyl acetate (300 mL). To the aqueous layer was added sodium chloride, followed by extraction with ethyl acetate (300 mL). The combined organic layers were dried over anhydrous magnesium sulfate, and evaporated under reduced pressure. The obtained crystal residue purified by silica gel column chromatography (toluene/ethyl acetate), followed by recrystallized from toluene to give 3,8-dioxo-2,3,5,6,7,8-hexahydroisoquinoline-4-carbonitrile (7-010) (0.92 g, 70%) as white crystal.
   ¹H NMR (300 MHz, CDCl₃ + (a small amount of CD₃OD)) : δ 2.17 (quint, *J* = 6.3 Hz, 2H), 2.63 (t, *J* = 6.3 Hz, 2H), 3.09 (t, *J* = 6.3 Hz, 2H), 8.34 (s, 1H).
d) Preparation of 2-butyl-3,8-dioxo-2,3,5,6,7,8-hexahydroisoquinoline-4-carbonitrile (7-011)
   3,8-Dioxo-2,3,5,6,7,8-hexahydroisoquinoline-4-carbonitrile (7-010) (770 mg, 4.1 mmol) was dissolved in DMF (15 mL) and to the reaction mixture was added 1-iodobutane (0.51 mL, 4.5 mmol) and sodium hydride (60% oil suspension, 180 mg, 4.5 mmol), and the reaction mixture was stirred at room temperature for 3 h. To the reaction mixture was added dilute hydrochloric acid (1 mol/L, 60 mL) and the reaction mixture was extracted with ethyl acetate (150 mL), washed with brine (50 mL), dried over anhydrous magnesium sulfate, and evaporated under reduced pressure. The obtained residue was subjected to silica gel column chromatography (toluene/ethyl acetate) to give 2-butyl-3,8-dioxo-2,3,5,6,7,8-hexahydroisoquinoline-4-carbonitrile (7-011) (610 mg, 61%) as a white crystal.
   ¹H NMR (300 MHz, CDCl₃): δ 0.97 (t, *J* = 7.5 Hz, 3H), 1.38 (sextet, *J* = 7.5 Hz, 2H), 1.76 (quint, *J* = 7.5 Hz, 2H), 2.15 (quint, *J* = 6.3 Hz, 2H), 2.61 (t, *J* = 6.3 Hz, 2H), 3.06 (t, *J* = 6.3 Hz, 2H), 4.03 (t, *J* = 7.5 Hz, 2H), 8.39 (s, 1H).
   e) Preparation of 2-butyl-3-oxo-2,3,5,6,7,8-hexahydroisoquinoline-4-carbonitrile (7-012) 2-Butyl-3,8-dioxo-2,3,5,6,7,8-hexahydroisoquinoline-4-carbonitrile (7-011) (100 mg, 0.41 mmol) was dissolved in THF (7 mL) and to the reaction mixture were added boron trifluoride diethyl ether complex (0.21 mL, 1.64 mmol) and sodium cyanoborohydried (90 mg, 1.44 mmol), and the reaction mixture was stirred at room temperature for 30 min. To the reaction mixture was added an saturated aqueous solution of sodium hydrogencarbaonate (30 mL) and the reaction mixture was extracted with ethyl acetate (60 mL), washed with brine (30 mL), dried over anhydrous magnesium sulfate, and evaporated under reduced pressure. The obtained residue was subjected to silica gel column chromatography (toluene/ethyl acetate) to give 2-butyl-3-oxo-2,3,5,6,7,8-hexahydroisoquinoline-4-carbonitrile (7-012) (70 mg, 74%) as a white solid.
   ¹H NMR (300 MHz, CDCl₃): δ 0.96 (t, *J* = 7.5 Hz, 3H), 1.37 (sextet, *J* = 7.5 Hz, 2H), 1.67-1.86 (m, 6H), 2.54 (t, *J* = 6.3 Hz, 2H), 2.87 (t, *J* = 6.3 Hz, 2H), 3.93 (t, *J* = 7.5 Hz, 2H), 7.22 (s, 1H).
f) Preparation of 2-butyl-3-oxo-2,3,5,6,7,8-hexahydroisoquinoline-4-carboxylic acid (4-501-05)
   2-Butyl-3-oxo-2,3,5,6,7,8-hexahydroisoquinoline-4-carbonitrile (7-012) (260 mg, 1.13 mmol) was dissolved in water (6 mL)/ethanol (26 mL), to the reaction mixture was added potassium hydroxide (444 mg, 7.91 mmol), and the reaction mixture was heated under reflux for 24 h, and then cooled under ice-cooling. To the reaction mixture was added dropwise diluted hydrochloric acid (2 mol/L, 8 mL) and the reaction mixture was extracted with ethyl acetate (70 mL), dried over anhydrous magnesium sulfate, and evaporated under reduced pressure. The crystal residue was recrystallized from hexane to give 2-butyl-3-oxo-2,3,5,6,7,8-hexahydroisoquinoline-4-carboxylic acid (4-501-05) (197 mg, 70%) as a white crystal.
   ¹H NMR (300 MHz, CDCl₃): δ 0.97 (t, *J* = 7.5 Hz, 3H), 1.37 (sextet, *J* = 7.5 Hz, 2H), 1.70-1.82 (m, 6H), 2.56 (t, *J* = 6.0 Hz, 2H), 2.87 (t, *J* = 6.0 Hz, 2H), 3.95 (t, *J* = 7.5 Hz, 2H), 7.27 (s, 1H).
g) Preparation of 2-butyl-3-oxo-2,3,5,6,7,8-hexahydroisoquinoline-4-carboxylic acid benzylamide (4-501)
   2-Butyl-3-oxo-2,3,5,6,7,8-hexahydroisoquinoline-4-carboxylic acid (4-501-05) (5 mg, 0.02 mmol) was dissolved in toluene (1 mL), and to the reaction mixture were added thionyl chloride (4.4 µL, 0.06 mmol) and catalytic amount of DMF, and the reaction mixture was reacted at 75 °C for 30 min. After concentration of the reaction mixture under reduced pressure, the residue was dissolved in methylene chloride (1 mL), and then benzylamine (6.2 µL, 0.06 mmol) was added to the reaction mixture, and the reaction mixture was stirred at room temperature for 10 min. To the reaction mixture was added diluted hydrochloric acid (1 mol/L, 3 mL), and the reaction mixture was extracted with ethyl acetate (8 mL), washed with brine (4 mL), dried over anhydrous magnesium sulfate, and evaporated under reduced pressure. The obtained residue was purified by silica gel column chromatography (toluene/ethyl acetate) to give 2-butyl-3-oxo-2,3,5,6,7,8-hexahydroisoquinoline-4-carboxylic acid benzylamide (4-501) (5 mg, 74%) as a white crystal.
   ¹H-NMR (CDCl₃, 300 MHz): δ 0.95 (t, *J* = 7.5 Hz, 3H), 1.37 (sextet, *J* = 7.5 Hz, 2H), 1.66-1.77 (m, 6H), 2.57 (br t, *J* = 6.3 Hz, 2H), 3.27 (br t, *J* = 6.3 Hz, 2H), 3.92 (t, *J* = 7.5 Hz, 2H), 4.60 (d, *J* = 5.7 Hz, 2H), 7.12 (s, 1H), 7.23-7.40 (m, 5H), 9.58 (br t, *J* = 5.7 Hz, 1H).

### Example S

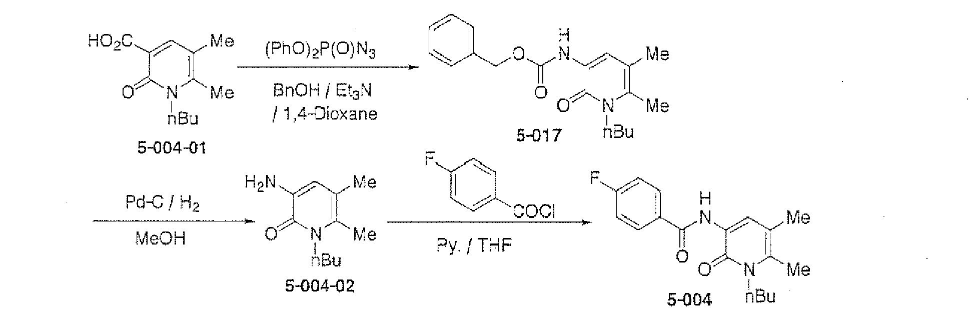

### a) Preparation of 3-benzyloxycarbonylamino-1-butyl-5,6-dimethyl-2-pyridone (5-017)

1-Butyl-3-carboxy-5,6-dimethyl-2-pyridone (5-004-01) (2.233 g) was dissolved in dioxane (50 mL), and to the reaction mixture were added triethylamine (4.2 mL) and diphenylphosphoryl azide (2.4 mL), and the reaction mixture was refluxed with stirring in an oil bath at 110 °C under nitrogen atmosphere. After 4 h, the reaction mixture was poured into ice-water, and to the reaction mixture were added ethyl acetate and aqueous hydrochloric acid solution, and then shaken to separate. The organic layer was washed each once with an aqueous solution of sodium bicarbonate and water, dried over magnesium sulfate, and evaporated under reduced pressure. The obtained residue was purified by silica gel column chromatography (Lobar column B, toluene/acetone=29/1) to give 3-benzyloxycarbonylamino-1-butyl-5,6-dimethyl-2-pyridone (5-017) (2.477 g, 75.4%, m.p. 65 to 66 °C) as an yellow crystal.

### b) Preparation of 3-amino-1-butyl-5,6-dimethyl-2-pyridone (5-004-02)

3-Benzyloxycarbonylamino-1-butyl-5,6-dimethyl-2-pyridone (5-017) (2.487 g) was dissolved in methanol (25 mL), and to the reaction mixture was added a suspension of 10% palladium on carbon (373 mg) in water (2.5 mL), and the reaction mixture was reacted in catalytic reduction under atmospheric pressure. After 4 h, the reaction mixture filtered off on Celite, washed with methanol, and evaporated under reduced pressure to give 3-amino-1-butyl-5,6-dimethyl-2-pyridone (5-004-02) (1.438 g, 97.8%, m.p. 94 to 97 °C) as a brown crystal.
¹H NMR (300 MHz, CDCl₃): δ 0.97 (t, *J* = 7.2 Hz, 3H), 1.37-1.49 (m, 2H), 1.60-1.71 (m, 2H), 4.08 (d, *J =* 7.8 Hz, 2H), 6.42 (s, 1H).

### c) Preparation of N-1-butyl-3-(4-fluorobenzoyl)amino-5,6-dimethyl-2-pyridone (5-004)

3-Amino-1-butyl-5,6-dimethyl-2-pyridone (5-004-02) (117 mg) was dissolved in pyridine (1 mL), and the reaction mixture was stirred under ice-cooling and nitrogen atmosphere, and to the reaction mixture was added dropwise a solution of 4-fluorobenzoyl chloride (0.08 mL) in tetrahydrofuran (1 mL) over 10 min, followed by stirring at the same condition. After 3 h, the reaction mixture was diluted with ethyl acetate and poured into ice-water, extracted once with ethyl acetate, washed with aqueous hydrochloric acid solution, water, an aqueous solution of sodium bicarbonate, and water, dried over magnesium sulfate, and evaporated under reduced pressure. The obtained crystal residue (202 mg) was recrystallized from methylene chloride/n-hexane to give 1-butyl-3-(4-fluo-robenzoyl)amino-5,6-dimethyl-2-pyridone (5-004) (103 mg, 54.2%, m.p. 129 to 130 °C) as a colorless needle.

### Example T

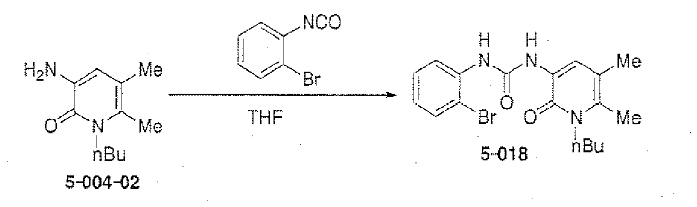

### a) Preparation of 4-(benzoxazol-2-yloxy)-1-benzyl-3-methoxy-5,6-dimethyl-1H-pyridin-2-one (5-018)

2-Bromophenylisocyanate (80 mg) was dissolved in tetrahydrofuran (2 mL), and the reaction mixture was stirred at room temperature under nitrogen atmosphere, and to the reaction mixture was added dropwise a solution of 3-amino-1-butyl-5,6-dimethyl-2-pyridone (5-004-02) (78 mg) in teterahydrofuran (2 mL) over 10 min, and the reaction mixture was stirred at room temperature overnight. The reaction mixture was evaporated under reduced pressure, and the obtained crystal was recrystallized from dichloromethne/diethyl ether to give 4-(benzoxazol-2-yloxy)-1-benzyl-3-methoxy-5,6-dimethyl-1*H*-pyridin-2-one (5-018) (142 mg, 89.9%, m.p. 197 to 198 °C).

### Example U

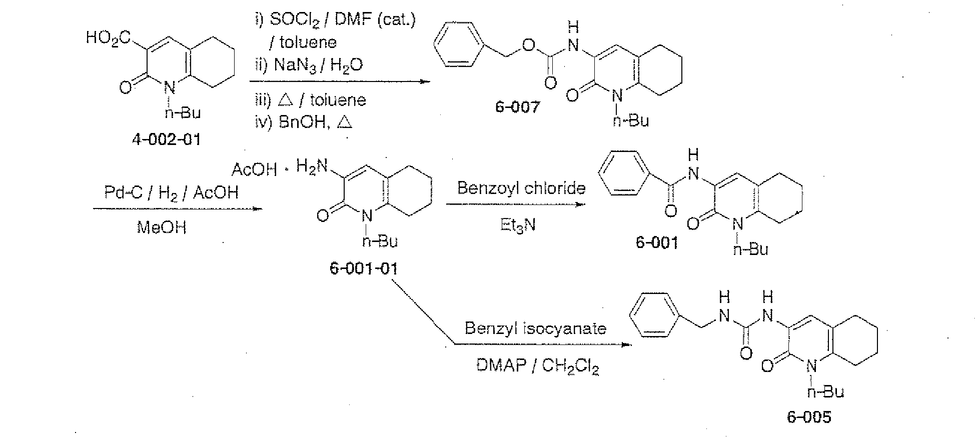

### a) Preparation of (1-butyl-2-oxo-1,2,5,6,7,8-hexahydroquinolin-3-yl)carbamic acid benzyl ester (6-007)

1-Butyl-2-oxo-1,2,5,6,7,8-hexahydroquinoline-3-carboxylic acid (4-002-01) (100 mg, 0.38 mmol) was dissolved in toluene (5 mL), and to the reaction mixture were added thionyl chloride (57 µL, 0.76 mmol) and catalytic amount of DMF, and the reaction mixture was reacted at 75 °C for 30 min. The reaction mixture was concentrated under reduced pressure, and the residue was dissolved in acetone (5 mL), and then to the reaction mixture was added an aqueous solution of sodium azide (29 mg, 0.42 mmol) (0.5 mL), and the reaction mixture was stirred at room temperature for 15 min. To the reaction mixture was added water (5 mL), the reaction mixture was extracted with ethyl acetate (10 mL), washed with brine (5 mL), dried over anhydrous magnesium sulfate, and evaporated under reduced pressure. The residue was dissolved in toluene (5 mL), and the reaction mixture was reacted at 120 °C for 30 min, and then benzyl alcohol (46 µL, 0.44 mmol) was added to the reaction mixture, and the reaction mixture was stirred at 120 °C for 2 h. The reaction mixture was purified by silica gel column chromatography (toluene/ethyl acetate) to give (1-butyl-2-oxo-1,2,5,6,7,8-hexahydroquinolin-3-yl)carbamic acid benzyl ester (6-007) (90 mg, 63%) as a white foamy substance.
¹H NMR (300 MHz, CDCl₃): δ 0.96 (t, *J* = 7.5 Hz, 3H), 1.41 (sextet, *J* = 7.5 Hz, 2H), 1.63 (quint, *J* = 7.5 Hz, 2H), 1.70 (quint, *J* = 6.0 Hz, 2H), 1.83 (quint, *J* = 6.0 Hz, 2H), 2.53 (t, *J* = 6.0 Hz, 2H), 2.63 (t, *J* = 6.0 Hz, 2H), 4.01 (t, *J* = 7.8 Hz, 2H), 5.19 (s, 2H), 7.29-7.41 (m, 5H), 7.76 (s, 1H), 7.86 (br s, 1H).

### b) Preparation of 3-amino-1-butyl-5,6,7,8-tetrahydroquinolin-2-one acetic acid salt (6-001-01)

(1-Butyl-2-oxo-1,2,d,6,7,8-hexahydroquinolin-3-yl)carbamic acid benzyl ester (6-007) (100 mg, 0.28 mmol) was dissolved in methanol (7 mL), and to the reaction mixture were added acetic acid (16 µL, 0.28 mmol) and palladium on carbon (10%, 30 mg), and the reaction mixture was stirred vigorously under hydrogen atmosphere for 1.5 h. Palladium on carbon was filtered off, and the filtrate was concentrated under reduced pressure, and the crystalloid residue was recrystallized from hexane to give 3-amino-1-butyl-5,6,7,8-tetrahydro-1*H*-quinolin-2-one acetic acid salt (6-001-01) (60 mg, 76%) as a white crystal.
¹H NMR (300 MHz, CDCl₃): 5 0.98 (t, *J* = 7.5 Hz, 3H), 1.43 (sextet, *J* = 7.5 Hz, 2H), 1.67 (quint, *J* = 7.5 Hz, 2H), 1.76 (quint, *J* = 6.0 Hz, 2H), 1.88 (quint, *J* = 6.0 Hz, 2H), 2.05 (s, 3H), 2.58 (t, *J* = 6.0 Hz, 2H), 2.67 (t, *J* = 6.0 Hz, 2H), 4.04 (t, *J* = 7.8 Hz, 2H), 8.27 (s, 1H).

### c) Preparation of N-(1-butyl-2-oxo-1,2,5,6,7,8-hexahydroquinolin-3-yl)banzamide (6-001)

3-Amino-1-butyl-5,6,7,8-tetrahydroquinolin-2-one acetic acid salt (6-001-01) (5 mg, 0.018 mmol) was dissolved in methylene chloride (1 mL), and to the reaction mixture were added benzoyl chloride (2.3 µL, 0.02 mmol) and triethylamine (5.6 µL, 0.04 mmol), followed by stirring at room temperature for 10 min. To the reaction mixture was added diluted hydrochloric acid (0.1 mol/L, 3 mL), and the reaction mixture was extracted with ethyl acetate (10 mL), washed with brine (3 mL), dried over anhydrous magnesium sulfate, and evaporated under reduced pressure. The obtained residue was purified by silica gel column chromatography (toluene/ethyl acetate) to give *N*-(1-butyl-2-oxo-1,2,5,6,7,8-hexahydroquinolin-3-yl)banzamide (6-001) (4.9 mg, 83%) as a white foamy crystal.
¹H NMR (300 MHz, CDCl₃): δ 0.99 (t, *J* = 7.5 Hz, 3H), 1.45 (sextet, *J* = 7.5 Hz, 2H), 1.66 (quint, *J* = 1.5 Hz, 2H), 1.74 (quint, *J* = 6.0 Hz, 2H), 1.87 (quint, *J* = 6.0 Hz, 2H), 2.60 (t, *J* = 6.0 Hz, 2H), 2.69 (t, *J* = 6.0 Hz, 2H), 4.06 (t, *J* = 7.8 Hz, 2H), 7.43-7.56 (m, 3H), 7.94 (d, *J* = 6.9 Hz, 2H), 8.31 (s, 1H), 9.26 (br s, 1H).

### d) Preparation of 1-benzyl-3-(1-butyl-2-oxo-1,2,5,6,7,8-hexahydroquinolin-3-yl)urea (6-005)

3-Amino-1-butyl-5,6,7,8-tetrahydro-1*H*-quinolin-2-one acetic acid salt (6-001-01) (5 mg, 0.018 mmol) was dissolved in methylene chloride (1 mL), and to the reaction mixture were added benzylisocyanate (2.5 µL, 0.02 mmol) and 4-dimethylaminopyridine (2.4 mg, 0.02 mmol), and the reaction mixture was stirred at room temperature for 4 h. To the reaction mixture was added diluted hydrochloric acid (0.1 mol/L, 3 mL), and the reaction mixture was extracted with ethyl acetate (10 mL), washed with brine (3 mL), dried over anhydrous magnesium sulfate, and evaporated under reduced pressure. The obtained residue was purified by silica gel column chromatography (toluene/ethyl acetate) to give 1-benzyl-3-(1-butyl-2-oxo-1,2,5,6,7,8-hexahydroquinolin-3-yl)urea (6-005) (5.0 mg, 79%) as a white crystal.
¹H NMR (300 MHz, CDCl₃): δ 0.92 (t, *J* = 7.5 Hz, 3H), 1.32 (sextet, *J* = 7.5 Hz, 2H), 1.57-1.65 (m, 2H), 1.69 (quint, *J* = 6.0 Hz, 2H), 1.82 (quint, *J* = 6.0 Hz, 2H), 2.55 (t, *J* = 6.0 Hz, 2H), 2.59 (t, *J* = 6.0 Hz, 2H), 3.90 (t, *J* = 7.8 Hz, 2H), 4.46 (d, *J* = 6.0 Hz, 2H), 5.72 (br s, 1H), 7.24-7.32 (m, 5H), 7.95 (s, 1H), 8.00 (br s, 1H).

### Example V

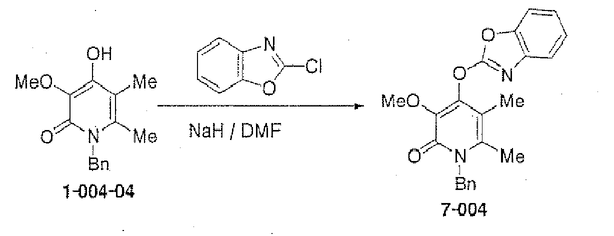

### a) Preparation of 1-(2-bromophenyl)-3-(1-butyl-5,6-dimethyl-2-oxo-1,2-dihydropyridin-3-yl)urea (7-004)

1-Benzyl-5,6-dimethyl-4-hydroxy-3-methoxy-2-pyridone (1-004-04) (259 mg) was dissolved in DMF (3 mL), and to the reaction mixture was added 60% sodium hydride (48 mg) at a time, after 10 min, added a solution of 2-chlorobenzoxazole (261 mg) in DMF (0.5 mL), and the reaction mixture was stirred at room temperature for 5 h and overnight. The reaction mixture was poured into an ice water, extracted twice with ethyl acetate, washed twice with water, dried over magnesium sulfate, and evaporated under reduced pressure. The obtained crystal residue was dissolved in acetone, after decolorization treatment, diethyl ether added to reaction mixture, followed by stand at room temperature. The resulting crystal wsa filtered to give 1-(2-bromophenyl)-3-(1-butyl-5,6-dimethyl-2-oxo-1,2-dihydropyridin-3-yl)urea (7-004) (144 mg, 38.3 %, m.p. 154 to 155 °C) as colorless prism.
¹H NMR (300 MHz, CDCl₃): δ 2.07 (s, 3H), 2.28 (s, 3H), 3.88 (s, 3H), 5.42 (br s, 2H), 7.19-7.54 (m, 9H).

### Example W

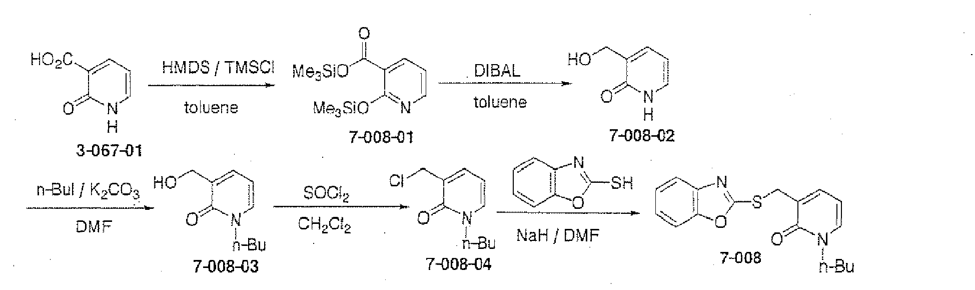

### a) Preparation of 3-hydroxymethyl-2(1H)-pyridone (7-008-01)

To a solution of 2- hydroxynicotinic acid (3-067-01) (5.0 g) in toluene (70 mL), and to the reaction mixture were added hexamethyldisilazane (HMDS, 19 mL) and chlorotrimethylsilane (TMSCI, 0.23 mL), and the reaction mixture was heated under reflux. After stirring for 2 h, the solvent was removed, and toluene (100 mL) was added to the residue. To the reaction mixture was added diisobutylaluminium hydride (DIBAL, 2 M toluene solution, 90 mL) at - 78 °C, after stirring for 4 h, the reaction was quenched with methanol. The insoluble substance was filtered off on Celite, and the filtrate was evaporated under reduced pressure. To the residue were added water and ethyl acetate, and the organic layer was separated, and then the aqueous layer was extracted with three times with ethyl acetate. The combined organic layers were washed with water, brine, dried over anhydrous magnesium sulfate, and evaporated to give 3-hydroxymethyl-2(1*H*)-pyridone (7-008-01) (2.6 g, 59%) as a white solid.
¹H NMR (300 MHz, CDCl₃): δ 4.50 (s, 2H), 6.43 (t, *J* = 6.7 Hz, 1H), 7.33-7.36 (m, 1H), 7.64-7.67 (m, 1H).

### b) Preparation of 1-butyl-3-hydroxymethyl-2-pyridone (7-008-02)

To a solution of 3-hydroxymethyl-2(1*H*)-pyridone (7-008-01) (0.63 g) in DMF (15 mL), and to the reaction mixture were added potassium carbonate (1.4 g) and 1-iodobutane (1.86 g). After stirring at 70 °C for 2 h, the solvent was removed. To the residue were added a saturated aqueous solution of ammonium chloride and ethyl acetate, and the organic layer was separated, and then the aqueous layer was extracted three times with ethyl acetate. The combined organic layers were washed with water and brine, dried over anhydrous magnesium sulfate, and evaporated under reduced pressure. The obtained crude product was purified by column chromatography (toluene/ethyl acetone=2/1) to give 1-butyl-3-hydroxymethyl-2-pyridone (7-008-02) (0.56 g, 61%) as an oil.
¹H NMR (300 MHz, CDCl₃): δ 0.96 (t, *J* = 7.3 Hz, 3H), 1.32-1.45 (m, 2H), 1.69-1.79 (m, 2H), 3.95 (t, *J* = 7.6 Hz, 2H), 4.57 (s, 2H), 6.20 (t, *J* = 6.7 Hz, 1H), 7.24 (dd, *J* = 6.7, 1.2 Hz, 1H), 7.28-7.31 (m, 1H).

### c) Preparation of 3-(benzoxazol-2-yloxymethyl)-1-butyl-2-pyridone (7-008-03)

3-(Benzoxazol-2-yloxymethyl)-1-butyl-2-pyridone (7-008-03) (50%) was synthesized in a similar manner to the preparation of 2-034-03.

### d) Preparation of 1-butyl-3-chloromethyl-2-pyridone (7-008-04)

To a solution of 3-(benzoxazol-2-yloxymethyl)-1-butyl-2-pyridone (7-008-03) (169 mg) in methylene chloride (4.0 mL) was added thionyl chloride (122 mg) at room temperature. After stirring for 1 h, the solvent was removed to 1-butyl-3-chloromethyl-2-pyridone (7-008-04) as an oil.
¹H NMR (300 MHz, CDCl₃): δ 0.96 (t, *J* = 7.3 Hz, 3H), 1.32-1.45 (m, 2H), 1.69-1.79 (m, 2H), 3.96 (t, *J* = 7.3 Hz, 2H), 6.19 (t, *J* = 6.7 Hz, 1H), 7.27 (dd, *J* = 6.7, 2.1 Hz, 1H), 7.49 -7.53 (m, 1H).

### e) Preparation of 3-(benzoxazol-2-ylsulfanylmethyl)-1-butyl-2-pyridone (7-008)

3-(Benzoxazol-2-ylsulfanylmethyl)-1-butyl-2-pyridone (7-008) (97%) was synthesized in a similar manner to the preparation of 2-035.

### Example X

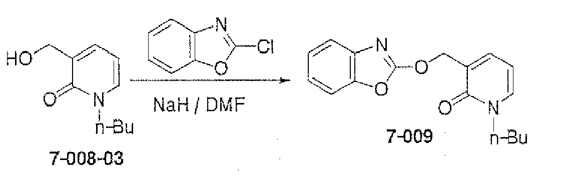

### a) Preparation of 3-(benzoxazol-2-yloxymethyl)-1-butyl-2-pyridone (7-009)

3-(Benzoxazol-2-yloxymethyl)-1-butyl-2-pyridone (7-009) (50%) was synthesized in a similar manner to the preparation of 2-035.

### Example Y

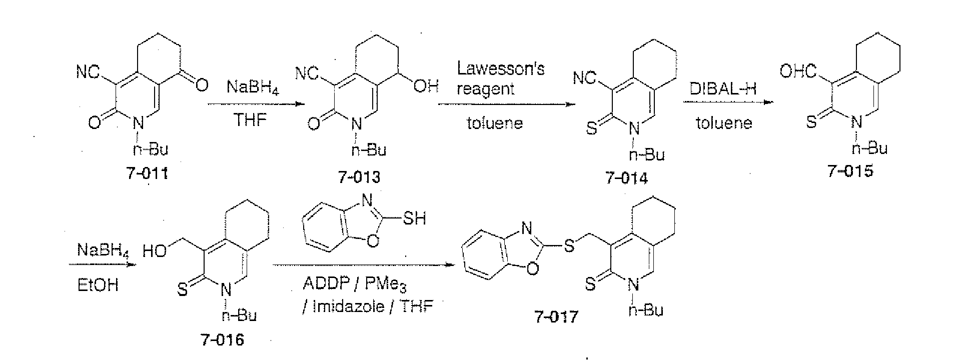

### a) Preparation of 2-butyl-8-hydroxy-3-oxo-2,3,5,6,7,8-hexahydroisoquinoline-4-carbonitrile (7-013)

2-Butyl-3,8-dioxo-2,3,5,6,7,8-hexahydroisoquinoline-4-carbonitrile (7-011) (10 mg, 0.04 mmol) was dissolved in THF (1 mL), and to the reaction mixture was added sodium borohydride (2.1 mg, 0.056 mmol), and the reaction mixture was stirred at room temperature for 10 min. To the reaction mixture was added diluted hydrochloric acid (1mol/L, 3 mL), and the reaction mixture was extracted with ethyl acetate (10 mL), washed with brine (5 mL), dried over anhydrous magnesium sulfate, and evaporated under reduced pressure. The obtained crystalloid residue was recrystallized from methylene chloride to give 2-butyl-8-hydroxy-3-oxo-2,3,5,6,7,8-hexahydroisoquinoline-4-carbonitrile (7-013) (7.4 mg, 75%) as a white crystal.
¹H NMR (300 MHz, CDCl₃): δ 0.97 (t, *J* = 7.5 Hz, 3H), 1.38 (sextet, *J* = 7.5 Hz, 2H), 1.76 (quint, *J* = 7.5 Hz, 2H), 2.15 (quint, *J* = 6.0 Hz, 2H), 2.61 (t, *J* = 6.0 Hz, 2H), 3.06 (t, *J* = 6.0 Hz, 2H), 3.45-3.58 (m, 1H), 4.03 (t, *J* = 7.5 Hz, 2H), 8.39 (s, 1H).

### b) Preparation of 2-butyl-3-thioxo-2,3,5,6,7,8-hexahydroisoquinoline-4-carbonitrile (7-014)

2-Butyl-3-oxo-2,3,5,6,7,8-hexahydroisoquinoline-4-carbonitrile (7-013) (80 mg, 0.35 mmol) was dissolved in toluene (8 mL), and to the reaction mixture was added Lawesson's reagent (169 mg, 0.42 mmol), the reaction mixture was heated under reflux for 12 h. The reaction mixture was cooled to room temperature, and methanol was added to the reaction mixture, and the reaction mixture was stirred at room temperature for 1 h, and evaporated under reduced pressure. The obtained residue was purified by silica gel column chromatography to give 2-butyl-3-thioxo-2,3,5,6,7,8-hexahydroisoquinoline-4-carbonitrile (7-014) (63 mg, 73%) as a pale brown powder.
¹H NMR (300 MHz, CDCl₃): δ 0.98 (t, *J* = 7.5 Hz, 3H), 1.41 (sextet, *J* = 7.5 Hz, 2H), 1.75-1.90 (m, 6H), 2.60 (t, *J* = 6.3 Hz, 2H), 2.87 (t, *J* = 6.3 Hz, 2H), 4.81 (t, *J* = 7.5 Hz, 2H), 7.50 (s, 1H).

### c) Preparation of 2-butyl-3-thioxo-2,3,5,6,7,8-hexahydroisoquinoline-4-carboaldehyde (7-015)

2-Butyl-3-thioxo-2,3,5,6,7,8-hexahydroisoquinoline-4-carbonitrile (7-014) (220 mg, 0.89 mmol) was dissolved in toluene (20 mL), and to the reaction mixture was added 1 M diisobutylaluminium hydride toluene solution (1.7 mL, 1.7 mmol) under ice-cooling, and the reaction mixture was stirred for 30 min. To the reaction mixture was added 1 mol/L diluted hydrochloric acid (5 mL), and the reaction mixture was extracted with ethyl acetate (10 mL), washed with brine (10 mL), dried over anhydrous magnesium sulfate, and evaporated under reduced pressure. The obtained residue was purified by silica gel column chromatography (toluene/ethyl acetate) to give 2-butyl-3-thioxo-2,3,5,6,7,8-hexahydrolsoquinoline-4-carboaldehyde (7-015) (44 mg, 20%) as a pale brown crystal.
¹H NMR (300 MHz, CDCl₃): δ 0.99 (t, *J* = 7.5 Hz, 3H), 1.44 (sextet, *J*= 7.5 Hz, 2H), 1.74 (quint, *J* = 3.3 Hz, 4H), 1.87 (quint, *J* = 7.5 Hz, 2H), 2.62 (br t, *J* = 6.3 Hz, 2H), 2.95 (br t, *J*= 6.3 Hz, 2H), 4.51 (t, *J*= 7.5 Hz, 2H), 7.53 (s, 1H), 10.60 (s, 1H).

### d) Preparation of 2-butyl-3-hydroxymethyl-5,6,7,8-tetrahydroisoquinoline-3-thione (7-016)

2-Butyl-3-thioxo-2,3,5,6,7,8-hexahydroisoquinoline-4-carboaldehyde (7-015) (10 mg, 0.04 mmol) was dissolved in methanol (2 mL), and to the reaction mixture was added sodium borohydride (4.6 mg, 0.12 mmol), and the reaction mixture was stirred at room temperature for 10 min. To the reaction mixture was added 1 mol/L diluted hydrochloric acid (4 mL), and the reaction mixture was extracted with ethyl acetate (10 mL), washed with brine (5 mL), dried over anhydrous magnesium sulfate, and evaporated under reduced pressure. The obtained residue was purified by silica gel column chromatography (toluene/ethyl acetate) to give 2-butyl-3-hydroxymethyl-5,6,7,8-tetrahydroisoquinoline-3-thione (7-016) (9 mg, 90%) as a pale brown powder.
¹H NMR (300 MHz, CDCl₃): δ 0.99 (t, *J* = 7.2 Hz, 3H), 1.43 (sextet, *J* = 7.2 Hz, 2H), 1.71-1.95 (m, 6H), 2.66 (br t, *J* = 6.3 Hz, 2H), 2.83 (t, *J* = 6.3 Hz, 2H), 4.58 (br t, *J* = 7.2 Hz, 2H), 4.79 (s, 2H), 7.61 (s, 1H).

### e) Preparation of 4-(benzoxazol-2-ylthiomethyl)-2-butyl-5,6,7,8-tetrahydro-2H-isoquinoline-3-thione (7-017)

2-Butyl-3-hydroxymethyl-5,6,7,8-tetrahydroisoquinoline-3-thione (7-016) (14 mg, 0.056 mmol) was dissolved in THF (1 mL), and to the reaction mixture were added 2-mercaptobenzoxazole (16.3 mg, 0.11 mmol), 1,1'-(azodicarbonyl)dipiperidine (28.1 mg, 0.11 mmol), imidazole (7.6 mg, 0.11 mmol), and 1 M trimethylphosphine toluene solution (0.11 mL, 0.11 mmol), and the reaction mixture was stirred at room temperature for 18 h. The reaction mixture was concentrated under reduced pressure, and to the reaction mixture was added toluene (2 mL). After the resulting insoluble substance was filtered off, the filtrate was purified by silica gel column chromatography (toluene/ethyl acetate) to give 4-(benzoxazol-2-ylthiomethyl)-2-butyl-5,6,7,8-tetrahydro-2*H*-isoquinoline-3-thione (7-017) (6.5 mg, 30%) as a pale brown powder.
¹H NMR (300 MHz, CDCl₃): δ 0.99 (t, *J* = 7.5 Hz, 3H), 1.43 (sextet, *J* = 7.5 Hz, 2H), 1.70-1.95 (m, 6H), 2.62 (t, *J* = 6.3 Hz, 2H), 3.01 (t, *J* = 6.3 Hz, 2H), 4.58 (t, *J* = 7.5 Hz, 2H), 5.05 (s, 2H), 7.15-7.30 (m, 2H), 7.42 (dd, *J* = 7.2 Hz, *J* = 1.8 Hz, 1H), 7.48 (br s, 1H), 7.60 (dd, *J* = 7.2 Hz, *J* = 1.8 Hz, 1H).

### Example Z

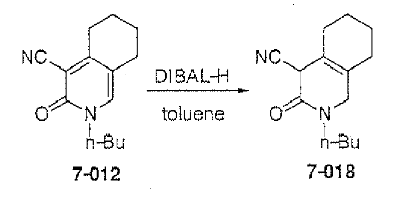

### a) Preparation of 2-butyl-3-oxo-1,2,3,4,5,6,7,8-octahydroisoquinoline-4-carbonitrile (7-018)

2-Butyl-3-oxol-2,3,5,6,7,8-hexahydroisoquinoline-4-carbonitrile (7-012) (100 mg, 0.43 mmol) was dissolved in toluene (10 mL), and to the reaction mixture was added 1 M diisobutylaluminium hydride toluene solution (0.8 mL, 0.8 mmol) under ice-cooling, and the reaction mixture was stirred for 10 min. To the reaction mixture was added 1 mol/L diluted hydrochloric acid (5 mL), and the reaction mixture was extracted with ethyl acetate (10 mL), washed with brine (5 mL), dried over anhydrous magnesium sulfate, and evaporated under reduced pressure. The obtained residue was purified by silica gel column chromatography (toluene/ethyl acetate) to give 2-butyl-3-oxo-1,2,3,4,5,6,7,8-octahydroisoquinoline-4-carbonitrile (7-018) (70 mg, 70%) as a white powder.
¹H NMR (300 MHz, CDCl₃): δ 0.93 (t, *J* = 7.5 Hz, 3H), 1.32 (sextet, *J* = 7.5 Hz, 2H), 1.42-1.59 (m, 5H), 1.88 (s, 1H), 1.97-2.08 (m, 2H), 2.20-2.32 (m, 1H), 2.54-2.66 (m, 1H), 3.06-3.19 (m, 2H), 3.33-3.43 (m, 3H).
The compounds shown in the following tables were prepared in accordance with the above Example. The numbers of left column in Tables represent Compound No..

**Table 1**

| No. | Structure | Physical Properties |
|---|---|---|
| I-1 | 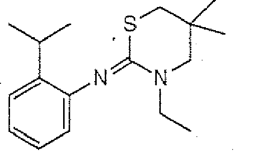 | ¹H-NMR (δ ppm TMS / CDCl₃) 1.13 (6H, s), 1.20 (6H, d, J = 6.9), 1.25 (3H, t, J = 7.4), 2.61 (2H, s),3.05 (2H,s), 3.17 (1H, m), 3.64 (2H, q, J = 6.9), 6.72-6.80 (1H, m), 6.98-7.07 (2H, m), 7.20-7.32 (1H, m). |
| I-2 | 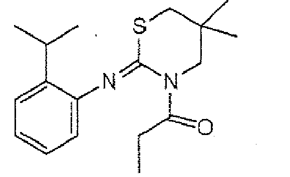 | ¹H-NMR (δ ppm TMS / CDCl₃) 1.14 (6H, s), 1.20 (6H, d, J = 6.9), 1.22 (3H, t, J = 7.4), 2.60 (2H, s), 2.95 (2H, q, J = 7.4), 2.96 (1H, q, J = 6.9), 3.73 (2H, s), 6.73-6.78 (1H, m), 7.10-7.17 (2H, m), 7.25-7.32 (1H, m). |
| I-3 | 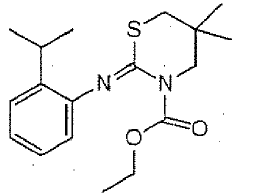 | ¹H-NMR (δ ppm TMS / CDCl₃) 1.16 (6H, s), 1.21 (6H, d, J = 6.9), 1.36 (3H, t, J = 7.1), 2.59 (2H, s), 3.17 (1H, q, J = 6.9), 3.65 (2H, s), 4.32 (2H, q, J = 7.1), 6.74-6.78 (1H,m), 7.12-7.16 (2H, m), 7.30-7.36 (1H, m). |
| I-4 | 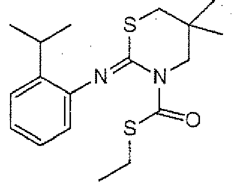 | ¹H-NMR (δ ppm TMS / CDCl₃) 1.16 (6H, s), 1.21 (6H, d, J = 6.9), 1.36 (3H, t, J = 7.1), 2.63 (2H, s), 2.89 (2H, q, J = 7.1), 3.15 (1H, q, J = 6.9), 3.77 (2H, s), 6.79-6.85 (1H,m),7.12-7.16 (2H, m), 7.30-7.36 (1H, m). |
| I-5 | 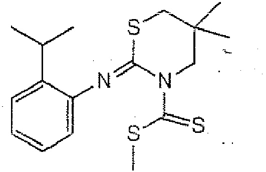 | ¹H-NMR (δ ppm TMS / CDCl₃) 1.20 (6H, d, J = 6.9), 1.23 (6H, s), 2.65 (3H, s), 2.68 (2H, s), 3.11 (1H, q, J = 6.9), 4.51 (2H, s), 6.83-6.90 (1H, m), 7.11-7.18 (2H, m), 7.28-7.35 (1H, m). |
| 1-6 | 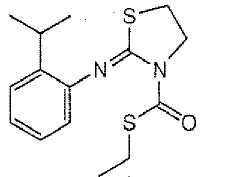 | ¹H-NMR (δ ppm TMS / CDCl₃) 1.20 (6H, d, J = 6.9), 1.30 (3H, t, J = 7.4), 2.90 (2H, t, J = 7.4), 3.15 (2H, t, J = 7.4), 3.20 (1H, q, J = 6.9), 4.31 (2H, t, J = 7.4), 6.79-6.82 (1H, m), 7.07-7.16 (2H, m), 7.28-7.32 (1H, m). |
| 1-7 | 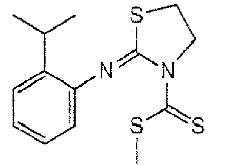 | ¹H-NMR (δ ppm TMS / CDCl₃) 1.23 (6H, d, J = 6.9), 2.65 (3H, s), 2.90 (2H, t, J = 7.4), 3.20 (1H, q, J = 6.9), 4.45 (2H, t, J = 7.4), 6.79-6.82 (1H, m), 7.07-7.16 (2H, m), 7.28-7.32 (1H, m). |

**Table 2**

| No. | Structure | Physical Properties |
|---|---|---|
| I-8 | 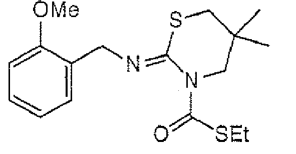 | ¹H-NMR (δ ppm TMS / CDCl₃) 1.15 (6H, s), 1.25 (3H, t, J = 7.4), 2.69 (2H, s), 2.83 (2H, q, J = 7.4), 3.69 (2H, s), 3.84 (3H, s), 4.61 (2H, s), 6.86 (1H, d, J = 8.2), 6.96 (1H, t, J = 8.2), 7.26 (1H, t, J = 8.2), 7.55 (1H, t, J = 8.2). |
| 1-9 | 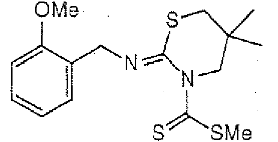 | ¹H-NMR (δ ppm TMS / CDCl₃) 1.25 (6H, s), 2.56 (3H, s), 2.72 (2H, s), 3.85 (3H, s), 4.43 (2H, s), 4.63 (2H, s), 6.86-6.88(2H, m), 7.20-7.30 (1H, m), 7.44-7.48 (1H, m). |
| I-10 | 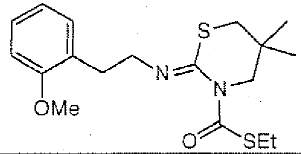 | ¹H-NMR (δ ppm TMS / CDCl₃) 1.11 (6H, s), 1.26 (3H, t, J = 7.4), 2.61 (2H, s), 2.83 (2H, q, J = 7.4), 2.99-3.05 (2H, m), 3.61-3.66 (2H, m), 3.62 (2H, s), 3.82 (3H, s), 6.86-6.91 2H, m), 7.17-7.26 (2H, m). |
| I-11 | 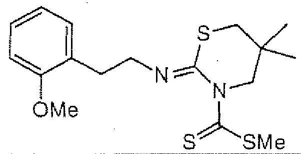 | ¹H-NMR (δ ppm TMS / CDCl₃) 1.19 (6H, s), 2.55 (3H,s), 2.64 (2H, s), 3.05 (2H, t, J = 7.5), 3.66 (2H, t, J = 7.5), 3.84 (3H, s), 4.35 (2H, s), 6.84- 6.91 (2H, m), 7.17- 7.30 (2H, m). |
| 1-12 | 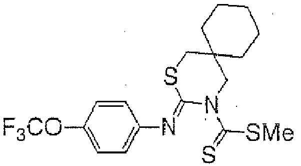 | ¹H-NMR (δ ppm TMS / CDCl₃) 1.35-1.67 (8H, m), 1.74-1.86 (2H, m), 2.64 (3H, s), 2.68 (2H, s), 4.56 (2H, s), 7.02-7.07 (2H, m), 7.22 (2H, d, J=8.2 Hz). |

**Table 3**

| | | | | | | | | |
|---|---|---|---|---|---|---|---|---|
| 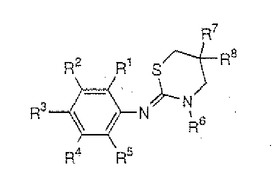 | | | | | | | | |

| No. | R¹ | R² | R³ | R⁴ | R⁵ | R⁶ | R⁷ | R⁸ |
|---|---|---|---|---|---|---|---|---|
| I-16 | H | H | H | H | H | COSEt | Me | Me |
| I-17 | F | H | H | H | H | COSEt | Me | Me |
| I-18 | Cl | H | H | H | H | COSEt | Me | Me |
| I-19 | Me | H | H | H | H | COSEt | Me | Me |
| I-20 | Et | H | H | H | H | COSEt | Me | Me |
| I-21 | Pr | H | H | H | H | COSEt | Me | Me |
| I-22 | Bu | H | H | H | H | COSEt | Me | Me |
| I-23 | Bu*^{s}* | H | H | H | H | COSEt | Me | Me |
| I-24 | Bu*^{t}* | H | H | H | H | COSEt | Me | Me |
| I-25 | Ph | H | H | H | H | COSEt | Me | Me |
| I-26 | CF₃ | H | H | H | H | COSEt | Me | Me |
| I-27 | OMe | H | H | H | H | COSEt | Me | Me |
| I-28 | OEt | H | H | H | H | COSEt | Me | Me |
| I-29 | OPr*ⁱ* | H | H | H | H | COSEt | Me | Me |
| I-30 | SMe | H | H | H | H | COSEt | Me | Me |
| I-31 | SEt | H | H | H | H | COSEt | Me | Me |
| I-32 | SPr*ⁱ* | H | H | H | H | COSEt | Me | Me |
| I-33 | NMe₂ | H | H | H | H | COSEt | Me | Me |
| I-34 | H | Pr*ⁱ* | H | H | H | COSEt | Me | Me |
| I-35 | H | H | Cl | H | H | COSEt | Me | Me |
| I-36 | H | H | Pr*ⁱ* | H | H | COSEt | Me | Me |
| I-37 | H | H | NO₂ | H | H | COSEt | Me | Me |
| I-38 | Me | Me | H | H | H | COSEt | Me | Me |
| I-39 | Me | H | Me | H | H | COSEt | Me | Me |
| I-40 | Me | H | H | Me | H | COSEt | Me | Me |
| I-41 | Me | H | H | H | Me | COSEt | Me | Me |
| I-42 | H | Me | Me | H | H | COSEt | Me | Me |
| I-43 | H | Me | H | Me | H | COSEt | Me | Me |
| I-44 | Me | H | Cl | H | H | COSEt | Me | Me |

**Table 4**

| | | | | | | | | |
|---|---|---|---|---|---|---|---|---|
| 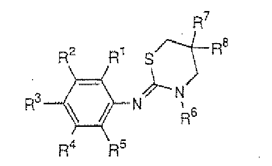 | | | | | | | | |

| No. | R¹ | R² | R³ | R⁴ | R⁵ | R⁶ | R⁷ | R⁸ |
|---|---|---|---|---|---|---|---|---|
| I-45 | Cl | H | Me | H | H | COSEt | Me | Me |
| I-46 | Pr*ⁱ* | H | NO₂ | H | H | COSEt | Me | Me |
| I-47 | Pr*ⁱ* | H | H | H | NO₂ | COSEt | Me | Me |
| I-48 | NO₂ | H | NO₂ | H | H | COSEt | Me | Me |
| I-49 | Pr | H | H | H | H | COSMe | Me | Me |
| I-50 | Pr*ⁱ* | H | H | H | H | COSMe | Me | Me |
| I-51 | Bu*^{s}* | H | H | H | H | COSMe | Me | Me |
| I-52 | H | Pr*ⁱ* | H | H | H | COSMe | Me | Me |
| I-53 | H | OMe | OMe | H | H | COSMe | Me | Me |
| I-54 | H | -OCH₂O- | | H | H | COSMe | Me | Me |
| I-55 | H | OMe | OMe | OMe | H | COSMe | Me | Me |
| I-56 | Et | H | H | H | H | CSSMe | Me | Me |
| I-57 | Bu*^{s}* | H | H | H | H | CSSMe | Me | Me |
| I-58 | CH₂OMe | H | H | H | H | CSSMe | Me | Me |
| I-59 | CH(Me)O Me | H | H | H | H | CSSMe | Me | Me |
| I-60 | OMe | H | H | H | H | CSSMe | Me | Me |
| I-61 | OEt | H | H | H | H | CSSMe | Me | M.e |
| I-62 | SMe | H | H | H | H | CSSMe | Me | Me |
| I-63 | SEt | H | H | H | H | CSSMe | Me | Me |
| I-64 | SPr*ⁱ* | H | H | H | H | CSSMe | Me | Me |
| I-65 | SOMe | H | H | H | H | CSSMe | Me | Me |
| I-66 | SO₂Me | H | H | H | H | CSSMe | Me | Me |
| I-67 | SOEt | H | H | H | H | CSSMe | Me | Me |
| I-68 | NMe₂ | H | H | H | H | CSSMe | Me | Me |
| I-69 | H | Pr*ⁱ* | H | H | H | CSSMe | Me | Me |
| I-70 | H | H | Cl | H | H | CSSMe | Me | Me |

**Table 5**

| | | | | | | | | |
|---|---|---|---|---|---|---|---|---|
| 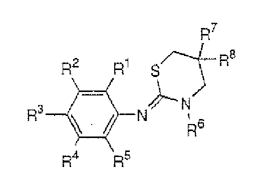 | | | | | | | | |

| No. | R¹ | R² | R³ | R⁴ | R⁵ | R⁶ | R⁷ | R⁸ |
|---|---|---|---|---|---|---|---|---|
| I-71 | Me | H | Me | H | H | CSSMe | Me | Me |
| I-72 | Me | H | H | Me | H | CSSMe | Me | Me |
| I-73 | Me | H | H | H | Me | CSSMe | Me | Me |
| I-74 | H | Me | Me | H | H | CSSMe | Me | Me |
| I-75 | H | Me | H | Me | H | CSSMe | Me | Me |
| I-76 | OMe | OMe | H | H | H | CSSMe | Me | Me |
| 1-77 | H | OMe | OMe | H | H | CSSMe | Me | Me |
| I-78 | OMe | H | H | OMe | H | CSSMe | Me | Me |
| I-79 | OMe | H | OMe | | H | CSSMe | Me | Me |
| I-80 | H | -OCH₂O- | | H | H | CSSMe | Me | Me |
| I-81 | Pr*ⁱ* | H | NO₂ | H | H | CSSMe | Me | Me |
| I-82 | Pr*ⁱ* | H | H | H | NO₂ | CSSMe | Me | Me |
| I-83 | H | OMe | OMe | OMe | H | CSSMe | Me | Me |
| I-84 | Pr*ⁱ* | H | H | H | H | CSSEt | Me | Me |
| I-85 | Bu*^{s}* | H | H | H | H | CSSEt | Me | Me |
| I-86 | OEt | H | H | H | H | CSSEt | Me | Me |
| I-87 | SMe | H | H | H | H | CSSEt | Me | Me |
| I-88 | H | Pr*ⁱ* | H | H | H | CSSEt | Me | Me |
| I-118 | H | OEt | OEt | H | H | CSSMe | Me | Me |
| I-119 | OMe | H | Me | H | H | CSSMe | Me | Me |
| I-120 | OMe | H | H | Me | H | CSSMe | Me | Me |
| I-121 | H | OMe | Me | H | H | CSSMe | Me | Me |
| I-122 | Me | Me | H | H | H | CSSMe | Me | Me |
| I-123 | N(Me)Ac | H | H | H | H | CSSMe | Me | Me |

**Table 6**

| | | | |
|---|---|---|---|
| 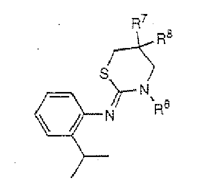 | | | |

| No. | R⁶ | R⁷ | R⁸ |
|---|---|---|---|
| I-89 | COPr | Me | Me |
| I-90 | COOMe | Me | Me |
| I-91 | COOPr | Me | Me |
| I-92 | CONHEt | Me | Me |
| I-93 | COCH₂OMe | Me | Me |
| I-94 | COCH₂SMe | Me | Me |
| I-95 | COCH₂SEt | Me | Me |
| I-96 | CSOEt | Me | Me |
| I-97 | CSNHEt | Me | Me |
| I-98 | CSSPr | Me | Me |
| I-99 | CSSPr*ⁱ* | Me | Me |
| I-100 | CSSBn | Me | Me |

**Table 7**

| | | | | | | | |
|---|---|---|---|---|---|---|---|
| 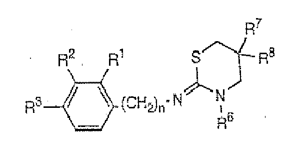 | | | | | | | |

| No. | R¹ | R² | R³ | n | R⁶ | R⁷ | R⁸ |
|---|---|---|---|---|---|---|---|
| I-101 | H | H | Cl | | COSEt | Me | Me |
| I-102 | H | H | Cl | 1 | CSSMe | Me | Me |
| I-103 | Cl | H | Cl | 2 | COSEt | Me | Me |
| I-104 | Cl | H | Cl | 2 | CSSMe | Me | Me |

**Table 8**

| | | |
|---|---|---|
| 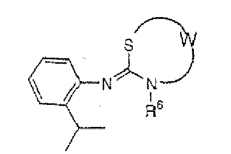 | | |

| No. | R⁶ | W |
|---|---|---|
| I-105 | COSEt | 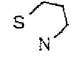 |
| I-106 | COSEt | 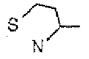 |
| I-I07 | COSEt | 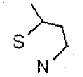 |
| I-108 | COSEt | 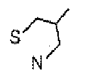 |
| I-109 | COSEt | 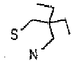 |
| I-110 | COSEt | 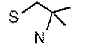 |
| I-111 | COSEt | 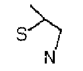 |
| I-112 | COSEt | 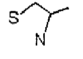 |
| I-113 | CSSMe | 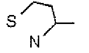 |
| I-114 | CSSMe | 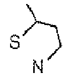 |
| I-115 | CSSMe | 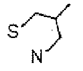 |
| I-116 | CSSMe | 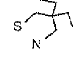 |
| I-117 | CSSMe | 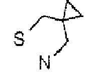 |

**Table 9**

| | | | | | | | | |
|---|---|---|---|---|---|---|---|---|
| 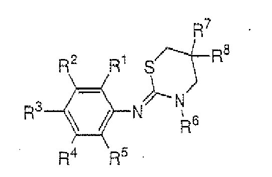 | | | | | | | | |

| No. | R¹ | R² | R³ | P⁴ | R⁵ | R⁶ | R⁷ | R⁸ |
|---|---|---|---|---|---|---|---|---|
| I-124 | H | H | OEt | H | H | CSSMe | Me | Me |
| I-125 | H | OEt | H | H | H | CSSMe | Me | Me |
| I-126 | H | H | OMe | H | H | CSSMe | Me | Me |
| I-127 | H | OMe | H | H | H | CSSMe | Me | Me |
| I-128 | H | OEt | OMe | H | H | CSSMe | Me | Me |
| I-129 | H | OPr | OMe | H | H | CSSMe | Me | Me |
| I-130 | H | OEt | OEt | H | H | CSSMe | Me | Me |
| I-131 | H | H | OPr | H | H | CSSMe | Me | Me |
| I-132 | H | OPr | H | H | H | CSSMe | Me | Me |
| I-133 | H | H | OBu | H | H | CSSMe | Me | Me |
| I-134 | H | OBu | H | H | H | CSSMe | Me | Me |
| I-135 | H | OMe | OEt | H | H | CSSMe | Me | Me |
| I-136 | H | OMe | OPr | H | H | CSSMe | Me | Me |
| I-137 | H | OBu | OMe | H | H | CSSMe | Me | Me |
| I-138 | H | H | OPr*ⁱ* | H | H | CSSMe | Me | Me |
| I-139 | H | OPr*ⁱ* | H | H | H | CSSMe | Me | Me |
| I-140 | H | H | H | H | H | CSSMe | Me | Me |
| I-141 | F | H | H | H | H | CSSMe | Me | Me |
| I-142 | Cl | H | H | H | H | CSSMe | Me | Me |
| I-143 | H | Cl | H | H | H | CSSMe | Me | Me |
| I-144 | Me | H | H | H | H | CSSMe | Me | Me |
| I-145 | H | Me | H | H | H | CSSMe | Me | Me |
| I-146 | H | H | Me | H | H | CSSMe | Me | Me |
| I-147 | H | Bu | H | H | H | CSSMe | Me | Me |
| I-148 | H | H | Bu | H | H | CSSMe | Me | Me |

**Table 10**

| | | | | | | | | |
|---|---|---|---|---|---|---|---|---|
| 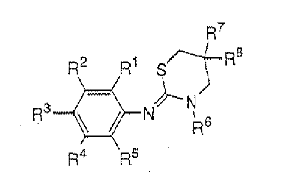 | | | | | | | | |

| No. | R¹ | R² | R³ | R⁴ | R⁵ | R⁶ | R⁷ | R⁸ |
|---|---|---|---|---|---|---|---|---|
| I-149 | Bu*^{t}* | H | H | H | H | CSSMe | Me | Me |
| I-150 | H | H | Et | H | H | CSSMe | Me | Me |
| I-151 | H | Et | H | H | H | CSSMe | Me | Me |
| I-I52 | H | H | F | H | H | CSSMe | Me | Me |
| I-153 | H | F | H | H | H | CSSMe | Me | Me |
| I-154 | H | H | Pr*ⁱ* | H | H | CSSMe | Me | Me |
| I-155 | H | H | Morphol ino | H | H | CSSMe | Me | Me |
| I-156 | H | Ac | H | H | H | CSSMe | Me | Me |
| I-157 | H | H | Br | H | H | CSSMe | Me | Me |
| I-158 | H | Br | H | H | H | CSSMe | Me | Me |
| I-159 | Br | H | H | H | H | CSSMe | Me | Me |
| I-160 | H | C(Me)=N OMe | H | H | H | CSSMe | Me | Me |
| I-161 | H | H | Ac | H | H | CSSMe | Me | Me |
| I-162 | H | H | C(Me)= NOMe | H | H | CSSMe | Me | Me |
| I-163 | OPr*ⁱ* | H | H | H | H | CSSMe | Me | Me |
| I-164 | Pr | H | H | H | H | CSSMe | Me | Me |
| I-165 | CF₃ | H | H | H | H | CSSMe | Me | Me |
| I-166 | H | H | OPh | H | H | CSSMe | Me | Me |
| I-167 | H | H | Pr | H | H | CSSMe | Me | Me |
| I-168 | H | H | Bu*^{t}* | H | H | CSSMe | Me | Me |
| I-169 | H | CF₃ | H | H | H | CSSMe | Me | Me |
| I-170 | H | H | CF₃ | H | H | CSSMe | Me | Me |
| I-171 | Pr*ⁱ* | H | NHAc | H | H | CSSMe | Me | Me |
| I-172 | Pr*ⁱ* | H | H | H | NHAc | CSSMe | Me | Me |
| I-173 | H | COOMe | H | H OMe | | CSSMe | Me | Me |

**Table 11**

| | | | | | | | | |
|---|---|---|---|---|---|---|---|---|
| 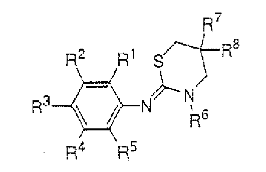 | | | | | | | | |

| No. | R¹ | R² | R³ | R⁴ | R⁵ | R⁶ | R⁷ | R⁸ |
|---|---|---|---|---|---|---|---|---|
| I-174 | Morpholino | H | H | H | H | CSSMe | Me | Me |
| I-175 | H | Morpholino | H | H | H | CSSMe | Me | Me |
| I-176 | Pr*ⁱ* | H | H | COOEt | H | CSSMe | Me | Me |
| I-177 | H | H | Piperidin o | H | H | CSSMe | Me | Me |
| I-178 | Pyrrolidino | H | H | H | H | CSSMe | Me | Me |
| I-179 | H | SMe | H | H | H | CSSMe | Me | Me |
| I-180 | H | H | SMe | H | H | CSSMe | Me | Me |
| I-181 | OCF₃ | H | H | H | H | CSSMe | Me | Me |
| I-182 | H | OCF₃ | H | H | H | CSSMe | Me | Me |
| I-183 | H | H | OCF₃ | H | H | CSSMe | Me | Me |
| I-184 | H | H | 3-Pyridyl | H | H | CSSMe | Me | Me |
| I-185 | H | 3-Pyridyl | H | H | H | CSSMe | Me | Me |
| I-186 | 3-Pyridyl | H | H | H | H | CSSMe | Me | Me |
| I-187 | OPh | H | H | H | H | CSSMe | Me | Me |
| I-188 | H | OEt | OEt | H | H | COOMe | Me | Me |
| I-189 | OMe | H | H | H | H | COOMe | Me | Me |
| I-190 | H | H | Et | H | H | COOMe | Me | Me |
| I-191 | H | H | Pr*ⁱ* | H | H | COOMe | Me | Me |
| I-192 | OMe | H | H | H | H | COSMe | Me | Me |
| I-193 | H | H | Et | H | H | COSMe | Me | Me |
| I-194 | H | H | Pr*ⁱ* | H | H | COSMe | Me | Me |
| I-195 | H | H | OEt | H | H | COSMe | Me | Me |
| I-196 | H | OMe | OEt | H | H | COSMe | Me | Me |
| I-197 | H | Piperidino | H | H | H | CSSMe | Me | Me |
| I-198 | H | H | NEt₂ | H | H | CSSMe | Me | Me |

**Table 12**

| | | | | | | | | |
|---|---|---|---|---|---|---|---|---|
| 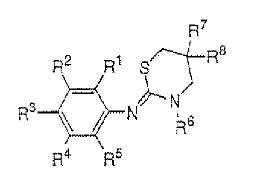 | | | | | | | | |

| No. | R¹ | R² | R³ | R⁴ | R⁵ | R⁶ | R⁷ | R⁸ |
|---|---|---|---|---|---|---|---|---|
| I-199 | OMe | H | COOMe | H | H | CSSMe | Me | Me |
| I-200 | H | 2-Oxopyrrolidino | H | H | H | CSSMe | Me | Me |
| I-201 | H | OPh | H | H | H | CSSMe | Me | Me |
| I-202 | H | H | Ph | H | H | CSSMe | Me | Me |
| I-203 | Ph | H | H | H | H | CSSMe | Me | Me |
| I-204 | H | Ph | H | H | H | CSSMe | Me | Me |
| I-205 | Pr*ⁱ* | H | H | H | H | CSOMe | Me | Me |
| I-206 | Pr*ⁱ* | H | I | H | H | COSME | Me | Me |
| I-207 | OMe | H | (Morpholin o)CO | H | H | CSSMe | Me | Me |
| I-208 | H | H | NMe₂ | H | H | CSSMe | Me | Me |
| I-209 | H | NMe₂ | H | H | H | CSSMe | Me | Me |
| I-210 | N(Me)Et | H | H | H | H | CSSMe | Me | Me |
| I-211 | N(Me)Pr | H | H | H | H | CSSMe | Me | Me |
| I-212 | NEt₂ | H | H | H | H | CSSMe | Me | Me |
| I-213 | F | H | H | H | F | CSSMe | Me | Me |
| I-214 | Pr*ⁱ* | H | Cl | H | H | CSSMe | Me | Me |
| I-215 | NMe₂ | Me | H | H | H | CSSMe | Me | Me |
| I-216 | NMe₂ | H | Me | H | H | CSSMe | Me | Me |
| I-217 | NMe₂ | H | H | Me | H | CSSMe | Me | Me |
| I-218 | NMe₂ | H | H | Cl | H | CSSMe | Me | Me |
| I-219 | Me | H | H | H | Me | CSSMe | Me | Me |
| I-220 | NMe₂ | H | H | H | H | CSSEt | Me | Me |
| I-221 | H | NMe₂ | H | H | H | CSSEt | Me | Me |
| I-222 | NMe₂ | H | Me | H | H | CSSEt | Me | Me |
| I-223 | H | H | Pr*ⁱ* | H | H | CSSEt | Me | Me |

**Table 13**

| | | | | | | | | |
|---|---|---|---|---|---|---|---|---|
| 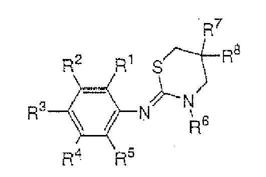 | | | | | | | | |

| No. | R¹ | R² | R³ | R⁴ | R⁵ | R⁶ | R⁷ | R⁸ |
|---|---|---|---|---|---|---|---|---|
| I-224 | OMe | H | CONHMe | H | H | CSSMe | Me | Me |
| I-225 | OCHF₂ | H | H | H | H | CSSMe | Me | Me |
| I-226 | H | OCHF₂ | H | H | H | CSSMe | Me | Me |
| I-227 | H | NEt₂ | H | H | H | CSSMe | Me | Me |
| I-228 | NMe₂ | H | Cl | H | H | CSSMe | Me | Me |
| I-229 | NMe₂ | H | F | H | H | CSSMe | Me | Me |
| I-230 | NMe₂ | H | H | F | H | CSSMe | Me | Me |
| I-231 | NMe₂ | H | Et | H | H | CSSMe | Me | Me |
| I-232 | NMe₂ | H | H | Et | H | CSSMe | Me | Me |
| I-233 | NMe₂ | H | Cl | H | H | CSSEt | Me | Me |
| I-234 | NMe₂ | H | F | H | H | CSSEt | Me | Me |
| I-235 | NMe₂ | H | Et | H | H | CSSEt | Me | Me |
| I-236 | Pr*ⁱ* | H | H | H | H | CSSBu*^{s}* | Me | Me |
| I-237 | Pr*ⁱ* | H | H | H | H | CSSBu*ⁱ* | Me | Me |
| I-238 | Pr*ⁱ* | H | H | H | H | CSNHMe | Me | Me |
| I-239 | Me | NMe₂ | H | H | H | CSSMe | Me | Me |
| I-240 | NMe₂ | OMe | H | H | H | CSSMe | Me | Me |
| I-241 | H | NMe₂ | Me | H | H | CSSMe | Me | Me |
| I-242 | NMe₂ | Cl | H | H | H | CSSMe | Me | Me |
| I-243 | H | NMe₂ | OMe | H | H | CSSMe | Me | Me |
| I-244 | Pr*ⁱ* | H | H | H | H | CSSEt | Et | Et |
| I-245 | Pr*ⁱ* | H | H | H | H | Me | Me | Me |
| I-246 | Pr*ⁱ* | H | H | H | H | Pr | Me | Me |
| I-247 | Pr*ⁱ* | H | H | H | H | Pr*ⁱ* | Me | Me |
| I-248 | Pr*ⁱ* | H | H | H | H | Bu*ⁱ* | Me | Me |

**Table 14**

| | | | | |
|---|---|---|---|---|
| 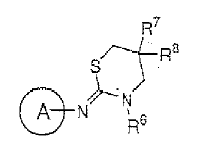 | | | | |

| No. | A | R⁶ | R⁷ | R⁸ |
|---|---|---|---|---|
| I-249 | 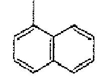 | CSSMe | Me | Me |
| I-250 | 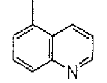 | CSSMe | Me | Me |
| I-251 | 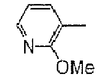 | CSSMe | Me | Me |
| I-252 | 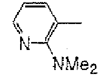 | CSSMe | Me | Me |
| I-253 | 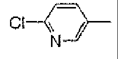 | CSSMe | Me | Me |
| I-254 | 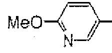 | CSSMe | Me | Me |
| I-255 | 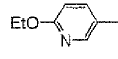 | CSSMe | Me | Me |
| I-256 | 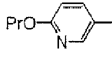 | CSSMe | Me | Me |
| I-257 | 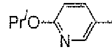 | CSSMe | Me | Me |
| I-258 | 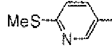 | CSSMe | Me | Me |
| I-259 | 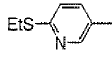 | CSSMe | Me | Me |
| I-260 | 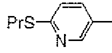 | CSSMe | Me | Me |
| I-261 | 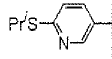 | CSSMe | Me | Me |

**Table 15**

| | | | | | | | | |
|---|---|---|---|---|---|---|---|---|
| 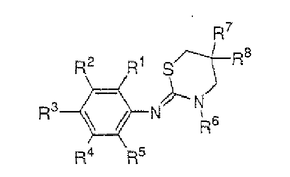 | | | | | | | | |

| No. | R¹ | R² | R³ | R⁴ | R⁵ | R⁶ | R⁷ | R⁸ |
|---|---|---|---|---|---|---|---|---|
| I-262 | NMe₂ | H | OMe | H | H | CSSMe | Me | Me |
| I-263 | NMe₂ | H | H | OMe | H | CSSMe | Me | Me |
| I-264 | Me | NEt₂ | H | H | H | CSSMe | Me | Me |
| I-265 | H | NEt₂ | Me | H | H | CSSMe | Me | Me |
| I-266 | H | NEt₂ | OMe | H | H | CSSMe | Me | Me |
| I-267 | Bu^{s} | H | H | H | H | CSSMe | Et | Et |
| I-268 | Pr*ⁱ* | H | H | H | H | CSSMe | Pr | Pr |
| I-269 | Pr*ⁱ* | H | H | H | H | CSSMe | -(CH₂)₄- | |
| I-270 | Pr*ⁱ* | H | H | H | H | CSSMe | -(CH₂)₅- | |

**Table 16**

| | | | | | | | | |
|---|---|---|---|---|---|---|---|---|
| 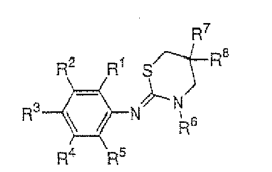 | | | | | | | | |

| No. | R¹ | R² | R³ | R⁴ | R⁵ | R⁶ | R⁷ | R⁸ |
|---|---|---|---|---|---|---|---|---|
| I-271 | Pr*ⁱ* | H | H | H | H | SO₂Me | Me | Me |
| I-272 | Pr*ⁱ* | H | H | H | H | 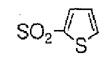 | Me | Me |
| I-273 | Pr*ⁱ* | H | H | H | H | 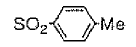 | Me | Me |
| I-274 | H | Pr*ⁱ* | H | H | H | 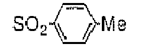 | Me | Me |
| I-275 | H | Pr*ⁱ* | H | H | H | SO₂Et | Me | Me |
| I-276 | H | Pr*ⁱ* | H | H | H | 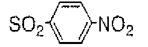 | Me | Me |
| I-277 | H | Pr*ⁱ* | H | H | H | 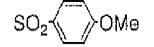 | Me | Me |
| I-278 | H | Pr*ⁱ* | H | H | H | 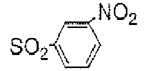 | Me | Me |
| I-279 | H | Pr*ⁱ* | H | H | H | 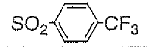 | Me | Me |
| I-280 | H | Pr*ⁱ* | H | H | H | 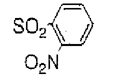 | Me | Me |

**Table 17**

| Compound | Physical Properties | |
|---|---|---|
| No. | m.p. | NMR (CDCl₃) |
| I-16 | 57-59°C | 1.16 (6H, s), 1.31 (3H, t, J = 7.3), 2.64 (2H, s), 2.91 (2H, q, J = 7.3), 3.78 (2H, s), 6.96 (1H,dd, J = 7.4, 1.2), 7.14 (1H, t, J = 7.4), 7.36 (2H, t, J = 7.4). |
| I-17 | | 1.15 (6H, s), 1.31 (3H, t, J = 7.3), 2.67 (2H, s), 2.91 (2H, q J = 7.3), 3.77 (2H, s), 7.10-7.15 (4H,m). |
| I-18 | | 1.16 (6H, s), 1.31 (3H, t, J = 7.3), 2.68 (2H, s), 2.92 (2H, q, J = 7.3), 3.80 (2H, s), 6.96 (1H, dd, J = 7.7, 1.2), 7.08 (1H, dt, J = 7.7, 1.6), 7.25 (2H, t, J = 7.4), 7.40 (1H, d, J = 7.4). |
| I-19 | | 1.15 (6H, s), 1.27 (3H, t, J = 7.3), 2.24 (3H, s), 2.62 (2H, s), 2.92 (2H, q, J = 7.4), 3.77 (2H, s), 6.83 (1H, d, J = 7.7), 7.04 (1H, t, J = 7.7), 7.16-7.22 (2H, m). |
| I-20 | | 1.15 (6H, s), 1.19 (3H, t, J = 7.4), 1.31 (3H, t, J = 7.3), 2.62 (2H, q, J = 7.3), 2.65 (2H, s), 2.94 (2H, q, J = 7.4), 3.77 (2H, s), 6.83 (1H, d, J =7.6), 7.10-7.22 (3H, m). |
| I-21 | | 0.95 (3H, t, J = 7.3), 1.15 (6H, s), 1.30 (3H, t, J = 7.4), 1.50-1.64 (2H, m), 2.56 (2H, q, J = 7.3), 2.59 (2H, s), 2.90 (2H, q, J = 7.4), 3.76 (2H, s), 6.82 (1H, d, J = 7.3), 7.06-7.28 (3H, m). |
| I-22 | | 0.90 (3H, t, J = 7.1), 1.15 (6H, s), 1.29 (3H, t, J = 7.4), 1.30-1.34 (2H, m), 1.52-1.58 (2H, m), 2.54 (2H, q, J = 7.1), 2.62 (2H, s), 2.92 (2H, q, J = 7,4), 3.76 (2H, s), 6.79 (1H, dd, J = 7.9, 1.4), 7.06-7.28 (3H, m). |
| I-23 | | 0.86 (3H, t, J = 7.4), 1.14 (6H, s), 1.16 (6H, d, J = 6.9), 1.29 (3H, t, J = 7.4), 1.48-1.58 (2H, m), 2.61 (2H, s), 2.89 (2H, q, J = 7.4), 2.88-2.92 (1H, m), 3.76 (2H, d, J = 13.6), 3.82 (1H,d, J = 13.6), 6.82-6.88 (1H, m), 7.10-7.18 (1H, m), 7.23-7.29 (1H, m). |
| I-24 | | 1.15 (6H, s), 1.27 (3H, t, J = 7.4), 1.33 (9H, s), 2.68 (2H, s), 2.86 (2H, q, J = 7.4), 3.75 (2H, s), 6.86 (1H, dd, J = 7.4, 1.6), 7.08-7.19 (2H, m), 7.38 (2H, dd, J = 7.4, 1.6). |
| I-25 | | 0.99 (6H, s), 1.25 (3H, t, J = 7.4), 2.45 (2H, s), 2.82 (2H, q, J = 7.4), 3.51 (2H, s), 6.98 (1H, d, J = 7.7), 7.20-7.36 (6H, m), 7.43 (2H, m). |
| I-26 | 82-83°C | 1.15 (6H, s), 1.29 (3H, t, J = 7.3), 2.66 (2H, s), 2.89 (2H, q, J = 7.4), 3.77 (2H, s), 6.98 (1H, d, J = 7.6), 7.19 (1H, t, J = 7.6), 7.49 (1H, t, J =7.6), 7.64 (1H, d, J = 7.6). |

**Table 18**

| Compound | Physical Properties | |
|---|---|---|
| No. | m.p. | NMR (CDCl₃) |
| I-27 | | 1.16 (6H, s), 1.25 (3H, t, J =7.4), 2.62 (2H, s), 2.88 (2H, q, J = 7.4), 3.78 (2H, s), 3.83 (3H, s), 6.91-6.96 (3H, m), 7.05-7.14 (1H, m). |
| I-28 | | 1.15 (6H, s), 1.30 (3H, t, J = 7.4), 1.40 (3H, t, J = 7.0), 2.60 (2H, s), 2.90 (2H, q, J= 7.4), 3.78 (2H, s), 4.08 (2H, q, J = 7.0), 6.90-6.94 (3H, m), 7.06-7.08 (1H, m). |
| I-29 | | 1.14 (6H, s), 1.29 (6H, d, J =7.4), 1.31 (6H, d, J = 6.0), 2.59 (2H, s), 2.89 (2H, q, J = 7.4), 3.76 (2H, s), 4.50 (1H, q, J = 6.0), 6.90-6.93 (3H, m), 7.01-7.07 (1H, m). |
| I-30 | 78-80°C | 1.15 (6H, s), 1.29 (3H, t, J = 7.4), 2.43 (3H, s), 2.63 (2H, s), 2.89 (2H, q, J = 7.4), 3.78 (2H, s), 6.87-6.91 (1H, m), 7.05-7.14 (2H, m), 7.20-7.29 (1H, m). |
| I-31 | 55-57°C | 1.15 (6H, s), 1.29 (3H, t, J = 7.4), 1.31 (3H, t, J = 7.4), 2.66 (2H, s), 2.89 (2H, q, J = 7.4), 2.94 (2H, q, J = 7.4), 3.78 (2H, s), 6.91 (1H, dd, J = 7.4, 1.6), 7.08-7.20 (2H, m), 7.32 (1H, dd, J = 7.4, 1.6). |
| I-32 | | 1.15 (6H, s), 1.27 (6H, d, J = 6.6), 1.28 (6H, d, J = 7.4), 2.65 (2H, s), 2.88 (2H, q, J = 7.4), 3.38-3.42 (1H, m), 3.78 (2H, s), 6.90 (1H, dd, J = 7.7, 1.6), 7.08-7.20 (2H, m), 7.32 (1H, dd, J = 7.7, 1.6). |
| I-33 | | 1.15 (6H, s), 1.29 (3H, t, J = 7.4), 2.60 (2H, s), 2.71 (6H, s), 2.89 (2H, q, J = 7.4), 3.77 (2H, s), 6.90-6.98 (3H, m), 7.05-7-10 (1H, m). |
| I-34 | | 1.16 (6H, s), 1.27 (6H, d, J = 6.9), 1.31 (3H, t, J = 7.4), 2.64 (2H, s), 2.91 (2H, q, J = 7.4), 2.98 (1H, q, J = 6.9), 3.77 (2H, s), 6.78-6.83 (2H, m), 7.01-7.04 (1H, m), 7.25-7.27 (1H, m). |
| I-35 | 68-69°C | 1.16 (6H, s), 1.30 (3H, t, J =7.3), 2.66 (2H, s), 2.90 (2H, q, J = 7.3), 3.76 (2H, s), 6.98 (2H, dd, J = 6.6, 2.1), 7.31 (2H, dd, J = 6.6, 2.1). |
| I-36 | 67-69°C | 1.15 (6H, s), 1.20 (6H, d, J = 6.9), 1.26 (3H, t, J = 7.4), 2.64 (2H, s), 2.86 (2H, q, J = 7.4), 2.89 (1H, q, J = 6.9), 3.75 (2H, s), 6.98 (2H, d, J = 8.2), 7.20 (2H, d, J = 8.3). |
| I-37 | 125-126°C | 1.15 (6H, s), 1.30 (3H, t, J = 7.3), 2.72 (2H, s), 2.92 (2H, q, J = 7.3), 3.78 (2H, s), 7.05 (2H, d, J =8.3), 7.31 (2H, d, J = 8.3). |
| I-38 | 76-78°C | 1.15 (6H, s), 1.30 (3H, t, J = 7.4), 2.14 (3H, s), 2.29 (3H, s), 2.63 (2H, s), 2.89 (2H, q, J = 7.4), 3.77 (2H, s), 6.70 (1H, d, J= 7.9), 6.94 (1H, d, J = 7.9), 7.06 (1H, s). |

**Table 19**

| Compound | Physical Properties | |
|---|---|---|
| No. | m.p. | NMR (CDCl₃) |
| I-39 | | 1.14 (6H, s), 1.29 (3H, t, J = 7.4), 2.21 (3H, s), 2.32 (3H, s), 2.65 (2H, s), 2.89 (2H, q, J = 7.4), 3.76 (2H, s), 6.73 (1H, d, J = 7.9), 6.97 (1H, d, J = 7.9), 7.02 (1H, s). |
| I-40 | | 1.15 (6H, s), 1.30 (3H, t, J = 7.4)., 2.19 (3H, s), 2.31 (3H, s), 2.64 (2H, s), 2.89 (2H, q, J = 7.4), 3.77 (2H, s), 6.65 (1H, s), 6.86 (1H, d, J = 7.9), 7.07 (1H, d, J = 7.7). |
| I-41 | 59-61°C | 1.15 (6H, s), 1.30 (3H, t, J = 7.3), 2.19 (6H, s), 2.62 (2H, s), 2.90 (2H, q, J = 7.3), 3.78 (2H, s), 6.90-6.96 (1H,m), 7.02-7.08 (2H, m). |
| I-42 | | 1.15 (6H, s), 1.31 (3H, t, J = 7.4), 2.26 (3H, s), 2.28 (3H, s), 2.65 (2H, s), 2.91 (2H, q, J = 7.4), 3.78 (2H, s), 6.74 (1H, dd, J = 7.9, 1.8), 6.80 (1H, d, J = 1.8), 7.13 (1H, d, J = 7.7). |
| I-43 | | 1.15 (6H, s), 1.31 (3H, t, J = 7.4), 2.31 (6H, s), 2.63 (2H, s), 2.90 (2H, q, J= 7.4), 3.76 (2H, s), 6.58 (2H, s), 6.77 (1H, s). |
| I-44 | | 1.15 (6H, s), 1.28 (3H, t, J = 7.4), 2.21 (3H, s), 2.64 (2H, s), 2.90 (2H, q, J = 7.4), 3.76 (2H, s), 6.74 (1H, d, J = 8.2), 7.10-7.18 (2H, m). |
| I-45 | | 1.15 (6H, s), 1.28 (3H, t, J = 7.4), 2.31 (3H, s), 2.66 (2H, s), 2.92 (2H, q, J = 7.4), 3.78 (2H, s), 6.74 (1H, d, J = 7.8), 7.04 (1H, d, J=7.8), 7.25 (1H, d, J = 7.8). |
| I-46 | 119-120°C | 1.16 (6H, s), 1.25 (6H, d, J = 6.9), 1.29 (3H, t, J = 7.4), 2.69 (2H, s), 2.90 (2H, q, J = 7.4), 3.15 (1H, m), 3.79 (2H, s), 6.92 (1H, d, J = 8.7), 8.01 (1H, dd, J = 8.5, 2.4), 8.18 (1H, d, J = 2.4). |
| I-47 | | 1.17 (6H, s), 1.23 (6H, d, J = 6.9), 1.30 (3H, t, J = 7.4), 2.69 (2H, s), 2.91 (2H, q, J = 7.4), 3.19 (1H, m), 3.79 (2H, s), 7.41 (1H, d, J = 8.7), 7.71 (1H, d, J = 2.4), 7.92 (1H, dd, J = 8.7, 2.4). |
| I-48 | | 1.15 (6H, s), 1.30 (3H, t, J = 7.4), 2.73 (2H, s), 2.93 (2H, q, J = 7.4), 3.82 (2H, s)7.15 (2H, d, J =8.3), 8.48 (1H, dd, J = 8.3, 1,4), 8.90 (1H, d, J =8.3). |
| I-49 | 64-66°C | 0.95 (3H, t, J = 7.3), 1.15 (6H, s), 1.50-1.64 (2H, m); 2.32 (3H, s), 2.56 (2H, q, J = 7.3), 2.63 (2H, s), 3.78 (2H, s), 6.82 (1H, d, J = 7.3), 7.06-7.28 (3H, m). |
| I-50 | 95-96°C | 1.16 (6H, s), 1.20 (6H, d, J = 6.9), 2.32 (3H, s), 2.64 (2H, s), 3.12 (1H, q, J = 6.9), 3.79 (2H, s), 6.78-6.82 (1H, m), 7.11-7.20 (2H, m), 7.30-7.34 (1H, m). |

**Table 20**

| Compound | Physical Properties | |
|---|---|---|
| No. | m.p. | NMR (CDCl₃) |
| I-51 | 53-56°C | 0.85 (3H, t, J = 7.3), 1.15 (6H, d, J = 6.9), 1.18 (6H, s), 1.57-1.70 (2H, m), 2.31 (3H, s), 2.62 (2H, s), 2.91 (1H, q, J = 6.9), 3.74 (1H, d, J = 13.7), 3.78 (1H, d, J = 13.7), 6.78-6.83 (1H, m), 7.11-7.18 (2H, m), 7.23-7.30 (1H, m). |
| 1-52 | 88-90°C | 1.17 (6H, s), 1.27 (6H, d, J = 6.9), 2.33 (3H, s), 2.65 (2H, s), 2.91 (1H, q, J = 6.9), 3.79 (2H, s), 6.78-6.83 (2H, m), 7.01-7.04 (1H, m), 7.20-7.24 (1H, m). |
| I-53 | | 1.16 (6H, s), 2.32 (3H, s), 2.65 (2H, s), 3.77 (2H, s), 3.87 (6H, s), 6.51-6.59 (2H, m), 6.80-6.89 (1H, m). |
| I-54 | 102-104°C | 1.15 (6H, s), 2.31 (3H, s), 2.65 (2H, s), 3.76 (2H, s), 5.96 (2H, s), 6.42 (1H, dd, J = 8.1, 1.8), 6.53 (1H, d, J = 1.8), 6.78 (1H, d, J = 8.1). |
| I-55 | 129-131°C | 1.16 (6H, s), 2.32 (3H, s), 2.67 (2H, s), 3.78 (2H, s), 3.85 (6H, s), 3.86 (3H, s), 6.20 (2H, s) |
| I-56 | 107-109°C | 1.17 (3H, t, J = 7.6), 1.22 (6H, s), 2.58 (2H, q, J = 7.6), 2.64 (3H, s), 2.66 (2H, s), 4.51 (2H, s), 6.91 (1H, dd, J = 7.5, 1.3), 7.02-7.19 (2H, m), 7.23-7.28 (1H, m). |
| I-57 | | 0.85 (3H, t, J = 7.3), 1.18 (6H, d, J = 6.9), 1.23 (6H, s), 1.57-1.70 (2H, m), 2.64 (3H, s), 2.66 (2H, s), 2.88 (1H, q, J = 6.9), 4.38 (1H, d, J = 13.7), 4.60 (1H, d, J = 13.7), 6.83-6.90 (1H, m), 7.11-7.18 (2H, m), 7.28-7.35 (1H, m). |
| I-58 | 85-87°C | 1.22 (6H, s), 2.62 (3H, s), 2.63 (2H, s), 3.35 (3H, s), 4.40 (2H, s), 4.48 (2H, s), 6.93-6.99 (1H, m), 7.11-7.29 (2H, m), 7.40-7.49 (1H, m). |
| I-59 | 113-114°C | 1.22 (3H, s), 1.24 (3H, s), 1.37 (3H, d, J = 6.4), 2.63 (3H, s), 2.65 (2H, s), 3.24 (3H, s), 4.35 (1H, d, J = 13.6), 4.55 (1H, q, J = 6.4), 4.66 (1H, d, J = 13.6), 6.91 (1H, d, J = 7.4), 7.19-7.40 (2H, m), 7.51 (1H, d, J = 7.4). |
| 1-60 | 128-130°C | 1.22 (6H, s), 2.62 (3H, s), 2.65 (2H, s), 3.85 (3H, s), 4.53 (2H, s), 6.93-6.99 (2H, m), 7.02-7.15 (2H, m). |
| I-61 | 100-101°C | 1.26 (6H, s), 1.43 (3H, t, J = 7.4), 2.66 (2H, s), 2.67(3H,s), 4.08 (2H, q, J = 7.0), 4.55 (2H, s), 6.95-6.99 (3H, m), 7.11-7.18 (1H, m). |
| I-62 | 137-139°C | 1.23 (6H, s), 2.43 (3H, s), 2.64 (3H,s), 2.67 (2H, s), 4.53 (2H, s), 6.87-6.92 (1H, m), 7.11-7.20 (2H, m), 7.23-7.29 (1H, m). |

**Table 21**

| Compound | Physical Properties | |
|---|---|---|
| No. | m.p. | NMR (CDCl₃) |
| I-63 | 103-105°C | 1.15 (6H, s), 1.29 (3H, t, J = 7.4), 1.31 (3H, t, J = 7.4), 2.66 (2H, s), 2.89 (2H, q, J = 7.4), 2.94 (2H, q, J = 7.4), 3.78 (2H, s), 6.91 (1H, dd, J = 7.4, 1.6), 7.08-7.20 (2H, m), 7.32 (1H, dd, J = 7.4, 1.6). |
| I-64 | 125-126°C | 1.24 (6H, s), 1.28 (6H, d, J =6.6), 2.63(3H, s), 2.66 (2H, s), 3.38-3.42 (1H, m), 4.53 (2H, s), 6.97 (1H, dd, J = 7.7, 1.6), 7.08-7.20 (2H, m), 7.32 (1H, dd, J = 7.7, 1.6). |
| I-65 | | 1.22 (6H, s), 2.63 (3H, s), 2.65 (2H, d, J = 13.6), 2.75 (3H, s), 4.17 (1H, d, J = 13.6), 4.77 (1H, d, J = 13.6), 7.06 (1H, dd, J = 7.7, 1.7), 7.19-7.40 (2H, m), 7.97 (1H, dd, J = 7.7, 1.7). |
| I-66 | 147-149°C | 1.23 (6H, s), 2.63 (3H, s), 2.71 (2H, s), 3.13 (3H, s), 4.52 (2H, s), 7.11 (1H, m,), 7.11-7.20 (2H, m), 7.23-7.29 (1H, m). |
| I-67 | 129-130°C | 1.22 (6H, s), 1.23 (3H, t, J = 6.9), 2.63 (3H, s), 2.66 (2H, s), 2.70-2.85 (1H, m), 2.90-3.15 (1H, m), 4.25 (1H, d, J = 13.6), 4.70 (1H, d, J = 13.6), 7.06 (1H, d, J = 7.5), 7.30-7.45 (2H, m), 7.90 (1H, d, J = 7.5). |
| I-68 | 100-102°C | 1.23 (6H, s), 2.62 (3H, s), 2.65 (2H, s), 2.71 (6H, s), 4.50 (2H, s), 6.93-6.99 (3H, m), 7.02-7.15 (1H, m). |
| I-69 | | 1.23 (6H, s), 1.25 (6H, d, J = 6.9), 2.64 (3H, s), 2.66 (2H, s), 2.92 (1H, q, J = 6.9), 4.52 (2H, s), 6.84-6.86 (2H, m), 7.08-7.13 (1H, m), 7.28-7.32 (1H, m). |
| I-70 | 116-118°C | 1.23 (6H, s), 2.64 (3H, s), 2.68 (2H, s), 4.51 (2H, s), 6.97 (2H, d, J = 8.6), 7.35 (2H, d, J = 8.6). |
| I-71 | 103-105°C | 1.22 (6H, s), 2.19 (3H, s), 2.30 (3H, s), 2.63 (3H, s), 2.65 (2H, s), 4.50 (2H, s), 6.79 (1H, d, J = 7.9), 6.98 (1H, d, J = 7.9), 7.02 (1H, s). |
| I-72 | 100-101°C | 1.23 (6H, s), 2.18 (3H, s), 2.32 (3H, s), 2.64 (3H, s), 2.65 (2H, s), 4.51 (2H, s), 6.71 (1H, s), 6.88 (1H, d, J = 7.9), 7.08 (1H, t, J = 7.9). |
| I-73 | 93-95°C | 1.22 (6H, s), 2.12 (3H, s), 2.30 (3H, s), 2.64 (3H, s), 2.65 (2H, s), 4.51 (2H, s), 6.76 (1H, d, J = 7.9), 6.98 (1H, d, J = 7.9), 7.08 (1H, t, J = 7.9). |
| I-74 | 126-128°C | 1.23 (6H, s), 2.25 (3H, s), 2.27 (3H, s), 2.64 (3H, s), 2.65 (2H, s), 4.51 (2H, s), 6.76 (1H, d, J = 7.9), 6.82 (1H, s), 713 (1H, d, J = 7.9). |
| 1-75 | 96-98°C | 1.23 (6H, s), 2.32 (6H, s), 2.63 (3H, s), 2.65 (2H, s), 4.51 (2H, s), 6.64 (2H, s), 6.80 (1H,s). |
| I-76 | | 1.22 (6H, s), 2.64 (3H, s), 2.65 (2H, s), 3.79 (3H, s), 3.88 (3H, s), 4.52 (2H, s), 6.60 (1H, d, J = 7.9), 6.73 (1H, d, J = 7.9), 7.04 (1H, d, J = 7.9). |

**Table 22**

| Compound | Physical Properties | |
|---|---|---|
| No. | m.p. | NMR (CDCl₃) |
| 1-77 | | 1.24 (6H, s), 2.63 (3H, s), 2.68 (2H, s), 3.87 (6H, s), 4.50 (2H, s), 6.61-6.65 (2H, m), 6.85-6.89 (1H, m). |
| 1-78 | | 1.22 (6H, s), 2.62 (3H, s), 2.66 (2H, s), 3.81 (6H, s), 4.52 (2H, s), 6.48 (1H, dd, J =8.5, 2.4), 6.51 (1H, d, J = 2.4), 6.92 (1H, d, J = 8.5). |
| I-79 | | 1.22 (6H, s), 2.62 (3H, s), 2.64 (2H, s), 3.77 (6H, s), 4.52 (2H, s), 6.56 (1H, d, J = 2.4), 6.68 (1H, dd, J =8.5, 2.4), 686 (1H, d, J = 8.5). |
| I-80 | 108-110°C | 1.23 (6H, s), 2.63 (3H, s), 2.66 (2H, s), 4.49 (2H, s), 6.04 (2H, s), 6.50 (1H, dd, J = 8.1, 1.8), 6.61 (1H, d, J = 1.8), 6.83 (1H, d, J = 8.1). |
| 1-81 | | 1.23 (6H, s), 1.25 (6H, d, J = 6.9), 2.65 (3H, s), 2.71 (2H, s), 3.11 (1H, q, J = 6.9), 4.51 (2H, s), 7.02 (1H, d, J = 8.5), 8.04 (1H, dd, J = 8.5, 2.7), 8.21 (1H, d, J = 2.7). |
| I-82 | | 1.21 (6H, s), 1.24 (6H, d, J = 6.9), 2.63 (3H, s), 2.66 (2H, s), 3.17 (1H, q, J = 6.9), 4.51 (2H, s), 7.45 (1H, d, J = 8.5), 7.80 (1H, d, J = 2.4), 7.99 (1H, dd, J = 8.5, 2.4). |
| I-83 | | 1.24 (6H, s), 2.64 (3H, s), 2.68 (2H, s), 3.85 (6H, s), 3.86 (3H, s), 4.51 (2H, s), 6.28 (2H, s). |
| I-84 | 68-70°C | 1.22 (6H, d, J = 6.9), 1.23 (6H, s), 1.35 (3H, t, J =7.4), 2.65 (2H, s), 3.11 (1H, q, J = 6.9), 3.25 (2H, q, J = 6.9), 4.48 (2H, s), 6.89-6.92 (1H, m), 7.14-7.20 (2H, m), 7.30-7.34 (1H, m). |
| 1-85 | | 0.85 (3H, t, J =7.4), 1.18 (6H, d, J = 6.9), 1.23 (6H, s), 1.35 (3H, t, J =7.4), 1.57-1.70 (2H, m), 2.56 (2H, s), 2.87 (1H, q, J = 6.9), 3.25 (2H, q, J = 7.4), 4.35 (1H, d, J = 13.7), 4.60 (1H, d, J = 13.7), 6.89-6.92 (1H, m), 7.10-7.18 (2H, m), 7.30-7.34 (1H, m). |
| I-86 | 96-97°C | 1.23 (6H, s), 1.36 (3H, t, J = 7.0), 1.40 (3H, t, J = 7.0), 2.63 (2H, s), 3.27 (2H, q, J = 7.4), 4.06 (2H, q, J = 7.0), 4.51 (2H, s), 6.92-7.08 (3H, m), 7.11-7.15 (1H, m). |
| I-87 | 105-106°C | 1.22 (6H, s), 1.35 (3H, t, J = 7.4), 2.43 (3H, s), 2.66 (2H, s), 3.26 (2H, q, J = 7.4), 4.50 (2H, s), 6.95-6.98 (1H, m), 7.10-7.17 (2H, m), 7.24-7.29 (1H, m). |

**Table 23**

| Compound | Physical Properties | |
|---|---|---|
| No. | m.p. | NMR (CDCl₃) |
| 1-88 | | 1.23 (6H, s), 1.25 (6H, d, J = 6.9), 1.35 (3H, t, J =7.4), 2.66 (2H, s), 2.90 (1H, q, J = 6.9), 3.28 (2H, q, J = 7.4), 4.50 (2H, s), 6.84-6.88 (2H, m), 7.08-7.13 (1H, m), 7.28-7.32 (1H, m). |
| I-89 | | 0.98 (3H,t, J = 7.4), 1.12 (6H, s), 1.22 (6H, d, J = 6.9), 1.72-1.80 (2H,m), 2.58 (2H, s), 2.90 (2H, t, J = 7.4), 3.06 (1H, q, J = 6.9), 3.71 (2H, s), 6.71-6.76 (1H, m), 7.11-7.20 (2H, m), 7.30-7.34 (1H, m). |
| I-90 | 99-1.01°C | 1.14 (6H, s), 1.21 (6H, d, J = 6.9), 2.58 (2H, s), 3.14 (1H, q, J = 6.9), 3.64 (2H, s), 3.86 (3H, s), 6.73-6.78 (1H, m), 7.11-7.18 (2H, m), 7.28-7.35 (1H, m). |
| I-91 | | 1.00 (3H, t, J = 7.3), 1.14 (6H, s), 1.20 (6H, d, J = 6.9), 1.74 (2H, q, J = 7.3), 2.58 (2H, s), 3.16 (1H, q, J = 6.9), 3.65 (2H, s), 4.23 (2H, q, J = 6.9), 6.73-6.80 (1H, m), 7.12-7.18 (2H, m), 7.31-7.34 (1H, m). |
| I-92 | 52-53°C | 1.13 (6H, s), 1.19 (6H, d, J = 6.9), 1.20 (3H, t, J = 7.4), 2.60 (2H, s), 2.98 (1H, q, J = 6.9), 3.38 (2H, q, J = 7.4), 3.77 (2H, s), 6.73-6.78 (1H, m), 7.09-7.18 (2H, m), 7.28-7.32 (1H, m). |
| I-93 | 76-78°C | 1.14 (6H, s), 1.22 (6H, d, J = 6.9), 2.62 (2H, s), 2.96 (1H, q, J = 6.9), 3.48 (3H, s), 3.75 (2H, s), 4.64 (2H, s), 6.73-6.78 (1H, m), 7.10-7.17 (2H, m), 7.25-7.32 (1H, m). |
| I-94 | 61-62°C | 1.14 (6H, s), 1.20 (6H, d, J = 6.9), 2.23 (3H, s), 2.68 (2H, s), 2.93 (1H, q, J = 6.9), 3.71 (2H, s), 3.94 (2H, s), 6.82-6.86 (1H, m), 7.10-7.18 (2H, m), 7.30-7.36 (1H, m). |
| I-95 | 50-52°C | 1.13 (6H, s), 1.20 (6H, d, J = 6.9), 1.31 (3H, t, J = 7.3), 2.65 (2H, J = 7.3), 2.68 (2H, s), 2.90 (1H, q, J = 6.9), 3.71 (2H, s), 3.97 (2H, s), 6.82-6.86 (1H, m), 7.12-7.19 (2H, m), 7.30-7.36 (1H, m). |
| I-96 | 73-75°C | 1.21 (6H, s), 1.22 (6H, d, J = 6.9), 1.42 (3H, t, J = 6.9), 2.61 (2H, s), 3.10 (1H, q, J = 6.9), 4.15 (2H, s), 4.65 (2H, q, J = 6.9), 6.74-6.78 (1H, m), 7.14-7.20 (2H, m), 7.30-7.34 (1H, m). |
| I-97 | 160-162°C | 1.18 (6H, s), 1.22 (6H, d, J = 6.9), 1.25 (3H, t, J = 7.4), 2.60 (2H, s), 2.90 (1H, q, J = 6.9), 3.71 (2H, q, J = 7.4), 4.40 (2H, s), 6.74-6.78 (1H, m), 7.14-7.20 (2H, m), 7.30-7.34 (1H, m). |
| I-98 | | 1.04 (3H, t, J = 7.4), 1.20 (6H, d, J = 6.9), 1.27 (6H, s), 1.73 (2H, m), 2.64 (2H, s), 3.12 (1H, q, J = 6.9), 3.22 (2H, t, J = 7.4), 4.48 (2H, s), 6.89-6.92 (1H, m), 7.10-7.20 (2H, m), 7.28-7.35 (1H, m). |

**Table 24**

| Compound | Physical Properties | |
|---|---|---|
| No. | m.p. | NMR (CDCl₃) |
| I-99 | 113-114°C | 1.04 (6H, d, J =6.9), 1.27 (6H, s), 1.42 (3H, d, J = 6.9), 2.63 (2H, s), 3.14 (1H, q, J = 6.9), 4.02 (1H, q, J = 6.9), 4.46 (2H, s), 6.89-6.93 (1H, m), 7.1.0-7.20 (2H, m), 7.28-7.35 (1H, m). |
| 1-100 | | 1.10 (6H, d, J = 6.9), 1.22 (6H, s), 2.64 (2H, s), 3.08 (1H, q, J = 6.9), 4.48 (2H, s), 4.49 (2H, s), 6.83-6.90 (1H, m), 7.11-7.18 (2H, m), 7.20-7.38 (6H, m). |
| 1-101 | | 1.15 (6H, s), 1.25 (3H, t, J = 7.4), 2.70 (2H, s), 2.87 (2H, q, J = 7.4), 3.69 (2H, s), 4.55 (2H, s), 7.30-7.40 (4H, m). |
| 1-102 | | 1.24 (6H, s), 2.57 (3H, s), 2.73 (2H, s), 4.43 (2H, s), 4.58 (2H, s), 7.23-7.40 (4H, m). |
| I-103 | | 1.11 (6H, s), 1.26 (3H, t, J = 7.4), 2.61 (2H, s), 2.83 (2H, q, J = 7.4), 3.10 (2H, t, J = 7.4), 3.65 (2H, s), 3.66 (2H, t, J = 7.4), 7.17 (1H, dd, J = 8.2, 2.1), 7.30 (1H, t, J = 8.2), 7.36 (1H, d, J = 2.1). |
| I-104 | | 1.16 (6H, s), 2.55 (3H,s), 2.63 (2H, s), 3.13 (2H, t, J = 7.5), 3.69 (2H, t, J = 7.5), 4.35 (2H, s), 7.15 (1H, dd, J = 8.2, 2.1), 7.25 (1H, t, J = 8.2), 7.36 (1H, d, J = 2.1). |
| I-105 | | 1.20 (6H, d, J = 6.9), 1.30 (3H, t, J = 7.4), 2.10-2.22 (2H, m), 2.88 (2H, t, J = 6.4), 2.94 (2H, q, J = 7.4), 3.11 (1H, q, J = 6.9), 4.05 (2H, t, J = 7.4), 6.82-6.86 (1H, m), 7.10-7.16 (2H, m), 7.28-7.34 (1H, m). |
| 1-106 | | 1.17-1.30 (12H, m), 1.45-1,52 (1H, m), 1.90-1.96 (1H, m), 2.92 (2H, q, J = 7.4), 2.95-3.05 (2H, m), 3.14-3.23 (1H, m), 3.72-3.75 (1H, m), 7.20-7.30 (2H, m), 7,40-7.45 (2H,m). |
| I-107 | | 1.22 (6H, d, J = 6.9), 1.28 (3H, d, J = 6.6), 1.29 (3H, t, J = 7.4), 1.75-1.77 (1H, m), 2.29-2.34 (1H, m), 2.88 (2H, q, J = 7.4), 3.14 (1H, m), 3.31-3.36 (1H, m ), 4.01-4.10 (2H, m), 6.81-6.85 (1H, m), 7.10-7.20 (2H, m), 7.28-7.35 (1H, m). |
| I-108 | | 1.12 (3H, d, J = 6.6), 1.20 (6H, d, J = 6.9), 1.29 (3H, t, J = 7.4), 2.40-2.50 (1H, m), 2.57 (1H, dd, J = 13.5, 6.6), 2.91 (2H, q, J = 7.4), 2.95 (1H, m), 3.14 (1H, m), 3.45 (1H, dd, J = 13.5, 8.4), 4.30 (1H, dd, J = 13.5, 8.4), 6.81-6.85 (1H, m), 7.10-7.20 (2H, m), 7.28-7.35 (1H, m). |

**Table 25**

| Compound | Physical Properties | |
|---|---|---|
| No. | m.p. | NMR (CDCl₃) |
| I-109 | | 0.88 (6H, t, J = 7.5), 1.22 (6H, d, J = 6.9), 1.29 (3H, t, J = 7.4), 1.45-1.52 (4H, m), 2.58 (2H, s), 2.89 (2H, q, J = 7.4), 3.15 (1H,m), 3.77 (2H, s), 6.78-6.83 (1H, m), 7.08-7.21 (2H, m), 7.30-7.35 (1H, m). |
| I-110 | 109-111°C | 1.21 (6H, d, J = 6.9), 1.23 (6H, s), 1.25 (3H, t, J = 7.4), 2.81 (2H, q, J = 7.4), 2.90 (1H, t, J = 6.9), 3.05 (2H, s), 7.13-7.30 (2H, m), 7.36-7.45 (2H, m). |
| I-111 | | 1.21 (6H, d, J = 6.9), 1.31 (3H, t, J = 7.4), 1.42 (3H, d, J = 6.7), 2.90 (2H, q, J = 7.4), 3.23 (1H, q, J = 6.9), 3.69 (1H, q, J = 6.6), 3.87-3.93 (1H, m), 6.78-6.82 (1H, m), 7.08-7.20 (2H, m), 7.25-7.30 (1H, m). |
| I-112 | | 1.19-1.25 (9H, m), 1.14 (3H, d, J = 6.3), 2.76 (1H, d, J = 10.9), 2.96 (2H, t, J = 7.4), 3.22 (1H, q, J = 6.9), 3.44-3.48 (1H, m), 5.12 (1H, q, J = 6.3), 6.81-6.85 (1H, m), 7.09-7.16 (2H, m), 7.28-7.32 (1H, m). |
| I-113 | 126-1.28°C | 1.18 (6H, d, J = 6.9), 1.22 (6H, d, J = 6.9), 1.45 (3H, t, J = 7.4), 1.80-1.91 (1H,m), 2.57-2.64 (2H, m), 2.61 (3H,s), 2.86-2.89 (1H, m), 3.07 (1H, m), 5.95-6.05 (1H, m), 6.98-7.00 (1H, m), 7.12-7.22 (2H, m), 7.28-7.35 (1H, m). |
| I-114 | | 1.20 (6H, d, J = 6.9), 1.28 (3H, d, J = 6.9), 1.82-1.88 (1H, m), 2.48-2.63 (1H, m), 2.63 (3H,s), 3.11 (1H, m), 3.29-3.35 (1H, m), 4.26(1H, m), 4.98 (1H, m), 6.90-6.95 (1H, m), 7.15-7.20 (2H, m), 7.30-7.35 (1H, m). |
| I-115 | | 1.14 (3H, d, J = 6.5), 1.20 (6H, d, J = 6.9), 2.53 (1H, dd, J = 13.0, 5.4), 2.75 (3H, s), 2.80-2.85 (1H, m), 2.95 (1H, dd, J = 13.0, 5.4), 3.11 (1H, m), 3.72 (1H, dd, J = 13.0, 9.0), 5.15 (1H, dd, J = 13.0, 9.0), 6.90-6.95 (1H, m), 7.15-7.25 (2H, m), 7,30-7.35 (1H, m) |
| I-116 | 119-121°C | 0.88 (6H, t, J = 7.5), 1.20 (6H, d, J = 6.9), 1.45-1.52 (4H, m), 2.62 (2H, s), 2.64 (3H, s), 3.15 (1H, m), 4.66 (2H, s), 6.78-6.83 (1H, m), 7.08-7.21 (2H, m), 7.30-7.35 (1H, m). |
| I-117 | 99-100°C | 0.71-0.79 (1H, m), 0.85-0.90 (2H, m), 1.22 (6H, d, J = 6.9), 1.22-1.25 (1H, m), 2.61 (3H, s), 2.79 (3H, s), 3.00-3.05 (1H, m), 4.40 (2H, s), 6.92-6.95 (1H, m), 7.15-7.21 (2H, m), 7.30-7.35 (1H, m). |

**Table 26**

| Compound | Physical Data | |
|---|---|---|
| No. | m.p. | NMR (CDCl₃) |
| I-118 | | 1.23 (6H, s), 1.45 (6H, t, J = 7.4), 2.63 (3H, s), 2.67(2H,s), 4.08 (2H, q, J = 7.0), 4.55 (2H, s), 6.57-6.63 (2H, m), 6.85 (1H, d, J = 7.9). |
| I-119 | 116-118°C | 1.24 (6H, s), 2.37 (3H, s), 2.64 (3H, s), 2.66 (2H, s), 3.84 (3H, s), 4.54 (2H, s), 6.75-6.80 (2H, m), 6.88 (1H, m). |
| I-120 | 92-93°C | 1.23 (6H, s), 2.27 (3H, s), 2.63 (3H, s), 2.67 (2H, s), 3.84 (3H, s), 4.51 (2H, s), 6.51-6.58 (2H, m), 7.10 (1H, d, J = 7.9). |
| I-121 | 129-130°C | 1.22 (6H, s), 2.30 (3H, s), 2.63 (3H, s), 2.65 (2H, s), 3.80 (3H, s), 4.53 (2H, s), 6.78-6.95 (3H, m). |
| I-122 | 93-95°C | 1.22 (6H, s), 2.12 (3H, s), 2.30 (3H, s), 2.64 (3H, s), 2.65 (2H, s), 4.51 (2H, s), 6.76 (1H, d, J = 7.9), 6.98 (1H, d, J = 7.9), 7.08 (1H, t, J = 7.9). |
| I-123 | 151-152°C | 1.22 (6H, s), 1.83 (3H, s), 2.63 (3H, s), 2.65 (2H, s), 3.17 (3H, s), 4.40 (1H, d, J = 13.6), 4.65 (1H, d, J = 13.6), 7.01 (1H, d, J = 7.9), 7.10-7.15 (2H, m), 7.30-7.35 (1H, m). |

**Table 27**

| Compound | Physical Data | |
|---|---|---|
| No. | m.p. | NMR (CDCl₃) |
| I-124 | 105-106°C | 1.23 (6H, s), 1.41 (3H, t, J=7.0), 2.63 (3H, s), 2.66 (2H, s),4.08 (2H, q, J=7.0), 4.50 (2H, s), 6.88 (2H, d, J=8.6), 6.98 (2H, d, J=8.6). |
| 1-125 | 92-94°C | 1.23 (6H, s), 1.40 (3H, t, J=7.0), 2.62 (3H, s), 2.66 (2H, s), 4.08 (2H, q, J=7.0), 4.50 (2H, s), 6.57-6.63 (2H, m), 6.70-6.75 (1H, m), 7.25-7.30 (1H, m). |
| I-126 | 108-109°C | 1.23 (6H, s), 2.63 (3H, s), 2.65 (2H, s), 3.81 (3H, s), 4.50 (2H, s), 6.92 (2H, d, J=8.6), 7.04 (2H, d, J=8.6). |
| I-127 | 62-64°C | 1.23 (6H, s), 2.63 (3H, s), 2.66 (2H, s), 3-82 (3H, s), 4.50 (2H, s),6.57-6.63 (2H, m), 6.70-6.75 (1H, m), 7.25-7.30 (1H, m). |
| I-128 | 78-79°C | 1.23 (6H, s), 1.44 (3H, t, J=7.0), 2.59 (3H, s), 2.63 (2H, s), 3.82 (3H, s), 4.10 (2H, q, J=7.0), 4.47 (2H, s), 6.57-6.63 (2H, m), 6.82-6.87 (1H, m). |
| I-129 | 58-60°C | 1.04 (3H, t, J=7.0), 1.23 (6H, s), 2.00 (2H, sext, J= 7.0), 2.63 (3H, s), 2.67 (2H, s), 3.87 (3H, s), 4.10 (2H, t, J=7.0), 4.50 (2H, s), 6.58-6.64 (2H, m), 6.86-6.91 (1H, m). |
| I-130 | | 1.13 (6H, s), 1.45 (6H, t, J=7.4), 2.28 (3H, s), 2.62 (2H, s), 3.74 (2H, s), 4.08 (4H, q, J=7.4), 6.46-6.53 (2H, m), 6.88-6.92 (1H, m). |
| I-131 | 91-93°C | 1.04 (3H, t, J=7.0), 1.22 (6H, s), 1.76 (2H, sext, J=7.0), 2.63 (3H, s), 2.65 (2H, s), 3.91 (2H, t, J=7.0), 4.50 (2H, s), 6.90 (2H, d, J=8.6), 6.98 (2H, d, J = 8.6). |
| I-132 | 103-104°C | 1.04 (3H, t, J = 7.0), 1.22 (6H, s), 1.76 (2H, sext, J = 7.0), 2.68 (3H, s), 2.65 (2H, s), 3.91 (2H, t, J=7.0), 4.50 (2H, s), 6.50 (1H, d, J=2.1), 6.60 (1H, d, J=7.4), 6.72 (1H, dd, J=7.4, 2.1), 7.28 (1H, d, J=7.4). |
| I-133 | 91-92°C | 0.98 (3H, t, J=7.0), 1.23 (6H, s), 1.42-1.48 (2H, m), 1.70-1.80 (2H, m), 2.63 (3H, s), 2.65 (2H, s), 3.96 (2H, t, J=7.0), 4.50 (2H, s), 6.90 (2H, d, J=8.6), 6.98 (2H, d, J=8.6). |
| I-134 | 86-87°C | 0.98 (3H, t, J=7.0), 1.23 (6H, s), 1.42-1.48 (2H, m), 1.70-1.80 (2H, m), 2.63 (3H, s), 2.65 (2H, s), 3.96 (2H, t, J=7.0), 4.50 (2H, s), 6.50 (1H, d, J=2.1), 6.60 (1H, d, J=7.8), 6.72 (1H, dd, J=7.8, 2.1), 7.28 (1H, d, J=7.8). |

**Table 28**

| Compound | Physical Data | |
|---|---|---|
| No. | m.p. | NMR (CDCl₃) |
| I-135 | 69-70°C | 1.22 (6H, s), 1.47 (3H, t, J=7.0), 2.64 (3H, s), 2.66 (2H, s), 3.88 (3H, s), 4.15 (2H, q, J=7.0), 4.51 (2H, s), 6.61 (1H, d, J=8.2), 6.62 (1H, d, J=2.1), 6.88 (1H, d, J=8.2). |
| I-136 | 88-89°C | 1.04 (3H, t, J=7.0), 1.23 (6H, s), 1.80 (2H, sext, J=7.0), 2.63 (3H, s), 2.67 (2H, s), 3.87 (3H, s), 3.90 (2H, t, J=7.0), 4.51 (2H, s), 6.61 (1H, dd, J=8.2, 2.1), 6.02 (1H, d, J=2.1), 6.88 (1H, d, J=8.2). |
| I-137 | 83-85°C | 0.98 (3H, t, J=7.0), 1.23 (6H, s), 1.42-1.48 (2H, m), 1.70-1.80 (2H, m), 2.64 (3H, s), 2.68 (2H, s), 3.87 (3H, s), 4.03 (2H, t, J=7.0), 4.50 (2H, s), 6.59 (1H, d, J=8.2), 6.61 (1H, s), 6.88 (1H, d, J=8.2). |
| 1-138 | 84-85°C | 1.23 (6H, s), 1.34 (6H, d, J=6.1), 2.63 (3H, s), 2.65 (2H, s), 4.50 (2H, s), 4.53 (1H, sept, J=6.1), 6.89 (2H, d, J=8.6), 7.04 (2H, d, J=8.6). |
| I-139 | 92-93°C | 1.23 (6H, s), 1.34 (6H, d, J=6.1), 2.63 (3H, s), 2.65 (2H, s), 4.50 (2H, s), 4.53 (1H, sept, J=6.1), 6.50 (1H, d, J=2.1), 6.60 (1H, d, J=8.0), 6.72 (1H, dd, J=8.0, 2.1), 7.28 (1H, d, J=8.0). |
| I-140 | 109-110°C | 1.22 (6H, s), 2.63 (3H, s), 2.65 (2H, s), 4.50 (2H, s), 7.04 (2H, d, J=7.5), 7.15 (1H, d, J=7.5), 7.32 (2H, t, J =7.5). |
| I-141 | 92-93°C | 1.23 (6H, s), 2.63 (3H, s), 2.69 (2H, s), 4.54 (2H, s), 7.01-7.08 (1H, m), 7.11-7.15 (3H, m). |
| I-142 | 133-135°C | 1.23 (6H, s), 2.63 (3H, s), 2.69 (2H, s), 4.54 (2H, s), 7.03 (1H, dd, J=8.0, 2.1), 7.08 (1H, dd, J=8.0, 2.1), 7.25 (1H, t, J=8.0), 7.44 (1H, t, J=8.0). |
| I-143 | 92-93°C | 1.23 (6H, s), 2.63 (3H, s), 2.67 (2H, s), 4.50 (2H, s), 6.88 (1H, dd, J = 8.0, 2.1), 7.03 (1H, d, J=2.1), 7.15 (1H, dd, J=8.0, 2.1), 7.28(1H, t, J=8.0). |
| I-144 | 134-135°C | 1.22 (6H, s), 2.22 (3H,s), 2.63 (3H, s), 2.65 (2H, s), 4.50 (2H, s), 7.00 (1H, d, J=8.1), 7.08 (1H, t, J=8.1), 7.15-7.25 (2H, m). |
| 1-145 | 87-89°C | 1.23 (6H, s), 2.37 (3H,s), 2.63 (3H, s), 2.66 (2H, s), 4.50 (2H, s), 6.82 (1H, d, J=8.1), 6.84 (1H, s), 6.98 (1H, d, J=8.1), 7.21 (1H, t, J=8.1). |

**Table 29**

| Compound | Physical Data | |
|---|---|---|
| No. | m.p. | NMR (CDCl₃) |
| I-146 | 91-93°C | 1.23 (6H, s), 2.35 (3H, s), 2.63 (3H, s), 2.65 (2H, s), 4.50 (2H, s), 6.92 (2H, d, J=8.6), 7.15 (2H, d, J=8.6). |
| I-147 | 82-83°C | 0.90 (3H, t, J=7.0), 1.22 (6H, s), 1.28-1.40 (2H, m), 1.48-1.55 (2H, m), 2.55 (2H, t, J = 7.0), 2.64 (3H, s), 2.66 (2H, s), 4.50 (2H, s), 6.90 (1H, d, J=7.8), 7.09 (1H, t, J=7.8), 7.11 (1H, t, J=7.8), 7.28 (1H, d, J=7.8). |
| I-148 | 72-73°C | 0.90 (3H, t, J=7.0), 1.22 (6H, s), 1.28-1.40 (2H, m), 1.48-1.55 (2H, m), 2.60 (2H, t, J=7.0), 2.64 (3H, s), 2.66 (2H, s), 4.50 (2H, s), 6.95 (2H, d, J=8.6), 7.18 (2H, d, J = 8.6). |
| I-149 | 133-134°C | 1.23 (6H, s), 1.35 (9H, s), 2.65 (3H, s), 2.69 (2H, s), 4.50 (2H, s), 6.97 (1H, d, J=7.8), 7.13 (1H, t, J=7.8), 7.19 (1H, t, J=7.8), 7.41 (1H, d, J=7.8). |
| 1-150 | 99-100°C | 1.22 (6H, s), 1.23 (3H, t, J=7.4), 2.62 (3H, s), 2.64 (2H, s), 2.66 (2H, q, J=7.4), 4.50 (2H, s), 6.95 (2H, d, J= 8.6), 7.20 (2H, d, J=8.6). |
| I-151 | 40-42°C | 1.23 (6H, s), 1.24 (3H, t, J=7.0), 2.64 (3H, s), 2.66 (2H, s), 2.67 (2H, q, J=7.0), 4.52 (2H, s), 6.83 (1H, d, J=8.1), 6.86 (1H, s), 7.00 (1H, d, J=8.1), 7.28 (1H, t, J=8.1). |
| 1-152 | 118-119°C | 1.23 (6H, s), 2.64 (3H, s), 2.67 (2H, s), 4.52 (2H, s), 6.97-7.10 (4H, m). |
| I-153 | 89-90°C | 1.23 (6H, s), 2.64 (3H, s), 2.67 (2H; s), 4.52 (2H, s), 6,73-6.90 (3H, m), 7.25-7.30 (1H, m). |
| I-154 | 111-112°C | 1.22 (6H, s), 1.25 (6H, d, J=7.0), 2.62 (3H, s), 2.64 (2H, s), 2.91 (1H, sept, J=7.0), 4.50 (2H, s), 6.95 (2H, d, J=8.6), 7.25 (2H, d, J=8.6). |
| I-155 | 127-1.29°C | 1.23 (6H, s), 2.62 (3H, s), 2.64 (2H, s), 3.14-3.18 (4H,m), 3.85-3.90 (4H, m), 4.50 (2H, s), 6.93 (2H, d, J = 8.6), 7.04 (2H, d, J=8.6). |
| I-156 | 91-93°C | 1.24 (6H, s), 2.62 (3H, s), 2.65 (3H, s), 2.68 (2H, s), 4.53 (2H, s), 7.21-7.25 (1H, m), 7.48 (1H, t, J=7.9), 7.61 (1H, t, J=1.8), 7.74-7.78 (1H, m). |

**Table 30**

| Compound | Physical Data | |
|---|---|---|
| No. | m.p. | NMR (CDCl₃) |
| I-157 | 103.5-104.5°C | 1.23 (6H, s), 2.63 (3H, s), 2.68 (2H, s), 4.50 (2H, s), 6.88-6.94 (2H, m), 7.46-7.51 (2H, m). |
| I-158 | 97-98°C | 1.23 (6H, s), 2.64 (3H, s), 2.68 (2H, s), 4.51 (2H, s), 6.93-6.97 (1H, m), 7.19-7.31 (3H, m). |
| I-159 | 155.5-156.5°C | 1.24 (6H, s), 2.65 (3H, s), 2.69 (2H, s), 4.54 (2H, s), 6.98-7.05 (2H, m), 7.28-7.34 (1H, m), 7.59-7.63 (1H, m). |
| I-160 | 102-106°C | 1.23 (6H, s), 2.23 (3H, s), 2.64 (3H, s), 2.67 (2H, s), 4.00 (3H, s), 4.52 (2H, s), 7.01-7.05 (1H, m), 7.28 (1H, t, J =1.8), 7.37 (1H, t, J=7.8), 7.45-7.49 (1H, m). |
| I-161 | 111-112°C | 1.23 (6H, s), 2.60 (3H, s), 2.65 (3H, s), 2.69 (2H, s), 4.53 (2H, s), 7.06-7.10 (2H, m), 7.97-8.03 (2H, m). |
| I-162 | 124-125°C | 1.23 (6H, s), 2.23 (3H, s), 2.64 (3H, s), 2.67 (2H, s), 4.00 (3H, s), 4.52 (2H, s), 7.00-7.05 (2H, m), 7.65-7.70 (2H, m). |
| I-163 | 102-103.5°C | 1.23 (6H, s), 1.32 (6H, d, J=6.3), 2.63 (2H, s), 2.64 (3H, s), 4.52 (2H, s), 4.52 (1H, sept, J=6.3), 6.90-6.98 (3H, m), 7.04-7.13 (1H, m) |
| I-164 | 90-92°C | 0.94 (3H, t, J=7.3), 1.23 (6H, s), 1.58 (2H, sext, J=7.3), 2.51-2.56 (2H, m), 2.65 (3H, s), 2.65 (2H, s), 4.51 (2H, s), 6.90 (1H, dd, J=7.6, 1.3), 7.07-7.25 (3H, m) |
| I-165 | 157-158°C | 1.23 (6H, s), 2.64 (3H, s), 2.68 (2H, s), 4.49 (2H, s), 7.08 (1H, d, J=7.9), 7.22 (1H, d, J=7.6), 7.50-7.56 (1H, m), 7.66-7.69 (1H, m) |
| 1-166 | 145-146°C | 1.24 (6H, s), 2.64 (3H, s), 2.69 (2H, s), 4.51 (2H, s), 7.00-7.13 (7H, m), 7.30-7.37 (2H, m) |
| I-167 | 77-79°C | 0.95 (3H, t, J=7.3), 1.23 (6H, s), 1.65 (2H, sext, J=7.3), 2.58 (2H, t, J=7.3), 2.63 (3H, s), 2.66 (2H, s), 4.51 (2H, s), 6.93-7.00 (2H, m), 7.14-7.20 (2H, m) |

**Table 31**

| Compound | Physical Data | |
|---|---|---|
| No. | m.p. | NMR (CDCl₃) |
| I-168 | 117-118°C | 1.23 (6H, s), 1.55 (9H, s), 2.63 (3H, s), 2.67 (2H, s), 4.52 (2H, s), 6.96-7.01 (2H, m), 7.37-7.42 (2H, m). |
| I-169 | 55-56°C | 1.24 (6H, s), 2.65 (3H, s), 2.69 (2H, s), 4.53 (2H, s), 7.19 (1H, d, J=7.6), 7.26-7.27 (1H, m), 7.40-7.52 (2H, m). |
| 1-170 | 88-90°C | 1.24 (6H, s), 2.65 (3H, s), 2.69 (2H, s), 4.53 (2H, s), 7.10 (2H, d, J=8.2), 7.63 (2H, d, J=8.2). |
| I-171 | | 1.15 (6H, s), 1.18 (6H, d, J=6.9), 2.17 (3H, s), 2.31 (3H, s), 2.64 (2H, s), 3.11 (1H, sept, J=6.9), 3.78 (2H, s), 6.80 (1H, d, J=8.2), 7.11- 7.18 (1H, m), 7.28-7.35 (1H, m). |
| I-172 | | 1.15 (6H, s), 1.18 (6H, d, J=6.9), 2.15 (3H, s), 2.31 (3H, s), 2.65 (2H, s), 3.11 (1H, sept, J=6.9), 3.78 (2H, s), 6.99 (1H, s), 7.11-7.18 (1H, m), 7.28-7.35 (1H, s). |
| I-173 | 121-123°C | 1.22 (6H, s), 2.64 (3H, s), 2.67 (2H, s), 3.89 (3H, s), 3.89 (3H, s), 4.54 (2H, s), 6.96 (1H, d, J=8.6), 7.67 (1H, d, J=2.1), 7.87 (1H, dd, J=8.6, 2.1). |
| I-174 | 146-147°C | 1.24 (6H, s), 2.59 (2H, s), 2.65 (3H, s), 2.96-2.99 (4H, m), 3.76-3.79 (4H, m), 4.52 (2H, s), 6.98-7.17 (4H, m). |
| I-175 | 155-157°C | 1.23 (6H, s), 2.64 (3H, s), 2.66 (2H, s), 3.16-3.20 (4H, m), 3.84-3.88 (4H, m), 4.51 (2H, s), 6.54-6.57 (2H, m), 6.70-6.74 (1H, m), 7.24-7.30 (1H, m). |
| 1-176 | | 1.22 (6H, d, J=6.6), 1.23 (6H, s), 1.38 (3H, t, J=7.1), 2.65 (3H, s), 2.67 (2H, s), 3.08-3.18 (1H, m), 4.37 (2H, q, J=6.9), 4.52 (2H, s), 7.38 (1H, d, J=7.9), 7.59 (1H, d, J=2.0), 7.82 (1H, dd, J=8.1, 1.8). |
| I-177 | 120-122°C | 1.23 (6H, s), 1.50-1.61 (2H, m), 1.67-1.75 (4H, m), 2.62 (3H, s), 2.66 (2H, s), 3.13-3.17 (4H, m), 4.50 (2H, s), 6.92-7.02 (4H, m). |
| I-178 | 124-125°C | 1.23 (6H, s), 1.85-1.90 (4H, m), 2.62 (3H, s), 2.68 (2H, s), 3.22-3.27 (4H, m), 4.48 (2H, s), 6.74-6.80 (2H, m), 6.95-6.98 (1H m), 7.03-7.10 (1H, m). |

**Table 32**

| Compound | Physical Data | |
|---|---|---|
| No. | m.p. | NMR (CDCl₃) |
| I-179 | | 1.23 (6H, s), 2.50 (3H, s), 2.64 (3H, s), 2.67 (2H, s), 4.51 (2H, s), 6.78-6.82 (1H, m), 6.91 (1H, t, J=2.0), 7.03-7.07 (1H, m), 7.25-7.31 (1H, m). |
| 1-180 | 102-103°C | 1.23 (6H, s), 2.49 (3H, s), 2.63 (3H, s), 2.67 (2H, s), 4.51 (2H, s), 6.96-7.01 (2H, m), 7.27-7.31 (2H, m). |
| I-181 | 82-83°C | 1.23 (6H, s), 2.64 (3H, s), 2.67 (2H, s), 4.52 (2H, s), 7.07 (1H, dd, J=7.6, 1.7), 7.14-7.20 (1H, m), 7.25-7-34 (2H, m). |
| I-182 | | 1.23 (6H, s), 2.64 (3H, s), 2.69 (2H, s), 4.52 (2H, s), 6.90 (1H, s), 6.93-7.04 (2H, m), 7.38 (1H, t, J=8.2) |
| 1-183 | 68-70°C | 1.24 (6H, s), 2.64 (3H, s), .2.69 (2H, s), 4.51 (2H, s), 7.01-7.07 (2H, m), 7.21-7.24 (2H, m). |
| I-184 | 169-170°C | 1.25 (6H, s), 2.66 (3H, s), 2.70 (2H, s), 4.54 (2H, s), 7.13-7.18 (2H, m), 7.34-7.39 (1H, m), 7.59-7.63 (2H, m), 7.86-7.91 (1H, m), 8.58 (1H, dd, J=4.8, 1-6), 8.87 (1H, t, J=1.5). |
| I-185 | 92.5-93.5°C | 1.24 (6H, s), 2.65 (3H, s), 2.69 (2H, s), 4.54 (2H, s), 7.05-7.09 (1H, m), 7.24 (1H, t, J=1.6), 7.34-7.40 (2H, m), 7.49 (1H, t, J=7.6), 7.87-7.92 (1H, m), 8.60 (1H, dd, J=4.9, 1.4), 8.87 (1H, dd, J=2.3, 0.7). |
| 1-186 | | 1.09 (6H, s), 2.56 (3H, s), 2.58 (2H, s), 4.20 (2H, s), 7.09-7.12 (1H, m), 7.24-7.30 (2H, m), 7.36-7.45 (2H, m), 7.75-7.79 (1H, m), 8.54 (1H, dd, J=4.9, 1.6), 8.68 (1H, dd, J=2.3, 0.7). |
| 1-187 | 110.5-111.5°C | 1.17 (6H, s), 2.51 (3H, s), 2.61 (2H, s), 4.33 (2H, s), 6.93-7.19 (7H, m), 7.23-7.30 (2H, m). |
| I-188 | 75-76°C | 1.14 (6H, s), 1.43 (6H, t, J=7.4), 2.61 (2H, s), 3.65 (2H, s), 3.84 (3H, s), 4.08 (4H, q, J=7.4), 6.46 (1H, dd, J=8.1, 2.2), 6.52 (1H, d, J=2.2), 6.84 (1H, d, J=8.4). |
| I-189 | | 1.19 (6H, s), 2.61 (2H, s), 3.65 (2H, s), 3.85 (3H, s), 3.88 (3H, s), 6.85-6.99 (3H, m), 7.02-7.15 (1H, m). |

**Table 33**

| Compound | Physical Data | |
|---|---|---|
| No. | m.p. | NMR (CDCl₃) |
| I-190 | | 1.13 (6H, s), 1.23 (3H, t, J=7.4), 2.62 (2H, s), 2.66 (2H, q, J=7.4), 3.64 (2H, s), 3.84 (3H, s), 6.84 (2H, d, J=8.6), 7.16 (2H, d, J=8.6). |
| I-191 | 45-47°C | 1.14 (6H, s), 1.25 (6H, d, J = 7.0), 2.62 (2H, s), 2.91 (1H, sept, J=7.0), 3.64 (2H, s), 3.84 (3H, s), 6.86 (2H, d, J=8.6), 7.19 (2H, d, J-8.6). |
| I-192 | 93-95°C | 1.15 (6H, s), 2.31 (3H, s), 2.62 (2H, s), 3.80 (2H, s), 3.85 (3H, s), 6.85-6.99 (3H, m), 7.02-7.15 (1H, m). |
| 1-193 | 65-67°C | 1.13 (6H, s), 1.23 (3H, t, J=7.4), 2.31 (3H, s), 2.62 (2H, s), 2.65 (2H, q, J=7.4), 3.77 (2H, s), 6.90 (2H, d, J=8.3), 7.21 (2H, d, J=8.3). |
| I-194 | 95-97°C | 1.15 (6H,s), 1.24 (6H, d, J=7.0), 2.31 (3H, s), 2.64 (2H, s), 2.91 (1H, sept, J=7.0), 3.77 (2H, s), 6.90 (2H, d, J=8.6), 7.21 (2H, d, J=8.6). |
| I-195 | 94-96°C | 1.15 (6H, s), 1.41 (3H, t, J=7.0), 2.31 (3H, s), 2.64 (2H, s), 3.77 (2H, s), 4.05 (2H, q, J=7.4), 6.90-6.99 (4H, m). |
| I-196 | 99-100°C | 1.15 (6H, s), 1.47 (3H, t, J=7.0), 2.32 (3H, s), 2.66 (2H, s), 3.77 (2H, s), 3.88 (3H, s), 4.08 (2H, q, J=7.0), 6.52 (1H, d, J= 8.2), 6.56 (1H, d, J=2.1), 6.88 (1H, d, J=8.2) . |
| I-197 | 133-134°C | 1.23 (6H, s), 1.50-1.75 (6H, m), 2.63 (3H, s), 2.65 (2H, s), 3.18 (4H, t, J=5.4), 4.51 (2H, s), 6.47-6.57 (2H, m), 6.72-6.76 (1H, m), 7.21 (1H, d, J=8.1) |
| I-198 | 124-125°C | 1.17 (6H, t, J=6.9), 1.23 (6H, s), 2.61 (3H, s), 2.68 (2H, s), 3.35 (4H, q, J=6.9), 4.49 (2H, s), 6.68 (2H, d, J=8.9), 7.04 (2H, d, J=8.9) |
| I-199 | 85-87°C | 1.22 (6H, s), 2.63 (3H, s), 2.67 (2H, s), 3.89 (3H, s), 3.92 (3H, s), 4.54 (2H, s), 7.01 (1H, d, J=7.9), 7.62 (1H, d, J=1.3), 7.67 (1H, dd, J=7.9, 1.7) |
| I-200 | 137-138°C | 1.23 (6H, s), 2.11-2.22 (2H, m), 2.62 (2H, t, J=7.9), 2.64 (3H, s), 2.67 (2H, s), 3.88 (2H, t, J=7.1), 4.52 (2H, s), 6.81-6.84 (1H, m), 7.30-7.50 (3H, m) |

**Table 34**

| Compound | Physical Data | |
|---|---|---|
| No. | m.p. | NMR (CDCl₃) |
| I-201 | 86.5-87.5°C | 1.22 (6H, s), 2.62 (3H, s), 2.67 (2H, s), 4.50 (2H, s), 6.71 (1H, t, J=2.0), 6.76-6.82 (2H, m), 7.02-7.13 (3H, m), 7.29-7.37 (3H, m) |
| I-202 | 162-163°C | 1.25 (6H, s), 2.65 (3H, s), 2.70 (2H, s), 4.54 (2H, s), 7.10-7.14 (2H, m), 7.33-7.46 (3H, m), 7.59-7.63 (4H, m) |
| I-203 | 56.5-57.5°C | 1.06 (6H, s), 2.51 (3H, s), 2.59 (2H, s), 4.14 (2H, s), 7.07 (1H, dd, J=8.2, 1.3), 7.21-7.45 (8H, m) |
| I-204 | 97-99°C | 1.24 (6H, s), 2.65 (3H, s), 2.68 (2H, s), 4.54 (2H, s), 7.00-7.04 (1H, m), 7.25-7.26 (1H, m), 7.33-7.48 (5H, m), 7.60-7.63 (2H, m) |
| I-205 | 95-96°C | 1.21 (6H, s), 1.21 (6H, d, J=6.9), 2.61 (2H, s), 4.13(3H, s), 4.16 (2H, s), 6.77-6-81 (1H, m), 7.13-7.16 (2H, m), 7.29-7.33 (1H, m) |
| I-206 | 128-129°C | 1.18 (6H, d, J=6.9), 1.22 (6H, s), 2.63 (3H, s), 2.66 (2H, s), 2.96-3.06 (1H, m), 4.48 (2H, s), 6.67 (1H, d, J=8.2), 7.47 (1H, dd, J=8.2, 1.7), 7.59 (1H, d, J=2.0) |
| 1-207 | 149-150°C | 1.23 (6H, s), 2.63 (3H, s), 2.67 (2H, s), 3.71 (8H, m), 3.86 (3H, s), 4.53 (2H, s), 6.95-7.05 (3H, m) |
| I-208 | 124-126°C | 1.23 (6H, s), 2.61 (3H, s), 2.67 (2H, s), 2.96 (6H, s), 4.50 (2H, s), 6.74 (2H, d, J=8.2), 7.04 (2H, d, J=8.2). |
| I-209 | 107-109°C | 1.23 (6H, s), 2.63 (3H, s), 2.65 (2H, s), 2.96 (6H, s), 4.51 (2H, s), 6.34 (1H, d, J=2.0), 6.38 (1H, d, J=8.0), 6.54 (1H, dd, J=8.0, 2.0), 7.24 (2H, d, J=8.0). |
| I-210 | 98-99°C | 1.06 (3H, t, J=7.4), 1.23 (6H, s), 2.63 (5H, s), 2.65 (3H, s), 2.99 (2H, q, J=7.4), 4.51 (2H, s), 6.98-7.10 (3H, m), 7.15-7.20 (1H, m). |
| I-211 | 94-96°C | 0.84 (3H, t, J = 7.4), 1.22 (6H, s), 1.49 (2H, sext, J = 7.3), 2.63 (3H, s), 2.65 (2H, s), 2.72 (3H, s), 2.84 (2H, t, J = 7.4), 4.51 (2H, s), 6.90-7.05 (3H, m), 7.10-7.15 (1H, m). |

**Table 35**

| Compound | Physical Data | |
|---|---|---|
| No. | m.p. | NMR (CDCl₃) |
| I-212 | 98-99°C | 1.02 (6H, t, J=7.4), 1.22 (6H, s), 2.61 (2H, s), 2.63 (3H, s), 3.06 (4H, q, J=7.4), 4.51 (2H, s), 6.98-7.10 (4H, m). |
| I-213 | 83-84°C | 1.23 (6H, s), 2.64 (3H, s), 2.71 (2H, s), 4.57 (2H, s), 6.90-7.12 (3H, m) |
| I-214 | | 1.19 (6H, d, J=6.9), 1.23 (6H, s), 2.64 (3H, s), 2.67 (2H, s), 3.06 (1H, sept, J=6.9), 4.49 (2H, s), 6.85 (1H, d, J=8.2), 7.14 (1H, dd, J=8.2, 2.3), 7.27 (1H, d, J=2.3) |
| I-215 | 83-85°C | 1.23 (6H, s), 2.32 (3H, s), 2.63 (3H, s), 2.66 (2H, s), 2.71 (6H, s), 4.50 (2H, s), 6.75-6.80 (1H, m), 6.98 (1H, s), 6.97-7.00 (1H, m). |
| I-216 | 99-100°C | 1.23 (6H, s), 2.33 (3H, s), 2.62 (3H, s), 2.65 (2H, s), 2.70 (6H, s), 4.50 (2H, s), 6.78 (2H, t, J=7.9), 6.91 (1H, d, J=7.9). |
| I-217 | 98-99°e | 1.23 (6H, s), 2.30 (3H, s), 2.63 (3H, s), 2.64 (2H, s), 2.67 (6H, s), 4.50 (2H, s), 6.81 (1H, s), 6.92 (2H, s). |
| I-218 | 117-119°C | 1.23 (6H, s), 2.63 (3H, s), 2.65 (2H, s), 2.68 (6H, s), 4.50 (2H, s), 6.89 (1H, d, J=8.5), 6.99 (1H, d , J=2.0), 7.04 (1H, dd , J=7.9, 2.0). |
| I-219 | 68-70°C | 1.22 (6H, s), 2.22 (6H, s), 2.64 (3H, s), 2.66 (2H, s), 4.54 (2H, s), 6.93-6.98 (1H, m), 7.04 (2H, d, J=8.0). |
| 1-220 | 97.99°C | 1.22 (6H, s), 1.34 (3H, t, J=7.4), 2.64 (2H, s), 2.72 (6H, s), 3.25 (2H, q, J=7.4), 4.47 (2H, s), 6.94-7.05 (3H, m), 7.15-7.20 (1H, m). |
| I-221 | 118-119°C | 1.22 (6H, s) 1.34 (3H, t, J=7.4), 2.64 (2H, s), 2.95 (6H, s), 3.25 (2H, q, J=7.4), 4.47 (2H, s), 6.34 (1H, d, J=7.5), 6.38 (1H, s), 6.52 (1H, d, J=7.5), 7.24 (1H, t, J=7.5). |
| I-222 | 74-76°C | 1.22 (6H, s), 1.34 (3H, t, J=7,4), 2.33 (3H, s), 2.63 (2H, s), 2.70 (6H, s), 3.25 (2H, q, J=7.4), 4.47 (2H, s), 6.78 (1H, d, J=7.5), 6.82 (1H, s), 6.91 (1H, t, J=7.5). |

**Table 36**

| Compound | Physical Data | |
|---|---|---|
| No. | m.p. | NMR (CDCl₃) |
| I-223 | | 1.22 (6H, s), 1.25 (6H, d, J=7.0), 1.34 (3H, t, J=7.4), 2.65 (2H, s), 2.91 (1H, sept, J=7.0), 3.25 (2H, q, J=7.4), 4.50 (2H, s), 6.98 (2H, d, J=8.2), 7.28 (2H, d, J = 8.2). |
| 1-224 | | 1.21 (6H, s), 2.62 (3H, s), 2.66 (2H, s), 2.97 (3H, d, J=4.9), 3.84 (3H, s), 4.51 (2H, s), 6.66 (1H, brs), 6.96 (1H, d, J=7.9), 7.30-7.33 (1H, m), 7.49 (1H, d, J=1.3) |
| I-225 | 69-71°C | 1.23 (6H, s), 2.64 (3H, s), 2.68 (2H, s), 4.52 (2H, s), 6.49 (1H, t, J=74.6), 7.04-7.26 (4H, m) |
| 1-226 | | 1.23 (6H, s), 2.64 (3H, s), 2.68 (2H, s), 4.51 (2H, s), 6.50 (1H, t, J=74.2), 7.00-7.05 (2H, s), 7.11-7.16 (2H, m) |
| 1-227 | 81-83°C | 1,17 (6H, t, J=7.0), 1.23 (6H, s), 2.63 (3H, s), 2.66 (2H, s), 3.35 (4H, q, J=7.0), 4.52 (2H, s), 6.29 (1H, s), 6.30 (1H, d,t, J=8.2,2.3), 6.49 (1H, dd, J=8.2, 2.3), 7.19 (1H, t, J=8.2). |
| I-228 | 106-107°C | 1.21 (6H, s), 2.61 (3H, s), 2.64 (2H, s), 2.70 (6H, s), 4.47 (2H, s), 6.90 (2H, s), 6.93 (1H, s). |
| 1-229 | 121-122°C | 1.23 (6H, s), 2.62 (3H, s), 2.65 (2H, s), 2.70 (6H, s), 4.48 (2H, s), 6.50-6.70 (2H, m),6.93 (1H, dd, J=8.5, 6.2). |
| I-230 | 85-86°C . | 1.21 (6H, s), 2.63 (3H, s), 2.64 (2H, s), 2,66 (6H, s), 4.49 (2H, s),6.74-6.79 (2H, m), 6.93-6.98 (1H, m). |
| I-231 | 82-84°C | 1.23 (6H, s), 1.25 (3H, t, J=7.6), 2.62 (3H, s), 2.66 (2H, s), 2.67 (2H, q, J=7.6), 2.71 (6H, s), 4.50 (2H, s), 6.80 (1H, d, J=7.6), 6.84 (1H, s), 6.93 (1H, d, J=7.6). |
| I-232 | 75-76°C | 1.22 (3H, t, J=7.6), 1.23 (6H, s), 2.60 (2H, q, J=7.6), 2.63 (3H, s), 2.64 (2H, s), 2.68 (6H, s), 4.50 (2H, s), 6.83 (1H, s), 6.93 (2H, s). |
| I-233 | 86-88°C | 1.22 (6H, s), 1.33 (3H, t, J=7.4), 2.64 (2H, s), 2.71 (6H, s), 3.24 (2H, q, J=7.4), 4.47 (2H, s), 6.92 (2H, s), 6.94(1H, s). |

**Table 37**

| Compound | Physical Data | |
|---|---|---|
| No. | m.p. | NMR (CDCl₃) |
| I-234 | 70-71°C | 1.22 (6H, s), 1.34 (3H, t, J=7.4), 2.64 (2H, s), 2.71 (6H, s), 3.25 (2H, q, J=7.4), 4.46 (2H, s), 6.60-6.68 (2H, m), 6.92-6.94(1H, m). |
| I-235 | 80-82°C | 1.22 (6H, s), 1.24 (3H, t, J=7.6), 1.33 (3H, t, J=7.4), 2.60 (2H, q, J=7.6), 2.61 (2H, s), 2.71 (6H, s), 3.24 (2H, q, J=7.4), 4.47 (2H, s), 6.81 (1H, d, J=7.6), 6.94(1H, s), 6.94 (1H, d, J=7.6). |
| I-236 | | 1.03 (3H, t, J=7.3), 1.20 (6H, d, J=6.9), 1.23 (6H, s), 1.40 (3H, d, J=6.9), 1.61-1.89 (2H, m), 2.63 (2H, s), 3.15 (1H, sept, J=6.9), 3.95 (1H, q, J=6.9), 4.47 (2H, s), 6.89-6.92 (1H, m), 7.13-7.20 (2H, m), 7.31-7.34 (1H, m) |
| 1-237 | | 1.05 (6H, d, J=6.6), 1.21 (6H, d, J=6.6), 1.23 (6H, s), 1.98-2.08 (1H, m), 2.64 (2H, s), 3.16 (1H, sept, J=6.6), 3.20 (2H, d, J=6.6), 4.49 (2H, s), 6.88-6.92 (1H, m), 7.13-7.22 (2H, m), 7.30-7.35 (1H, m) |
| I-238 | 102-104°C | 1.20 (6H, d, J=6.9), 1.22 (6H, s), 2.61 (2H, s), 2.85-2.95 (1H, m), 3.19 (3H, d, J=4.6), 4.46 (2H, s), 6.73-6.79 (1H, m), 7.14-7.20 (2H, m), 7.29-7.34 (1H, m), 12.40 (1H, brs) |
| I-239 | 58-60°C | 1.23 (6H, s), 2.17 (3H, s), 2.64 (3H, s), 2.65 (2H, s), 2.70 (6H, s), 4.52 (2H, s), 6.63 (1H, d, J=7.9), 6.87 (1H, d, J=7.9), 7.14 (1H, d, J=7.9). |
| I-240 | 100-101°C | 1.23 (6H, s), 2.62 (3H, s), 2.64 (2H, s), 2.78 (6H, s), 3.89 (3H, s), 4.52 (2H, s), 6.60-6.70 (2H, m), 6.94 (1H, d, J=7.9). |
| I-241 | 82-83°C | 1.23 (6H, s), 2.30 (3H, s), 2.63 (3H, s), 2.65 (2H, s), 2.70 (6H, s), 4.52 (2H, s), 6.63 (1H, d,t, J=7.9,1.9), 6.70 (1H, d , J=1.9), 7.14 (1H, d, J=7.9). |
| I-242 | 99-100°C | 1.23 (6H, s), 2.63 (3H, s), 2.68 (2H, s), 2.81 (6H, s), 4.50 (2H, s), 6.91 (1H, d,t, J=8.4,2.6), 7.06 (1H, d , J=8.4), 7.14 (1H, d, J=2.6). |
| I-243 | 63-64°C | 1.23 (6H, s), 2.63 (3H, s), 2.67 (2H, s), 2.78 (6H, s), 3.89 (3H, s), 4.52 (2H, s), 6.67 (1H, s), 6.70 (1H, d, J=7.9), 6.81 (1H, d , J=7.9). |
| I-244 | 68-70°C | 0.88 (6H, t, J=7.5), 1.22 (6H, d, J=6.9), 1.35 (3H, t, J=7.4), 1.50-1.70 (4H, m), 2.61 (2H, s), 3.15 (1H, sept, J=6.9), 3.29 (2H, q, J=7.4), 4.44 (2H, s), 6.89-6.92 (1H, m), 7.08-7.21 (2H, m), 7.30-7.35 (1H, m). |

**Table 38**

| Compound | Physical Data | |
|---|---|---|
| No. | m.p. | NMR (CDCl₃) |
| 1-245 | 81-82°C | 1.14 (6H, s), 1.20 (6H, d, J=6.9), 2.63 (2H, s), 3.06 (2H, s), 3.08 (1H, sept, J=6.9), 3.18 (3H, s), 6.74 (1H, dd, J=7.3, 1.7), 6.98-7.10 (2H, m), 7.20-7.24 (1H, m) |
| I-246 | 47-49°C | 0.95 (3H, t, J=7.3), 1.13 (6H, s), 1.20 (6H, d, J=6.9), 1.55-1.74 (2H, m), 2.62 (2H, s), 3.03-3.11 (3H, m), 3.52-3.57 (2H, m), 6.73 (1H, dd, J=7.6, 1.7), 6.96-7-10 (2H, m), 7.21 (1H, dd, J=7.3, 1.7) |
| I-247 | 68-70°C | 1.11 (6H, s), 1.18 (6H, d, J=6.9), 1.19 (6H, d, J=6.9), 2.56 (2H, s), 2.89 (2H, s), 3.08 (1H, sept, J=6.9), 5.08 (1H, sept, J=6.9), 6.73 (1H, dd, J=7.9, 1.7), 6.99-7.10 (2H, m), 7.21 (1H, dd, J=7.9, 1.7) |
| I-248 | | 0.97 (6H, d, J=6.9), 1.14 (6H, s), 1.18 (6H, d, J=6.9), 2.05-2.15 (1H, m), 2.62 (2H, s), 3.07 (2H, s), 3.08 (1H, sept, J=6.9), 3.44 (2H, d, J=7.6), 6.71(1H, dd, J=7.6, 1.7), 6.96-7.09 (2H, m), 7.21 (1H, dd, J=7.6, 1.7) |
| I-249 | 96-97°C | 1.23 (6H, s), 2.64 (3H, s), 2.68 (2H, s), 4.59 (2H, s), 7.04 (1H, d, J=7.3), 7.41-7.50 (3H, m), 7.67 (1H, d, J=7.3), 7.87 (1H, dd, J = 7.3, 2.1), 8.05 (1H, d, J=7.3). |
| I-250 | 108-109°C | 1.24 (6H, s), 2.67 (3H, s), 2.69 (2H, s), 4.59 (2H, s), 7.15 (1H, d, J=7.3), 7.41 (1H, q, J=7.3), 7.69 (1H, t, J=8.4), 7.91 (1H, d, J=7.3), 8.45 (1H, d, J=8.4), 8.92-8.95 (1H, m). |
| I-251 | 105-107°C | 1.22 (6H, s), 2.62 (3H, s), 2.65 (2H, s), 3.97 (3H, s), 4.53 (2H, s), 6.87-6.90 (1H, m), 7.25-7.30 (1H, m), 7.96-7.99 (1H, m). |
| I-252 | 132-133°C | 1.23 (6H, s), 2.63 (3H, s), 2.68 (2H, s), 2.92 (3H, s), 4.49 (2H, s), 6.73-6.78 (1H, m), 7.20-723 (1H, m), 8.05-8.07 (1H, m) |
| I-253 | 118-120°C | 1.23 (6H, s), 2.60 (3H, s), 2.63 (2H, s), 4.52 (2H, s), 7.30 (2H, s), 8.12 (1H, s) |
| I-254 | 112-113°C | 1.23 (6H, s), 2.63 (3H, s), 2.69 (2H, s), 3.94 (3H, s), 4.51 (2H, s), 6.76 (1H, d, J = 8.1), 7.35 (1H, dd, J = 8.1, 2.1), 7.92 (1H, d, J = 2.1). |
| I-255 | 109-110°C | 1.23 (6H, s), 1.40 (3H, t, J=7.0), 2.62 (3H, s), 2.66 (2H, s), 4.38 (2H, q, J=7.0), 4.51 (2H, s), 6.75 (1H, d, J= 8.1). 7.35 (1H, dd, J=8.1, 2.1), 7.90 (1H, d, J=2.1). |

**Table 39**

| Compound | Physical Data | |
|---|---|---|
| No. | m.p. | NMR (CDCl₃) |
| I-256 | 75-76°C | 1.03 (3H, t, J=7.6), 1.22 (6H, s), 1.76 (2H, sext, J= 7.6), 2.63 (3H, s), 2.65 (2H, s), 4.24 (2H, t, J=7.6), 4.51 (2H, s), 6.76 (1H, d, J=8.1), 7.35 (1H, dd, J=8.1, 2.1), 7.92 (1H, d, J=2.1). |
| 1-257 | 74-76°C | 1.24 (6H, s), 1.36 (6H, d, J=6.3), 2.63 (3H, s), 2.70 (2H, s), 4.51 (2H, s), 5.28 (1H, sept, J=6.3), 6.70 (1H, d, J=8.1), 7.32 (1H, dd, J=8.1, 2.1), 7.92 (1H, d, J=2.1). |
| I-258 | 102-104°C | 1.23 (6H, s), 2.58 (3H, s), 2.63 (2H, s), 2.69 (3H, s), 4.51 (2H, s), 7.20-7.26 (2H, m), 8.21 (1H, d, J=2.1). |
| I-259 | 81-83°C | 1.23 (6H, s), 1.38 (3H, t, J=7.3), 2.63 (3H, s), 2.63 (2H, s), 3.18 (2H, q, J=7.3), 4.51 (2H, s), 7.15-7.26 (2H, m), 8.21 (1H, d, J=2.1). |
| I-260 | 78-79°C | 1.05 (3H, t, J = 7.4), 1.23 (6H, s), 1.75 (2H, sext, J=7.3), 2.63 (3H, s), 2.65 (2H, s), 3.15 (2H, t, J=7.4), 4.51 (2H, s), 7.15-7.26 (2H, m), 8.20 (1H, d, J=2.1). |
| I-261 | 102-103°C | 1.23 (6H, s), 1.40 (6H, d, J=6.6), 2.63 (3H, s), 2.66 (2H, s), 4.00 (1H, sept, J=6.6), 4.51 (2H, s), 7.15-7.26 (2H, m), 8.22 (1H, d, J=2.1). |
| I-262 | 109-110°C | 1.22 (6H, s), 2.61 (3H, s), 2.65 (2H, s), 2.70 (6H, s), 3.80 (3H, s), 4.48 (2H, s), 6.47 (1H, dd, J=7.9, 2.1), 6.56 (1H, d, J=2.1), 6.95 (1H, d, J=7.9). |
| I-263 | 99-100°C | 1.22 (6H, s), 2.62 (3H, s), 2.63 (2H, s), 2.64 (6H, s), 3.78 (3H, s), 4.48 (2H, s), 6.59 (1H, d, J=2.1), 6.64 (1H, dd, J=7.9, 2.1), 6.98 (1H, d, J=7.9). |
| I-264 | 114-115°C | 0.98 (6H, t, J=7.0), 1.23 (6H, s), 2.16 (3H, s), 2.63 (3H, s), 2.64 (2H, s), 2.98 (4H, q, J=7.0), 4.52 (2H, s), 6.65 (1H, d, J=7.9), 6.89 (1H, d, J=7.9), 7.13 (1H, t, J=7.9). |
| I-265 | 66-67°C | 0.98 (6H, t, J=7.0), 1.23 (6H, s), 2.16 (3H, s), 2.63 (3H, s), 2.64 (2H, s), 2.98 (4H, q, J=7.0), 4.52 (2H, s), 6.63 (1H, dd, J=7.9,2.1), 6.70 (1H, d , J=2.1), 7.16 (1H, d, J = 7.9). |
| I-266 | 88-90°C | 1.04 (6H, t, J=7.0), 1.24 (6H, s), 2.63 (3H, s), 2.67 (2H, s), 3.17 (4H, q, J=7.0), 3.86 (3H, s), 4.51 (2H, s), 6.67 (1H, s), 6.70 (1H, d , J=7.9), 6.85 (1H, d , J=7.9). |

**Table 40**

| Compound | Physical Data | |
|---|---|---|
| No. | m.p. | NMR (CDCl₃) |
| I-267 | 138-140°C | 0.82-0.92 (9H, m), 1.18 (3H, d, J=6.9), 1.51-1.65 (6H, m), 2.62 (2H, s), 2.65 (3H, s), 2.87 (1H, sept, J=6.9), 4.33 (1H, d, J=13.5), 4.59 (1H, d, J=13.5), 6.89-6.92 (1H, m), 7.13-7.28 (3H, m) |
| 1-268 | 161-163°C | 0.89-0.95 (6H, m), 1.21 (6H, d, J=6.9), 1.25-1.54 (8H, m), 2.62 (2H, s), 2.65 (3H, s), 3.10 (1H, sept, J=6.9), 4.47 (2H, s), 6.88-6.92 (1H, m), 7.14-7.18 (2H, m), 7.31-7.34 (1H, m) |
| I-269 | | 1.21 (6H, d, J=6.9), 1.65-1.88 (8H, m), 2.64 (3H, s), 2.75 (2H, s), 3.09 (1H, sept, J=6.9), 4.57 (2H, s), 6.90-6.94 (1H, m), 7.13-7-20 (2H, m), 7.30-7.35 (1H, m) |
| 1-270 | | 1.21 (6H, d, J=6.9), 1.37-1.54 (8H, m), 1.76-1.80 (2H, m), 2.65 (3H, s), 2.67 (2H, s), 3.09 (1H, sept, J=6.9), 4.54 (2H, s), 6.89 (1H, m), 7.11-7.21 (2H, m), 7.29-7.34 (1H, m) |

**Table 41**

| Compound | Physical data | |
|---|---|---|
| No. | m.p. | NMR (CDCl₃) |
| I-271 | | 1.04 (3H, s), 1.08 (3H, s), 1.29 (6H, d), J=6.9), 2.69(2H, s), 3.40 (1H, sept, J=6.9), 3.43 (3H, s), 3.51 (2H, s), 7.18-7.29 (2H, m), 7.36-7.45 (2H, m) |
| I-272 | | 0.96 (3H, s), 1.05 (3H, s), 1.25 (3H, d, J=6.9), 1.26 (3H, d, J=6.9), 2.61 (1H, d, J=12), 2.70 (1H, d, J=12), 3.39 (1H, sept, J=6.9), 3.45-3.58 (2H, m), 7.02.7.07 (2H, m), 7.11-7.18 (1H, m), 7.38-7.45 (2H, m), 7.61-7.70 (2H, m) |
| 1-273 | | 0.84 (3H, s), 1.00 (3H, s), 1.25 (3H, d, J=6.9), 1.29 (3H, J=6.9), 2.43 (3H, s), 2.53 (1H, d, J=12), 2.64 (1H, d, J=12), 3.29 (1H, d, J=16), 3.42 (1H, d, J=16), 3.47 (1H, sept, J=6.9), 7.09-7.19 (2H, m), 7.24-7.29 (2H, m), 7.38-7.45 (2H, m), 7.81-7.86 (2H, m) |
| 1-274 | | 0.99 (6H, s), 1.19 (6H, d, J=6.9), 2.40 (3H, s), 2.67 (2H, s), 2.87 (1H, sept, J=6.9), 3.43 (2H, s), 7.11-7.29 (6H, m), 7.68 (2H, d, J=8.1) |
| 1-275 | | 1.07 (6H, s), 1.26 (6H, d, J=6.9), 1.38 (3H, t, J=7.2), 2.71 (2H, s), 2.93 (1H, sept, J=6.9), 3.51 (2H, s), 3.60 (2H, q, J=7.2), 7.20-7.30 (4H, m) |
| I-276 | | 1.19 (6H, s), 1.23 (6H, d, J=6.9), 2.77 (2H, s), 2.87 (1H, sept, J=6.9), 3.58 (2H, s), 6.65-6.69 (2H, m), 6.91 (1H, d, J=7.5), 7.20 (1H, t, J=7.5), 7.51 (2H, d, J=9.3), 8.22 (2H, d, J=9.3) |
| 1.277 | | 0.99 (6H, s), 1.20 (6H, d, J=6.9), 2.67 (2H, s), 2.88 (1H, sept, J=6.9), 3.44 (2H, s), 3.85 (3H, s), 6.86-6.90 (2H, m), 7.11-7.26 (4H, m), 7.72. 7.76 (2H, m) |

**Table 42**

| Compound | Physical Data | |
|---|---|---|
| No. | m.p. | NMR (CDCl₃) |
| I-278 | | 1.03 (6H, s), 1.20 (6H, d, J=6.9), 2.70 (2H, s), 2.88 (1H, sept, J=6.9), 3.44 (2H, s), 7.08-7.31 (4H, m), 7.60 (1H, t, J=8.4), 8.04 (1H, d, J=8.4), 8.39 (d, J=8.4), 8.74 (1H, s) |
| I-279 | | 1.01 (6H, s), 1.19 (6H, d, J=6.9), 2.69 (2H, s), 2.88 (1H, sept, J=6.9), 3.42 (2H, s), 7.09-7.32 (4H, m), 7.68 (2H, d, J=8.4), 7.92 (2H, d, J=8.4), |
| I-280 | | 1.19 (3H, s), 1.21 (3H, s), 1.23-1.30 (6H, m), 2.62 (1H, d, J=12), 2.82 (1H, sept, J=6.9), 3.02 (1H, d, J=12), 3.46-3.70 (2H, m), 6.53-6.60 (2H, m), 6.86 (1H, d, J=7.8), 7.13 (1H, t, J=7.8), 7.28-7.40 (2H, m), 7.61-7.66 (1H, m), 7.90 (1H, dd, J=7.5, 1.2) |

**Table 43**

| | | | | | | | | |
|---|---|---|---|---|---|---|---|---|
| 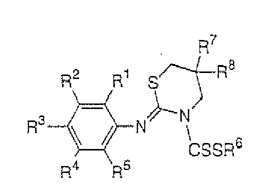 | | | | | | | | |

| No. | R¹ | R² | R³ | R⁴ | R⁵ | R⁶ | R⁷ | R⁸ |
|---|---|---|---|---|---|---|---|---|
| II-1 | Pr*ⁱ* | H | H | H | H | Allyl | Me | Me |
| II-2 | Pr*ⁱ* | H | H | H | H | Propargyl | Me | Me |
| II-3 | Pr*ⁱ* | H | H | H | H | CH₂CN | Me | Me |
| II-4 | Pr*ⁱ* | H | H | H | H | CH₂OMe | Me | Me |
| II-5 | Pr*ⁱ* | H | H | H | H | CH₂CH=CHMe | Me | Me |
| II-6 | Pr*ⁱ* | H | H | H | H | CH₂CH=CMe₂ | Me | Me |
| II-7 | Pr*ⁱ* | H | H | H | H | CH₂CH₂CH=CH₂ | Me | Me |
| II-8 | Pr*ⁱ* | H | H | H | H | CH₂COMe | Me | Me |
| II-9 | Pr*ⁱ* | H | H | H | H | CH₂CO₂H | Me | Me |
| II-10 | Pr*ⁱ* | H | H | H | H | CH₂CO₂Me | Me | Me |
| II-11 | Pr*ⁱ* | H | H | H | H | CH₂CO₂Et | Me | Me |
| II-12 | Pr*ⁱ* | H | H | H | H | CH₂CO₂Pr | Me | Me |
| II-13 | Pr*ⁱ* | H | H | H | H | CH₂CO₂Pr*ⁱ* | Me | Me |
| II-14 | Pr*ⁱ* | H | H | H | H | CH₂CO₂Bu*^{t}* | Me | Me |
| II-15 | Pr*ⁱ* | H | H | H | H | CH₂CO₂CH=CH₂ | Me | Me |
| II-16 | Pr*ⁱ* | H | H | H | H | CH₂CO₂CH₂CH=CH₂ | Me | Me |
| II-17 | Pr*ⁱ* | H | H | H | H | CH₂CO₂(CH₂)₂OMe | Me | Me |
| II-18 | Pr*ⁱ* | H | H | H | H | CH(Me)CO₂Me | Me | Me |
| II-19 | Pr*ⁱ* | H | H | H | H | C(Me)₂CO₂Et | Me | Me |
| II-20 | Pr*ⁱ* | H | H | H | H | CH₂CONH₂ | Me | Me |
| II-21 | Pr*ⁱ* | H | H | H | H | CH₂CONMe₂ | Me | Me |
| II-22 | Pr*ⁱ* | H | H | H | H | CH₂CON(Me)OMe | Me | Me |
| II-23 | Pr*ⁱ* | H | H | H | H | CH₂CF₃ | Me | Me |
| II-24 | Pr*ⁱ* | H | H | H | H | CH₂CH₂OCOMe | Me | Me |
| II-25 | Pr*ⁱ* | H | H | H | H | CH₂CH₂OPh | Me | Me |

**Table 44**

| | | | | | | | | |
|---|---|---|---|---|---|---|---|---|
| 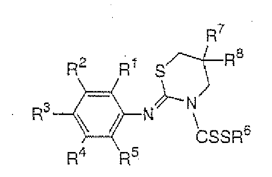 | | | | | | | | |

| No. | R¹ | R² | R³ | R⁴ | R⁵ | R⁶ | R⁷ | R⁸ |
|---|---|---|---|---|---|---|---|---|
| II-26 | Pr*ⁱ* | H | H | H | H | CH₂CH₂OCH=CH₂ | Me | Me |
| II-27 | Pr*ⁱ* | H | H | H | H | 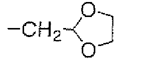 | Me | Me |
| II-28 | Pr*ⁱ* | H | H | H | H | 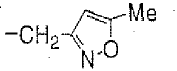 | Me | Me |
| II-29 | Pr*ⁱ* | H | H | H | H | 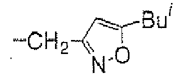 | Me | Me |
| II-30 | Pr*ⁱ* | H | H | H | H | 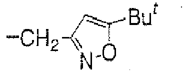 | Me | Me |
| II-3 | Pr*ⁱ* | H | H | H | H | 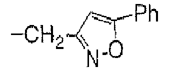 | Me | Me |
| II-32 | Pr*ⁱ* | H | H | H | H | 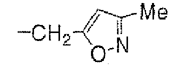 | Me | Me |
| II-33 | Pr*ⁱ* | H | H | H | H | 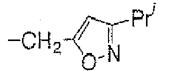 | Me | Me |
| II-34 | Pr*ⁱ* | H | H | H | H | 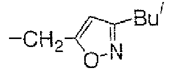 | Me | Me |
| II-35 | Pr*ⁱ* | H | H | H | H | 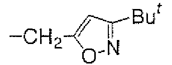 | Me | Me |
| II-36 | Pr*ⁱ* | H | H | H | H | 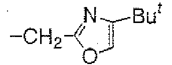 | Me | Me |
| II-37 | Pr*ⁱ* | H | H | H | H | 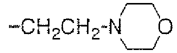 | Me | Me |
| II-38 | Pr*ⁱ* | H | H | H | H | 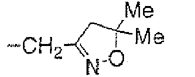 | Me | Me |
| II-39 | Pr*ⁱ* | H | H | H | H | Allyl | Et | Et |
| II-40 | Pr*ⁱ* | H | H | H | H | CH₂CO₂Et | Et | Et |
| II-41 | Pr*ⁱ* | H | H | H | H | CH₂CO₂Pr*ⁱ* | Et | Et |
| II-42 | Pr*ⁱ* | H | H | H | H | CH₂CO₂Bu*^{t}* | Et | Et |
| II-43 | Pr*ⁱ* | H | H | H | H | CH₂CH₂CO₂Et | Et | Et |

**Table 45**

| | | | | | | | | |
|---|---|---|---|---|---|---|---|---|
| 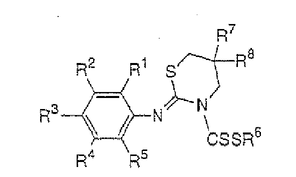 | | | | | | | | |

| No. | R¹ | R² | R³ | R⁴ | R⁵ | R⁶ | R⁷ | R⁸ |
|---|---|---|---|---|---|---|---|---|
| II-44 | Pr*ⁱ* | H | H | H | H | CH₂CH=CHMe | Et | Et |
| II-45 | Pr*ⁱ* | H | H | H | H | CH₂CH=CMe₂ | Et | Et |
| II-46 | Pr*ⁱ* | H | H | H | H | CH₂CH₂CH=CH₂ | Et | Et |
| II-47 | Bu*^{s}* | H | H | H | H | CH₂CO₂Et | Me | Me |
| II-48 | Bu*^{s}* | H | H | H | H | CH₂CO₂Bu*^{t}* | Me | Me |
| II-49 | Bu*^{s}* | H | H | H | H | Allyl | Et | Et |
| II-50 | Bu*^{s}* | H | H | H | H | CH₂CH₂OCOMe | Et | Et |
| II-51 | Bu*^{s}* | H | H | H | H | 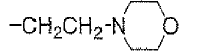 | | |
| II-52 | H | H | Et | H | H | CH₂CO₂Et | Me | Me |
| II-53 | H | Pr*ⁱ* | H | H | H | CH₂CO₂Et | Me | Me |
| II-54 | NMe ₂ | H | H | H | H | CH₂CO₂Et | Me | Me |
| II-55 | H | NMe₂ | H | H | H | CH₂CO₂Et | Me | Me |
| II-56 | H | NEt₂ | H | H | H | CH₂CO₂Et | Me | Me |
| II-57 | H | H | Et | H | H | CH₂CO₂Bu*^{t}* | Me | Me |
| II-58 | H | Pr*ⁱ* | H | H | H | CH₂CO₂Bu*^{t}* | Me | Me |
| II-59 | NMe ₂ | H | H | H | H | CH₂CO₂Bu*^{t}* | Me | Me |
| II-60 | H | NMe₂ | H | H | H | CH₂CO₂Bu*^{t}* | Me | Me |
| II-61 | H | NEt₂ | H | H | H | CH₂CO₂Bu*^{t}* | Me | Me |
| II-62 | H | NEt₂ | H | H | H | Allyl | Me | Me |
| II-63 | Me | NEt₂ | H | H | H | Allyl | Me | Me |
| II-64 | Me | NMe₂ | H | H | H | Allyl | Me | Me |
| II-65 | NMe ₂ | H | H | H | H | Allyl | Et | Et |
| II-66 | NMe ₂ | H | H | H | H | CH₂CO₂Bu*^{t}* | Et | Et |
| II-67 | OMe | H | H | H | H | Allyl | Et | Et |
| II-68 | OMe | H | H | H | H | CH₂CO₂Bu*^{t}* | Et | Et |
| II-69 | H | H | Et | H | H | Allyl | Et | Et |
| II-70 | H | H | Et | H | H | CH₂CO₂Bu*^{t}* | Et | Et |

**Table 46**

| | | | | | | | | |
|---|---|---|---|---|---|---|---|---|
| 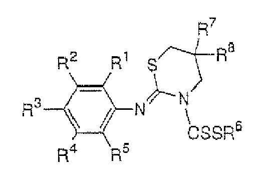 | | | | | | | | |

| No. | R¹ | R² | R³ | R⁴ | R⁵ | R⁶ | R⁷ | R⁸ |
|---|---|---|---|---|---|---|---|---|
| II-71 | H | H | OCF₃ | H | H | Allyl | Et | Et |
| II-72 | H | H | OCF₃ | H | H | CH₂CO₂Bu*^{t}* | Et | Et |
| II-73 | NMe₂ | H | H | H | H | CH₂OMe | Et | Et |
| II-74 | Pr*ⁱ* | H | H | H | H | Allyl | -(CH₂)₄- | |
| II-75 | NMe₂ | H | H | H | H | Allyl | -(CH₂)₄- | |
| II-76 | NMe₂ | H | H | H | H | CH₂CO₂Bu*^{t}* | -(CH₂)₄- | |
| II-77 | Pr*ⁱ* | H | H | H | H | CH₂CO₂(CH₂)₂OMe | -(CH₂)₄- | |
| II-78 | Pr*ⁱ* | H | H | H | H | 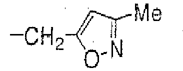 | -(CH₂)₄- | |
| II-79 | OMe | H | H | H | H | Allyl | -(CH₂)₄- | |
| II-80 | OMe | H | H | H | H | CH₂CO₂Bu*^{t}* | -(CH₂)₄- | |
| II-81 | NMe₂ | H | H | H | H | CH₂OMe | -(CH₂)₄- | |
| II-82 | H | H | Et | H | H | Allyl | -(CH₂)₄- | |
| II-83 | H | H | OCF₃ | H | H | Allyl | -(CH₂)₄- | |
| II-84 | NMe₂ | H | H | H | H | Allyl | -(CH₂)₅- | |
| II-85 | NMe₂ | H | H | H | H | CH₂CO₂Bu*^{t}* | -(CH₂)₅- | |
| II-86 | OMe | H | H | H | H | Ally | -(CH₂)₅- | |
| II-87 | OMe | H | H | H | H | CH₂CO₂Bu*^{t}* | -(CH₂)₅- | |
| II-88 | H | H | Et | H | H | Allyl | -(CH₂)₅- | |
| II-89 | Pr*ⁱ* | H | H | H | H | 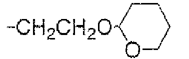 | -(CH₂)₅- | |
| II-90 | Pr*ⁱ* | H | H | H | H | CH₂CH₂OH | -(CH₂)₅- | |
| II-91 | H | H | OCF₃ | H | H | Allyl | -(CH₂)₅- | |
| II-92 | Pr*ⁱ* | H | H | H | H | Allyl | -(CH₂)₂O(CH₂)₂- | |
| II-93 | Pr*ⁱ* | H | H | H | H | Me | -(CH₂)₂O(CH₂)₂- | |
| II-94 | Pr*ⁱ* | H | H | H | H | CH₂CO₂H | Et | Et |

**Table 47**

| | | | | |
|---|---|---|---|---|
| 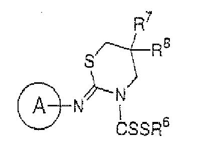 | | | | |

| No. | A | R⁶ | R⁷ | R⁸ |
|---|---|---|---|---|
| II-95 | 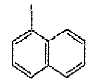 | Allyl | Me | Me |
| II-96 | 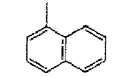 | CH₂CO₂Bu*^{t}* | Me | Me |
| II-97 | 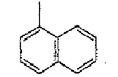 | CH₂CO₂(CH₂)₂OMe | Me | Me |
| II-98 | 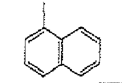 | Allyl | Et | Et |
| II-99 | 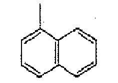 | CH₂CO₂Bu*^{t}* | Et | Et |
| II-100 | 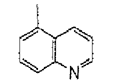 | Allyl | Et | Et |
| II-101 | 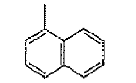 | Allyl | -(CH₂)₄- | |
| II-102 | 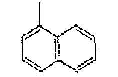 | CH₂CO₂Bu*^{t}* | -(CH₂)₄- | |
| II-103 | 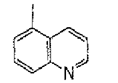 | Allyl | -(CH₂)₄- | |
| II-104 | 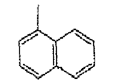 | Allyl | -(CH₂)₅- | |
| II-105 | 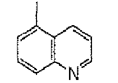 | Allyl | -(CH₂)₅- | |

**Table 48**

| | | | | | | | | |
|---|---|---|---|---|---|---|---|---|
| 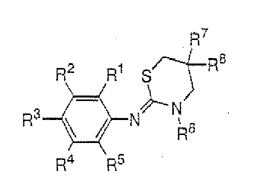 | | | | | | | | |

| No. | R¹ | R² | R³ | R⁴ | R⁵ | R⁶ | R⁷ | R⁸ |
|---|---|---|---|---|---|---|---|---|
| II-106 | Pr*ⁱ* | H | H | H | H | 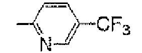 | Me | Me |
| II-107 | Pr*ⁱ* | H | H | H | H | 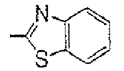 | Me | Me |
| II-108 | Pr*ⁱ* | H | H | H | H | 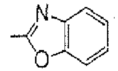 | Me | Me |
| II-I09 | Pr*ⁱ* | H | H | H | H | 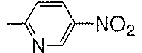 | Me | Me |
| II-110 | H | H | Pr | H | H | 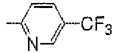 | Me | Me |
| II-111 | Pr*ⁱ* | H | H | H | H | 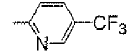 | Et | Et |
| II-112 | Pr*ⁱ* | H | H | H | H | 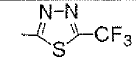 | Me | Me |
| II-113 | Pr*ⁱ* | H | H | H | H | CSSMe | -(CH₂)₂N(CH₂Ph)(CH₂)₂- | |

**Table 49**

| | | | | | | | | |
|---|---|---|---|---|---|---|---|---|
| 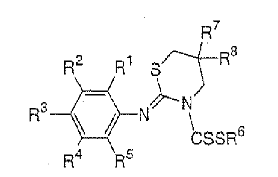 | | | | | | | | |

| No. | R¹ | R² | R³ | R⁴ | R⁵ | R⁶ | R⁷ | R⁸ |
|---|---|---|---|---|---|---|---|---|
| II-114 | H | SMe | H | H | H | Allyl | Et | Et |
| II-115 | H | SMe | H | H | H | Allyl | -(CH₂)₄- | |
| II-116 | H | SMe | H | H | H | Allyl | -(CH₂)₅- | |
| II-117 | H | H | SMe | H | H | Allyl | -(CH₂)₄- | |
| II-118 | H | H | SMe | H | H | Allyl | -(CH₂)₅- | |
| II-119 | OMe | H | Et | H | H | Allyl | Me | Me |
| II-120 | OMe | H | Pr*ⁱ* | H | H | Allyl | Me | Me |
| II-121 | Pr*ⁱ* | H | One | H | H | Allyl | Me | Me |
| II-122 | Pr*ⁱ* | H | OEt | H | H | Allyl | Me | Me |
| II-123 | H | OEt | OEt | H | H | Allyl | Me | Me |
| II-124 | H | OPr | OPr | H | H | Allyl | Me | Me |
| II-125 | H | OMs | OEt | H | H | Allyl | Me | Me |
| II-126 | H | H | (CH₂)₂OEt | H | H | Allyl | Me | Me |
| II-127 | H OMe | | OEt | H | H | Allyl | Et | Et |
| II-128 | H | OEt | OEt | H | H | Allyl | Et | Et |
| II-129 | H | OEt | OPr | H | H | Allyl | Et | Et |
| II-130 | H | OMs | OPr | H | H | Allyl | Et | Et |
| II-131 | H | OPr | OPr | H | H | Allyl | Et | Et |
| II-132 | H | OPr*ⁱ* | OPr | H | H | Allyl | Et | Et |
| II-133 | H | H | (CH₂)₂NMe₂ | H | H | Allyl | Me | Me |
| II-134 | Pr*ⁱ* | H | H | H | H | CH₂CO₂B u*^{t}* | -(CH₂)₅- | |
| II-135 | Pr*ⁱ* | H | H | H | H | Me | -(CH₂)₂N(Me)(CH₂)₂- | |
| II-136 | Pr*ⁱ* | H | H | H | H | Me | -(CH₂)₂N(Et)(CH₂)₂- | |
| II-137 | F | H | F | H | H | Allyl | Me | Me |
| II-138 | H | Cl | Cl | H | H | Allyl | Me | Me |
| II-139 | Me | H | Cl | H | H | Allyl | Me | Me |
| II-140 | Cl | H | Me | H | H | Allyl | Me | Me |
| II-141 | H | H | (CH₂)₂OMe | H | H | Allyl | Me | Me |
| II-142 | H | H | Pr*ⁱ* | H | H | Allyl | -(CH₂)₄- | |
| II-143 | H | H | Pr*ⁱ* | H | H | CH₂CO₂B u*^{t}* | -(CH₂)₄- | |

**Table 50**

| | | | | | | | | |
|---|---|---|---|---|---|---|---|---|
| 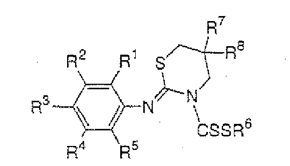 | | | | | | | | |

| No. | R¹ | R² | R³ | R⁴ | R⁵ | R⁶ | R⁷ | R⁸ |
|---|---|---|---|---|---|---|---|---|
| II-144 | H | H | Pr*ⁱ* | H | H | Allyl | Et | Et |
| II-145 | H | H | Pr*ⁱ* | H | H | CH₂CO₂Bu*^{t}* | Et | Et |
| II-146 | H | H | Pr*ⁱ* | H | H | Allyl | -(CH₂)₅- | |
| II-147 | OMe | H | H | H | H | CH₂CO₂Bu*^{t}* | Pr | Pr |
| II-148 | OMe | H | H | H | H | CH₂CO₂Bu*^{t}* | Pr*ⁱ* | Pr*ⁱ* |
| II-149 | OMe | H | H | H | H | Allyl | Pr | Pr |
| II-150 | Bu*^{s}* | H | H | H | H | Me | -(CH₂)₂N(Me)(CH₂)₂- | |

**Table 51**

| | | | | |
|---|---|---|---|---|
| 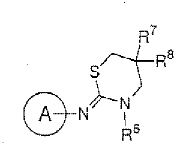 | | | | |

| No. | A | R⁶ | R⁷ | R⁸ |
|---|---|---|---|---|
| II-151 | 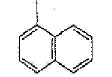 | CSSCH₂CO₂Bu*^{t}* | -(CH₂)₅- | |
| II-152 | 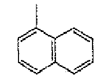 | CSSCH₂CO₂Bu*^{t}* | Et | Et |
| II-153 | 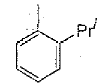 | COSMe | -(CH₂)₂N(Me)(CH₂)₂- | |
| II-154 | 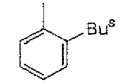 | COSMe | -(CH₂)₂N(Me)(CH₂)₂- | |

**Table 52**

| Compound | Physical Properties | |
|---|---|---|
| No. | m.p. | NMR (CDCl₃) |
| II-1 | | 1.20 (6H, d, J=6.9), 1.23 (6H, s), 2.66 (2H, s), 3.09 (1H, sept, J=6.9), 3.93-3.97 (2H, m), 4.49 (2H, s), 5.15-5.19 (1H, m), 5.28-5.39 (1H, m), 5.86-6.01 (1H, m), 6.89-6.94 (1H, m), 7.11-7.21 (2H, m), 7.29-7.34 (1H, m) |
| II-2 | 93.5-94.5°C | 1.21 (6H, d, J=6.9), 1.23 (6H, s), 2.20 (1H, t, J=2.6), 2.69 (2H, s), 3.09 (1H, sept, J=6.9), 3.99 (2H, d, J=2.6), 4.49 (2H, s), 6.90-6.94 (1H, m), 7.14-7.22 (2H, m), 7.32-7.35 (1H, m) |
| II-3 | | 1.21 (6H, d, J=6.9), 1.25 (6H, s), 2.74 (2H, s), 3.02 (1H, sept, J=6.9), 4.00 (2H, s), 4.50 (2H, s), 6.87-6.90 (1H, m), 7.15-7.22 (2H, m), 7.32-7.36 (1H, m) |
| II-4 | 73-74°C | 1.21 (6H, d, J=6.9), 1.24 (6H, s), 2.67 (2H, s), 3.10 (1H, sept, J=6.9), 3.44 (3H, s), 4.48 (2H, s), 5.45 (2H, s), 6.92-6.96 (1H, m), 7.16-7.20 (2H, m), 7.32-7.35 (1H, m) |
| II-5 | | 1.19 (6H, d, J=6.9), 1.22 (6H, s), 1.71 (3H, d, J=6.6), 2.64 (2H, s), 3.15 (1H, sept, J=6.9), 3.88 (2H, d, J=6.9), 4.49 (2H, s), 5.56-5.62 (1H, m), 5.69-5.78 (1H, m), 6.89-6.94 (1H, m), 7.11-7.21 (2H, m), 7.29-7.34 (1H, m) |
| II-6 | | 1.19 (6H, d, J=6.9), 1.23 (6H, s), 1.72 (3H, d, J=6.9), 2.65 (2H, s), 3.15 (1H, sept, J=6.9), 3.89 (2H, d, J=6.9), 4.49 (2H, s), 5.28-5.35 (1H, m), 6.87-6.92 (1H, m), 7.11-7.21 (2H, m), 7.29-7.34 (1H, m) |
| II-7 | | 1.19 (6H, d, J=6.9), 1.23 (6H, s), 2.47 (2H, q, J=7.4), 2.64 (2H, s), 3.15 (1H, sept, J=6.9), 3.34 (2H, t, J=7.4), 4.48 (2H, s), 5.01-5.14 (2H, m), 5.74-5.98 (1H, m), 6.82-6.89 (1H, m), 7.11-7.21 (2H, m), 7.29-7.34 (1H, m) |
| II-8 | 92-96°C | 1.20 (6H, d, J=6.9), 1.22 (6H, s), 2.35 (3H, s), 2.70 (2H, s), 3.08 (1H, sept, J=6.9), 4.12 (2H, s), 4.46 (2H, s), 6.92-6.97 (1H, m), 7.11-7.22 (2H, m), 7.30-7.35 (1H, m) |
| II-9 | | 1.20 (6H, d, J=6.9), 1.24 (6H, s), 2.74 (2H, s), 3.05 (1H, sept, J=6.9), 4.17 (2H, s), 4.39 (2H, s), 6.93-6.97 (1H, m), 7.18-7.24 (2H, m), 7.33-7.38 (1H, m) |
| II-10 | 82-83°C | 1.20 (6H, d, J=6.9), 1.22 (6H, s), 2.70 (2H, s), 3.09 (1H, sept, J=6.9), 3.75 (3H, s), 4.07 (2H, s), 4.48 (2H, s), 6.92-6.95(1H, m), 7.13-7.21 (2H, m), 7.31-7.35 (1H, m) |

**Table 53**

| Compound | Physical Properties | |
|---|---|---|
| No. | m.p. | NMR (CDCl₃) |
| II-11 | 95.5-96.5°C | 1.20 (6H, d, J=6.9), 1.22 (6H, s), 1.29 (3H, t, J=7.3), 2.70 (2H, s), 3.09 (1H, sept, J=6.9), 4.06 (2H, s), 4.21 (2H, q, J=7.3), 4.48 (2H, s), 6.92-6.96 (1H, m), 7.15-7.19 (2H, m), 7.31-7.34 (1H, m) |
| II-12 | 83-86°C | 0.96 (3H, t, J=7.3), 1.20 (6H, d, J=6.9), 1.22 (6H, s), 1.68 (2H, sext, J=7.3), 2.70 (2H, s), 3.09 (1H, sept, J=6.9), 4.07 (2H, s), 4.11 (2H, t, J=7.3), 4.48 (2H, s), 6.92-6.95 (1H, m), 7.13-7.20 (2H, m), 7.31-7.34 (1H, m) |
| II-13 | 95-96°C | 1.20 (6H, d, J=6.9), 1.22 (6H, s), 1.27 (6H, d, J=6.3), 2.70 (2H, s), 3.09 (1H, sept, J=6.9), 4.02 (2H, s), 4.47 (2H, s), 5.06 (1H, sept, J=6.3), 6.92-6.97 (1H, m), 7.13-7.21 (2H, m), 7.29-7.34 (1H, m) |
| II-14 | | 1.20 (6H, d, J=6.9), 1.22 (6H, s), 1.47 (9H, s), 2.69 (2H, s), 3.09 (1H, sept, J=6.9), 3.97 (2H, s), 4.47 (2H, s), 6.92-6-96 (1H, m), 7.11-7.20 (2H, m), 7.31-7.34 (1H, m) |
| II-15 | | 1.21 (6H, d, J=6.9), 1.22 (6H, s), 2.70 (2H, s), 3.08 (1H, sept, J=6.9), 4.13 (2H, s), 4.48 (2H, s), 4.62 (1H, dd, J=6.3, 1.7), 4.95 (1H, dd, J=13.9, 1.7), 6.92-6.95 (1H, m), 7.13-7.35 (4H, m) |
| II-16 | | 1.20 (6H, d, J=6.9), 1.22 (6H, s), 2.69 (2H, s), 3.08 (1H, sept, J=6.9), 4.10 (2H, s), 4.47 (2H, s), 4.63-4.66 (2H, m), 5.23-5.39 (2H, m), 5.86-5.98 (1H, m), 6.92-6.95 (1H, m), 7.15-7.21 (2H, m), 7.31-7.34 (1H, m) |
| II-17 | | 1.20 (6H, d, J=6.9), 1.22 (6H, s), 2.70 (2H, s), 3.08 (1H, sept, J=6.9), 3.40 (3H, s), 3.61-3.65 (2H, m), 4.11 (2H, d, J=2.3), 4.29-4.37 (2H, m), 4.47 (2H, s), 6.92-6.95 (1H, m), 7.13-7.20 (2H, m), 7.31-7.34 (1H, m) |
| II-18 | | 1.19-1.23 (12H, m), 1.58 (3H, d, J=7.3), 2.62 (1H, d, J=13.2), 2.74 (1H, d, J=13.2), 3.11 (1H, sept, J=6.9), 3.74 (3H, s), 4.18 (1H, d, J=13.5), 4.66 (1H, q, J=7.3), 4.72 (1H, d, J=13.5), 6.91-6.94 (1H, m), 7.13-7.21 (2H, m), 7.31-7.35 (1H, m) |
| II-19 | | 1.21 (6H, d, J=6.9), 1.21 (6H, s), 1.28 (3H, t, J=7.3), 1.71 (6H, s), 2.66 (2H, s), 3.14 (1H, sept, J=6.9), 4.18 (2H, q, J=7.3), 4.40 (2H, s), 6.88-6.92 (1H, m), 7.13-7.21 (2H, m), 7.31-7.35 (1H, m) |
| II-20 | 117-119°C | 1.21 (6H, d, J=6.9), 1.24 (6H, s), 2.69 (2H, s), 3.05 (1H, sept, J=6.9), 4.03 (2H, s), 4.48 (2H, s), 5.35 (1H, brs), 6.50 (1H, brs), 6.89-6.92 (1H, m), 7.14-7.22 (2H, m), 7.32-7.35 (1H, m) |

**Table 54**

| Compound | Physical Properties | |
|---|---|---|
| No. | m.p. | NMR (CDCl₃) |
| II-21 | | 1.20 (6H, d, J=6.9), 1.22 (6H, s), 2.69 (2H, s), 2.97 (3H, s), 3.10 (1H, sept, J=6.9), 3.15 (3H, s), 4.20 (2H, s), 4.47 (2H, s), 6.94-6.97 (1H, m), 7.12-7.20 (2H, m), 7.30-7.33 (1H, m) |
| II-22 | | 1.20 (6H, d, J=6.9), 1.22 (6H, s), 2.71 (2H, s), 3.10 (1H, sept, J=6.9), 3.23 (3H, s), 3.82 (3H, s), 4.33 (2H, s), 4.47 (2H, s), 6.95-7.00 (1H, m), 7.12-7.21 (2H, m), 7.30-7.34 (1H, m) |
| II-23 | | 1.20 (6H, d, J=6.9), 1.23 (6H, s), 2.68 (2H, s), 3.09 (1H, sept, J =6.9), 4.22 (2H, q, J=9.9), 4.50 (2H, s), 6.89-6.95 (1H, m), 7.14-7.23 (2H, m), 7.31-7.36 (1H, m) |
| II-24 | | 1.18 (6H, d, J=6.9), 1.23 (6H, s), 2.07 (3H, s), 2.67 (2H, s), 3.09 (1H, sept, J=6.9), 3.57 (2H, t, J=6.6), 4.35 (2H, t, J=6.6), 4.49 (2H, s), 6.88-6.92 (1H, m), 7.13-7.22 (2H, m), 7.30-7.35 (1H, m) |
| II-25 | | 1.20 (6H, d, J=6.9), 1.23 (6H, s), 2.65 (2H, s), 3.1.0 (1H, sept, J=6.9), 3.71 (2H, t, J=6.6), 4.29 (2H, t, J=6.6), 4.49 (2H, s), 6.89-6.97 (4H, m), 7.15-7.21 (2H, m), 7.25-7.34 (3H, m) |
| II-26 | | 1.21 (6H, d, J=6.9), 1.23 (6h, s), 2.66 (2H, s), 3.10 (1H, sept, J=6.9), 3.60 (2H, t, J=6.6), 3.99-4.05 (3H, m), 4.24(1H, dd, 14.2, 1.9), 4.49 (2H, s), 6.47 (1H, dd, 14.2, 6.9), 6.89-6.94 (1H, m), 7.15-7.21 (2H, m), 7.31-7.34 (1H, m) |
| II-27 | | 1.20 (6H, d, J=6.9), 1.23 (6H, s), 3.09 (1H, sept, J=6.9), 3.64 (2H, s, J=4.6), 3.84-4.03 (4H, m), 4.49 (2H, s), 5.21 (1H, t, J=4.6), 6.91-6.96 (1H, m), 7.12-7.21 (2H, m), 7.30-7.34 (1H, m) |
| II-28 | 124-126°C | 1.17 (6H, d, J=6.9), 1.23 (6H, s), 2.38 (3H, s), 2.67 (2H, s), 3.06 (1H, sept, J=6.9 ), 4.50 (2H, s), 4.55 (2H, s), 6.05 (1H, s), 6.86-6.90 (1H, m), 7.12-7.19 (2H, m), 7.30-7.33 (1H, m) |
| II-29 | | 0.94 (6H, d, J=6.6), 1.17 (6H, d, J=6.9), 1.23 (6H, s), 1.93-2.08 (1H, m), 2.58 (2H, d, J=6.6), 2.66 (2H, s), 3.07 (1H, sept, J=6.9), 4.50 (2H, s), 4.55 (2H, s), 6.05 (1H, s), 6.85-6.91 (1H, m), 7.12-7.19 (2H, m), 7.28-7.33 (1H, m) |
| II-30 | 129-130°C | 1.17 (6H, d, J=6.9), 1.23 (6H, s), 1.31 (9H, s), 2.67 (2H, s), 3.08 (1H, sept, J=6.9), 4.51 (2H, s), 4.59 (2H, s), 6.00 (1H, s), 6.87-6.91 (1H, m), 7.14-7.19 (2H, m), 7.30-7.33 (1H, m) |

**Table 55**

| Compound | Physical Properties | |
|---|---|---|
| No. | m.p. | NMR (CDCl₃) |
| 11-31 | | 1.18 (6H, d, J=6.9), 1.24 (6H, s), 2.68 (2H, s), 3.07 (1H, sept, J=6.9), 4.52 (2H, s), 4.64 (2H, s), 6.61 (1H, s), 6.88-6.91 (1H, m), 7.12-7.19 (2H, m), 7.29-7.33 (1H, m), 7.41.7.48 (3H, m), 7.71-7.76 (2H, m) |
| II-32 | | 1.18 (6H, d, J=6.9), 1.22 (6H, s), 2.26 (3H, s), 2.66 (2H, s), 3.06 (1H, sept, J=6.9), 4.48 (2H, s), 4.58 (2H, s), 6.09 (1H, s), 6.87-6.92 (1H, m), 7.13-7.20 (2H, m), 7.28-7.34 (1H, m) |
| II-33 | | 1.18 (6H, d, J=6.9), 1.21 (6H, s), 1.25 (6H, d, J=6.9), 2.66 (2H, s), 3.02 (1H, sept, J=6.9), 3.04 (1H, sept, J=6.9), 4.49 (2H, s), 4.59 (2H, s), 6.12 (1H, s), 6.88-6.92 (1H, m), 7.13-7.21 (2H, m), 7.29-7.34 (1H, m) |
| II-34 | | 0.94 (6H, d, J=6.6), 1.18 (6H, d, J=6.9), 1.21 (6H, s), 1.88-2.05 (1H, m), 2.49 (2H, d, J=6.6), 2.63 (2H, s), 3.07 (1H, sept, J=6.9), 4.49 (2H, s), 4.59 (2H, s), 6.09 (1H, s), 6.87-6.91 (1H, m), 7.13-7.20 (2H, m), 7.29-7.34 (1H, m) |
| II-35 | 124-125°C | 1.18 (6H, d, J=6.9), 1.21 (6H, s), 1.30 (9H, s), 2.65 (2H, s), 3.07 (1H, sept, J=6.9), 4.49 (2H, s), 4.59 (2H, s), 6.15 (1H, s), 6.88-6.93 (1H, m), 7.13-7.21 (2H, m), 7.29-7.34 (1H, m) |
| II-36 | | 1.17 (6H, d, J=6.9), 1.22 (6H, s), 1.26 (9H, s), 2.67 (2H, s), 3.07 (1H, sept, J=6.9), 4.49 (2H, s), 4.59 (2H, s), 6.61 (1H, s), 6.88-6.92 (1H, m), 7.11-7.18 (2H, m), 7.29-7.32 (1H, m) |
| II-37 | | 1.21 (6H, d, J=6.9), 1.23 (6H, s), 2.52-2.56 (4H, m), 2.65 (2H, s), 2.68-2.73 (2H, m), 3.11 (1H, sept, J=6.9), 3.41-3.52 (2H, m), 3.70-3.73 (4H, m), 4.48 (2H, s), 6.87-6.92 (1H, m), 7.15-7.19 (2H, m), 7.31-7.35 (1H, m) |
| II-38 | 123.5-124.5° C | 1.20 (6H, d, J=6.9), 1.23 (6H, s), 1.38 (6H, s), 2.67 (2H, s), 2.80 (2H, s), 3.08 (1H, sept, J=6.9), 4.32 (2H, s), 4.49 (2H, s), 6.87-6.91 (1H, m), 7.16-7.21 (2H, m), 7.31-7.35 (1H, m) |
| II-39 | | 0.88 (6H, t, J=7.4), 1.20 (6H, d, J=6.9), 1.47-1.62 (4H, m ), 2.61 (2H, s), 3.08 (1H, sept, J=6.9), 3.93-3.97 (2H, m), 4.43 (2H, s), 5.15-5.19 (1H, m), 5.28-5.39 (1H, m), 5.86-6.01 (1H, m), 6.89-6.94 (1H, m), 7.16-7.21 (2H, m), 7.30-7.36 (1H, m) |
| II-40 | | 0.87 (6H, t, J=7.4), 1.20 (6H, d, J=6.9), 1.28 (3H, t, J=7.3), 1.42-1.60 (4H, m ), 2.64 (2H, s), 3.11 (1H, sept, J=6.9), 4.06 (2H, s), 4.21 (2H, q, J=7.3), 4.43 (2H, s), 6.91-6.96 (1H, m), 7.15-7.19 (2H, m), 7.31-7.34 (1H, m) |

**Table 56**

| Compound | Physical Properties | |
|---|---|---|
| No. | m.p. | NMR (CDCl₃) |
| II-41 | | 0.87 (6H, t, J=7.4), 1-20 (6H, d, J=6.9), 1.27 (6H, d, J=7.0), 1.48-1.63 (4H, m), 2.65 (2H, s), 3.11 (1H, sept, J=6.9), 4.02 (2H, s), 4.43 (2H, s), 5.01 (1H, sept, J=7.0), 6.91-6.96 (1H, m), 7.15-7.19 (2H, m), 7.31-7.34 (1H, m) |
| II-42 | | 0.88 (6H, t, J=7.4), 1.20 (6H, d, J=6.9), 1.46 (9H, s), 1.42-1.60 (4H, m ), 2.64 (2H, s), 3.11 (1H, sept, J=6.9), 3.90 (2H, s), 4.42 (2H, s), 6.89-6.96 (1H, m), 7.18-7.23 (2H, m), 7.31-7.34 (1H, m) |
| II-43 | | 0.88 (6H, t, J=7.4), 1.20 (6H, d, J=6.9), 1.26 (3H, t, J=7.0), 1.42-1.60 (4H, m ), 2.60 (2H, s), 2.79 (2H, t, J=7.2), 3.08 (1H, sept, J=6.9), 3.54 (2H, t, J=7.2), 4.16 (2H, q, J=7.0), 4.43 (2H, s), 6.89-6.94 (1H, m), 7.15-7.19 (2H, m), 7.31-7.34 (1H, m) |
| II-44 | | 0.88 (6H, t, J=7.4), 1.19 (6H, d, J=6.9), 1.50-1.70 (4H, m ), 1.71 (3H, d, J=6.9), 2.61 (2H, s), 3.15 (1H, sept, J=6.9), 3.88 (2H, d, J=6.9), 4.43 (2H, s), 5.56-5.62 (1H, m), 5.69-5.78 (1H, m), 6.89-6.94 (1H, m), 7.11-7.21 (2H, m), 7.29-7.34 (1H, m) |
| II-45 | | 0.88 (6H, t, J=7.2), 1.19 (6H, d, J=6.9), 1.48-1.65 (4H, m ), 1.72 (6H, d, J=6.9), 2.61 (2H, s), 3.15 (1H, sept, J=6.9), 3.89 (2H, d, J=6.9), 4.44 (2H, s), 5.28-5.35 (1H, m), 6.87-6.92 (1H, m), 7.11-7.21 (2H, m), 7.29-7.34 (1H, m) |
| II-46 | | 0.88 (6H, t, J=7.1), 1.19 (6H, d, J=6.9), 1.48-1.65 (4H, m ), 2.47 (2H, q, J=7.4), 2.60 (2H, s), 3.12 (1H, sept, J=6.9), 3.34 (2H, t, J=7.4), 4.44 (2H, s), 5.01-5.14 (2H, m), 5.74-5.98 (1H, m), 6.82-6.89 (1H, m), 7.11-7.21 (2H, m), 7.29-7.34 (1H, m) |
| II-47 | | 0.85 (3H, t, J=7.4), 1.18 (3H, d, J=7.4), 1.23 (6H, s), 1.26 (3H, t, J=7.0), 1.42-1.60 (4H, m ), 2.68 (2H, s), 3.11 (1H, sext, J=7.0), 4.06 (2H, s), 4.15 (2H, q, J=7.0), 4.38 (1H, d, J=13.5), 4.57 (1H, d, J=13.5), 6.83-6.90 (1H, m), 7.11-7.19 (2H, m), 7.28-7.31 (1H, m) |
| II-48 | | 0.85 (3H, t, J=7.4), 1.18 (3H, d, J=7.4), 1.23 (6H, s), 1.47 (9H, s), 1.42-1.60 (4H, m ), 2.68 (2H, s), 3.00 (1H, sext, J=7.0), 4.01 (2H, s), 4.38 (1H, d, J=13.5), 4.57 (1H, d, J=13.5), 6.89-6.95 (1H, m), 7.11-7.19 (2H, m), 7.28-7.31 (1H, m) |
| II-49 | | 0.82-0.91 (9H, m), 1.17 (3H, d, J=6.9), 2.61 (2H, s), 2.87 (1H, sext, J=6.9), 3.65 (2H, d, J=6.9), 4.30 (1H, d, J=13.5), 4.57 (1H, d, J=13.5), 5.15-5.35 (2H, m), 5.86-5.99 (1H, m), 6.88-6.92 (1H, m), 7.11-7.28 (3H, m) |
| 11-50 | | 0.83-0.92 (9H, m), 1.18 (3H, d, J=6.9), 1.47-1.69 (6H, m), 2.06 (3H, s), 2.62 (2H, s), 2.87 (1H, sext, J=6.9), 3.58 (2H, t, J=6.6), 4.31 (1H, d, J=13.9), 4.35 (2H, t, J=6.6), 4.55 (1H, d, J=13.9), 6.88-6.91 (1H, m), 7.11-7.20 (2H, m), 7.25-7.29 (1H, m) |

**Table 57**

| Cormpound | Physical Properties | |
|---|---|---|
| No. | m.p. | NMR (CDCl₃) |
| II-51 | | 0.83-0.92 (9H, m), 1.18 (3H, d, J=6.9), 2.53-2.56 (4H, m), 2.60 (2H, s), 2.71 (2H, t, J=7.3), 2.90 (1H, sept, J=6.9), 3.45 (2H, t, J=7.3), 3.69-3.73 (6H, m), 4.32 (1H, d, J=13.9), 4.55 (1H, d, J=13.9), 6.89-6.91 (1H, m), 7.14-7.20 (2H, m), 7.25-7.29 (1H, m) |
| II-52 | | 1.22 (6H, s), 1.24 (3H, t, J=7.3), 1.33 (3H, t, J=7.2), 2.64 (2H, q, J=7.3), 2.66 (2H, s), 4.06 (2H, s), 4.20 (2H, q, J=7.2), 4.48 (2H, s), 6.9 (2H, d, J=8.3), 7.20 (2H, d, J=8.3) |
| II-53 | | 1.22 (6H, s), 1.26 (6H, d, J=6.9), 1.29 (3H, t, J=7.2), 2.70 (2H, s), 2.94 (1H, sept, J=6.9), 4.06 (2H, s), 4.12 (2H, q, J=7.2), 4.49 (2H, s), 6.85-6.90 (2H, m), 7.04-7.10 (1H, m), 7.31-7.34 (1H, m) |
| II-54 | | 1.23 (6H, s), 1.29 (3H, t, J=7.3), 2.68 (2H, s), 2.72 (6H, s), 4.07 (2H, s), 4.22 (2H, q, J=7.3), 4.49 (2H, s), 6.98-7.10 (4H, m) |
| II-55 | | 1.27 (6H, s), 1.33 (3H, t, J=7.3), 2.73 (2H, s), 3.01 (6H, s), 4.10 (2H, s), 4.25 (2H, q, J=7.3), 4.54 (2H, s), 6.41 (1H, d, J=2.3), 6.48 (1H, d, J=7.6), 6.60 (1H, dd, J=7.6, 2.3), 7.20 (1H, d, J=7.6) |
| II-56 | | 1.16 (6H, t, J=7.3), 1.21 (6H, s), 1.28 (3H, t, J=7.3), 2.68 (2H, s), 3.35 (4H, q, J=7.3), 4.05 (2H, s), 4.19 (2H, q, J=7.3), 4.48 (2H, s), 6.29 (1H, d, J=2.3), 6.32 (1H, d, J=8.6), 6.50 (1H, dd, J=8.6, 2.3), 7.20 (1H, d, J=8.6) |
| II-57 | | 1.21 (6H, s), 1.22 (3H, t, J=7.6), 1.46 (9H, s), 2.65 (2H, q, J=7.6), 2.69 (2H, s), 3.96 (2H, s), 4.48 (2H, s), 6.97 (2H, d, J=8.3), 7.20 (2H, d, J=8.3) |
| II-58 | | 1.21 (6H, s), 1.25 (6H, d, J=6.9), 1.56 (9H, s), 2.69 (2H, s), 2.90 (1H, sept, J=6.9), 3.9 (2H, s), 4.48 (2H, s), 6.85-6.90 (2H, m), 7.04-7.10 (1H, m), 7.31.-7.34 (1H, m) |
| II-59 | | 1.21 (6H, s), 1.56 (9H, s), 2.67 (2H, s), 2.69 (6H, s), 3.96 (2H, s), 4.47 (2H, s), 6.98.7.10 (4H, m) |
| II-60 | | 1.21 (6H, s), 1.47 (9H, s), 2.68 (2H, s), 2.96 (6H, s), 3.96 (2H, s), 4.48 (2H, s), 6.36 (1H, d, J=7.6), 6.37 (1H, d, J=2.3), 6.55 (1H, dd, J=7.6, 2.3), 7.20 (1H, d, J=7.6) |

**Table 58**

| Com-pound | Physical Properties | |
|---|---|---|
| No. | m.p. | NMR (CDCl₃) |
| II-61 | | 1.16 (6H, t, J=7.3), 1.21 (6H, s), 1.57 (9H, s), 2.68 (2H, s), 3.35 (4H, q, J=7.3), 3.93 (2H, s), 4.48 (2H, s), 6.29 (1H, d, J=2.3), 6.32 (1H, d, J=8.6), 6.50 (1H, dd, J=8.6, 2.3), 7.20 (1H, d, J=8.6) |
| II-62 | | 1.15 (6H, t, J=7.2), 1.22 (6H, s), 2.65 (2H, s), 3.31 (4H, q, J=7.3), 3.93.3.97 (2H, m), 4.49 (2H, s), 5.15-5.19 (1H, m), 5.28-5.39 (1H, m), 5.86-6.01 (1H, m), 6.28 (1H, d, J=2.2), 6.32 (1H, d, J=8.6), 6.50 (1H, dd, J=8.6, 2.2), 7.20 (1H, d, J=8.6) |
| II-63 | | 0.97 (6H, t, J=7.2), 1.22 (6H, s), 2.15 (3H, s), 2.64 (2H, s), 2.97 (4H, q, J=7.3), 3.93-3.97 (2H, m), 4.49 (2H, s), 5.15-5.19 (1H, m), 5.28-5.39 (1H, m), 5.86-6.01 (1H, m), 6.64 (1H, d, J=7.9), 6.90 (1H, d, J=7.9), 7.15 (1H, d, J=7.9) |
| II-64 | | 1.22 (6H, s), 2.16 (3H, s), 2.64 (2H, s), 2.68 (6H, s), 3.93-3.97 (2H, m), 4.49 (2H, s), 5.15-5.19 (1H, m), 5.28-5.39 (1H, m), 5.86-6.01 (1H, m), 6.63 (1H, d, J=7.9), 6.85 (1H, d, J=7.9), 7.12 (1H, d, J=7.9) |
| 11-65 | | 0.88 (6H, t, J=7.3), 1.43-1.65 (4H, m), 2.60 (2H, s), 2.70 (6H, s), 3.94 (2H, d, J=6.9), 4.43 (2H, s), 5.16 (2H, d, J=10.2), 5.31 (1H, dd, J=16.8, 1.3), 5.86-6.01 (1H, m), 6.93-7.03 (3H, m), 7.08-7.14 (1H, m) |
| II-66 | | 0.87 (6H, t, J=7.3), 1.47 (9H, s), 1.48-1.63 (4H, m), 2.62 (2H, s), 2.70 (6H, s), 3.96 (2H, s), 4.43 (2H, s), 6.92-7.14 (4H, m) |
| II-67 | | 0.88 (6H, t, J=7.6), 1.47-1.65 (4H, m), 2.60 (2H, s), 3.82 (3H, s), 3.92-3.95 (2H, m), 4.48 (2H, s), 5.14-5.19 (1H, m), 5.32 (1H dd, J=16.8, 1.3), 5.87-6.00 (1H, m), 6.93-7.00 (3H, m), 7.10.7.17 (1H, m) |
| II-68 | | 0.87 (6H, t, J=7.6), 1.47 (9H, s), 1.51-1.60 (4H, m), 2.63 (2H, s), 3.83 (3H, s), 3.96 (2H, s), 4.47 (2H, s), 6.93-7.03 (3H, m), 7.10-7.14 (1H, m) |
| II-59 | | 0.86 (6H, t, J=7.6), 1.24 (3H, t, J=7.6), 1.41-1.65 (4H, m), 2.61-2.71 (4H, m), 3.94 (2H, d, J=7.3), 4.45 (2H, s), 5.16 (1H, d, J=9.9), 5.28-5.34 (1H, m), 5.86-6.01 (1H, m), 6.94-6.98 (1H, m), 7.18-7.21 (2H, m) |
| II-70 | | 0.88 (6H, t, J=7.6), 1.47 (9H, s), 1.49-1.58 (4H, m), 2.61-2.70 (4H, m), 3.97 (2H, s), 4.45 (2H, s), 6.96-6.99 (2H, m), 7.18-7.21 (2H, m) |

**Table 59**

| Compound | Physical Properties | |
|---|---|---|
| No. | m.p. | NMR (CDCl₃) |
| II-71 | | 0.89 (6H, t, J=7.6 ), 1.47-1.65 (4H, m), 2.64 (2H, s), 3.94 (2H, d, J=7.3), 4.45 (2H, s), 5.18 (1H, d, J=9.9), 5.32 (1H, dd, J=17.2, 1.3), 5.86-6.01 (1H, m), 7.01-7.06 (2H, m), 7.20-7.23 (2H, m) |
| II-72 | | 0.88 (6H, t, J=7.3), 1.47 (9H, s), 1.48-1.66 (4H, m), 2.67 (2H, s), 3.97 (2H, s), 4.44 (2H, s), 7.03-7.08 (2H, m), 7.20-7.26 (2H, m) |
| II-73 | 103.5-104.5° C | 0.88 (6H, t, J=7.3), 1.50-1.63 (4H, m), 2.62 (2H, s), 2.72 (6H, s), 3.43 (3H, s), 4.43 (2H, s), 5.45 (2H, s), 6.95-7.18 (4H, m) |
| II-74 | | 1.20 (6H, d, J=6.9), 1.60-1.87 (8H, m), 2.74 (2H, s), 3.10 (1H, sept, J=6.9), 3.93-3.96 (2H, m), 5.15 (1H, dd, J=9.9, 1.3), 5.31 (1H, dd, J=17.1, 1.3), 5.86-6.01 (1H, m), 6.90-9.94 (1H, m), 7.12-7.20 (2H, m), 7.31-7.34 (1H, m) |
| II-75 | | 1.62-1.86 (8H, m), 2.72 (6H, s), 3.92-3.95 (2H, m), 4.55 (2H, s), 5.15 (1H, d, J=10.0), 5.26-5.33 (1H, m), 5.86-5.98 (1H, m), 6.93-7.01 (3H, m), 7.09-7.16 (1H, m) |
| II-76 | | 1.47 (9H, s), 1.64-1.76 (8H, m), 2.71 (6H, s), 2.76 (2H, s), 3.95 (2H, s), 4.54 (2H, s), 6.92-7.05 (3H, m), 7.09-7.15 (1H, m) |
| II-77 | 85.5-87.5°C | 1.20 (6H, d, J=6.9), 1.60-1.84 (8H, m), 2.79 (2H, s), 3.09 (1H, sept, J=6.9), 3.40 (3H, s), 3.61-3.64 (2H, m), 4.09 (2H, s), 4.29-4.32 (2H, m), 4.52 (2H, s), 6.92-6.95 (1H, m), 7.13-7.20 (2H, m), 7.31-7.34 (1H, m) |
| II-78 | | 1.19 (6H, d, J=6.9), 1.60-1.87 (8H, m), 2.23 (3H, s), 2.76 (2H, s), 3.06 (1H, sept, J=6.9), 4.53 (2H, s), 4.57 (2H, s), 6.09 (1H, s), 6.87-6.92 (1H, m), 7.13-7.20 (2H, m), 7.29-7.34 (1H, m) |
| II-79 | | 1.64-1.84 (8H, m), 2.75 (2H, s), 3.83 (3H, s), 3.93 (2H, d, J=6.9), 4.56 (2H, s), 5.16 (1H, d, J=9.9), 5.31 (1H, dd, J=17.1, 1.7), 5.87-5.99 (1H, m), 6:92-7.01 (3H, m), 7.11-7.18 (1H, m) |
| 11-80 | | 1.47 (9H, s), 1.64-1.83 (8H, m), 2.78 (2H, s), 3.84 (3H, s), 3.96 (2H, s), 4.55 (2H, s), 6.92-7.04 (3R, m), 7.11-7.18 (1H, m) |

**Table 60**

| Compound | Physical Properties | |
|---|---|---|
| No. | m.p. | NMR (CDCl₃) |
| II-81 | | 1.57-1.86 (8H, m), 2.73 (6H, s), 2.74 (2H, s), 3.42 (3H, s), 4.55 (2H, s), 5.44 (2H, s), 6.94-7.04 (3H, m), 7.11-7.17 (1H, m) |
| II-82 | | 1.24 (3H, t, J=7.6), 1.65-1.87 (8H, m), 2.65 (2H, m), 3.93-3.95 (2H, m), 4.54 (2H, m), 5.16 (1H, d, J=9.9 ), 5.27.5.35 (1H, m), 5.86-6.01 (1H, m), 6.93-6.98 (2H, m), 7.19-7.22 (1H, m) |
| II-83 | | 1.55-1.84 (8H, m), 2.77 (2H, s), 3.92-3.95 (2H, m), 4.55 (2H, s), 5.18 (1H, d, J=9.9), 5.28-5.35 (1H, m), 5.86-6.01 (1H, m), 7.01-7.06 (2H, m), 7.22 (2H, d, J=8.9) |
| II-84 | | 1.37-1.60 (8H, m), 1.73-1.86 (2H, m), 2.65 (2H, s), 2.70 (6H, s), 3.94 (2H, d, J=7.3), 4.52 (2H, s), 5.15 (1H, d, J=9.9), 5.30 (1H, dd, J=17.2, 1.3), 5.86-6.01 (1H, m), 6.93-7.15 (4H, m) |
| II-85 | | 1.36-1.62 (8H, m), 1.47 (9H, s), 1.69-1.82 (2H, m), 2.67 (2H, s), 2.70 (6H, s), 3.79 (2H, s), 4.52 (2H ,s), 6.93-7.14 (4H, m) |
| II-86 | 108.5-109.5° C | 1.33-1.62 (8H, m), 1.75-1.82 (2H, m), 2.65 (2H, s), 3.82 (3H, s), 3.94 (2H, d, J=6.9), 4.56 (2H, s), 5.15 (1H, d, J=10.2), 5.31 (1H, dd, J=17.2, 1.6), 5.88-6.02 (1H, m), 6.93-7.02 (3H, m), 7.10-7.17 (1H, m) |
| II-87 | | 1.23-1.78 (10H, m), 1.46 (9H, s), 2.67 (2H, s), 3.83 (3H, s), 3.97 (2H, s), 4.55 (2H, s), 6.89-7.05 (3H, m), 7.10-7.17 (12H, m) |
| 11-88 | 98-100°C | 1.24 (3H, t, J=7.6 ), 1.36-1.54 (8H, m), 1.76-1.81 (2H, m), 2.61-2.69 (4H, m), 3.94 (2H, d, J=6.9), 4.53 (2H, s), 5.16 (1H, d, J=9.9), 5.27-5.34 (1H, m), 5.86-5.98 (1H, m), 6.95-6.98 (2H, m), 7.18-7.21 (2H, m) |
| II-89 | | 1.20 (6H, d, J=6.9), 1.37-1.90 (16H, m), 2.66 (2H, s), 3.10 (1H, sept, J=6.9), 3.4 7-3.59 (3H, m), 3.69-4.06 (3H, m), 4.45 (1H, d, J=13.9), 4.59 (1H, d, J=13.9), 4.65-4.68 (1H, m), 6.90-6.93 (1H, m), 7.12-7.19 (2H, m), 7.29-7.34 (1H, m) |
| II-90 | | 1.20 (6H, d, J=6.9), 1.30-1.60 (8H, m), 1.72-1.83 (2H, m), 2.04 (2H, brs), 2.67 (2H, s), 3.09 (1H, sept, J=6.9), 3.56 (2H, t, J=5.9), 3.93 (2H, brs), 4.51 (2H, s), 6.91-6.94 (1H, m), 7.13-7.21 (2H, m), 7.29-7.34 (1H,m) |

**Table 61**

| Compound | Physical Properties | |
|---|---|---|
| No. | m.p. | NMR (CDCl₃) |
| II-91 | | 1.30-1.63 (8H, m), 1.75-1.82 (2H, m), 2.68 (2H, s), 3.93-3.96 (2H, m), 4.54 (2H, s), 5.17 (1H, dd, J=9.9, 1.3), 5.28-5.35 (1H, m), 5.86-6.01 (1H, m), 7.01-7.07 (2H, m), 7.20-7.23 (2H, m) |
| II-92 | 73.5-75.0°C | 1.20 (6H, d, J=6.9), 1.58-1.67 (2H, m), 1.89-1.95 (2H, m), 2.73 (2H, s), 3.09 (1H, sept, J=6.6), 3.94 (2H, d, J=7.3), 4.66 (2H, s), 5.18 (1H, d, J=9.9), 5.29-5.36 (1H, m), 5.87-5.98 (1H, m), 7.15-7.19 (2H, m), 7.31-7.35 (1H, m) |
| II-93 | 127-128°C | 1.21 (6H, d, J=6.6), 1.55-1.67 (2H, m), 1.89-1.97 (2H, m), 2.65 (3H, s), 2.74 (2H, s), 3.09 (1H, sept, J=6.6), 3.69-3.76 (4H, m), 4.69 (2H, s), 6.89-6.92 (1H, m), 7.13-7.21 (2H, m), 7.30-7.35 (1H, m) |
| 11-94 | | 0.90 (6H, t, J=7.3), 1.20 (6H, d, J=7.3), 1.48-1.62 (4H, m), 2.69 (2H, s), 3.05 (1H, sept, J=7.3), 4.16 (2H, s), 4.38 (2H, s), 4.97 (1H, brs), 6.92-6.96 (1H, m), 7.13-7.21 (2H, m), 7.32-7.36 (1H, m) |
| II-95 | 98-99°C | 1.23 (6H, s), 2.65 (2H, s), 4.00 (2H, d, J=6.9), 4.58 (2H, s), 5.19 (1H, d, J=6.9), 5.35 (1H, dd, J=17.2, 1.3), 5.90-6.03 (1H, m), 7.09 (1H, d, J=7.3), 7.42-7.53 (3H, m), 7.67 (1H, d, J=8.2), 7.85 (1H, dd, J=7.3, 3.0), 8.05 (1H, d, J=6.9) |
| II-96 | 120-121°C | 1.23 (6H, s), 1.49 (9H, s), 2.69 (2H, s), 4.01 (2H, s), 4.57 (2H, s), 7.11 (1H, d, J=8.2), 7.42-7.51 (3H, m), 7.67 (1H, d, J=8.2), 7.84-7.87 (1H, m), 8.06 (1H, d, J=7.6) |
| II-97 | | 1.23 (6H, s), 2.69 (2H, s), 3.40 (3H, s), 3.61-3.65 (2H, m), 4.15 (2H, s), 4.30-4.33 (2H, m), 4.56 (2H, s), 7.11 (1H, dd, J=7.3, 1.0), 7.42-7.54 (3H, m), 7.67 (1H, d, J=8.2), 7.84-7.88 (1H, m), 8.04 (1H, dd, J=6.9, 3.3) |
| II-98 | 99-100°C | 0.92 (6H, t, J=7.3), 1.22-1.60 (4H, m), 2.62 (2H, s), 4.00 (2H, s), 4.54 (2H, s), 5.19 (1H, d, J=9.9), 5.35 (1H, dd, J=17.2, 1.7), 5.93-6.03 (1H, m), 7.09 (1H, d, J=7.3), 7.42-7.52 (3H, m), 7.66 (1H, d, J=8.2), 7.83-7.86 (1H, m), 8.06 (1H, d, J=7.9) |
| II-99 | 111-113°C | 0.90 (6H, t, J=6.9), 1.16-1.56 (4H, m), 1.49 (9H, s), 2.65 (2H, s), 4.02 (2H, s), 4.54 (2H, s), 7.10-7.12 (1H, m), 7.42-7.53 (3H, m), 7.66 (1H, d, J=8.2), 7.83-7.86 (1H, m), 8.05-8.08 (1H, m) |
| II-100 | 86-87°C | 0.90 (6H, t, J=7.3), 1.43-1.66 (4H, m), 2.63 (2H, s), 4.00 (2H, d, J=6.9), 4.54 (2H, s), 5.20 (2H, d, J=9.9), 5.35 (1H, dd, J=16.8, 1.3), 5.90-6.05 (1H, m), 7.15-7.18 (1H, m), 7.38 (1H, dd, J=8.6, 4.3). 7.69 (1H, dd, J=8.6, 7.3), 7.92 (1H, d, J=8.6), 8.45 (1H, d, J=7.3), 8.93 (1H, dd, J-4.3, 1.7) |

**Table 62**

| Compound | Physical Properties | |
|---|---|---|
| No. | m.p. | NMR (CDCl₃) |
| 11-101 | 103-104°C | 1.59-1.84 (8H, m), 2.74 (2H, s), 3.97 (2H, d, J=6.9), 4.61 (2H, s), 5.17 (1H, d, J=10.2), 5.32 (1H, dd, J=16.8, 1.3), 5.88-6.01 (1H, m), 7.08 (1H, d, J=8.2), 7.41-7.52 (3H, m), 7.60 (1H, d, J=8.2), 7.84 (1H, dd, J=7.3, 2.6), 8.02 (1H, d, J=6.6) |
| II-102 | | 1.49 (9H, s), 1.54-1.90 (8H, m), 2.79 (2H, s), 4.00 (2H, s), 4.61 (2H, s), 7.11 (1H, dd, J=7.6, 1.3), 7.42-7.53 (3H, m), 7.67 (1H, d, J=8.2), 7.84-7.89 (1H, m), 8.02-8.06 (1H, m) |
| II-103 | | 1.58-1.85 (8H, m), 2.77 (2H, s), 3.99 (2H, d, J=7.3), 4.62 (2H, s), 5.19 (1H, d, J=8.9), 5.31-5.38 (1H, m), 5.91-6.04 (1H, m), 7.17 (1H, d, J=7.6), 7.39 (1H, dd, J=8.6, 4.3), 7.66-7.73 (1H, m), 7.93 (1H, d, J=8.6), 8.42 (1H, d, J=8.6), 8.93 (1H, dd, J=4.3, 2.0) |
| II-104 | 109-110°C | 1.33-1.84 (10H, m), 2.66 (2H, s), 4.00 (2H, d, J=6,9), 4.63 (2H,s), 5.19 (1H, d, J=9.9), 5.35 (1H, dd, J=16.8, 1.3), 5.91-6.06 (1H, m), 7.10 (1H, d, J=7.3), 7.42-7.52 (3H, m), 7.66 (1H, J=8.2), 7.83-7.86 (1H, m), 8.06 (1H, d, J=7.3) |
| II-105 | | 1.30-1.63 (8H, m), 1.72-1.84 (2H, m), 2.68 (2H, s), 4.00 (2H, d, J=6.9), 4.62 (2H, s), 5.20 (1H, d, J=9.9), 5.35 (1H, dd, J=16.8, 1.3), 5.92-6.04 (1H, m), 7.17 (1H, d, J=6.9), 7.38 (1H, dd, J=8.6, 4.3), 7.66-7-72 (1H, m), 7.9 (1H, d, J=8.6), 8.45 (1H, d, J=8.6), 8.93 (1H, dd, J=4.3, 1.7) |
| II-106 | | 1.15 (6H, s), 1.22 (6H, d, J=6.9), 2.67 (2H, s), 3.02 (1H, sept, J=6.9), 4.08 (2H, s), 6.77-6.80 (1H, m), 7.07-7.18 (2H, m), 7.28-7.31 (1H, m), 7.77 (1H, dd, J=8.6, 2.6), 8.11 (1H, d, J=8.9), 8.57-8.58 (1H, m) |
| II-107 | 121.5-122.5° C | 1.23 (6H, d, J=6.9), 1.27 (6H, s), 2.80 (2H, s), 3.17 (1H, sept, J=6.9), 4.36 (2H, s), 6.80-6.84 (1H, m), 7.13-7.23 (3H, m), 7.32-7.42 (2H, m), 7.70-7.79 (2H, m) |
| II-108 | 158.5-159.5° C | 1.20 (6H, s), 1.27 (6H, d, J=6.9), 2.72 (2H, s), 3.29 (1H, sept, J=6.9), 3.99 (2H, s), 6.80-6.84 (1H, m), 7.09-7.39 (6H, m), 7.53-7.56 (1H, m) |
| II-109 | | 1.16 (6H, s), 1.23 (6H, d, J=6.9), 2.67 (2H, s), 3.00 (1H, sept, J=6.9), 4.19 (2H, s), 6.79-6.83 (1H, m), 7.11-7.21 (2H, m), 7.30-7.34 (1H, m), 8.18 (1H, d, J=9.2), 8.32 (1H, dd, J=9.2, 2.6), 9.17 (1H, d, J=2.6) |
| II-110 | | 0.94 (2H, t, J=7.3), 1.14 (6H, s), 1.57-1.71 (2H, m), 2.57 (2H, t, J=7.3), 2.67 (2H, s), 4.09 (2H, s), 6.81-6.87 (2H, m), 7.08-7.16 (2H, m), 7.75 (1H, dd, J=8.9, 2.6), 8.09 (1H, d, J=8.9), 8.55 (1H, s) |

**Table 63**

| Compound | Physical Properties | |
|---|---|---|
| No. | m.p. | NMR (CDCl₃) |
| II-111 | | 0.88 (6H, t, J=7.4), 1.22 (6H, d, J=6.9), 1.42-1.52 (4H, m), 2.61 (2H, s), 3.06 (1H, sept, J=6.9), 4.11 (2H, s), 6.75-5.80 (1H, m), 7.07-7.18 (2H, m), 7.29-7.34 (1H, m), 7.75 (1H, dd, J=8.6, 2.6), 8.08 (1H, d, J=8.9), 8.57-8.58 (1H, m) |
| II-112 | | 1.20 (6H, d, J=6.9), 1.28 (6H< s), 2.85 (2H, s), 2.95 (1H, sept, J=6.9), 4.34 (2H, s), 6.72-6.79 (1H, m), 7.14-7.20 (2H, m), 7.31-7.36 (1H, m) |
| II-113 | 120-121°C | 1.19 (6H, d, J=6.9), 1.58-1.66 (2H, m), 1.88-1.98 (2H, m), 2.38-2.60 (4H, m), 2.64 (3H, s), 2.69 (2H, s), 3.08 (1H, sept, J=6.9), 3.52 (2H, s), 4.59 (2H, s), 6.89-6.92 (1H, m), 7.12-7.34 (8H, m) |
| II-114 | | 0.89 (6H, t, J=7.3), 1.43-1.65 (4H, m), 2.49 (3H, s), 2.62 (2H, s), 3.93-3.96 (2H, m), 4.45 (2H, s), 5.17 (1H, m), 5.31 (1H, m), 5.89 (1H, m), 6.80 (1H, m), 6.91 (1H, m), 7.04 (1H, m), 7.24-7.30 (2H, m) |
| II-115 | | 1.57-1.88 (8H, m), 2.49 (3H, s), 2.75 (2H, s), 3.95 (2H, m), 4.55 (2H, s), 5.17 (1H, m), 5.32 (1H, m), 5.93 (1H, m), 6.80 (1H, m), 6.91 (1H, m), 7.05 (1H, m), 7.29 (1H, m) |
| II-116 | | 1.32-1.60 (8H, m), 1.72-1.84 (2H, m), 2.49 (3H, s), 2.66 (2H, s), 3.95 (2H, m), 4.54 (2H, s), 5.17 (1H, d, J=10.2), 5.32 (1H, dd, J=17.2, 1.3), 5.89 (1H, m), 6.80 (1H, m), 6.91 (1H, m), 7.04 (1H, m), 7.28 (1H, m) |
| II-117 | | 1.65-1.86 (8H, m), 2.49 (3H, s), 2.75 (2H, s), 3.93 (2H, m), 4.54 (2H, s), 5.17 (1H, m), 5.31 (1H, m), 5.89 (1H, m), 6.96-7.01 (2H, m), 7.26-7.31 (2H, m) |
| II-118 | 111-112°C | 1.37-1.63 (8H, m), 1.73-1.84 (2H, m), 2.49 (3H, s), 2.67 (2H, s), 3.94 (2H, m), 4.53 (2H, s), 5.17 (1H, d, J=10.2), 5.31 (1H, dd, J=17.2, 1.7), 5.92 (1H, m), 6.97-7.01 (2H, m), 7.26-7.30 (2H, m) |
| II-119 | | 1.22 (6H, s), 1.25 (3H, t, J=6.9), 2.62 (2H, s), 2.65 (2H, q, J=6.9), 3.81(3H, s ), 3.95 (2H, m), 4.50 (2H, s), 5.17 (1H, m), 529 (1H, m), 5.94 (1H, m), 6.80-6.84 (2H, m), 6.33 (1H, m). |
| II-120 | | 1.22 (6H, s), 1.24 (6H, d, J=6.9), 2.64 (2H, s), 2.89 (1H, sept, J=6.9), 3.82 (3H, s), 3.95 (2H, m), 4.49 (2H, s), 5.17 (1H, m), 5.28 (1H, m), 5.94 (1H, m), 6.89-6.94 (2H, m), 6.93 (1H, m). |
| II-121 | | 1.18 (6H, d, J=6.9), 1.22 (6H, s), 2.64 (2H, s), 3.10 (1H, sept, J=6.9), 3.81 (3H, s), 3.95 (2H, m), 4.47 (2H, s), 5.17 (1H, m), 5.28 (1H, m), 5.97 (1H, m), 6.72 (1H, m), 6.85-6.95 (2H, m). |
| II-122 | | 1.17 (6H, d, J=6.9), 1.22 (6H, s), 1.43 (3H, t, J=7.5), 2.65 (2H, s), 3.05 (1H, sept, J=6.9), 3.95 (2H, m), 4.05 (2H, q, J=7.5), 4.46 (2H, s), 5.17 (1H, m), 528 (1H, m), 5.97 (1H, m), 6.72 (1H, m), 6.85-6.90 (2H,m). |

**Table 64**

| Compound | Physical Properties | |
|---|---|---|
| No. | m.p. | NMR (CDCl₃) |
| II-123 | | 1.22 (6H, s), 1.45 (6H, t, J=7.4), 2.64 (2H, s), 3.95 (2H, m), 4.10 (4H, q, J=7.4), 4.48 (2H, s), 5.17 (1H, m), 5.28 (1H, m), 5.97 (1H, m), 6.55-6.63 (2H, m), 6.88 (1H, m). |
| II-124 | | 1.05 (6H, t, J=7.4), 1.22 (6H, s), 1.78-1.86 (4H, m), 2.66 (2H, s), 3.93 (4H, q, J=7.4), 3.95 (2H, m), 4.48 (2H, s), 5.17 (1H, m), 5.28 (1H, m), 5.97 (1H, m), 6.55-6.68 (2H, m), 6.88 (1H, m). |
| II-125 | 86-88°C | 1.23 (6H, s), 1.45 (3H, t, J=7.4), 2.67 (2H, s), 3.22(3H, s), 3.95 (2H, m), 4.12 (2H, q, J=7.4), 4.47 (2H, s), 5.17 (1H, m), 5.28 (1H, m), 5.97 (1H, m), 6.95-6.99 (2H, m), 7.12 (1H, m). |
| II-126 | 65-66°C | 1.22 (6H, s), 1.25 (3H, t, J=6.9), 2.65 (2H, s), 3.54 (2H, q, J=6.9), 3.95 (2H, m), 4.49 (2H, s), 5.17 (1H, m), 5.28 (1H, m), 5.97 (1H, m), 6.99 (2H, d, J=7.9), 7.34 (2H, d, J=7.9). |
| II-127 | | 0.88 (6H, t, J=7.4), 1.45 (3H, t, J=7.4), 1.44-1.58 (4H, m), 2.62 (2H, s), 3.80 (3H, s), 3.95 (2H, m), 4.11 (2H, q, J=7.4), 4.45 (2H, s), 5.17 (1H, m), 5.28 (1H, m), 5.97 (1H, m), 6.50-6.65 (2H, m), 6.88 (1H, m). |
| II-128 | | 0.88 (6H, t, J=7.4), 1.45 (6H, t, J=7.4), 1.44-1.58 (4H, m), 2.62 (2H, s), 3.95 (2H, m), 4.11 (4H, q, J=7.4), 4.45 (2H, s), 5.17 (1H, m), 5.28 (1H, m), 5.97 (1H, m), 6.55-6.65 (2H, m), 6.88 (1H, m). |
| II-129 | 62-64°C | 0.88 (6H, t, J=7.4), 1.04 (3H, t, J=7.4), 1.43 (3H, t, J=7.4), 1.44-1.58 (4H, m), 1.86 (2H, sext, J=7.4), 2.62 (2H, s), 3.95 (2H, m), 3.98 (2H, t, J=7.4), 4.10 (2H, q, J=7.4), 4.49 (2H, s), 5.13 (1H, m), 5.28 (1H, m), 5.97 (1H, m), 6.55-6.65 (2H, m), 6.88 (1H, m). |
| II-130 | 104-105°C | 0.88 (6H, t, J=7.4), 1.06 (3H, t, J=7.4), 1.44-1.58 (4H, m), 1.86 (2H, sext, J=7.4), 2.62 (2H, s), 3.21 (3H, s), 3.95 (2H, m), 3.98 (2H, t, J=7.4), 4.43 (2H, s), 5.13 (1H, m), 5.28 (1H, m), 5.97 (1H, m), 6.84-688 (2H, m) 7.13 (1H, m). |
| II-131 | 70-72°C | 0.88 (6H, t, J=7.4), 1.04 (6H, t, J=7.4), 1.44-1.58 (4H, m), 1.86 (4H, m), 2.64 (2H, s), 3.95 (2H, m), 3.98 (2H, t, J=7.4), 4.49 (2H, s), 5.13 (1H, m), 5.28 (1H, m), 5.97 (1H, m), 6.55-6.65 (2H, m), 6.88 (1H, m). |
| II-132 | 59-60°C | 0.88 (6H, t, J=7.4), 1.04 (3H, t, J=7.4), 1.35 (6H, d, J=6.9), 1.44-1.58 (4H, m), 1.79 (2H, sext, J=7.4), 2.62 (2H, s), 3.95 (2H, m), 3.98 (2H, t, J=7.4), 4.46 (1H, sept, J=6.9), 4.46 (2H, s), 5.13 (1H, m), 5.28 (1H, m), 5.97 (1H, m), 6.52-6.61 (2H, m), 6.88 (1H, m). |
| II-133 | | 1.22 (6H, s), 2.30 (6H, s), 2.51-2.60 (2H, m), 2.65 (2H, s), 2.81-2.88 (2H, m), 3.95 (2H, m), 4.49 (2H, s), 5.17 (1H, m), 5.28 (1H, m), 5.97 (1H, m), 6.98 (2H, d, J=7.9), 7.20 (2H, d, J=7.9). |

**Table 65**

| Compound | Physical Properties | |
|---|---|---|
| No. | m.p. | NMR (CDCl₃) |
| II-134 | | 1.20 (6H, d, J=6.9), 1.32-1.60 (8H, m), 1.47 (9H, s), 1.70-1.81 (2H, m), 2.70 (2H, s), 3.09 (1H, sept, J=6.9), 3.97 (2H, s), 4.52 (2H, s), 6.95 (1H, m), 7.11-7.20 (2H, m), 7.31 (1H, m) |
| II-135 | | 1.20 (6H, d, J=6.9), 1.58-1.68 (2H, m), 1.93-1.97 (2H, m), 2.31 (3H, s), 2.38-2.59 (4H, m), 2.64 (3H, s), 2.68 (2H, s), 3.09 (1H, sept, J=6.9), 4.59 (2H, s), 6.91 (1H, m), 7.13-7.21 (2H, m), 7.33 (1H, m) |
| II-136 | | 1.11 (3H, t, J=6.9), 1.20 (6H, d, J=6.9), 1.65-1.70 (2H, m), 1.94-2.00 (2H, m), 2.41-2.50 (4H, m), 2.56-2.69 (2H, m), 2.65 (3H, s), 2.69 (2H, s), 3.09 (1H, sept, J=6.9), 4.60 (2H, s), 6.91 (1H, m), 7.13-7.21 (2H, m), 7.33 (1H, m) |
| II-137 | 67-68°C | 1.22 (6H, s), 2.65 (2H, s), 3.93-3.97 (2H, m), 4.45 (2H, s), 5.17 (1H, m), 5.28 (1H, m), 5.97 (1H, m), 6.85-6.91 (2H, m), 7.02 (1H, m). |
| II-138 | 80-82°C | 1.22 (6H, s), 2.66 (2H, s), 3.95 (2H, m), 4.46 (2H, s), 5.17 (1H, m), 5.28 (1H, m), 5.97 (1H, m), 6.85 (1H, dd, J=8.2,2.0), 7.16 (1H, d, J=2.0), 7.44 (1H, d, J=8.2). |
| II-139 | | 1.22 (6H, s), 2.21 (3H, s), 2.64 (2H, s), 3.93-3.97 (2H, m), 4.51 (2H, s), 5.17 (1H, m), 5.28 (1H, m), 5.97 (1H, m), 6.85 (1H, d, J=8.2), 7.16 (1H, dd, J=8.2, 2.0), 7.22 (1H, d, J=2.0). |
| II-140 | | 1.22 (6H, s), 2.30 (3H, s), 2.64 (2H, s), 3.95 (2H, m), 4.51 (2H, s), 5.17 (1H, m), 5.28 (1H, m), 5.97 (1H, m), 6.89 (1H, d, J=8.2), 7.16 (1H, dd, J=8.2, 2.0), 7.30 (1H, d, J=2.0). |
| II-141 | | 1.22 (6H, s), 2.65 (2H, s), 2.88 (2H, t, J=7.1), 3.36 (3H, s), 3.66 (2H, t, J=7.1), 3.95 (2H, m), 4.49 (2H, s), 5.17 (1H, m), 5.28 (1H, m), 5.97 (1H, m), 6.98 (2H, d, J=8.3), 7.20 (2H, d, J=8.3). |
| II-142 | | 1.25 (6H, d, J=6.9), 1.55-1.87 (8H, m), 2.72 (2H, s), 2.91 (1H, sept, J=6.9), 3.93 (2H, m), 4.54 (2H, s), 5.16 (1H, m), 5.30 (1H, m), 5.93 (1H, m), 6.95-7.00 (2H, m), 7.21-7.24 (2H, m) |
| II-143 | | 1.25 (6H, d, J=6.9), 1.47 (9H, s), 1.63-1.85 (8H, m), 2.78 (2H, s), 2.91 (1H, sept, J=6.9), 3.95 (2H, s), 4.53 (2H, ), 6.96-7.01 (2H, m), 7.20-7.24 (2H, m) |

**Table 66**

| Compound | Physical Properties | |
|---|---|---|
| No. | m.p. | NMR (CDCl₃) |
| II-144 | | 0.88 (6H, t, J=7.3), 1.25 (6H, d, J=6.9), 1.43-1.68 (4H, m), 2.61 (2H, s), 2.90 (1H, sept, J=6.9), 3.94 (2H, m), 4.45 (2H, s), 5.15 (1H, m), 5.31 (1H, m), 5.94 (1H, m), 6.95-6.99 (2H, m), 7.20-7.24 (2H, m) |
| II-145 | | 0.87 (6H, t, J=7.3), 1.25 (6H, d, J=6.9), 1.47 (9H, s), 1.48-1.70 (4H, m), 2.65 (2H, s), 2.90 (1H, sept, J=6.9), 3.96 (2H, s), 4.44 (2H, s), 6.97-7.01 (2H, m), 7.20-7.23 (2H, m) |
| II-146 | 90.5-92.5°C | 1.25 (6H, d, J=6.9), 1.30-1.62 (8H, m), 1.73-1.85 (2H, m), 2.66 (2H, s), 2.91 (1H, sept, J=6.9), 3.94 (2H, m), 4.54 (2H, s), 5.16 (1H, dd, J=9.9, 1.3), 5.31 (1H, m), 5.94 (1H, m), 6.96-7.00 (2H, m), 7.20-7.24 (2H, m) |
| II-147 | | 0.90 (6H, t, J=6.9), 1.15-1.57 (8H, m), 1.47 (9H, s), 2.64 (2H, s), 3.83 (3H, s), 3.96 (2H, s), 4.46 (2H, s), 6.92-6.97 (2H, m), 7.02 (1H, dd, J=7.9, 1.6), 7.13 (1H, m) |
| II-148 | | 1.00 (6H, d, J=6.9), 1.06 (6H, d, J=6.9), 1.46 (9H, s), 2.01 (2H, sept, J=6.9), 2.80 (2H, s), 3.82 (3H, s), 3.87 (2H, s), 4.66 (2H, s), 6.91-7.01 (3H, m), 7.13 (1H, m) |
| II-149 | | 0.92 (6H, t, J=7.3), 1.16-1.54 (8H, m), 2.61 (2H, s), 3.82 (3H, s), 3.94 (2H, dd, J=6.9, 1.0), 4.47 (2H, s), 5.16 (1H, m), 5.32 (1H, m), 5.94 (1H, m), 6.92-7.01 (3H, m), 7.13 (1H, m) |
| II-150 | | 0.85 (3H, t, J=7.3), 1.18 (3H, d, J=6.9), 1.47-1.68 (4H, m), 1.90-2.00 (2H, m), 2.31 (3H, s), 2.39-2.63 (4H, m), 2.65 (3H, s), 2.69 (2H, d, J=2.3), 2.89 (1H, sext, J=7.3), 4.46 (1H, d, J=13.8), 4.71 (1H, d, 13.8), 6.92 (1H, m), 7.12-7.29 (3H, m) |
| II-151 | | 1.37-1.63 (8H, m), 1.48 (9H, s), 1.70-1.83 (2H, m), 2.67 (2H, s), 4.02 (2H, s), 4.62 (2H, s), 7.11 (1H, dd, J=7.6, 1.3), 7.42-7.53 (3H, m), 7.67 (1H, d, J=8.2), 7.85 (1H, dd, J=6.9, 3.3), 8.07 (1H, m) |
| II-152 | | 0.88 (6H, t, J=7.3), 1.44-1.65 (4H, m), 1.49 (9H, s), 2.65 (2H, s), 4.02 (2H, s), 4.54 (2H, s), 7.11 (1H, dd, J=7.3, 1.0), 7.42-7.53 (3H, m), 7.67 (1H, J=8.2), 7.85 (1H, dd, J=5.6, 3.3), 8.07 (1H, dd, J=7.3, 3.3) |
| II-153 | | 1.21 (6H, d, J=6.9), 1.58-1.67 (2H, m), 2.31 (3H, s), 2.33 (3H, s), 2.41-2.45 (4H, m), 2.67 (2H, s), 3.13 (1H, sept, J=6.9), 3.89 (2H, s), 6.80 (1H, m), 7.10-7.18 (2H, m), 7.31 (1H, m) |
| II-154 | | 0.85 (3H, t, J=7.3), 1.19 (3H, d, J=7.3), 1.47-1.81 (6H, m), 2.31 (3H, s), 2.32 (3H, s), 2.40-2.50 (4H, m), 2.67 (2H, s), 2.92 (1H, sext, J=7.3), 3.84 (1H, d, J=13.9), 6.80 (1H, m), 7.11-7.17 (2H, m), 7.25 (1H, m) |

**Table 67**

| | | | | | |
|---|---|---|---|---|---|
| 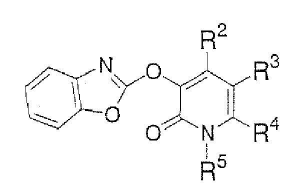 | | | | | |

| Compound No. | R² | R³ | R⁴ | R⁵ | ¹H-NMR (CDCl₃) |
|---|---|---|---|---|---|
| 1-001 | H | Me | Me | Me | 2.16 (s, 3H), 2.33 (s, 3H), 3.62 (s, 3H), 7.17-7.26 (m, 2H), 7.34 (s, 1H), 7.34-7.43 (m, 1H), 7.46-7.50 (m, 1H). |
| 1-002 | H | Me | Me | Et | 1.32 (t, *J* = 7.2 Hz, 3H), 2.15 (s, 3H), 2.36 (s, 3H), 4.19 (q, *J* = 7.2 Hz, 2H), 7.19-7.25 (m, 2H), 7.34 (s, 1H), 7.39-7.42 (m, 1H), 7.46-7.49 (m, 1H). |
| 1-003 | H | Me | Me | nPr | 0.98 (t, *J* = 7.2 Hz, 3H), 1.65-1.78 (m, 2H), 2.15 (s, 3H), 2.34 (s, 3H), 4.03-4.08 (m, 2H), 7.16-7.26 (m, 2H), 7.33 (s, 1H), 7.38-7.41 (m, 1H), 7.46-7.49 (m, 1H). |
| 1-004 | H | Me | Me | nBu | 0.95 (t, *J* = 7.5 Hz, 3H), 1.35-1.48 (m, 2H), 1.62-1.72 (m, 2H), 2.15 (s, 3H), 2.35 (s, 3H), 4.10 (t, *J* = 7.8 Hz, 2H), 7.19-7.25 (m, 2H), 7.33 (s, 1H), 7.38-7.42 (m, 1H), 7.46-7.49 (m, 1H). |
| 1-005 | H | Me | Me | Bn | 2.14 (s, 3H), 2.25 (s, 3H), 5.42 (br s, 2H), 7.17-7.51 (m, 10H). |
| 1-006 | H | 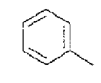 | H | nBu | 0.94 (t, *J* = 7.4 Hz, 3H), 1.35-1.48 (m, 2H), 1.76-1.86 (m, 2H), 4.06 (t, *J* = 7.4 Hz, 2H), 7.22-7.28 (m, 3H), 7.34-7.51 (m, 7H), 7.81 (d, *J* = 2.5 Hz, 1H). |
| 1-007 | H | 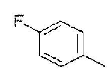 | H | nBu | 0.96 (t, *J* = 7.3 Hz, 3H), 1.35-1.48 (m, 2H), 1.75-1.85 (m, 2H), 4.05 (t, *J* = 7.3 Hz, 2H), 7.10-7.17 (m, 2H), 7.22-7.24 (m, 3H), 7.37-7.44 (m, 3H), 7.48-7.52 (m, 1H), 7.76 (d, *J* = 2.7 Hz, 1H). |
| 1-008 | H | 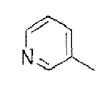 | H | nBu | 0.97 (t, *J* = 7.3 Hz, 3H), 1.36-1.49 (m, 2H), 1.79-1.87 (m, 2H), 4.08 (t, *J* = 7.3 Hz, 2H), 7.23-7.27 (m, 2H), 7.37-7.44 (m, 2H), 7.45-7.52 (m, 1H), 7.50 (d, *J* = 2.7 Hz, 1H), 7.75-7.78 (m, 1H), 7.81 (d, *J* = 2.7 Hz, 1H), 8.61 (d, *J* = 3.7 Hz, 1H), 8.74 (s, 1H). |

**Table 68**

| | | | | | |
|---|---|---|---|---|---|
| 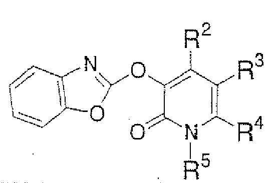 | | | | | |

| Compound No. | R² | R³ | R⁴ | R⁵ | ¹H-NMR (CDCl₃) |
|---|---|---|---|---|---|
| 1-009 | H | 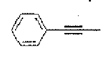 | H | nBu | 0.97 (t, *J* = 7.4 Hz, 3H), 1.38-1.48 (m, 2H), 1.75-1.85 (m, 2H), 4.05 (t, *J* = 7.4 Hz, 2H), 6.92 (s, 1H), 7.23-7.52 (m, 6H), 7.67-7.69 (m, 2H), 7.71 (d, *J* = 2.7 Hz, 1H), 7.89 (d, *J* = 2.7 Hz, 1H). |
| 1-010 | Me | H | Me | nBu | 0.94 (t, *J* = 7.5 Hz, 3H), 1.39 (sextet, *J* = 7.5 Hz, 2H), 1.61-1.71 (m, 2H), 2.21 (s, 3H), 2.37 (s, 3H), 3.99 (t, *J* = 7.8 Hz, 2H), 5.95 (s, 1H), 7.18 (ddd, *J* = 7.5, 7.5, 1.8 Hz), 7.23 (ddd, J = 7.5, 7.5, 1.8 Hz, 1H), 7.40 (m, 1H), 7.47 (m, 1H), |
| 1-011 | 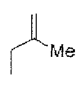 | H | Me | nBu | 0.94 (t, *J* = 7.5 Hz, 3H), 1.37 (sextet, *J* = 7.5 Hz, 2H), 1.68-1.78 (m, 2H), 1.73 (d, J= 1.0 Hz, 3H), 3.32 (s, 2H), 3.94 (t, *J* = 7.5 Hz, 2H), 4.82 (s, 1H), 4.88 (s, 1H), 6.13 (d, *J* = 7.2 Hz, 1H), 7.17 (d, *J* = 7.2 Hz, 1H), 7.19 (ddd, *J* = 7.5, 7.5, 1.5 Hz, 1H), 7.23 (ddd, J = 7.5, 7.5, 1.5 Hz, 1H), 7.40 (m, 1H), 7.48 (m, 1H), |

**Table 69**

| Compound No. | Structure | ¹H-NMR (CDCl₃) |
|---|---|---|
| 1-012 | 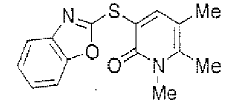 | 2.16 (s, 3H), 2.36 (s, 3H), 7.20-7.29 (m, 2H), 7.40-7.44 (m, 1H9, 7.58-7.61 (m, 1H), 7.83 (s, 1H). |
| 1-013 | 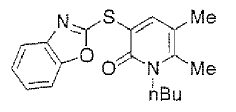 | 0.95 (t, *J* = 7.2 Hz), 1.35-1.48 (m, 2H), 1.60-1.72 (m, 2H), 2.15 (s, 3H), 2.39 (s, 3H), 4.11 (t, *J* = 7.8 Hz, 2H), 7.22-7.29 (m, 2H), 7.41-7.44 (m, 1H), 7.57-7.61 (m, 1H), 7.81 (s, 1H). |
| 1-014 | 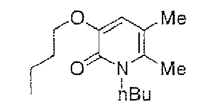 | 0.96 (t, *J* = 7.2 Hz, 6H), 1.30-1.60 (m, 4H), 1.60-1.75 (m, 2H), 1.76-1.90 (m, 2H), 2.31 (s, 3H), 3.89 (t, *J* = 6.9 Hz, 2H), 4.02 (t, *J* = 8.1 Hz, 2H), 5.88 (d, *J* = 7.8 Hz, 1H), 6.52 (d, *J* = 7.2 Hz, 1H). |
| 1-015 | 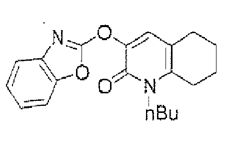 | 0.94 (t, *J* = 7.5 Hz, 3H), 1.40 (sextet, *J* = 7.5 Hz, 2H), 1.66 (quint, *J* = 7.5 Hz, 2H), 1.74 (quint, *J* = 6.0 Hz, 2H), 1.87 (quint, *J* = 6.0 Hz, 2H), 2.58 (t, *J* = 6.0 Hz, 2H), 2.69 (t, *J* = 6.0 Hz, 2H), 4.02 (t, *J* = 7.8 Hz, 2H), 7.16-7.26 (m, 2H), 7.24 (s, 1H), 7.40 (dd, *J* = 6.9 Hz, 2.4 Hz, 1H), 7.48 (dd, *J* = 6.9 Hz, 2.4 Hz, 1H). |
| 1-016 | 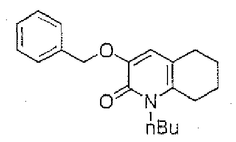 | 0.96 (t, *J* = 7.5 Hz, 3H), 1.42 (sextet, *J* = 7.5 Hz, 2H), 1.80-1.76 (m, 4H), 1.81 (quint, *J* = 6.0 Hz, 2H), 2.43 (t, *J* = 6.0 Hz, 2H), 2.61 (t, *J* = 6.0 Hz, 2H), 4.01 (t, *J* = 7.8 Hz, 2H), 5.07 (s, 2H), 6.43 (s, 1H), 7.28-7.39 (m, 1H), 7.34 (d, *J* = 7.5 Hz, 2H), 7.45 (d, *J* = 7.5 Hz, 2H). |
| 1-017 | 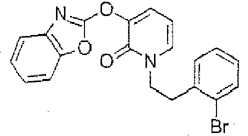 | 3.23 (t, *J* = 7.5 Hz, 2H), 4.24 (t, *J* = 7.5 Hz, 2H), 6.10 (t, *J* = 6.9 Hz, 1H), 6,99 (dd, *J* = 1.8, 6.9 Hz, 1H), 7.08-7.29 (m, 5H), 7.42-7.45 (m, 1H), 7.49-7.52 (m, 2H), 7.56 (dd, *J* = 1.2, 7.8 Hz, 1H). |
| 1-018 | 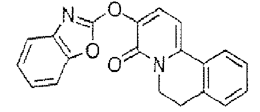 | 3.03 (t, *J* = 6.1 Hz, 2H), 4.34 (t, *J* = 6.1 Hz, 2H), 6.74 (d, *J* = 7.9 Hz, 1H), 7.19-7.45 (m, 6H), 7.50 (d, *J* = 6.4 Hz, 1H), 7.61 (d, *J* = 7.9 Hz, 1H), 7.73 (d, *J* = 7.3Hz, 1H). |
| 1-019 | 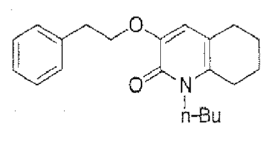 | 0.96 (t, *J* = 7.5 Hz, 3H), 1.41 (sextet, *J* = 7.5 Hz, 2H), 1.58-1.73 (m, 4H), 1.81 (quint, *J* = 6.0 Hz, 2H), 2.45 (t, *J* = 6.0 Hz, 2H), 2.61 (t, *J* = 6.0 Hz, 2H), 3.18 (t, *J* = 7.5 Hz, 2H), 4.00 (t, *J* = 7.8 Hz, 2H), 4.07 (t, *J* = 7.5 Hz, 2H), 6.34 (s, 1H), 7.21-7.33 (m, 5H). |

**Table 70**

| | | | |
|---|---|---|---|
| 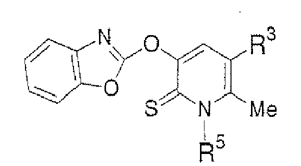 | | | |

| Compound No. | R³ | R⁵ | ¹H-NMR (CDCl₃) |
|---|---|---|---|
| 2-001 | Me | Me | 2.28 (s, 3H), 2.49 (s, 3H), 4.17 (s, 3H), 7.19-7.24 (m, 2H), 7.40 (s, 1H), 7.43-7.49 (m, 2H). |
| 2-002 | Me | Et | 1.46 (t, *J* = 7.2 Hz, 3H), 2.25 (s, 3H), 2.55 (s, 3H), 4.92 (br s, 2H), 7.18-7.24 (m, 2H), 7.37 (s, 1H), 7.42-7.49 (m, 2H). |
| 2-003 | Me | nPr | 1.04 (t, *J* = 7.2 Hz, 3H), 1.89 (br s, 2H), 2.25 (s, 3H), 2.52 (s, 3H), 4.71 (br s, 2H), 7.19-7.26 (m, 2H), 7.36 (s, 1H), 7.42-7.49 (m, 2H). |
| 2-004 | Me | nBu | 0.99 (t, *J* = 7.2 Hz, 3H), 1.42-1.54 (m, 2H), 1.83 (by s, 2H), 2.25 (s, 3H), 2.53 (s, 3H), 4.80 (br s, 2H), 7.18-7.26 (m, 2H), 7.36 (s, 1H), 7.42-7.49 (m, 2H). |
| 2-005 | Me | iBu | 0.97-0.99 (m, 6H), 2.27 (s, 3H), 2.51 (s, 3H), 2.51-2.66 (m, 1H), 3.81 (br s, 1H), 5.64 (br s, 1H), 7.20-7.24 (m, 2H), 7.39 (s, 1H), 7.42-7.48 (m, 2H). |
| 2-006 | Me | nPent | 0.92 (t, *J* = 7.2 Hz, 3H), 1.36-1.48 (m, 4H), 1.85 (br s, 2H), 2.25 (s, 3H), 2.53 (s, 3H), 4.76 (br s, 2H), 7.18-7.26 (m, 2H), 7.36 (s, 1H), 7.42-7.49 (m, 2H). |
| 2-007 | Me | nHexyl | 0.89 (t, *J* = 7.2 Hz, 3H), 1.30-1.50 (m, 6H), 1.84 (br s, 2H), 2.25 (s, 3H), 2.52 (s, 3H), 4.79 (br s, 2H), 7.17-7.26 (m, 2H), 7.35 (s, 1H), 7,42-7.49 (m, 2H). |
| 2-008 | Me | Bn | 2.24 (s, 3H), 2.38 (s, 3H), 6.27 (br s, 2H), 7.14-7.52 (m, 10H). |
| 2-009 | Et | Me | 1.23 (t, *J* = 7.8 Hz, 3H), 2.50 (s, 3H), 2.61 (q, *J* = 7.8 Hz, 2H), 4.17 (s, 3H), 7.19-7.24 (m, 2H), 7.42 (s, 1H), 7.42-7.49 (m, 2H). |
| 2-010 | Et | Et | 1.23 (t, *J* = 7.5 Hz, 3H), 1.47 (t, *J* = 7.2 Hz, 3H), 2.57 (s, 3H), 2.59 (q, *J* = 7.5 Hz, 2H), 4.92 (br s, 2H), 7.18-7.24 (m, 2H), 7.39 (s, 1H), 7.43-7.49 (m, 2H). |
| 2-011 | Et | nPr | 1.04 (t, *J* = 7.2 Hz, 3H), 1.22 (t, *J* = 7.5 Hz, 3H), 1.89 (br s, 2H), 2.54 (s, 3H), 2.59 (q, *J* = 7.5 Hz, 2H), 4.72 (br s, 2H), 7.18-7.24 (m, 2H), 7.38 (s, 1H), 7.42-7.49 (m, 2H). |
| 2-012 | Et | nBu | 0.99 (t, *J* = 7.2 Hz, 3H), 1.22 (t, *J* = 7.5 Hz, 3H), 1.42-1.54 (m, 2H), 1.83 (br s, 2H), 2.55 (s, 3H), 2.59 (q, *J* = 7.5 Hz, 2H), 4.77 (br s, 2H), 7.20-7.24 (m, 2H), 7.38 (s, 1H), 7.42-7.49 (m, 2H). |
| 2-013 | Et | Bn | 1.22 (t, *J* = 7.5 Hz, 3H), 2.40 (s, 3H), 2.57 (q, *J* = 7.5 Hz, 2H), 6.26 (br s, 2H), 7.13-7.51 (m, 10H). |

**Table 71**

| | | | |
|---|---|---|---|
| 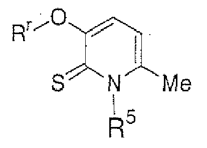 | | | |

| Compound No. | R^{r} | R⁵ | ¹H-NMR (CDCl₃) |
|---|---|---|---|
| 2-014 | 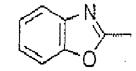 | Me | 2.55 (s, 3H), 4.10 (s, 3H), 6.57 (d, *J* = 7.8 Hz, 1H), 7.20-7.26 (m, 2H), 7.40-7.50 (m, 3H). |
| 2-015 | 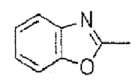 | nBu | 0.99 (t, *J* = 7.2 Hz, 3H), 1.47 (sextet, *J* = 7.5 Hz, 2H), 1.84 (m, 2H), 2.58 (s, 3H), 4.69 (br s, 2H), 6.52 (d, *J* = 7.8 Hz, 1H), 7.20-7.26 (m, 2H), 7.30-7.50 (m, 3H). |
| 2-016 | 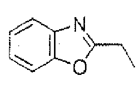 | nBu | 0.82 (t, *J* = 7.5 Hz, 3H), 1.32 (sextet, *J* = 7.5 Hz, 2H), 1.47-1.52 (m, 2H), 2.46 (s, 3H), 4.37 (br s, 2H), 4.80 (s, 2H), 7.06 (d, *J* = 9.0 Hz, 1H), 7.26-7.35 (m, 3H), 7.38-7.44 (m, 1H), 7.60-7.67 (m, 1H). |
| 2-017 | Ac | nBu | 0.99 (t, *J* = 7.5 Hz, 3H), 1.47 (sextet, *J* = 7.5 Hz, 2H), 1.83 (m, 2H), 2.38 (s, 3H), 2.54 (s, 3H), 4.70 (br s, 2H), 6.44 (d, *J* = 7.8 Hz, 1H), 7.04 (d, *J* = 7.5 Hz, 1H). |
| 2-018 | H | nBu | 1.02 (t, *J* = 7.8 Hz, 3H), 1.50 (sextet, *J* = 7.8 Hz, 2H), 1.80-1.90 (m, 2H), 2.51 (s, 3H), 4.66 (br s, 2H), 6.49 (d, *J* = 8.1 Hz, 1H), 6.91 (d, *J* = 7.8 Hz, 1H), 8.44 (br s, 1H). |
| 2-019 | 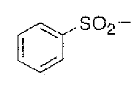 | nBu | 0.96 (t, *J* = 7.5 Hz, 3H), 1.41 (sextet, *J* = 7.5 Hz, 2H), 1.70 (m, 2H), 2.43 (s, 3H), 2.52 (s, 3H), 4.61 (brs, 2H), 6.38 (d, *J* = 8.1 Hz, 1H), 7.26-7.35 (m, 3H), 7.97 (d, *J* = 8.7 Hz, 1H). |
| 2-020 | H₃C-SO₂- | nBu | 1.01 (t, *J* = 7.5 Hz, 3H), 1.49 (sextet, *J* = 7.2 Hz, 2H), 1.82 (m, 2H), 2.57 (s, 3H), 3.48 (dd, *J* = 3.0, 1.5 Hz, 3H), 4.70 (brs, 2H), 6.47 (d, *J* = 7.8 Hz, 1H), 7.31 (dd, *J* = 7.8, 1.8 Hz, 1H). |
| 2-021 | 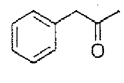 | nBu | 0.98 (t, *J* = 7.2 Hz, 3H), 1.46 (sextet, *J* = 7.5 Hz, 2H), 1.81 (m, 2H), 2.51 (s, 3H), 4.00 (s, 2H), 4.67 (brs, 2H), 6.39 (d, *J* = 7.8 Hz, 1H), 6.98 (d, *J* = 7.8 Hz, 1H), 7.10-7.50 (m, 5H). |
| 2-022 | 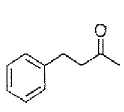 | nBu | 0.99 (t, *J* = 7.2 Hz, 3H), 1.47 (sextet, *J* = 7.2 Hz, 2H), 1.85 (m, 2H), 2.54 (s, 3H), 2.90-3.00 (m, 2H), 3.10-3.20 (m, 2H), 4.70 (brs, 2H), 3.10-3.20 (m, 2H), 4.70 (brs, 2H), 6.42 (d, *J* = 8.1 Hz, 1H), 6.97 (d, *J* = 8.1 Hz, 1H), 7.18-7.34 (m, 5H). |

**Table 72**

| | | | |
|---|---|---|---|
| 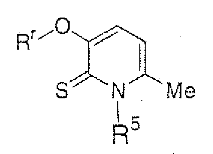 | | | |

| Compound No. | R^{r} | R⁵ | ¹H-NMR (CDCl₃) |
|---|---|---|---|
| 2-023 | 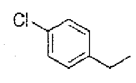 | nBu | 0.92 (t, *J* = 6.9 Hz, 3H), 1.37 (m 4H), 2.41 (s, 3H), 4.17 (brs, 2H), 4.47 (s, 2H), 6.99 (d, *J* = 9.0 Hz, 1H), 7.00-7.30 (m, 5H). |
| 2-024 | 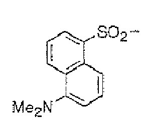 | nBu | 0.94 (t, *J* = 6.9 Hz, 3H), 1.40 (sextet, *J* = 7.8 Hz, 2H), 1.70 (m ,2H), 2.48 (s, 3H), 2.89 (s, 6H), 4.60 (br s, 2H), 6.27 (d, *J* = 8.1 Hz, 1H), 6.97 (dd, *J* = 8.1, 1-2 Hz, 1H), 7.21 (d, *J* = 7.8 Hz, 1H), 7.51 (dd, *J* = 8.1, 7.8 Hz, 1H), 7.61 (dd, *J* = 8.4, 7.8 Hz, 1H), 8.28 (dd, *J* = 7.2, 0.9 Hz, 1H), 8.61 (t, *J* = 8.4 Hz, 2H). |
| 2-025 | 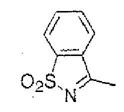 | nBu | 1.02 (t, *J* = 7.5 Hz, 3H), 1.50 (sextet, *J* = 7.5 Hz, 2H), 1.80-1.85 (m ,2H), 2.51 (s, 3H), 4.67 (br s, 2H), 6.51 (dd, *J* = 5-1, 4.8 Hz, 1H), 6.57 (d, *J* = 7.8 Hz, 1H), 7.38 (d, *J* = 8.1 Hz, 1H), 7.70-7.85 (m, 2H). |
| 2-026 | nBu | nBu | 0.90-1.03 (m, 6H), 1.4-1.6 (m, 4H), 1.8-1.9 (m, 4H), 2.50 (s, 3H), 3.98 (t, *J* = 6.9 Hz, 2H), 4.76 (brs, 2H), 6.40 (d, *J* = 8.1 Hz, 1H), 6.60 (d, *J* = 7.8 Hz, 1H). |
| 2-027 | 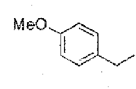 | nBu | 0.91 (t, *J* = 7.2Hz, 3H), 1.25-1.44 (m, 4H,), 1.25-1.44 (m, 4H), 2.40 (s, 3H), 3.75 (s, 3H), 4.18 (brs, 2H), 4.44 (s,2H), 6.73 (A₂B₂-type, *J* = 8.7Hz, 2H), 6.98 (d, *J* =9.3Hz, 1H), 7.09 (A₂B₂-type, *J* = 8.4Hz, 2H), 7.25 (d, *J* = 9.0Hz, 1H). |
| 2-028 | EtO₂C- | nBu | 0.99 (t, *J* = 7.2 Hz, 3H), 1.40 (t, *J* = 7.2 Hz, 3H), 1.47 (sextet, *J* = 7.5 Hz, 2H), 1.84 (m, 2H), 2.55 (s, 3H), 4.35 (q, *J* = 7.5 Hz, 2H), 4.69 (brs, 2H), 6.45 (dd, J= 7.5, 0.6 Hz, 1H), 7.12 (d, *J* = 7.5 Hz, 1H). |
| 2-029 | 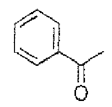 | nBu | 0.99 (t, *J* = 7.2 Hz, 3H), 1.48 (sextet, J= 7.2 Hz, 2H), 1.85 (m, 2H), 2.57 (s, 3H), 4.73 (brs, 2H), 6.48 (d, *J* = 7.8 Hz, 1H), 7.18 (d, *J* = 7.5 Hz, 1H), 7.20-7.70 (m, 3H), 8.20-8.30 (m, 2H). |

**Table 73**

| | | | | | |
|---|---|---|---|---|---|
| 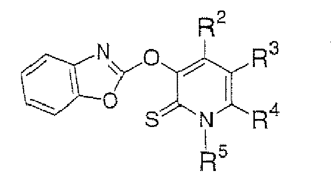 | | | | | |

| Compound No. | R² | R³ | R⁴ | R⁵ | ¹H-NMR (CDCl₃) |
|---|---|---|---|---|---|
| 2-030 | H | H | H | iPr | 1.45 (s, 3H), 1.48 (s, 3H), 6.31-6.45 (m, 1H), 6.76 (t, *J* = 7.0 Hz, 1H), 7.03-7.29 (m, 3H), 7.43-7.29 (m, 3H), 7.43-7.54 (m, 2H), 7.74 (dd, *J* = 1.5, 7.0 Hz, 1H). |
| 2-031 | Me | H | H | nPr | 1.00 (t, *J* = 7.3 Hz, 3H), 1.83-2.02 (m, 2H), 4.48 (t, *J* = 7.7 Hz, 2H), 6.56 (d, *J* = 6-6 Hz, 1H), 7.20-7.28 (m, 2H), 7.43-7.49 (m, 2H), 7.57 (d, *J* = 6.6 Hz, 1H). |
| 2-032 | -CH₂OMe | H | H | nPr | 0.96 (t, *J* = 7.3 Hz, 3H), 1.35-1.47 (m, 2H), 1.81-1.91 (m, 2H), 3.43 (s, 3H), 4.48-4.56 (m, 3H), 6.89 (d, *J* = 6.7 Hz, 1H), 6.97-7.48 (m, 4H), 7.68 (d, *J* = 6.7 Hz, 1H). |
| 2-033 | H | H | H | nBu | 0.98 (t, *J* = 7.3 Hz, 3H), 1.37-1.49 (m, 2H), 1.83-1.94 (m, 2H), 4.57 (t, *J* = 7.6 Hz, 2H), 6.65-6.70 (m, 1H), 7.22-7.27 (m, 2H), 7.43-7.51 (m, 3H), 7.68 (dd, *J* = 1.5, 6.4 Hz, 1H). |
| 2-034 | Me | H | H | nBu | 0.95 (t, *J* = 7.3 Hz, 3H), 1.34-1.46 (m, 2H), 1.79-1.90 (m, 2H), 2.29 (s, 3H), 4.51 (t, *J* = 7.4 Hz, 2H), 6.55 (d, *J* = 6.6 Hz, 1H), 7.20-7.28 (m, 2H), 7.43-7.48 (m, 2H), 7.59 (d, *J* = 6.6 Hz, 1H). |
| 2-035 | H | Me | H | nBu | 0.97 (t, *J* = 7.3 Hz, 3H), 1.36-1.46 (m, 2H), 1.82-1.92 (m, 2H), 4.54 (t, *J* = 7.6 Hz, 2H), 7.19-7.27 (m, 2H), 7.40-7.52 (m, 4H). |
| 2-036 | H | Br | H | nBu | 0.99 (t, *J* = 7.5 Hz, 3H), 1.43 (sextet, *J* = 7.5 Hz, 2H), 1.83-1.93 (m , 2H), 4.53 (t, *J* = 7.5 Hz, 2H), 7.21-7.30 (m, 2H), 7.42-7.52 (m, 2H), 7.64 (d, J= 2.1 Hz, 1H), 7.79 (d, *J* = 2.1 Hz, 1H). |
| 2-037 | H | 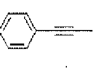 | H | nBu | 1.00 (t, *J* = 7.3 Hz, 3H), 1.45 (sextet, *J* = 7.3 Hz, 2H), 1.85-1.97 (m, 2H), 4.57 (t, *J* = 7.6 Hz, 2H), 7.22-7.28 (m, 2H), 7.34-7.44 (m, 3H), 7.44-7.52 (m, 4H), 7.61 (d, *J* = 1.8 Hz, 1H), 7.89 (d, *J* = 1.8 Hz, 1H). |

**Table 74**

| | | | |
|---|---|---|---|
| 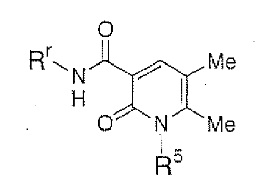 | | | |

| Compound No. | R^{r} | R⁵ | ¹H-NMR (CDCl₃) |
|---|---|---|---|
| 3-001 | 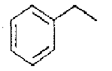 | Me | 2.20 (s, 3H), 2.39 (s, 3H), 3.62 (s, 3H), 4.65 (d, *J* = 6.0 Hz, 2H), 7.21-7.38 (m, 5H), 8.37 (s, 1H), 10.28 (br s, 1H). |
| 3-002 | 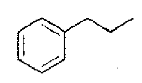 | Me | 2.19 (s, 3H), 2.38 (s, 3H), 2.93 (t, *J* = 7.2 Hz, 2H), 3.62 (s, 3H), 3.65-3.72 (m, 2H), 7.21-7.33 (m, 5H), 8.34 (s, 1H), 9.99 (br s, 1H). |
| 3-003 | 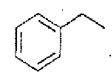 | Et | 1.32 (t, *J* = 7.2 Hz, 3H), 2.18 (s, 3H), 2.42 (s, 3H), 4.20 (q, *J* = 7.2 Hz, 2H), 4.64 (d, *J* = 6.0 Hz, 2H), 7.24-7.38 (m, 5H), 8.35 (s, 1H), 10.30 (br s, 1H), |
| 3-004 | 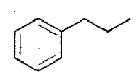 | Et | 1.33 (t, *J* = 7.2 Hz, 3H), 2.18 (s, 3H), 2.42 (s, 3H), 2.93 (t, *J* = 7.5 Hz, 2H), 3.64-3.71 (m, 2H), 4.21 (q, *J* = 7.2 Hz, 2H), 7.18-7.33 (m, 5H), 8.32 (s, 1H), 10.03 (br s, 1H). |
| 3-005 | 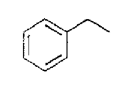 | nPr | 1.03 (t, *J* = 7.8 Hz, 3H), 1.65-1.78 (m, 2H), 2.19 (s, 3H), 2.42 (s, 3H), 4.07 (t, *J* = 8.1 Hz, 2H), 4.65 (d, *J* = 6.0 Hz, 2H), 7.24-7.38 (m, 5H), 8.36 (s, 1H), 10.30 (br s, 1H). |
| 3-006 | 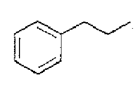 | nPr | 1.05 (t, *J* = 7.5 Hz, 3H), 1.67-1.80 (m, 2H), 2.19 (s, 3H), 2.42 (s, 3H), 2.92-2.97 (m, 2H), 3.64-3.72 (m, 2H), 4.09 (t, *J* = 7.8 Hz, 2H), 7.20-7.35 (m, 5H), 8.33 (s, 1H), 10.05 (br s, 1H). |
| 3-007 | 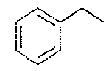 | iPr | 1.60 (s, 3H), 1.63 (s, 3H), 2.17 (s, 3H), 2.40 (s, 3H), 4.64 (d, *J* = 6.0 Hz, 3H), 7.24-7.34 (m, 5H), 8.31 (s, 1H), 10.31 (br s, 1H). |
| 3-008 | 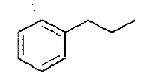 | iPr | 1.62 (s, 3H), 1.64 (s, 3H), 2.17 (s, 3H), 2.40 (s, 3H), 2.93 (d, *J* = 7.8 Hz, 2H), 3.62-3.69 (m, 2H), 4.64 (br s, 1H), 7.18. 7.33 (m, 5H), 8.28 (s, 1H), 10.04 (br s, 1H). |
| 3-009 | 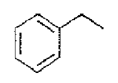 | nBu | 0.98 (t, *J* = 7.2 Hz, 3H), 1.38-1.51 (m, 2H), 1.61-1.71 (m, 2H), 2.18 (s, 3H), 2.41 (s, 3H), 4.10 (t, *J* = 8.1 Hz, 2H), 4.64 (d, *J* = 6.0 Hz, 2H), 7.21-7.38 (m, 5H), 8.35 (s, 1H), 10.30 (br s, 1H). |
| 3-010 | 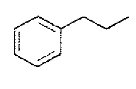 | nBu | 1.00 (t, *J* = 7.2 Hz, 3H), 1.40 (m, 2H), 1.61-1.72 (m, 2H), 2.93 (t, *J* = 7.2 Hz, 2H), 3.63.3.70 (m, 2H), 4.11 (t, *J* = 7.8 Hz, 2H), 7.18-7.32 (m, 5H), 8.32 (s, 1H), 10-03 (br s, 1H). |

**Table 75**

| | | | |
|---|---|---|---|
| 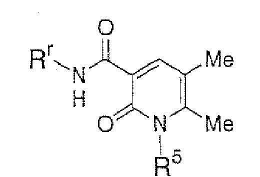 | | | |

| Compound No. | R^{r} | R⁵ | ¹H-NMR (CDCl₃) |
|---|---|---|---|
| 3-011 | 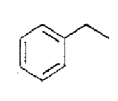 | nHexyl | 0.89 (t, *J =* 7.2 Hz, 3H), 1.30-1.50 (m, 6H), 1.60-1.75 (m, 2H), 2.18 (s, 3H), 2.41 (s, 3H), 4.09 (t, *J* = 7.8 Hz, 2H), 4.64 (d, *J* = 5.7 Hz, 2H), 7.23-7.38 (m, 5H), 8.35 (s, 1H). |
| 3-012 | 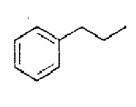 | nHexyl | 0.91 (t, *J* = 6.9 Hz, 3H), 1.32-1.45 (m, 6H), 1.63-1.70 (m, 2H), 2.18 (s, 3H), 2.40 (s, 3H), 2.93 (t, *J* = 7.5 Hz, 2H), 3,63-3,70 (m, 2H), 4.10 (t, *J* = 7.8 Hz, 2H), 7.18-7.32 (m, 5H), 8.31 (s, 1H), 10.04 (br s, 1H). |
| 3-013 | 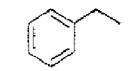 | Bn | 2.19 (s, 3H), 2.31 (s, 3H), 4.64 (d, *J* = 5.7 Hz, 2H), 5.44 (br s, 2H), 7.07-7.38 (m, 10H), 8.44 (s, 1H), 10.24 (br s, 1H). |
| 3-014 | 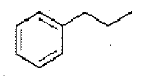 | Bn | 2.18 (s, 3H), 2.31 (s, 3H), 2.93 (t, *J* = 7.5 Hz, 2H), 3.64-3.71 (m, 2H), 5.45 (br s, 2H), 7.08-7.36 (m, 10H), 8.41 (s, 1H), 9.98 (br s, 1H). |
| 3-015 | 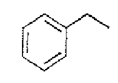 | Ph | 2.00 (s, 3H), 2.22 (s, 3H), 4.58 (d, *J* = 5.7 Hz, 2H), 7.15-7.32 (m, 7H), 7.49-7.58 (m, 3H), 8.49 (s, 1H), 10.02 (br s, 1H). |
| 3-016 | 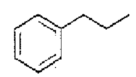 | Ph | 2.00 (s, 3H), 2.22 (s, 3H), 2.88 (t, *J* = 7.8 Hz, 2H), 3.59-3.66 (m, 2H), 7.16-7.29 (m, 7H), 7.51-7.61 (m, 3H), 8.46 (s, 1H), 9.82 (br s, 1H). |

**Table 76**

| | | | |
|---|---|---|---|
| 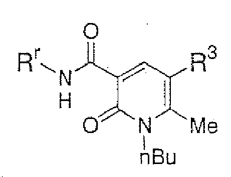 | | | |

| Compound No. | R^{r} | R³ | ¹H-NMR (CDCl₃) |
|---|---|---|---|
| 3-033 | 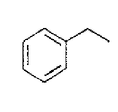 | nBu | 0.93 (t, *J* = 7.2 Hz, 3H), 0.98 (t, *J* = 7.2 Hz, 3H), 1.32-1.51 (m, 6H), 1.61-1.69 (m, 2H), 2.41 (s, 3H), 2.48 (t, *J* = 7.8 Hz, 2H), 4.09 (t, *J* = 7.8 Hz, 2H), 4.64 (d, *J* = 6.0 Hz, 2H), 7.23-7.38 (m, 5H), 8.35 (s, 1H), 10.30 (br s, 1H). |
| 3-034 | 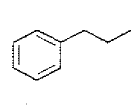 | nBu | 0.93 (t, *J* = 7.2 Hz, 3H), 1.00 (t, *J* = 7.2 Hz, 3H), 1.30-1.54 (m, 6H), 1.63-1.72 (m, 2H), 2.42 (s, 3H), 2.48 (t, *J* = 7.8 Hz, 2H), 2.93 (m, 2H), 3.62-3.70 (m, 2H), 4.10 (t, *J* = 7.8 Hz, 2H), 7.16-7.32 (m, 5H), 8.32 (s, 1H), 10.04 (br s, 1H). |
| 3-035 | 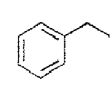 | nPentyl | 0.90 (t, *J* = 6.9 Hz, 3H), 0.98 (t, *J* = 7.2 Hz, 3H), 1.30-1.53 (m, 8H), 1.62-1.69 (m, 2H), 2.47 (s, 3H), 2.48 (t, *J* = 7.5 Hz, 2H), 4.09 (t, *J* = 7.8 Hz, 2H), 4.64 (d, *J* = 5.7 Hz, 2H), 7.23-7.38 (m, 5H), 8.35 (s, 1H), 10.31 (br s, 1H). |
| 3-036 | 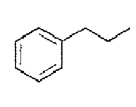 | nPentyl | 0.90 (t, *J* = 6.9 Hz, 3H) , 1.00 (t, *J* = 7.2 Hz, 3H), 1.28-1.39 (m, 4H), 1.40-1.55 (m, 4H), 1.62-1.72 (m, 2H), 2.42 (s, 3H), 2.47 (t, *J* = 7.5 Hz, 2H), 2.93 (t, *J* = 7.2 Hz, 2H), 3.63-3.70 (m, 2H), 4.10 (t, *J* = 7.8 Hz, 2H), 7.20-7.32 (m, 5H), 8.32 (s, 1H), 10.04 (br s, 1H). |
| 3-037 | 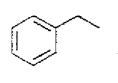 | I | 0.98 (t, *J* = 7.3 Hz, 3H), 1.38-1.50 (m, 2H), 1.61-1.71 (m, 2H), 2.71 (s, 3H), 4.16 (t, *J* = 7.9 Hz, 2H), 4.63 (d, *J* = 5.8 Hz, 2H), 7.22-7.37 (m, 5H), 8.78 (s, 1H), 10.4 (br s, 1H). |
| 3-038 | 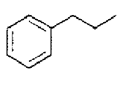 | I | 1.00 (t, *J* = 7.3 Hz, 3H), 1.39-1.51 (m, 2H), 1.59 (s, 3H), 1.61-1.71 (m, 2H), 2.71 (s, 3H), 2.92 (t, *J* = 7.6 Hz, 2H), 3.62-3.69 (m, 2H), 4.17 (t, *J* = 7.9 Hz, 2H), 7.19-7.33 (m, 5H), 8.74 (s, 1H), 9.77 (br s, 1H). |
| 3-039 | 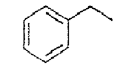 | 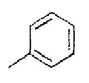 | 1.00 (t, *J* = 7.3 Hz, 3H), 1.41-1.53 (m, 2H), 1.68-1.78 (m, 2H), 4.15 (t, J= 7.6 Hz, 2H), 4.65 (d, *J* = 5.8 Hz, 2H), 7.22-7.45 (m, 10H), 8.46 (s, 1H), 10.25 (br s, 1H). |
| 3-040 | 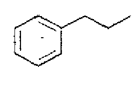 | 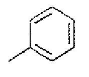 | 1.02 (t, *J* = 7.3 Hz, 3H), 1.43-1.55 (m, 2H), 1.69-1.79 (m, 2H), 2.41 (s, 3H), 2.94 (t, *J* = 7.9 Hz, 2H), 3.65-3.72 (m, 2H), 4.16 (t, *J* = 7.6 Hz, 2H), 7.19-7.45 (m, 10H), 8.43 (s, 1H), 9.98 (br s, 1H). |

**Table 77**

| | | | |
|---|---|---|---|
| 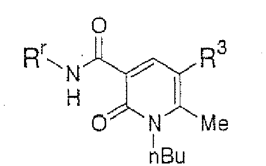 | | | |

| Compound No. | R^{r} | R³ | ¹H-NMR (CDCl₃) |
|---|---|---|---|
| 3-044 | 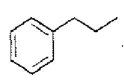 | CF₃ | 1.02 (t, *J* = 6.7 Hz, 3H), 1.42-1.54 (m, 2H), 1.66-1.74 (m, 2H), 2.61 (s, 3H), 2.93 (t, *J* = 7.3 Hz, 2H), 3.64-3.69 (m, 2H), 4.14 (t, *J* = 7.9 Hz, 2H), 7.20-7.33 (m, 5H), 8.69 (s, 1H), 9.61 (brs, 1H). |

**Table 78**

| | | | |
|---|---|---|---|
| 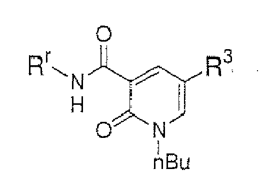 | | | |

| Compound No. | R^{r} | R³ | ¹H-NMR (CDCl₃) |
|---|---|---|---|
| 3-061 | n-Hexyl | 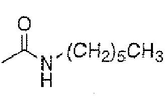 | 0.86-0.91 (m, 6H), 0.95 (t, *J* = 7.3 Hz, 3H), 1.26-1.47 (m, 16H), 1.54. 1.65 (m, 4H), 1.73-1.83 (m, 2H), 3.38-3.45 (m, 4H), 4.07 (t, *J* = 7.3 Hz, 2H), 6.72 (t, *J* = 5.5 Hz, 1H), 8.40 (d, *J* = 2.7 Hz, 1H), 8.83 (d, J= 2.7 Hz, 1H), 9.69 (t, *J* = 5.5 Hz, 1H). |
| 3-062 | 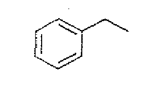 | 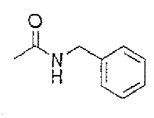 | 1.02 (t, *J* = 7.3 Hz, 3H), 1.33-1.45 (m, 2H), 1.72-1.82 (m, 2H), 4.06 (t, *J* = 7.6 Hz, 2H), 4.58 (d, *J* = 5.5 Hz, 4H), 6.81 (br s, 1H), 7.24-7.36 (m, 10H), 7.42 (d, *J* = 2.7 Hz, 1H), 8.78 (d, *J* = 2.7 Hz, 1H), 10.00 (br s, 1H). |
| 3-063 | 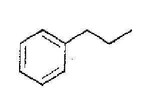 | 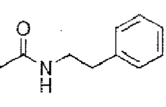 | 0.97 (t, *J* = 7.3 Hz, 3H), 1.33-1.46 (m, 2H), 1.72-1.82 (m, 2H), 2.88-2.94 (m, 4H), 3.63-3.72 (m, 4H), 4.06 (t, *J* = 7.6Hz, 2H), 7.20-7.34 (m, 10H), 8.37 (d, J= 2.7 Hz, 1H), 8.65 (d, *J* = 2.7 Hz, 1H), 9.52 (br s, 1H). |
| 3-064 | 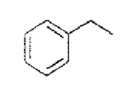 | 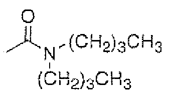 | 0.91-0.96 (m, 6H), 0.93 (t, *J* = 7.3 Hz, 3H), 1.32-1.44 (m, 4H), 1.54-1.65 (m, 6H), 1.71-1,81 (m, 2H), 3.38 (br s, 4H), 4.02 (t, *J* = 7.3 Hz, 2H), 4.64 (d, *J* = 5.8 Hz, 2H), 7.23-7.39 (m, 5H), 7.85 (d, *J* = 2.7 Hz, 1H), 8.58 (d, *J* = 2.7 Hz, 1H), 10.04 (t, *J* = 5.5 Hz, 1H). |
| 3-065 | 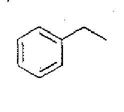 | 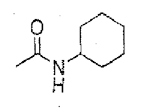 | 0.96 (t, *J* = 7.3 Hz, 3H), 1.15-1.49 (m, 6H), 1.64-1.81 (m, 6H), 1.96-2.05 (m, 2H), 3.87-3.99 (m, 1H), 4.05 (t, *J* = 7.3 Hz, 2H), 4.64 (d, *J* = 5.8 Hz, 2H), 6.10 (d, *J* = 7.9 Hz, 2H), 6.92-7.38 (m, 5H), 8.38 (d, *J* = 2.7 Hz, 1H), 8.72 (d, *J* = 2.7 Hz, 1H), 10.05 (t, *J* = 5.8 Hz, 1H). |
| 3-066 | 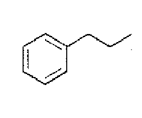 | 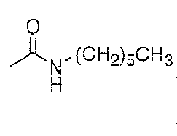 | 0.89 (t, *J* = 6.7 Hz, 3H), 0.97 (t, *J* = 7.3 Hz, 3H), 1.27-1.45 (m, 8H), 1.54-1.63 (m, 2H), 1.73-1.82 (m, 2H), 2.93 (t, *J* = 7.6 Hz, 2H), 3-38-3.45 (m, 2H), 3.65-3.72 (m, 2H), 4.06 (t, *J* = 7.6 Hz, 2H), 6.44 (t, *J* = 5.5 Hz, 1H), 7.20-7.34 (m, 5H), 8.39 (d, *J* = 2.7 Hz, 1H), 8.74 (d, *J* = 2.7 Hz, 1H), 9.78 (t, *J* = 5.5 Hz, 1H). |

**Table 79**

| | | | |
|---|---|---|---|
| 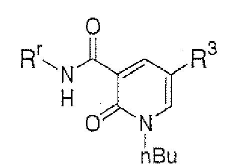 | | | |

| Compound No. | R^{r} | R³ | ¹H-NMR (CDCl₃) |
|---|---|---|---|
| 3-067 | 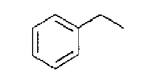 | I | 0.96 (t, *J* = 7.3 Hz, 3H), 1.31-1.44 (m, 2H), 1.68-1.78 (m, 2H), 3.95 (t, *J* = 7.3 Hz, 2H), 4.62 (d, *J* = 7.3 Hz, 2H), 7.23-7.36 (m, 5H), 7.70 (d, *J* = 2.6 Hz, 1H), 8.67 (d, *J* = 2.6 Hz, 1H), 10.03 (br s, 1H). |
| 3-068 | 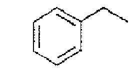 | 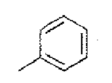 | 0.98 (t, *J* = 7.3 Hz, 3H), 1.36-1.48 (m, 2H), 1.75-1.85 (m, 2H), 4.08 (t, *J* = 7.6 Hz, 2H), 4.67 (d, *J* = 5.8 Hz, 2H), 7.22-7.50 (m, 10H), 7.69 (d, *J* = 2.7 Hz, 1H), 8.87 (d, *J* = 2.7 Hz, 1H), 10.25 (br s, 1H). |
| 3-069 | 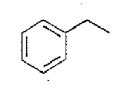 | 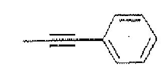 | 0.98 (t, *J* = 7.6 Hz, 3H), 1.34-1.46 (m, 2H), 1.72-1.82 (m, 2H), 4.01 (t, *J* = 7.6 Hz, 2H), 4.65 (d, *J* = 5.8 Hz, 2H), 7.23-7.40 (m, 8H), 7.45-7.51 (m, 2H), 7.73 (d, *J* = 2.7 Hz, 1H), 8.66 (d, *J* = 2.7 Hz, 1H), 10.03 (t, *J* = 5.8 Hz, 1H). |
| 3-070 | nBuO | H | 0.95 (t, *J* = 7.5 Hz, 3H), 1.38 (sextet, *J* = 7.8 Hz, 2H), 1.73-1.79 (m, 2H), 3.90 (s, 3H), 3.98 (t, *J* = 7.5 Hz, 2H), 6.24 (d, *J* = 6.9 Hz, 1H), 7.53 (dd, *J* = 6.7, 2.1 Hz, 1H), 8.14 (dd, *J* = 7.5, 2.4 Hz, 1H), |
| 3-071 | 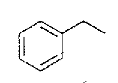 | H | 0.95 (t, *J* = 6.9 Hz, 3H), 1.36 (sextet, *J* = 7.8 Hz, 2H), 1.66-1.80 (m, 2H), 3.96 (t, *J* = 7.2 Hz, 2H), 4.60 (d, *J* = 6.0 Hz, 2H), 6.36 (t, *J* = 7.5 Hz, 1H), 7.20-7.40 (m, 5H), 7.46 (dd, *J* = 6.3, 2.1 Hz, 1H), 8.4 (dd, *J* = 7.2, 2.4 Hz, 1H). |
| 3-072 | 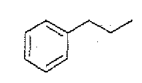 | CF₃ | 0.99 (t, *J* = 7.3 Hz, 3H), 1.34-1.47 (m, 2H), 1.72-1.82 (m, 2H), 2.93 (t, *J* = 7.3 Hz, 2H), 3.66-3.73 (m, 2H), 7.20-7.34 (m, 5H), 7.83 (m, 1H), 8.69 (d, *J* = 2.7 Hz, 1H), 9.62 (br s, 1H). |
| 3-073 | 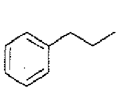 | 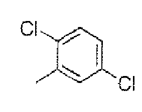 | 0.99 (t, *J* = 7.3 Hz, 3H), 1.37-1.49 (m, 2H), 2.95 (t, *J* = 7.3 Hz, 2H), 3.66-3.73 (m, 2H), 4.07 (t, *J* = 7.3 Hz, 2H), 7.19-7.31 (m, 6H), 7.34 (d, J = 2.4 Hz, 1H), 7.42 (d, *J* = 8.5 Hz, 1H), 7.65 (d, *J* = 2.7 Hz, 1H), 8.63 (dd, *J* = 2.7, 0.6 Hz, 1H), 9.89 (t, *J* = 5.8 Hz, 1H). |

**Table 80**

| | | | |
|---|---|---|---|
| 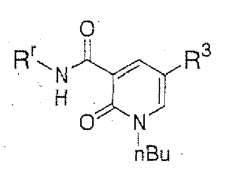 | | | |

| Compound No. | R^{r} | R³ | ¹H-NMR (CDCl₃) |
|---|---|---|---|
| 3-074 | 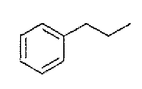 | 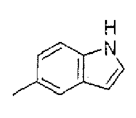 | 1.00 (t, *J* = 7.3 Hz, 3H), 1.38-1.50 (m, 2H), 1.70-1.87 (m, 2H), 2.97 (t, *J* = 7.3 Hz, 2H), 3.69-3.76 (m, 2H), 4.09 (t, *J* = 7.3 Hz, 2H), 6.58 (brs, 1H), 7.20-7.34 (m, 6H), 7.44-7.47 (m, 2H), 8.63 (s, 1H), 8.89 (d, *J* = 2.4 Hz, 1H), 10.11 (t, *J* = 5.8 Hz, 1H). |

**Table 81**

| | | | |
|---|---|---|---|
| 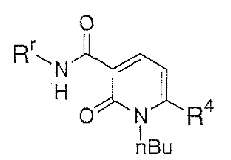 | | | |

| Compound No. | R^{r} | R⁴ | ¹H-NMR (CDCl₃) |
|---|---|---|---|
| 3.081 | 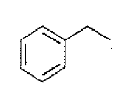 | Me | 0.98 (t, *J* = 7.2 Hz, 3H), 1.43 (sextet, *J* = 7.5 Hz, 2H), 1.60-1.70 (m, 2H), 2.46 (s, 3H), 4.05 (t, *J* = 8.1 Hz, 2H), 4.27 (dd, *J* = 7.2, 6.6 Hz, 1H), 4.64 (d, *J* = 5.7 Hz, 2H), 7.20-7.40 (m, 5H), 8.41 (d, *J* = 7.5 Hz, 1H), 10.2 (br s, 1H). |
| 3-082 | 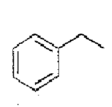 | nPentyl | 0.93 (t, J= 7.2 Hz, 3H), 0.98 (t, *J* = 7.2 Hz, 3H), 1.37-1.50 (m, 6H), 1.62-1.70 (m, 4H), 2.67 (t, *J* = 7.8 Hz, 2H), 4.05 (t, *J* = 7.8 Hz, 2H), 4.64 (d, *J* = 6.0Hz, 2H), 6.27 (d, *J* = 7.5 Hz, 1H), 7.20-7.40 (m, 5H), 8.44 (d, *J* = 7.5 Hz, 1H), 10.21 (br s, 1H). |
| 3-083 | 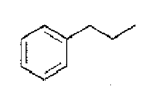 | nPentyl | 0.93 (t, *J* = 6.9 Hz, 3H), 1.00 (t, *J* = 7.2 Hz, 3H), 1.38-1.49 (m, 6H), 1.63-1.70 (m, 4H), 2.66 (t, *J* = 7.8 Hz, 2H), 2.93 (t, *J* = 7.5 Hz, 2H), 3.63-3.68 (m, 2H), 4.06 (t, *J* = 7.8 Hz, 2H), 6.27 (d, *J* = 7.5 Hz, 1H), 7.17-7.32 (m, 5H), 8.40 (d, *J* = 7.5 Hz, 1H), 9.94 (br s, 1H). |
| 3-084 | 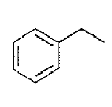 | nHexyl | 0.91 (t, *J* = 7.2 Hz, 3H), 0.98 (t, *J* = 7.2 Hz, 3H), 1.30-1.50 (m, 8H), 1.60-1.72 (m, 4H), 2.67 (t, *J* = 7.8 Hz, 2H), 4.05 (t, *J* = 8.1 Hz, 2H), 4.64 (d, *J* = 5.7 Hz, 2H), 6.28 (d, *J* = 7.8 Hz, 1H), 7.20-7.40 (m, 5H), 8.44 (d, *J* = 7.8 Hz, 1H),10.21 (br s, 1H). |
| 3-085 | 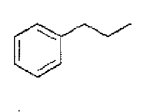 | nHexyl | 0.91 (t, *J* = 7.2 Hz, 3H), 1.00 (t, *J* = 7.2 Hz, 3H), 1.31-1.49 (m, 8H), 1.61-1.71 (m, 4H), 2.67 (t, *J* = 7.8 Hz, 2H), 2.93 (t, *J* = 7.2 Hz, 2H), 3.63-3.70 (m, 2H), 4.06 (t, *J* = 7.8 Hz, 2H), 6.27 (d, *J* = 7.8 Hz, 1H), 7.18-7.33 (m, 5H), 8.41 (d, *J* = 7.8 Hz, 1H), 9.94 (t, *J* = 5.1 Hz,1H). |

**Table 82**

| Compound No. | Structure | ¹H-NMR (CDCl₃) |
|---|---|---|
| 3-101 | 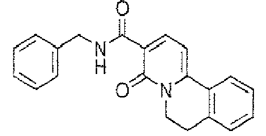 | 3.03 (t, *J* = 6.4 Hz, 2H), 4.35 (t, *J* = 6.4 Hz, 2H), 4.68 (d, *J* = 5.8 Hz, 2H), 6.94 (d, *J* = 7.9 Hz, 1H), 7.23-7.49 (m, 8H), 7.81 (d, *J* = 7.3 Hz, 1H), 8.63 (d, *J* =7.9 Hz, 1H), 10.22 (br s, 1H). |
| 3-102 | 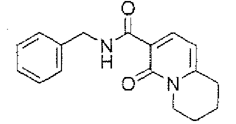 | 1.79-1.88 (m, 2H), 1.95-2.03 (m, 2H), 2.88 (t, *J* = 6.4 Hz, 2H), 4.04 (t, *J* = 6.1 Hz, 2H), 4.65 (d, *J* = 5.8 Hz, 2H), 6.26 (d, *J* = 7.3 Hz, 1H), 7.20-7.38 (m, 5H), 8.46 (d, *J* = 7.3 Hz, 1H), 10.19 (br s, 1H). |
| 3-103 | 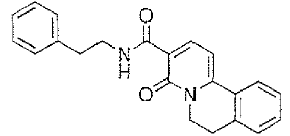 | 2.97 (t, *J* = 7.3 Hz, 2H), 3.04 (t, *J* = 6.4 Hz, 2H), 3.68-3.75 (m, 2H), 4.35 (t, *J* = 6.4 Hz, 2H), 6.92 (d, *J* = 7.9 Hz, 1H), 7.19-7.35 (m, 5H), 7.37-7.43 (m, 3H), 7.80 (dd, *J* = 1.5, 7.3 Hz, 1H), 8.59 (d, *J* = 7.9 Hz, 1H), 9.93 (br s, 1H). |
| 3-104 | 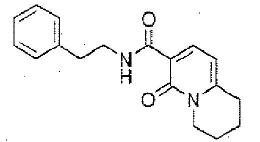 | 1.79-1.88 (m, 2H), 1.95-2.04 (m, 2H), 2.87 (t, *J* = 6.4 Hz, 2H), 2.93 (t; *J* = 7.3 Hz, 2H), 3.65-3.72 (m, 2H), 4.04 (t, *J* = 6.4 Hz, 2H), 6.24 (d, *J* =7.3 Hz, 1H), 7.18-7.33 (m, 5H), 8.42 (d, *J* = 7.3 Hz; 1H), 9.90 (br s, 1H). |
| 3-105 | 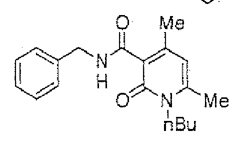 | 0.97 (t, *J* = 7.5 Hz, 3H), 1.42 (sextet, *J* = 7.5 Hz, 2H), 1.60-1.70 (m, 2H), 2.39 (s, 3H), 2.63 (s, 3H), 3.91 (t, *J* = 7.9 Hz, 2H), 4.60 (s, 2H), 6.05 (s, 1H), 7.20-7.40 (m, 5H). |
| 3-106 | 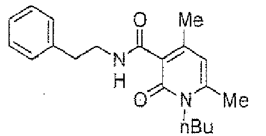 | 0.98 (t, *J* = 7.5 Hz, 3H), 1.43 (sextet, *J* = 7.5 Hz, 2H), 1.60-1.72 (m, 2H), 2.39 (s, 3H), 2.61 (s, 3H), 2.93 (t-like, 2H), 3.63 (t-like, 2H), 4.00 (t, *J* = 7.9 Hz, 2H), 6.04 (s, 1H), 7.17-7.33 (m, 5H). |
| 3-107 | 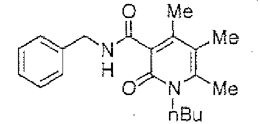 | 0.97 (t, *J* = 7.5 Hz, 3H), 1.42 (sextet, *J* = 7.5 Hz, 2H), 1.58-1.72 (m, 2H), 2.08 (s, 3H), 2.41 (s, 3H), 2.52 (s, 3H), 4.08 (t, *J* = 7.5 Hz, 2H), 4.62 (s, 2H), 7.20-7.42 (m, 5H), 9.02 (br s, 1H). |
| 3-108 | 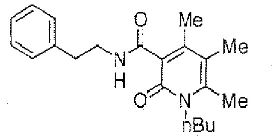 | 0.98 (t, *J* = 7.5 Hz, 3H), 1.43 (sextet, *J* = 7.5 Hz, 2H), 1.58-1.72 (m, 2H), 2.07 (s, 3H), 2.40 (s, 3H), 2.44 (s, 3H), 2.93 (t, *J* = 7.5 Hz, 2H), 3.67 (t, *J* = 7.5 Hz, 2H), 4.07 (t, *J* = 7.8 Hz, 2H), 7.16-7.34 (m, 5H), 8.47 (br s, 1H). |

**Table 83**

| Compound No. | Structure | ¹H-NMR (CDCl₃) |
|---|---|---|
| 3-109 | 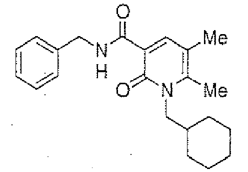 | 1.00-1.28 (m,. 4H), 1.56-1.90 (m, 7H), 2.18 (s, 3H), 2.39 (s, 3H), 4.00 (br s, 2H), 4.64 (d, *J* = 6.0 Hz, 2H), 7.20-7.40 (m, 5H), 8.35 (s, 1H), 10.3 (br s, 1H). |
| 3-110 | 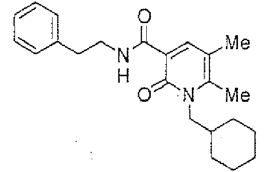 | 1.00-1.30 (m,. 4H), 1.58-1.90 (m, 7H), 2.93 (t, *J* = 7.5 Hz, 2H), 3.62-3.69 (m, 2H), 4.01 (br s, 2H), 7.18-7.35 (m, 5H), 8.32 (s, 1H), 10.3 (br s, 1H). |
| 3-111 | 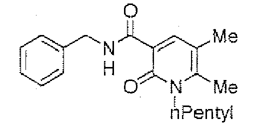 | 0.92 (t, *J* = 7.2 Hz, 3H), 1.37-1.42 (m, 4H), 1.60-1.75 (m, 2H), 2.18 (s, 3H), 2.40 (s, 3H), 4.08 (t, *J* = 8.1 Hz, 2H), 4.64 (d, *J* = 5.7 Hz, 2H), 7.20-7.40 (m, 5H), 8.35 (s, 1H), 10.3 (br s, 1H). |
| 3-112 | 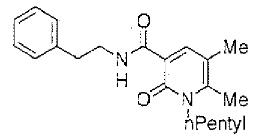 | 0.94 (t, *J* = 7.2 Hz, 3H), 1.38-1.42 (m, 4H), 1.60-1.75 (m, 2H), 2.18 (s, 3H), 2.40 (s, 3H), 2.93 (t, *J* = 7.8 Hz, 2H), 3.60-3.70 (m, 2H), 4.10 (t, *J* = 7.8 Hz, 2H), 7.20-7.35 (m, 5H), 8.31 (s, 1H), 10.03 (br s, 1H). |

**Table 84**

| | | | |
|---|---|---|---|
| 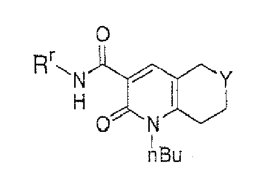 | | | |

| Compound No. | R^{r} | 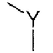 | ¹H-NMR (CDCl₃) |
|---|---|---|---|
| 4-001 | 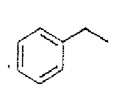 | -CH₂- | 0.97 (t, *J* = 7.5 Hz, 3H), 1.43 (sextet, *J* = 7.5 Hz, 2H), 1.62 (quint, *J* = 7.5 Hz, 2H), 1.74 (quint, *J* = 6.0 Hz, 2H), 1.88 (quint, *J* = 6.0 Hz, 2H), 2.62 (t, *J* = 6.0 Hz, 2H), 2.74 (t, *J* = 6.0 Hz, 2H), 4.03 (t, *J* = 7.8 Hz, 2H), 4.64 (d, *J* = 6.0 Hz, 2H), 7.23-7.38 (m, 5H), 8.28 (s, 1H), 10.32 (br t, *J* = 6.0 Hz, 1H). |
| 4-002 | 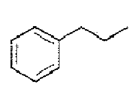 | -CH₂- | 0.99 (t, *J* = 7.5 Hz, 3H), 1.45 (sextet, *J* = 7.5 Hz, 2H), 1.63 (quint, *J* = 7.5 Hz, 2H), 1.74 (quint, *J* = 6.0 Hz, 2H), 1.88 (quint, *J* = 6.0 Hz, 2H), 2.62 (t, *J* = 6.0 Hz, 2H), 2.74 (t, *J* = 6.0 Hz, 2H), 2.93 (t, *J* = 7.8 Hz, 2H), 3.66 (dt, *J* = 9.0 Hz, 6.0 Hz, 2H), 4.03 (t, *J* = 7.8 Hz, 2H), 7.20-7.33 (m, 5H), 8.25 (s, 1H), 10.05 (br t, *J* = 6.0 Hz, 1H). |
| 4-003 | 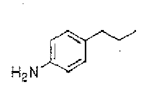 | -CH₂- | 0.99 (t, *J* = 7.5 Hz, 3H), 1.45 (sextet, *J* = 7.5 Hz, 2H), 1.66 (quint, *J* = 7.5 Hz, 2H), 1.73 (quint, *J* = 6.0 Hz, 2H), 1.87 (quint, *J* = 6.0 Hz, 2H), 2.61 (t, *J* = 6.0 Hz, 2H), 2.73 (t, *J* = 6.0 Hz, 2H), 2.82 (t, *J* = 7.8 Hz, 2H), 3.60 (dt, *J* = 9.0 Hz, 6.0 Hz, 2H), 4.03 (t, *J* = 7.5 Hz, 2H), 6.65 (dd, *J* = 6.3 Hz, 2.1 Hz, 2H), 7.05 (dd, *J* = 6.3 Hz, 2.1 Hz, 2H), 8.23 (s, 1H), 10.01 (br t, *J* = 6.0 Hz, 1H). |
| 4-004 | 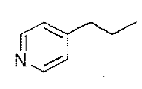 | -CH₂- | 0.99 (t, *J* = 7.2 Hz, 3H), 1.44 (sextet, *J* = 7.2 Hz, 2H), 1.65 (quint, *J* = 7.2 Hz, 2H), 1.74 (quint, *J* = 6.0 Hz, 2H), 1.88 (quint, *J* = 6.0 Hz, 2H), 2.64 (t, *J* = 6.0 Hz, 2H), 2.74 (t, *J* = 6.0 Hz, 2H), 2.94 (t, *J* = 7.5 Hz, 2H), 8.70 (q, *J* = 6.9 Hz, 2H), 4.03 (t, *J* = 7.8 Hz, 2H), 7.20 (d, *J* = 4.8 Hz, 2H), 8.22 (s, 1H), 8.51 (br s, 2H), 10.10 (br t, *J* = 6.0 Hz, 1H). |
| 4-005 | 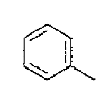 | -CH₂- | 1.01 (t, *J* = 7.5 Hz, 3H), 1.44 (sextet, *J* = 7.5 Hz, 2H), 1.70 (quint, *J* = 7.5 Hz, 2H), 1.76 (quint, *J* = 6.0 Hz, 2H), 1.91 (quint, *J* = 6.0 Hz, 2H), 2.66 (t, *J* = 6.0 Hz, 2H), 2.78 (t, *J* = 6.0 Hz, 2H), 4.09 (t, *J* = 7.8 Hz, 2H), 7.09 (t, *J* = 7.5 Hz, 1H), 7.34 (t, *J* = 7.5 Hz, 2H), 7.77 (d, *J* = 7.5 Hz, 2H), 8.34 (s, 1H), 12.18 (br s, 1H). |

**Table 85**

| | | | |
|---|---|---|---|
| 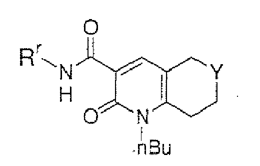 | | | |

| Compound No. | R^{r} | 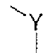 | ¹H-NMR (CDCl₃) |
|---|---|---|---|
| 4-006 | 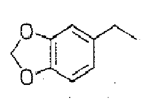 | -CH₂- | 0.98 (t, *J* = 7.5 Hz, 3H), 1.43 (sextet, *J* = 7.5 Hz, 2H), 1.65 (quint, *J* = 7.5 Hz, 2H), 1.74 (quint, *J* = 6.0 Hz, 2H), 1.88 (quint, *J* = 6.0 Hz, 2H), 2.62 (t, *J* = 6.0 Hz, 2H), 2.74 (t, *J* = 6.0 Hz, 2H), 4.02 (t, *J* = 7.8 Hz, 2H), 4.53 (d, *J* = 6.0 Hz, 2H), 5.02 (s, 2H), 6.74 (d, *J* = 7.8 Hz, 1H), 6.81 (dd, *J* = 7.8 Hz, 1.8 Hz, 1H), 6.86 (d, *J* = 1.8 Hz, 1H), 8.27 (s, 1H), 10.26 (br t, *J* = 6.0 Hz, 1H). |
| 4-007 | 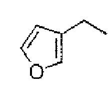 | -CH₂- | 0.98 (t, *J* = 7.5 Hz, 3H), 1.44 (sextet, *J* = 7.5 Hz, 2H), 1.63 (quint, *J* = 7.5 Hz, 2H), 1.73 (quint, *J* = 6.0 Hz, 2H), 1.88 (quint, *J* = 6.0 Hz, 2H), 2.62 (t, *J* = 6.0 Hz, 2H), 2.74 (t, *J* = 6.0 Hz, 2H), 4.03 (t, *J* = 7.8 Hz, 2H), 4.62 (d, *J* = 5.4 Hz, 2H), 6.25 (dd, *J* = 3.0 Hz, 0.9 Hz, 1H), 6.28-6.31 (m, 1H), 7.35 (d, *J* = 0.9 Hz, 1H), 8.26 (s, 1H), 10.25 (br t, *J* = 5.4 Hz, 1H). |

**Table 86**

| | | | |
|---|---|---|---|
| 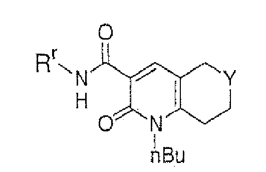 | | | |

| Compound No. | R^{r} | 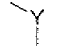 | ¹H-NMR (CDCl₃) |
|---|---|---|---|
| 4-008 | 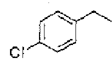 | -CH₂- | 0.98 (t, *J* = 7.5 Hz, 3H), 1.43 (sextet, *J* = 7.5 Hz, 2H), 1.62 (quint, *J* = 7.5 Hz, 2H), 1.74 (quint, *J* = 6.0 Hz, 2H), 1.88 (quint, *J* = 6.0 Hz, 2H), 2.62 (t, *J* = 6.0 Hz, 2H), 2.74 (t, *J* = 6.0 Hz, 2H), 4.03 (t, *J* = 7.8 Hz, 2H), 4.59 (d, *J* = 6.0 Hz, 2H), 7.26 (s, 2H), 7.28 (s, 2H), 8.26 (s, 1H), 10.35 (br t, *J* = 6.0 Hz, 1H). |
| 4-009 | 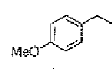 | -CH₂- | 0.97 (t, *J* = 7.5 Hz, 3H), 1.43 (sextet, *J* = 7.5 Hz, 2H), 1.64 (quint, *J* = 7.5 Hz, 2H), 1.74 (quint, *J* = 6.0 Hz, 2H), 1.88 (quint, *J* = 6.0 Hz, 2H), 2.62 (t, *J* = 6.0 Hz, 2H), 2.73 (t, *J* = 6.0 Hz, 2H), 3.78 (s, 3H), 4.01 (t, *J* = 7.8 Hz, 2H), 4.57 (d, *J* = 6.0 Hz, 2H), 6.85 (d, *J* = 9.0 Hz, 2H), 7.29 (d, *J* = 9.0 Hz, 2H), 8.27 (s, 1H), 10.24 (br t, *J* = 6.0 Hz, 1H). |
| 4-010 | 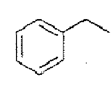 | -O- | 0.98 (t, *J* = 7.5 Hz, 3H), 1.43 (sextet, *J* = 7.5 Hz, 2H), 1.66 (quint, *J* = 7.5 Hz, 2H), 2.82 (t, *J* = 6.0 Hz, 2H), 4.01 (t, *J* = 6.0 Hz, 2H), 4.02 (t, *J* = 7.5 Hz, 2H), 4.60 (s, 2H), 4.64 (d, *J* = 6.0 Hz, 2H), 7.24-7.38 (m, 5H), 8.22 (s, 1H), 10.22 (br t, *J* = 6.0 Hz, 1H). |
| 4-011 | 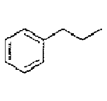 | -O- | 1.00 (t, *J* = 7.5 Hz, 3H), 1.45 (sextet, *J* = 7.5 Hz, 2H), 1.69 (quint, *J* = 7.5 Hz, 2H), 2.83 (t, *J* = 6.0 Hz, 2H), 2.93 (t, *J* = 7.5 Hz, 2H), 3.67 (dt, *J* = 9.0 Hz, 6.0 Hz, 2H), 4.01 (t, *J* = 6.0 Hz, 2H), 4.03 (t, *J* = 7.5 Hz, 2H), 4.60 (s, 2H), 7.18-7.36 (m, 5H), 8.19 (s, 1H), 9.96 (br t, *J* = 6.0 Hz, 1H). |
| 4-012 | 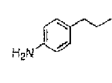 | -O- | 0.99 (t, *J* = 7.5 Hz, 3H), 1.45 (sextet, *J* = 7.5 Hz, 2H), 1.67 (quint, *J* = 7.5 Hz, 2H), 2.82 (t, *J* = 6.0 Hz, 2H), 2.83 (t, *J* = 7.5 Hz, 2H), 3.61 (dt, *J* = 9.0 Hz, 6.0 Hz, 2H), 4.01 (t, *J* = 6.0 Hz, 2H), 4.03 (t, *J* = 7.5 Hz, 2H), 4.59 (s, 2H), 6.71 (d, *J* = 7.5 Hz, 2H), 7.07 (d, *J* = 7.5 Hz, 2H), 8.17 (s, 1H), 9.92 (br t, *J* = 6.0 Hz, 1H). |

**Table 87**

| | | | |
|---|---|---|---|
| 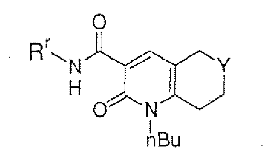 | | | |

| Compound No. | R^{r} | 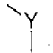 | ¹H-NMR (CDCl₃) |
|---|---|---|---|
| 4-013 | 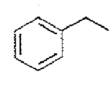 | 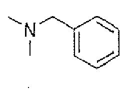 | 0.96 (t, *J* = 7.5 Hz, 3H), 1.41 (sextet, *J* = 7.5 Hz, 2H), 1.60-1.73 (m, 2H), 2.78 (d, *J* = 4.2 Hz, 2H), 2.84 (d, *J* = 4.2 Hz, 2H), 3.48 (s, 2H), 3.69 (s, 2H), 3.99 (t, *J* = 7.5 Hz, 2H), 4.63 (d, *J* = 6.0 Hz, 2H), 7.26-7.37 (m, 10H), 8.21 (s, 1H), 10.24 (br t, *J* = 6.0 Hz, 1H). |
| 4-014 | 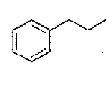 | 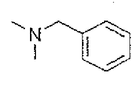 | 0.98 (t, *J* = 7.5 Hz, 3H), 1.43 (sextet, *J* = 7.5 Hz, 2H), 1.65 (quint, *J* = 7.5 Hz, 2H), 2.78 (d, *J* = 4.5 Hz, 2H), 2.85 (d, *J* = 4.5 Hz, 2H), 2.92 (t, *J* = 7.5 Hz, 2H), 3.48 (s, 2H), 3.66 (dt, *J* = 9.0 Hz, 6.0 Hz, 2H), 3.69 (s, 2H), 4.01 (t, *J* = 7.8 Hz, 2H), 7.23-7.38 (m, 10H), 8.18 (s, 1H), 9.99 (br t, *J* = 6.0 Hz, 1H). |

**Table 88**

| | | | |
|---|---|---|---|
| 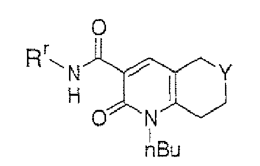 | | | |

| Compound No. | R^{r} | 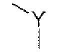 | ¹H-NMR (CDCl₃) |
|---|---|---|---|
| 4-015 | 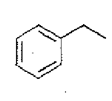 | 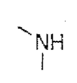 | 0.97 (t, *J* = 7.5 Hz, 3H), 1.43 (sextet, *J* = 7.5 Hz, 2H), 1.65 (quint, *J* = 7.5 Hz, 2H), 2.77 (t, *J* = 6.0 Hz, 2H), 3.19 (t, *J* = 6.0 Hz, 2H), 3.86 (s, 2H), 4.01 (t, *J* = 7.8 Hz, 2H), 4.64 (d, *J* = 6.0 Hz, 2H), 7.23-7.38 (m, 5H), 8.24 (s, 1H), 10.27 (br t, *J* = 6.0 Hz, 1H). |
| 4-016 | 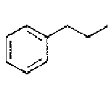 | 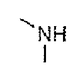 | 0.99 (t, *J* = 7.5 Hz, 3H), 1.45 (sextet, *J* = 7.5 Hz, 2H), 1.66 (quint, *J* = 7.5 Hz, 2H), 2.78 (t, *J* = 6.0 Hz, 2H), 2.93 (t, *J* = 7.5 Hz, 2H), 3.19 (t, *J* = 6.0 Hz, 2H), 3.67 (dt, *J* = 9.0 Hz, 6.0 Hz, 2H), 3.86 (s, 2H), 4.02 (t, *J* = 7.8 Hz, 2H), 7.18-7.34 (m, 5H), 8.21 (s, 1H), 10.01 (br t, *J* = 6.0 Hz, 1H). |
| 4-017 | 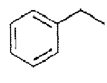 | 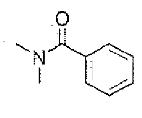 | 0.98 (t, *J* = 7.5 Hz, 3H), 1.44 (sextet, *J* = 7.5 Hz, 2H), 1.66 (quint, *J* = 7.5 Hz, 2H), 2.93 (br t, *J* = 6.0 Hz, 2H), 3.80 (br t, *J* = 6.0 Hz, 2H), 4.02 (t, *J* = 7.8 Hz, 2H), 4.49 (s, 2H), 4.62 (d, *J* = 6.0 Hz, 2H), 7.23-7.35 (m, 5H), 7.43-7.51 (m, 5H), 8.10 (s, 1H), 10.16 (br t, *J* = 6.0 Hz, 1H). |
| 4-018 | 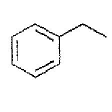 | 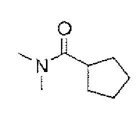 | 0.98 (t, *J* = 7.5 Hz, 3H), 1.43 (sextet, *J* = 7.5 Hz, 2H), 1.55-1.90 (m, 10H), 2.84 (quint, *J* = 6.0 Hz, 1H), 2.91 (t, *J* = 7.5 Hz, 2H), 3.82 (t, *J* = 6.0 Hz, 1/3 × 2H), 3.91 (t, *J* = 6.0 Hz, 2/3 × 2H), 4.01 (t, *J* = 7.8 Hz, 2H), 4.52 (s, 2/3 × 2H), 4.59 (s, 1/3 × 2H), 4.65 (d, *J* = 6.0 Hz, 2H), 7.24-7.39 (m, 5H), 8.31 (s, 2/3 × 1H), 8.33 (s, 1/3 × 1H), 10.20 (br t, *J* = 6.0 Hz, 1H). |

**Table 89**

| | | | |
|---|---|---|---|
| 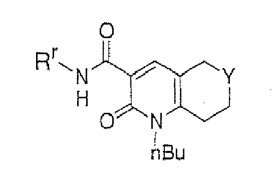 | | | |

| Compound No. | R^{r} | 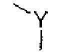 | ¹H-NMR (CDCl₃) |
|---|---|---|---|
| 4-019 | 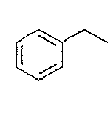 | 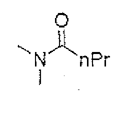 | 0.98 (t, *J* = 7.5 Hz, 3H), 0.99 (t, *J* = 7.5 Hz, 3H), 1.43 (sextet, *J* = 7.5 Hz, 2H), 1.66 (quint, *J* = 7.5 Hz, 2H), 1.67 (quint, *J* = 7.5 Hz, 2H), 2.37 (t, *J* = 7.5 Hz, 2H), 2.84 (t, *J* = 6.0 Hz, 2/3 × 2H), 2.89 (t, *J* = 6.0 Hz, 1/3 × 2H), 3.77 (t, *J* = 6.0 Hz, 1/3 × 2H), 3.90 (t, *J* = 6.0 Hz, 2/3 × 2H), 4.01 (t, *J* = 7.8 Hz, 2H), 4.47 (s, 2/3 × 2H), 4.58 (s, 1/3 × 2H), 4.65 (d, *J* = 6.0 Hz, 2H), 7.24-7.39 (m, 5H), 8.30 (s, 2/3 × 1H), 8.33 (s, 1/3 × 1H), 10.19 (br t, *J* = 6.0 Hz, 1H). |
| 4-020 | 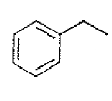 | 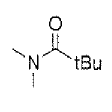 | 0.98 (t, *J* = 7.5 Hz, 3H), 1.29 (s, 9H), 1.43 (sextet, *J* = 7.5 Hz, 2H), 1.65 (quint, *J* = 7.5 Hz, 2H), 2.85 (t, *J* = 6.0 Hz, 2H), 3.90 (t, *J* = 6.0 Hz, 2H), 4.00 (t, *J* = 7.8 Hz, 2H), 4.62 (s, 2H), 4.64 (d, *J* = 6.0 Hz, 2H), 7.24-7.38 (m, 5H), 8.31 (s, 1H), 10.20 (br t, *J* = 6.0 Hz, 1H). |

**Table 90**

| | | | |
|---|---|---|---|
| 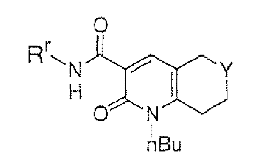 | | | |

| Compound No. | R^{r} | 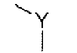 | ¹H-NMR (CDCl₃) |
|---|---|---|---|
| 4-021 | 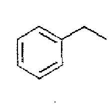 | 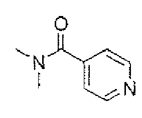 | 0.88 (t, *J* = 7.5 Hz, 1/3 × 3H), 0.99 (t, *J* = 7.5 Hz, 2/3 × 3H), 1.44 (sextet, *J* = 7.5 Hz, 2H), 1.66 (quint, *J* = 7.5 Hz, 2H), 2.86 (t, *J* = 6.0 Hz, 1/3 × 2H), 2.99 (t, *J* = 6.0 Hz, 2/3 × 2H), 3.69 (t, *J* = 6.0 Hz, 1/3 × 2H), 4.02 (t, *J* = 6.0 Hz, 2/3 × 2H), 4.06 (t, *J* = 7.8 Hz, 2H), 4.40 (s, 1/3 × 2H), 4.62 (s, 2/3 × 2H), 4.63 (d, *J* = 6.0 Hz, 2H), 7.24-7.38 (m, 7H), 8.11 (s, 2/3 × 1H), 8.39 (s, 1/3 × 1H), 8.76 (d, *J* = 5.4 Hz, 2H), 10.12 (br t, *J* = 6.0 Hz, 1H). |
| 4-022 | 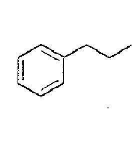 | 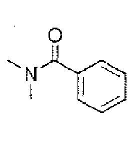 | 1.00 (t, *J* = 7.5 Hz, 3H), 1.46 (sextet, *J* = 7.5 Hz, 2H), 1.67 (quint, *J* = 7.5 Hz, 2H), 2.91 (t, *J* = 7.5 Hz, 2H), 2.92 (t, *J* = 6.0 Hz, 2H), 3.66 (dt, *J* = 6.3 Hz, 6.9 Hz, 2H), 4.03 (t, *J* = 6.0 Hz, 2H), 4.04 (t, *J* = 7.5 Hz, 2H), 4.48 (br s, 2/3 × 2H), 4.68 (br s, 1/3 × 2H), 7.20-7.32 (m, 5H), 7.44-7.51 (m, 5H), 8.08 (br s, 2/3 × 1H), 8.37 (br s, 1/3 × 1H), 9.89 (br t, *J* = 6.0 Hz, 1H). |
| 4-023 | 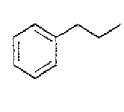 | 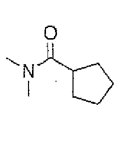 | 0.99 (t, *J* = 7.5 Hz, 3H), 1.44 (sextet, *J* = 7.5 Hz, 2H), 7.60-1.88 (m, 10H), 2.83 (t, *J* = 6.0 Hz, 2H). 2.89 (quint, *J* = 6.0 Hz, 1H), 2.93 (t, *J* = 7.5 Hz, 2H), 3.68 (dt, *J* = 6.6 Hz, 7.2 Hz, 2H), 3.82 (t, *J* = 6.0 Hz, 1/3 × 2H), 3.91 (t, *J* = 6.0 Hz, 2/3 × 2H), 4.02 (t, *J* = 7.8 Hz, 2H), 4. 52 (s, 2/3 × 2H), 4.58 (s, 1/3 × 2H), 7.18-7.34 (m, 5H), 8.27 (s, 2/3 × 1H), 8.30 (s, 1/3 × 1H), 9.93 (br t, *J* = 6.0 Hz, 1H). |

**Table 91**

| | | | |
|---|---|---|---|
| 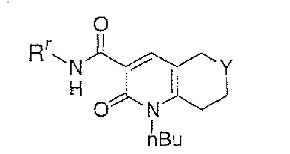 | | | |

| Compound No. | R^{r} | 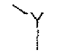 | ¹H-NMR (CDCl₃) |
|---|---|---|---|
| 4-024 | 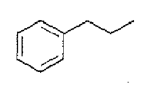 | 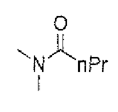 | 0.98 (t, *J* = 7.5 Hz, 3H), 0.99 (t, *J* = 7.5 Hz, 3H), 1.45 (sextet, *J* = 7.5 Hz, 2H), 1.66 (quint, *J* = 7.5 Hz, 2H), 1.67 (quint, *J* = 7.5 Hz, 2H), 2.37 (t, *J* = 7.5 Hz, 2H), 2.84 (t, *J* = 6.0 Hz, 2H), 2.93 (t, *J* = 7.5 Hz, 2H), 3.68 (q, *J* = 6.9 Hz, 2H), 3.77 (t, *J* = 6.0 Hz, 1/3 × 2H), 3.90 (t, *J* = 6.0 Hz, 2/3 × 2H), 4.03 (t, *J* = 7.8 Hz, 2H), 4.47 (s, 2/3 × 2H), 4.58 (s, 1/3 × 2H), 7.20-7.33 (m, 5H), 8.27 (s, 2/3 × 1H), 8.30 (s, 1/3 × 1H), 9.81 (br t, *J* = 6.0 Hz, 1/3 × 1H), 9.93 (br t, *J* = 6.0 Hz, 2/3 × 1H). |
| 4-025 | 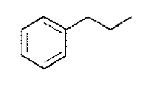 | 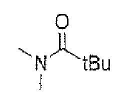 | 1.00 (t, *J* = 7.5 Hz, 3H), 1.30 (s, 9H), 1.45 (sextet, *J* = 7.5 Hz, 2H), 1.66 (quint, *J* = 7.5 Hz, 2H), 2.85 (t, *J* = 6.0 Hz, 2H), 2.93 (t, *J* = 7.5 Hz, 2H), 3.68 (dt, *J* = 9.0 Hz, 6.0 Hz, 2H), 3.90 (t, *J* = 6.0 Hz, 2H), 4.01 (t, *J* = 7.8 Hz, 2H), 4.62 (s, 2H), 7.18-7.33 (m, 5H), 8.28 (s, 1H), 9.94 (br t, *J* = 6.0 Hz, 1H). |
| 4-026 | 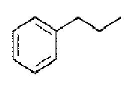 | 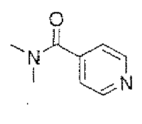 | 0.88 (t, *J* = 7.5 Hz, 1/3 × 3H), 1.00 (t, *J* = 7.5 Hz, 2/3 × 3H), 1.46 (sextet, *J* = 7.5 Hz, 2H), 1.65 (quint, *J* = 7.5 Hz, 2H), 2.82-3.01 (m, 4H), 3.66 (dt, *J* = 9.0 Hz, 6.0 Hz, 2H), 4.04 (t, *J* = 6.0 Hz, 2H), 4.07 (t, *J* = 7.8 Hz, 2H), 4.39 (br s, 2/3 × 2H), 4.73 (br s, 1/3 × 2H), 7.20-7.37 (m, 7H), 8.07 (s, 2/3 × 1H), 8.35 (s, 1/3 × 1H), 8.76 (d, *J* = 4.8 Hz, 2H), 9.85 (br t, *J* = 6.0 Hz, 1H). |

**Table 92**

| | | | |
|---|---|---|---|
| 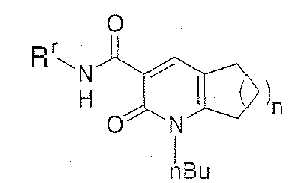 | | | |

| Compound No. | R^{r} | n | ¹H-NMR (CDCl₃) |
|---|---|---|---|
| 4-051 | 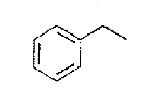 | 1 | 0.97 (t, *J* = 7.5 Hz, 3H), 1.41 (sextet, *J* = 7.5 Hz, 2H), 1.69 (quint, *J* = 7.5 Hz, 2H), 2.19 (quint, *J* = 7.5 Hz, 2H), 2.85 (t, *J* = 7.5 Hz, 2H), 3.00 (t, *J* = 7.5 Hz, 2H), 3.98 (t, *J* = 7.8 Hz, 2H), 4.64 (d, *J* = 6.0 Hz, 2H), 7.23-7.39 (m, 5H), 8.46 (s, 1H), 10.31 (br t, *J* = 6.0 Hz, 1H). |
| 4-052 | 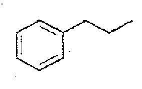 | 1 | 0.98 (t, *J* = 7.5 Hz, 3H), 1.41 (sextet, *J* = 7.5 Hz, 2H), 1.70 (quint, *J* = 7.5 Hz, 2H), 2.19 (quint, *J* = 7.5 Hz, 2H), 2.85 (t, *J* = 7.5 Hz, 2H), 2.93 (t, *J* = 7.5 Hz, 2H), 3.00 (t, *J* = 7.5 Hz, 2H), 3.67 (dt, *J* = 9.0 Hz, 6.0 Hz, 2H), 3.99 (t, *J* = 7.8 Hz, 2H), 7.18-7.34 (m, 5H), 8.43 (s, 1H), 10.05 (br t, *J* = 6.0 Hz, 1H). |
| 4-053 | 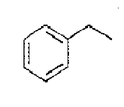 | 1 | 0.98 (t, *J* = 7.5 Hz, 3H), 1.44 (sextet, *J* = 7.5 Hz, 2H), 1.57-1.65 (m, 4H), 1.68 (quint, *J* = 6.0 Hz, 2H), 1.86 (quint, *J* = 6.0 Hz, 2H), 2.71 (t, *J* = 6.0 3 Hz, 2H), 2.94 (t, *J* = 6.0 Hz, 2H), 4.15 (br t, *J* = 7.8 Hz, 2H), 4.64 (d, *J* = 6.0 Hz, 2H), 7.22-7.38 (m, 5H), 8.33 (s, 1H), 10.31 (br t, *J* = 6.0 Hz, 1H). |
| 4-054 | 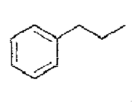 | 3 | 0.99 (t, *J* = 7.5 Hz, 3H), 1.46 (sextet, *J* = 7.5 Hz, 2H), 1.60-1.67 (m, 4H), 1.69 (quint, *J* = 6.0 Hz, 2H), 1.86 (quint, *J* = 6.0 Hz, 2H), 2.71 (t, *J* = 6.0 3 Hz, 2H), 2.71 (t, *J* = 7.5 Hz, 2H), 2.72 (t, *J* = 6.0 Hz, 2H), 3.66 (dt, *J* = 9.0 Hz, 6.0 Hz, 2H), 4.17 (br t, *J* = 7.8 Hz, 2H), 7.19-7.34 (m, 5H), 8.29 (s, 1H), 10.05 (br t, *J* = 6.0 Hz, 1H). |
| 4-055 | 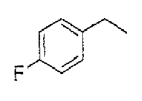 | 3 | 0.98 (t, *J* = 7.5 Hz, 3H), 1.44 (sextet, *J* = 7.5 Hz, 2H), 1.58-1.65 (m, 4H), 1.69 (quint, *J* = 6.0 Hz, 2H), 1.86 (quint, *J* = 6.0 Hz, 2H), 2.71 (t, *J* = 6.0 Hz, 2H), 2.94 (t, *J* = 6.0 Hz, 2H), 4.15 (br t, *J* = 7.8 Hz, 2H), 4.59 (d, *J* = 6.0 Hz, 2H), 6.99 (t, *J* = 9.0 Hz, 2H), 7.32 (dd, *J* = 9.0 Hz, 6.0 Hz, 2H), 8.32 (s, 1H), 10.32 (br t, *J* = 6.0 Hz, 1H). |
| 4-056 | 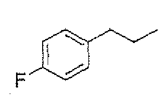 | 3 | 0.99 (t, *J* = 7.5 Hz, 3H), 1.45 (sextet, *J* = 7.5 Hz, 2H), 1.57-1.66 (m, 4H), 1.69 (quint, *J* = 6.0 Hz, 2H), 1.86 (quint, *J* = 6.0 Hz, 2H), 2.71 (t, *J* = 6.0 Hz, 2H), 2.89 (t, *J* = 7.5 Hz, 2H), 2.94 (t, *J* = 6.0 Hz, 2H), 3.63 (dt, *J* = 9.0 Hz, 6.0 Hz, 2H), 4.16 (br t, *J* = 7.8 Hz, 2H), 6.97 (t, *J* = 9.0 Hz, 2H), 7.20 (dd, *J* = 9.0 Hz, 6.0 Hz, 2H), 8.29 (s, 1H), 10.04 (br t, *J* = 6.0 Hz, 1H). |

**Table 93**

| | | | |
|---|---|---|---|
| 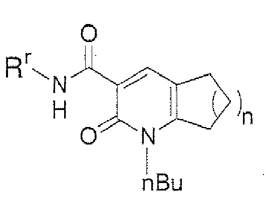 | | | |

| Compound No. | R^{r} | n | ¹H-NMR (CDCl₃) |
|---|---|---|---|
| 4-057 | 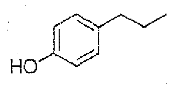 | 3 | 0.99 (t, *J* = 7.5 Hz, 3H), 1.45 (sextet, *J* = 7.5 Hz, 2H), 1.57-1.68 (m, 4H), 1.69 (quint, *J* = 6.0 Hz, 2H), 1.86 (quint, *J* = 6.0 Hz, 2H), 2.70 (t, *J* =6.0 Hz, 2H), 2.84 (t, *J* = 7.5 Hz, 2H), 2.94 (t, *J* = 6.0 Hz, 2H), 3.63 (dt, *J* = 9.0 Hz, 6.0 Hz, 2H), 4.16 (br t, *J* = 7.8 Hz, 2H), 6.22 (br s, 1H), 6.76 (d, *J* = 8.4 Hz, 2H), 7.06 (d, *J* = 8.4 Hz, 2H), 8.29 (s, 1H), 10.10 (br t, *J* = 6.0 Hz, 1H). |

**Table 94**

| | | | |
|---|---|---|---|
| 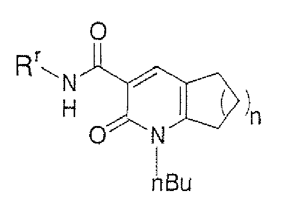 | | | |

| Compound No. | R^{r} | n | ¹H-NMR (CDCl₃) |
|---|---|---|---|
| 4-058 | 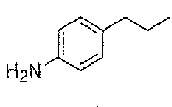 | 3 | 0.99 (t, *J* = 7.5 Hz, 3H), 1.45 (sextet, *J* = 7.5 Hz, 2H), 1.57-1.68 (m, 4H), 1.69 (quint, *J* = 6.0 Hz, 2H), 1.86 (quint, *J* = 6.0 Hz, 2H), 2.70 (t, *J* = 6.0 Hz, 2H), 2.81 (t, *J* = 7.5 Hz, 2H), 2.93 (t, *J* = 6.0 Hz, 2H), 3.60 (dt, *J* = 9.0 Hz, 6.0 Hz, 2H), 4.16 (br t, *J* = 7.8 Hz, 2H), 6.40 (d, *J* = 8.4 Hz, 2H), 7.05 (d, *J* = 8.4 Hz, 2H), 8.29 (s, 1H), 10,00 (br t, *J* = 6.0 Hz, 1H). |
| 4-059 | 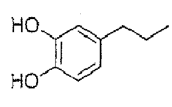 | 3 | 0.98 (t, *J* = 7.5 Hz, 3H), 1.44 (sextet, *J* = 7.5 Hz, 2H), 1.56-1.68 (m, 4H), 1.69 (quint, *J* = 6.0 Hz, 2H), 1.87 (quint, *J* = 6.0 Hz, 2H), 2.71 (t, *J* = 6.0 Hz, 2H), 2.94 (t, *J* = 6.0 Hz, 2H), 4.16 (br t, *J* = 7.8 Hz, 2H), 4.48 (d, *J* = 6.0 Hz, 2H), 6.05 (br s, 1H), 6.53 (br s, 1H), 6.74 (s, 2H), 6.87 (s, 1H), 8.30 (s, 1H), 10.35 (br t, *J* = 6.0 Hz, 1H). |
| 4-060 | 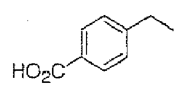 | 3 | 0.99 (t, *J* = 7.5 Hz, 3H), 1.45 (sextet, *J* = 7.5 Hz, 2H), 1.56-1.69 (m, 4H), 1.70 (quint, *J* = 6.0 Hz, 2H), 1.87 (quint, *J* = 6.0 Hz, 2H), 2.72 (t, *J* = 6.0 Hz, 2H), 2.95 (t, *J* = 6.0 Hz, 2H), 4.18 (br t, *J* = 7.8 Hz, 2H), 4.70 (d, *J* = 6.0 Hz, 2H), 7.43 (d, *J* = 8.1 Hz, 2H), 8.00 (d, *J* = 8.1 Hz, 2H), 8.33 (s, 1H), 10.44 (br t, *J* = 6.0 Hz, 1H). |
| 4-061 | 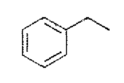 | 6 | 0.97 (t, *J* = 7.5 Hz, 3H), 1.26-1.34 (m, 4H), 1.42 (sextet, *J* = 7.5 Hz, 2H), 1.46-1.60 (m, 4H), 1.65 (quint, *J*= 7.5 Hz, 2H), 1.80 (quint, *J* = 6.0 Hz, 2H), 1.87 (quint, *J* = 6.0 Hz, 2H), 2.73 (t, *J* = 6.0 Hz, 2H), 2.93 (br t, *J* = 6.0 Hz, 2H), 4.12 (br t, *J* = 7.8 Hz, 2H), 4.64 (d, *J* = 6.0 Hz, 2H), 7.23-7.41 (m, 5H), 8.38 (s, 1H), 10.36 (br t, *J* = 6.0 Hz, 1H |
| 4-062 | 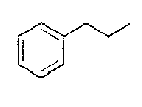 | 6 | 0.98 (t, *J* = 7.5 Hz, 3H), 1.24-1.33 (m, 4H), 1.46 (sextet, *J* = 7.5 Hz, 2H), 1.47-1.58 (m, 4H), 1.66 (quint, *J* = 7.5 Hz, 2H), 1.80 (quint, *J* = 6.0 Hz, 2H), 1.86 (quint, *J* = 6.0 Hz, 2H), 2.73 (t, *J* = 6.0 Hz, 2H), 2.94 (t, *J* = 7.5 Hz, 2H), 2.95 (t, *J* = 6.0 Hz, 2H), 3.67 (dt, *J* = 9.0 Hz, 6.0 Hz, 2H), 4.14 (br t, *J* = 7.8 Hz, 2H), 7.21-7.34 (m, 5H), 8.35 (s, 1H), 10.10 (br t, *J* = 6.0 Hz, 1H). |

**Table 95**

| | | |
|---|---|---|
| 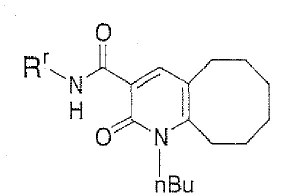 | | |

| Compound No. | R^{r} | ¹H-NMR (CDCl₃) |
|---|---|---|
| 4-101 | 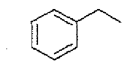 | 0.97 (t, *J* = 7.5 Hz, 3H), 1.35-1.53 (m, 4H), 1.44 (sextet, *J* = 7.5 Hz, 2H), 1.60-1.78 (m, 6H), 2.64 (t, *J* = 6.0 Hz, 2H), 2.88 (t, *J* = 6.0 Hz, 2H), 4.09 (br t, *J* = 7.8 Hz, 2H), 4.64 (d, *J* = 6.0 Hz, 2H), 7.17-7.39 (m, 5H), 8.34 (s, 1H), 10.34 (br t, *J* = 6.0 Hz, 1H). |
| 4-102 | 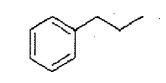 | 0.99 (t, *J* = 7.5 Hz, 3H), 1.34-1.53 (m, 4H), 1.46 (sextet, *J* = 7.5 Hz, 2H), 1.62-1.80 (m, 6H), 2.64 (t, *J* = 6.0 Hz, 2H), 2.89 (t, *J* = 6.0 Hz, 2H), 2.94 (t, *J* = 7.5 Hz, 2H), 3.67 (dt, *J* = 9.0 Hz, 6.0 Hz, 2H), 4.10 (br t, *J* = 7.8 Hz, 2H), 7.18-7.34 (m, 5H), 8.31 (s, 1H), 10.07 (br t, *J* = 6.0 Hz, 1H). |
| 4-103 | 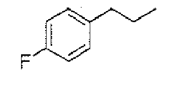 | 0.99 (t, *J* = 7.5 Hz, 3H), 1.36-1.58 (m, 4H), 1.46 (sextet, *J* = 7.5 Hz, 2H), 1.59-1.74 (m, 4H), 1.76 (quint, *J* = 6.0 Hz, 2H), 2.64 (t, *J* = 6.0 Hz, 2H), 2.89 (t, *J* = 6.0 Hz, 2H), 2.90 (t, *J* = 7.5 Hz, 2H), 3.64 (dt, *J* = 9.0 Hz, 6.0 Hz, 2H), 4.09 (br t, *J* = 7.8 Hz, 2H), 6.98 (t, *J* = 8.4 Hz, 2H), 7.21 (dt. *J* = 9.0 Hz, 6.0 Hz, 2H), 8.30 (s, 1H), 10.06 (br t, *J* = 6.0 Hz, 1H). |
| 4-104 | 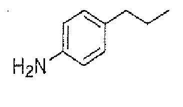 | 0.99 (t, *J* = 7.5 Hz, 3H), 1.37-1.52 (m, 4H), 1.46 (sextet, *J* = 7.5 Hz, 2H), 1.61-1.73 (m, 4H), 1.76 (quint, *J* = 6.0 Hz, 2H), 2.64 (t, *J* = 6.0 Hz, 2H), 2.82 (t, *J* = 7.5 Hz, 2H), 2.88 (t, *J* = 6.0 Hz, 2H), 3.61 (dt, *J* = 9.0 Hz, 6.0 Hz, 2H), 4.09 (br t, *J* = 7.8 Hz, 2H), 6.64 (d, *J* = 8.4 Hz, 2H), 7.05 (d, *J* = 8.4 Hz, 2H), 8.30 (s, 1H), 10.02 (br t, *J* = 6.0 Hz, 1H). |
| 4-105 | 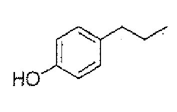 | 0.99 (t, *J* = 7.5 Hz, 3H), 1.36-1.52 (m, 4H), 1.45 (sextet, *J* = 7.5 Hz, 2H), 1.60-1.72 (m, 4H), 1.76 (quint, *J* = 6.0 Hz, 2H), 2.63 (t, *J* = 6.0 Hz, 2H), 2.85 (t, *J* = 7.5 Hz, 2H), 2.88 (t, *J* = 6.0 Hz, 2H), 3.63 (dt, *J* = 9.0 Hz, 6.0 Hz, 2H), 4.10 (br t, *J* = 7.8 Hz, 2H), 6.76 (d, *J* = 8.4 Hz, 2H), 7.08 (d, *J* = 8.4 Hz, 2H), 8.31 (s, 1H), 10.10 (br t, *J* = 6.0 Hz, 1H). |

**Table 96**

| | | | |
|---|---|---|---|
| 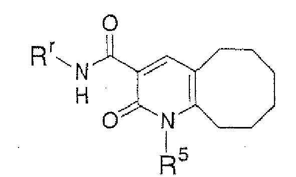 | | | |

| Compound No. | R^{r} | R⁵ | ¹H-NMR (CDCl₃) |
|---|---|---|---|
| 4-301 | 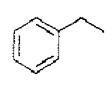 | 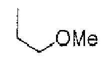 | 1.36 (quint, *J* = 6.0 Hz, 2H), 1.49 (quint, *J* = 6.0 Hz, 2H), 1.61-1.68 (m, 2H), 1.69 (quint, *J* = 6.0 Hz, 2H), 2.66 (t, *J* = 6.0 Hz, 2H), 3.03 (t, *J* = 6.0 Hz, 2H), 3.30 (s, 3H), 3.67 (t, *J* = 5.4 Hz, 2H), 4.32 (t, *J* = 5.4 Hz, 2H), 4.64 (d, *J* = 6.0 Hz, 2H), 7.26-7.40 (m, 5H), 8.36 (s, 1H), 10.25 (br t, *J* = 6.0 Hz, 1H). |
| 4-302 | 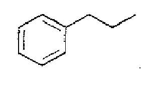 | 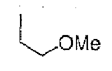 | 1.38 (quint, *J* = 4.8 Hz, 2H), 1.49 (quint, *J* = 4.8 Hz, 2H), 1.60-1.67 (m, 2H), 1.70 (quint, *J* = 6.0 Hz, 2H), 2.66 (t, *J* = 6.0 Hz, 2H), 2.94 (t, *J* = 7.5 Hz, 2H), 3.03 (t, *J* = 6.0 Hz, 2H), 3.31 (s, 3H), 3.67 (dt, *J* = 9.0 Hz, 6.0 Hz, 2H), 3.68 (t, *J* = 5.4 Hz, 2H), 4.33 (t, *J* = 5.4 Hz, 2H), 7.24-7.34 (m, 5H), 8.33 (s, 1H), 9.98 (br t, *J* = 6.0 Hz, 1H). |
| 4-303 | 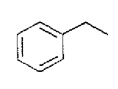 | 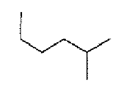 | 0.99 (d, *J* = 6.7 Hz, 6H), 1.32-1.82 (m, 11H), 2.64 (t, *J* = 6.3 Hz, 2H), 2.87 (t, *J* = 6.3 Hz, 2H), 3.98-4.20 (br s, 2H), 4.64 (d, *J* = 5.8 Hz, 2H), 7.23-7.40 (m, 5H), 8.34 (s, 1H), 10.3 (t-like). |
| 4-304 | 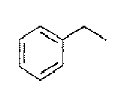 | 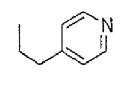 | (CD₃OD): 1.24-1.57 (m, 2H), 1.64-1.85 (m, 2H), 2.70 (t-like, 2H), 2.94 (t-like, 2H), 3.06 (t, *J* = 7.5 Hz, 2H), 4.41 (t, *J* = 7.5 Hz, 2H), 4.61 (s, 2H), 7.22-7.40 (m, 7H), 8.44 (A₂B₂, *J* = 5.2 Hz), 8.26 (d, *J* = 0.9 Hz, 1H). |
| 4-305 | 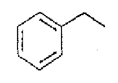 | 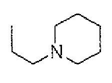 | 1.32-1.82 (m, 14H), 2.38-2.53 (m, 4H), 2.57 (t, *J* = 7.5 Hz, 2H), 2.64 (t, *J* = 6.0 Hz, 2H), 2.94 (t, *J* = 6.4 Hz, 2H), 4.26 (t-like, 1H), 4.64 (d, *J* = 5.8 Hz, 2H), 7.22-7.39 (m, 5H), 8.34 (s, 1H), 10.29 (d, *J* = 5.8 Hz, 2H). |
| 4-306 | 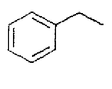 | 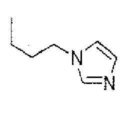 | 1.32-1.50 (m, 4H), 1.52-1.72 (m, 4H), 2.17 (quint, *J* = 6.7 Hz, 2H), 2.52-2.70 (m, 4H), 3.98-4.10 (m, 2H), 4.10 (t, *J* = 6.7 Hz, 2H), 4.65 (d, *J* = 5.8 Hz, 2H), 6.98 (s, 1H), 7.10 (s, 1H), 7.22-7.40 (m, 5H), 7.54 (s, 1H), 8.35 (s, 1H), 10.19 (t, *J* = 5.8 Hz, 1H). |

**Table 97**

| | | | |
|---|---|---|---|
| 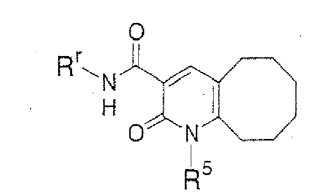 | | | |

| Compound No. | R^{r} | R⁵ | ¹H-NMR (CDCl₃) |
|---|---|---|---|
| 4-307 | 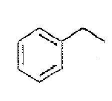 | 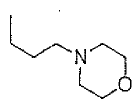 | 1.34-1.54 (m ,4H), 1.60-1.81 (m, 4H), 1.82-1.94 (m, 2H), 2.28-2.50 (m, 6H), 2.64 (t, *J* = 6.4 Hz, 2H), 2.93 (t, *J* = 6.4 Hz, 2H), 3.70 (t, *J* = 4.5 Hz, 2H), 4.17 (t, *J* = 7.5 Hz, 2H), 4.64 (d, *J* = 5.8 Hz, 2H), 7.20-7.39 (m, 5H), 8.34 (s, 1H), 10.29 (t-like, 1H). |
| 4-308 | 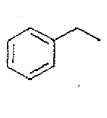 | 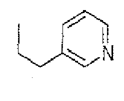 | 1.30-1.42 (m, 2H), 1.42-1.52 (m, 2H), 1.60-1,80 (m, 4H), 2.64 (t, *J* = 5.9 Hz, 2H), 2,79 (t, *J* = 6.1 Hz, 2H), 3.01 (t, *J* = 7.7 Hz, 2H), 4.31 (t, *J =* 7.7 Hz, 2H), 4.87 (t, *J* = 5.8 Hz, 2H), 7.14-7.28 (m, 2H), 7.30-7.42 (m, 4H), 7.57 (ddd, *J* = 6.0, 1.9, 1.9 Hz, 2H), 8.38 (s, 1H), 8.51 (d-like, 2H), 10.3 (t, *J* = 5.8 Hz, 1H). |
| 4-309 | 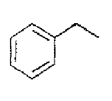 | 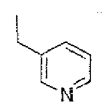 | 1.37-1.53 (m, 4H), 1.60-1.80 (m, 4H), 2.66 (t, *J* = 6.1 Hz, 2H), 2.81 (t, *J* = 6.4 Hz, 2H), 4.64 (t, *J* = 5.8 Hz, 2H), 5.44 (br s, 2H), 7.20-7.42 (m, 7H), 8.45 (s, 1H), 8.45-8.58 (m, 2H), 10.1 (t, *J* = 5.8 Hz,1H). |
| 4-310 | 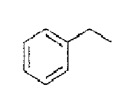 | 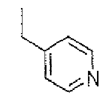 | 1.35-1.55 (m, 4H), 1.60-1.80 (m, 4H), 2.68 (t, *J* = 5.9 Hz, 2H), 2.74 (t, *J* = 6.1 Hz, 2H), 4.62 (t, *J* = 5.8 Hz, 2H), 5.42 (br s, 2H), 6.97 (A₂B₂, *J* = 6.1 Hz, 2H), 7.19-7.37 (m, 5H), 8.47 (s, 1H), 8.54-8.58 (m, 2H), 10.1 (t-like, 1H). |

**Table 98**

| Compound No. | Structure | ¹H-NMR (CDCl₃) |
|---|---|---|
| 4-311 | 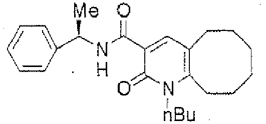 | 0.99 (t, *J* = 7.4 Hz, 3H), 1.36-1.75 (m, 12H), 2.62 (t, *J* = 5.9 Hz, 2H), 2.88 (t, *J* = 6.3 Hz, 2H), 4.08 (brs, 2H), 5.31 (m, 1H), 7.14-7.42 (m, 5H), 8.29 (s, 1H), 10.35 (d, *J* = 7.5 Hz, 1H). |
| 4-312 | 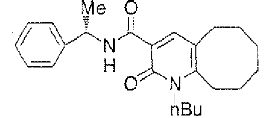 | 0.99 (t, *J* = 7.4 Hz, 3H), 1.36-1.75 (m, 12H), 2.62 (t, *J* = 5.9 Hz, 2H), 2.88 (t, *J* = 6.3 Hz, 2H), 4.08 (brs, 2H), 5.31 (m, 1H), 7.14-7.42 (m, 5H). 8.29 (s, 1H), 10.35 (d, *J* = 7.5 Hz, 1H). |
| 4-313 | 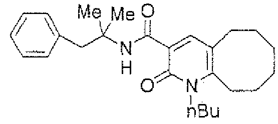 | 0.98 (t, *J* = 7.1 Hz, 3H), 1.40-1.76 (m, 12H), 1.42 (s, 6H), 2.64 (t, *J* = 6.0 Hz, 2H), 2.88 (t, *J* = 6.5 Hz, 2H), 3.19 (s, 2H), 4.07 (brs, 2H), 7.16-7.26 (m, 5H), 8.33 (s, 1H), 9.87 (s, 1H). |
| 4-314 | 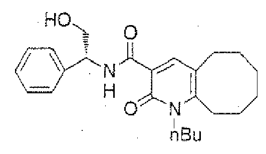 | 0.98 (t, *J* = 7.4 Hz, 3H), 1.39-1.76 (m, 12H), 2.64 (t, *J* = 5.9 Hz, 2H), 2.89 (t, *J* = 6.3 Hz, 2H), 3.93 (dd, *J* = 11.4, 4.5 Hz, 1H), 3.97 (dd, *J* = 11.4, 6.9 Hz, 1H), 4.10 (brs, 2H), 5.31 (m, 1H), 7.27-7.46 (m, 5H), 8.31 (s, 1H), 10.75 (d, *J* = 6.3 Hz, 1H). |
| 4-315 | 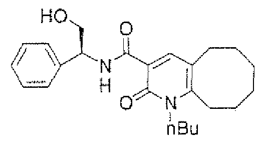 | 0.98 (t, *J* = 7.4 Hz, 3H), 1.39-1.76 (m, 12H), 2.64 (t, *J* = 5.9 Hz, 2H), 2.89 (t, *J* = 6.3 Hz, 2H), 3.93 (dd, *J* = 11.4, 4.5 Hz, 1H), 3.97 (dd, *J* = 11.4, 6.9 Hz, 1H), 4.10 (brs, 2H), 5.31 (m, 1H), 7.27-7.46 (m, 5H), 8.31 (s, 1H), 10.75 (d, *J* = 6.3 Hz, 1H). |
| 4-316 | 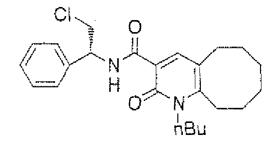 | 0.99 (t, *J* = 7.2 Hz, 3H), 1.32-1.76 (m, 12H), 2.63 (t, *J* = 5.9 Hz, 2H), 2.89 (t, *J* = 6.0 Hz, 2H), 3.91 (d, *J* = 5.7 Hz, 2H), 4.12 (brs, 2H), 5.54 (m, 1H), 7.28-7.45 (m, 5H), 8.29 (s, 1H), 10.77 (d, *J* = 7.5 Hz, 1H). |
| 4-317 | 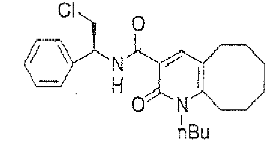 | 0.99 (t, *J* = 7.2 Hz, 3H), 1.32-1.76 (m, 12H), 2.63 (t, *J* = 5.9 Hz, 2H), 2.89 (t, *J* = 6.0 Hz, 2H), 3.91 (d, *J* = 5.7 Hz, 2H), 4.12 (brs, 2H), 5.54 (m, 1H), 7.28-7.45 (m, 5H), 8.29 (s, 1H), 10.77 (d, *J* = 7.5 Hz, 1H). |
| 4-318 | 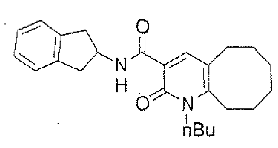 | 0.97 (t, *J* = 7.4 Hz, 3H), 1.38-1.75 (m, 12H), 2.64 (t, *J* = 6.0 Hz, 2H), 2.88 (t, *J* = 6.2 Hz, 2H), 2.99 (dd, *J* = 16.0, 6.6 Hz, 2H), 3.41 (dd, *J* = 16.0, 7.5 Hz, 2H), 4.07 (brs, 2H), 4.88 (m, 1H), 7.15-7.24 (m, 4H), 8.32 (s, 1H), 10.17 (d, *J* = 6.0 Hz, 1H). |

**Table 99**

| Compound No. | Structure | ¹H-NMR (CDCl₃) |
|---|---|---|
| 4-319 | 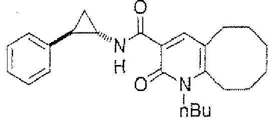 | 0.99 (t, *J* = 7.2 Hz, 3H), 1.25-1.77 (m, 12H), 2.18 (m, 1H), 2.64 (t, *J* = 6.0 Hz, 2H), 2.89 (t, *J* = 6.4 Hz, 2H), 3.14 (m, 1H), 4.10 (brs, 2H), 7.16-7.30 (m, 5H), 8.33 (s, 1H), 10.12 (d, *J* = 3.6 Hz, 1H). |
| 4-320 | 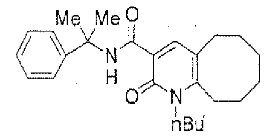 | 1.00 (t, *J* = 7.2 Hz, 3H), 1.30-1.55 (m, 6H), 1.59 (s, 6H), 1.56-1.89 (m, 6H), 2.58 (t, *J* = 6.0 Hz, 2H), 2.88 (t, *J* = 6.3 Hz, 2H), 4.00-4,23 (m, 2H), 7.10-7.40 (m, 5H), 7.46 (d, *J* = 8.4 Hz, 2H), 8.23 (s, 1H). |
| 4-321 | 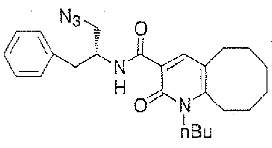 | 1.35-2.04 (m, 8H), 2.03-2.15 (m, 2H), 2.64 (t, *J* = 6.0 Hz, 2H), 2.88-2.96 (m, 4H), 3.63-3.71 (m, 2H), 3.78 (t, *J* = 6.0 Hz, 2H), 4.05 (dd, *J* = 6.9, 2.1 Hz, 2H), 4.1.8-4.27 (m, 3H), 6.49 (dd, *J* = 14.1, 6.6 Hz, 1H), 7.15-7.35 (m, 5H), 8.32 (s, 1H), 10.01 (brs, 1H). |
| 4-322 | 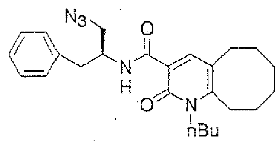 | 1.35-2.04 (m, 8H), 2.03-2.15 (m, 2H), 2.64 (t, *J* = 6.0 Hz, 2H), 2.88-2.96 (m, 4H), 3.63-3.71 (m, 2H), 3.78 (t, *J* = 6.0 Hz, 2H), 4.05 (dd, *J* = 6.9, 2.1 Hz, 2H), 4.18-4.27 (m, 3H), 6.49 (dd, *J* = 14.1, 6.6 Hz, 1H), 7,15-7.35 (m, 5H), 8.32 (s, 1H), 10.01 (brs, 1H). |
| 4-323 | 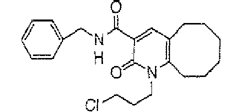 | 1-20-1.90 (m, 8H), 2.13-2.28 (m, 2H), 2.55-2.72 (m, 2H), 2.82-3.02 (m, 2H), 3.62-3.78 (m, 2H), 4.20-4.38 (m, 2H), 4.64 (d, *J* = 6.3 Hz, 2H), 7.18-7.43 (m, 5H), 8.36 (s, 1H). |
| 4-324 | 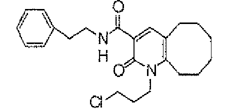 | 1.20-1.90 (m, 8H), 2.12-2.28 (m, 2H), 2.65 (t, *J* = 6.6 Hz, 2H), 2.92-3.02 (m, 4H), 3.60 3.78 (m, 4H), 4.29 (t, *J* = 9.0 Hz, 2H), 7.10-7.40 (m, 5H), 8.33 (s, 1H). |
| 4-325 | 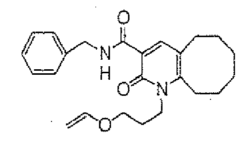 | 1.37-1.80 (m, 8H), 2.02 (m, 2H), 2.65 (t, *J* = 6.3 Hz, 2H), 2.92 (t, *J* = 6.6 Hz, 2H), 3.77 (t, *J* = 5.4 Hz, 2H), 4.04 (dd, *J* = 6.9, 2.1 Hz, 1H), 4.16-4-26 (m, 3H), 4.65 (d, J = 6.0 Hz, 2H), 6.48 (dd, *J* = 14.1, 6.6 hz, 1H), 7.21-7.42 (m, 5H), 8.35 (s, 1H), 10.29 (brs, 1H). |
| 4-326 | 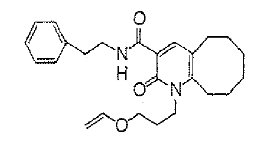 | 1.37 (m, 8H), 2.07 (m, 2H), 2.64 (t, *J* = 6.0 Hz, 2H), 2.64 (t, *J* = 6.0 Hz, 2H), 2.90-2.96 (m, 4H), 3.63-3.71 (m, 2H), 3.78 (t, *J* = 6.0 Hz, 2H), 4.05 (dd, *J* = 6.9 Hz, 2.1 Hz, 1H), 4.18-4.27 (m, 3H), 6.49 (dd, *J* = 14.1, 6.6 Hz, 1H), 7.18-7.33 (m, 5H), 8.32 (s, 1H), 10.01 (brs, 1H). |

**Table 100**

| Compound No. | Structure | ¹H-NMR (CDCl₃) |
|---|---|---|
| 4-327 | 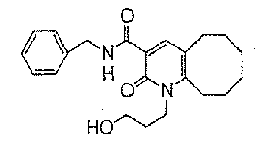 | 1.38-1.81 (m, 8H), 1.88-1.96 (m, 2H), 2.66 (t, *J* = 6.3 Hz, 2H), 2.93 (t, *J* = 6.3 Hz, 2H), 3.52 (t, *J* = 5.1 Hz, 2H), 3.72 (brs, 1H), 4.65 (d, *J* = 6.3 Hz, 2H), 7.22-7.38 (m, 5H), 8.40 (s, 1H), 10.18 (brs, 1H). |
| 4-328 | 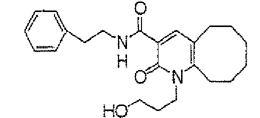 | 1.38-1.95 (m, 10H), 2.65 (t, *J* = 6.0 Hz, 2H), 2.90-2.94 (m, 4H), 3.45-3.52 (m, 2H), 3.65-3.72 (m, 2H), 3.91 (brs, 1H), 4.34 (brs, 2H), 7.20-7.35 (m, 5H), 8.37 (s, 1H), 9.88 (brs, 1H). |
| 4-329 | 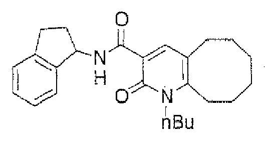 | 0.96 (t, *J* = 6.9 Hz, 3H), 1.30-1.55 (m, 6H), 1.55-1.82 (m, 6H), 1.97 (ddd, *J* = 16.5, 12.9, 8.4 Hz, 1H), 2.60-2.73 (m, 3H), 2.84-2.96 (m, 3H), 3.03 (ddd, *J* = 16.5, 9.3, 3.6 Hz, 1H), 3.93-4.20 (m, 2H), 5.67 (q-like, 1H), 7.10-7.35 (m, 3H), 7.38 (m, 1H), 8.37 (s, 1H). |
| 4-330 | 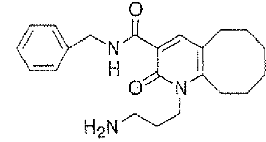 | 1.25-1.28 (m, 14H), 2.65 (t, *J* = 6.0 Hz, 2H), 2.80 (brs, 2H), 2.92 (t, *J* = 6.0 Hz, 2H), 4.22 (m, 2H), 4.65 (d, *J* = 6.0 Hz, 2H), 7.24-7.39 (m, 5H), 8.36 (s, 1H), 10.25 (brs, 1H). |
| 4-331 | 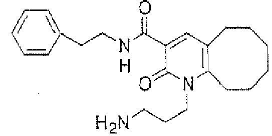 | 1.37-1.90 (m, 12H), 2.64 (t, *J* = 6.3 Hz, 2H), 2.80 (m, 2H), 2.90-2.96 (m, 4H), 3.68 (q, *J* = 6.3 Hz, 2H), 4.23 (brs, 2H), 7.21-7.33 (m, 5H), 8.33 (s, 1H), 9.98 (brs, 1H). |
| 4-332 | 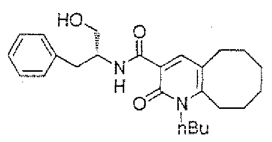 | 1.00 (t, *J* = 7.5 Hz, 3H), 1.33-1.54 (m, 6H), 1.55-1.79 (m, 6H), 2.63 (t, *J* = 6.0 Hz, 2H), 2.89 (t, *J* = 6.3 Hz, 2H), 2.98 (d, *J* = 7.5 Hz, 2H), 3.66 (dd, *J* = 10.1, 6.3 Hz, 1H), 3.79 (dd, *J* = 10.1, 3.6 Hz, 1H), 4.33 (m, 1H), 7.18-7.40 (m, 5H), 8.27 (s, 1H). |
| 4-333 | 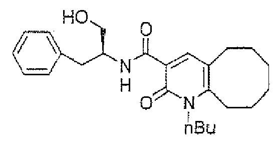 | 1.00 (t, *J* = 7.5 Hz, 3H), 1.33-1.54 (m, 6H), 1.55-1.79 (m, 6H), 2.63 (t, *J* = 6.0 Hz, 2H), 2.89 (t, *J* = 6.3 Hz, 2H), 2.98 (d, *J* = 7.5 Hz, 2H), 3.66 (dd, *J* = 10.1, 6.3 Hz, 1H), 3.79 (dd, *J* = 10.1, 3.6 Hz, 1H), 4.33 (m, 1H), 7.18-7.40 (m, 5H), 8.27 (s, 1H). |

**Table 101**

| Compound No. | Structure | ¹H-NMR (CDCl₃) |
|---|---|---|
| 4-501 | 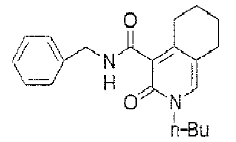 | 0.95 (t, *J* = 7.5 Hz, 3H), 1.37 (sextet, *J* = 7.5 Hz, 2H), 1.66-1.77 (m, 6H), 2.57 (br t, *J* = 6.3 Hz, 2H), 3.27 (br t, *J* = 6.3 Hz, 2H), 3.92 (t, *J* = 7.5 Hz, 2H), 4.60 (d, *J* = 5.7 Hz, 2H), 7,12 (s, 1H), 7.23-7.40 (m, 5H), 9.58 (br t, *J* = 5.7 Hz, 1H). |
| 4-502 | 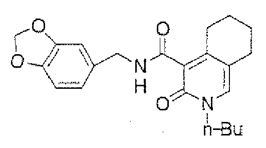 | 0.95 (t, *J* = 7.5 Hz, 3H), 1.37 (sextet, *J* = 7.5 Hz, 2H), 1.66-1.77 (m, 6H), 2.56 (br t, *J* = 6.3 Hz, 2H), 3.27 (br t, *J* = 6.3 Hz, 2H), 3.92 (t, *J* = 7.5 Hz, 2H), 4.50 (s, 2H), 5.92 (s, 2H), 6.75 (d, *J* = 8.4 Hz, 1H), 6.83 (d, *J* = 8.4 Hz, 1H), 6.88 (s, 1H), 7.13 (s, 1H), 9.58 (br s, 1H). |
| 4-503 | 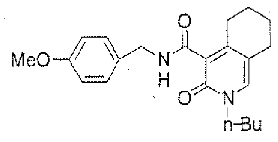 | 0.95 (t, *J* = 7.5 Hz, 3H), 1.37 (sextet, *J* = 7.5 Hz, 2H), 1.65-1.77 (m, 6H), 2.56 (br t, *J* = 6.3 Hz, 2H), 3.27 (br t, *J* = 6.3 Hz, 2H), 3.79 (s, 3H), 3.91 (t, *J* = 7.5 Hz, 2H), 4.53 (s, 2H), 6.86 (d, *J* = 8.4 Hz, 2H), 7.12 (s, 1H), 7.30 (d, *J* = 8.4 Hz, 2H), 9.54 (br s, 1H). |
| 4-504 | 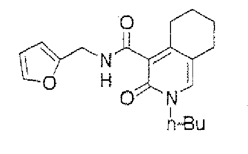 | 0.95 (t, *J* = 7.2 Hz, 3H), 1.37 (sextet, *J* = 7.2 Hz, 2H), 1.66-1.78 (m, 6H), 2.56 (br t, *J* = 6.3 Hz, 2H), 3.27 (br t, *J* = 6.3 Hz, 2H), 3.92 (t, *J* = 7.2 Hz, 2H), 4.58 (s, 2H), 6.27 (dd, *J* = 3.0 Hz, 0.9 Hz, 1H), 6.30 (dd, *J* = 3.0 Hz, 1.8 Hz, 1H), 7.13 (s, 1H), 7.35 (dd, *J* = 1.8 Hz, 0.9 Hz, 1H), 9.65 (br s, 1H). |
| 4-505 | 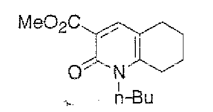 | 0.97 (t, *J* = 7.5 Hz, 3H), 1.43 (sextet, *J* = 7.5 Hz, 2H), 1.63-1.78 (m, 4H), 1.87 (quint, *J* = 6.0 Hz, 2H), 2.57 (t, *J* = 6.0 Hz, 2H), 2.73 (t, *J* = 6.0 Hz, 2H), 3.90 (s, 3H), 4.02 (t, *J* = 7.8 Hz, 2H), 7.92 (s, 1H). |

**Table 102**

| Compound No. | Structure | ¹H-NMR (CDCl₃) |
|---|---|---|
| 4-506 | 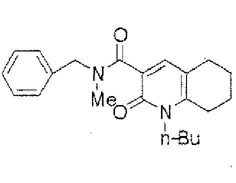 | 0.96 (t, *J* = 7.5 Hz, 3H), 1.42 (sextet, *J* = 7.5 Hz, 2H), 1.60-1.77 (m, 4H), 1.84 (quint, *J* = 6.0 Hz, 2H), 2.54 (t, *J* = 6.0 Hz, 2H), 2.70 (t, *J* = 6.0 Hz, 2H), 2.88 (s, 3H), 4.02 (t, *J* = 7.8 Hz, 2H), 4.75 (s, 2H), 7.17-7.40 (m, 6H). (minor isomer): δ 0.95 (t, *J* = 7.5 Hz, 3H), 1.41 (sextet, *J* = 7.5 Hz, 2H), 1.60-1.77 (m, 4H), 1.83 (quint, *J* = 6.0 Hz, 2H), 2.50 (t, *J* = 6.0 Hz, 2H), 2.68 (t, *J* = 6.0 Hz, 2H), 2.97 (s, 3H), 4.01 (t, *J* = 7.8 Hz, 2H), 4.49 (s, 2H), 7.17-7.40 (m, 6H). |
| 4-507 | 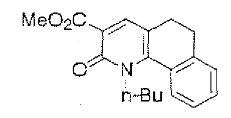 | 0.89 (t, *J* = 7.5 Hz, 3H), 1.30 (sextet, *J* = 7.5 Hz, 2H), 1.92 (quint, *J* = 6.9 Hz, 2H), 2.57, 2.77 (ABq, *J* = 9.0 Hz, 2H), 2.58, 2.76 (ABq, *J* = 7.5 Hz, 2H), 3.93 (s, 3H), 4.30 (t, *J* = 7.5 Hz, 2H), 7.31-7.41 (m, 3H), 7.56 (dd, *J* = 7.2 Hz, 2.4 Hz, 1H), 8.10 (s, 1H). |
| 4-508 | 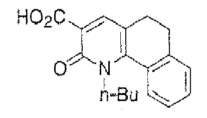 | 0.91 (t, *J* = 7.5 Hz, 3H), 1.30 (sextet, *J* = 7.5 Hz, 2H), 1.91 (quint, *J* = 6.6 Hz, 2H), 2.65, 2.80 (ABq, *J* = 9.0 Hz, 2H), 2.66, 2.79 (ABq, *J* = 7.5 Hz, 2H), 4.42 (t, *J* = 7.5 Hz, 2H), 7.38-7.46 (m, 3H), 7.57 (d, *J* = 7.5 Hz, 1H), 8.39 (s, 1H), 14.77 (br s, 1H). |
| 4-509 | 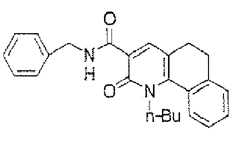 | 0.88 (t, *J* = 7.5 Hz, 3H), 1.27 (sextet, *J* = 7.5 Hz, 2H), 1.87 (quint, *J* = 6.0 Hz, 2H), 2.62, 2.78 (ABq, *J* = 9.0 Hz, 2H), 2.64, 2.76 (ABq, *J* = 7.5 Hz, 2H), 4.35 (t, *J* = 7.5 Hz, 2H), 4.68 (d, *J* = 6.0 Hz, 2H), 7.21-7.41 (m, 8H), 7.53 (dd, *J* = 6.9 Hz, 2.4 Hz, 1H), 8.46 (s, 1H), 10.33 (br t, *J* = 6.0 Hz, 1H). |
| 4-510 | 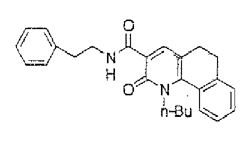 | 0.90 (t, *J* = 7.5 Hz, 3H), 1.29 (sextet, *J* = 7.5 Hz, 2H), 1.87 (quint, *J* = 6.6 Hz, 2H), 2.62, 2.78 (ABq, *J* = 9.0 Hz, 2H), 2.63, 2.76 (ABq, *J* = 7.5 Hz, 2H), 2.96 (t, *J* = 7.5 Hz, 2H), 3.66-3.74 (m, 2H), 4.36 (t, *J* = 7.5 Hz, 2H), 7.19-7.37 (m, 8H), 7.53 (dd, *J* = 6.9 Hz, 2.4 Hz, 1H), 8.42 (s, 1H), 10.06 (br t, *J* = 6.0 Hz, 1H). |

**Table 103**

| | | |
|---|---|---|
| 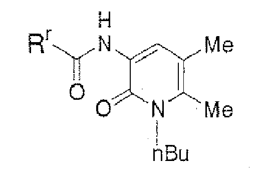 | | |

| Compound No. | R^{r} | ¹H-NMR (CDCl₃) |
|---|---|---|
| 5-001 | Me | 0.98 (t, *J* = 7.5 Hz, 3H), 1.37-1.50 (m, 2H), 1.60-1.70 (m, 2H), 2.12 (s, 3H), 2.17 (s, 3H), 2.30 (s, 3H), 4.10 (t, *J* = 7.8 Hz, 2H), 8.20 (s, 1H), 8.35 (br s, 1H). |
| 5-002 | 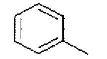 | 1.00 (t, *J* = 7.2 Hz, 3H), 1.40-1.52 (m, 2H), 1.64-1.74 (m, 2H), 2.17 (s, 3H), 2.34 (s, 3H), 4.14 (t, *J* = 7.8 Hz, 2H), 7.44-7.57 (m, 3H), 7.92-7.95 (m, 2H), 8.41 (s, 1H), 9.22 (br s, 1H). |
| 5-003 | 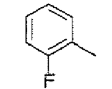 | 0.99 (t, *J* = 7.2 Hz, 3H), 1.39-1.52 (m, 2H), 1.64-1.74 (m, 2H), 2.16 (s, 3H), 2.34 (s, 3H), 4.13 (t, *J* = 7.8 Hz, 2H), 7.15-7.24 (m, 1H), 7.30 (dd, 1.8, 8.4 Hz, 1H), 7.47-7.54 (m, 1H), 8.12 (dt, 1.8, 7.8 Hz, 1H), 8.42 (s, 1H), 9.75 (br s, 1H). |
| 5-004 | 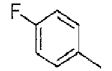 | 1.00 (t, *J* = 7.2 Hz, 3H), 1.40-1.52 (m, 2H), 1.63-1.74 (m, 2H), 2.17 (s, 3H), 2.34 (s, 3H), 4.13 (t, *J=* 7.8 Hz, 2H), 7.12-7.18 (m, 2H), 7.93-7.97 (m, 2H), 8.37 (s, 1H), 9.16 (br s, 1H). |
| 5-005 | 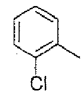 | 0.98 (t, *J=* 7.2 Hz, 3H), 1.38-1.50 (m, 2H), 1.64-1.72 (m, 2H), 2.17 (s, 3H), 2.34 (s, 3H), 4.11 (t, *J* = 7.8 Hz, 2H), 7.31-7.47 (m, 3H), 7.73 (dd, *J* = 2.1,7.2 Hz, 1H), 8.41 (s, 1H), 9.13 (br s, 1H). |
| 5-006 | 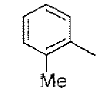 | 0.98 (t, *J* = 7.2 Hz, 3H), 1.38-1.50 (m, 2H), 1.61-1.72 (m, 2H), 2.17 (s, 3H), 2.34 (s, 3H), 2.53 (s, 3H), 4.11 (t, *J* = 7.8 Hz, 2H), 7.20-7.26 (m, 2H), 7.32-7.37 (m, 1H), 7.54 (d, *J* = 7.8 Hz, 1H), 8.39 (s, 1H), 8.74 (br s, 1H). |
| 5-007 | 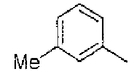 | 1.00 (t, *J* = 7.2 Hz, 3H), 1.40-1.52 (m, 2H), 1.64-1.74 (m, 2H), 2.17 (s, 3H), 2.34 (s, 3H), 2.42 (s, 3H), 4.13 (t, *J* = 7.8 Hz, 2H), 7.35 (m, 2H), 7.74 (m, 2H), 8.41 (s, 1H), 9.21 (br s, 1H). |
| 5-008 | 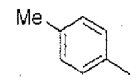 | 0.99 (t, *J* = 7.2 Hz, 3H), 1.40-1.52 (m, 2H), 1.64-1.74 (m, 2H), 2.16 (s, 3H), 2.34 (s, 3H), 2.41 (s, 3H), 4.13 (t, *J* = 7.8 Hz, 2H), 7.27 (d, *J* = 8.1 Hz, 2H), 7.84 (d, *J* = 8.1 Hz, 2H), 8.40 (s, 1H), 9.20 (br s, 1H). |
| 5-009 | 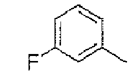 | 1.00 (t, *J* = 7.2 Hz, 3H), 1.40-1.52 (m, 2H), 1.64-1.74 (m, 2H), 2.17 (s, 3H), 2.35 (s, 3H), 4.13 (t, *J* = 7.8 Hz, 2H), 7.20-7.27 (m, 1H), 7.41-7.48 (m, 1H), 7.63-7.70 (m, 2H), 8.38 (s, 1H), 9.19 (br s, 1H). |
| 5-010 | 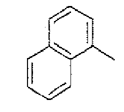 | 0.98 (t, *J* = 7.2 Hz, 3H), 1.38-1.50 (m, 2H), 1.62-1.73 (m, 2H), 2.20 (s, 3H), 2.36 (s, 3H), 4.12 (t, *J* = 7.8 Hz, 2H), 7.46-7.59 (m, 3H), 7.79 (dd, *J* = 1.2, 7.2 Hz, 1H), 7.88 (dd, 1.5, 7.2 Hz, 1H), 7.95 (d, *J* = 8.1 Hz, 1H), 8.45 (dd, *J* = 1.5, 7.5 Hz, 1H), 8.50 (s, 1H), 8.95 (br s, 1H). |
| 5-011 | 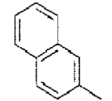 | 1.01 (t, *J* = 7.2 Hz, 3H), 1.42-1.54 (m, 2H), 1.66-1.76 (m, 2H), 2.19 (s, 3H), 2.36 (s, 3H), 4.16 (t, *J* = 7.8 Hz, 2H), 7.53-7.62 (m, 2H), 7.88-8.03 (m, 4H), 8.47 (s, 2H), 9.41 (br s, 1H). |

**Table 104**

| | | |
|---|---|---|
| 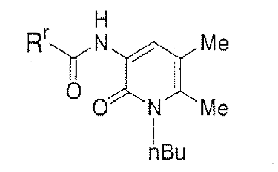 | | |

| Compound No. | R^{r} | ¹H-NMR (CDCl₃) |
|---|---|---|
| 5-012 | 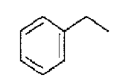 | 0.96 (t, *J* = 7.2 Hz, 3H), 1.35-1.47 (m, 2H), 1.57-1.67 (m, 2H), 2.09 (s, 3R), 2.28 (s, 3H), 3.72 (s, 2H), 4.05 (t, *J* = 7.8 Hz, 2H), 7.28-7.40 (m, 5H), 8.22 (s, 1H), 8.40 (br s, 1H). |
| 5-013 | 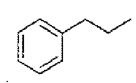 | 0.98 (t, *J* = 7.2 Hz, 3H), 1.37-1.49 (m, 2H), 1.60-1.70 (m, 2H), 2.12 (s, 3H), 2.31 (s, 3H), 2.69 (t, *J* = 7.5 Hz, 2H), 3.04 (t, *J* = 7.5 Hz, 2H), 4.09 (t, *J* = 7.8 Hz, 2H), 7.17-7.34 (m, 5H), 8.23 (s, 1H), 8.35 (br s, 1H). |
| 5-014 | 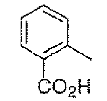 | 0.92 (t, *J* = 7.2 Hz, 3H), 1.31-1.39 (m, 2H), 1.51-1.62 (m, 2H), 2.11 (s, 3H), 2.33 (s, 3H), 4.05 (t, *J* = 7.8 Hz, 2H), 7.54-7.64 (m, 3H), 7.83 (d, *J* = 7.5 Hz, 1H), 8.14 (s, 1H), 8.99 (s, 1H), 13.06 (br s, 1H). |
| 5-015 | nBuO- | 0.94 (t, *J* = 7.5 Hz, 3H), 1.35-1.49 (m, 4H), 1.60-1.70 (m, 4H), 2.11 (s, 3H), 2.29 (s, 3H), 4.09 (t, *J* = 7.8 Hz, 2H), 4.15 (t, *J* = 6.6 Hz, 2H), 7.73 (br s, 1H), 7.85 (br s, 1H). |
| 5-016 | 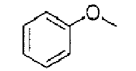 | 0.99 (t, *J* = 7.2 Hz, 3H), 1.39-3.51 (m, 2H), 1.62-1.73 (m, 2H), 2.11 (s, 3H), 2.31 (s, 3H), 4.13 (t, *J* = 7.8 Hz, 2H), 7.16-7.41 (m, 5H), 7.88 (s, 1H), 8.09 (br s, 1H). |
| 5-017 | BnO- | 0.97 (t, *J* = 7.2 Hz, 3H), 1.36-1.48 (m, 2H), 1.59-1.69 (m, 2H), 2.11 (s, 3H), 2.29 (s, 3H), 4.08 (t, *J* = 7.8 Hz, 2H), 5.19 (s, 2H), 7.26-7.41 (m, 5H), 7.83 (s, 1H), 7.86 (s, 1H). |
| 5-018 | 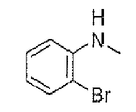 | 0.90 (t, *J* = 7.2 Hz, 3H), 1.27-1.40(m, 2H), 1.57-1.67(m, 2H), 2.14(s, 3H), 2.31(s, 3H), 4.09(t, *J* = 7.8 Hz, 2H), 6.91-6.97(m, 1H), 7.28-7.34(m, 1H), 7.49(br s, 1H), 7.50-7.54(m, 1H), 8.08-8.11(m, 1H), 8.10(s, 1H), 8.38(br s, 1H). |
| 5-019 | 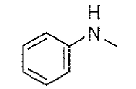 | 1.00 (t, *J* = 7.2 Hz, 3H), 1.39-1.51 (m, 2H), 1.61-1.71 (m, 2H), 2.15 (s, 3H), 2.35 (s, 3H), 4.15 (t, *J* = 7.8 Hz, 2H), 6.98-7.03 (m, 1H), 7.25-7.30 (m, 2H), 7.41-7.45 m, 2H), 8.03 (s, 1H). |
| 5-020 | 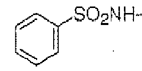 | 0.98 (t, *J* = 7.2 Hz, 3H), 1.42-1.75 (m, 4H), 2.12 (s, 3H), 4.27 (t, *J* = 7.8Hz, 2H), 7.48-7.61 (m, 3H), 8.04-8.09 (m, 3H), 8.98 (s, 1H), 10.35 (br s, 1H). |

**Table 105**

| Compound No. | Structure | ¹H-NMR (CDCl₃) |
|---|---|---|
| 5-101 | 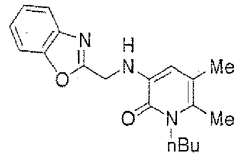 | 0.97 (t, *J* = 7.2 Hz, 3H), 1.36-1.49 (m, 2H), 1.60-1.70 (m, 2H), 2.04 (s, 3H), 2.22 (s, 3H), 4.08 (t, *J* = 7.5 Hz, 2H), 4.51 (s, 2H), 6.27 (s, 1H), 7.30-7.35 (m, 2H), 7.48-7.52 (m, 1H), 7.69-7.72 (m, 1H). |
| 5-102 | 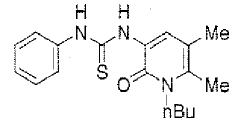 | 0.98 (t, *J* = 7.2 Hz, 3H), 1.37-1.49 (m, 2H), 1.59-1.69 (m, 2H), 2.18 (s, 3H), 2.38 (s, 3H), 4.10 (t, *J* = 7.8 Hz, 2H), 7.23-7.43 (m, 1H), 7.42-7.43 (m, 4H), 8.86 (s, 1H). |
| 5-103 | 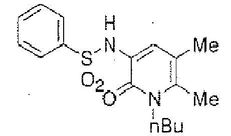 | 0.92 (t, *J* = 7.2 Hz, 3H), 1.25-1.37 (m, 2H), 1.45-1.55 (m, 2H), 2.08 (s, 3H), 2.23 (s, 3H), 3.97 (t, *J* = 7.8 Hz, 2H), 7.35 (s, 1H), 7.41-7.55 (n, 2H), 7.65 (br s, 1H), 7.83-7.87 (m, 2H). |
| 5-104 | 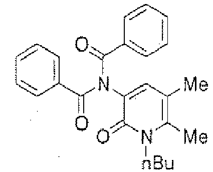 | 0.95 (t, *J* = 7.2 Hz, 3H), 1.33-1.45 (m, 2H), 1.59-1.70 (m, 2H), 2.00 (s, 3H), 2.30 (s, 3H), 4.10 (t, *J* = 7.8 Hz, 2H), 7.03 (s, 1H), 7.28-7.44 (m, 6H), 7.79-7.82 (m, 4H). |
| 5-105 | 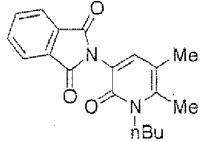 | 0.96 (t, *J* = 7.2 Hz, 3H), 1.37-1.49 (m, 2H), 1.62-1.73 (m, 2H), 2.16 (s, 3H), 2.39 (s, 3H), 4.11 (t, *J* = 8.1 Hz, 2H), 7.30 (s, 1H), 7.72-7.77 (m, 2H), 7.88-7.94 (m, 2H). |
| 5-106 | 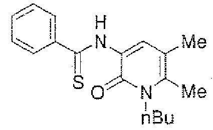 | 0.99 (t, *J* = 7.2 Hz, 3H), 1.40-1.52 (m, 2H), 1.64-1.75 (m, 2H), 2.20 (s, 3H), 2.37 (s, 3H), 4.15 (t, *J* = 7.8 Hz, 2H), 7.38-7.50 (m, 3H), 7.89-7.92 (m, 2H), 9.57 (s, 1H), 10.60 (br s, 1H). |

**Table 106**

| | | |
|---|---|---|
| 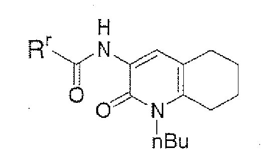 | | |

| Compound No. | R^{r} | ¹H-NMR (CDCl₃) |
|---|---|---|
| 6-001 | 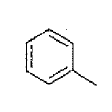 | 0.99 (t, *J* = 7.5 Hz, 3H), 1.45 (sextet, *J* = 7.5 Hz, 2H), 1.66 (quint, *J* = 7.5 Hz, 2H), 1.74 (quint, *J* = 6.0 Hz, 2H), 1.87 (quint, *J* = 6.0 Hz, 2H), 2.60 (t, *J* = 6.0 Hz, 2H), 2.69 (t, *J* = 6.0 Hz, 2H), 4.06 (t, *J* = 7.8 Hz, 2H), 7.43-7.56 (m, 3H), 7.94 (d, *J* = 6.9 Hz, 2H), 8.31 (s, 1H), 9.26 (br s, 1H). |
| 6-002 | 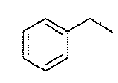 | 0.95 (t, *J* = 7.5 Hz, 3H), 1.40 (sextet, *J* = 7.5 Hz, 2H), 1.56-1.65 (m, 2H), 1.69 (quint, *J* = 6.0 Hz, 2H), 1.82 (quint, *J* = 6.0 Hz, 2H), 2.52 (t, *J* = 6.0 Hz, 2H), 2.62 (t, *J* = 6.0 Hz, 2H), 3.72 (s, 2H), 3.98 (t, *J* = 7.8 Hz, 2H), 7.27-7.39 (m, 5H), 8.13 (s, 1H), 8.44 (br s, 1H). |
| 6-003 | 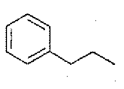 | 0.97 (t, *J* = 7.5 Hz, 3H), 1.42 (sextet, *J* = 7.5 Hz, 2H), 1.61 (quint, *J* = 7.5 Hz, 2H), 1.71 (quint, *J* = 6.0 Hz, 2H), 1.84 (quint, *J* = 6.0 Hz, 2H), 2.55 (t, *J* = 6.0 Hz, 2H), 2.65 (t, *J* = 6.0 Hz, 2H), 2.70 (t, *J* = 7.8 Hz, 2H), 3.04 (t, *J* = 7.8 Hz, 2H), 4.02 (t, *J* = 7.8 Hz, 2H), 7.18-7.33 (m, 5H), 8.15 (s, 1H), 8.41 (br s, 1H). |
| 6-004 | 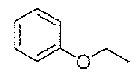 | 0.98 (t, *J* = 7.5 Hz, 3H), 1.41 (sextet, *J* = 7.5 Hz, 2H), 1.64 (quint, *J* = 7.5 Hz, 2H), 1.72 (quint, *J* = 6.0 Hz, 2H), 1.85 (quint, *J* = 6.0 Hz, 2H), 2.57 (t, *J* = 6.0 Hz, 2H), 2.67 (t, *J* = 6.0 Hz, 2H), 4.03 (t, *J* = 7.8 Hz, 2H), 4.60 (s, 2H), 7.03 (d, *J* = 7.8 Hz, 2H), 7.32 (d, *J* = 7.8 Hz, 3H), 8.19 (s, 1H), 9.49 (br s, 1H). |
| 6-005 | 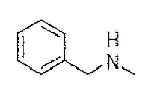 | 0.92 (t, *J* = 7.5 Hz, 3H), 1.32 (sextet, *J* = 7.5 Hz, 2H), 1.57-1.65 (m, 2H), 1.69 (quint, *J* = 6.0 Hz, 2H), 1.82 (quint, *J* = 6.0 Hz, 2H), 2.55 (t, *J* = 6.0 Hz, 2H), 2.59 (t, *J* = 6.0 Hz, 2H), 3.90 (t, *J* = 7.8 Hz, 2H), 4.46 (d, *J* = 6.0 Hz, 2H), 5.72 (br s, 1H), 7.24-7.32 (m, 5H), 7.95 (s, 1H), 8.00 (br s, 1H). |
| 6-006 | 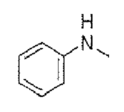 | (in *d₆*-DMSO): 0.93 (t, *J* = 7.5 Hz, 3H), 1.35 (sextet, *J* = 7.5 Hz, 2H), 1.56 (quint, *J* = 7.5 Hz, 2H), 1.60-1.70 (m, 2H), 1.71-1.80 (m, 2H), 2.51 (t, *J* = 6.0 Hz, 2H), 2.67 (t, *J* = 6.0 Hz, 2H), 4.00 (t, *J* = 7.5 Hz, 2H), 6.96 (t, *J* = 7.2 Hz, 1H), 7.27 (t, *J=* 7.5 Hz, 2H), 7.43 (d, *J* = 7.5 Hz, 2H), 7.84 (s, 1H), 8.53 (br s, 1H), 9.51 (br s, 1H). |
| 6-007 | 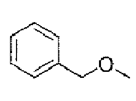 | 0.96 (t, *J* = 7.5 Hz, 3H), 1.41 (sextet, *J* = 7.5 Hz, 2H), 1.63 (quint, *J* = 7.5 Hz, 2H), 1.70 (quint, *J* = 6.0 Hz, 2H), 1.83 (quint, *J* = 6.0 Hz, 2H), 2.53 (t, *J* = 6.0 Hz, 2H), 2.63 (t, *J* = 6.0 Hz, 2H), 4.01 (t, *J* = 7.8 Hz, 2H), 5.19 (s, 2H), 7.29-7.41 (m, 5H), 7.76 (s, 1H), 7.86 (br s, 1H). |

**Table 107**

| Compound No. | Structure | ¹H-NMR (CDCl₃) |
|---|---|---|
| 7-001 | 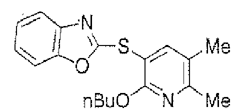 | 0.71 (t, *J* = 7.2 Hz, 3H), 1.10-1.23(m, 2H), 1.46-1.55(m, 2H), 2.22(s, 3H), 2.43(s, 3H), 4.28 (t, *J* = 6.3 Hz, 2H), 7.20-7.29(m, 2H), 7.38-7.41(m, 1H), 7.58-7.60(m, 1H), 7.67(s, 1H). |
| 7-002 | 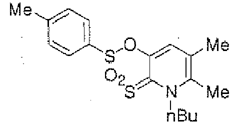 | 0.97 (t, *J* = 7.2 Hz, 3H), 1.37-1.49 (m, 2H), 1.60-1.80 (m, 2H), 2.21 (s, 3H), 2.44 (s, 3H), 2.48 (s, 3H), 4.71 (br s, 2H), 7.30-7.35 (m, 3H), 8.01 (s, 1H), 8.04 (s, 1H). |
| 7-003 | 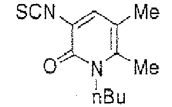 | 0.97 (t, *J* = 7.5 Hz, 3H), 1.37-1.50 (m, 2H), 1.62-1.73 (m, 2H), 2.07 (s, 3H), 2.33 (s, 3H), 4.09 (t, *J* = 7.8 Hz, 2H), 6.96 (s, 1H). |
| 7-004 | 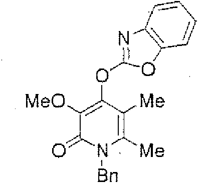 | 2.07 (s, 3H), 2.28 (s, 3H), 3.88 (s, 3H), 5.42 (br s, 2H), 7.19-7.54 (m, 9H). |
| 7-005 | 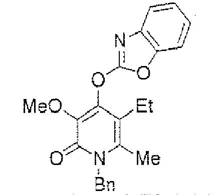 | 1.10 (t, *J* = 7.5 Hz, 3H), 2.30 (s, 3H), 2.53 (q, *J* = 7.5 Hz, 2H), 3.85 (s, 3H), 5.41 (br s, 2H), 7.18-7.55 (m, 9H). |
| 7-006 | 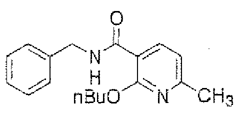 | 0.84 (t, *J* = 7.2 Hz, 3H), 1.28 (sextet, *J* = 7.5 Hz, 2H), 1.61-1.68 (m, 2H), 2.46 (s, 3H), 4.42 (t, *J* = 6.6 Hz, 2H), 4.64 (d, *J* = 5.1 Hz, 1H), 6.87 (d, *J* = 6.0 Hz, 1H), 7.26-7.37 (m, 5H), 8.32 (br s, 1H), 8.42 (d, *J* = 7.5 Hz, 1H). |
| 7-007 | 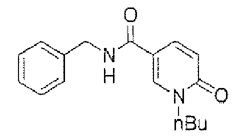 | 0.95 (t, *J* = 7.2 Hz, 3H), 1.39 (m, 2H), 1.74 (m, 2H), 3.98 (t, *J* = 7.5 Hz, 2H), 4.50 (d, *J* = 5.7 Hz, 1H), 4.60 (d, 5.7 Hz, 1H), 5.9 (brs, 1H), 6.36 (brs, 1H), 6.56 (dd, *J* = 9.6, 3.6 Hz, 1H), 7.25-7.36 (m, 5H), 7.86 (m, 1H), 8.22 (m, 2H). |
| 7-008 | 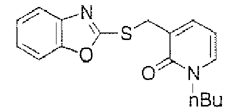 | 0.96 (t, *J* = 7.3 Hz, 3H), 1.32-1.45 (m, 2H), 1.69-1.79 (m, 2H), 3.96 (t, *J* = 7.6 Hz, 2H), 4.44 (s, 2H), 7.19-7.30 (m, 3H), 7.40-7.43 (m, 1H), 7.59-7.62 (m, 1H), 7.66 (dd, *J* = 7.0 Hz, 1H). |
| 7-009 | 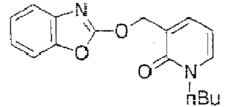 | 0.96 (t, *J* = 7.3 Hz, 3H), 1.32-1.44 (m, 2H), 1.68-1.79 (m, 2H), 3.96 (t, *J* = 7.6 Hz, 2H), 6.14 (t, *J* = 7.0 Hz, 1H), 7.06-7.27 (m, 5H), 7.39 (dd, *J* = 1.8, 7.0 Hz, 1H). |

**Table 108**

| Compound No. | Structure | ¹H-NMR (CDCl₃) |
|---|---|---|
| 7-010 | 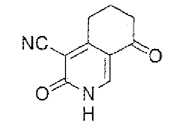 | (in CDCl₃ + CD₃OD): 2.17 (quint, *J* = 6.3 Hz, 2H), 2.63 (t, *J* = 6.3 Hz, 2H), 3.09 (t, *J* = 6.3 Hz, 2H), 8.34 (s, 1H). |

**Table 109**

| Compound No. | Structure | ¹H-NMR (CDCl₃) |
|---|---|---|
| 7-011 | 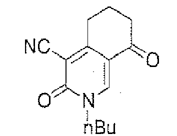 | 0.97 (t, *J* = 7.6 Hz, 3H), 1.38 (sextet, *J* = 7.5 Hz, 2H), 1.76 (quint, *J* = 7.5 Hz, 2H), 2.15 (quint, *J* = 6.3 Hz, 2H), 2.61 (t, *J* = 6.3 Hz, 2H), 3.06 (t, *J* = 6.3 Hz, 2H), 4.03 (t, J = 7.5 Hz, 2H), 8.39 (s, 1H). |
| 7-012 | 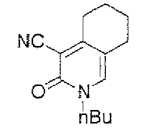 | 0.96 (t, *J* = 7.5 Hz, 3H), 1.37 (sextet, *J* = 7.5 Hz, 2H), 1.67-1.86 (m, 6H), 2.54 (t, *J* = 6.3 Hz, 2H), 2.87 (t, *J* = 6.3 Hz, 2H), 3.93 (t, *J* = 7.5 Hz, 2H), 7.22 (s, 1H). |
| 7-013 | 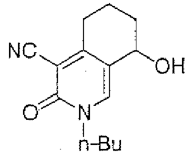 | 0.97 (t, *J* = 7.5 Hz, 3H), 1.38 (sextet, *J* = 7.5 Hz, 2H), 1.76 (quint, *J* = 7.5 Hz, 2H), 2.15 (quint, *J* = 6.0 Hz, 2H), 2.61 (t, *J* = 6.0 Hz, 2H), 3.06 (t, *J* = 6.0 Hz, 2H), 3.45-3.58 (m, 1H), 4.03 (t, *J* = 7.5 Hz, 2H), 8.39 (s, 1H). |
| 7-014 | 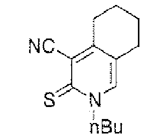 | 0.98 (t, *J* = 7.5 Hz, 3H), 1.41 (sextet, *J* = 7.5 Hz, 2H), 1.75-1.90 (m, 6H), 2.60 (t, *J* = 6.3 Hz, 2H), 2.87 (t, *J* = 6.3 Hz, 2H), 4.81 (t, *J* = 7.5 Hz, 2H), 7.50 (s, 1H). |
| 7-015 | 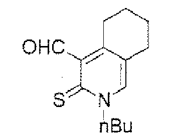 | 0.99 (t, *J* = 7.5 Hz, 3H), 1.44 (sextet, *J* = 7.5 Hz, 2H), 1.74 (quint, *J* = 3.3 Hz, 4H), 1.87 (quint, *J* = 7.5 Hz, 2H), 2.62 (br t, *J* = 6.3 Hz, 2H), 2.95 (br t, *J* = 6.3 Hz, 2H), 4.51 (t, *J* = 7.5 Hz, 2H), 7.53 (s, 1H), 10.60 (s, 1H). |
| 7-016 | 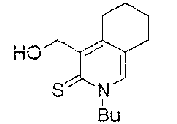 | 0.99 (t, *J* = 7.2 Hz, 3H), 1.43 (sextet, *J* = 7.2 Hz, 2H), 1.71-1.95 (m, 6H), 2.66 (br t, *J* = 6.3 Hz, 2H), 2.83 (t, *J* = 6.3 Hz, 2H), 4.58 (br t, *J* = 7.2 Hz, 2H), 4.79 (s, 2H), 7.61 (s, 1H). |
| 7-017 | 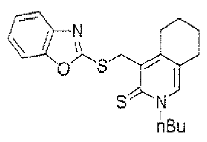 | 0.99 (t, *J* = 7.5 Hz, 3H), 1.43 (sextet, *J* = 7.5 Hz, 2H), 1.70-1.95 (m, 6H), 2.62 (t, *J* = 6.3 Hz, 2H), 3.01 (t, *J* = 6.3 Hz, 2H), 4.58 (t, *J* = 7.5 Hz, 2H), 5.05 (s, 2H), 7.15-7.30 (m, 2H), 7.42 (dd, *J* = 7.2 Hz, *J* = 1.8 Hz, 1H), 7.48 (br s, 1H), 7.60 (dd, *J* = 7.2 Hz, *J* = 1.8 Hz, 1H). |
| 7-018 | 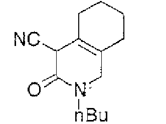 | 0.93 (t, *J* = 7.5 Hz, 3H), 1.32 (sextet, *J* = 7.5 Hz, 2H), 1.42-1.59 (m, 5H), 1.88 (s, 1H), 1.97-2.08 (m, 2H), 2.20-2.32 (m, 1H), 2.54-2.66 (m, 1H), 3.06-3.19 (m, 2H), 3.33-3.43 (m, 3H). |
| 7-019 | 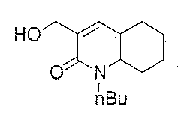 | 0.98 (t, *J* = 7.5 Hz, 3H), 1.43 (sextet, *J* = 7.5 Hz, 2H), 1.65 (quint, *J* = 7.5 Hz, 2H), 1.71 (quint, *J* = 6.0 Hz, 2H), 1.85 (quint, *J* = 6.0 Hz, 2H), 2.52 (t, *J* = 6.0 Hz, 2H), 2.68 (t, *J* = 6.0 Hz, 2H), 4.00 (t, *J* = 7.8 Hz, 2H), 4.53 (s, 2H), 7.02 (s, 1H). |

**Table 110**

| Compound No. | Structure | ¹H-NMR (CDCl₃) |
|---|---|---|
| 7-020 | 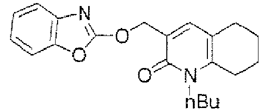 | 0.97 (t, *J* = 7.5 Hz, 3H), 1.43 (sextet, *J* = 7.5 Hz, 2H), 1.57-1.72 (m, 4H), 1.81 (quint, *J* = 6.0 Hz, 2H), 2.45 (t, *J* = 6.0 Hz, 2H), 2.65 (t, *J* = 6.0 Hz, 2H), 4.01 (t, *J* = 7.8 Hz, 2H), 4.93 (s, 2H), 7.08-7.30 (m, 4H), 7.13 (s, 1H): |
| 7-021 | 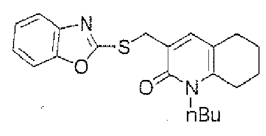 | 0.97 (t, *J* = 7.5 Hz, 3H), 1.43 (sextet, *J* = 7.5 Hz, 2H), 1.59-1.73 (m, 4H), 1.81 (quint, *J* = 6.0 Hz, 2H), 2.49 (t, *J* = 6.0 Hz, 2H), 2.66 (t, *J* = 6.0 Hz, 2H), 4.01 (t, *J* = 7.8 Hz, 2H), 4.43 (s, 2H), 7.24 (quint d, *J* = 7.5 Hz, 1.5 Hz, 2H), 7.40 (s, 1H), 7.42 (dd, *J* = 7.5 Hz, 1.5 Hz, 1H), 7.60 (dd, *J* = 7.5 Hz, 1.5 Hz, 1H). |
| 7-022 | 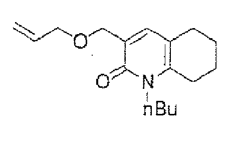 | 0.96 (t, *J* = 7.5 Hz, 3H), 1.42 (sextet, *J* = 7.5 Hz, 2H), 1.64 (quint, *J* = 7.5 Hz, 2H), 1.70 (quint, *J* = 6.0 Hz, 2H), 1.84 (quint, *J* = 6.0 Hz, 2H), 2.53 (t, *J* = 6.0 Hz, 2H), 2.67 (t, *J* = 6.0 Hz, 2H), 3.99 (t, *J* = 7.8 Hz, 2H), 4.12 (dt, *J* = 6.0 Hz, 1.5 Hz, 2H), 4.46 (s, 2H), 5.20 (dq, *J* = 10.5 Hz, 1.8 Hz, 1H), 5.33 (dq, *J* = 17.1 Hz, 1.8 Hz, 1H), 5.91-6.05 (m, 1H), 7.19 (s, 1H). |
| 7-023 | 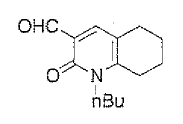 | 0.99 (t, *J* = 7.5 Hz, 3H), 1.46 (sextet, *J* = 7.5 Hz, 2H), 1.68 (quint, *J* = 7.5 Hz, 2H), 1.74 (quint, *J* = 6.0 Hz, 2H), 1.88 (quint, *J* = 6.0 Hz, 2H), 2.59 (t, *J* = 6.0 Hz, 2H), 2.76 (t, *J* = 6.0 Hz, 2H), 4.05 (t, *J* = 7.8 Hz, 2H), 7.76 (s, 1H), 10.34 (s, 1H). |

**Table 111**

| | | | |
|---|---|---|---|
| 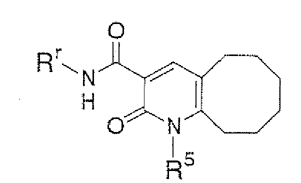 | | | |

| Compound No. | R^{r} | R⁵ | ¹H-NMR (CDCl₃) |
|---|---|---|---|
| 10-001 | 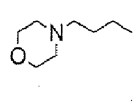 | nBu | 0.99 (d, *J* = 7.3 Hz, 3H), 1.22-1.53 (m, 6H), 1.62-1.86 (m, 6H), 2.36-2.42 (m, 6H), 2.64 (t, *J* = 6.0 Hz, 2H), 2.89 (t, *J* = 6.3 Hz, 2H), 3.48 (dt, *J* = 7.2, 6.9 Hz, 2H), 3.72 (t, *J* = 4.8 Hz, 4H), 4.05-4.14 (m, 2H), 8.29 (s, 1H), 10.1 (t, *J* = 5.4 Hz, 1H). |
| 10-002 | 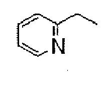 | nBu | 0.98 (d, *J* = 7.3 Hz, 3H), 1.34-1.54 (m, 6H), 1.62-1.83 (m, 6H), 2.65 (t, *J* = 6.0 Hz, 2H), 2.90 (t, *J* = 6.4 Hz, 2H), 4.05-4.20 (m, 2H), 4.80 (d, *J* = 5.5 Hz, 2H), 7.16 (m, 1H), 7.35 (d, *J* = 7.8 Hz, 1H), 7.64 (ddd, *J* = 7.8, 7.8, 1.8 Hz, 1H), 8.34 (s, 1H), 8.59 (dlike, 1H), 10.6 (t, *J* = 5.5 Hz, 1H). |
| 10-003 | 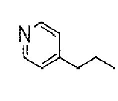 | nBu | 0.99 (d, *J* = 7.3 Hz, 3H), 1.34-1.53 (m, 6H), 1.60-1.90 (m, 6H), 2.64 (t, *J* = 6.0 Hz, 2H), 2.89 (t, *J* = 6.3 Hz, 2H), 2.94 (t, *J* = 7.5 Hz, 2H), 3.70 (dt, *J* = 7.5, 6.0 Hz, 2H), 4.03-4.14 (m, 2H), 7.18-7.20 (m, 2H), 8.29 (s, 1H), 8.50-8.52 (m, 2H), 10.1 (t, *J* = 6.0 Hz, 1H). |
| 10-004 | 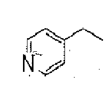 | nBu | 0.99 (d, *J* = 7.2 Hz, 3H), 1.34-1.54 (m, 6H), 1.62-1.85 (m, 6H), 2.66 (t, *J* = 6.0 Hz, 2H), 2.91 (t, *J* = 6.3 Hz, 2H), 4.12 (t, *J* = 7.3 Hz, 2H), 4.65 (d, *J* = 5.8 Hz, 2H), 7.28 (A2B2, *J* = 5.0 Hz, 2H), 8.53 (s, 1H), 8.54 (A2B2, *J* = 5.0 Hz, 2H), 10.5 (t, *J* = 5.8 Hz, 1H). |
| 10-005 | 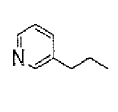 | nBu | 0.99 (d, *J* = 7.3 Hz, 3H), 1.34-1.54 (m, 6H), 1.60-1.82 (m, 6H), 2.64 (t, *J* = 6.1 Hz, 2H), 2.89 (t, *J* = 6.4 Hz, 2H), 2.94 (t, *J* = 7.5 Hz, 2H), 3.68 (dt, *J* = 7.5, 6.3 Hz, 2H), 4.04-4.14 (m, 2H), 7.23 (dd, *J* = 7.8, 4.6 Hz, 1H), 7.59 (ddd, *J* = 7.8, 2.1, 1.5 Hz, 1H), 8.29 (s, 1H), 8.47 (dd, *J* = 7.8, 1.5 Hz, 1H), 8.50 (d, *J* = 2.1 Hz, 1H), 10.1 (t, *J* = 6.3 Hz, 1H). |
| 10-006 | 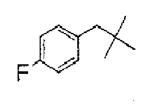 | nBu | 0.98 (d, *J* = 7.3 Hz, 3H), 1.34-1.53 (m, 6H), 1.41 (s, 6H), 1.61-1.80 (m, 6H), 2.63 (t, *J* = 6.1 Hz, 2H), 2.88 (t, *J* = 6.4 Hz, 2H), 3.16 (s, 2H), 4.06 (br.s, 2H), 6.88-6.94 (m, 2H), 7.11-7.16 (m, 2H), 8.32 (s, 1H), 9.83 (br.s, 1H). |

**Table 112**

| | | | |
|---|---|---|---|
| 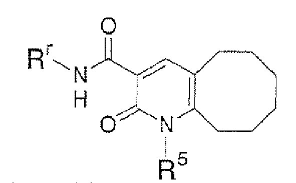 | | | |

| Compound No. | R^{r} | R⁵ | ¹H-NMR (CDCl₃) |
|---|---|---|---|
| 10-007 | 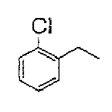 | nBu | 0.98 (d, *J* = 7.2 Hz, 3H), 1.38-1.49 (m, 6H), 1.51-1.80 (m, 6H), 2.64 (t, *J* = 6.4 Hz, 2H), 2.89 (t, *J* = 6.4 Hz, 2H), 4.10 (br.s, 2H), 4.73 (d, *J* = 5.8 Hz, 2H), 7.16-7,25 (m, 4H), 7.35 (m, 1H), 7.46 (m, 1H), 8.32 (s, 1H), 10.4 (br.s, 1H). |
| 10-008 | 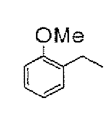 | nBu | 0.98 (d, *J* = 7.3 Hz, 3H), 1.37-1.48 (m, 6H), 1.51-1.79 (m, 6H), 2.63 (t, *J* = 6.4 Hz, 2H), 2.87 (t, *J* = 6.4 Hz, 2H), 3.88 (s, 3H), 4.09 (m, 2H), 4.65 (d, *J* = 5.8 Hz, 2H), 6.85-6.93 (m, 2H), 7.22 (dt, *J* = 7.6, 1.8 Hz, 1H), 7.35 (dd, *J* = 7.6, 1.8 Hz, 1H), 8.32 (s, 1H), 10.3 (br.s, 1H). |
| 10-009 | 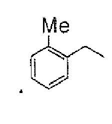 | nBu | 0.97 (d, *J* = 7.3 Hz, 3H), 1.38-1.50 (m, 6H), 1.60-1.80 (m, 6H), 2.38 (s, 3H), 2.64 (t, *J* = 6.1 Hz, 2H), 2.88 (t, *J* = 6.4 Hz, 2H), 4.08 (m, 2H), 4.63 (d, *J* = 5.5 Hz, 2H), 7.15-7.20 (m, 3H), 7.33 (m, 1H), 8.33 (s, 1H), 10.2 (br.s, 1H). |
| 10-010 | 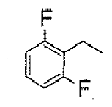 | nBu | 0.97 (d, *J* = 7.3 Hz, 3H), 1.36-1.50 (m, 6H), 1.61-1.78 (m, 6H), 2.64 (t, *J* = 6.1 Hz, 2H), 2.87 (t, *J* = 6.7 Hz, 2H), 4.08 (br.s, 2H), 4.72 (d, *J* = 5.5 Hz, 2H), 6.84-6.92 (m, 2H), 7.20 (m, 1H), 8.32 (s, 1H), 10.2 (br.s, 1H). |
| 10-011 | 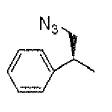 | nBu | 0.99 (t, *J* = 7.2 Hz, 3H), 1.34-1.54 (m, 4H), 1.47 (sextet, *J* = 7.2 Hz, 2H), 1.65-1.81 (m, 6H), 2.63 (t, *J* = 6.3 Hz, 2H), 2.89 (t, *J* = 6.3 Hz, 2H), 3.71 (d, *J* = 6.3 Hz, 2H), 4.11 (br t, *J* = 7.2 Hz, 2H), 5.43 (dt, *J* = 8.1 Hz, 6.0 Hz, 1H), 7.26-7.70 (m, 5H), 8.23 (s, 1H), 10.74 (d, *J* = 8.1 Hz, 1H). |
| 10-012 | 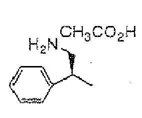 | nBu | 0.99 (t, *J* = 7.2 Hz, 3H), 1.34-1.56 (m, 4H), 1.47 (sextet, *J* = 7.2 Hz, 2H), 1.61-1.80 (m, 6H), 2.06 (s, 3H), 2.63 (t, *J* = 6.3 Hz, 2H), 2.89 (t, *J* = 6.3 Hz, 2H), 3.15 (d, *J* = 6.0 Hz, 2H), 4.12 (br t, *J* = 7.2 Hz, 2H), 5.25 (dt, *J* = 7.8 Hz, 6.0 Hz, 1H), 7.35-7.42 (m, 5H), 8.28 (s, 1H), 10.67 (d, *J* = 8.4 Hz, 1H). |

**Table 113**

| | | | |
|---|---|---|---|
| 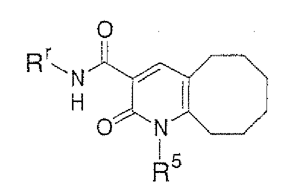 | | | |

| Compound No. | R^{r} | R⁵ | ¹H-NMR (CDCl₃) |
|---|---|---|---|
| 10-013 | 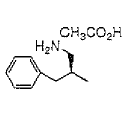 | nBu | 1.00 (t, *J* = 7.5 Hz, 3H), 1.34-1.55 (m, 4H), 1.47 (sextet, *J* = 7.5 Hz, 2H), 1.62-1.80 (m, 6H), 2.01 (s, 3H), 2.63 (t, *J* = 6.0 Hz, 2H), 2.78-3.06 (m, 4H), 2.88 (t, *J* = 6.0 Hz, 2H), 4.10 (br t, *J* = 7.5 Hz, 2H), 4.35-4.45 (m, 1H), 7.18-7.30 (m, 5H), 8.24 (s, 1H), 10.25 (d, *J* = 7.8 Hz, 1H). |
| 10-014 | 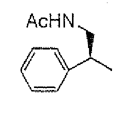 | nBu | 0.99 (t, *J* = 7.5 Hz, 3H), 1.36-1.55 (m, 4H), 1.46 (sextet, *J* = 7.5 Hz, 2H), 1.63-1.79 (m, 6H), 1.97 (s, 3H), 2.64 (t, *J* = 6.0 Hz, 2H), 2.89 (t, *J* = 6.0 Hz, 2H), 3.70-3.81 (m, 2H), 4.09 (br t, *J* = 7.5 Hz, 2H), 5.31-5.39 (m, 1H), 6.61 (br t, *J* = 4.5 Hz, 1H), 7.28-7.44 (m, 5H), 8.28 (s, 1H), 10.66 (d, *J* = 7.5 Hz, 1H). |
| 10-015 | 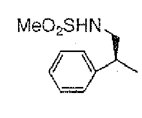 | nBu | 0.99 (t, *J* = 7.2 Hz, 3H), 1.38-1.57 (m, 4H), 1.47 (sextet, *J* = 7.2 Hz, 2H), 1.62-1.80 (m, 6H), 2.64 (t, *J* = 6.0 Hz, 2H), 2.90 (s, 3H), 2.90 (t, *J* = 6.0 Hz, 2H), 3.60 (d, *J* = 6.6 Hz, 2H), 4.10 (br t, *J* = 7.2 Hz, 2H), 5.38 (dt, *J* = 7.5 Hz, 6.0 Hz, 1H), 7.35-7.43 (m, 5H), 8.29 (s, 1H), 10.75 (d, *J* = 9.0 Hz, 1H). |
| 10-016 | 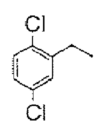 | nBu | 0.99 (d, *J* = 7.3 Hz, 3H), 1.34-1.54 (m, 6H), 1.62-1.82 (m, 6H), 2.65 (t, *J* = 6.0 Hz, 2H), 2.90 (t, *J* = 6.3 Hz, 2H), 4.06-4.17 (m, 2H), 4.68 (d, *J* = 6.4 Hz, 2H), 7.16 (dd, *J* = 8-5, 2.4 Hz, 1H), 7.28 (d, *J* = 8.5 Hz, 1H), 7.41 (d, *J* = 2.4 Hz, 1H), 8.32 (s, 1H), 10.5 (t, *J* = 6.4 Hz, 1H). |
| 10-017 | 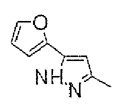 | nBu | 1.01 (t, *J* = 7.3 Hz, 3H), 1.35-1.57 (m, 6H), 1.65-1.85 (m, 6H), 2.69 (t, *J* = 6.0 Hz, 2H), 2.94 (t, *J* = 6.2 Hz, 2H), 4.08-4.20 (m, 2H), 6.33 (s, 1H), 6.48 (dd, *J* = 3.4, 1.8 Hz, 1H), 6.68 (d, *J* = 3.4 Hz, 1H), 7.46 (m, 1H), 8.35 (s, 1H), 12.8 (s, 1H). |

**Table 114**

| | | | |
|---|---|---|---|
| 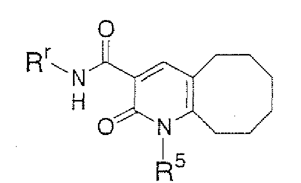 | | | |

| Compound No. | R^{r} | R⁵ | ¹H-NMR (CDCl₃) |
|---|---|---|---|
| 10-018 | 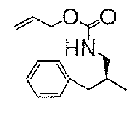 | nBu | 1.00 (t, *J* = 7.5 Hz, 3H), 1.37-1.57 (m, 4H), 1.47 (sextet, *J* = 7.5 Hz, 2H), 1.61-1.80 (m, 6H), 2.62 (t, *J* = 6.0 Hz, 2H), 2.88 (t, *J* = 6.0 Hz, 2H), 2.94 (t, *J* = 7.5 Hz, 2H), 3.23-3.33 (m, 1H), 3.45-3.55 (m, 1H), 4.09 (br t, *J* = 7.5 Hz, 2H), 4.40-4.50 (m, 1H), 4.53 (d, *J* = 6.0 Hz, 2H), 5.14-5.30 (m, 2H), 5.52 (br s, 1H), 5.82-5.96 (m, 1H), 7.17-7.28 (m, 5H), 8.25 (s, 1H), 10.18 (d, *J* = 7.5 Hz, 1H). |
| 10-019 | 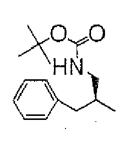 | nBu | 1.00 (t, *J* = 7.5 Hz, 3H), 1.40 (s, 9H), 1.41-1.55 (m, 4H), 1.47 (sextet, *J* = 7.5 Hz, 2H), 1.64-1.80 (m, 6H), 2.62 (t, *J* = 6.0 Hz, 2H), 2.88 (t, *J* = 6.0 Hz, 2H), 2.88-3.00 (m, 2H), 3.18-3.28 (m, 1H), 3.38-3.48 (m, 1H), 4.09 (br t, *J* = 7.5 Hz, 2H), 4.37-4.47 (m, 1H), 5.11 (br s, 1H), 7.22-7.32 (m, 5H), 8.25 (s, 1H), 10.13 (d, *J* = 7.8 Hz, 1H). |
| 10-020 | 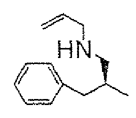 | nBu | 1.00 (t, *J* = 7.2 Hz, 3H), 1.36-1.81 (m, 12H), 2.62 (t, *J* = 6.0 Hz, 2H), 2.74-3.04 (m, 6H), 3.21-3.37 (m, 2H), 4.09 (br t, *J* = 7.2 Hz, 2H), 4.44-4.53 (m, 1H), 5.06-5.17 (m, 2H), 5.08-5.95 (m, 1H), 7.18-7.29 (m, 5H), 8.25 (s, 1H), 10.17 (br s, 1H). |
| 10-021 | 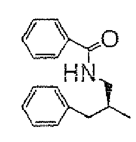 | nBu | 1.00 (t, *J* = 7.2 Hz, 3H), 1.36-1.52 (m, 4H), 1.47 (sextet, *J* = 7.2 Hz, 2H), 1.61-1.79 (m, 6H), 2.63 (t, *J* = 6.0 Hz, 2H), 2.88 (t, *J* = 6.0 Hz, 2H), 2.95-3.11 (m, 2H), 3.49-3.59 (m, 1H), 3.68-3.76 (m, 1H), 4.10 (br t, *J* = 7.2 Hz, 2H), 4.51-4.64 (m, 1H), 7.17-7.85 (m, 10H), 7.92 (br s, 1H), 8.27 (s, 1H), 10.45 (d, *J* = 7.5 Hz, 1H). |
| 10-022 | 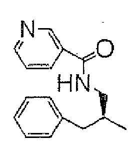 | nBu t, | 1.00 (t, *J* = 7.5 Hz, 3H), 1.36-1.57 (m, 4H), 1.48 (sextet, *J* = 7.5 Hz, 2H), 1.60-1.80 (m, 6H), 2.65 (t, *J* = 6.0 Hz, 2H), 2.89 (t, *J* = 6.0 Hz, 2H), 2.96-3.12 (m, 2H), 3.46-3.56 (m, 1H), 3.70-3.79 (m, 1H), 4.11 (br *J* = 7.5 Hz, 2H), 4.51-4.63 (m, 1H), 7.17-7.39 (m, 6H), 8.17 (d, *J* = 8.1 Hz, 1H), 8.28 (s, 1H), 8.37 (br s, 1H), 8.69 (d, *J* = 3.9 Hz, 1H), 9.07 (br s, 1H), 10.52 (d, *J* = 7.2 Hz, 1H). |

**Table 115**

| | | | |
|---|---|---|---|
| 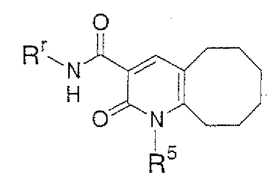 | | | |

| Compound No. | R^{r} | R⁵ | ¹H-NMR (CDCl₃) |
|---|---|---|---|
| 10-023 | 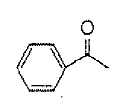 | nBu = | 1.01 (t, *J* = 7.5 Hz, 3H), 1.39-1.59 (m, 4H), 1.50 (sextet, *J* = 7.5 Hz, 2H), 1.63-1.85 (m, 6H), 2.68 (t, *J* = 6.0 Hz, 2H), 2.94 (t, *J* 6.0 Hz, 2H), 4.17 (br t, *J* = 7.5 Hz, 2H), 7.46-7.61 (m, 3H), 8.11 (d, *J* = 7.2 Hz, 2H), 8.42 (s, 1H), 13.76 (br s, 1H). |
| 10-024 | H- | nBu | 0.99 (t, *J* = 7.2 Hz, 3H), 1.37-1.56 (m, 4H), 1.47 (sextet, *J* = 7.2 Hz, 2H), 1.63-1.81 (m, 6H), 2.64 (t, *J* = 6.0 Hz, 2H), 2.90 (t, *J* = 6.0 Hz, 2H), 4.11 (t, *J* = 7.2 Hz, 2H), 5.69 (br s, 1H), 8.30 (s, 1H), 9.63 (br s, 1H). |
| 10-025 | 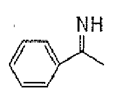 | nBu | 1.00 (t, *J* = 7.5 Hz, 3H), 1.37-1.84 (m, 12H), 2.69 (t, *J* = 6.0 Hz, 2H), 2.94 (t, *J* = 6.0 Hz, 2H), 4.13 (br t, *J* = 7.5 Hz, 2H), 7.50-7.53 (m, 3H), 8.00-8.08 (m, 2H), 8.37 (s, 1H), 13.04 (br s, 1H). |
| 10-026 | 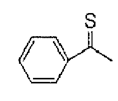 | nBu | 1.00 (t, *J* = 7.2 Hz, 3H), 1.38-1.57 (m, 4H), 1.48 (sextet, *J* = 7.2 Hz, 2H), 1.63-1.87 (m, 6H), 2.68 (t, *J* = 6.0 Hz, 2H), 2.94 (t, *J* = 6.0 Hz, 2H), 4.16 (br t, *J* = 7.2 Hz, 2H), 7.40-7.56 (m, 3H), 7.95 (d, *J* = 7.2 Hz, 2H), 8.42 (s, 1H), 14.37 (br s, 1H). |
| 10-027 | 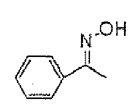 | nBu | 0.99 (t, *J* = 7.2 Hz, 3H), 1.38-1.56 (m, 4H), 1.47 (sextet, *J* = 7.2 Hz, 2H), 1.63-1.82 (m, 6H), 2.62 (t, *J* = 6.0 Hz, 2H), 2.90 (t, *J* = 6.0 Hz, 2H), 4.11 (br t, *J* = 7.2 Hz, 2H), 6.12 (br s, 1H), 7.39-7.51 (m, 3H), 7.75-7.79 (m, 2H), 8.12 (s, 1H). |
| 10-028 | 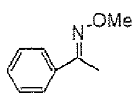 | nBu | 1.01 (t, *J* = 7.5 Hz, 3H), 1.37-1.58 (m, 4H), 1.48 (sextet, *J* = 7.2 Hz, 2H), 1.60-1.82 (m, 6H), 2.60 (t, *J* = 6.0 Hz, 2H), 2.91 (t, *J* = 6.0 Hz, 2H), 4.06 (s, 3H), 4.17 (br t, *J* = 7.5 Hz, 2H), 7.36-7.56 (m, 3H), 7.52-7.57 (m, 2H), 8.23 (s, 1H), 12.43 (br s, 1H). |
| 10-029 | 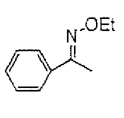 | nBu | 1.01 (t, *J* = 7.5 Hz, 3H), 1.37-1.55 (m, 4H), 1.40 (t, *J* = 6.9 Hz, 3H), 1.48 (sextet, *J* = 7.5 Hz, 2H), 1.60-1.83 (m, 6H), 2.60 (t, *J* = 6.0 Hz, 2H), 2.91 (t, *J* = 6.0 Hz, 2H), 4.17 (br t, *J* = 7.5 Hz, 2H), 4.30 (q, *J* = 6.9 Hz, 2H), 7.32-7.40 (m, 3H), 7.51-7.61 (m, 2H), 8.23 (s, 1H), 12.44 (br s, 1H). |

**Table 116**

| | | | |
|---|---|---|---|
| 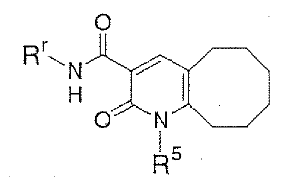 | | | |

| Compound No. | R^{r} | R⁵ | ¹H-NMR (CDCl₃) |
|---|---|---|---|
| 10-030 | 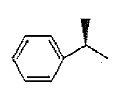 | 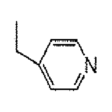 | 1.37-1.53 (m, 4H), 1.56 (d, *J* = 7.2 Hz, 3H), 1.61-1.79 (m, 4H), 2.65 (t, *J* = 6.0 Hz, 2H), 2.72 (t, *J* = 6.3 Hz, 2H), 5.30 (quint, *J* = 6.9 Hz, 1H), 5.42 (br s, 2H), 6.98 (d, *J* = 5.1 Hz, 2H), 7,25-7.41 (m, 5H), 8.43 (s, 1H), 8.58 (brs, 2H), 10.11 (d, *J* = 7.8 Hz, 1H). |
| 10-031 | 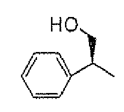 | 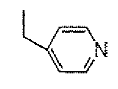 | 1.37-1.80 (m, 8H), 2.67 (t, *J* = 6.0 Hz, 2H), 2.74 (t, *J* = 6.0 Hz, 2H), 3.07 (br t, *J* = 6.0 Hz, 1H), 3.94 (t, *J* = 6.0 Hz, 2H), 5.30 (q, *J* = 6.9 Hz, 1H), 5.45 (br s, 2H), 6.99 (d, *J* = 5.4 Hz, 2H), 7.29-7.42 (m, 5H), 8.44 (s, 1H), 8.57 (d, *J* = 5.4 Hz, 2H), 10.50 (d, *J* = 7.5 Hz, 1H). |
| 10-032 | 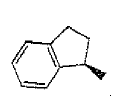 | nBu | 0.96 (t, *J* = 7.5 Hz, 3H), 1.37-1.58 (m, 6H), 1.60-1.80 (m, 6H), 1.92-2.05 (m, 1H), 2.62-2.73 (m, 1H), 2.66 (t, *J* = 6.0 Hz, 2H), 2.84-2.95 (m, 1H), 2.89 (t, *J* = 6.0 Hz, 2H), 2.99-3.09 (m, 1H), 3.95-4.16 (m, 2H), 5.68 (q, *J* = 7.5 Hz, 1H), 7.15-7.29 (m, 3H), 7.36-7.40 (m, 1H), 8.37 (s, 1H), 10.25 (d. *J* = 8.4 Hz, 1H). |
| 10-033 | 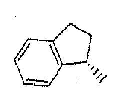 | nBu | 0.96 (t, *J* = 7.5 Hz, 3H), 1.35-1.56 (m, 6H), 1.59-1.79 (m, 6H), 1.91-2.04 (m, 1H), 2.62-2.72 (m, 1H), 2.66 (t, *J* = 6.0 Hz, 2H), 2.84-2.95 (m, 1H), 2.89 (t, *J* = 6.0 Hz, 2H), 2.99-3.09 (m, 1H), 3.98-4.18 (m, 2H), 5.68 (q, *J* = 7.5 Hz, 1H), 7.15-7.26 (m, 3H), 7.36-7.40 (m, 1H), 8.37 (s, 1H), 10.25 (d, *J* = 8.1 Hz, 1H). |
| 10-034 | 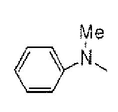 | nBu | 1.00 (t, *J* = 7.5 Hz, 3H), 1.38-1.56 (m, 4H), 1.48 (sextet, *J* = 7.5 Hz, 2H), 1.63-1.83 (m, 6H), 2.65 (t, *J* = 6.0 Hz, 2H), 2.91 (t, *J* = 6.0 Hz, 2H), 3.29 (s, 3H), 4.13 (t, *J* = 7.5 Hz, 2H), 6.82 (t, *J* = 7.5 Hz, 1H), 6.90 (d, *J* = 8.7 Hz, 2H), 7.22-7.27 (m, 2H), 8.34 (s, 1H), 11.48 (br s, 1H). |
| 10-035 | 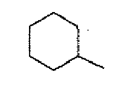 | nBu | 0.99 (t, *J* = 7.2 Hz, 3H), 1.20-1.53 (m, 12H), 1.59-1.80 (m, 8H), 1.95-2.01 (m, 2H), 2.63 (t, *J* = 6.0 Hz, 2H), 2.88 (t, *J* = 6.0 Hz, 2H), 3.91-4.02 (m, 1H), 4.09 (br t, *J* = 7.2 Hz, 2H), 8.30 (s, 1H), 9.88 (d, *J* = 7.5 Hz, 1H). |
| 10-036 | 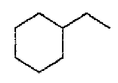 | nBu | 0.99 (t, *J* = 7.2 Hz, 3H), 1.13-1.30 (m, 2H), 1.36-1.83 (m, 21H), 2.66 (t, *J* = 6.0 Hz, 2H), 2.88 (t, *J* = 6.0 Hz, 2H), 3.28 (t, *J* = 6.0 Hz, 2H), 4.10 (br t, *J* = 7.5 Hz, 2H), 8.31 (s, 1H), 9.98 (br s, 1H). |

**Table 117**

| | | | |
|---|---|---|---|
| 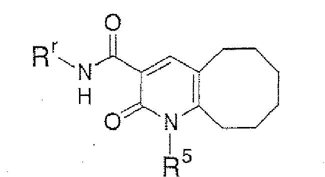 | | | |

| Compound No. | R^{r} | R⁵ | ¹H-NMR (CDCl₃) |
|---|---|---|---|
| 10-037 | Me | nBu | 0.99 (t, *J* = 7.2 Hz, 3H), 1.36-1.53 (m, 6H), 1.62-1.81 (m, 6H), 2.64 (t, *J* = 6.0 Hz, 2H), 2.89 (t, *J* = 6.0 Hz, 2H), 2.98 (d, *J* = 3.6 Hz, 3H), 4.10 (br t, *J* = 7.2 Hz, 2H), 8.31 (s, 1H), 9.85 (br s, 1H). |
| 10-038 | Et | nBu | 0.99 (t, *J* = 7.2 Hz, 3H), 1.24 (t, *J* = 7.2 Hz, 3H), 1.34-1.54 (m, 4H), 1.47 (sextet, *J* = 7.2 Hz, 2H), 1.63-1.80 (m, 6H), 2.64 (t, *J* = 6.0 Hz, 2H), 2.89 (t, *J* = 6.0 Hz, 2H), 3.42-3.51 (m, 2H), 4.10 (br t, *J* = 7.2 Hz, 2H), 8.31 (s, 1H), 9.90 (br s, 1H). |
| 10-039 | iPr | nBu | 0.99 (t, *J* = 7.2 Hz, 3H), 1.26 (d, *J* = 6.9 Hz, 6H), 1.34-1.52 (m, 4H), 1.47 (sextet, *J* = 7.2 Hz, 2H), 1.60-1.80 (m, 6H), 2.65 (t, *J* = 6.0 Hz, 2H), 2.88 (t, *J* = 6.0 Hz, 2H), 4.09 (br t, *J* = 7.2 Hz, 2H), 4.25 (sextet, *J* = 6.6 Hz, 1H), 8.31 (s, 1H), 9.82 (br s, 1H). |
| 10-040 | tBu | nBu | 0.99 (t, *J* = 7.5 Hz, 3H), 1.35-1.56 (m, 6H), 1.47 (s, 9H). 1.61-1.79 (m, 6H), 2.62 (t, *J* = 6.0 Hz, 2H), 2.88 (t, *J* = 6.0 Hz, 2H), 4.09 (br t, *J* = 7.5 Hz, 2H), 8.30 (s, 1H), 9.92 (br s, 1H). |
| 10-041 | 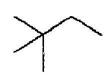 | nBu | 0.99 (t, *J* = 7.5 Hz, 3H), 1.01 (s, 9H), 1.36-1.57 (m, 6H), 1.62-1.80 (m, 6H), 2.64 (t, *J* = 6.0 Hz, 2H), 2.89 (t, *J* = 6.0 Hz, 2H), 3.27 (t, *J* = 6.0 Hz, 2H), 4.12 (br t, *J* = 7.5 Hz, 2H), 8.32 (s, 1H), 1010 (br s, 1H). |
| 10-042 | 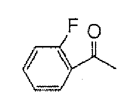 | nBu | 0.99 (t, *J* = 7.5 Hz, 3H), 1.38-1.54 (m, 6H), 1.62-1.83 (m, 6H), 2.67 (t, *J* = 6.0 Hz, 2H), 2.92 (t, *J* = 6,0 Hz, 2H), 4.14 (br t, *J* = 7.5 Hz, 2H), 7.25-7.30 (m, 2H), 7.47-7.56 (m, 1H), 7.96 (td, *J* = 7.8 Hz, 1.5 Hz, 1H), 8.38 (s, 1H), 13.37 (br s, 1H). |
| 10-043 | 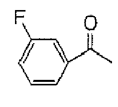 | nBu | 1.02 (t, *J* = 7.2 Hz, 3H), 1.40-1.58 (m, 6H), 1.67-1.85 (m, 6H), 2.69 (t, *J* = 6.0 Hz, 2H), 2.94 (t, *J* = 6.0 Hz, 2H), 4.18 (br t, *J* = 7.2 Hz, 2H), 7.25-7.32 (m, 1H), 7.47-7.55 (m, 1H), 7.80 (dt, *J* = 9.6 Hz, 2.4 Hz, 1H), 7.89 (t, *J* = 7.8 Hz, 1H), 8.41 (s, 1H), 13.84 (br s, 1H). |
| 10-044 | 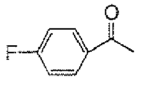 | nBu | 1.01 (t, *J* = 7.5 Hz, 3H), 1.39-1.62 (m, 6H), 1.65-1.89 (m, 6H), 2.69 (t, *J* = 6.0 Hz, 2H), 2.94 (t, *J* = 6.0 Hz, 2H), 4.17 (br t, *J* = 7.5 Hz, 2H), 7.20 (t, *J* = 9.0 Hz, 2H), 8.11-8.16 (m, 2H), 8.42 (s, 1H), 13.79 (br s, 1H). |

**Table 118**

| | | | |
|---|---|---|---|
| 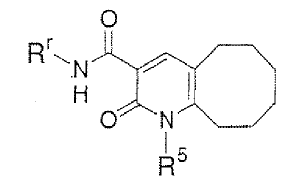 | | | |

| Compound No. | R^{r} | R⁵ | ¹H-NMR (CDCl₃) |
|---|---|---|---|
| 10-045 | 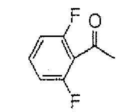 | nBu | 0.99 (t, *J* = 7.5. Hz, 3H), 1.38-1.54 (m, 6H), 1.62-1.83 (m, 6H), 2.63 (t, *J* = 6.0 Hz, 2H), 2.91 (t, *J* = 6.0 Hz, 2H), 4.12 (br t, *J* = 7.5 Hz, 2H), 6.96 (t, *J* = 7.8 Hz, 2H), 7.34-7.44 (m, 1H), 8.29 (s, 1H), 13.18 (br s, 1H). |
| 10-046 | 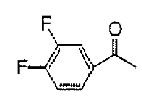 | nBu | 1.02 (t, *J* = 7.2 Hz, 3H), 1.39-1.56 (m, 6H), 1.67-1.85 (m, 6H), 2.69 (t, *J* = 6.0 Hz, 2H), 2.94 (t, *J* = 6.0 Hz, 2H), 4.17 (br t, *J* = 7.2 Hz, 2H), 7.32 (dt, *J* = 9.0 Hz, 1.2 Hz, 1H), 7.86-8.01 (m, 2H), 8.41 (s, 1H), 13.88 (br s, 1H). |
| 10-047 | 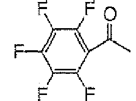 | nBu | 1.02 (t; *J* = 7.2 Hz, 3H), 1.42-1.57 (m, 6H), 1.63-1.82 (m, 6H), 2.66 (t, *J* = 6.0 Hz, 2H), 2.95 (t, *J* = 6.0 Hz, 2H), 4.15 (br t, *J* = 7.2 Hz, 2H), 8.25 (s, 1H), 13.37 (br s, 1H). |
| 10-048 | 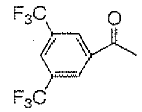 | nBu | 1.03 (t, *J* = 7.5 Hz, 3H), 1.40-1.55 (m, 6H), 1.68-1.87 (m, 6H), 2.70 (t, *J* = 6.0 Hz, 2H), 2.96 (t, *J* = 6.0 Hz, 2H), 4.20 (br t, *J* = 7.5 Hz, 2H), 8.08 (s, 1H), 8.41 (s, 1H), 8.57 (s, 2H), 14.30 (br s, 1H). |
| 10-049 | 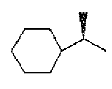 | nBu | 0.99 (t, *J* = 7.5 Hz, 3H), 1.01-1.34 (m, 6H), 1.18 (d, *J* = 6.9 Hz, 3H), 1.37-1.57 (m, 8H), 1.62-1.88 (m, 9H), 2.63 (t, *J* = 6.0 Hz, 2H), 2.88 (t, *J* = 6.0 Hz, 2H), 3.99-4.10 (m, 2H), 4.05-4.21 (m, 1H), 8.30 (s, 1H), 9.88 (d, *J* = 9.0 Hz, 1H). |
| 10-050 | 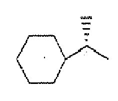 | nBu | 0.99 (t, *J* = 7.5 Hz, 3H), 1.01-1.32 (m, 6H), 1.18 (d, *J* = 6.6 Hz, 3H), 1.34-1.52 (m, 8H), 1.62-1.86 (m, 9H), 2.63 (t, *J* = 6.0 Hz, 2H), 2.88 (t, *J* = 6.0 Hz 2H), 4.01-4.11 (m, 2H), 4.05-4.22 (m, 1H), 8.30 (s, 1H), 9.88 (d, *J* = 8.4 Hz, 1H). |
| 10-051 | 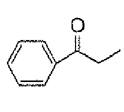 | nBu | 0.99 (t, *J* = 7.2 Hz, 3H), 1.36-1.55 (m, 4H), 1.48 (sextet, *J* = 7.2 Hz, 2H), 1.65-1.82 (m, 6H), 2.65 (t, *J* = 6.0 Hz, 2H), 2.90 (t, *J* = 6.0 Hz, 2H), 4.16 (br t, *J* = 7.2 Hz, 2H), 4.97 (d, *J* = 4.2 Hz, 2H), 7.47-7.63 (m, 3H), 8.02-8.06 (m, 2H), 8.31 (s, 1H), 10.81 (br s, 1H). |
| 10-052 | 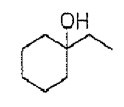 | nBu | 0.99 (t, *J* = 7.5 Hz, 3H), 1.28-1.55 (m, 12H), 1.59-1.80 (m, 10H), 1.99 (br s, 1H), 2.64 (t, *J* = 6.0 Hz, 2H), 2.89 (t, *J* = 6.0 Hz, 2H), 3.47 (d, *J* = 6.3 Hz, 2H), 4.11 (br t, *J* = 7.5 Hz, 2H), 8.30 (s, 1H), 10.27 (br s, 1H). |

**Table 119**

| | | | |
|---|---|---|---|
| 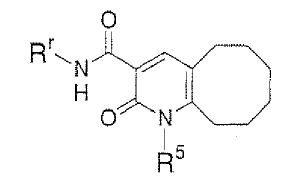 | | | |

| Compound No. | R^{r} | R⁵ | ¹H-NMR (CDCl₃) |
|---|---|---|---|
| 10-053 | 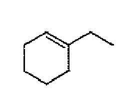 | nBu | 0.99 (t, *J* = 7.5 Hz, 3H), 1.35-1.80 (m, 14H), 1.46 (sextet, *J* = 7.5 Hz, 2H), 2.00 (br s, 4H), 2.64 (t, *J* = 6.0 Hz, 2H), 2.88 (t, *J* = 6.0 Hz, 2H), 3.94 (d, *J*= 6.0 Hz, 2H), 4.10 (br t, *J* = 7.5 Hz, 2H), 5.63 (br s, 1H), 8.3 (s, 1H), 9.97 (br s, 1H). |
| 10-054 | 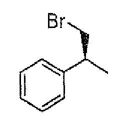 | nBu | 1.00 (t, *J* = 7.2 Hz, 3H), 1.35-1.79 (m, 10H), 1.47 (sextet, *J* = 7.2 Hz, 2H), 2.64 (t, *J* = 6.0 Hz, 2H), 2.90 (t, *J* = 6.0 Hz, 2H), 3.79 (d, *J* = 6.0 Hz, 2H), 4.13 (br t, *J* = 7.2 Hz, 2H), 5.52-5.59 (m, 1H), 7.26-7.45 (m, 5H), 8.32 (br s, 1H), 10.80 (br s, 1H). |
| 10-055 | 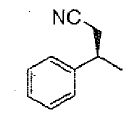 | nBu | 0.98 (t, *J* = 7.5 Hz, 3H), 1.36-1.79 (m, 10H), 1.45 (sextet, *J* = 7.5 Hz, 2H), 2.64 (t, *J* = 6.0 Hz, 2H), 2.89 (t, *J* = 6.0 Hz, 2H), 3.03 (dd, *J* = 6.0 Hz, 2.4 Hz, 2H), 4.10 (br t, *J* = 7.5 Hz, 2H), 5.47 (q, *J* = 6.6 Hz, 1H), 7.31-7.50 (m, 5H), 8.28 (s, 1H), 10.82 (d, *J* = 7.5 Hz, 1H). |
| 10-056 | 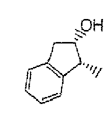 | nBu | 0.96 (t, *J* = 7.5 Hz, 3H), 1.38-1.79 (m, 12H), 2.66 (t, *J* = 6.0 Hz, 2H), 2.89 (t, *J* = 6.0 Hz, 2H), 3.04, 3.21 (ABx, *J* = 16.2 Hz, 3.0 Hz, 2H), 4.07 (br t, *J* = 7.5 Hz, 2H), 4.74-4.80 (m, 1H), 5.56 (dd, *J* = 7.2 Hz, 5.1 Hz, 1H), 7.20-7.30 (m, 3H), 7.34-7.39 (m, 1H), 8.35 (s, 1H), 0.43 (d, *J* = 7.2 Hz, 1H). |
| 10-057 | 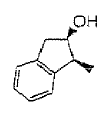 | nBu | 0.96 (t, *J* = 7.5 Hz, 3H), 1.35-1.81 (m, 12H), 2.66 (t, *J* = 6.0 Hz, 2H), 2.90 (t, *J* = 6.0 Hz, 2H), 3.04, 3.21 (ABx, *J* = 16.2 Hz, 3.0 Hz, 2H), 4.07 (br t, *J* = 7.5 Hz, 2H), 4.77 (sextet, *J* = 3.0 Hz, 1H), 5.56 (dd, *J* = 7.2 Hz, 5.1 Hz, 1H), 7.23-7.32 (m, 3H), 7.35-7.39 (m, 1H), 8.35 (s, 1H), 10.44 (d, *J* = 7.5 Hz, 1H). |
| 10-058 | 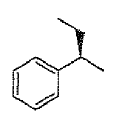 | nBu | 0.96 (t, *J* = 7.5 Hz, 3H), 0.99 (t, *J* = 7.5 Hz, 3H), 1.35-1.53 (m, 4H), 1.46 (sextet, *J* = 7.5 Hz, 2H), 1.64-1.79 (m, 6H), 1.85-1.98 (m, 2H), 2.61 (t, *J* = 6.0 Hz, 2H), 2.88 (t, *J* = 6.0 Hz, 2H), 4.11 (br t, *J* = 7.5 Hz, 2H), 5.08 (q, *J* = 7.5 Hz, 1H), 7.18-7.40 (m, 5H), 8.27 (s, 1H), 10.40 (d, *J* = 7.8 Hz, 1H). |

**Table 120**

| | | | |
|---|---|---|---|
| 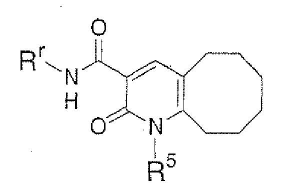 | | | |

| Compound No. | R^{r} | R⁵ | ¹H-NMR (CDCl₃) |
|---|---|---|---|
| 10-059 | 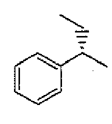 | nBu | 0.96 (t, *J* = 7.5 Hz, 3H), 0.99 (t, *J* = 7.5 Hz, 3H), 1.35-1.52 (m, 4H), 1.46 (sextet, *J* = 7.5 Hz, 2H), 1.61-1.79 (m, 6H), 1.84-1.98 (m, 2H), 2.61 (t, *J* = 6.0 Hz, 2H), 2.88 (t, *J* = 6.0 Hz, 2H), 4.10 (br t, *J* = 7.5 Hz, 2H), 5.08 (q, *J* = 7.5 Hz, 1H), 7.18-7.40 (m, 5H), 8.27 (s, 1H), 10.40 (d, *J* = 7.8 Hz, 1H). |
| 10-060 | 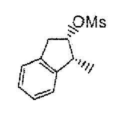 | nBu | 0.96 (t, *J* = 7.2 Hz, 3H), 1.35-1.56 (m, 4H), 1.42 (sextet, *J* = 7.2 Hz, 2H), 1.59-1.80 (m, 6H), 2.67 (t, *J* = 6.0 Hz, 2H), 2.90 (t, *J* = 6.0 Hz, 2H), 3.04 (s, 3H), 3.28-3.43 (m, 2H), 3.97-4.09 (m, 2H), 5.58 (td, *J* = 4.5 Hz, 1.5 Hz, 1H), 5.90 (dd, *J* = 7.5 Hz, 4.5 Hz, 1H), 7.18-7.35 (m, 4H), 8.35 (s, 1H), 10.64 (d, *J* = 8.4 Hz, 1H). |
| 10-061 | 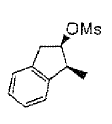 | nBu | 0.96 (t, *J* = 7.5 Hz, 3H), 1.35-1.56 (m, 4H), 1.42 (sextet, *J* = 7.5 Hz, 2H), 1.61-1.81 (m, 6H), 2.67 (t, *J* = 6.0 Hz, 2H), 2.90 (t, *J* = 6.0 Hz, 2H), 3.04 (s, 3H), 3.28-3.44 (m, 2H). 3.92-4.09 (m, 2H), 5.58 (td, *J* = 4.5 Hz, 1.5 Hz, 1H), 5.90 (dd, *J* = 7.5 Hz, 4.5 Hz, 1H), 7.20-7.35 (m, 4H), 8.35 (s, 1H), 10.64 (d, *J* = 8.4 Hz, 1H). |
| 10-062 | 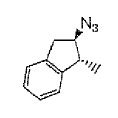 | nBu | 0.97 (t, *J* = 7.2 Hz, 3H), 1.36-1.54 (m, 6H), 1.61-1.80 (m, 6H), 2.66 (t, *J* = 6.0 Hz, 2H), 2.90 (t, *J* = 6.0 Hz, 2H), 2.95, 3.34 (ABx, *J* = 7.8 Hz, 2H), 3.98-4.10 (m, 2H), 4.23 (q, *J* = 7.2 Hz, 1H), 5.65 (t, *J* = 7.2 Hz, 1H), 7.19-7.32 (m, 4H). 8.39 (s, 1H), 3.0.36 (d, *J* = 8.4 Hz, 1H). |
| 10-063 | 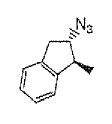 | nBu | 0.97 (t, *J* = 7.5 Hz, 3H), 1.36-1.54 (m, 6H), 1.61-1.81 (m, 6H), 2.66 (t, *J* = 6.0 Hz, 2H), 2.90 (t, *J* = 6.0 Hz, 2H), 2.95, 3.34 (ABx, *J* = 7.5 Hz, 2H), 3.98-4.10 (m, 2H), 4.23 (q, *J* = 7.2 Hz, 1H), 5.65 (t, *J* = 7.2 Hz, 1H), 7.19-7.32 (m, 4H), 8.39 (s, 1H), 10.36 (d, *J* = 8.4 Hz, 1H). |
| 10-064 | 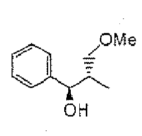 | nBu | 0.99 (t, *J* = 7.2 Hz, 3H), 1.36-1.52 (m, 6H), 1.63-1.80 (m, 6H), 1.99 (br s, 1H), 2.62 (t, *J* = 6.0 Hz, 2H), 2.88 (t, *J* = 6.0 Hz, 2H), 3.36 (s, 3H), 3.45, 3.58 (ABx, *J* = 5.4 Hz, 4.2 Hz, 2H), 4.02-4.21 (m, 2H), 4.33-4.41 (m, 1H), 5.03 (d, *J* = 5.1 Hz, 1H), 7.26-7.35 (m, 3H), 7.42-7.46 (m, 2H), 8.25 (s, 1H), 10.62 (d, *J* = 7.8 Hz, 1H). |

**Table 121**

| | | | |
|---|---|---|---|
| 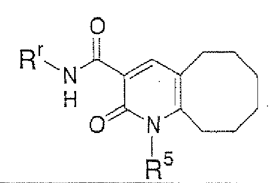 | | | |

| Compound No. | R^{r} | R⁵ | 1H-NMR (CDCl₃) |
|---|---|---|---|
| 10-065 | 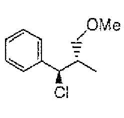 | Bu | 1.00 (t, *J* = 7.2 Hz, 3H), 1.35-1.53 (m, 6H), 1.61-1.80 (m, 6H), 2.62 (t, *J* = 6.0 Hz, 2H), 2.89 (t, *J* = 6.0 Hz, 2H), 3.31, 3.57 (ABx, *J* = 5.4 Hz. 4.2 Hz, 2H), 3.34 (s, 3H), 4.12 (br t, *J* = 7.2 Hz, 2H), 4.76-4.85 (m, 1H), 5.36 (d, *J* = 5.4 Hz, 1H), 7.26-7.35 (m, 3H), 7.45-7.49 (m, 2H), 8.24 (s, 1H), 10.53 (d, *J* = 8.1 Hz, 1H). |
| 10-066 | 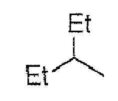 | Bu | 0.95 (t, *J* = 7.5 Hz, 3H×2), 0.99 (t, *J* = 7.5 Hz, 3H), 1.35-1.79 (m, 16H), 2.63 (t, *J* = 6.0 Hz, 2H), 2.88 (t, *J* = 6.0 Hz, 2H), 3.92-4.05 (m, 1H), 4.10 (br t, *J* = 7.2 Hz, 2H), 8.31 (s, 1H), 9.76 (d, *J* = 7.5 Hz, 1H). |
| 10-067 | 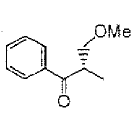 | Bu | 0.98 (t, *J* = 7.5 Hz, 3H), 1.34-1.51 (m, 6H), 1.61-1.79 (m, 6H), 2.62 (t, *J* = 6.0 Hz, 2H), 2.88 (t, *J* = 6.0 Hz, 2H), 3.31, (s, 3H), 3.79-3.90 (m, 2H), 4.11 (br t, *J* = 7.5 Hz, 2H), 5.87-5.94 (m, 1H), 7.45-7.62 (m, 3H), 8.05-8.09 (m, 2H), 8.26 (s, 1H), 10.80 (d, *J* = 7.8 Hz, 1H). |
| 10-068 | 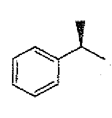 | 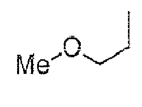 | 1.35 (quint, *J* = 6.0 Hz, 2H), 1.48 (quint, *J* = 6.0 Hz, 2H), 1.58 (d, *J* = 6.9 Hz, 3H), 1.61-1.75 (m, 4H), 2.64 (t, *J* = 6.0 Hz, 2H), 3.04 (t, *J* = 6.0 Hz, 2H), 3.31 (s, 3H), 3.70 (t, *J* = 5.4 Hz, 2H), 4.26-4.42 (m, 2H), 5.31 (quint, *J* = 7.2 Hz, 1H), 7.22-7.44 (m, 5H), 8.32 (s, 1H), 10.29 (d, *J* = 7.8 Hz, 1H). |
| 10-069 | 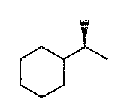 | 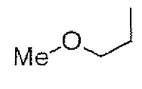 | 0.98-1.54 (m, 8H), 1.18 (d, *J* = 6.6 Hz, 3H), 1.61-1.85 (m, 11H), 2.65 (t, *J* = 6.0 Hz, 2H), 3.04 (t, *J* = 6.0 Hz, 2H), 3.31 (s, 3H), 3.70 (t, *J* = 6.0 Hz, 2H), 4.00-4.14 (m, 1H), 4.25-4.43 (m, 2H), 8.33 (s, 1H), 9.81 (d, *J* = 8.7 Hz, 1H). |
| 10-070 | 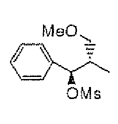 | nBu | 1.00 (t, *J* = 7.2 Hz, 3H), 1.34-1.53 (m, 6H), 1.63-1.80 (m, 6H), 2.62 (t, *J* = 6.0 Hz, 2H), 2.83(s, 3H), 2.88 (t, *J* = 6.0 Hz, 2H), 3.22, 3.50 (ABx, *J* = 4.8 Hz, 2H), 3.32 (s, 3H), 4.02-4.21 (m, 2H), 4.68-4.78 (m, 1H), 5.86 (d, *J* = 6.9 Hz, 1H), 7.32-7.49 (m, 5H), 8.23 (s, 1H), 10.50 (d, *J* = 9.6 Hz, 1H). |

**Table 122**

| | | | |
|---|---|---|---|
| 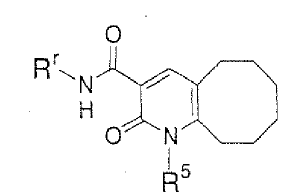 | | | |

| Compound No. | R^{r} | R⁵ | ¹H-NMR (CDCl₃) |
|---|---|---|---|
| 10-071 | 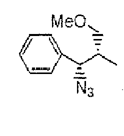 | nBu | 0.99 (t, *J* = 7.2 Hz, 3H), 1.33-1.50 (m, 6H), 1.62-1.79 (m, 6H), 2.60 (t, *J* = 6.0 Hz, 2H), 2.86 (t, *J* = 6.0 Hz, 2H), 3.38, 3.68 (ABx, *J* = 4.8 Hz, 2H), 3.36 (s, 3H), 4.01-4.19 (m, 2H), 4.60-4.69 (m, 1H), 5.01 (d, *J* = 7.2 Hz, 1H), 7.28-7.45 (m, 5H), 8.20 (s, 1H), 10.25 (d, *J* = 7.2 Hz, 1H). |
| 10-072 | 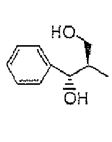 | nBu | 0.99 (t, *J* = 7.2 Hz, 3H), 1.35-1.52 (m, 6H), 1.62-1.80 (m, 6H), 2.63 (t, *J* = 6.0 Hz, 2H), 2.89 (t, *J* = 6.0 Hz, 2H), 3.77, 3.87 (ABx, *J* = 6.6 Hz, 4.8 Hz, 2H), 4.11 (br t, *J* = 7.2 Hz, 2H), 4.25 (quint, *J* = 4.8 Hz, 1H), 5.07 (d, *J* = 6.0 Hz, 1H), 7.26-7.36 (m, 3H), 7.44-7.48 (m, 2H), 8.27 (s, 1H), 10.70 (d, *J* = 5.4 Hz, 1H). |
| 10-073 | 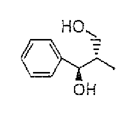 | nBu | 0.99 (t, *J* = 7.2 Hz, 3H), 1.38-1.54 (m, 6H), 1.61-1.80 (m, 6H), 2.63 (t, *J* = 6.0 Hz, 2H), 2.89 (t, *J* = 6.0 Hz, 2H), 3.77, 3.87 (ABx, *J* = 6.6 Hz, 4.5 Hz, 2H), 4.08-4.16 (m, 2H), 4.25 (quint, *J* = 6.0 Hz, 1H), 5.07 (d, *J* = 5.4 Hz, 1H), 7.23-7.36 (m, 3H), 7.44-7.49 (m, 2H), 8.28 (s, 1H), 10.70 (d, *J* = 6.6 Hz, 1H). |
| 10-074 | 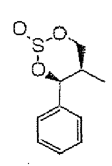 | nBu | 1.01 (t, *J* = 7.5 Hz, 3H), 1.32-1.54 (m, 4H), 1.46 (sextet, *J* = 7.5 Hz, 2H), 1.61-1.78 (m, 6H), 2.57 (t, *J* = 6.0 Hz, 2H), 2.86 (t, *J* = 6.0 Hz, 2H), 4.03-4.22 (m, 2H), 4.12, 5.32 (ABx, *J* = 12.0 Hz, 1.5 Hz, 2H), 4.60 (dd, *J* = 9.0 Hz, 1.5 Hz, 1H), 6.32 (d, *J* = 1.5 Hz, 1H), 7.24-7.32 (m, 3H), 7.38-7.42 (m, 2H), 8.09 (s, 1H), 10.97 (d, *J* = 8.7 Hz, 1H). |
| 10-075 | 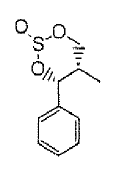 | nBu | 1.01 (t, *J* = 7.5 Hz, 3H), 1.35-1.56 (m, 4H), 1.45 (sextet, *J* = 7.5 Hz, 2H), 1.61-1.79 (m, 6H), 2.57 (t, *J* = 6.0 Hz, 2H), 2.86 (t, *J* = 6.0 Hz, 2H), 4.02-4.23 (m, 2H), 4.12, 5.32 (ABx, *J* = 9.9 Hz, 1.5 Hz, 2H), 4.56-4.63 (m, 1H), 6.32 (d, *J* = 1.8 Hz, 1H), 7.24-7.32 (m, 3H), 7.38-7.42 (m, 2H), 8.09 (s, 1H), 10.97 (d, *J* = 8.7 Hz, 1H). |

**Table 123**

| | | | |
|---|---|---|---|
| 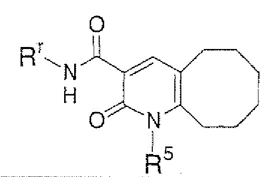 | | | |

| Compound No. | R^{r} | R⁵ | ¹H-NMR (CDCl₃) |
|---|---|---|---|
| 10-076 | 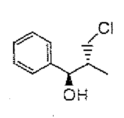 | nBu | 0.99 (t, *J* = 7.5 Hz, 3H), 1.38-1.52 (m, 4H), 1.45 (sextet, *J* = 7.5 Hz, 2H), 1.63-1.79 (m, 6H), 2.63 (t, *J* = 6.0 Hz, 2H), 2.89 (t, *J* = 6.0 Hz, 2H), 3.55, 3.77 (ABx, *J* = 6.0 Hz, 5.1 Hz, 2H), 3.73 (br s, 1H), 4.03-4.19 (m, 2H), 4.43-4.52 (m, 1H), 5.10 (d, *J* = 6.0 Hz, 1H), 7.26-7.37 (m, 3H), 7.46-7.50 (m, 2H), 8.25 (s, 1H), 10.67 (d, *J* = 8.1 Hz, 1H). |
| 10-077 | 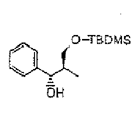 | nBu | 0.05 (s, 3H), 0.06 (s, 3H), 0.93 (s, 9H), 0.98 (t, *J* = 7.5 Hz, 3H), 1.33-1.51 (m, 6H), 1.62-1.79 (m, 6H), 1.90 (br s, 1H), 2.61 (t, *J* = 6.0 Hz, 2H), 2.88 (t, *J* = 6.0 Hz, 2H), 3.73, 3.82 (ABx, *J* = 6.0 Hz, 4.5 Hz, 2H), 4.01-4.19 (m, 2H), 4.26 (sextet, *J* = 4.5 Hz, 1H), 5.10 (d, *J* = 5.4 Hz, 1H), 7.24-7.33 (m, 3H), 7.43-7.48 (m, 2H), 8.22 (s, 1H), 10.71 (d, *J* = 7.5 Hz, 1H). |
| 10-078 | 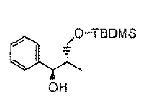 | nBu | 0.05 (s, 3H), 0.06 (s, 3H), 0.93 (s, 9H), 0.99 (t, *J* = 7.5 Hz, 3H), 1.33-1.50 (m, 6H), 1.62-1.79 (m, 6H), 2.05 (br s, 1H), 2.61 (t, *J* = 6.0 Hz, 2H), 2.88 (t, *J* = 6.0 Hz, 2H), 3.73, 3.82 (ABx, *J* = 6.0 Hz, 4.5 Hz, 2H), 4.02-4.20 (m, 2H), 4.27 (sextet, *J* = 4.5 Hz, 1H), 5.10 (d, *J* = 4.8 Hz, 1H), 7.22-7.34 (m, 3H), 7.44-7.47 (m, 2H), 8.22 (s, 1H), 10.72 (d, *J* = 8.1 Hz, 1H). |
| 10-079 | 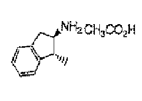 | nBu | 0.97 (t, *J* = 7.5 Hz, 3H), 1.37-1.52 (m, 6H), 1.61-1.80 (m, 6H), 2.03 (s, 3H), 2.67 (t, *J* = 6.0 Hz, 2H), 2.91 (t, *J* = 6.0 Hz, 2H), 3.06, 3.40 (ABx, *J* = 9.0 Hz, 7.2 Hz, 2H), 3.74 (quint, *J* = 7.5 Hz, 1H), 3.99-4.21 (m, 2H), 5.46 (t, *J* = 6.9 Hz, 1H), 7.22-7.29 (m, 3H), 7.33-7.38 (m, 1H), 8.33 (s, 1H), 10.60 (d, *J* = 6.3 Hz, 1H). |
| 10-080 | 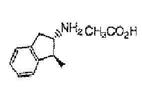 | nBu | 0.97 (t, *J* = 7.5 Hz, 3H), 1.37-1.54 (m, 6H), 1.61-1.78 (m, 6H), 2.01 (s, 3H), 2.67 (t, *J* = 6.0 Hz, 2H), 2.91 (t, *J* = 6.0 Hz, 2H), 3.11, 3.41 (ABx, *J* = 9.0 Hz, 7.5 Hz, 2H), 3.76 (quint, *J* = 7.5 Hz, 1H), 3.99-4.23 (m, 2H), 5.48 (t, *J* = 6.6 Hz, 1H), 7.21-7.30 (m, 3H), 7.34-7.38 (m, 1H), 8.32 (s, 1H), 10.64 (d, *J* = 6.0 Hz, 1H). |

**Table 124**

| | | | |
|---|---|---|---|
| 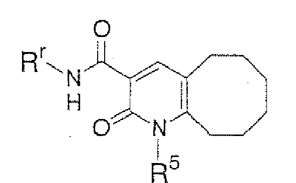 | | | |

| Compound No. | R^{r} | R⁵ | ¹H-NMR (CDCl₃) |
|---|---|---|---|
| 10-081 | 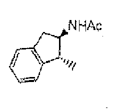 | nBu | 0.97 (t, *J =* 7.5 Hz, 3H), 1.37-1.57 (m, 6H), 1.62-1.81 (m, 6H), 2.02 (s, 3H), 2.67 (t, *J* = 6.0 Hz, 2H), 2.71, 3.68 (ABx, *J* = 15.6 Hz, 7.8 Hz, 2H), 2.91 (t, *J =* 6.0 Hz, 2H), 3.92-4.07 (m, 1H), 4.11-4.27 (m, 2H), 5.60 (t, *J =* 9.0 Hz, 1H), 7.21-7.35 (m, 5H), 8.31 (s, 1H), 10.66 (d, *J* = 8.1 Hz, 1H). |
| 10-082 | 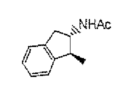 | nBu | 0.97 (t, *J* = 7.5 Hz, 3H), 1.38-1.58 (m, 6H), 1.66-1.82 (m, 6H), 2.02 (s, 3H), 2.67 (t, *J* = 6.0 Hz, 2H), 2.71, 3.68 (ABx, *J* = 15.6 Hz, 7.8 Hz, 2H), 2.91 (t, *J =* 6.0 Hz, 2H), 3.92-4.06 (m, 1H), 4.12-4.28 (m, 2H), 5.60 (t, *J* = 9.0 Hz, 1H), 7.20-7.34 (m, 5H), 8.31 (s, 1H), 10.66 (d, *J* = 7.8 Hz, 1H). |
| 10-083 | 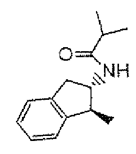 | nBu | 0.98 (t, *J* = 7.5 Hz, 3H), 1.17 (t, *J* = 7.5 Hz, 6H), 1.37-1.56 (m, 4H), 1.44 (sextet, *J* = 7.5 Hz. 2H), 1.63-1.80 (m, 6H), 2.43 (quint, *J* = 7.2 Hz, 1H), 2.67 (t, *J=* 6.0 Hz, 2H), 2.70, 3.71 (ABx, *J=* 15.6 Hz, 7.8 Hz, 2H), 2.91 (t, *J* = 6.0 Hz, 2H), 3.98-4.06 (m, 1H), 4.13-4.21 (m, 2H), 5.60 (t, *J* = 9.0 Hz, 1H), 7.22-7.37 (m, 5H), 8.31 (s, 1H), 10.65 (d, *J* = 7.5 Hz, 1H). |
| 10-084 | 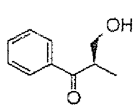 | | 0.98 (t, *J =* 7.2 Hz, 3H), 1.38-1.52 (m, 6H), 1.62-1.80 (m, 6H), 2.64 (t, *J* = 6.0 Hz, 2H), 2.90 (t, *J* = 6.0 Hz, 2H), 3.94, 4.14 (ABx, *J* = 12.0 Hz, 6.0 Hz, 2H), 4.08-4.18 (m, 2H), 5.76-5.82 (m, 1H), 7.47-7.53 (m, 2H), 7.57-7.63 (m, 1H), 8.08-8.11 (m, 2H), 8.29 (s, 1H), 11.13 (d, *J* = 6.6 Hz, 1H). |
| 10-085 | 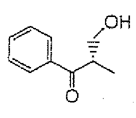 | | 0.98 (t, *J* = 7.5 Hz, 3H), 1.37-1.56 (m, 6H), 1.61-1.80 (m, 6H), 2.65 (t, *J* = 6.0 Hz, 2H), 2.90 (t, *J* = 6.0 Hz, 2H), 3.94, 4.14 (ABx, *J* = 12.0 Hz, 6.0 Hz, 2H), 4.08-4.18 (m, 2H), 5.77-5.83 (m, 1H), 7.48-7.53 (m, 2H), 7.57-7.65 (m, 1H), 8.08-8.12 (m, 2H), 8.30 (s, 1H), 11.14 (d, *J* = 6.6 Hz, 1H). |
| 10-086 | 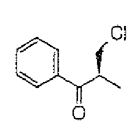 | nBu | 0.98 (t, *J* = 7.5 Hz, 3H), 1.38-1.53 (m, 6H), 1.65-1.81 (m, 6H), 2.64 (t, *J* = 6.0 Hz, 2H), 2.89 (t, *J =* 6.0 Hz, 2H), 3.89, 4.11 (ABx, *J* = 11.4 Hz, 5.1 Hz, 2H), 4.08-4.19 (m, 2H), 6.01-6.08 (m, 1H), 7.45-7.53 (m, 2H), 7.57-7.63 (m, 1H), 8.04-8.08 (m, 2H), 8.28 (s, 1H), 10.92 (d, *J* = 7.5 Hz. 1H). |

**Table 125**

| | | | |
|---|---|---|---|
| 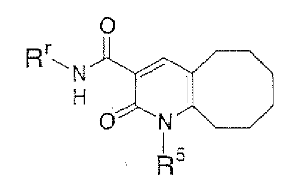 | | | |

| Compound No. | R^{r} | R⁵ | ¹H-MIR (CDCl₃) |
|---|---|---|---|
| 10-087 | 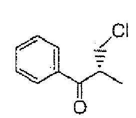 | nBu | 0.98 (t, *J* = 7.5 Hz, 3H), 1.36-1.51 (m, 6H), 1.62-1.79 (m, 6H), 2.64 (t, *J=* 6.0 Hz, 2H), 2.89 (t, *J* = 6.0 Hz, 2H), 3.90, 4.11 (ABx, *J* = 11.4 Hz, 5.1 Hz, 2H), 4.08-4.19 (m, 2H), 6.00-6.08 (m, 1H), 7.45-7.53 (m, 2H), 7.56-7.63 (m, 1H), 8.03.8.07 (m, 2H), 8.28 (s, 1H), 10.92 (d, *J* = 7.8 Hz, 1H). |
| 10-088 | 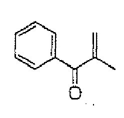 | nBu | 0.98 (t, *J* = 7.5 Hz, 3H), 1.38-1.54 (m, 6H), 1.64-1.80 (m, 6H), 2.66 (t, *J* = 6.0 Hz, 2H), 2.91 (t, *J* = 6.0 Hz, 2H), 4.17 (br t, *J* = 7.5 Hz, 2H), 5.58 (s, 1H), 7.10 (s, 1H), 7.42-7.49 (m, 2H), 7.53- 7.59 (m, 1H), 7.77-7.82 (m, 2H), 8.31 (s, 1H), 12.52 (br s, 1H). |
| 10-089 | 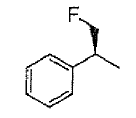 | nBu | 0.99 (t, *J* = 7.5 Hz, 3H), 1.37-1.55 (m, 6H), 1.60-1.81 (m, 6H), 2.63 (t, *J* = 6.0 Hz, 2H), 2.89 (t, *J* = 6.0 Hz, 2H), 3.91 (d, *J* = 6.0 Hz, 2H), 4.12 (br t, *J* = 7.5 Hz, 2H), 5.51-5-58 (m, 1H), 7.27-7.48 (m, 5H), 8.29 (s, 1H), 10.77 (d, *J* = 8.1 Hz, 1H). |
| 10-090 | 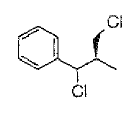 | nBu | 0.98 (t, *J* = 7.5 Hz, 3H × 2/5), 0.99 (t, *J* = 7.5 Hz, 3H × 3/5), 1.36-1.79 (m, 12H), 2.61 (t, *J* = 6.0 Hz, 2H × 2/5), 2.62 (t, *J* = 6.0 Hz, 2H × 3/5), 2.87 (t, *J* = 6.0 Hz, 2H × 2/5), 2.88 (t, *J* = 6.0 Hz, 2H × 3/5), 3.52, 4.15 (ABx, *J* = 11.1 Hz, 4.2 Hz, 2H), 4.10-4.20 (m, 2H), 4.83-4.91 (m, 1H × 3/5), 4.98-5.08 (m, 1H × 2/5), 5.30 (d, *J* = 5.4 Hz, 1H × 2/5), 5.44 (d, *J =* 5.4 Hz, 1H × 3/5), 7.28-7.38 (m, 3H), 7.46-7.53 (m, 2H), 8.16 (s, 1H × 2/5), 8.23 (s, 1H × 3/5), 10.46 (d, *J* = 8.4 Hz, 1H × 2/5), 10.72 (d, *J* = 8.4 Hz, 1H × 3/5). |
| 10-091 | 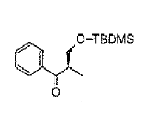 | nBu | -0.12 (s, 3H), -0.11 (s, 3H), 0.74 (s, 9H), 0.98 (t, *J* = 7.5 Hz, 3H), 1.36-1.53 (m, 6H), 1.62-1.78 (m, 6H), 2.62 (t, *J* = 6.0 Hz, 2H), 2.88 (t, *J* = 6.0 Hz, 2H), 3.96-4.10 (m, 2H), 4.11-4.22 (m, 2H), 5.80-5.88 (m, 1H), 7.43-7.49 (m, 2H), 7.53-7.61 (m, 1H), 8.05-8.09 (m, 2H), 8.26 (s, 1H), 10.81 (d, *J* = 7.5 Hz, 1H). |

**Table 126**

| | | | |
|---|---|---|---|
| 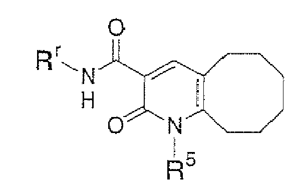 | | | |

| Compound No. | R^{r} | R⁵ | ¹H-NMR (CDCl₃) |
|---|---|---|---|
| 10-092 | 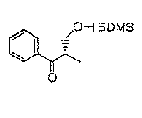 | nBu | -0.12 (s, 3H), -0.11 (s, 3H), 0.74 (s, 9H), 0.98 (t, *J* = 7.5 Hz, 3H), 1.37-1.52 (m, 6H), 1.62-1.78 (m, 6H), 2.62 (t, *J* = 6.0 Hz, 2H), 2.88 (t, J= 6.0 Hz, 2H), 3.96-4.10 (m, 2H), 4.10-4.23 (m, 2H), 5.80-5.87 (m, 1H), 7-43-7.49 (m, 2H), 7.53-7.61 (m, 1H), 8.05-8.09 (m, 2H), 8.26 (s, 1H), 10.80 (d, *J=* 7.5 Hz, 1H). |
| 10-093 | 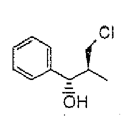 | | 0.99 (t, *J* = 7.2 Hz, 3H), 1.37-1.52 (m, 6H), 1.63-1.81 (m, 6H), 2.63 (t, *J=* 6.0 Hz, 2H), 2.89 (t, *J* = 6.0 Hz, 2H), 3.56, 3.77 (ABx, *J* = 11.1 Hz, 5.1 Hz, 2H), 4.08-4.18 (m, 2H), 4.44-4.53 (m, 1H), 5.10 (d, *J* = 6.0 Hz, 1H), 7.25-7.37 (m, 3H), 7.46-7.49 (m, 2H), 8.28 (s, 1H), 10.69 (d, *J* = 7.8 Hz, 1H). |
| 10-094 | 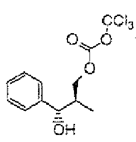 | nBu | 1.00 (t, *J* = 7.2 Hz, 3H), 1.38-1.52 (m, 6H), 1.63-1.80 (m, 6H), 2.63 (t, *J* = 6.0 Hz, 2H), 2.89 (t, *J* = 6.0 Hz, 2H), 4.11 (br t, *J* = 7.2 Hz, 2H), 4.34-4.42 (m, 1H), 4.48-4.57 (m, 2H), 5.03 (d, *J=* 4.8 Hz, 1H), 7-26-7.37 (m, 3H), 7.41-7.46 (m, 2H), 8.26 (s, 1H), 10.62 (d, *J* = 7.5 Hz, 1H). |
| 10-095 | 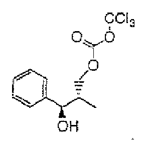 | nBu | 0.99 (t, *J=* 7.2 Hz, 3H), 1.38-1.52 (m, 6H), 1.62-1.79 (m, 6H), 2.63 (t, *J* = 6.0 Hz, 2H), 2.89 (t, *J* = 6.0 Hz, 2H), 4.11 (br t, *J* = 7.2 Hz, 2H), 4.34-4.42 (m, 1H), 4.48-4.57 (m, 2H), 5.03 (d, *J* = 4.5 Hz, 1H), 7.26-7.37 (m, 3H), 7.41-7.46 (m, 2H), 8.24 (s, 1H), 10.60 (d, *J* = 7.5 Hz, 1H). |
| 10-096 | 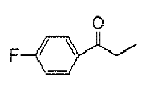 | | 0.99 (t, *J* = 7.2 Hz, 3H), 1.38-1.51 (m, 6H), 1.64-1.78 (m, 6H), 2.65 (t, *J* = 6.0 Hz, 2H), 2.90 (t, *J* = 6.0 Hz, 2H), 4.15 (br t, *J* = 7.2 Hz, 2H), 4.93 (d, *J* = 4.5 Hz, 2H), 7.14-7.21 (m, 2H), 8.04-8.10 (m, 2H), 8.30 (s, 1H), 10.79 (br s, 1H). |
| 10-097 | 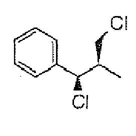 | nBu | 0.98 (t, *J* = 7.5 Hz, 3H), 1.36-1.50 (m, 6H), 1.62-1.78 (m, 6H), 2.59 (t, *J* = 6.0 Hz, 2H), 2.86 (t, *J* = 6.0 Hz, 2H), 3.76, 4.15 (ABx, *J* = 11.7 Hz, 4.5 Hz, 2H), 4.00-4.13 (m, 2H), 4.98-5.07 (m, 1H), 5.3D (d, *J* = 8.1 Hz, 1H), 7.24-7.37 (m, 3H), 7.49-7.53 (m, 2H), 8.16 (s, 1H), 10.46 (d, *J* = 8.7 Hz, 1H). |

**Table 127**

| | | | |
|---|---|---|---|
| 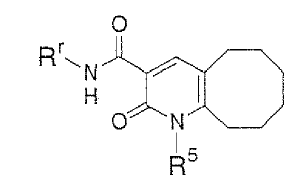 | | | |

| Compound No. | R^{r} | R⁵ | ¹H-NMR (CDCl₃) |
|---|---|---|---|
| 10-098 | 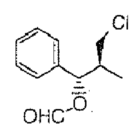 | nBu | 1.01 (t, *J* = 7.2 Hz, 3H), 1.38-1.52 (m, 6H), 1.63-1.81 (m, 6H), 2.62 (t, *J* = 6.0 Hz, 2H), 2.89 (t, *J=* 6.0 Hz, 2H), 3.47, 3.65 (ABx, *J* = 11.1 Hz, 5.1 Hz, 2H), 4.08-4.19 (m, 2H), 4.83-4.92 (m, 1H), 6.28 (d, *J* = 6.6 Hz, 1H), 7.29-7.39 (m, 3H), 7.44-7.53 (m, 2H), 8.17 (s, 1H), 8.23 (s, 1H), 10.64 (d, *J=* 8.7 Hz, 1H). |
| 10-099 | 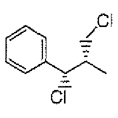 | nBu | 0.99 (t, *J* = 7.5 Hz, 3H), 1.38-1.52 (m, 6H); 1.62-1.79 (m, 6H), 2.59 (t, *J* = 6.0 Hz, 2H), 2.86 (t, *J=* 6.0 Hz, 2H), 3.76, 4.15 (AF3x, *J* = 11.4 Hz, 4.5 Hz, 2H), 3.99-4.13 (m, 2H), 4.98-5.07 (m, 1H), 5.30 (d, *J* = 7.8 Hz, 1H), 7.24-7.41 (m, 3H), 7.50-7.53 (m 2H), 8.16 (s, 1H), 10.46 (d, *J* = 8.4 Hz, 1H). |
| 10-100 | 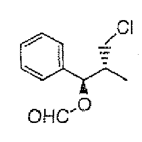 | nBu | 1.01 (t, *J* = 7.5 Hz, 3H), 1.40-1.53 (m, 6H), 1.64-1.80 (m, 6H), 2.62 (t, *J* = 6.0 Hz, 2H), 2.89 (t; *J =* 6.0 Hz, 2H), 3.47, 3.65 (ABx, *J* = 11.1 Hz, 5.1 Hz, 2H), 4.08-4.18 (m, 2H), 4.83-4.92 (m, 1H), 6.28 (d, *J* = 6.3 Hz, 1H), 7.30-7.38 (m, 3H), 7.45-7.49 (m, 2H), 8.17 (s, 1H), 8.23 (s, 1H), 10.64 (d, *J* = 9.0 Hz, 1H). |
| 10-101 | 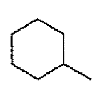 | 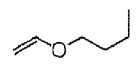 | 1.21-1.52 (m, 8H), 1.59-1.79 (m, 8H), 1.94-1.99 (m, 2H), 2.09 (quint, *J* = 6.0 Hz, 2H), 2.64 (t, *J* = 6.0 Hz, 2H), 2.92 (t, *J* = 6.0 Hz, 2H), 3.78 (t, *J* = 6.0 Hz, 2H), 3.93-4.03 (m, 1H), 4.05 (dd, *J* = 6.6 Hz, 2.1 Hz, 1H), 4.20 (dd, *J* = 14.1 Hz, 2.1 Hz, 1H), 4.25 (br t, *J* = 7.5 Hz, 2H), 6.49 (dd, *J=* 14.4 Hz, 6.9 Hz, 1H), 8.31 (s, 1H), 9.85 (d, *J* = 7.8 Hz, 1H). |
| 10-102 | 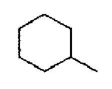 | 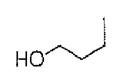 | 1.30-1.54 (m, 8H), 1.60-1.78 (m, 8H), 1.87-2.00 (m, 4H). 2.65 (t, *J* = 6.0 Hz, 2H), 2.93 (t, *J* = 6.0 Hz, 2H), 3.53 (t, *J* = 6.0 Hz, 2H), 3.91-4.02 (m, 1H), 4.34 (br t, *J* = 7.5 Hz, 2H), 8.37 (s, 1H), 9.77 (d, *J=* 7.2 Hz, 1H). |
| 10-103 | 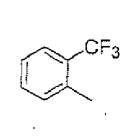 | nBu | 0.99 (t, *J* = 7.5 Hz, 3H), 1.39-1.55 (m, 4H), 1.49 (sextet, *J* = 7.5 Hz, 2H), 1.68-1.83 (m, 6H), 2.67 (t, *J* = 6.0 Hz, 2H), 2.92 (t, *J* = 6.0 Hz, 2H), 4.18 (br t, *J* = 7.5 Hz, 2H), 7.21 (t, *J* = 7.5 Hz, 1H), 7.55 (t, *J* = 7.5 Hz, 1H), 7.64 (d, *J* = 8.1 Hz, 1H), 8.32 (d, *J* = 8.1 Hz, 1H), 8.36 (s, 1H), 12.41 (br s, 1H), |

**Table 128**

| | | | |
|---|---|---|---|
| 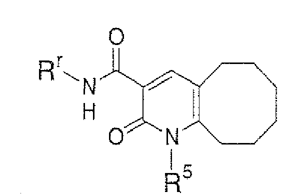 | | | |

| Compound No. | R^{r} | R⁵ | ¹H-NMR (CDCl₃) |
|---|---|---|---|
| 10-104 | 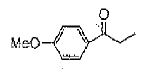 | nBu | 0.99 (t, *J* = 7.2 Hz, 3H), 1.38-1.52 (m, 6H), 1.63-1.80 (m, 6H), 2.65 (t, *J* = 6.0 Hz, 2H), 2.90 (t, *J* = 6.0 Hz, 2H), 3.88 (s, 3H), 4.14 (br t, J= 7.2 Hz, 2H), 4.91 (d, *J* = 4.5 Hz, 2H), 6.97 (d, *J* = 8.4 Hz, 2H), 8.03 (d, *J* = 8.4 Hz, 2H), 8.30 (s, 1H), 10.80 (br s, 1H). |
| 10-105 | 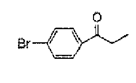 | nBu | 0.99 (t, *J* = 7.2 Hz, 3H), 1.36-1.53 (m, 4H), 1.47 (sextet, *J* = 7.2 Hz, 2H), 1.63-1.80 (m, 6H), 2.65 (t, *J* = 6.0 Hz, 2H), 2.90 (t, *J* = 6.0 Hz, 2H), 4.15 (br t, *J* = 7.2 Hz, 2H), 4.91 (d, *J* = 4.5 Hz, 2H), 7.64 (d, *J* = 8.7 Hz, 2H), 7.90 (d, *J* = 8.7 Hz, 2H), 8.29 (s, 1H), 10.79 (br s, 1H). |
| 10-106 | 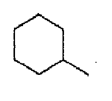 | 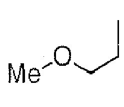 | 1.30-1.53 (m, 8H), 1.58-1.79 (m, 8H), 1.96-2.02 (m, 2H), 2.65 (t, *J* = 6.0 Hz, 2H), 3.03 (t, *J* = 6.0 Hz, 2H), 3.31 (s, 3H), 3.69 (t, *J* = 5.4 Hz, 2H), 3.92-4.03 (m, 1H), 4.33 (t, *J* = 5.4 Hz, 2H), 8.32 (s, 1H), 9.83 (d, *J* = 7.2 Hz, 1H). |
| 10-107 | 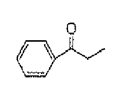 | 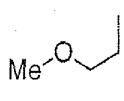 | 1.35-1.42 (m, 2H), 1.43-1.52 (m, 2H), 1.62-1.79 (m, 4H), 2.70 (t, *J* = 6.0 Hz, 2H), 3.06 (t, *J* = 6.0 Hz, 2H), 3.31 (s, 3H), 3.74 (t, *J* = 5.4 Hz, 2H), 4.40 (d, *J* = 5.4 Hz, 2H), 4.97 (d, *J* = 4.5 Hz, 2H), 7.50 (t, *J* = 7.5 Hz, 2H), 7.61 (t, *J* = 7.5 Hz, 1H), 8.05 (d, *J* = 7.5 Hz, 2H), 8.33 (s, 1H), 10.75 (br s, 1H). |
| 10-108 | 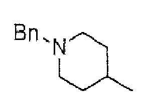 | nBu | 0.99 (t, *J* = 7.2 Hz, 3H), 1.38-1.52 (m, 6H), 1.63-1.79 (m, 8H), 1.95-2.07 (m, 2H), 2.20-2.33 (m, 2H), 2.63 (t, *J* = 6.0 Hz, 2H), 2.82-2.94 (m, 2H), 2.88 (t, *J* = 6.0 Hz, 2H), 3.55 (br s, 2H), 3.96-4.07 (m, 1H), 4.10 (br t, *J* = 7.2 Hz, 2H), 7.23-7.40 (m, 5H), 5.27 (s, 1H), 9.97 (br s, 1H). |
| 10-109 | 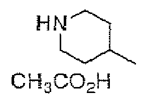 | nBu | 1.00 (t, *J* = 7.5 Hz, 3H), 1.37-1.50 (m, 6H), 1.63-1.79 (m, 6H), 1.81-1.97 (m, 2H), 2.04 (s, 3H), 2.15-2.24 (m, 2H), 2.63 (t, *J* = 6.0 Hz, 2H), 2.89 (t, *J* = 6-0 Hz, 2H), 3.01 (t, *J* = 10.2 Hz, 2H), 3.33-3.41 (m, 2H), 4.09 (br t, *J* = 7.5 Hz, 2H), 4.10-4.25 (m, 1H), 8.26 (s, 1H), 10.21 (d, *J* = 7.2 Hz, 7.H). |

**Table 129**

| | | | |
|---|---|---|---|
| 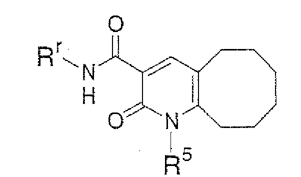 | | | |

| Compound No. | R^{r} | R⁵ | ¹H-NMR (CDCl₃) |
|---|---|---|---|
| 10-110 | 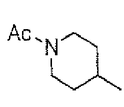 | nBu | 0.99 (t, *J* = 7.5 Hz, 3H), 1.36-1.58 (m, 4H), 1.46 (sextet, *J* = 7.5 Hz, 2H), 1.62.1.79 (m, 8H), 1.97-2.11 (m, 2H), 2.11 (s, 3H), 2.64 (t, *J* = 6.0 Hz, 2h), 2.89 (t, *J* = 6.0 Hz, 2H), 2.92-3.02 (m, 1H), 3.21-3.31 (m, 1H), 3.75-3.81 (m, 1H), 4.09 (br t, *J* = 7.5 Hz, 2H), 4.11-4.23 (m, 1H), 4.37-4.43 (m, 1H), 8.29 (s, 1H), 10.08 (d. *J* = 7.5 Hz, 1H). |
| 10-1.11 | 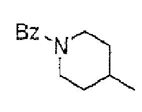 | nBu | 0.99 (t, *J* = 7.2 Hz, 3H), 1.36-1.57 (m, 4H), 1.46 (sextet, *J* = 7.2 Hz, 2H), 1.62-1.79 (m, 8H), 1.93-2.15 (m, 2H), 2.64 (t, *J* = 6.0 Hz, 2H), 2.89 (t, *J* = 6.0 Hz, 2H), 3.11-3.27 (m, 2H), 3.69-3.79 (m, 1H), 4.10 (br t, *J* = 7.2 Hz, 2H), 4.19-4.30 (m, 1H), 4.50-4.60 (m, 1H), 7.41 (s, 5H), 8.29 (s, 1H), 10.12 (d, *J* = 7.5 Hz, 1H). |
| 10-112 | 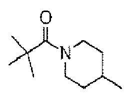 | nBu | 0.99 (t, *J* = 7.5 Hz, 3H), 1.29 (s, 9H), 1.35-1.79 (m, 14H), 2.00-2.09 (m, 2H), 2.65 (t, *J* = 6.0 Hz, 2H), 2.89 (t, *J* = 6.0 Hz, 2H), 3.09 (t, *J* = 11.4 Hz, 2H), 4.06-4.35 (m, 5H), 8.30 (s, 1H), 10.06 (d, *J* = 7.5 Hz, 1H). |
| 10-113 | 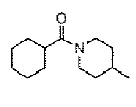 | nBu | 0.99 (t, *J* = 7.2 Hz, 3H), 1.28-1.81 (m, 24H), 1.98.2.10 (m, 2H), 2.44-2.53 (m, 1H), 2.65 (t, *J* = 6.0 Hz, 2H), 2.89 (t, *J* = 6.0 Hz, 2H), 2.89-2.99 (m, 1H), 3.15-3.28 (m, 1H), 3.85-3.93 (m, 1H), 4.09 (br t, *J* = 7.2 Hz, 2H), 4.10-4.25 (m, 1H), 4.40-4-48 (m, 1H), 8.30 (s, 1H), 10.06 (d, *J* = 6.9 Hz, 1H). |
| 10-114 | 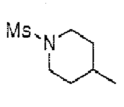 | nBu | 1.02 (t, *J* = 7.5 Hz, 3H), 1.38-1.78 (m, 14H), 2.07-2.20 (m, 2H), 2.65 (t, *J* = 6.0 Hz, 2H), 2.81 (s, 3H), 2.90 (t, *J* = 6.0 Hz, 2H), 2.94-3.01 (m, 2H), 3.70-3.79 (m, 2H), 4.11 (br t, *J* = 7.5 Hz, 2H), 4.11-4.23 (m, 1H), 8.29 (s, 1H), 10.11 (d, *J* = 7.2 Hz, 1H), |
| 10-115 | 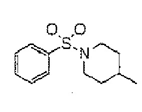 | nBu | 0.99 (t, *J* = 7.2 Hz, 3H), 1.37-1.51 (m, 6H), 1.62-1.78 (m, 8H), 2.03-2.09 (m, 2H), 2.53 (td, *J* = 11.4 Hz, 2.4 Hz, 2H), 2.62 (t, *J* = 6.0 Hz, 2H), 2.88 (t, *J* = 6.0 Hz, 2H), 3.71-3.77 (m, 2H), 3.82-3.94 (m, 1H), 4.07 (br t, *J* = 7.5 Hz, 2H), 7.52-7.65 (m, 3H), 7.76-7.71 (m, 2H), 8.25 (s, 1H), 10.00 (d, *J* = 6.9 Hz, 1H). |

**Table 130**

| | | | |
|---|---|---|---|
| 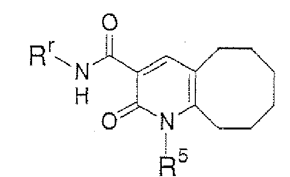 | | | |

| Compound No. | R^{r} | R⁵ | ¹H-NMR (CDCl₃) |
|---|---|---|---|
| 10-116 | 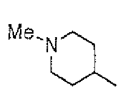 | nBu | 0.99 (t, *J* = 7.2 Hz, 3H), 1.38-1.50 (m, 4H), 1.47 (quint, *J* = 7.2 Hz, 2H), 1.64-1.82 (m, 8H), 2.01-2.09 (m, 2H), 2.25-2.34 (m, 2H), 2.35 (s, 3H), 2.64 (t, *J* = 6.0 Hz, 2H), 2.82-2.90 (m, 2H), 2.88 (t, *J* = 6.0 Hz, 2H), 3.97-4.06 (m, 1H), 4.10 (br t, *J* = 7.2 Hz, 2H), 8-28 (s, 1H), 9.98 (d, *J* = 7.2 Hz, 1H). |
| 10-117 | 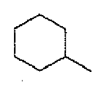 | 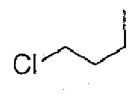 | 1.21-1.53 (m, 8H), 1.62-1.82 (m, 8H), 1.95-2.00 (m, 2H), 2.20 (dt, *J* = 15.0 Hz, 6.0 Hz, 2H), 2.65 (t, *J* = 6.0 Hz, 2H), 2.94 (t, *J* = 6.0 Hz, 2H), 3.69 (t, *J* = 6.0 Hz, 2H), 3.92-4.02 (m, 1H), 4.28 (t, *J* = 7.5 Hz, 2H), 8.33 (s, 1H), 9.80 (d, *J* = 6.9 Hz, 1H). |
| 10-118 | 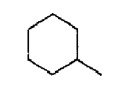 | 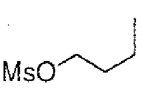 | 1.26-1.52 (m, 8H), 1.60-1.80 (m, 8H), 1.94-2.00 (m, 2H), 2.19 (quint, *J* = 6.3 Hz, 2H), 2.64 (t, *J* = 6.0 Hz, 2H), 2.90 (t, *J* = 6.0 Hz, 2H), 3.06 (s, 3H), 3.92-4.01 (m, 1H), 4.27 (t, *J* = 7.5 Hz, 2H), 4.38 (t, *J* = 6.0 Hz, 2H), 8.33 (s, 1H), 9.78 (d, *J* = 8.1 Hz, 1H). |
| 10-119 | 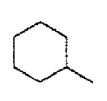 | 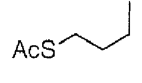 | 1.25-1.52 (m, 8H), 1.59-1.79 (m, 8H), 1.93-2.05 (m, 4H), 2.32 (s, 3H), 2.63 (t, *J* = 6.0 Hz, 2H), 2.88 (t, *J* = 6.0 Hz, 2H), 2.98 (t, *J* = 6.9 Hz, 2H), 3.91-4.01 (m, 1H), 4.15 (t, *J* = 7.2 Hz, 2H), 8.30 (s, 1H), 9.82 (d, *J* = 7.5 Hz, 1H). |
| 10-120 | 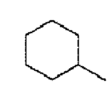 | 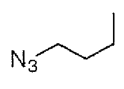 | 1.25-1.52 (m, 8H), 1.58-1.80 (m, 8H), 1.93-2.03 (m, 4H), 2.64 (t, *J* = 6.0 Hz, 2H), 2.91 (t, *J* = 6.0 Hz, 2H), 3.48 (t, *J* = 6.0 Hz, 2H), 3.92-4.03 (m, 1H), 4.20 (t, *J* = 7.5 Hz, 2H), 8.32 (s, 1H), 9.81 (d, *J* = 6.9 Hz, 1H). |
| 10-121 | 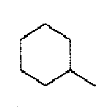 | 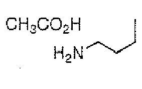 | 1.27-1.53 (m, 8H), 1.60-1.81 (m, 8H), 1.92-2.01 (m, 2H), 2.05 (s, 3H), 2.20-2.29 (m, 2H), 2.66 (t, *J* = 6.0 Hz, 2H), 2.90 (t, *J* = 6.0 Hz, 2H), 2.98 (br s, 2H), 3.90-4.00 (m, 1H), 4.27-4.35 (m, 2H), 8.40 (s, 1H), 9.50 (d, *J* = 7.5 Hz, 1H). |
| 10-122 | 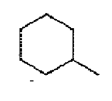 | 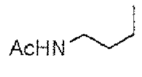 | 1.24-1.52 (m, 8H), 1.62-1.79 (m, 8H), 1.88-2.03 (m, 4H), 2.03 (s, 3H), 2.65 (t, *J* = 6.0 Hz, 2H), 2.89 (t, *J* = 6.0 Hz, 2H), 3.24-3.30 (m, 2H), 3.97-4.05 (m, 1H), 4.22 (br t, *J* = 7.5 Hz, 2H), 6.72 (br s, 1H), 8.35 (s, 1H), 9.81 (d, *J* = 7.8 Hz, 1H)- |

**Table 131**

| | | | |
|---|---|---|---|
| 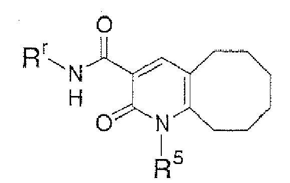 | | | |

| Compound No. | R^{r} | R⁵ | ¹H-NMR (CDCl₃) |
|---|---|---|---|
| 10-123 | 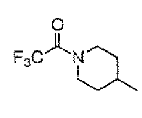 | nBu | 0.99 (t, *J* = 7.2 Hz, 3H), 1.37-1.52 (m, 6H), 1.60-1.72 (m, 6H), 1.71-3..80 (m, 2H), 2.07-2.17 (m, 2H), 2.65 (t, *J* = 6.0 Hz, 2H), 2.89 (t, *J* = 6.0 Hz, 2H), 3.17 (t, *J* = 11.1 Hz, 1H), 3.37 (t, *J*= 11.1 Hz, 1H), 3.92-3.99 (m, 1H), 4.03-4.12 (m, 2H), 4.22-4.37 (m, 2H), 8.28 (s, 1H), 10.17 (d, *J* = 7.2 Hz, 1H). |
| 10-124 | 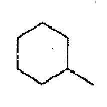 | 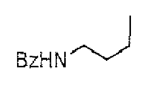 | 1.29-1.51 (m, 8H), 1.65-1.77 (m, 8H), 1.92-2.05 (m, 4H), 2.65 (t, *J* = 6.0 Hz, 2H), 2.93 (t, *J* = 6.0 Hz, 2H), 3.45 (br s, 2H), 3.97-4.05 (m, 1H), 4.32 (br t, *J* = 7.5 Hz, 2H), 7.42-7.55 (m, 3H), 7.89 (br s, 1H), 7.90-7.96 (m, 2H), 8.37 (s, 1H), 9.90 (d, *J* = 7.8 Hz, 1H). |
| 10-125 | 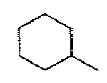 | 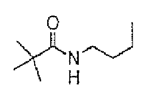 | 1.25 (s, 9H), 1.28-1.51 (m, 8H), 1.60-1.78 (m, 8H), 1.85-2.00 (m, 4H), 2.64 (t, *J* = 6.0 Hz, 2H), 2.90 (t, *J* = 6.0 Hz, 2H), 3.23 (br s, 2H), 3.95-4.04 (m, 1H), 4.22 (br t, *J* = 7.5 Hz, 2H), 7.12 (br s, 1H), 8.35 (s, 1H), 9.89 (d, *J* = 7.5 Hz, 1H). |
| 10-126 | 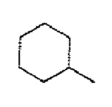 | 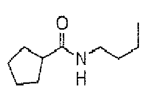 | 1.27-1.52 (m, 8H), 1.57-1.80 (m, 7.4H), 1.83-2.01 (m, 6H), 2.55-2.65 (m, 1H), 2.64 (t, *J* = 6.0 Hz, 2H), 2.89 (t, *J* = 6.0 Hz, 2H), 3.25-3.29 (m, 2H), 3.95-4.04 (m, 1H), 4.22 (br t, *J* = 7.5 Hz, 2H), 6.73 (br s, 1H), 8.34 (s, 1H), 9.85 (d, *J* = 8.1 Hz, 1H). |
| 10-127 | 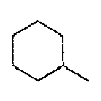 | 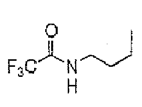 | 1.29-1.52 (m, 8H), 1.61-1.79 (m, 8H), 1.91-2.02 (m, 4H), 2.66 (t, *J* = 6.0 Hz, 2H), 2.91 (t, *J* = 6.0 Hz, 2H), 3. 34 (br s, 2H), 3.97-4.04 (m, 1H), 4.2 (br t, *J* = 7.5 Hz, 2H), 8.39 (s, 1H), 8.67 (br s, 1H), 9-76 (d, *J* = 8.1 Hz, 1H). |
| 10-128 | 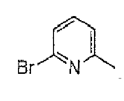 | nBu | 1.00 (t, *J* = 7.5 Hz, 3H), 1.40-1.54 (m, 6H), 1.68-1.81 (m, 6H), 2.67 (t, *J* = 6.0 Hz, 2H), 2.92 (t, *J* = 6.0 Hz, 2H), 4.15 (br t, *J* = 7.5 Hz, 2H), 7.18 (d, *J* = 7.5 Hz, 1H), 7.53 (t, *J* = 7.5 Hz, 1H), 8.33 (d, *J* = 7.5 Hz, 1H), 8.34 (s, 1H), 12.69 (br s, 1H). |

**Table 132**

| | | | |
|---|---|---|---|
| 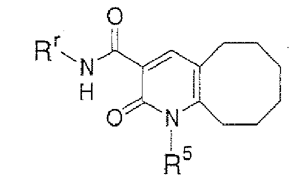 | | | |

| Compound No. | R^{r} | R⁵ | ¹H-NMR (CDCl₃) |
|---|---|---|---|
| 10-129 | 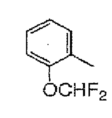 | nBu | 1.00 (t, *J* = 7.2 Hz, 3H), 1.37-1.54 (m, 6H), 1.65-1.81 (m, 6H), 2.67 (t, *J* = 6.0 Hz, 2H), 2.92 (t, *J* = 6.0 Hz, 2H), 4.17 (br t, *J* = 7.2 Hz, 2H), 6.22 (s, 1H×1/5), 6.39 (s, 1H×1/5), 6.47 (s, 1H×1/5), 6.64 (s, 1H×1/5), 6.88 (s, 1H× 1/5), 7.01-7.10 (m, 1H), 7.18-7.27 (m, 2H), 8.36 (s, 1H), 8.60 (dd, *J* = 7.8 Hz, 1.8 Hz, 1H), 12.59 (br s, 1H). |
| 10-130 | 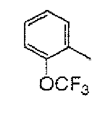 | nBu | 1.00 (t, *J* = 7.5 Hz, 3H), 1.37-1.53 (m, 4H), 1.47 (sextet, *J* = 7.5 Hz, 2H), 1.65-1.82 (m, 6H), 2.67 (t, *J* = 6.0 Hz, 2H), 2.92 (t, *J* = 6.0 Hz, 2H), 4.18 (br t, *J* = 7.5 Hz, 2H), 7.08 (td, *J* = 8.4 Hz, 1.8 Hz, 1H), 7.26-7.34 (m, 2H), 8.36 (s, 1H), 8.64 (dd, *J* = 9.0 Hz, 1.8 Hz, 1H), 12.76 (br s, 1H). |
| 10-131 | 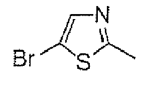 | nBu | 1.00 (t, *J* = 7.5 Hz, 3H), 1.38-1.54 (m, 4H), 1.47 (sextet, *J* = 7.5 Hz, 2H), 1.61-1.82 (m, 6H), 2.68 (t, *J* = 6.0 Hz, 2H), 2.93 (t, *J* = 6.0 Hz, 2H), 4.16 (br t, *J* = 7.5 Hz, 2H), 7.41 (s, 1H), 8.34 (s, 1H), 13.49 (br s, 1H). |
| 10-132 | 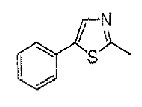 | nBu | 1.01 (t, *J* = 7.5 Hz, 3H), 1.38-1.52 (m, 4H), 1.48 (sextet, *J* = 7.5 Hz, 2H), 1.63-1.85 (m, 6H), 2.68 (t, *J* = 6.0 Hz, 2H), 2.94 (t, *J* = 6.0 Hz, 2H), 4.17 (br t, *J* = 7.5 Hz, 2B), 7.27-7.32 (m, 1H), 7.36-7.42 (m, 2H), 7.57-7.61 (m, 2H), 7.71 (s, 1H), 8.37 (s, 1H), 13.52 (br s, 1H). |
| 10-133 | 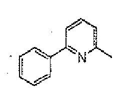 | nBu | 1.01 (t, *J* = 7.5 Hz, 3H), 1.40-1.55 (m, 4H), 1.49 (sextet, *J* = 7.5 Hz, 2H), 1.65-1.81 (m, 6H), 2.68 (t, J= 6.0 Hz, 2H), 2.93 (t, *J* = 6.0 Hz, 2H), 4.19 (br t, *J* = 7.5 Hz, 2H), 7.38-7.52 (m, 4H), 7.78 (t, *J* = 8.1 Hz, 1H), 8.09 (d, *J* = 8.1 Hz, 2H), 8.33 (d, *J* = 8.1 Hz, 1H), 8.40 (s, 1H), 12.61 (br s, 1H). |
| 10-134 | 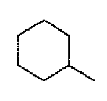 | 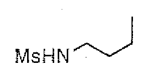 | 1.28-1.52 (m, 8H), 1.63-1.80 (m, 8H), 1.92-2.01 (m, 4H), 2.64 (t, *J* = 6.0 Hz, 2H), 2.90 (t, *J* = 6.0 Hz, 2H), 2.94 (s, 3H), 3.08 (q, *J* = 5.4 Hz, 2H), 3.94-4.02 (m, 1H), 4.29 (br t, *J* = 7.5 Hz, 2H), 5.84 (br t, *J* = 7.5 Hz, 1H), 8.35 (s, 1H), 9.72 (d, *J* = 7.5 Hz, 1H). |

**Table 133**

| | | | |
|---|---|---|---|
| 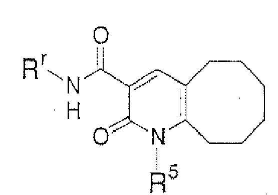 | | | |

| Compound No. | R^{r} | R⁵ | ¹H-NMR (CDCl₃) |
|---|---|---|---|
| 10-135 | 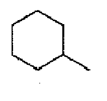 | 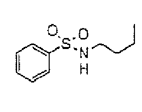 | 1.29-1.50 (m, 8H), 1.63-1.78 (m, 8H), 1.86-2.00 (m, 4H), 2.61 (t, *J* = 6.0 Hz, 2H), 2.79-2.90 (m, 2H), 2.85 (t, *J* = 6.0 Hz, 2H), 3.95-4.03 (m, 1H), 4.24 (br t, *J* = 7.5 Hz, 2H), 6.19 (br s, 1H), 7.44-7.58 (m, 3H), 7.82-7.87 (m, 2H), 8.34 (s, 1H), 9.75 (d, *J* = 7.5 Hz, 1H). |
| 10-136 | 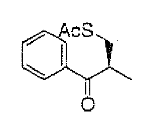 | nBu | 0.98 (t, *J* = 7.5 Hz, 3H), 1.36-1.53 (m, 4H), 1.45 (sextet, *J* = 7.5 Hz, 2H), 1.64-1.77 (m, 6H), 2.31 (s, 3H), 2.62 (t, *J* = 6.0 Hz, 2H), 2.88 (t, *J* = 6.0 Hz, 2H), 3.21, 3.66 (ABx, *J* = 13.8 Hz, 6.0 Hz, 2H), 4.10 (br t, *J* = 7.5 Hz, 2H), 5.92-6.00 (m, 1H), 7.46-7.52 (m, 2H), 7.55-7.62 (m, 1H), 8.12-8.16 (m, 2H), 8.27 (s, 1H), 10.76 (d, *J* = 8.4 Hz, 1H). |
| 10-137 | 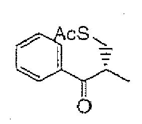 | nBu | 0.98 (t, *J* = 7.5 Hz, 3H), 1.36-1.52 (m, 4H), 1.45 (sextet, *J* = 7.5 Hz, 2H), 1.63-1.77 (m, 6H), 2.31 (s, 3H), 2.62 (t, *J* = 6.0 Hz, 2H), 2.88 (t, *J* = 6.0 Hz, 2H), 3.21, 3.66 (ABx, *J* = 13.8 Hz, 6.0 Hz, 2H), 4.11 (br t, *J* = 7.5 Hz, 2H), 5.92-6.00 (m, 1H), 7.46-7.62 (m, 3H), 8.12-8.16 (m, 2H), 8.27 (s, 1H), 10.75 (d, *J* = 7.8 Hz, 1H). |
| 10-138 | 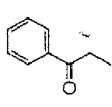 | 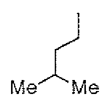 | 1.01 (d, *J* = 6.9 Hz, 6H), 1.38-152 (m, 4H), 1.60-1.73 (m, 5H), 1.78 (quint, *J* = 6.0 Hz, 2H), 2.65 (t, *J* = 6.0 Hz, 2H), 2.96 (t, *J* = 6.0 Hz, 2H), 4.16 (br t, *J* = 7.5 Hz, 2H), 4.97 (d, *J* = 4.8 Hz, 2H), 7.50 (t, *J* = 7.5 Hz, 2H), 7.61 (t, *J* = 7.5 Hz, 1H), 8.02-8.06 (m, 2H), 8.30 (s, 1H), 10.79 (br s, 1H). |
| 10-139 | 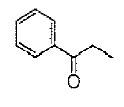 | 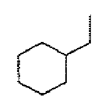 | 1.05-1.38 (m, 6H), 1.50 (br s, 2H), 1.63-1.77 (m, 10H), 1.82-1.93 (m, 1H), 2.65 (t, *J* = 6.0 Hz, 2H), 2.93 (t, *J* = 6.0 Hz, 2H), 4.04 (br s, 2H), 4.97 (d, *J* = 4.5 Hz, 2H), 7.50 (t, *J* = 7.5 Hz, 2H), 7.61 (t, *J* = 7.5 Hz, 1H), 8.02-8.06 (m, 2H), 8.31 (s, 1H), 10.79 (br s, 1H). |
| 10-140 | 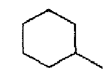 | 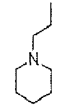 | 1.31-1.51 (m, 10H), 1.57-1.79 (m, 14H), 1.94-2.01 (m, 2H), 2.50-2.66 (m, 8H), 3.90-4.01 (m, 1H), 4.28 (br t, *J* = 7.5 Hz, 2H), 8.30 (s, 1H), 9.84 (d, *J* = 7.8 Hz, 1H). |

**Table 134**

| | | | |
|---|---|---|---|
| 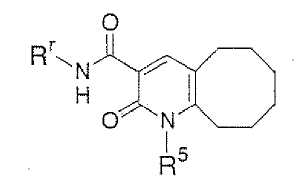 | | | |

| Compound No. | R^{r} | R⁵ | ¹H-NMR (CDCl₃) |
|---|---|---|---|
| 10-141 | 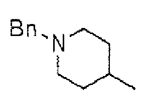 | 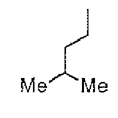 | 1.01 (t, *J* = 6.3 Hz, 6H), 1.34-1.53 (m, 4H), 1.56-1.80 (m, 9H), 1.97-2.05 (m, 2H), 2.17-2.27 (m, 2H), 2.62 (t, *J* = 6.0 Hz, 2H), 2.81-2.89 (m, 2H), 2.87 (t, *J* = 6.0 Hz, 2H), 3.54 (s, 2H), 3.96-4.04 (m, 1H), 4.11 (br t, *J* = 7.5 Hz, 2H), 7.26-7.35 (m, 5H), 8.27 (s, 1H), 9.96 (d, *J* = 7.2 Hz, 1H). |
| 10-142 | 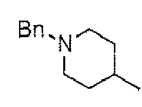 | 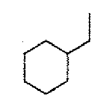 | 1.05-1.36 (m, 8H), 1.48 (br s, 2H), 1.64-1.88 (m, 11H), 1.96-2.05 (m, 2H), 2.22 (t, *J* = 9.9 Hz, 2H), 2.63 (t, *J* = 6.0 Hz, 2H), 2.81-2.86 (m, 2H), 2.91 (t, *J* = 6.0 Hz, 2H), 3.54 (s, 2H), 3.93-4.02 (m, 3H), 7.23-7.36 (m, 5H), 8.29 (s, 1H), 9.96 (d, *J* = 7.8 Hz, 1H). |
| 10-143 | 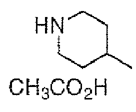 | 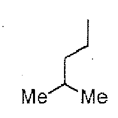 | 1.02 (d, *J* = 6.6 Hz, 6H), 1.36-1.73 (m, 8H), 1.77 (quint, *J* = 6.0 Hz, 4H), 1.98-2.10 (m, 1H), 2.05 (s, 3H), 2.21-2.30 (m, 2H), 2.61-2.68 (m, 2H), 2.89 (t, *J* = 6.0 Hz, 2H), 3.09 (t, *J* = 9.9 Hz, 2H), 3.42-3.50 (m, 2H), 4.05-4.21 (m, 3H), 8.25 (s, 1H), 10.27 (d, *J* = 6.3 Hz, 1H). |
| 10-144 | 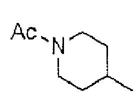 | 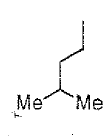 | 1.01 (d, *J* = 6.6 Hz, 6H), 1.36-1.72 (m, 9H), 1.76 (quint, *J* = 6.0 Hz, 4H), 1.99-2.10 (m, 2H), 2.11 (s, 3H), 2.64 (t, *J* = 6.0 Hz, 2H), 2.88 (t, *J* = 6.0 Hz, 2H), 2.91-3.00 (m, 1H), 3.21-3.03 (m, 1H), 3.76-3.81 (m, 1H), 4.10 (br t, *J* = 7.5 Hz, 2H), 4.11-4.25 (m, 1H), 4.36-4.44 (m, 1H), 8.28 (s, 1H), 10.07 (d, *J* = 7.2 Hz, 1H). |
| 10-145 | 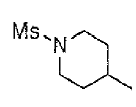 | 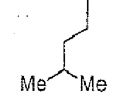 | 1.01 (d, *J* = 6.3 Hz, 6H), 1.37-1.80 (m, 13H), 2.08-2,16 (m, 2H), 2.64 (t, *J* = 6.0 Hz, 2H), 2.81 (s, 3H), 2.88 (t, *J* = 6.0 Hz, 2H), 2.89-2.99 (m, 2H), 3.68-3.76 (m, 2H), 4.05-4.16 (m, 3H), 8.27 (s, 1H), 10.10 (d, *J* = 7.2 Hz, 1H). |
| 10-146 | 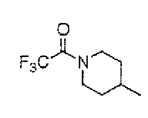 | 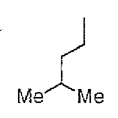 | 1.01 (d, *J* = 6.3 Hz, 6H), 1.38-1.78 (m, 13H), 2.08-2.15 (m, 2H), 2.64 (t, *J* = 6.0 Hz, 2H), 2.89 (t, *J* = 6.0 Hz, 2H), 3.11-3.21 (m, 1H), 3.31-3.41 (m, 1H), 3.96 (t, *J* = 14.4 Hz, 1H), 4.14 (br t, *J* = 7.5 Hz, 2H), 4.21-4.38 (m, 2H), 8.28 (s, 1H), 10.16 (d, *J* = 7.5 Hz, 1H). |

**Table 135**

| | | | |
|---|---|---|---|
| 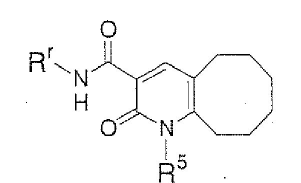 | | | |

| Compound No. | R^{r} | R⁵ | ¹H-NMR (CDCl₃) |
|---|---|---|---|
| 10-147 | 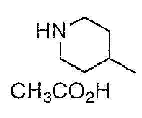 | 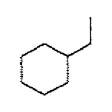 | 1.06-1.40 (m, 8H), 1.49 (br s, 2H), 1.61-1.99 (m, 11H), 2.18-2.28 (m, 2H), 2.64 (t, *J* = 6.0 Hz, 2H), 2.92 (t, *J* = 6.0 Hz, 2H), 3.04 (t, *J* = 10.5 Hz, 2H), 3.38-3.44 (m, 2H), 4.05-4.20 (m, 3H), 8.26 (s, 1H), 10.23 (d, *J* = 7.2 Hz, 1H). |
| 10-148 | 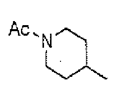 | 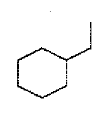 | 1.02-1.33 (m, 8H), 1.45-1.80 (m, 13H), 1.98-2.10 (m, 2H), 2.11 (s, 3H), 2.65 (t, *J* = 6.0 Hz, 2H), 2.92 (t, *J* = 6.0 Hz, 2H), 2.93-3.01 (m, 1H), 3.21-3.31 (m, 1H), 3.74-3.81 (m, 1H), 3.99 (br s, 1H), 4.10-4.23 (m, 2H), 4.37-4.43 (m, 1H), 8.29 (s, 1H), 10.08 (d, *J* =7.8 Hz, 1H). |
| 10-149 | 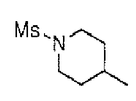 | 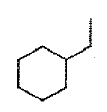 | 1.02-1.33 (m, 8H), 1.49 (br s, 2H), 1.60-1.81 (m, 11H), 2.08-2.15 (m, 2H), 2.65 (t, *J* = 6.0 Hz, 2H), 2.81 (s, 3H), 2.90-3.00 (m, 4H), 3.69-3.76 (m, 2H), 3.99 (br s, 1H), 4.06-4.12 (m, 2H), 8.28 (s, 1H), 10.10 (d, *J* = 7.2 Hz, 1H). |
| 10-150 | 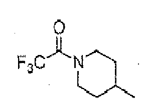 | 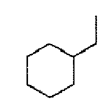 | 1.03-1.38 (m, 8H), 1.49 (br s, 2H), 1.60-1.85 (m, 11H), 2.07-2.17 (m, 2H), 2.65 (t, *J* = 6.0 Hz, 2H), 2.91 (t, *J* = 6.0 Hz, 2H), 3.11-3-21 (m, 1H), 3.31-3.41 (m, 1H), 3.96 (d, *J* = 14.4 Hz, 2H), 4.00 (br s, 1H), 4.22-4.38 (m, 2H), 8.29 (s, 1H), 10.17 (d, *J* = 7.5 Hz, 1H). |
| 10-151 | 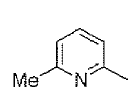 | nBu | 1.00 (t, *J* = 7.2 Hz, 3H), 1.37-1.54 (m, 4H), 1.47 (sextet, *J* = 7.2 Hz, 2H), 1.66-1.83 (m, 6H), 2.49 (s, 3H), 2.67 (t, *J* = 6.0 Hz, 2H), 2.92 (t, *J* = 6.0 Hz, 2H), 4.16 (br t, *J* = 7.2 Hz, 2H), 6.87 (d, *J* = 7.5 Hz, 1H), 7.59 (t, *J* = 7.5 Hz, 1H), 8.15 (d, *J* = 8.4 Hz, 1H), 8.37 (s, 1H), 12.55 (br s, 1H). |
| 10-152 | 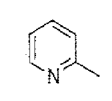 | nBu | 0.99 (t, *J* = 7.2 Hz, 3H), 1.38-1.54 (m, 4H), 1.46 (sextet, *J* = 7.2 Hz, 2H), 1.66-1.83 (m, 6H), 2.67 (t, *J* = 6.0 Hz, 2H), 2.92 (t, *J* = 6.0 Hz, 2H), 4.15 (t, *J* = 7.2 Hz, 2H), 6.99-7.04 (m, 1H), 7.67-7.74 (m, 1H), 8.33-8.37 (m, 2H), 8.36 (s, 1H), 12.77 (br s, 1H). |
| 10-153 | 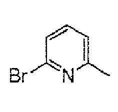 | 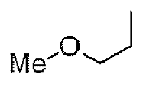 | 1.36-1.43 (m, 2H), 1.47-1.55 (m, 2H), 1.65-1.80 (m, 4H), 2.69 (t, *J* = 6.0 Hz, 2H), 3.08 (t, *J* = 6.0 Hz, 2H), 3.31 (s, 3H), 3.74 (t, *J* = 5.1 Hz, 2H), 4.39 (t, *J* = 5.1 Hz, 2H), 7.18 (dd, *J* = 7.5 Hz, 0.9 Hz, 1H), 7.54 (t, *J* = 7.8 Hz, 1H), 8.33 (dd, *J* = 8.1 Hz, 0.9 Hz, 1H), 8.37 (s, 1H), 12.62 (br s, 1H). |

**Table 136**

| | | | |
|---|---|---|---|
| 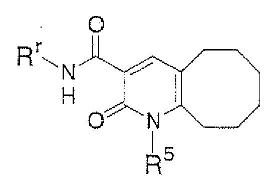 | | | |

| Compound No. | R^{r} | R⁵ | ¹H-NMR (CDCl₃) |
|---|---|---|---|
| 10-154 | 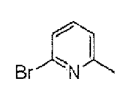 | 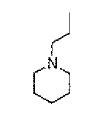 | 1.38-1.83 (m, 14H), 2.52-2.73 (m, 6H), 2.67 (t, *J* = 6.0 Hz, 2H), 3.02 (br t, *J* = 6.0 Hz, 2H), 4.39 (br t, *J* = 7.5 Hz, 2H), 7.19 (dd, *J* = 7.5 Hz, 0.6 Hz, 1H), 7.54 (t, *J* = 7.5 Hz, 1H), 8.33 (dd, *J* = 8.4 Hz, 0.6 Hz, 1H), 8.35 (s, 1H), 12.58 (br s, 1H). |
| 10-155 | 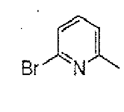 | 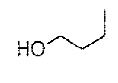 | 1.37-1.55 (m, 4H), 1.67-1.86 (m, 4H), 1.97 (quint, *J* = 6.0 Hz, 2H), 2.68 (t, J = 6.0 Hz, 2H), 2.97 (t, *J* = 6.0 Hz, 2H), 3.60 (t, *J* = 5.7 Hz, 2H), 4.39 (br t, *J* = 7.5 Hz, 2H), 7.20 (dd, *J* = 7.5 Hz, 0.6 Hz, 1H), 7.54 (t, *J* = 7.8 Hz, 1H), 8.33 (dd, *J* = 8.1 Hz, 0.6 Hz, 1H), 8.40 (s, 1H), 12.45 (br s, 1H). |
| 10-156 | 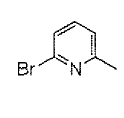 | 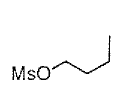 | 1.39-1.46 (m, 2H), 1.47-1.56 (m, 2H), 1.66-1.74 (m, 2H), 1.77-1.85 (m, 2H), 2.23 (quint, *J* = 6.0 Hz, 2H), 2.68 (t, *J* = 6.0 Hz, 2H), 2.94 (t, *J* = 6.0 Hz, 2H), 3.10 (s, 3H), 4.33 (t, *J* = 7.5 Hz, 2H), 4.40 (t, *J* = 6.0 Hz, 2H), 7.19 (dd, *J* = 7.5 Hz, 0.6 Hz, 1H), 7.55 (t, *J* = 7.8 Hz, 1H), 8.32 (dd, *J* = 8.4 Hz, 0.6 Hz, 1H), 8.37 (s, 1H), 12.56 (br s, 1H). |
| 10-157 | 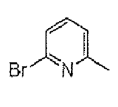 | 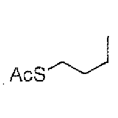 | 1.37-1.45 (m, 2H), 1.47-1.56 (m, 2H), 1.66-1.74 (m, 2H), 1.76-1.85 (m, 2H), 2.03 (quint, *J* = 7.5 Hz, 2H), 2.37 (s, 3H), 2.66 (t, *J* = 6.0 Hz, 2H), 2.92 (t, *J* = 6.0 Hz, 2H), 3.00 (t, *J* = 7.2 Hz, 2H), 4.21 (t, *J* = 7.5 Hz, 2H), 7.18 (d, *J* = 7.5 Hz, 1H), 7.54 (t, *J* = 7.5 Hz, 1H), 8.32 (d, *J* = 8.4 Hz, 1H), 8.35 (s, 1H), 12.59 (br s, 1H). |
| 10-158 | 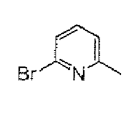 | 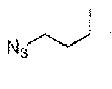 | 1.38-1.45 (m, 2H), 1.47-1.56 (m, 2H), 1.66-1.74 (m, 2H), 1.77-1.86 (m, 2H), 1.97-2,07 (m, 2H), 2.68 (t, *J* = 6.0 Hz, 2H), 2.95 (t, *J* = 6.0 Hz, 2H), 3.52 (t, *J* = 6.0 Hz, 2H), 4.25 (t, *J* = 7.5 Hz, 2H), 7.19 (dd, *J* = 7.5 Hz, 0.6 Hz, 1H), 7.54 (t, *J* = 7.8 Hz, 1H), 8.32 (dd, *J* = 8.4 Hz, 0.6 Hz, 1H), 8.36 (s, 1H), 12.58 (br s, 1H). |

**Table 137**

| | | | |
|---|---|---|---|
| 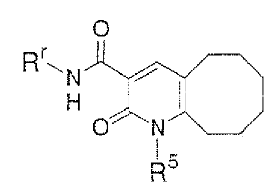 | | | |

| Compound No. | R^{r} | R⁵ | ¹H-NMR (CDCl₃) |
|---|---|---|---|
| 10-159 | 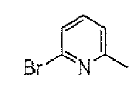 | 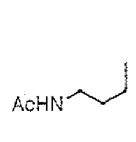 | 1.38-1.44 (m, 2H), 1.47-1.56 (m, 2H), 1.65-1.74 (m, 2H), 1.76-1.84 (m, 2H), 1.94 (quint, *J* = 6.6 Hz, 2H), 2.10 (s, 3H), 2.68 (t, *J* = 6.0 Hz, 2H), 2.93 (t, *J* = 6.0 Hz, 2H), 3.30 (q, *J* = 6.0 Hz, 2H), 4.26 (br t, *J* = 7.5 Hz, 2H), 6.63 (br t, *J* = 7.5 Hz, 1H), 7.21 (dd, *J* = 8.1 Hz, 0.6 Hz, 1H), 7.56 (t, *J* = 7.8 Hz, 1H), 8.34 (dd, *J* = 8.1 Hz, 0.6 Hz, 1H), 8.39 (s, 1H), 12.51 (br s, 1H). |
| 10-160 | 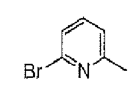 | 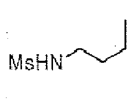 | 1.41-1.85 (m, 8H), 2.01-2.11 (m, 2H), 2.68 (t, *J* = 6.0 Hz, 2H), 2.94 (t, *J* = 6.0 Hz, 2H), 3.04 (s, 3H), 3.18 (q, *J* = 6.0 Hz, 2H), 4.34 (br t, *J* = 7.5 Hz, 2H), 5.58 (br t, *J* = 7.5 Hz, 1H), 7.20 (dd, *J* = 7.5 Hz, 0.6 Hz, 1H), 7.55 (t, *J* = 7.8 Hz, 1H), 8.31 (dd, *J* = 8.1 Hz, 0.6 Hz, 1H), 8.39 (s, 1H), 12.45 (br s, 1H). |
| 10-161 | 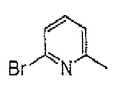 | 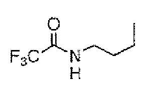 | 1.42-1.83 (m, 8H), 2.03 (quint, *J* = 6.0 Hz, 2H), 2.69 (t, *J* = 6.0 Hz, 2H), 2.93 (t, *J* = 6.0 Hz, 2H), 3.41 (q, *J* = 6.0 Hz, 2H), 4.30 (br t, *J* = 7.5 Hz, 2H), 7.22 (dd, *J* = 7.8 Hz, 0.6 Hz, 1H), 7.56 (t, *J* = 7.8 Hz, 1H), 8.13 (br s, 1H), 8.31 (dd, *J* = 7.8 Hz, (9.6 Hz, 1H), 8.42 (s, 1H), 12.40 (br s, 1H). |
| 10-162 | 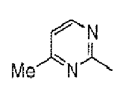 | nBu | 0.99 (t, *J* = 7.2 Hz, 3H), 1.39-1.57 (m, 4H), 1.47 (sextet, *J* = 7.2 Hz, 2H), 1.65-1.83 (m, 6H), 2.51 (s, 3H), 2.68 (t, *J* = 6.0 Hz, 2H), 2.92 (t, *J* = 6:0 Hz, 2H), 4.16 (br t, *J* = 7.2 Hz, 2H), 6.86 (d, *J* = 4.8 Hz, 1H), 8.41 (s, 1H), 8.54 (d, *J* = 4.8 Hz, 1H). 12.91 (br s, 1H). |
| 10-163 | 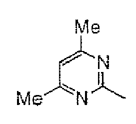 | nBu | 0.99 (t, *J* = 7.5 Hz, 3H), 1.39-1.53 (m, 4H), 1.46 (sextet, *J* = 7.5 Hz, 2H), 1.65-1.82 (m, 6H), 2.47 (s, 6H), 2.67 (t, *J* = 6.0 Hz, 2H), 2.91 (t, *J* = 6.0 Hz, 2H), 4.15 (br t, *J* = 7.5 Hz, 2H), 6.74 (s 1H), 8.41 (s, 1H), 12,75 (br s, 1H). |
| 10-164 | 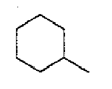 | 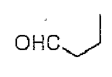 | 1.22-1.52 (m, 8H), 1.63-1.79 (m, 8H), 1.92-2.00 (m, 2H), 2.64 (t, *J* = 6.0 Hz, 2H), 2.89 (t, *J* = 6.0 Hz, 2H), 2.96 (t, *J* = 7.2 Hz, 2H), 3.92-4.03 (m, 1H), 4.42 (t, *J* = 7.2 Hz, 2H), 8.32 (s, 1H), 9.75 (d, *J* = 7.5 Hz, 1H), 9.84 (s, 1H). |

**Table 138**

| | | | |
|---|---|---|---|
| 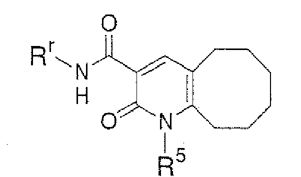 | | | |

| Compound No. | R^{r} | R⁵ | ¹H-NMR (CDCl₃) |
|---|---|---|---|
| 10-165 | 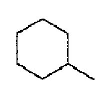 | 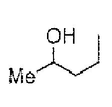 | 1.20 (d, *J* = 6.6 Hz, 3H), 1.21-1.87 (m, 18H), 1.90-2.01 (m, 2H), 2.55-2.73 (m, 2H), 2.85-3.02 (m, 2H), 3.62-3.70 (m, 1H), 3.92-4.01 (m, 2H), 4.65-4.78 (m, 1H), 8.36 (s, 1H), 9.77 (d, *J* = 7.5 Hz, 1H). |
| 10-166 | 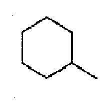 | 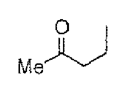 | 1.23-1.51 (m, 8H), 1.58-1.78 (m, 8H), 1.94-2.00 (m, 2H), 2.20 (s, 3H), 2.63 (t, *J* = 6.0 Hz, 2H), 2.88 (t, *J* = 6.0 Hz, 2H), 2.93 (t, *J* = 7.5 Hz, 2H), 3.92-4.01 (m, 1H), 4.36 (t, *J* = 7.5 Hz, 2H), 8.31 (s, 1H), 9.78 (d, *J* = 8.1 Hz, 1H). |
| 10-167 | 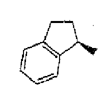 | 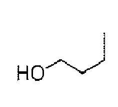 | 1.38-1.43 (m, 2H), 1.44-1.52 (m, 2H), 1.65-1.83 (m, 4H), 1.86-1.95 (m, 2H), 1.91-2.05 (m, 1H), 2.63-2.74 (m, 1H), 2.67 (t, *J* = 6.0 Hz, 2H), 2.85-2.96 (m, 1H), 2.93 (t, *J* = 6.0 Hz, 2H), 2.99-3.09 (m, 1H), 3.51 (br t, *J* = 4.5 Hz, 2H), 4.22-4.38 (m, 2H), 5.66 (q, *J* = 7.5 Hz, 1H), 7.19-7-38 (m, 4H), 8.43 (s, 1H), 10.11 (d, *J* = 6.9 Hz, 1H). |
| 10-168 | 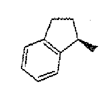 | 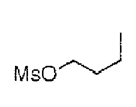 | 1.38-1.42 (m, 2H), 1.44-1.52 (m, 2H), 1.64-1.80 (m, 4H), 1.91.2.08 (m, 1H), 2.11-2.21 (m, 2H), 2.62-2.73 (m, 1H), 2.67 (t, *J* = 6.0 Hz, 2H), 2.83-2.96 (m, 1H), 2.91 (t, *J* = 6.0 Hz, 2H), 2.99-3.06 (m, 1H), 3.02 (s, 3H), 4.25 (t, *J* = 6.9 Hz, 2H), 4.33 (t, *J* = 6.0 Hz, 2H), 5.67 (q, *J* = 7.8 Hz, 1H), 7.16-7.26 (m, 3H), 7.35-7.39 (m, 1H), 8.39 (s, 1H), 10.13 (d, *J* = 8.4 Hz, 1H). |
| 10-169 | 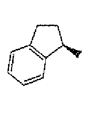 | 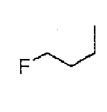 | 1.37-1.43 (m, 2H), 1.44-1.53 (m, 2H), 1.67-1.80 (m, 4H), 1.91-2.10 (m, 1H), 2.00-2.20 (m, 2H), 2.62-2.73 (m, 1H), 2.6 (t, *J* = 6.0 Hz, 2H), 2.84-2.96 (m, 1H), 2.93 (t, *J* = 6.0 Hz, 2H), 2.98-3.08 (m, 1H), 4.25 (sextet, *J* = 7.5 Hz, 2H), 4.45 (t, *J* = 7.8 Hz, 1H), 4.61 (t, *J* = 5.4 Hz, 1H), 5.67 (q, *J* = 7.5 Hz, 1H), 7.16-7,28 (m, 3H), 7.35-7.39 (m, 1H), 8.39 (s, 1H), 10.17 (d, *J* = 6.6 Hz, 1H). |

**Table 139**

| | | | |
|---|---|---|---|
| 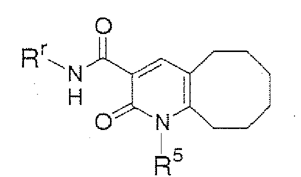 | | | |

| Compound No. | R^{r} | R⁵ | ¹H-NMR (CDCl₃) |
|---|---|---|---|
| 10-170 | 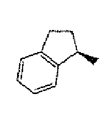 | 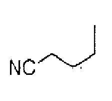 | 1.38-1.43 (m, 2H), 1.44-1.54 (m, 2H), 1.67-1.82 (m, 4H), 1.91-2.06 (m, 1H), 2.06 (quint, *J* = 7.5 Hz, 2H), 2.49 (t, *J* = 7.2 Hz, 2H), 2.62-2.74 (m, 1H), 2.67 (t, *J* = 6.0 Hz, 2H), 2.85-2.96 (m, 2H), 2.91 (t, *J* = 6.0 Hz, 2H), 2.99-3.09 (m, 1H), 4.21 (sextet, *J* = 7.2 Hz, 2H), 5.67 (q, *J* = 7.5 Hz, 1H), 7.19-7.26 (m, 3H), 7.35-7.39 (m, 1H), 8.40 (s, 1H), 10.08 (d, *J* = 8.1 Hz, 1H). |
| 10-171 | 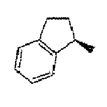 | 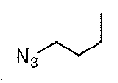 | 1.37-1.44 (m, 2H), 1.45-1.55 (m, 2H), 1.67-1.80 (m, 4H), 1.93-2.03 (m, 3H), 2.62-2.73 (m, 1H), 2.66 (t, *J* = 6.0 Hz, 2H), 2.84-2.96 (m, 1H), 2.91 (t, *J* = 6.0 Hz, 2H), 2.99-3.09 (m, 1H), 3.50 (t, J = 6.0 Hz, 2H), 4.17 (sextet, *J* = 7.5 Hz, 2H), 5.6 (q, *J* = 7.8 Hz, 1H), 7.18-7.26 (m, 3H), 7.35-7.39 (m, 1H), 8.39 (s, 1H), 10.16 (d, *J* = 8.4 Hz, 1H). |
| 10-172 | 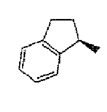 | 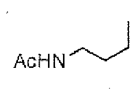 | 1.37-1.43 (m, 2H), 1.44-1.52 (m, 2H), 1.63-1.78 (m, 4H), 1.83-2.02 (m, 1H), 1.87 (quint, *J* = 6.0 Hz, 2H), 1.95 (s, 3H), 2.67 (t, *J* = 6.0 Hz, 2H), 2.69-2.76 (m, 1H), 2.88-2.95 (m, 1H), 2.90 (t, *J* = 6.0 Hz, 2H), 2.97-3.08 (m, 1H), 3.23 (quint, *J* = 6.0 Hz, 2H), 4.19 (br t, *J* = 7.5 Hz, 2H), 5.67 (q, *J* = 7.5 Hz, 1H), 6.65 (br t, *J* = 7.5 Hz, 1H), 7.18-7.28 (m, 3H), 7.36-7.39 (m, 1H), 8.41 (s, 1H), 10. 15 (d, *J* = 8.1 Hz, 1H). |
| 10-173 | 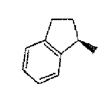 | 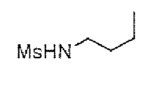 | 1.37-1.42 (m, 2H), 1.44-1.53 (m, 2H), 1.63-1.78 (m, 4H), 1.90-2.02 (m, 3H), 2.62-2.73 (m, 1H), 2.67 (t, *J* = 6.0 Hz, 2H), 2-84 (s, 3H), 2.85-2.97 (m, 1H), 2.90 (t, *J* = 6.0 Hz, 2H), 3.00-3.10 (m, 2H), 4.25 (br s, 2H), 5.67 (q, *J* = 7.5 Hz, 2H), 7.19-7.36 (zu, 4H), 8.42 (s, 1H), 0.06 (d, *J* = 8.1 Hz, 1H). |
| 10-174 | 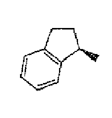 | 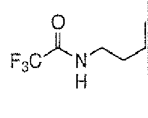 | 1.38-1.43 (m, 2H), 1.44-1.52 (m, 2H), 1.65-1.80 (m, 4H), 1.88-2.00 (m, 3H), 2.68 (t, *J* = 6.0 Hz, 2H), 2.69-2.76 (m, 1H), 2.88-2.98 (m, 1H), 2.91 (t, *J* = 6.0 Hz, 2H), 3.00-3.10 (m, 1H), 3.25-3.37 (m, 2H), 4.24 (br s, 2H), 5.61 (q, *J* = 7.5 Hz, 1H), 7.18-7.39 (m, 4H), 8.42 (br s, 1H), 8.44 (s, 1H), 10.05 (d, *J* = 7.2 Hz, 1H). |

**Table 140**

| | | | |
|---|---|---|---|
| 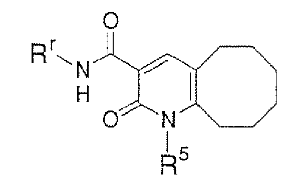 | | | |

| Compound No. | R^{r} | R⁵ | ¹H-NMR (CDCl₃) |
|---|---|---|---|
| 10-175 | 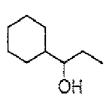 | nBu | 0.99 (t, *J* = 7.5 Hz, 3H), 1.02-1.28 (m, 6H), 1.36-1.52 (m, 8H), 1.62-1.80 (m, 8H), 1.92 (br d, *J* = 12.0 Hz, 1H), 2.64 (t, *J* = 6.0 Hz, 2H), 2.89 (t, *J* = 6.0 Hz, 2H), 3.45-3.62 (m, 3H), 4.07-4.15 (m, 2H), 8.30 (s, 1H), 10.28 (br s, 1H). |
| 10-176 | 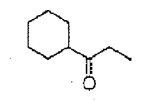 | nBu | 0.98 (t, *J* = 7.5 Hz, 3H), 1.20-1.51 (m, 14H), 1.58-1.91 (m, 8H), 2.41-2.50 (m, 1H), 2.63 (t, *J* = 6.0 Hz, 2H), 2.88 (t, *J* = 6.0 Hz, 2H), 4.12 (br t, *J* = 7.5 Hz, 2H), 4.36 (d, *J* = 5.4 Hz, 2H), 8.26 (s, 1H), 10.50 (br s, 1H). |
| 10-177 | 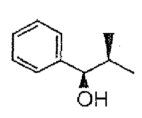 | nBu | 0.98 (t, *J* = 7.5 Hz, 3H), 1.15 (d, *J* = 6.6 Hz, 3H), 1.37-1.53 (m, 4H), 1.44 (sextet, *J* = 7.5 Hz, 2H), 1.61-1.81 (m, 6H), 2.66 (t, *J* = 6.0 Hz, 2H), 2.90 (t, *J* = 6.0 Hz, 2H), 4.09 (br t, *J* = 7.5 Hz, 2H), 4.39-4.48 (m, 1H), 4.98 (d, *J* = 2. Hz, 1H), 7.23-7.39 (m, 5H), 8.33 (s, 1H), 10.10 (d, *J* = 7.5 Hz, 1H). |
| 10-178 | 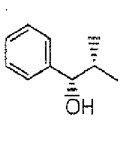 | nBu | 0.98 (t, *J* = 7.2 Hz, 3H), 1.15 (d, *J* = 6.9 Hz, 3H), 1.37-1.53 (m, 4H), 1.44 (sextet, *J* = 7.2 Hz, 2H), 1.62-1.80 (m, 6H), 2.66 (t, *J* = 6.0 Hz, 2H), 2.90 (t, *J* = 6.0 Hz, 2H), 4.09 (br t, *J* = 7.2 Hz, 2H), 4.39-4.49 (m, 1H), 4.98 (d, *J* = 2.7 Hz, 1H), 7.23-7.40 (m, 5H), 8.33 (s, 1H), 10.10 (d, *J* = 6.9 Hz, 1H). |
| 10-179 | 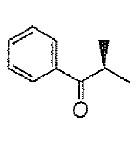 | nBu | 0.99 (t, *J* = 7.5 Hz, 3H), 1.37-L50 (m, 4H), 1.43 (sextet, *J* = 7.5 Hz, 2H), 1.54 (d, *J* = 7.5 Hz, 3H), 1.63-1.80 (m, 6H), 2.63 (t, *J* = 6.0 Hz, 2H), 2.88 (t, *J* = 6.0 Hz, 2H), 4.12 (br t, *J =* 7.5 Hz, 2H), 5.69-5.79 (m, 1H), 7.45-7.51 (m, 2H), 7.55-7.61 (m, 1H), 8.05-8.09 (m, 2H), 8.28 (s, 1H), 10.73 (d, *J* = 6.9 Hz, 1H). |
| 10-180 | 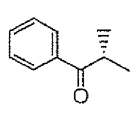 | nBu | 0.99 (t, *J* = 7.5 Hz, 3H), 1.37-1.50 (m, 4H), 1.43 (sextet, *J* = 7.5 Hz, 2H), 1.54 (d, *J* = 6.9 Hz, 3H), 1.65-1.80 (m, 6H), 2.63 (t, *J* = 6.0 Hz, 2H), 2.89 (t, *J* = 6.0 Hz, 2H), 4.12 (br t, *J* = 7.5 Hz, 2H), 5.69-5.79 (m, 1H), 7.45-7.51 (m, 2H), 7.55-7.6 (m, 1H), 8.05-8.09 (m, 2H), 8.28 (s, 1H), 10.73 (d, *J* = 7.2 Hz, 1H). |

**Table 141**

| | | | |
|---|---|---|---|
| 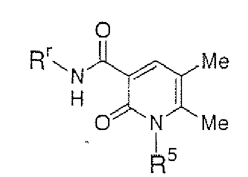 | | | |

| Compound No. | R^{r} | R⁵ | ¹H-NMR (CDCl₃) |
|---|---|---|---|
| 11-001 | 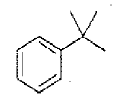 | nBu | 1.01 (t, *J* = 7.2 Hz, 3H), 1.42-1.54 (m, 2H), 1.65-1.79 (m, 2H), 1.79 (s, 6H), 2.13 (s, 3H), 2.41 (s, 3H), 4.13 (t, *J* = 7.8 Hz, 2H), 7.16-7.22 (m, 2H), 7.26-7.33 (m, 2H), 7.42-7.46 (m, 2H), 8.25 (s, 3.H), 10.40 (br s, 1H). |
| 11-002 | 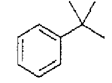 | Bn | 1.79 (s, 6H), 2.13 (s, 3H), 2.29 (s, 3H), 5.50(br s, 2H), 7.09-7.47 (m, 10H), 8.35 (s, 1H), 10.35 (br s, 1H). |
| 11-003 | 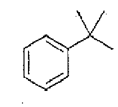 | 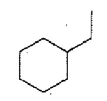 | 1.05-1.32 (m, 4H), 1.58-1.91 (m, 7H), 1.79 (s, 6H), 2.12 (s, 3H), 2.38 (s, 3H), 4.01 (br s, 2H), 7.16-7.21 (m, 1H), 7.26-7.32 (m, 2H), 7.43-7.46 (m, 2H), 8.24 (s, 1H), 10.39 (br s, 1H). |
| 11-004 | 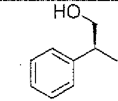 | 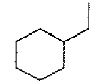 | 1.00-1.30 (m, 4H), 1.55-1.90 (m, 7H), 2.18 (s, 3H), 2.40 (s, 3H), 3.89-4.00 (m, 2H), 4.03 (br s, 2H), 5.26-5.32 (m, 1H), 7.26-7.43 (m, 5H), 8.33 (s, 1H), 10.72 (br d, *J* = 6.9 Hz, 1H). |
| 11-005 | 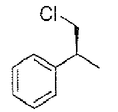 | 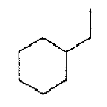 | 1.00-1.30 (m, 4H), 1.60-1.92 (m, 7H), 2.17 (s, 3H), 2.39 (s, 3H), 3.90 (d, *J* = 6.0 Hz, 2H), 4.04 (br s, 2H), 5.50-5.56 (m, 1H), 7.26-7.44 (m, 5H), 8.30 (s, 1H), 10.73 (d, *J* = 8.1 Hz, 1H). |
| 11-006 | 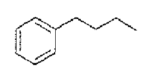 | 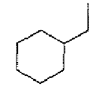 | 1.00-1.30 (m, 4H), 1.56-1.88 (m, 7H), 1.90-2.00 (m, 2H), 2.18 (s, 3H), 2.39 (s, 3H), 2.71 (t, *J* = 8.1 Hz, 2H), 3.46 (quint, *J* = 6.9 Hz, 2H), 4.03 (br s, 2H), 7.14-7.30 (m, 5H), 8.32 (s, 1H), 9.98 (br s, 1H). |
| 11-007 | 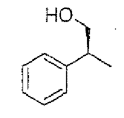 | nBu | 0.99 (t, *J* = 7.5 Hz, 3H), 1.39-1.51 (m, 2H), 1.62-1.73 (m, 2H), 2.18 (s, 3H), 2.42 (s, 3H), 3.89-4.00 (m, 2H), 4.12 (dd, *J* = 9.0 Hz, *J* = 5.1 Hz, 2H), 5.26-5.32 (m, 1H), 7.26-7.43 (m, 5H), 8.32 (s, 1H), 10.72 (br d, *J* = 6.9 Hz, 1H). |
| 11-008 | 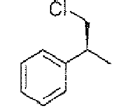 | nBu | 1.00 (t, *J* = 7.5 Hz, 3H), 1.41-1.53 (m, 2H), 1.64-1.74 (m, 2H), 2.16 (s, 3H), 2.41 (s, 3H), 3.91 (d, *J* = 5.7 Hz, 2H), 4.13 (t, *J* = 7.5 Hz, 2H), 5.50-5.57 (m, 1H), 7.28-7.45 (m, 5H), 8.30 (s, 1H), 10.73 (br d, *J* = 8.1 Hz, 1H). |
| 11-009 | 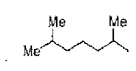 | 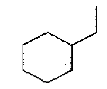 | 0.84 (d, *J* = 6.6 Hz, 6H), 1.06-1-85 (m, 21H), 2.17 (s, 3H), 2.38 (s, 3H), 4.00 (br s, 2H), 4.09-4.18 (m, 1H), 8.31 (s, 1H), 9.77 (d, *J* = 7.5 Hz, 1H). |

**Table 142**

| | | | |
|---|---|---|---|
| 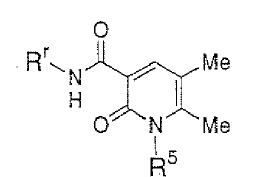 | | | |

| Compound No. | R^{r} | R⁵ | ¹H-NMR (CDCl₃) |
|---|---|---|---|
| 11-010 | 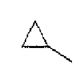 | 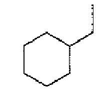 | 0.60-0.65 (m, 2H), 0.77-0.84 (m, 2H), 1.05-1.26 (m, 5H), 1.59-1.85 (m, 6H), 2.18 (s, 3H), 2.38 (s, 3H), 2.89-2.98 (m, 1H), 4.00 (br s, 2H), 8.32 (s, 1H), 9.89 (br s, 1H). |
| 11-011 | 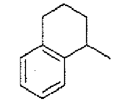 | 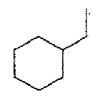 | 0.86-2.19 (m, 15H), 2.19 (s, 3H), 2.38 (s, 3H), 2.72-2.91 (m, 2H), 3.94 (br s, 2H), 5.37-5.44 (m, 1H), 7.06-7.16 (m, 3H), 7.34-7.37 (m, 1H), 8.38 (s, 1H), 10.22 (br d, *J* = 8.7 Hz, 1H). |
| 11-012 | 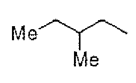 | 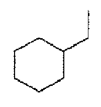 | 0.92 (t, *J* = 7.5 Hz, 3H), 0.95 (d, *J* = 6.6 Hz, 3H), 1.06-1.85 (m, 14H), 2.18 (s, 3H), 2.39 (s, 3H), 3.20-3.29 (m, 1H), 3.34-3.42 (m, 1H), 4.03 (br s, 2H), 8.32 (s, 1H), 9.95 (br s, 1H). |
| 11-013 | 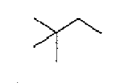 | 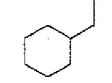 | 0.98 (s, 9H), 1.07-1.23 (m, 5H), 1.62-1.83 (m, 6H), 2.18 (s, 3H). 2.39 (s, 3H), 3.26 (d, *J* = 6.0 Hz, 2H), 4.03 (br s, 2H), 8.33 (s, 1H), 10.06 (br s, 1H). |
| 11-014 | 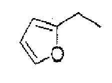 | 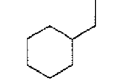 | 1.05-1.23 (m, 5H), 1.62-1.87 (m, 6H), 2.18 (s, 3H), 2.39 (s, 3H), 4.00 (br s, 2H), 4.62 (d, J= 5.4 Hz, 2H), 6.25-6.31 (m, 2H), 7.35 (s, 1H), 8.34 (s, 1H), 10.23 (br s, 1H). |
| 11-015 | 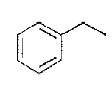 | 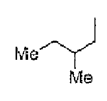 | 0.88 (d, *J* = 6.9 Hz, 3H), 0.93 (t, *J* = 7.5 Hz, 3H), 1.16-1.30 (m, 1H), 1.35-1.48 (m, 1H), 1.89-2.00 (m, 1H), 2.19 (s, 3H), 2.39 (s, 3H), 4.03 (br s, 1H), 4.64 (d, *J* = 6.0 Hz, 2H), 7.20-7.38 (m, 5H), 8.37 (s, 1H), 10.30 (br s, 1H). |
| 11-016 | 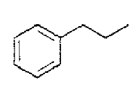 | 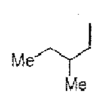 | 0.90 (t, *J* = 7.2 Hz, 3H), 0.95 (t, *J* = 7.5 Hz, 3H), 1.17-1.32 (m, 1H), 1.35-1.49 (m, 1H), 1.88-2.00 (m, 1H), 2.18 (s, 3H), 2.39 (s, 3H), 2.93 (t, *J* = 7.5 Hz, 2H), 3.62-3.69 (m, 2H), 4.06 (br s, 2H), 7.17-7.31 (m, 5H), 8.33 (s, 1H), 10.03 (br s, 1H). |
| 11-017 | 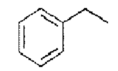 | 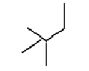 | 0.98 (s, 9H), 2.18 (s, 3H), 2.40 (s, 3H), 4.34 (br s, 2H), 7.20-7.37 (m, 5H), 8.34 (s, 1H), 10.31 (br s, 1H). |
| 11-018 | 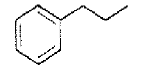 | 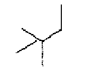 | 0.99 (s, 9H), 2.17 (s, 3H), 2.39 (s, 3H), 2.91 (t, *J* = 7.5 Hz, 2H), 3.63-3.70 (m, 2H), 7.16-7.31 (m, 5H), 8.30 (s, 1H), 10.01 (br s, 1H). |
| 11-019 | 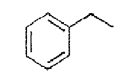 | 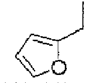 | 2.19 (s, 3H), 2.53 (s, 3H), 4.63 (d, *J* = 5.7 Hz, 2H), 5.34 (s, 2H), 6.33 (m, 2H), 7.21-7-37 (m, 6H), 8.38 (s, 1H), 10.18 (br s, 1H). |

**Table 143**

| | | | |
|---|---|---|---|
| 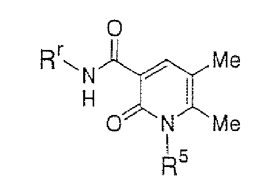 | | | |

| Compound No. | R^{r} | R⁵ | ¹H-NMR (CDCl₃) |
|---|---|---|---|
| 11-020 | 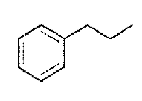 | 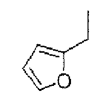 | 2.19 (s, 3H), 2.53 (s, 3H), 2.92 (t, *J* = 7.5 Hz, 2H), 3.62-3.69 (m, 2H), 5.34 (s, 2H), 6.35 (m, 2H), 7.17-7.32 (m, 5H), 7.35 (t, *J* = 1.5 Hz, 1H), 8.34 (s, 1H), 9.92 (br s, 1H). |
| 11-021 | 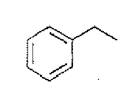 | 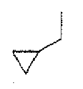 | 0.45-0.66 (m, 4H), 1.08-1.18 (m, 1H), 2.19 (s, 3H), 2.45 (s, 3H), 4.11 (d, *J* = 6.9 Hz, 2H), 4.64 (d, *J* = 5.7 Hz, 2H), 7.20-7.38 (m, 5H), 8.36 (s, 1H), 10.31 (br s, 1H). |
| 11-022 | 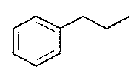 | 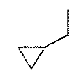 | 0.47-0.61 (m, 4H), 1.09-1.17 (m, 1H), 2.19 (s, 3H), 2.45 (s, 3H), 2.93 (t, *J* = 7.8 Hz, 2H), 3.63-6.70 (m, 2H), 4.12 (d, *J* = 6.9 Hz, 2H), 7.17-7.32 (m, 5H), 8.33 (s, 1H), 10.03 (br s, 1H). |
| 11-023 | H | nBu | 1.00 (t, *J* = 7.5 Hz, 3H), 1.40-1.53 (m, 2H), 1.63-1.73 (m, 2H), 2.18 (s, 3H), 2.42 (s, 3H), 4.13 (t, *J* = 8.1 Hz, 2H), 5.73 (br s, 1H), 8.31 (s, 1H), 9.62 (br s, 1H). |
| 11-024 | 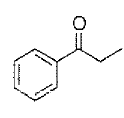 | 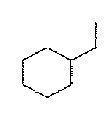 | 1.05-1.26 (m, 6H), 1.66-1.77 (m, 4H), 1.83-1.92 (m, 1H), 2.19 (s, 3H), 2.41 (s, 3H), 4.08 (br s, 2H), 4.97 (d, *J* = 4.5 Hz, 2H), 7.50 (t, *J* = 7.5 Hz, 2H), 7.61 (t, *J* = 7.5 Hz, 1H), 8.02-8.06 (m, 2H), 8.32 (s, 1H), 10.78 (br s, 1H). |
| 11-025 | 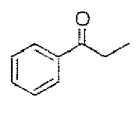 | Bn | 2.19 (s, 3H), 2.33 (s, 3H), 4.98 (d, *J* = 4.5 Hz, 2H), 5.52 (br s, 2H), 7.14 (d, *J* = 7.5 Hz, 2H), 7.29-7.36 (m, 3H), 7.50 (t, *J* = 7.5 Hz, 2H), 7.61 (t, *J* = 7.5 Hz, 1H), 8.03 (d, *J* = 7.5 Hz, 2H), 8.41 (s, 1H), 10.74 (br s, 1H). |

**Table 144**

| | | | |
|---|---|---|---|
| 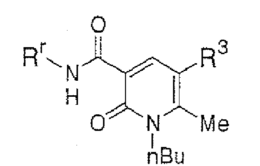 | | | |

| Compound No. | R^{r} | R³ | ¹H-NMR (CDCl₃) |
|---|---|---|---|
| 12-001 | 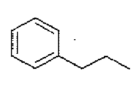 | 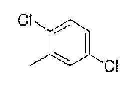 | 1.04 (t, *J* = 7.3 Hz, 3H), 1.42-1.54 (m, 2H), 1.67-1.78 (m, 2H), 2.28 (s, 3H), 2.94 (t, *J* = 7.3 Hz, 2H), 3.65-3.72 (m, 2H), 4.12-4.18 (m, 8H), 8.29 (s, 1H), 9.91 (t, *J* = 5.5 Hz, 1H). |
| 12-002 | 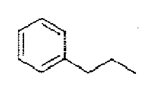 | 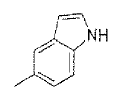 | 1.04 (t, *J* = 7.3 Hz, 3H), 1.43-1.55 (m, 2H), 1.70-1.80 (m, 2H), 2.44 (s, 3H), 2.97 (m, 2H), 3.67-3.74 (m, 2H), 4.18 (t, *J* = 7.9 Hz, 3H), 6.55 (m, 1H), 6.90-6.94 (m, 1H), 7.19-7.46 (m, 8H), 8.50 (s, 1H), 8.79 (brs, 1H), 10.14 (t, *J* = 5.8 Hz, 1H). |
| 12-003 | 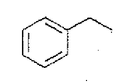 | Et | 0.98 (t, *J* = 7.5 Hz, 3H), 1.16 (t, *J =* 7.5 Hz, 3H), 1.38-1.51 (m, 2H), 1.60-1.72 (m, 2H), 2.43 (s, 3H), 2.53 (quint, *J* = 7.5 Hz, 2H), 4.09 (t, *J* = 7.8 Hz, 2H), 4.64 (d, *J* = 6.0 Hz, 2H), 7.20-7.38 (m, 5H), 8.38 (s, 1H), 10.30 (br s, 1H). |
| 12-004 | 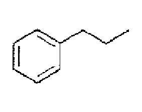 | Et | 1.00 (t, *J* = 7.5 Hz, 3H) , 1.16 (t, *J* = 7.5 Hz, 3H), 1.40-1.52 (m, 2H), 1.61-1.73 (m, 2H), 2.43 (s, 3H), 2.52 (quint, *J* = 7.5 Hz, 2H), 2.94 (t, *J* = 7.8 Hz, 2H), 3.63-3.70 (m, 2H), 4.11 (t, *J* = 7.8 Hz, 2H), 7.17-7.32 (m, 5H), 8.35 (s, 1H), 10.04 (br s, 1H). |

**Table 145**

| Compound No. | Structure | ¹H-NMR (CDCl₃) |
|---|---|---|
| 13-001 | 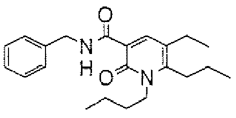 | 0.99 (t, *J* = 7.5 Hz, 3H), 1.08 (t, *J* = 7.5 Hz, 3H), 1.19 (t, *J* = 7.5 Hz, 3H), 1.38-1.50 (m, 2H), 1.53-1.72 (m, 4H), 2.50 (quint, *J* = 7.5 Hz, 2H), 2.62-2.68 (m, 2H), 4.06 (m, 2H), 4.64 (t, *J* = 6.0 Hz, 2H), 7.23-7.37 (m, 5H), 8.40 (s, 1H), 10.32 (br s, 1H). |
| 13-002 | 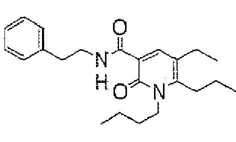 | 1.01 (t, *J* = 7.2 Hz, 3H), 1.09 (t, *J* = 7.5 Hz, 3H), 1.19 (t, *J* = 7.5 Hz, 3H), 1.40-1.52 (m, 2H), 1.54-1.73 (m, 4H), 2.50 (quint, *J* = 7.5 Hz, 2H), 2.62-2.68 (m, 2H), 2.93 (t, *J* = 7.8 Hz, 2H), 3.63-3.70 (m, 2H), 4.04-4.10 (m, 2H), 7.18-7.32 (m, 5H), 8.37 (s, 1H), 10.06 (br s, 1H). |
| 13-003 | 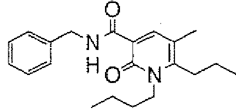 | 0.98 (t, *J* = 7.2 Hz, 3H), 1.08 (t, *J* = 7.2 Hz, 3H), 1.38-1.50 (m, 2H), 1.53-1.72 (m, 4H), 2.19 (s,3H), 2.62-2.68 (m, 2H), 4.04-4.10 (m, 2H), 4.64 (d, *J* = 5.7 Hz, 2H), 7.21-7.38 (m, 5H), 8.35 (s, 1H), 10.30 (br s, 1H). |
| 13-004 | 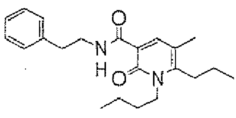 | 1.00 (t, *J* = 7.5 Hz, 3H), 1.08 (t, *J* = 7.5 Hz, 3H), 1.40-1.52 (m, 2H), 1.53-1.72 (m, 4H), 2.18 (s, 3H), 2.62-2.68 (m, 2H), 2.93 (t, *J* = 7.5 Hz, 2H), 3.63-3.70 (m, 2H), 4.04-4.10 (m, 2H), 7.18-7.32 (m, 5H), 8.31 (s, 1H), 10.03 (br s, 1H). |
| 13-005 | 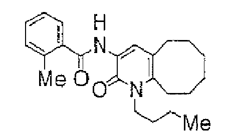 | 0.98 (t, *J* = 7.3 Hz, 3H), 1.38-1.53 (m, 6H), 1.62-1.72 (m, 6H), 2.54 (s, 3H), 2.62 (t, *J* = 6.1 Hz, 2H), 2.83 (t, *J* = 6.4 Hz, 2H), 4.10 (t, *J* = 7.9 Hz, 2H), 7.21-7.38 (m, 2H), 7.55 (d, *J* = 7.6 Hz, 1H), 8.38 (s, 1H), 8.79 (br s, 1H). |
| 13-006 | 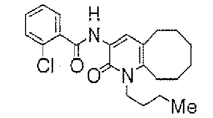 | 0.97 (t, *J* = 7.3 Hz, 3H), 1.38-1.53 (m, 6H), 1.62-1.75 (m, 6H), 2.62 (t, *J* = 6.1 Hz, 2H), 2.83 (t, *J* = 6.1 Hz, 2H), 4.10 (t, *J* = 7.9 Hz, 2H), 7.32-7.47 (m, 3H), 7.72-7.75 (m, 1H), 8.39 (s, 1H), 9.18 (br s, 1H). |
| 13-007 | 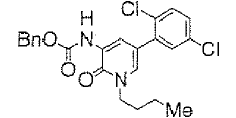 | 0.97 (t, *J* = 7.3 Hz, 3H), 1.34-1.46 (m, 2H), 1.72-1.82 (m, 2H), 4.03 (t, *J* = 7.3 Hz, 2H), 5.21 (s, 2H), 7.06 (d, *J* = 2.1 Hz, 1H), 7.23-7.40 (m, 8H), 7.95 (s, 1H), 8.15 (br s, 1H). |
| 13-008 | 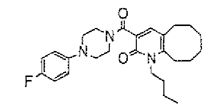 | 0.96 (d, *J* = 7.3 Hz, 3H), 1.36-1.56 (m, 6H), 1.58-1.71 (m, 4H), 1.71-1.81 (m, 2H), 2.57 (t, *J* = 6.0 Hz, 2H), 2.85 (t, *J* = 6.3 Hz, 2H), 3.08-3.20 (m, 2H), 3.18 (t, *J* = 5.0 Hz, 2H), 3.92 (t, *J* = 5.0 Hz, 2H), 4.06 (tlike, 2H), 6.87-7.00 (m, 5H). |
| 13-009 | 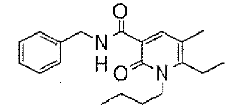 | 0.98 (t, *J* = 7.5 Hz, 3H), 1.22 (t, *J* = 7.5 Hz, 3H), 1.36-1.51 (m, 2H), 1.61-1.72 (m, 2H), 2.19 (s, 3H), 2.73 (quint, *J* = 7.5 Hz, 2H), 4.08 (t, *J* = 7.8 Hz, 2H), 4.64 (d, *J* = 6.0 Hz, 2H), 7.20-7.39 (m, 5H), 8.35 (s, 1H), 10.03 (br s, 1H). |

**Table 146**

| Compound No. | Structure | ¹H-NMR (CDCl₃) |
|---|---|---|
| 13-010 | 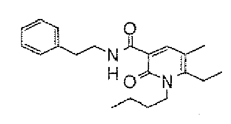 | 1.00 (t, *J* = 7.5 Hz, 3H), 1.23 (t, *J* = 7.5 Hz, 3H), 1.40-1.52 (m, 2H), 1.61.-1.73 (m, 2H), 2.19 (s, 3H), 2.73 (quint, *J* = 7.5 Hz, 2H), 2.93 (t, *J* = 7.5 Hz, 2H), 3.63-3.70 (m, 2H), 4.08 (t, *J* = 7.5 Hz, 2H), 7.17-7.32 (m, 5H), 8.31 (s, 1H), 10.03 (br s, 1H). |
| 13.011 | 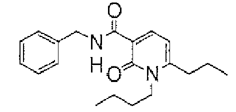 | 0.98 (t, *J* = 7.5 Hz, 3H), 1.06 (t, *J* = 7.5 Hz, 3H), 1.38-1.50 (m, 2H), 1.61-1.77 (m, 4H), 2.66 (t, *J* = 7.8 Hz, 2H), 4.05 (t, *J* = 7.8 Hz, 2H), 4.64 (d, *J* = 6.0 Hz, 2H), 6.28 (d, *J* = 7.8 Hz, 1H), 7.20-7.40 (m, 5H), 8.44 (d, *J* = 7.8 Hz, 1H), 10.21 (br s, 1H). |
| 13-012 | 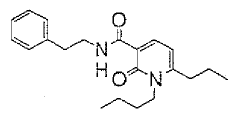 | 1.00 (t, *J* = 7.5 Hz, 3H), 1.06 (t, *J* = 7.5 Hz, 3H), 1.39-1.55 (m, 2H), 1.61-1.77 (m, 4H), 2.66 (t, *J* = 7.8 Hz, 2H), 2.93 (t, *J* = 7.8 Hz, 2H), 3.62-3.70 (m, 2H), 4.06 (t, *J* = 7.8 Hz, 2H), 6.27 (d, *J* = 7.5 Hz, 1H), 7.18-7.32 (m, 5H), 8.41 (d, *J* = 7.5 Hz, 1H), 9.95 (br s, 1H). |
| 13-013 | 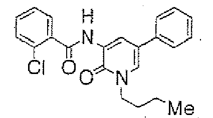 | 0.98 (t, *J* = 7.3 Hz, 3H), 1.37-1.49 (m, 2H), 1.76-1.86 (m, 2H), 4.08 (t, *J* = 7.3 Hz, 2H), 7.26 (d, *J* = 2.4 Hz, 1H), 7.32-7.54 (m, 8H), 7.76-7.79 (m, 1H), 8.92 (d, *J* = 2.4 Hz, 1H), 9.29 (s, 1H). |
| 13-014 | 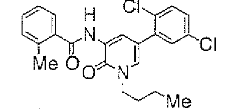 | 0.99 (t, *J* = 7.3 Hz, 3H), 1.38-1.50 (m, 2H), 1.60 (d, *J* = 7.0 Hz), 1.77-1.87 (m, 2H), 4.09 (dt, *J* = 7.1, 3.7 Hz, 2H), 5.32 (dt, *J* = 7.3, 7.0 Hz, 1H), 7.21-7.48 (m, 5H), 7.69 (d, *J* = 2.7 Hz, 1H), 8.83 (d, *J* = 2.7 Hz, 1H), 10.29 (d, *J* = 7.9 Hz). |
| 13-015 | 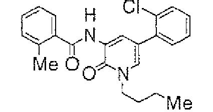 | 0.98 (t, *J* = 7.3 Hz, 3H), 1.37-1.49 (m, 2H), 1.76-1.86 (m, 2H), 2.54 (s, 3H), 4.06 (t, *J* = 7.3 Hz, 2H), 7.18-7.59 (m, 4H), 8.70 (d, *J* = 2.4 Hz, 1H), 8.04 (br s, 1H). |
| 13-016 | 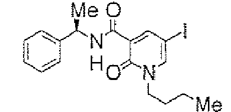 | 0.98 (t, *J* = 7.3 Hz, 3H), 1.38-1.46 (m, 2H), 1.57 (d, *J* = 7.0 Hz, 3H), 1.70-1.80 (m, 2H), 3.97 (dt, *J* = 4.3, 7.0 Hz, 2H), 5.29 (q, *J* = 7.3 Hz, 2H), 7.21-7.40 (m, 5H), 7.69 (d, *J* = 2.4 Hz, 1H), 8.62 (d, *J* = 2.4 Hz, 1H), 10.08 (d, *J* = 7.3 Hz, 1H). |
| 13-017 | 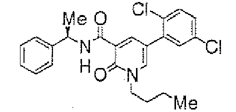 | 0.99 (t, *J* = 7.3 Hz, 3H), 1.37-1.49 (m, 2H), 1.59 (d, *J* = 7.0 Hz, 3H), 1.77-1.87 (m, 2H), 4.00-4.15 (m, 2H), 5.31 (dt, *J* = 7.6, 7.3 Hz, 1H), 7.21-7.43 (m, 3H), 7.65 (d, *J* = 2.7 Hz, 1H), 8.61 (d, *J* = 2.7 Hz, 1H), 10.19 (d, *J* = 7.6 Hz, 1H). |
| 13-018 | 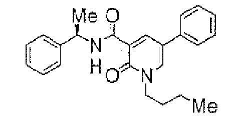 | 0.99 (t, *J* = 7.3 Hz, 3H), 1.38-1.50 (m, 2H), 1.60 (d, *J* = 7.0 Hz), 1.77-1.87 (m, 2H), 4.09 (dt, *J* = 7.1, 3.7 Hz, 2H), 5.32 (dt, *J* = 7.3, 7.0 Hz, 1H), 7.21-7.48 (m, 5H), 7.69 (d, *J* = 2.7 Hz, 1H), 8.83 (d, *J* = 2.7 Hz, 1H), 10.29 (d, *J* = 7.9 Hz). |

**Table 147**

| Compound No. | Structure | ¹H-NMR (CDCl₃) |
|---|---|---|
| 13-019 | 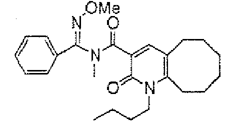 | 0.90 (t, *J* = 7.2 Hz, 3H), 1.23-1.71 (m, 12H), 2.41 (br t, *J* = 6.0 Hz, 2H), 2.68 (br t, *J* = 6.0 Hz, 2H), 3.27 (s, 3H), 3.70-4.00 (m, 2H), 4.01 (s, 3H), 7.11-7.61 (m, 6H). |
| 13-020 | 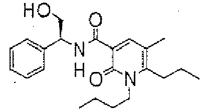 | 0.99 (t, *J* = 7.2 Hz, 3H), 1.08 (t, *J* = 7.5 Hz, 3H), 1.39-1.73 (m, 6H), 2.18 (s, 3H), 2.63-2.69 (m, 2H), 3.89-3.99 (m, 2H), 4.08 (s, 2H), 5.26-5.32 (m, 1H), 7.27-7.43 (m, 5H), 8.31 (s, 1H), 10.72 (d, *J* = 5.7 Hz, 1H). |
| 13-021 | 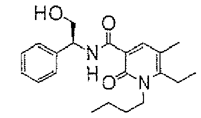 | 0.99 (t, *J* = 7.5 Hz, 3H), 1.23 (t, *J* = 7.5 Hz, 3H), 1.39-1.52 (m, 2H), 1.63-1.74 (m, 2H), 2.19 (s, 3H), 2.74 (q, *J* = 7.5 Hz, 2H), 3.89-4.00 (m, 2H), 4.09 (s, 2H), 5.26-5.32 (m, 1H), 7.26-7.43 (m, 5H), 8.32 (s, 1H), 10.72 (d, *J* = 7.2 Hz, 1H). |
| 13-022 | 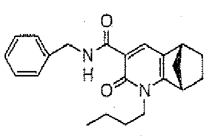 | 1.00 (t, *J* = 7.5 Hz, 3H), 1.19-1.26 (m, 2H), 1.45 (sextet, *J* = 7.5 Hz, 2H), 1.52-1.62 (m, 1H), 1.72 (quint, *J* = 7.5 Hz, 2H), 1.81-1.87 (m, 1H), 1.92-2.07 (m, 2H), 3.40 (br s,1H), 3.47 (br s, 1H), 3.89-3.99 (m, 1H), 4.17-4.26 (m, 1H), 4.57-4.71 (m, 2H), 7.20-7.38 (m, 5H), 8.44 (s, 1H), 10.30 (br s, 1H). |
| 13-023 | 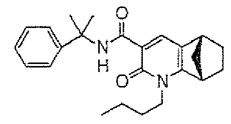 | 1.01 (t, *J* = 7.5 Hz, 3H), 1.18-1.30 (m, 4H), 1.43-1.60 (m, 2H), 1.64-1.81 (m, 2H), 1.78 (s, 6H), 1.89-2.05 (m, 2H), 3.33 (br s, 2H), 3.47 (br s, 1H), 3.92-4.01 (m, 1H), 4.21-4.31 (m, 1H), 7.24-7.32 (m, 3H), 7.43-7.46 (m, 2H), 8.33 (s, 1H), 10.42 (br s, 1H). |
| 13-024 | 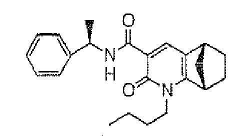 | 1.00 (t, *J* = 7.5 Hz, 3H), 1.17-1.26 (m, 2H), 1.39-1.60 (m, 4H), 1.55 (d, *J* = 3.0 Hz, 3H), 1.68-1.85 (m, 2H), 1.90-2.07 (m, 2H), 3.37 (br s, 1H), 3.47 (br s, 1H), 3.88-4.01 (m, 1H), 4.17-4.30 (m, 1H), 5.30 (quint, *J* = 7.5 Hz, 1H), 7.18-7.41 (m, 5H), 8.40 (s, 1H), 10.34 (d, *J* = 7.8 Hz, 1H). |
| 13-025 | 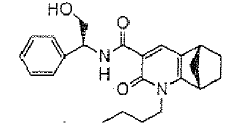 | 1.00 (t, *J* = 7.2 Hz, 3H), 1.19-1.26 (m, 1H), 1-41-2.10 (m, 9H), 3.39 (br s,1H), 3.49 (or s, 1H), 3.89-3.99 (m, 3H), 4.20-4.30 (m, 1H), 5.29 (q, *J* = 6.0 Hz, 1H), 7.26-7.43 (m, 5H), 8.40 (s, 1H), 10.71 (d, *J* = 7.2 Hz, 1H). |
| 13-026 | 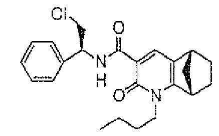 | 1.01 (t, *J* = 7.5 Hz, 3H), 1.20-1.31 (m, 2H), 1.41-1.55 (m, 2H), 1.70-1.88 (m, 4H), 1.90-2.08 (m, 2H), 3.38 (br s,1H), 3.48 (br s, 1H), 3.90 (d, *J* = 4.8 Hz, 2H), 3.95-4.02 (m, 1H), 4.20-4.31 (m, 1H), 5.50-5.58 (m, 1H), 7.26-7.44 (m, 5H), 8.39 (s, 1H), 10.74 (d, *J* = 7.8 Hz, 1H). |
| 13-027 | 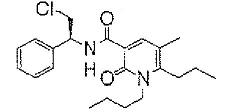 | 1.00 (t, *J* = 7.5 Hz, 3H), 1.08 (t, *J* = 7.5 Hz, 3H), 1.40-1.72 (m, 6H), 2.17 (s, 3H), 2.63-2.68 (m, 2H), 3.90 (d, *J* = 5.7 Hz, 2H), 4.11 (br s, 2H), 5.54 (s, 1H), 7.26-7.44 (m, 5H), 8.30 (s, 1H), 10.74 (br d, *J* = 7.8 Hz, 1H). |

**Table 148**

| Compound No. | Structure | ¹H-NMR (CDCl₃) |
|---|---|---|
| 13-028 | 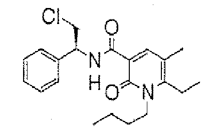 | 1.00 (t, *J* = 7.5 Hz, 3H), 1.23 (t, *J* = 7.5 Hz, 3H), 1.41-1.53 (m, 2H), 1.65-1.78 (m, 2H), 2.18 (s, 3H), 2.74 (quint, *J* = 7.8 Hz, 2H), 3.90 (d, *J* = 5.7 Hz, 2H), 4.11 (br s, 2H), 5.50-5.57 (m, 1H), 7.26-7.44 (m, 5H), 8.30 (s, 1H), 10.74 (br d, *J* = 7.5 Hz, 1H). |
| 13-029 | 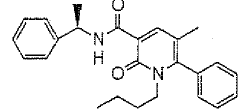 | 0.70 (t, *J* = 7.2 Hz, 3H), 1.11 (sextet, *J* = 7.2 Hz, 2H), 1.54 (quint, *J* = 7.2 Hz, 2H), 1.61 (s, 3H), 1.81 (s, 3H), 3.70-3.86 (m, 2H), 5.34 (quint, *J* = 7.2 Hz, 1H), 7.17-7.58 (m, 10H), 8.43 (s, 1H), 10.41 (d, *J* = 7.8 Hz, 1H). |
| 13-030 | 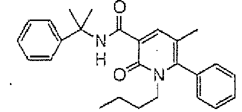 | 0.72 (t, *J* = 7.5 Hz, 3H), 1.12 (sextet, *J* = 7.5 Hz, 2H), 1.57 (quint, *J* = 7.5 Hz, 2H), 1.78 (s, 3H), 1.82 (s, 6H), 3.81 (t, *J* = 8.4 Hz, 2H), 7.16-7.57 (m, 10H), 8.37 (s, 1H), 10.49 (br s, 1H). |
| 13-031 | 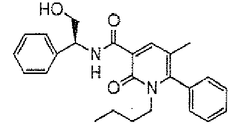 | 0.70 (t, *J* = 7.2 Hz, 3H), 1.10 (sextet, *J* = 7.2 Hz, 2H), 1.54 (quint, *J* = 7.2 Hz, 2H), 1.83 (s, 3H), 3.76-3.86 (m, 2H), 3.91-4.03 (m, 2H), 5.29-5.36 (m, 1H), 7.16-7.57 (m, 10H), 8.48 (s, 1H), 10.82 (d, *J* = 6.6 Hz, 1H). |
| 13-032 | 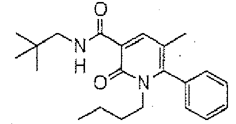 | 0.70 (t, *J* = 7.2 Hz, 3H), 1.01 (s, 9H), 1.11 (sextet, *J* = 7.2 Hz, 2H), 1.54 (quint, *J* = 7.2 Hz, 2H), 1.83 (s, 3H), 3.29 (t, *J* = 4.8 Hz, 2H), 3.80 (t, *J* = 7.2 Hz, 2H), 7.20-7.67 (m, 2H), 7.50-7.60 (m, 3H), 8.46 (s, 1H), 10.15 (br s, 1H). |
| 13-033 | 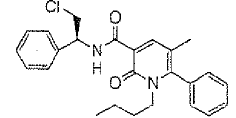 | 0.71 (t, *J* = 7.5 Hz, 3H), 1.11 (sextet, *J* = 7.5 Hz, 2H), 1.56 (quint, *J* = 7.5 Hz, 2H), 1.82 (s, 3H), 3.81 (dd, *J* = 6.0 Hz, 3.6 Hz, 2H), 3.93 (d, *J* = 6.0 Hz, 2H), 5.53-5.61 (m, 1H), 7.19-7.57 (m, 10H), 8.44 (s, 1H), 10.83 (d, *J* = 8.4 Hz, 1H). |
| 13-034 | 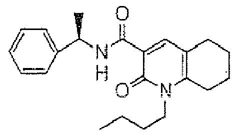 | 0.99 (t, *J* = 7.2 Hz, 3H), 1.45 (sextet, *J* = 7.2 Hz, 2H), 1.58 (d, *J* = 7.2 Hz, 3H), 1.64-1.77 (m, 4H), 1.83-1.92 (m, 2H), 2.60 (t, *J* = 6.0 Hz, 2H), 2.74 (t, *J* = 6.0 Hz, 2H), 4.00-4.10 (m, 2H), 5.30 (quint, *J* = 7.2 Hz, 1H), 7.19-7.42 (m, 5H), 8.23 (s, 1H), 10.34 (d, *J* = 7.5 Hz, 2H). |
| 13-035 | 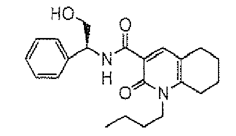 | 0.98 (t, *J* = 7.5 Hz, 3H), 1.44 (sextet, *J* = 7.5 Hz, 2H), 1.60-1.70 (m, 2H), 1.63-1.80 (m, 2H), 1.83. 1.93 (m, 2H), 2.62 (t, *J* = 6.0 Hz, 2H), 2.75 (t, *J* = 6.0 Hz, 2H), 3.89-3.98 (m, 2H), 4.00-4.08 (m, 2H), 5.25-5.32 (m, 1H), 7.27.7.43 (m, 5H), 8.27 (s, 1H), 10.75 (d, *J* = 5.4 Hz, 1H). |
| 13-036 | 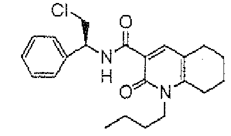 | 0.99 (t, *J* = 7.2 Hz, 3H), 1.45 (sextet, *J* = 7.2 Hz, 2H), 1.62-1.78 (m, 4H), 1.83-1.93 (m, 2H), 2.61 (t, *J* = 6.0 Hz, 2H), 2.75 (t, *J* = 6.0 Hz, 2H), 3.91 (d, *J* = 6.0 Hz, 2H), 4.06 (t, *J* = 7.2 Hz, 2H), 5.50-5.58 (m, 1H), 7.27-7.45 (m, 5H), 8.23 (s, 1H), 10.75 (t, *J* = 7.5 Hz, 1H). |

**Table 149**

| Compound No. | Structure | ¹H-NMR (CDCl₃) |
|---|---|---|
| 13-037 | 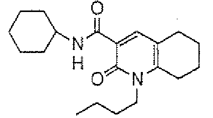 | 0.99 (t, *J* = 7.2 Hz, 3H), 1.18-1.51 (m, 8H), 1.61-1.77 (m, 6H), 1.83-1.92 (m, 2H), 1.96-2.02 (m, 2H), 2.61 (t, *J* = 6.0 Hz, 2H), 2.73 (t, *J* = 6.0 Hz, 2H), 3.90-4.01 (m, 1H), 4.03 (t, *J* = 7.2 Hz, 2H), 8.24 (s, 1H), 9.86 (d, *J* = 7.5 Hz, 1H). |
| 13-038 | 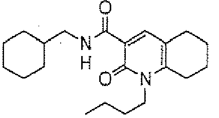 | 0.99 (t, *J* = 7.5 Hz, 3H), 1.13-1.30 (m, 2H), 1.45 (sextet, *J* = 7.5 Hz, 2H), 1.59-1.92 (m, 15H), 2.61 (t, *J* = 6.0 Hz, 2H), 2.74 (t, *J* = 6.0 Hz, 2H), 3.28 (t, *J* = 6.0 Hz, 2H), 4.04 (t, *J* = 7.5 Hz, 2H), 8.25 (s, 1H), 9.96 (br s, 1H). |
| 13-039 | 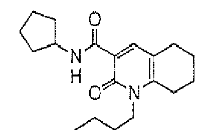 | 0.98 (t, J= 7.5 Hz, 3H), 1.44 (sextet, J= 7.5 Hz, 2H), 1.52-1.78 (m, 10H), 1.82-1.91 (m, 2H), 1.99-2.11 (m, 2H), 2.61 (t, *J* = 6.0 Hz, 2H), 2.73 (t, J= 6.0 Hz, 2H), 4.02 (t, *J* = 7.5 Hz, 2H), 4.36 (sextet, J= 6.6 Hz, 1H), 8.24 (s, 1H), 9.91 (d, J= 6.9 Hz, 1H). |
| 13-040 | 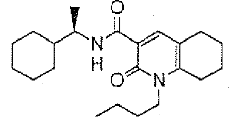 | 0.99 (t, *J* = 7.2 Hz, 3H), 1.05-1.29 (m, 6H), 1.18 (d, *J* = 6.6 Hz, 3H), 1.45 (sextet, *J* = 7.2 Hz, 2H), 1.59-1.92 (m, 11H), 2.61 (t, *J* = 6.0 Hz, 2H), 2.73 (t, *J* = 6.0 Hz, 2H), 3.93-4.13 (m, 1H+2H), 8.24 (s, 1H), 9.85 (d, *J* = 8.7 Hz, 1H). |
| 13-041 | 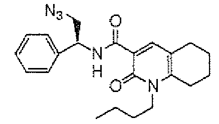 | 0.99 (t, *J* = 7.2 Hz, 3H), 1.45 (sextet, *J* = 7.2 Hz, 2H), 1.60-1.78 (m, 4H), 1.83-1.91 (m, 2H), 2.61 (t, *J* = 6.0 Hz, 2H), 2.75 (t, *J* = 6.0 Hz, 2H), 3.70 (d, *J* = 6.0 Hz, 2H), 4.06 (t, *J* = 7.2 Hz, 2H), 5.38-5.46 (m, 1H), 7.26-7.45 (m, 5H), 8.23 (s, 1H), 10.73 (t, *J* = 8.7 Hz, 1H). |
| 13-042 | 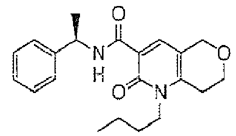 | 0.99 (t, *J* = 7.5 Hz, 3H), 1.45 (sextet, J= 7.5 Hz, 2H), 1.58 (d, *J* = 7.2 Hz, 3H), 1.68 (quint, *J* = 7.5 Hz, 2H), 2.82 (t, *J* = 6.0 Hz, 2H), 4.00 (t, *J* = 6.0 Hz, 2H), 4.05 (t, *J* = 7.5 Hz, 2H), 4.58 (s, 2H), 5.29 (quint, *J* = 7.2 Hz, 1H), 7.23-7.42 (m, 5H), 8.17 (s, 1H), 10.25 (d, *J* = 7.5 Hz, 1H). |
| 13-043 | 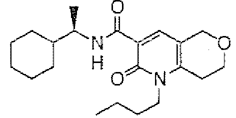 | 0.99 (t, *J* = 7.2 Hz, 3H), 1.03-1.30 (m, 4H), 1.18 (d, *J* = 6.6 Hz, 3H), 1.45 (sextet, *J* = 7.2 Hz, 2H), 1.60-1.84 (m, 9H), 2.82 (t, *J* = 6.0 Hz, 2H), 3.92-4.13 (m, 5H), 4.59 (s, 2H), 8.18 (s, 1H), 9.77 (d, *J* = 8.1 Hz, 1H). |
| 13-044 | 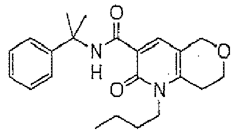 | 1.01 (t, *J* = 7.5 Hz, 3H), 1.47 (sextet, *J* = 7.5 Hz, 2H), 1.71 (quint, *J* = 7.5 Hz, 2H), 1.79 (s, 6H), 2.82 (t, *J* = 6.0 Hz, 2H), 4.00 (t, *J* = 6.0 Hz, 2H), 4.05 (t, *J* = 7.5 Hz, 2H), 4.54 (s, 2H), 7.26-7.34 (m, 3H), 7.42-7.46 (m, 2H), 8.11 (s, 1H), 10.34 (br s, 1H). |
| 13-045 | 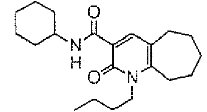 | 0.99 (t, *J* = 7.5 Hz, 3H), 1.19-1.52 (m, 8H), 1.62-1.79 (m, 8H), 1.80-1.88 (m, 2H), 1.91-2.02 (m, 2H), 2.71 (t, *J* = 6.0 Hz, 2H), 2.93 (t, *J* = 6.0 Hz, 2H), 3.93-4.02 (m, 1H), 4.16 (br t, *J* = 7.5 Hz, 2H), 8.29 (s, 1H), 9.86 (d, *J* = 6.9 Hz, 1H). |

**Table 150**

| Compound No. | Structure | ¹H-NMR (CDCl₃) |
|---|---|---|
| 13-046 | 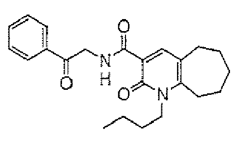 | 1.00 (t, *J* = 7.2 Hz, 3H), 1.47 (sextet, *J* = 7.2 Hz, 2H), 1.63-1.74 (m, 6H), 1.83-1.90 (m, 2H), 2.72 (t, *J* = 6.0 Hz, 2H), 2.96 (t, *J* = 6.0 Hz, 2H), 4.23 (br t, *J* = 7.2 Hz, 2H),4.96 (d, *J* = 4.5 Hz, 2H), 7.50 (t, *J* = 7.5 Hz, 2H), 7.61 (t, *J* = 7.5 Hz, 1H), 8.04 (d, *J* = 7.5 Hz, 2H), 8.29 (s,1H), 10.79 (br s, 1H). |
| 13-047 | 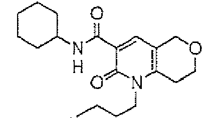 | 0.99 (t, *J* = 7.2 Hz, 3H), 1.22-1.49 (m, 6H), 1.60-1.78 (m, 6H), 1.94-2.01 (m, 2H), 2.82 (t, *J* = 6.0 Hz, 2H), 3.91-4.05 (m, 5H), 4.60 (s, 2H), 8.18 (s, 1H), 9.79 (d, *J* = 6.3 Hz, 1H). |
| 13-048 | 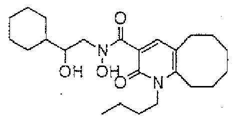 | 0.98 (t, *J* = 7.2 Hz, 3H), 1.10-1.29 (m, 6H), 1.40-1.56 (m, 8H), 1.65-1.82 (m, 8H), 1.93 (br t, *J* = 12.0 Hz, 1H), 2.66 (t, *J* = 6.0 Hz, 2H), 2.93 (t, *J* = 6.0 Hz, 2H), 3.78-3.87 (m, 2H), 4.00-4,12 (m, 1H), 4.16 (br t, *J* = 7.2 Hz, 2H), 7.29 (br s, 1H), 8.00 (s, 1H). |
| 13-049 | 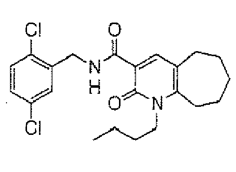 | 0.99 (t, *J* = 7.2 Hz, 3H), 1.46 (sextet, *J* = 7.2 Hz, 2H), 1.61-1.73 (m, 6H), 1.87 (sextet, *J* = 6.0 Hz, 2H), 2.74 (t, *J* = 6.0 Hz, 2H), 2.96 (t, *J* = 6.0 Hz, 2H), 4.19 (br t, *J* = 7.2 Hz, 2H), 4.68 (d, *J* = 6.0 Hz, 2H), 7.15 (dd, *J* = 8.4 Hz, 2.4 Hz, 1H), 7.23-7.29 (m, 1H), 7.39 (d, *J* = 2.4 Hz, 1H), 8.3 (s,1H), 10.43 (br s, 1H). |
| 13-050 | 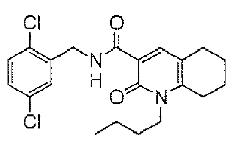 | 0.99 (t, *J* = 7.2 Hz, 3H), 1.45 (sextet, *J* = 7.2 Hz, 2H), 1.63-1.79 (m, 4H), 1.89 (quint, *J* = 6.0 Hz, 2H), 2.63 (t, *J* = 6.0 Hz, 2H), 2.76 (t, *J* = 6.0 Hz, 2H), 4.05 (t, *J* = 8.1 Hz, 2H), 4.68 (d, *J* = 6.0 Hz, 2H), 7.15 (dd, *J* = 8.7 Hz, 2.4 Hz, 1H), 7.25-7.29 (m, 1H), 7.38 (d, *J* = 2.4 Hz, 1H), 8.27 (s, 1H), 10.44 (br s, 1H). |

### Experimental Example

The above compounds of the present invention were examined as shown below. Experimental Example 1: Experiments for Human cannabinoid receptor binding inhibition

The crude membrane fractions as Human cannabinoid receptor were then prepared from the CB1R or CB2R-expressing CHO cells. Receptor binding assay was performed by incubating the membranes with each test compound and 38,000 dpm [³H]CP55940 (at a final concentration of 0.5 nM: NEN Life Science Products) in the assay buffer (50 mM Tris-HCl, 1 mM EDTA, 3 mM MgCl₂, pH 7.4) containing 0.5% bovine serum albumin (BSA) for 2 hr at 25 °C. The incubation mixture was filtered through 1% polyethylenimine (PEI)-treated GF/C glass filter and washed with 50 mM Tris-HCl (pH 7.4) containing 0.1% BSA. The radioactivity was then counted with a liquid scintillation counter. Nonspecific binding was determined in the presence of 10 µM WIN55212-2 (a CB agonist described in the patent US508122, Sigma), and the specific binding was calculated by subtracting the nonspecific binding from the total binding. The IC₅₀ value for each test compound was determined as the concentration at which 50 % of the specific binding was inhibited. Ki value of test compound is calculated by obtained IC₅₀ value and Kd value of [³H]CP55940.

**Table 151**

| Compound | Ki (nM) | |
|---|---|---|
| | CB1 receptor | CB2 receptor |
| 1-5 | >5000 | 61 |
| I-23 | >5000 | 29 |
| I-50 | >5000 | 39 |
| I-51 | n.t. | 23 |
| I-52 | n.t. | 35 |
| I-56 | n.t. | 54 |
| 1-6 | >5000 | 9 |
| 1-57 | 4134 | 6 |
| I-69 | n.t. | 33 |
| I-60 | 2097 | 18 |
| 1-62 | n.t. | 44 |
| I-63 | n.t. | 43 |
| I-74 | n.t. | 48 |
| I-77 | n.t. | 53 |
| I-84 | >5000 | 35 |
| 1-85 | n.t. | 25 |

| | | |
|---|---|---|
| n.t.: not tested | | |

**Table 152**

| Compound | Ki (nM) | |
|---|---|---|
| | CB1 receptor | CB2 receptor |
| II-13 | n.t. | 6 |
| II-14 | >5000 | 2 |
| II-17 | n.t. | 8 |
| II-39 | 906 | 2 |
| II-40 | n.t. | 0.5 |
| II-41 | n.t. | 1 |
| II-42 | >5000 | 0.3 |
| II-44 | 321 | 1.1 |
| II-45 | 386 | 1.2 |
| II-46 | 3226 | 2 |
| II-49 | 1116 | 2.9 |
| II-74 | 704 | 1.2 |
| II-78 | 1015 | 8 |
| II-80 | >5000 | 2.2 |
| II-88 | n.t. | 8 |
| II-89 | n.t. | 8 |
| II-92 | 1312 | 6 |
| II-93 | 1537 | 3 |

| | | |
|---|---|---|
| n.t.: not tested | | |

**Table 153**

| Compound | Ki (nM) | |
|---|---|---|
| | CB1 receptor | CB2 receptor |
| 2-004 | nt | 101 |
| 3-010 | nt | 57 |
| 3-038 | 1252 | 12 |
| 4-001 | 2851 | 28 |
| 4-002 | 746 | 17 |
| 4-003 | 680 | 44 |
| 4-052 | 1497 | 24 |
| 4-053 | 254 | 6 |
| 4-054 | 482 | 6 |
| 4-056 | 551 | 8 |
| 4-061 | 124 | 2.5 |
| 4-062 | >5000 | 4 |
| 4-101 | 890 | 1.5 |
| 4-102 | 908 | 1.6 |
| 4-104 | 54 | 6 |
| 4-105 | 91 | 2.1 |
| 4-301 | 1769 | 8 |
| 4-302 | >5000 | 10 |
| 4-310 | 512 | 9 |
| 5-005 | 391 | 16 |
| 5-006 | 390 | 14 |

### Experimental Example 2: Inhibition experiments for cannabinoid receptor-mediated suppression of cAMP synthesis

The CHO cells expressing human CB1R or CB2R were incubated with test compounds for 15 min. After the incubation, 4 µM forskolin (Sigma) was added and the cells were incubated for 20 min. The reaction was stopped by the addition of 1N HCl and the amount of cAMP in the cell supernatant was measured using an Cyclic AMP kit (CIS Diagnostic). The cAMP amount increased by forskolin compared to that in the absence of forskolin was defined as 100%, and the IC₅₀ value of each test compound was determined as the concentration at which 50 % of the forskolin-stimulated cAMP synthesis was inhibited. As shown in Table, the test compounds were found to possess agonistic activity toward CB1R or CB2R.

**Table 154**

| Compound | CB2 receptor IC₅₀ |
|---|---|
| | (nM) |
| I-5 | 6.5 |
| I-23 | 2.6 |
| I-51 | 2.8 |
| I-6 | 2.7 |
| I-57 | 5.5 |

**Table 155**

| Compound | CB2 receptor IC₅₀ |
|---|---|
| | (nM) |
| I-46 | 5.4 |
| I-33 | 13.7 |
| I-49 | 2.2 |
| I-74 | 1.6 |
| I-92 | <0.2 |
| I-93 | 0.6 |

**Table 156**

| Compound | CB2 receptor IC₅₀ |
|---|---|
| | (nM) |
| 3-038 | 28.6- |
| 4-001 | 64.2 |
| 4-053 | 7.9 |
| 4-054 | 4.2 |
| 4-056 | 4.3 |
| 4-061 | 2.3 |
| 4-062 | 1.3 |
| 4-101 | 1.4 |
| 4-102 | 1.7 |
| 4-104 | 9.8 |

**Table 157**

| Compound | CB1 receptor IC₅₀ (nM) |
|---|---|
| 3-109 | 2.7 |
| 11-001 | 1.2 |
| 11-002 | 0.4 |
| 13-006 | 1.6 |
| 11-011 | 0.6 |
| 11-012 | 7.8 |
| 11-013 | 7.5 |
| 13-023 | 0.3 |
| 10-041 | 3.5 |
| 10-045 | 1.2 |
| 13-030 | 0.7 |
| 10-050 | 2.0 |
| 10-058 | 2.4 |
| 10-059 | 6.1 |
| 13-044 | 1.8 |
| 10-089 | 9.4 |
| 10-103 | 6.3 |

The compound of the present invention was examined by the following Experimental Examples 3, 4, and 5.

### Experimental Example 3: Experiment on the binding inhibitory activity to mouse cannabinoid receptors

The membrane fractions of mouse brain and spleen that abundantly express CB1 and CB2 receptor, respectively, were used as mouse cannabinoid receptor preparations (C57BL/6J mice). Experiment on the binding inhibitory activity was performed according to the method shown in Experimental Example 1.

### Experimental Example 4: Inhibitory effect on compound 48/80-induced pruritus in ICR strain mice

The experiment was carried out by the method of Inagaki et al. (Eur J Pharmacol 1999; 367: 361-371) with some modifications. Briefly, the back of female ICR strain mice was clipped, and compound 48/80 (3 µg/50 µL/site) was injected intradermally to elicit the response. The number of scratching behavior to the injection site by hind paws, which was observed immediately after the injection, was counted for 30 min. Because mice generally showed several scratches in one bout, a series of these behaviors was counted as one incidence of scratching. Test compounds, dissolved in sesame oil or suspended in 0.5% methylcellulose, were orally administered once, and then pruritus was elicited by the injection of compound 48/80 at predetermine time when the maximal plasma concentration of the compound was obtained. The evaluation of inhibitory effect against pruritus was performed by comparing the number of scratching in the compound administered group with that in the vehicle administered group. The statistical analysis was curried out by Dunnett's test, and P value less than 0.05 obtained was considered to be statistically significant. As shown in Table, Δ⁹-tetrahydrocannabinol, an active substance of marijuana, and test compounds acting as agonists to cannabinoid receptors that have three different fundamental skeletons were inhibited compound 48/80-induced pruritus.

**Table 158**

| Compound | Binding Assay: Ki (nM) | | cAMP Inhibition: IC50 (nM) | | Antipruritic Effect(%) |
|---|---|---|---|---|---|
| | Brain (CB1) | Spleen (CB2) | CB1 | CB2 | 100 mg/kg(p.o.) |
| I-270 | 80 | 1.2 | 68.7 | 7.9 | 98** |
| II-86 | 12 | 1.1 | 16.8 | 1 | 98** |
| 4-101 | 35 | 0.6 | 60.3 | 1.4 | 81** |
| 10-035 | 12 | 0.1 | 29.0 | 0.2 | 93** |
| WIM55212-2 | 7 | 6 | 12.2 | 1.1 | 70* |
| THC | 40 | 9.2 | 11.4 | 6.6 | 98**# |

| | | | | | |
|---|---|---|---|---|---|
| *p<0.05, **p<0.01, # 10mg/kg(i.p.) | | | | | |

### Experimental Example 5: Effect of cannabinoid receptor antagonists on antipruritic effect of cannabinoid receptor agonist

Concerning the inhibitory effect of test compounds against compound 48/80-induced pruritus that was observed in above Experimental Example, the receptor specificity of that effect was evaluated using well-known cannabinoid type 1 receptor antagonist, SR141716A and cannabinoid type 2 receptor antagonist, SR144528. The effect of test compound on compound 48/80-induced pruritus was evaluated by the method as described in Experimental Example 1 following the oral administration of SR141716A or SR144528 at 1 h prior to the administration of test compound. The statistical analysis was curried out by Welch's t-test, and P value less than 0.05 obtained was considered to be statistically significant. As shown in Fig. 1, the inhibitory effect of I-270 on compound 48/80-induced pruritus was antagonized with a cannabinoid type 1 receptor antagonist, SR141716A. From these results, it is confirmed that test compounds exhibit their antipruritic effect via cannabinoid type 1 receptor.

### Formulation Example

It is to be noted that the following Formulation Examples 1 to 8 are mere illustration, but not intended to limit the scope of the invention. The term "active ingredient" means the compounds of the present invention, a tautomer, a prodrug, a pharmaceutically acceptable salt or a solvate thereof.

### Formulation Example 1

Hard gelatin capsules are prepared using of the following ingredients:

| | Dose (mg/capsule) |
|---|---|
| Active ingredient | 250 |
| Starch, dried | 200 |
| Magnesium stearate | 10 |
| Total | 460 mg |

### Formulation Example 2

A tablet is prepared using of the following ingredients:

| | Dose (mg/tablet) |
|---|---|
| Active ingredient | 250 |
| Cellulose, microcrystals | 400 |
| Silicon dioxide, fumed | 10 |
| Stearic acid | 5 |
| Total | 665 mg |

The components are blended and compressed to form tablets each weighing 665 mg.

### Formulation Example 3

An aerosol solution is prepared containing the following components:

| | Weight |
|---|---|
| Active ingredient | 0.25 |
| Ethanol | 25.75 |
| Propellant 22 (chlorodifluoromethane) | 74.00 |
| Total | 100.00 |

The active compound is mixed with ethanol and the admixture added to a portion of the propellant 22, cooled to -30 °C and transferred to filling device. The required amount is then fed to stainless steel container and diluted with the reminder of the propellant. The valve units are then fitted to the container.

### Formulation Example 4

Tablets, each containing 60 mg of active ingredient, are made as follows.

| | |
|---|---|
| Active ingredient | 60 mg |
| Starch | 45 mg |
| Microcrystals cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |
| Total | 150 mg |

The active ingredient, starch, and cellulose are passed through a No. 45 mesh U.S. sieve, and the mixed thoroughly. The aqueous solution containing polyvinylpyrrolidone is mixed with the resultant powder, and the admixture then is passed through a No. 14 mesh U.S. sieve. The granules so produced are dried at 50 °C and passed through a No. 18 mesh U.S. sieve. The sodium carboxymethyl starch, magnesium stearate, and talc, previously passed through No. 60 mesh U.S. sieve, are then added to the granules which, after mixing, are compressed on a tablet machine to yield tablets each weighing 150 mg.

### Formulation Example 5

Capsules, each containing 80 mg of active ingredient, are made as follows:

| | |
|---|---|
| Active ingredient | 80 mg |
| Starch | 59 mg |
| Microcrystals cellulose | 59 mg |
| Magnesium stearate | 2 mg |
| Total | 200 mg |

The active ingredient, cellulose, starch, and magnesium stearate are blended, passed through a No. 45 mesh U.S. sieve, and filled into hard gelatin capsules in 200 mg quantities.

### Formulation Example 6

Suppository, each containing 225 mg of active ingredient, are made as follows:

| | |
|---|---|
| Active ingredient | 225 mg |
| Saturated fatty acid glycerides | 2000 mg |
| Total | 2225 mg |

The active ingredient is passed through a No. 60 mesh U.S. sieve and suspended in the saturated fatty acid glycerides previously melted using the minimum heat necessary. The mixture is then poured into a suppository mold of nominal 2g capacity and allowed to cool.

### Formulation Example 7

Suspensions, each containing 50 mg of active ingredient per 5 ml dose, are made as follows:

| | |
|---|---|
| Active ingredient | 50 mg |
| Sodium carboxymethyl cellulose | 50 mg |
| Syrup | 1.25 mL |
| Benzoic acid solution | 0.10 mL |
| Flavor | q.v. |
| Color | q.v. |
| Purified water to total | 5 mL |

The active ingredient is passed through a No. 45 U.S. sieve, and mixed with the sodium carboxymethyl cellulose and syrup to form a smooth paste. The benzoic acid solution, flavor and color are diluted with a portion of the water and added, with stirring. Sufficient water is then added to produce the required volume.

### Formulation Example 8

An intravenous formulation may be prepared as follows:

| | |
|---|---|
| Active ingredient | 100 mg |
| Saturated fatty acid glycerides | 1000 mL |

The solution of the above ingredients generally is administered intravenously to a subject at a rate of 1 mL per minute.

The present invention is summarized by the following items:
1. An antipruritics which contains as an active ingredient a compound having an agonistic activity to the cannabinoid receptor, a prodrug, a pharmaceutically acceptable salt or solvate thereof.
2. An antipruritics according to item 1 wherein a compound having an agonistic activity to the cannabinoid receptor is a compound having an agonistic activity to the cannabinoid type 1 receptor.
3. An antipruritics according to item 1 wherein a compound having an agonistic activity to the cannabinoid receptor is a compound having an agonistic activity to the cannabinoid type 2 receptor.
4. An antipruritics according to item 1 wherein a compound having an agonistic activity to the cannabinoid receptor is a compound having an agonistic activity to the cannabinoid type 1 receptor and the cannabinoid type 2 receptor.
5. An antipruritics according to item 1 wherein a compound having an agonistic activity to the cannabinoid receptor is a compound selected from compounds represented by the formula (I): 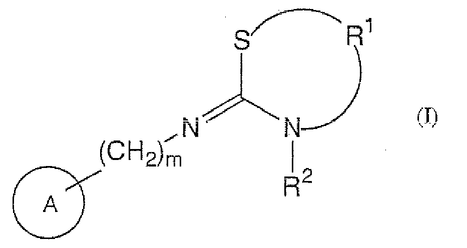 wherein R¹ is optionally substituted alkylene;
   R² is alkyl; a group of the formula: -C(=R³)-R⁴ wherein R³ is O or S, R⁴ is alkyl, alkoxy, alkylthio, alkenylthio, optionally substituted amino, optionally substituted aralkyloxy, optionally substituted aralkylthio, optionally substituted aralkylamino, alkoxyalkyl, alkylthioalkyl or optionally substituted aminoalkyl; or a group of the formula: -SO₂R⁵ wherein R⁵ is alkyl, optionally substituted amino, optionally substituted aryl or optionally substituted heteroaryl;
   m is an integer of 0 to 2;
   A is optionally substituted aryl or optionally substituted heteroaryl.
6. An antipruritics according to item 5 wherein R¹ is C2-C9 straight or branched alkylene optionally substituted with alkylene; R² is a group of the formula: -C(=R³)-R⁴ wherein R³ is O or S, R⁴ is alkoxy, alkylthio, or alkenylthio; m is 0; A is aryl optionally substituted with 1 or 2 substitutent(s) selected from the group consisting of alkyl, alkoxy, haloalkoxy and alkylthio.
7. An antipruritics according to item 1 wherein a compound having an agonistic activity to the cannabinoid receptor is a compound selected from compounds represented by the formula (II): 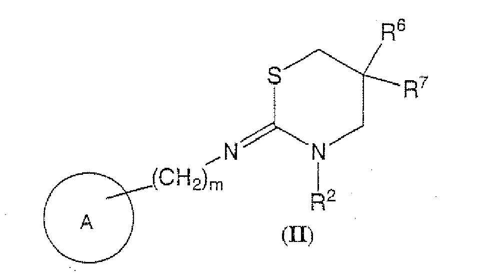 wherein R² is optionally substituted heterocyclic group or a group of the formula: - C(=Z)W-R⁸ wherein Z is O or S; W is O or S; R⁸ is optionally substituted alkyl, optionally substituted alkenyl or optionally substituted alkynyl;
   R⁶ and R⁷ each is independently hydrogen, optionally substituted alkyl, optionally substituted alkoxyalkyl, optionally substituted aminoalkyl, or optionally substituted cycloalkyl; or
   R⁶ and R⁷ taken together form optionally substituted alkylene which may contain a heteroatom(s);
   m is an integer of 0 to 2;
   A is optionally substituted aryl or optionally substituted heteroaryl.
8. An antipruritics according to item 7 wherein m is 0; A is aryl optionally substituted with 1 or 2 substitutent(s) selected from group consisting of alkyl, alkoxy, haloalkoxy, or alkylthio.
9. An antipruritics according to item 7 or 8 wherein R² is a group of the formula: - C(=Z)W-R⁸ wherein Z is O or S; W is O or S, R⁸ is optionally substituted alkyl or optionally substituted alkenyl; R⁶ and R⁷ each is independently optionally substituted alkyl; or R⁶ and R⁷ taken together form optionally substituted alkylene which may contain a heteroatom(s).
10. A antipruritics according to item 1 wherein a compound having an agonistic activity to the cannabinoid receptor is a compound selected from compounds represented by the formula (III): 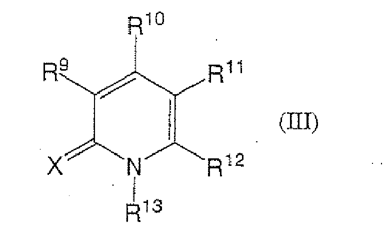 wherein R⁹ is hydrogen, halogen, cyano, formyl, acyl, carboxy, alkoxycarbonyl, optionally substituted carbamoyl, isothiocyanato, optionally substituted amino, hydroxy, alkoxy, alkylthio, alkenyloxy, alkynyloxy, alkylsulfinyl, alkylsulfonyl, nitro, or a group of the formula: -Y¹-Y²-Y³-R^{a} wherein Y¹ and Y³ each is independently single bond or optionally substituted alkylene; Y² is single bond, -O-, -O-C(=O)-, -O-C(=O)-O-, -O-C(=O)-NR^{b}-, -O-SO₂-, -NR^{b}-, -NR^{b}-C(=O)-, -NR^{b}-SO₂-, -NR^{b}-C(=NH)-, -NR^{b}-C(=O)-O-, -NR^{b}-C(=O)-NR^{b}-, -NR^{b}-C(=O)-NR^{b}-SO₂-, -NR^{b}-C(=S)-, -NR^{b}-C(=S)-NR^{b}-, -NR^{b}-SO₂-NR^{b}-, -NR^{b}-C(=NH)-NR^{b}-, -S-, -SO₂-O-, -SO₂-NR^{b}-, -SO₂-NR^{b}C(=O)-NR^{b}-, -C(=O)-O-, -C(=O)-NR^{b}-, -C(=O)-NR^{b}-C(=O)-, -C(=O)-NR^{b}C(=S)-, -C(=S)-NR^{b}-, -C(=S)-NR^{b}-C(=O)-, C(=NH)-NR^{b}-, -C(=O)-, -C(=O)-NR^{b}-C(=NR^{b})-, or -C(=O)-NR^{b}NR^{b}-; R^{a} is optionally substituted alkyl, optionally substituted alkenyl, optionally substituted alkynyl, optionally substituted carbocyclic group, optionally substituted heterocyclic group, or acyl; R^{b} each is independently hydrogen, optionally substituted alkyl, optionally substituted alkenyl, optionally substituted alkynyl, optionally substituted carbocyclic group, optionally substituted heterocyclic group, acyl, hydroxy, or alkoxy;
   R¹⁰ is hydrogen, optionally substituted alkyl, optionally substituted alkenyl, optionally substituted alkynyl, halogen, cyano, formyl, acyl, carboy, alkoxycarbonyl, optionally substituted carbamoyl, isothiocyanato, optionally substituted amino, hydroxy, alkoxy, alkylthio, alkenyloxy, alkynyloxy, alkylsulfinyl, alkylsulfonyl, nitro, or a group of the formula: -Y⁴-R^{c} wherein Y⁴ is single bond, -O-, -S-, -SO-, -SO₂-, -NH-, - C(=O)-, -CH₂-, -C(=O)-NH-, or -NH-C(=O)-; R^{c} is optionally substituted carbocyclic group or optionally substituted heterocyclic group;
   R¹¹ and R¹² each is independently hydrogen, optionally substituted alkyl, optionally substituted alkenyl, optionally substituted alkynyl, halogen, cyano, formyl, acyl, carboxy, alkoxycarbonyl, optionally substituted carbamoyl, isothiocyanato, optionally substituted amino, hydroxy, alkoxy, alkylthio, alkenyloxy, alkynyloxy, alkylsulfinyl, alkylsulfonyl, nitro or a group of the formula: -Y⁵-R^{d} wherein Y⁵ is single bond, optionally substituted alkylene, alkenylene, alkynylene, -O- -S-, -SO-, -SO₂-, NH-, -C(=O)-, -CH₂-, -C(=O)-NH-E-, or -NH-C(=O)-; E is single bond or optionally substituted alkylene; R^{d} is optionally substituted carbocyclic group or optionally substituted heterocyclic group;
   R¹³ is hydrogen, optionally substituted alkyl which may have heteroatom and/or unsaturated bond or a group of the formula: -Y⁶-R-^{e} wherein Y⁶ is single bond, optionally substituted alkylene, alkenylene, alkynylene, -O-, -S-, -SO-, -SO₂-, -NH-, - C(=O)-, -CH₂-, -C(=O)-NH-E-, or -NH-C(=O)-; E is single bond or optionally substituted alkylene; R^{e} is optionally substituted carbocyclic group or optionally substituted heterocyclic group; or
   any one of combinations of R¹⁰ and R¹¹, R¹¹ and R¹², and R¹² and R¹³, taken together with the adjacent atoms form optionally substituted cyclic group which may have heteroatom(s) and/or unsaturated bond(s);
   X is S or O.
11. An antipruritics according to item 10 wherein R⁹ is a group of the formula: -Y¹-Y²-Y³-R^{a} wherein Y¹, Y², Y³ and R^{b} are as defined in claim 10; R^{a} is optionally substituted carbocyclic group, optionally substituted heterocyclic group, or acyl; R¹⁰ is hydrogen atom or optionally substituted alkyl; R¹¹ is optionally substituted alkyl, halogen atom, or a group of the formula: -Y⁵-R^{d} wherein Y⁵ is a single bond or alkynylene; R^{d} are as defined in claim 10; R¹² is hydrogen atom or optionally substituted alkyl; R¹³ is optionally substituted C3 or more alkyl which may have heteroatom(s) and/or unsaturated bond(s) or a group of the formula: -Y⁶-R^{e} wherein Y⁶ and R^{e} are as defined in claim 10; or R¹¹ and R¹² taken together with the adjacent atoms form optionally substituted cyclic group which may have heteroatom(s) and/or unsaturated bond(s).
12. An antipruritics according to item 10 wherein R⁹ is a group of the formula: -Y¹-Y²-Y³-R^{a} wherein Y¹ is a single bond; Y² is -C(=O)-NH-; Y³ is single bond or optionally substituted alkyl; R^{a} is optionally substituted carbocyclic group, or acyl; R¹⁰ is hydrogen atom; R¹¹ and R¹² taken together with the adjacent atoms form optionally substituted cyclic group which may have heteroatom(s) and/or unsaturated bond(s); R¹³ is optionally substituted alkyl which may have heteroatom(s) and/or unsaturated bond(s); X is S or O.
13. A method for treating pruritus which comprises the administration of a compound having an agonistic activity to the cannabinoid receptor, a prodrug, a pharmaceutically acceptable salt or solvate thereof.
14. Use of a compound having an agonistic activity to the cannabinoid receptor, a prodrug, a pharmaceutically acceptable salt or solvate thereof for the preparation of an antipruritics.

### Industrial Applicability

The compound of the present invention has a cannabinoid receptor agonism can be used for inhibiting itching.

## Claims

1. Use of a compound having an agonistic activity to the cannabinoid receptor, a prodrug, a pharmaceutically acceptable salt or solvate thereof for the preparation of an antipruritic, wherein the compound having an agonistic activity to the cannabinoid receptor is a compound selected from 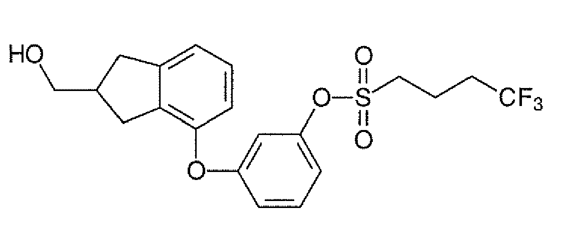 (R,S)-4,4,4-trifiuoro-1-butane sulfonic acid-3-(2-hydroxymethyl-indanyl-4-oxy)-phenylester; 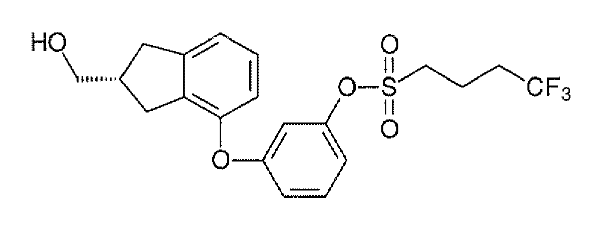 (S)-1-(4,4,4-trifluoro-1-butane sulfonic acid-3-(2-hydroxymethyl-indanyl-4-oxy)phenyl ester; and 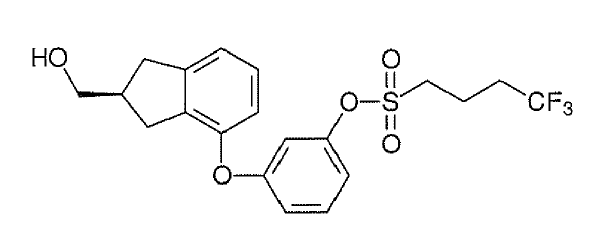 (R)-1-(4,4,4-trifluoro-1-butane sulfonic acid-3-(2-hydroxymethyl-indanyl-4-oxy)phenyl ester.

2. Use according to claim 1, wherein the compound is 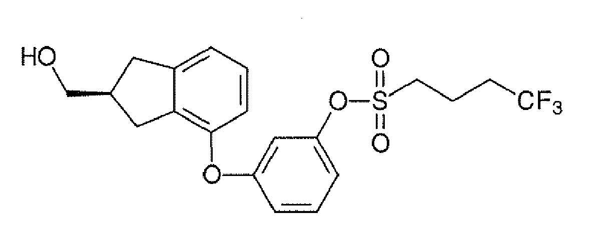 (R)-1-(4,4,4-trifluoro-1-butane sulfonic acid-3-(2-hydroxymethyl-indanyl-4-oxy)phenyl ester.

3. A compound having an agonistic activity to the cannabinoid receptor, a prodrug, a pharmaceutically acceptable salt or solvate thereof for use as an antipruritic, wherein the compound is as defined in claim 1 or 2.
